# EUROPEAN PATENT APPLICATION

(11) **EP 2 075 309 A2**
(43) Date of publication of application: **01.07.2009**
(21) Application number: 08254194.7
(22) Date of filing: 31.12.2008
(51) Int. Cl.: C09K 11/06, C09K 11/07, C07C 211/47, C07C 211/58, C07C 211/60, C07C 211/61

(54) **Novel organic electroluminescent compounds and organic electroluminescent device using the same**

(30) Priority: 31.12.2007 KR 20070142000
(71) Applicant: Gracel Display Inc., Seoul 133-833 (KR)
(72) Inventor: Eum, Sung Jin, Seoul 152-053 (KR); Cho, Young Jun, Seoul 136-060 (KR); Kwon, Hyuck Joo, Seoul 130-100 (KR); Kim, Bong Ok, Seoul 135-090 (KR); Kim, Sung Min, Seoul-city 157-886 (KR); Yoon, Seung Soo, Seoul 135-884 (KR)
(74) Representative: Kent, Venetia Katherine

(57) **Abstract**

The present invention relates to novel organic electroluminescent compounds, and organic electroluminescent devices employing the same as electroluminescent material. Specifically, the organic electroluminescent compounds according to the invention are **characterized in that** they are represented by Chemical Formula (1):

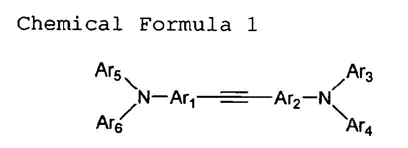

Since the organic electroluminescent compounds according to the invention have good luminous efficiency and life property of material, OLED's having very good operation lifetime can be manufactured.

## Description

### FIELD OF THE INVENTION

The present invention relates to novel organic electroluminescent compounds, and organic electroluminescent devices employing the same as electroluminescent material.

### BACKGROUND ART

Among display devices, electroluminescence devices (EL devices) are self-luminescent display devices showing the advantage of wide angle of view, excellent contrast and rapid response rate. Eastman Kodak developed in 1987 an organic EL device which employs a low molecular weight aromatic diamine and an aluminum complex, as a material for forming an EL layer, for the first time [Appl. Phys. Lett. 51, 913, 1987].

The most important factor to determine luminous efficiency, lifetime or the like in an organic EL device is electroluminescent material. Several properties required for such electroluminescent materials include that the material should have high fluorescent quantum yield in solid state and high mobility of electrons and holes, is not easily decomposed during vapor-deposition in vacuo, and forms uniform and stable thin film.

Organic electroluminescent materials can be generally classified into high-molecular materials and low-molecular materials. The low-molecular materials include metal complexes and purely organic electroluminescent materials which do not contain metal, from the aspect of molecular structure. Such electroluminescent materials include chelate complexes such as tris(8-quinolinolato)aluminum complexes, coumarin derivatives, tetraphenylbutadiene derivatives, bis(styrylarylene) derivatives, oxadiazole derivatives. From those materials, it is reported that light emission of visible region from blue to red can be obtained, so that the realization of full-colored display devices is expected.

In the meanwhile, for blue materials, a number of materials have been developed and commercialized since the development of DPVBi (Chemical Formula a) by Idemitsu-Kosan. In addition to the blue material system from Idemitsu-Kosan, dinaphthylanthracene (Chemical Formula b), tetra(t-butyl)perylene (Chemical Formula c) system or the like have been known. However, extensive research and development should be performed with respect to these materials. The distryl compound system of Idemitsu-Kosan, which is known to have highest efficiency up to now, has 6 lm/W of power efficiency and beneficial device lifetime of more than 30,000 hr. However, when it is applied to a full-colored display, the lifetime is merely several thousand hours, owing to the reduction of color purity over operation time. In case of blue electroluminescentce, it becomes advantageous from the aspect of the luminous efficiency, if the electroluminescent wavelength is shifted a little toward longer wavelength. However, it is not easy to apply the material to a display of high quality because of unsatisfactory color purity in blue. In addition, the research and development of such materials are urgent because of the problems in color purity, efficiency and thermal stability.

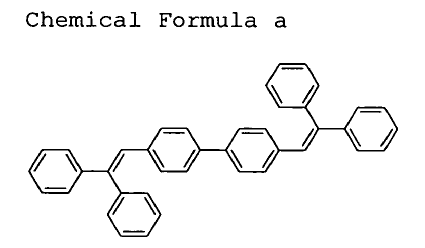

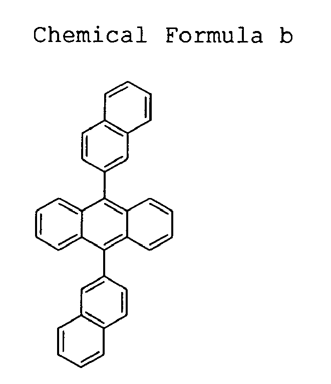

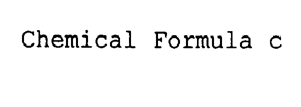

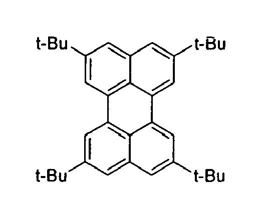

### SUMMARY OF THE INVENTION

Thus, the inventors have intensively endeavored to overcome the problems described above and to develop a novel electroluminescent compound which can realize an organic electroluminescent device having excellent luminous efficiency and noticeably improved device life.

The object of the invention is to overcome the drawbacks of blue material as described above and to provide an organic electroluminescent compound having improved luminous efficiency and device life.

Another object of the invention is to provide an organic electroluminescent device having high efficiency and long life, which comprises the organic electroluminescent compounds described above as electroluminescent material. Another object of the present invention is to provide an organic electroluminescent device comprising an electroluminescent region which employs one or more compound(s) selected from anthracene derivatives and benz[a]anthracene derivatives as an electroluminescent host, in addition to one or more organic electroluminescent compound(s) as mentioned above.

Still another object of the present invention is to provide organic solar cells comprising said novel organic electroluminescent compounds.

Specifically, the present invention relates to novel organic electroluminescent compounds represented by Chemical Formula (1), and organic electroluminescent devices employing the same in the electroluminescent layer. 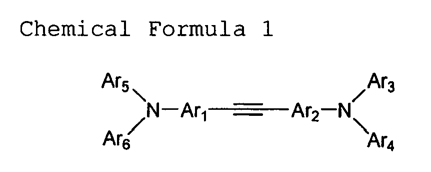 wherein,
Ar₁ and Ar₂ independently represent (C6-C60)arylene or (C5-C60)heteroarylene, and the arylene or heteroarylene of Ar₁ and Ar₂ may be further substituted by one or more substituent(s) selected from deuterium, linear or branched (C1-C60)alkyl and (C6-C60)aryl;
Ar₃ through Ar₆ independently represent linear or branched (C1-C60)alkyl, (C3-C60)cycloalkyl, 5- or 6-membered heterocycloalkyl containing one or more heteroatom(s) selected from N, O and S, (C6-C60)aryl or (C3-C60)heteroaryl, or Ar₃ and Ar₅, or Ar₆ and Ar₇ may be linked via (C3-C60)alkylene or (C3-C60)alkenylene with or without a fused ring to form an alicyclic ring, or a monocyclic or polycyclic aromatic ring; and
the aryl or heteroaryl of Ar₃ through Ar₆ may be further substituted by one or more substituent(s) selected from a group consisting of deuterium, (C6-C60)aryl with or without linear or branched (C1-C60)alkyl or (C6-C60)aryl substituent, linear or branched (C1-C60)alkyl with or without halogen substituent(s), (C1-C30)alkoxy, (C3-C60)cycloalkyl, halogen, cyano, tri(C1-C30)alkylsilyl, di(C1-C30)alkyl(C6-C30)arylsilyl and tri(C6-C30)arylsilyl.

### BRIEF DESCRIPTION OF THE DRAWINGS

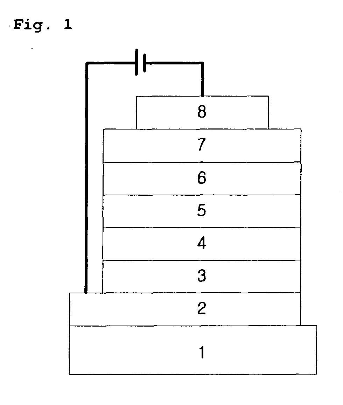

Fig. 1 is a cross-sectional view of an organic light emitting diode (OLED).

### DETAILED DESCRIPTION OF THE INVENTION

Referring now to the Drawings, Fig. 1 illustrates AN OLED of the present invention comprising a Glass 1, a Transparent electrode 2, a Hole injection layer 3, a Hole transport layer 4, an Electroluminescent layer 5, an Electron transport layer 6, an Electron injection layer 7 and an A1 cathode 8.

The term "alkyl", "alkoxy" described herein and any substituents comprising "alkyl" moiety include both linear and branched species:

The term "aryl" described herein means an organic radical derived from aromatic hydrocarbon via elimination of one hydrogen atom. Each ring comprises a monocyclic or fused ring system containing from 4 to 7, preferably from 5 to 6 cyclic atoms. Specific examples include phenyl, naphthyl, biphenyl, anthryl, tetrahydronaphthyl, indenyl, fluorenyl, phenanthryl, triphenylenyl, pyrenyl, perylenyl, chrysenyl, naphthacenyl and fluoranthenyl, but they are not restricted thereto.

The term "heteroaryl" described herein means an aryl group containing from 1 to 4 heteroatom(s) selected from N, O and S as the aromatic cyclic backbone atom(s), and carbon atom(s) for remaining aromatic cyclic backbone atoms. The heteroaryl may be a 5- or 6-membered monocyclic heteroaryl or a polycyclic heteroaryl which is fused with one or more benzene ring(s), and may be partially saturated. The heteroaryl group may comprise a bivalent aryl group, of which the heteroatoms may be oxidized or quaternized to form N-oxide and quaternary salt. Specific examples include monocyclic heteroaryl groups such as furyl, thiophenyl, pyrrolyl, imidazolyl, pyrazolyl, thiazolyl, thiadiazolyl, isothiazolyl, isoxazolyl, oxazolyl, oxadiazolyl, triazinyl, tetrazinyl, triazolyl, tetrazolyl, furazanyl, pyridyl, pyrazinyl, pyrimidinyl, pyridazinyl; polycyclic heteroaryl groups such as benzofuranyl, benzothiophenyl, isobenzofuranyl, benzimidazolyl, benzothiazolyl, benzisothiazolyl, benzisoxazolyl, benzoxazolyl, isoindolyl, indolyl, indazolyl, benzothiadiazolyl, quinolyl, isoquinolyl, cinnolinyl, quinazolinyl, quinoxalinyl, carbazolyl, phenanthridinyl and benzodioxolyl; and corresponding N-oxides (for example, pyridyl N-oxide, quinolyl N-oxide) and quaternary salts thereof; but they are not restricted thereto.

The substituents comprising "(C1-C60)alkyl" moiety described herein may contain 1 to 60 carbon atoms, 1 to 20 carbon atoms, or 1 to 10 carbon atoms. The substituents comprising "(C6-C60)aryl" moiety may contain 6 to 60 carbon atoms, 6 to 20 carbon atoms, or 6 to 12 carbon atoms. The substituents comprising "(C3-C60)heteroaryl" moiety may contain 3 to 60 carbon atoms, 4 to 20 carbon atoms, or 4 to 12 carbon atoms. The substituents comprising "(C3-C60)cycloalkyl" moiety may contain 3 to 60 carbon atoms, 3 to 20 carbon atoms, or 3 to 7 carbon atoms. The substituents comprising "(C2-C60)alkenyl or alkynyl" moiety may contain 2 to 60 carbon atoms, 2 to 20 carbon atoms, or 2 to 10 carbon atoms.

In Chemical Formula (1), Ar₁ and Ar₂ are independently selected from the following structures, but they are not restricted thereto: 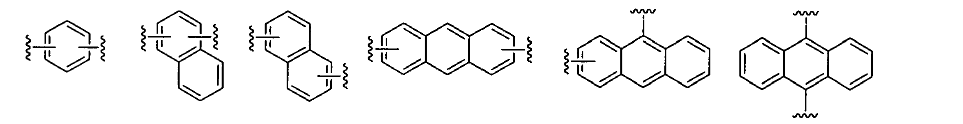 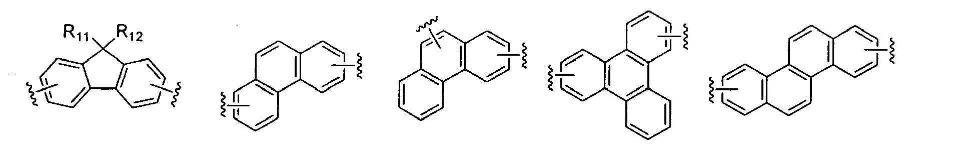 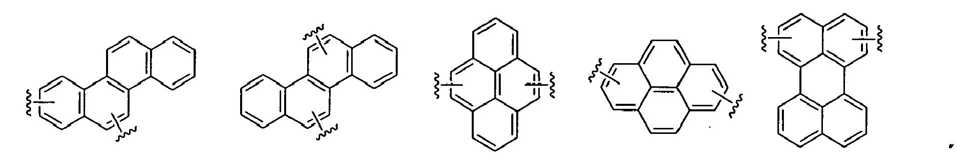 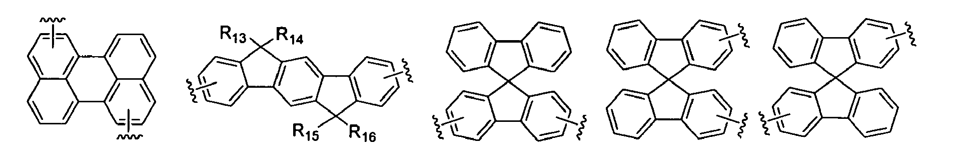 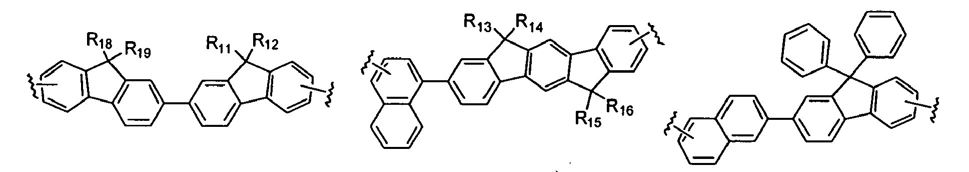 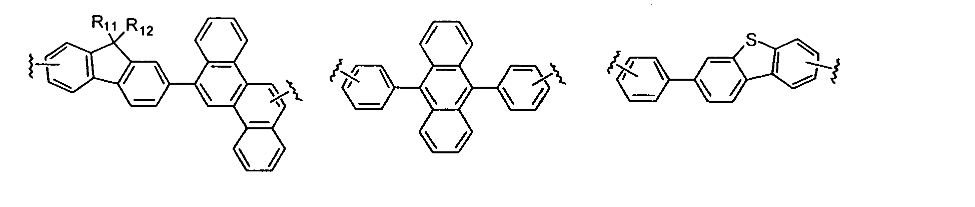 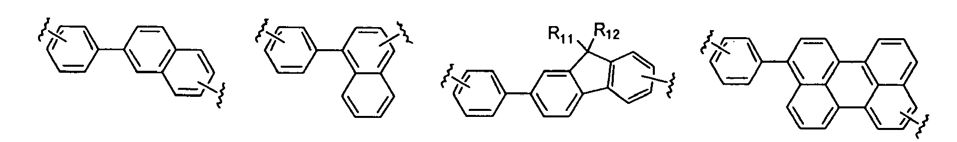 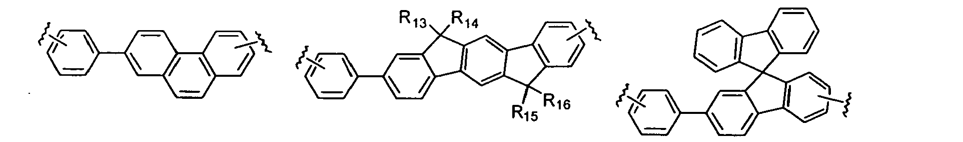 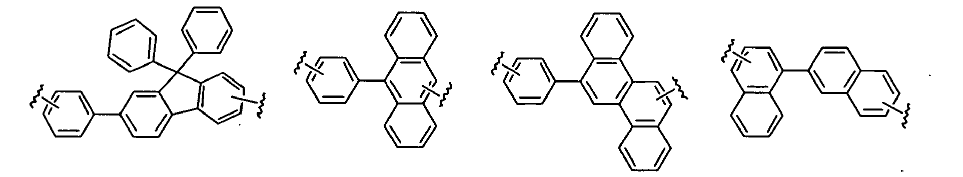 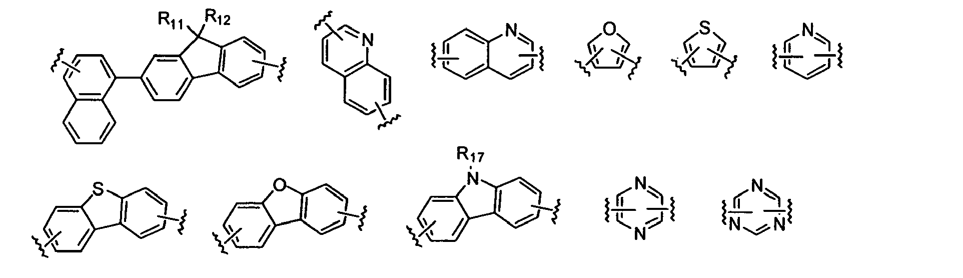 wherein, R₁₁ through R₁₉ independently represent hydrogen, linear or branched (C1-C60)alkyl or (C6-C60)aryl, and the aryl may be further substituted by linear or branched (C1-C60)alkyl.

In Chemical Formula (1), Ar₃ through Ar₆ independently represent phenyl, naphthyl, fluorenyl, anthryl, phenanthryl, pyrenyl, perylenyl, fluoranthenyl, pyridyl, pyrrolyl, furanyl, thiophenyl, imidazolyl, benzimidazolyl, pyrazinyl, pyrimidinyl, pyridazinyl, quinolyl, triazinyl, benzofuranyl, benzothiophenyl, pyrazolyl, indolyl, carbazolyl, thiazolyl, oxazolyl, benzothiazolyl, benzoxazolyl, morpholino or thiomorpholino; and the phenyl, naphthyl, fluorenyl, anthryl, phenanthryl, pyrenyl, perylenyl, fluoranthenyl, pyridyl, pyrrolyl, furanyl, thiophenyl, imidazolyl, benzimidazolyl, pyrazinyl, pyrimidinyl, pyridazinyl, quinolyl, triazinyl, benzofuranyl, benzothiophenyl, pyrazolyl, indolyl, carbazolyl, thiazolyl, oxazolyl, benzothiazolyl, benzoxazolyl, morpholino or thiomorpholino may be further substituted by one or more substituent(s) selected from a group consisting of deuterium, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopentyl, cyclohexyl, cycloheptyl, cyclooctyl, fluoro, cyano, trimethylsilyl, triethylsilyl, tripropylsilyl, tri(t-butyl)silyl, t-butyldimethylsilyl, dimethylphenylsilyl, triphenylsilyl, phenyl, biphenyl, 9,9-dimethylfluorenyl, 9,9-diphenylfluorenyl, naphthyl, phenanthryl and anthryl.

The naphthyl of Chemical Formula (1) may be 1-naphthyl or 2-naphthyl; the anthryl may be 1-anthryl, 2-anthryl or 9-anthryl; and the fluorenyl may be 1-fluorenyl, 2-fluorenyl, 3-fluorenyl, 4-fluorenyl or 9-fluorenyl.

The organic electroluminescent compounds according to the present invention can be specifically exemplified by the following compounds, but they are not restricted thereto: 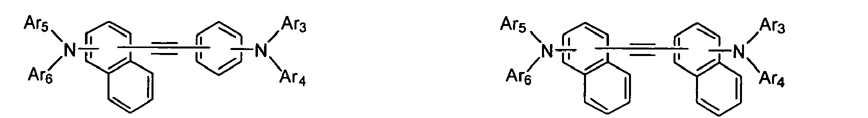 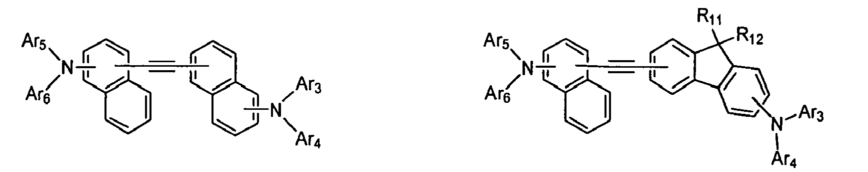 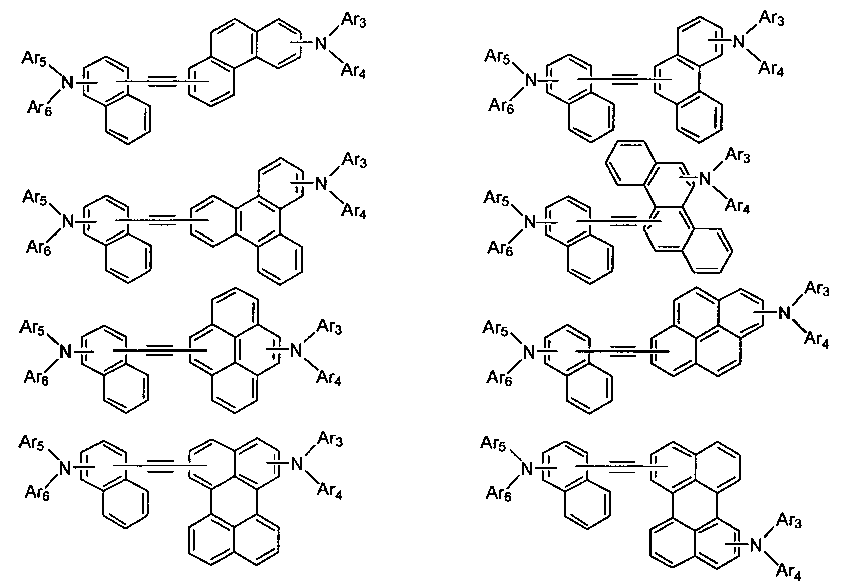 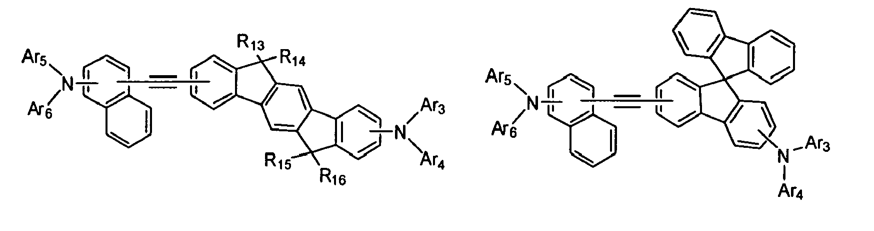 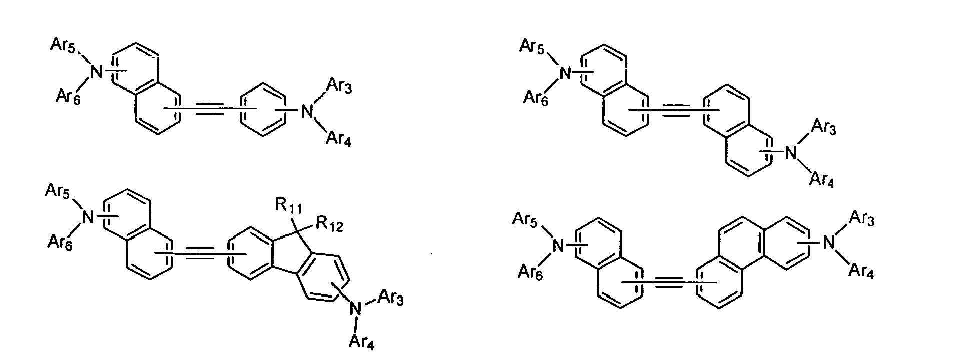 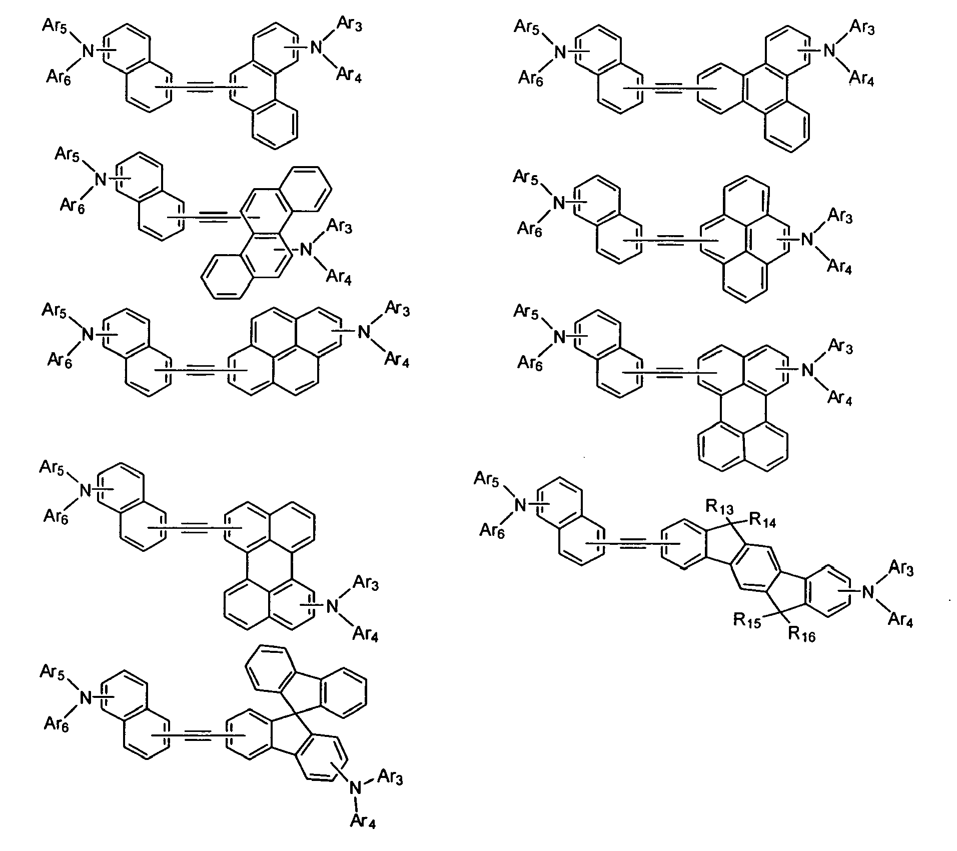 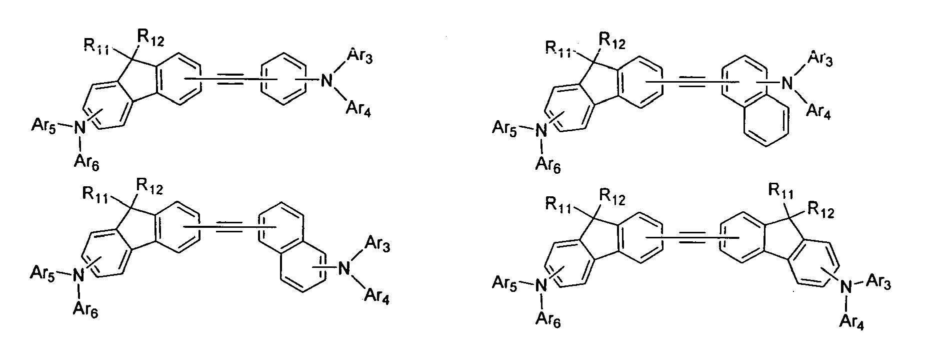 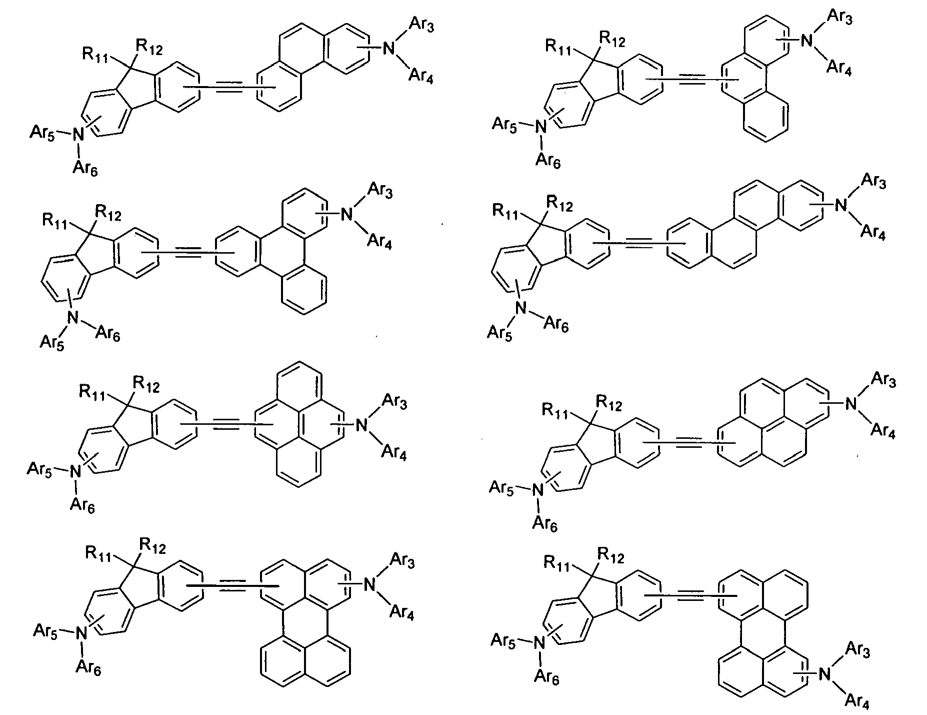 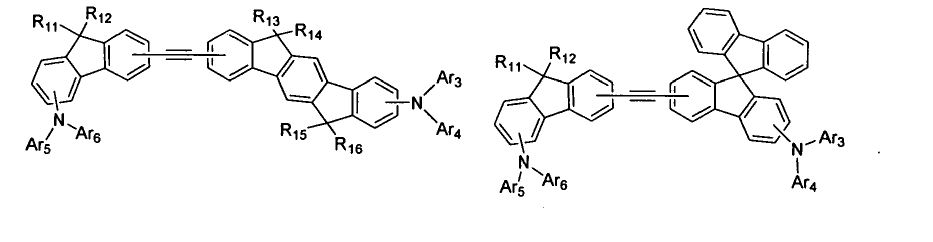 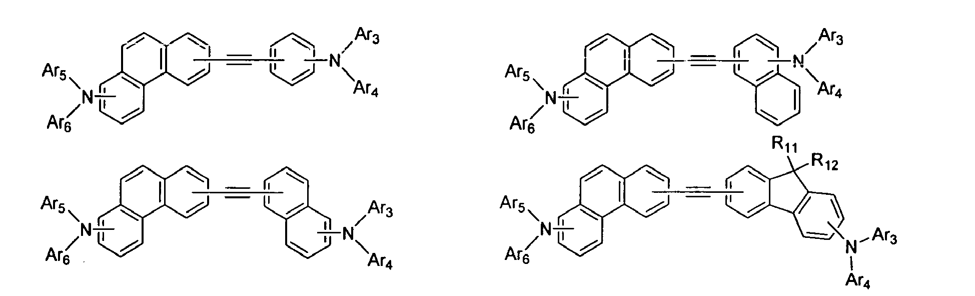 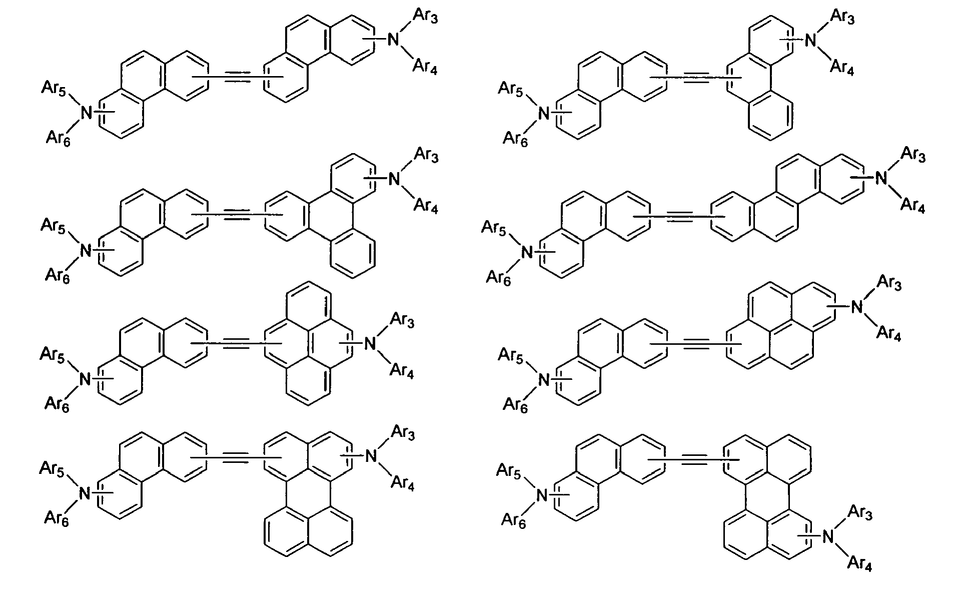 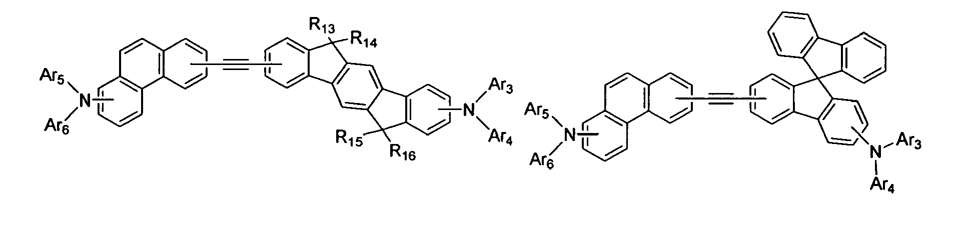 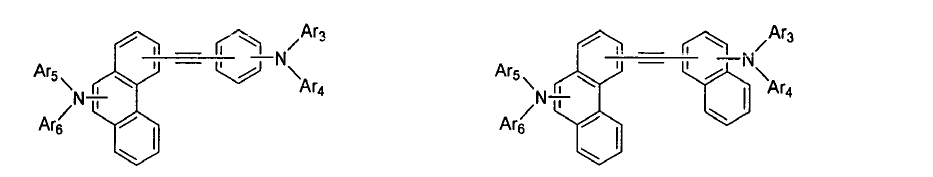 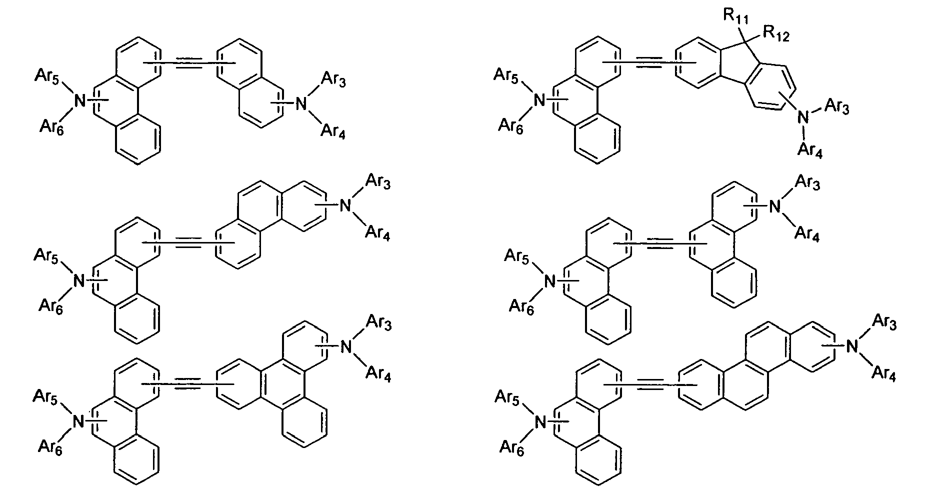 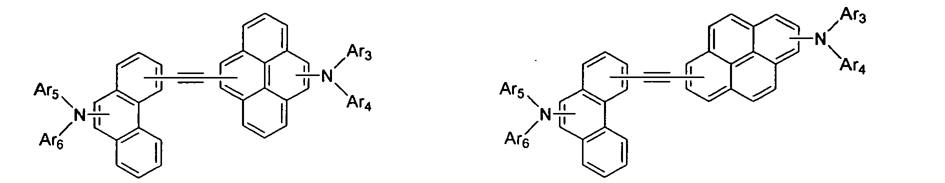 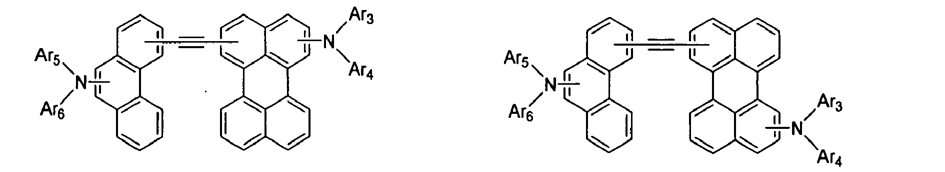 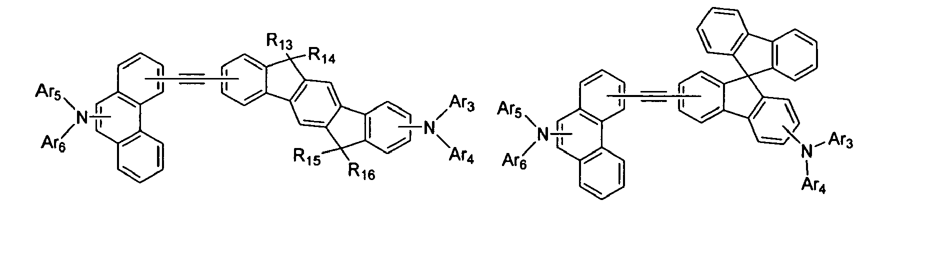 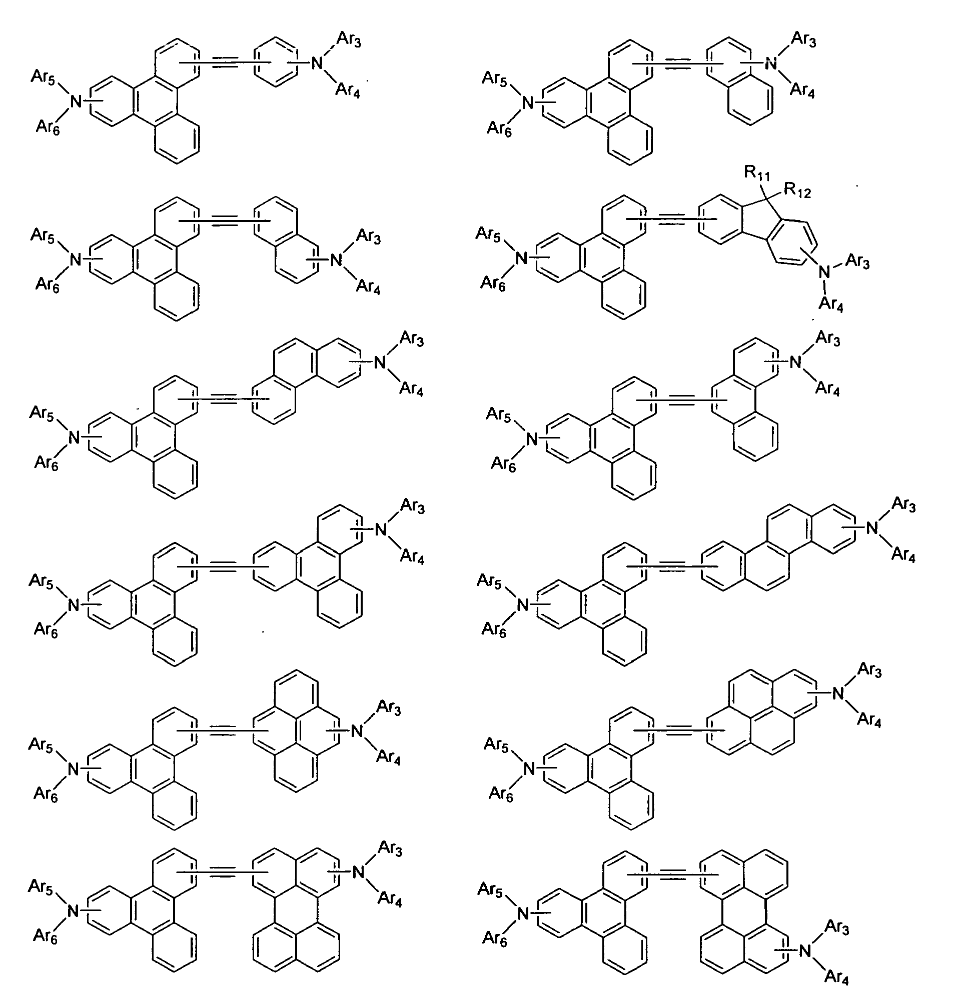 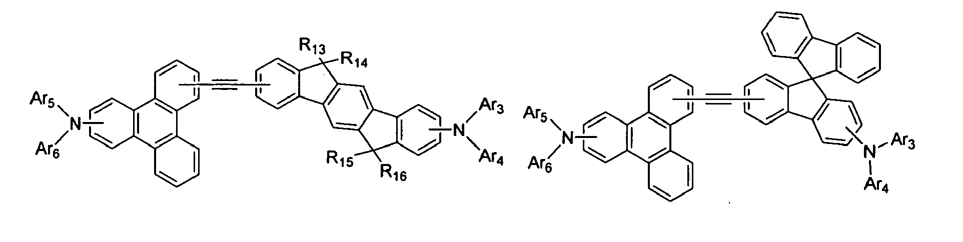 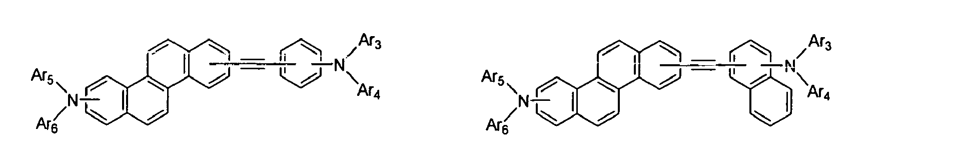 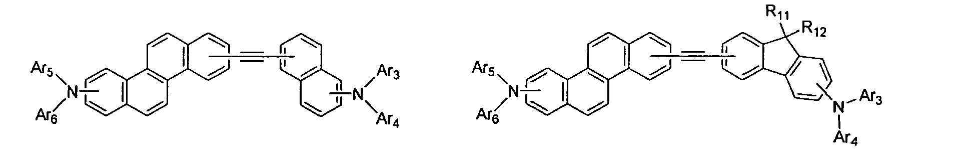 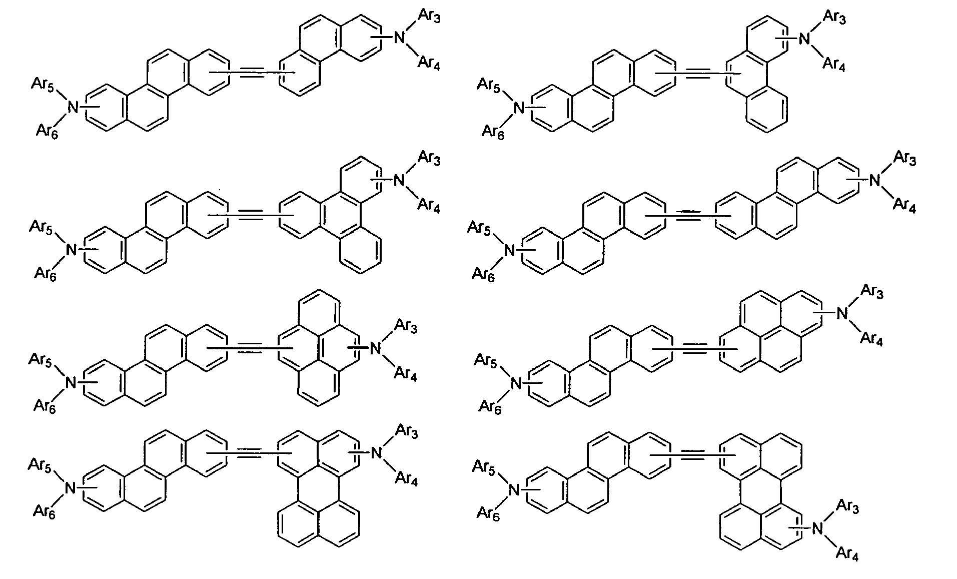 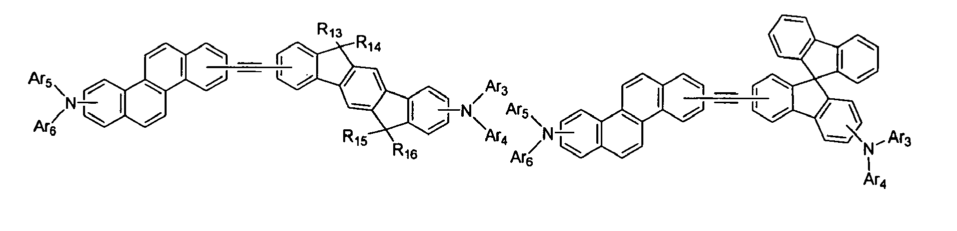 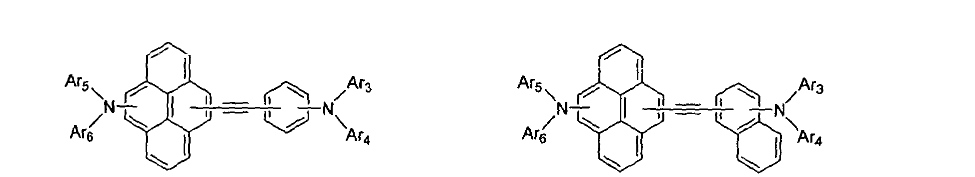 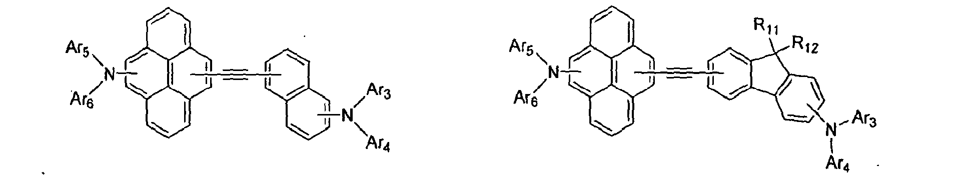 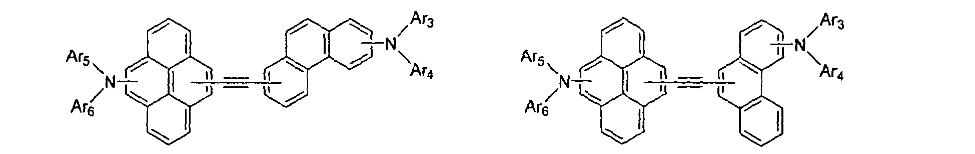 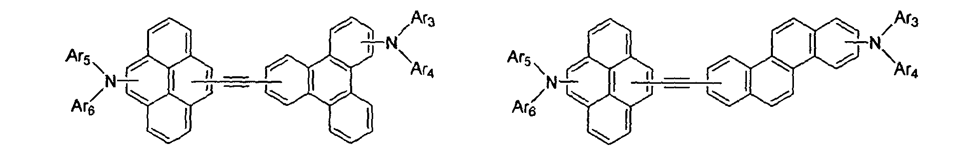 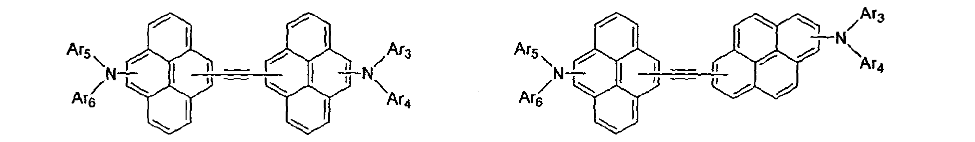 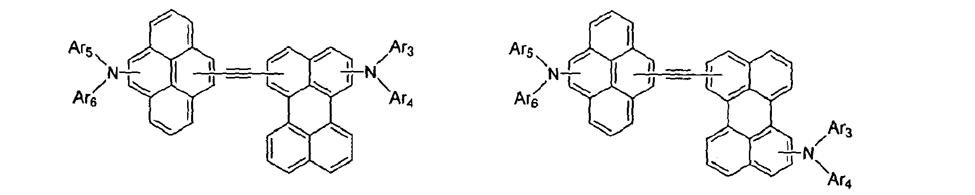 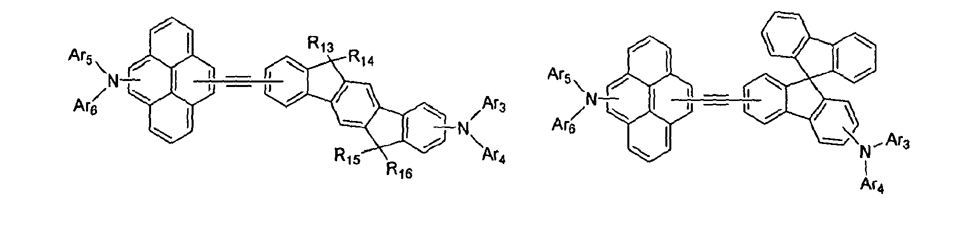 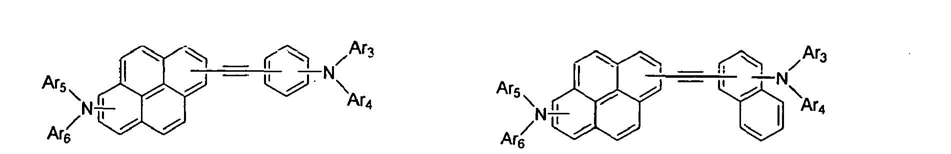 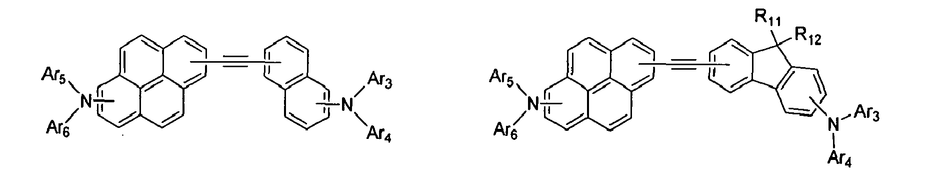 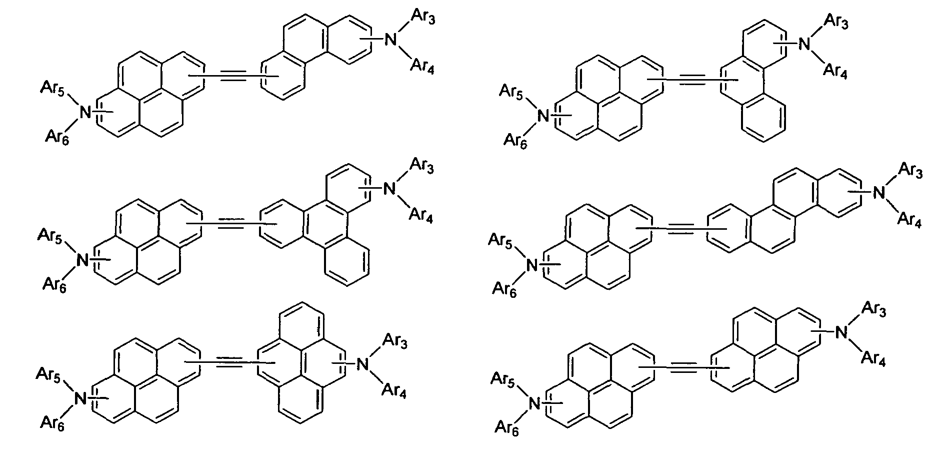 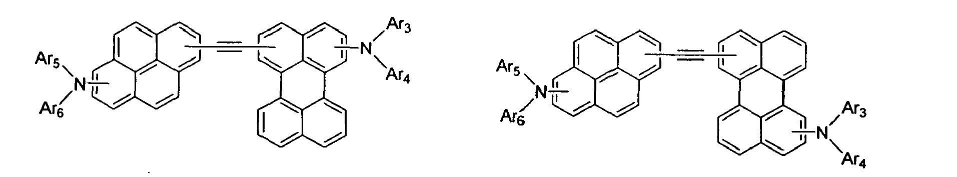 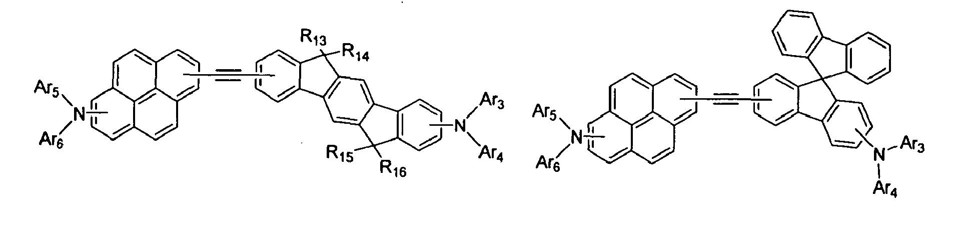 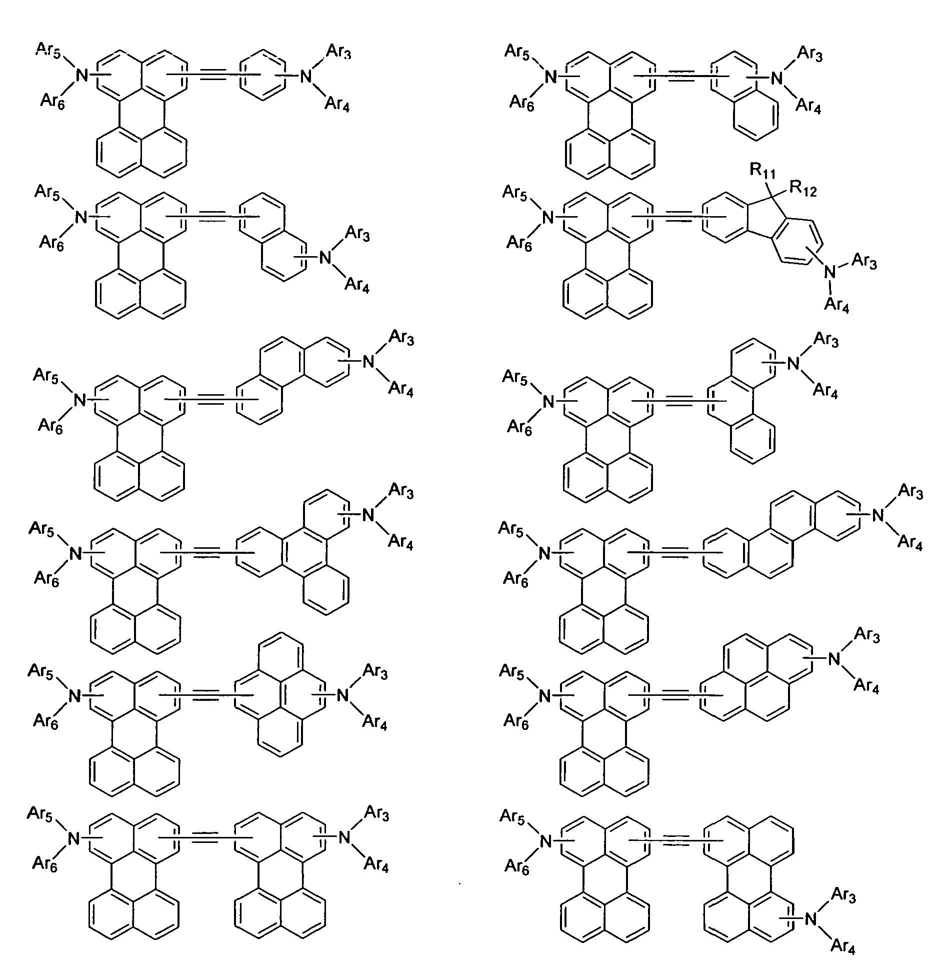 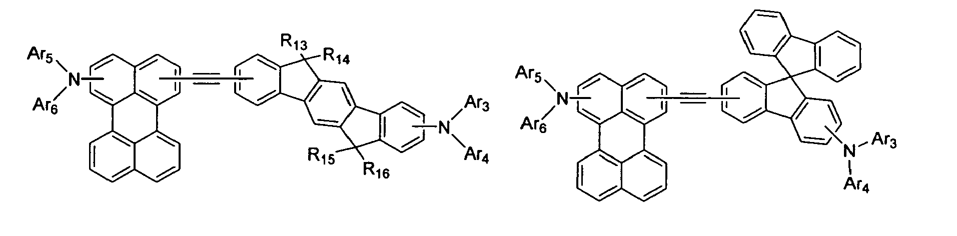 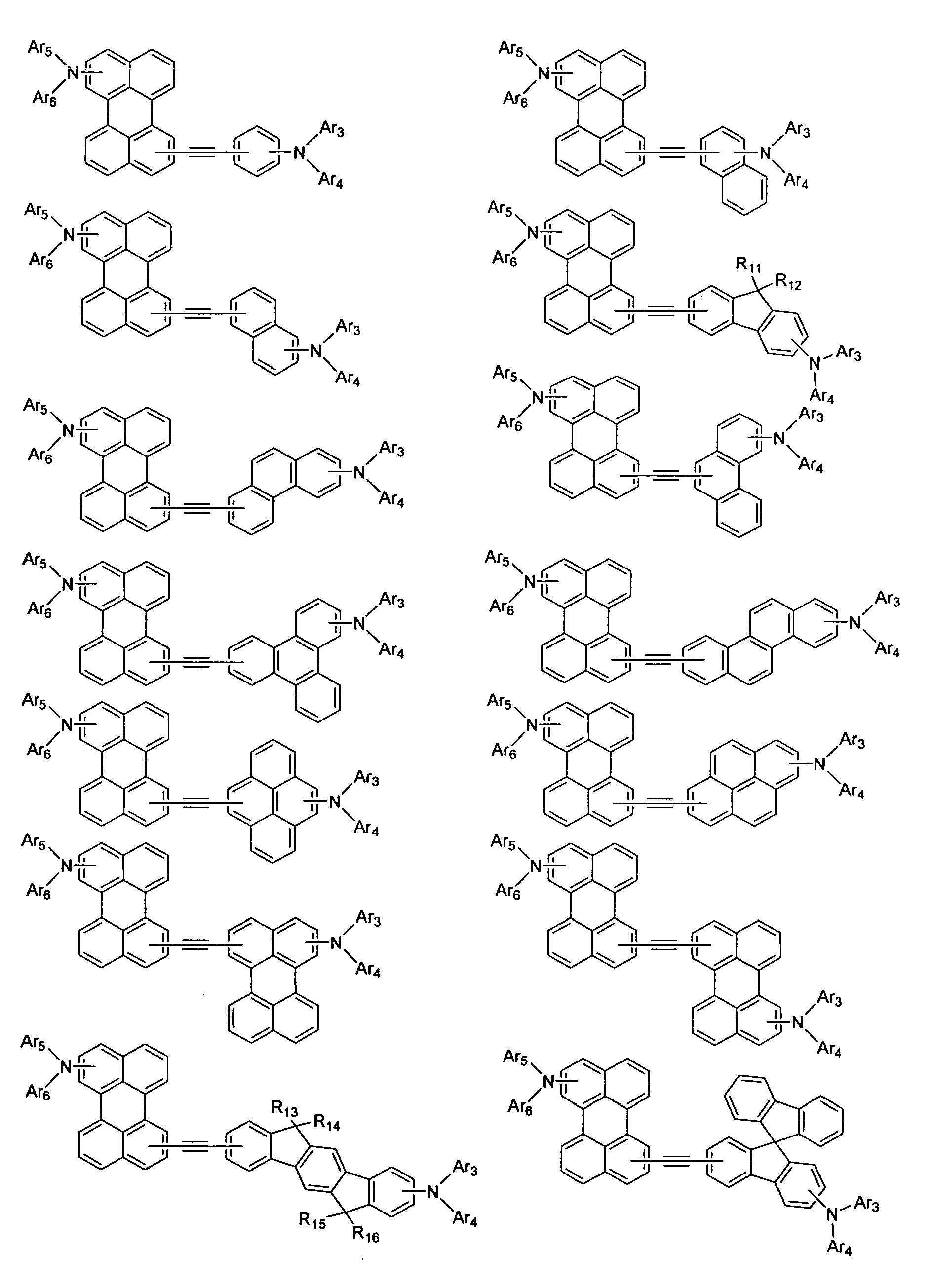 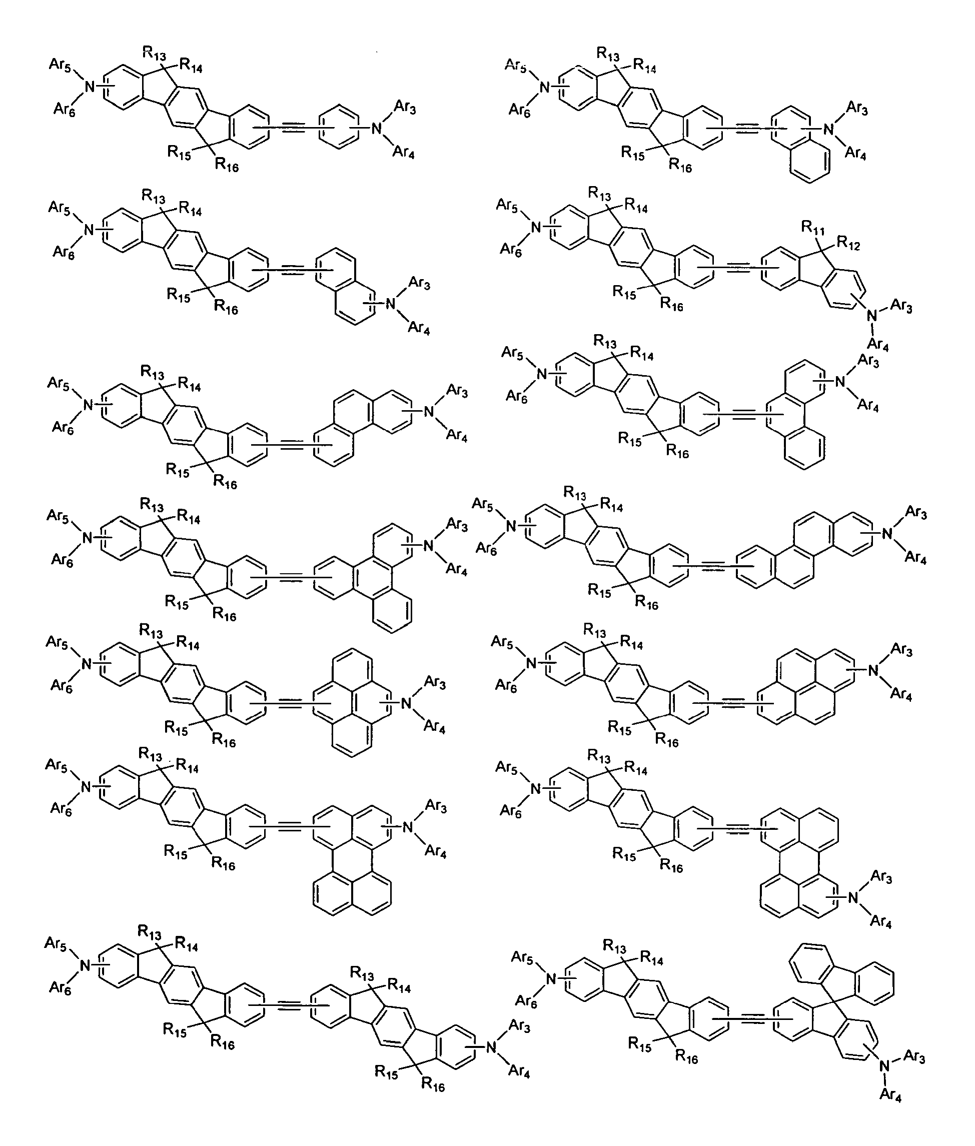 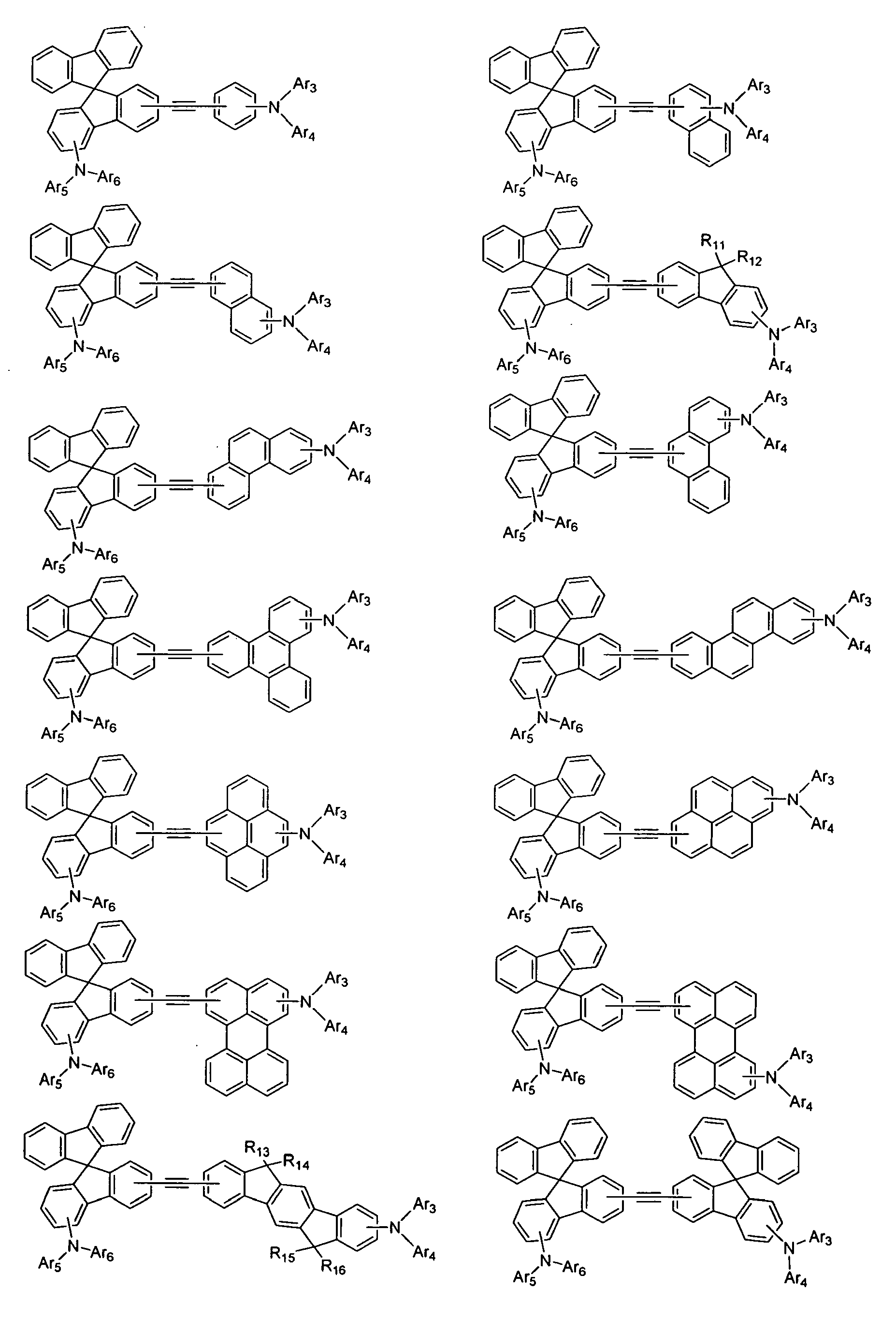 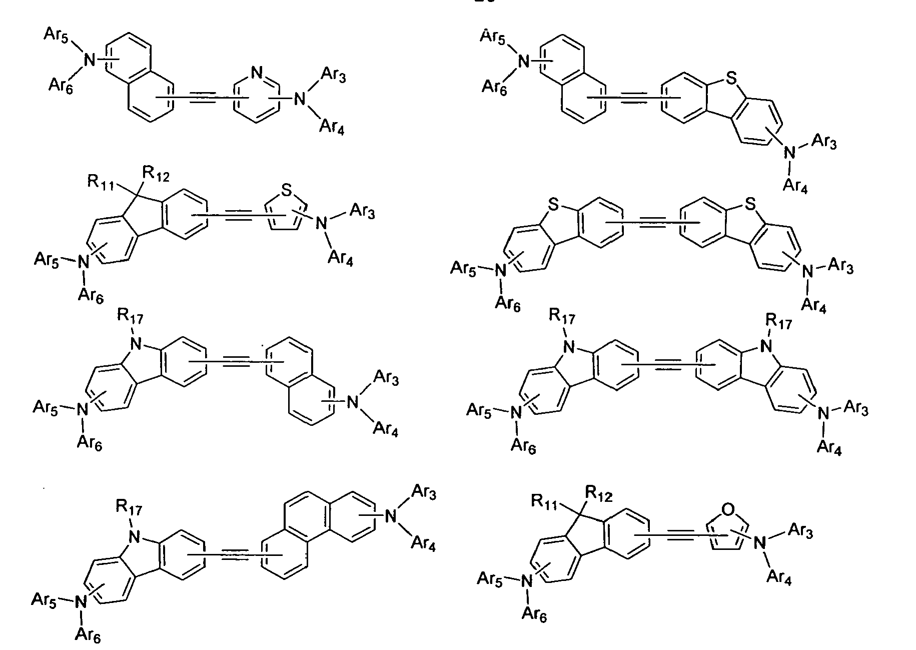 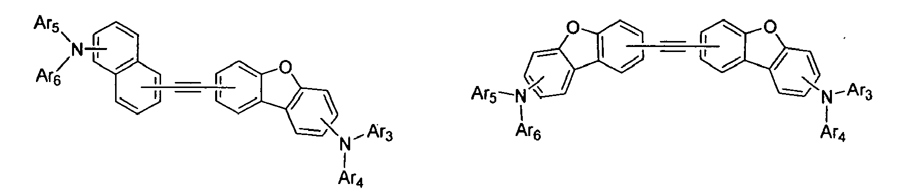 wherein, Ar₃ through Ar₆ independently represent phenyl, naphthyl, fluorenyl, anthryl, phenanthryl, pyrenyl, perylenyl, fluoranthenyl, pyridyl, pyrrolyl, furanyl, thiophenyl, imidazolyl, benzimidazolyl, pyrazinyl, pyrimidinyl, pyridazinyl, quinolyl, triazinyl, benzofuranyl, benzothiophenyl, pyrazolyl, indolyl, carbazolyl, thiazolyl, oxazolyl, benzothiazolyl, benzoxazolyl, morpholino or thiomorpholino;
the phenyl, naphthyl, fluorenyl, anthryl, phenanthryl, pyrenyl, perylenyl, fluoranthenyl, pyridyl, pyrrolyl, furanyl, thiophenyl, imidazolyl, benzimidazolyl, pyrazinyl, pyrimidinyl, pyridazinyl, quinolyl, triazinyl, benzofuranyl, benzothiophenyl, pyrazolyl, indolyl, carbazolyl, thiazolyl, oxazolyl, benzothiazolyl, benzoxazolyl, morpholino or thiomorpholino of Ar₃ through Ar₆ may be further substituted by one or more substituent(s) selected from a group consisting of deuterium, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, trifluoromethyl, perfluoroethyl, trifluoroethyl, perfluoropropyl, perfluorobutyl, methoxy, ethoxy, butoxy, hexyloxy, cyclopentyl, cyclohexyl, cycloheptyl, cyclooctyl, fluoro, cyano, trimethylsilyl, triethylsilyl, tripropylsilyl, tri(t-butyl)silyl, t-butyldimethylsilyl, dimethylphenylsilyl, triphenylsilyl, phenyl, biphenyl, 9,9-dimethylfluorenyl, 9,9-diphenylfluorenyl, naphthyl, phenanthryl and anthryl;
R₁₁ through R₁₆ independently represent hydrogen, deuterium, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl, hexadecyl, phenyl, naphthyl, biphenyl, fluorenyl, phenanthryl, anthryl, fluoranthenyl, triphenylenyl, pyrenyl, chrysenyl, naphthacenyl or perylenyl; and
the phenyl, naphthyl, biphenyl, fluorenyl, phenanthryl, anthryl, fluoranthenyl, triphenylenyl, pyrenyl, chrysenyl, naphthacenyl or perylenyl of R₁₁ through R₁₆ may be further substituted by one or more substituent(s) selected from deuterium, methyl, ethyl, n-propyl, i-propyl, n-butyl, i-butyl, t-butyl, n-pentyl, i-pentyl, n-hexyl, n-heptyl, n-octyl, 2-ethylhexyl, n-nonyl, decyl, dodecyl and hexadecyl.

More specifically, the organic electroluminescent compounds according to the present invention can be exemplified by the following compounds, but they are not restricted thereto.

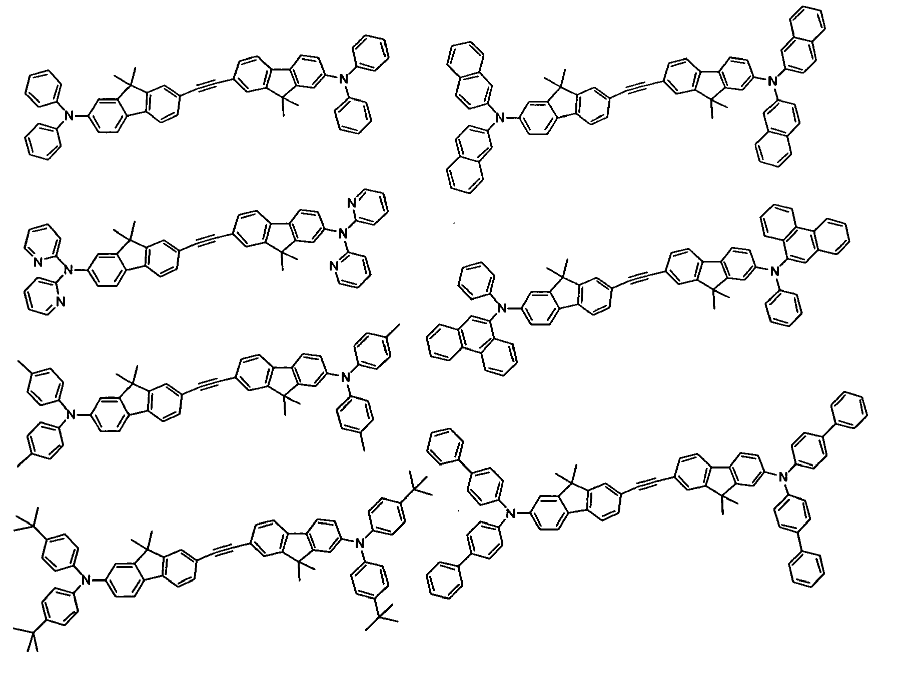

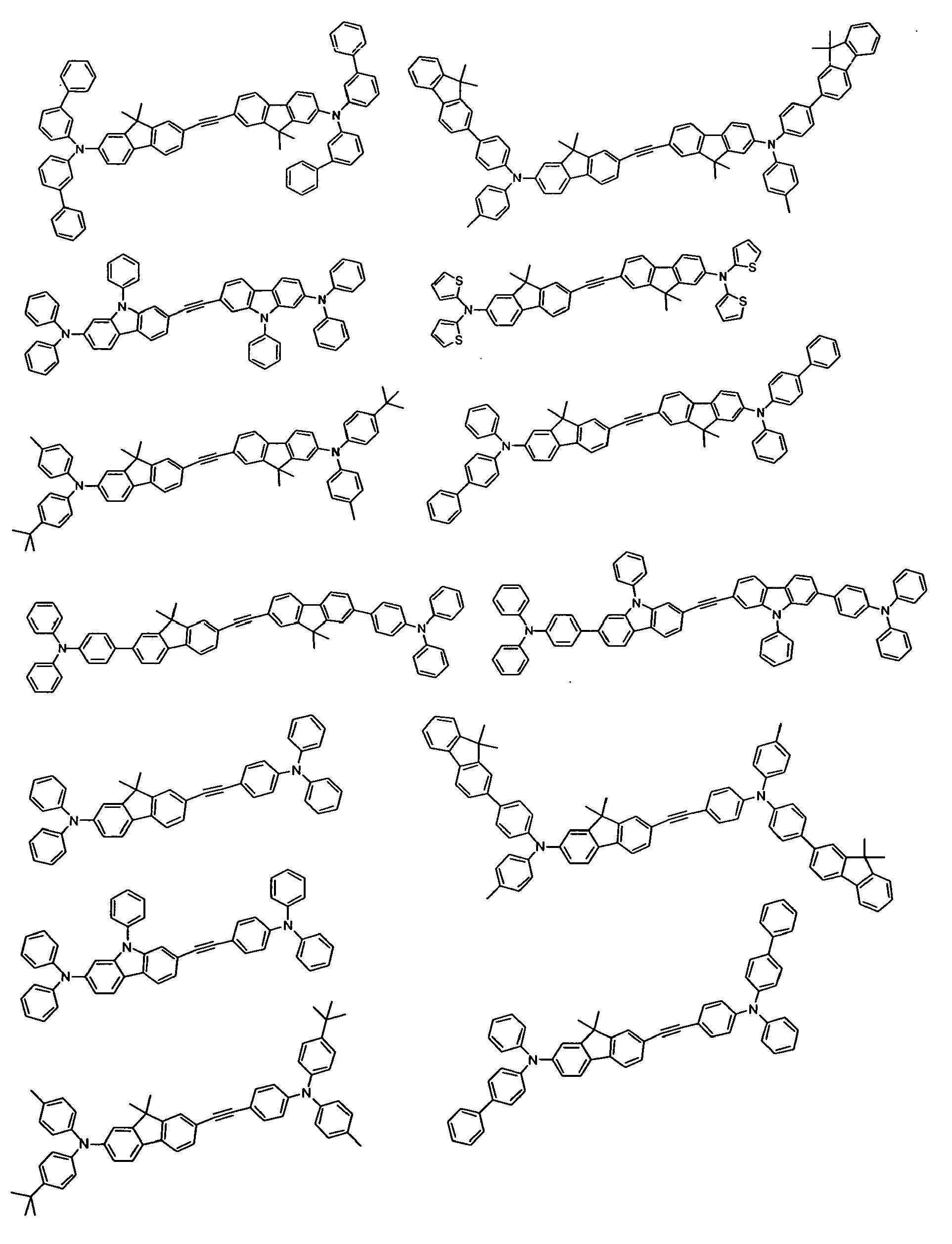

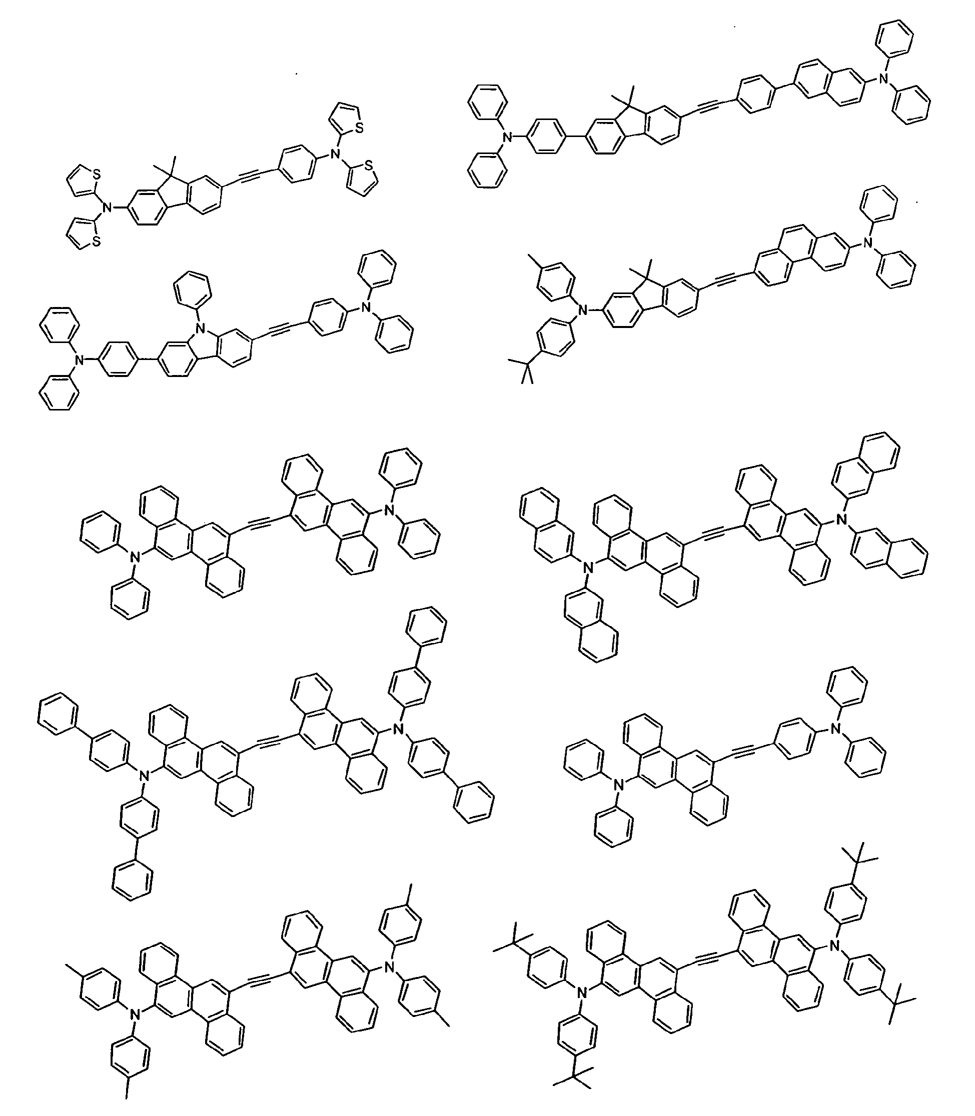

The organic electroluminescent compounds according to the present invention can be prepared according to the procedure illustrated by Reaction Scheme (1): 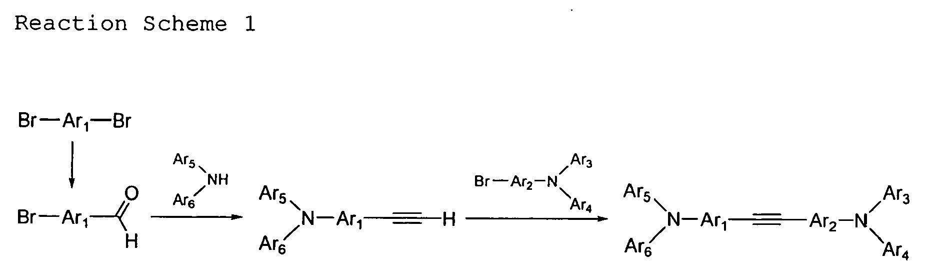 wherein, Ar₁, Ar₂, Ar₃, Ar₄, Ar₅ and Ar₆ are defined as in Chemical Formula (1).

In addition, the present invention provides organic solar cells, which comprise one or more organic electroluminescent compound(s) represented by Chemical Formula (1).

The present invention also provides an organic electroluminescent device which is comprised of a first electrode; a second electrode; and at least one organic layer(s) interposed between the first electrode and the second electrode; wherein the organic layer comprises one or more organic electroluminescent compound(s) represented by Chemical Formula (1).

The organic electroluminescent device according to the present invention is characterized in that the organic layer comprises an electroluminescent region, and the region comprises one or more compound(s) represented by Chemical Formula (1) as electroluminescent dopant, and one or more host(s).

The host applied to the electroluminescent device according to the invention is not particularly restricted, but preferably selected from the compounds represented Chemical Formula (2) or (3):

Chemical Formula 2 (Ar₁₁)ₐ-A-(Ar₁₂)_{b}

Chemical Formula 3 (Ar₁₁)ₐ-An-(Ar₁₂)_{b}

wherein,
Ar₁₁ and Ar₁₂ are independently selected from hydrogen, (C1-C60)alkyl, (C1-C60)alkoxy, halogen, (C4-C60)heteroaryl, (C5-C60)cycloalkyl and (C6-C60)aryl; and the cycloalkyl, aryl or heteroaryl of Ar₁₁ and Ar₁₂ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
A represents (C6-C60)arylene or (C4-C60)heteroarylene;
a and b independently represent an integer from 0 to 4; and
An comprises anthracene backbone with or without a substituent.

The hosts represented by Chemical Formula (2) or (3) can be exemplified by anthracene derivatives or benz[a]anthracene derivatives represented by one of Chemical Formulas (4) to (7).

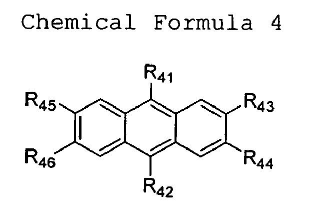

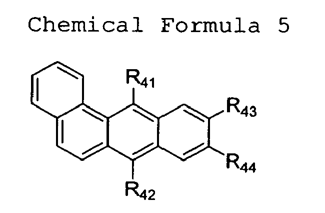

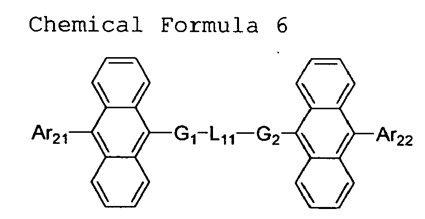

In Chemical Formulas (4) to (6),
R₄₁ and R₄₂ independently represent (C6-C60)aryl, (C4-C60)heteroaryl, a 5- or 6-membered heterocycloalkyl containing one or more heteroatom(s) selected from N, O and S, or (C3-C60)cycloalkyl, and the aryl or heteroaryl of R₄₁ and R₄₂ may be further substituted by one or more substituent(s) selected from a group consisting of (C1-C60)alkyl, halo(C1-C60)alkyl, (C1-C60)alkoxy, (C3-C60)cycloalkyl, (C6-C60)aryl, (C4-C60)heteroaryl, halogen, cyano, tri(C1-C60)alkylsilyl, di(C1-C60)alkyl(C6-C60)arylsilyl and tri(C6-C60)arylsilyl;
R₄₃ through R₄₆ independently represent hydrogen, (C1-C60)alkyl, (C1-C60)alkoxy, halogen, (C4-C60)heteroaryl, (C5-C60)cycloalkyl or (C6-C60)aryl, and the heteroayl, cycloalkyl or aryl of R₄₃ through R₄₆ may be further substituted by one or more substituent(s) selected from a group consisting of (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
G₁ and G₂ independently represent a chemical bond, or (C6-C60)arylene with or without one or more substituent(s) selected from (C1-C60)alkyl, (C1-C60)alkoxy, (C6-C60)aryl, (C4-C60)heteroaryl and halogen;
Ar₂₁ and Ar₂₂ represent (C4-C60)heteroaryl or aryl selected from the following structures: 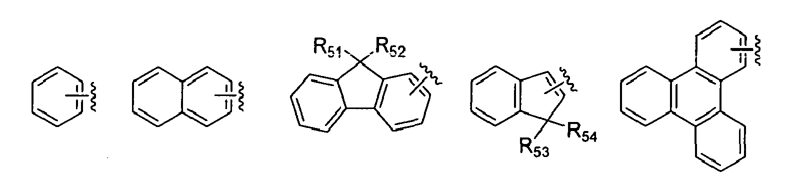 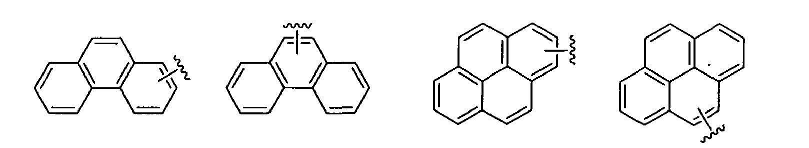 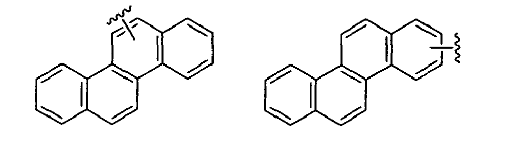 the aryl or heteroaryl of Ar₂₁ and Ar₂₂ may be substituted by one or more substituent(s) selected from (C1-C60)alkyl, (C1-C60)alkoxy, (C6-C60)aryl and (C4-C60)heteroaryl;
L₁₁ represents (C6-C60)arylene, (C4-C60)heteroarylene or a compound represented by the following structural formula: 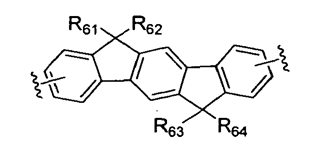 the arylene or heteroarylene of L₁₁ may be substituted by one or more substituent(s) selected from (C1-C60)alkyl, (C1-C60)alkoxy, (C6-C60)aryl, (C4-C60)heteroaryl and halogen;
R₅₁ through R₅₄ independently represent hydrogen, (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring;
R₆₁ through R₆₄ independently represent hydrogen, (C1-C60)alkyl, (C1-C60)alkoxy, (C6-C60)aryl, (C4-C60)heteroaryl or halogen, or each of them may be linked to an adjacent substituent via (C3-C60)alkylene or (C3-C60)alkenylene with or without a fused ring to form an alicyclic ring, or a monocyclic or polycyclic aromatic ring.

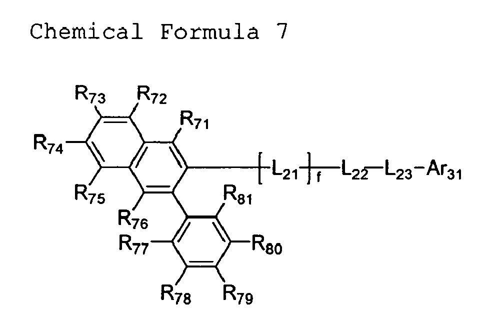

In Chemical Formula 7,
L₂₁ represents (C6-C60)arylene, (C3-C60)heteroarylene containing one or more heteroatom(s) selected from N, O and S, or a divalent group selected from the following structures: 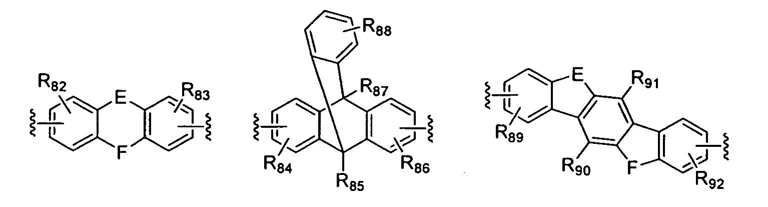 L₂₂ and L₂₃ independently represent a chemical bond, (C1-C60)alkyleneoxy, (C1-C60)alkylenethio, (C6-C60)aryleneoxy, (C6-C60)arylenethio, (C6-C60)arylene, or (C3-C60)heteroarylene containing one or more heteroatom(s) selected from N, O and S;
Ar₃₁ represents NR₉₃R₉₄, (C6-C60) aryl, (C3-C60) heteroaryl containing one or more heteroatom(s) selected from N, O and S, 5- or 6-membered heterocycloalkyl containing one or more heteroatom(s) selected from N, O and S, (C3-C60)cycloalkyl, adamantyl, (C7-C60)bicycloalkyl, or a substituent selected from the following structures: 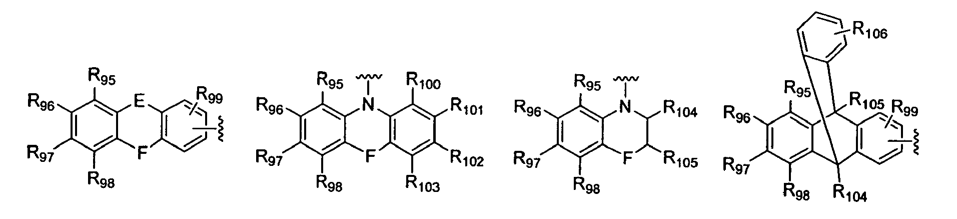 wherein, R₇₁ through R₈₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of R₇₁ through R₈₁ may be linked to an adjacent substituent via (C3-C60)alkylene or (C3-C60)alkenylene with or without a fused ring to form an alicyclic ring, or a monocyclic or polycyclic aromatic ring;
R₈₂ through R₉₂ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of R₈₂ through R₉₂ may be linked to an adjacent substituent via (C3-C60)alkylene or (C3-C60)alkenylene with or without a fused ring to form an alicyclic ring, or a monocyclic or polycyclic aromatic ring;
R₉₃ and R₉₄ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylth (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₉₃ and R₉₄ may be linked via (C3-C60)alkylene or (C3-C60)alkenylene with or without a fused ring to form an alicyclic ring, or a monocyclic or polycyclic aromatic ring;
R₉₅ through R₁₀₆ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsil tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or each of R₉₅ through R₁₀₆ may be linked to an adjacent substituent via (C3-C60)alkylene or (C3-C60)alkenylene with or without a fused ring to form an alicyclic ring, or a monocyclic or polycyclic aromatic ring;
E and F independently represent a chemical bond, - (CR₁₀₇R₁₀₈)_{g}-, -N(R₁₀₉)-, -S-, -O-, -Si(R₁₁₀)(R₁₁₁)-, -P(R₁₁₂)-, - C(=O)-, -B(R₁₁₃)-, -In(R₁₁₄)-, -Se-, -Ge(R₁₁₅)(R₁₁₆)-, - Sn(R₁₁₇)(R₁₁₈) -, -Ga(R₁₁₉)- or -(R₁₂₀)C=C(R₁₂₁)-;
R₁₀₇ through R₁₂₁ independently represent hydrogen, deuterium, halogen, (C1-C60)alkyl, (C6-C60)aryl, (C3-C60)heteroaryl containing one or more heteroatom(s) selected from N, O and S, morpholino, thiomorpholino, 5- or 6-membered heterocyloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro or hydroxyl, or R₁₀₇ and R₁₀₈, R₁₁₀ and R₁₁₁, R₁₁₅ and R₁₁₆, R₁₁₇ and R₁₁₈, or R₁₂₀ and R₁₂₁ may be linked via (C3-C60)alkylene or (C3-C60)alkenylene with or without a fused ring to form an alicyclic ring, or a monocyclic or polycyclic aromatic ring;
the arylene or heteroarylene of L₂₁ through L₂₃, the aryl or heteroaryl of Ar₃₁, or the alkyl, aryl, heteroaryl, heterocycloalkyl, cycloalkyl, trialkylsilyl, dialkylarylsilyl, triarylsilyl, alkenyl, alkynyl, alkylamino or arylamino of R₇₁ through R₁₂₁ may be further substituted independently by one or more substituent(s) selected from deuterium, halogen, (C1-C60)alkyl, halo(C1-C60)alkyl, (C6-C60)aryl, (C3-C60)heteroaryl containing one or more heteroatom(s) selected from N, O and S wherein the (C6-C60)aryl is with or without a substituent, morpholino, thiomorpholino, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C1-C60)alkyloxy, (C1-C60)alkylthio, (C6-C60)aryloxy, (C6-C60)arylthio, (C1-C60)alkoxycarbonyl, (C1-C60)alkylcarbonyl, (C6-C60)arylcarbonyl, carboxyl, nitro, hydroxyl, 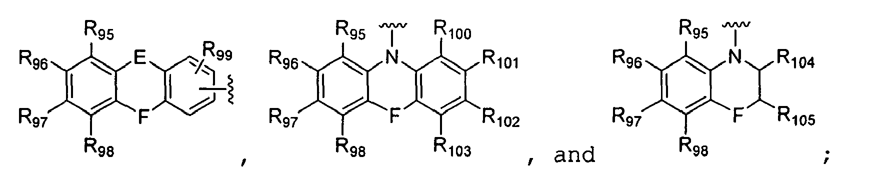 g is an integer from 1 to 4; and
f is an integer from 1 to 4.

The electroluminescent layer means the layer where electroluminescence occurs, and it may be a single layer or a multi-layer consisting of two or more layers laminated. When a mixture of host-dopant is used according to the construction of the present invention, noticeable improvement in luminous efficiency due to the inventive electroluminescent host could be confirmed, as compared to the device simply employing the electroluminescent compound according to the present invention. This can be achieved by the doping concentration of 0.5 to 10% by weight. The host according to the present invention exhibits higher hole and electron conductivity, and excellent stability of the material as compared to other conventional host materials, and provides improved device life as well as luminous efficiency.

Thus, it can be described that use of the compound represented by one of Chemical Formulas (4) to (7) as an electroluminescent host significantly supplements electronic drawback of the organic electroluminescent compounds of Chemical Formula (1) according to the present invention.

The host compounds represented by one of Chemical Formulas (4) to (7) can be exemplified by the following compounds, but are not restricted thereto.

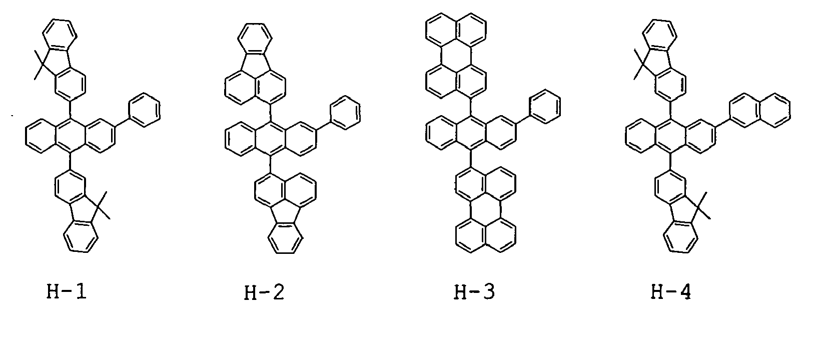

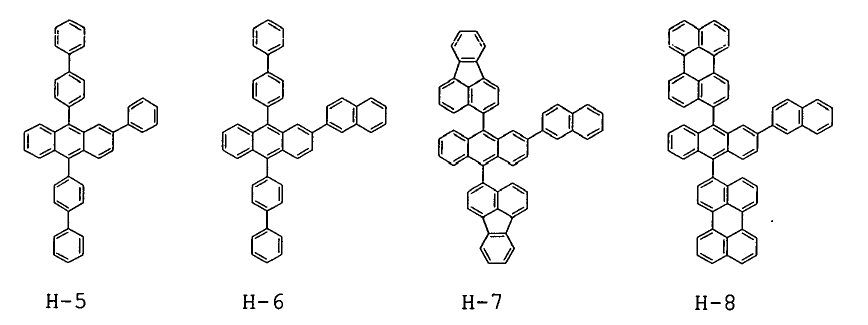

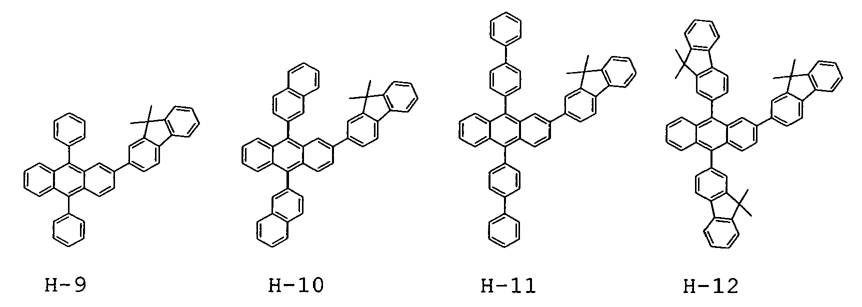

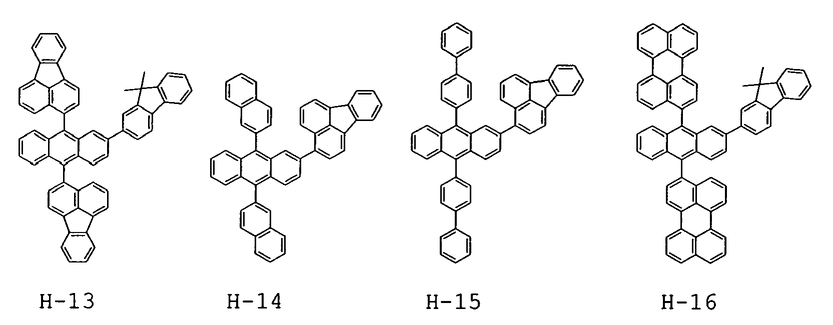

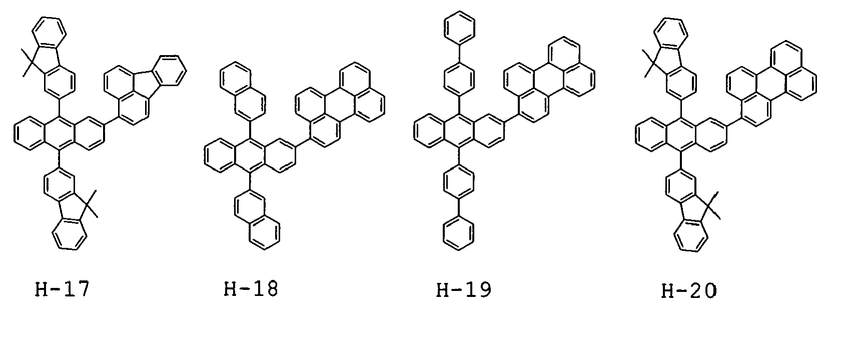

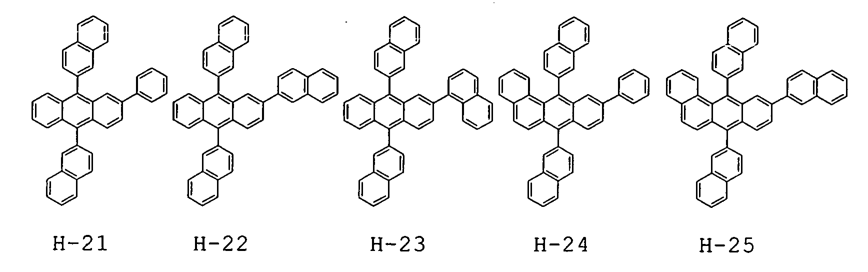

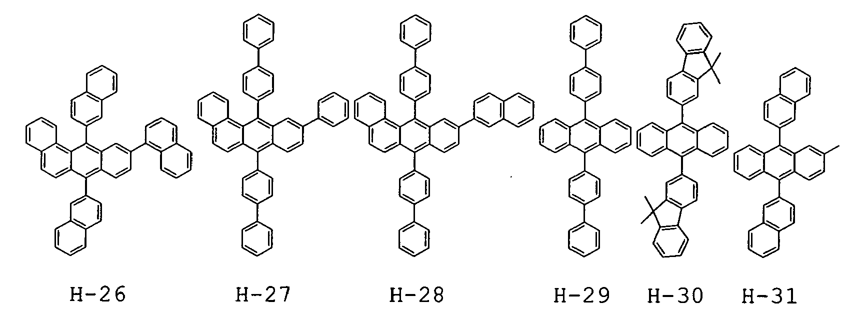

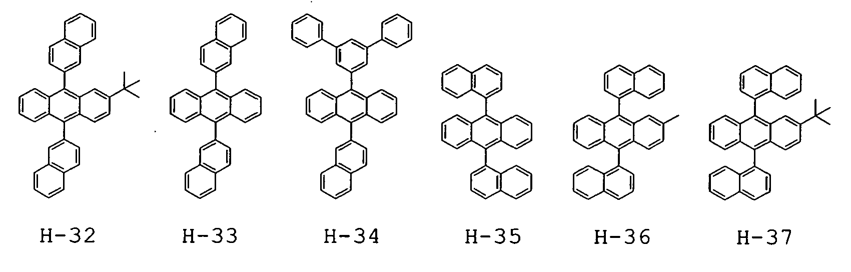

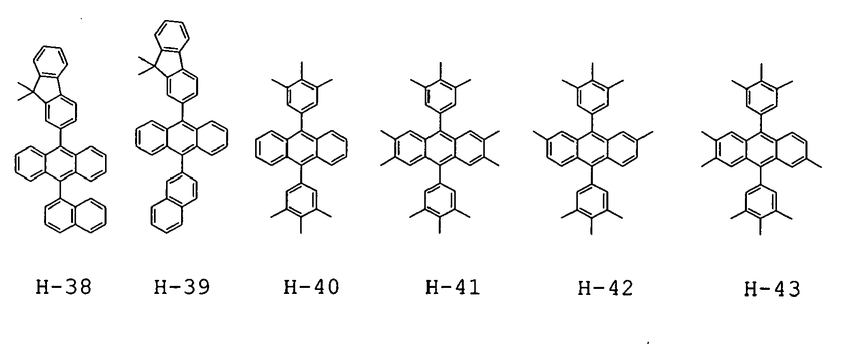

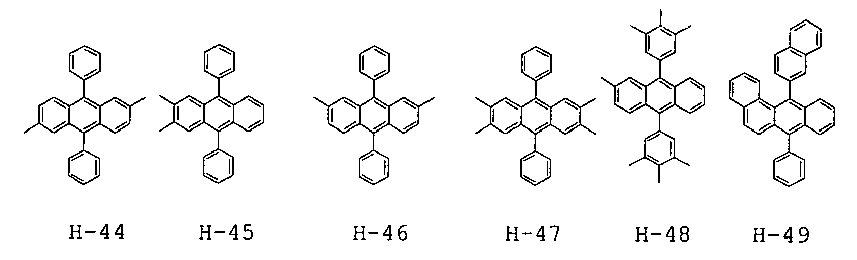

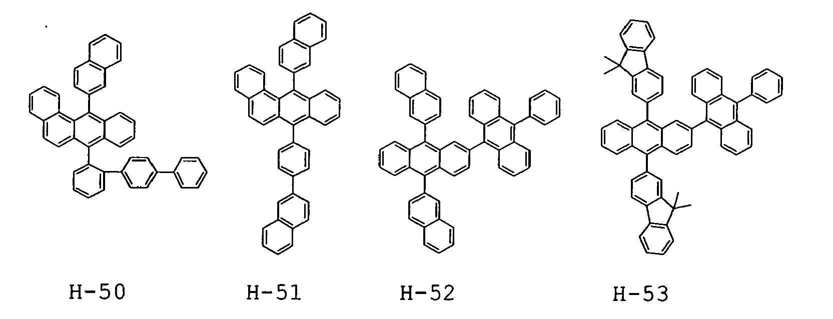

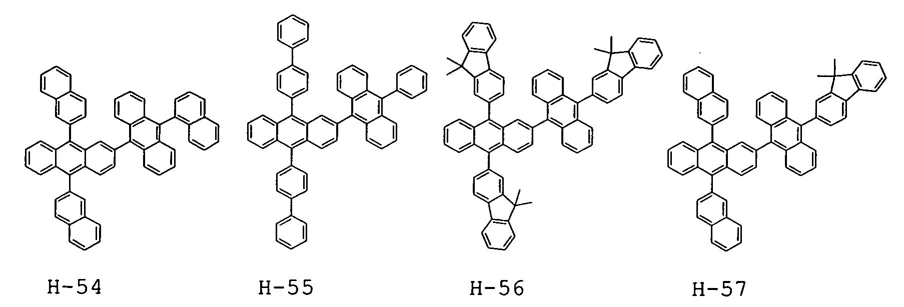

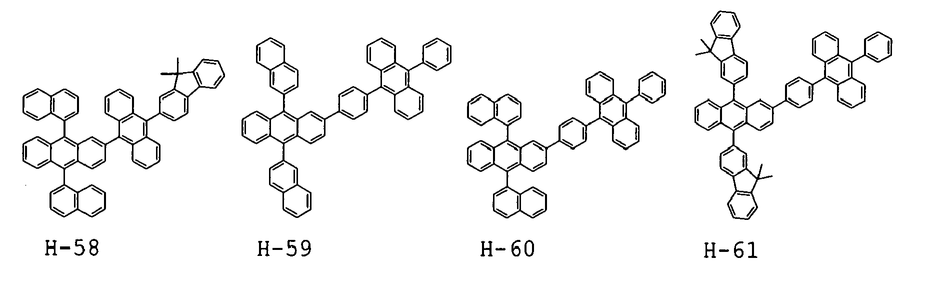

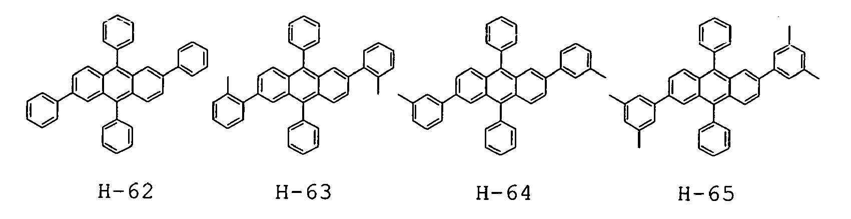

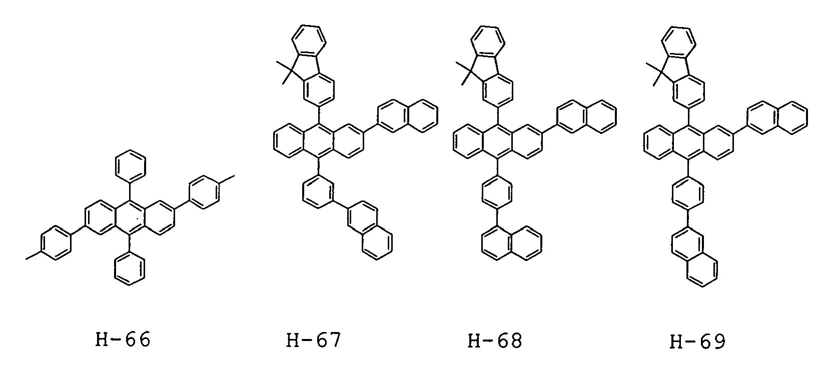

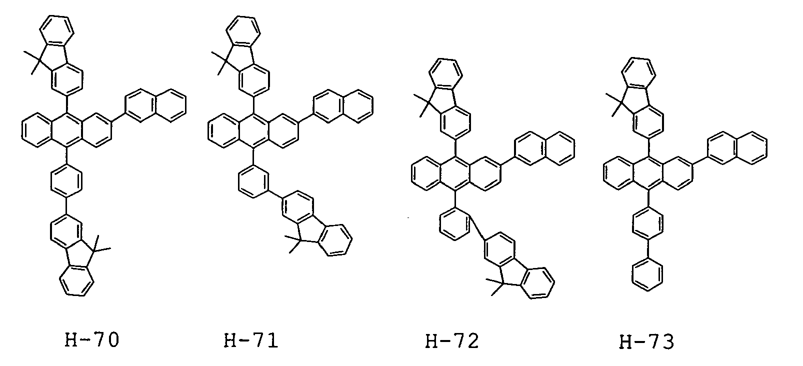

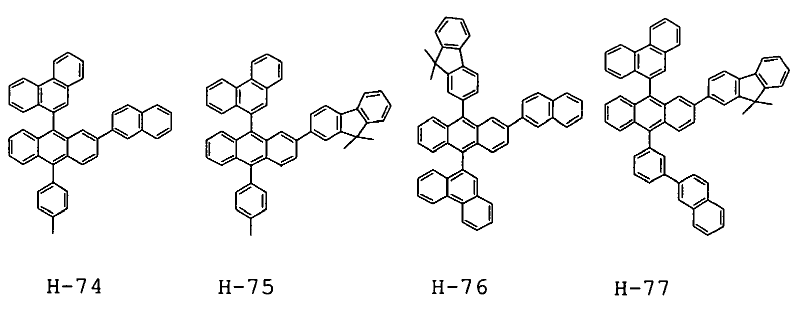

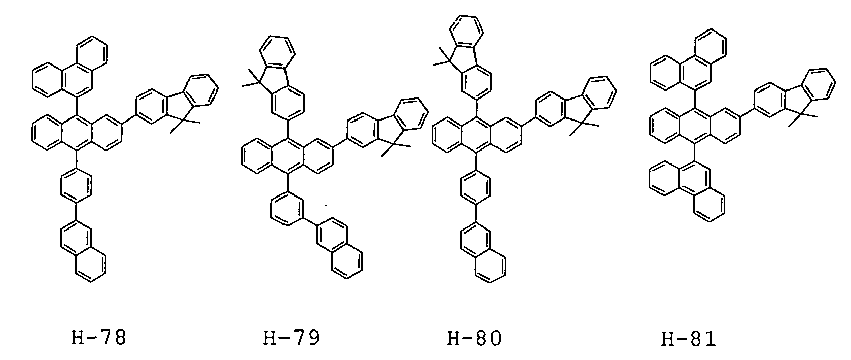

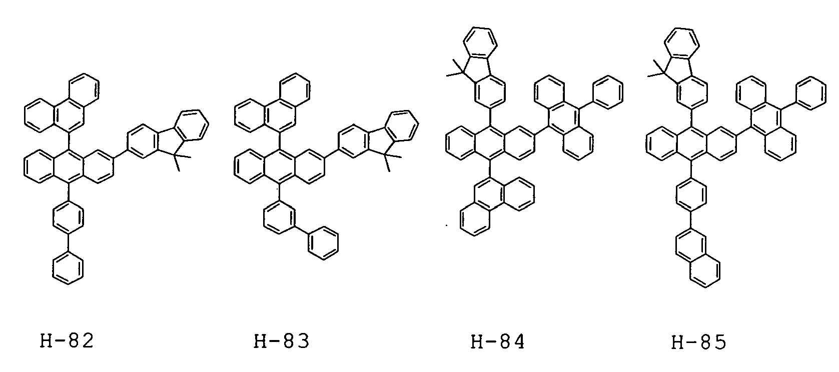

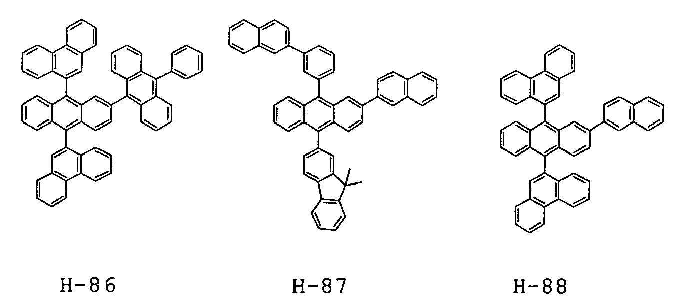

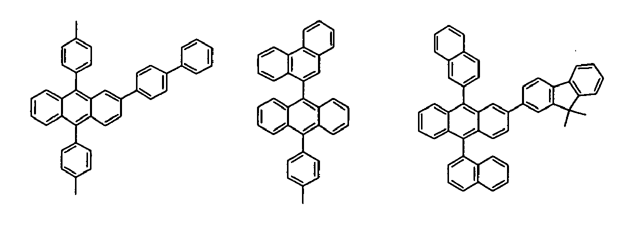

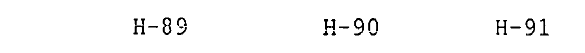

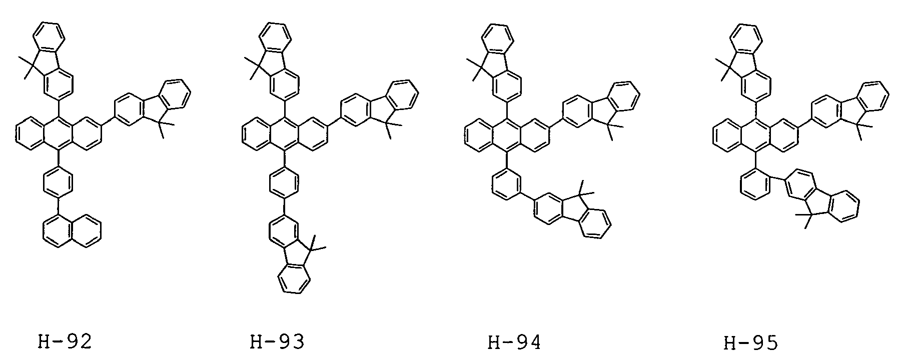

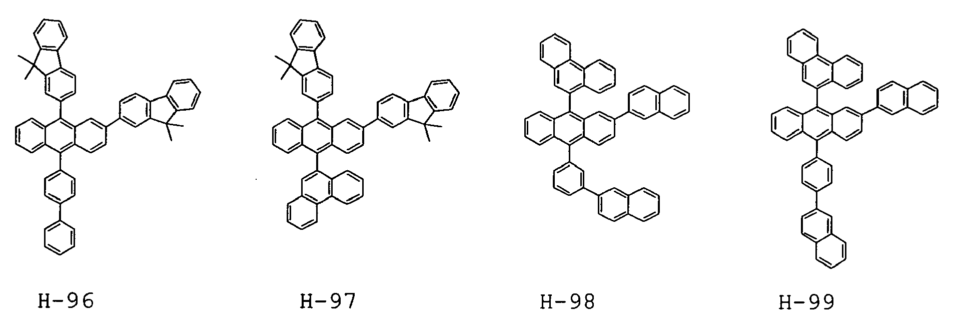

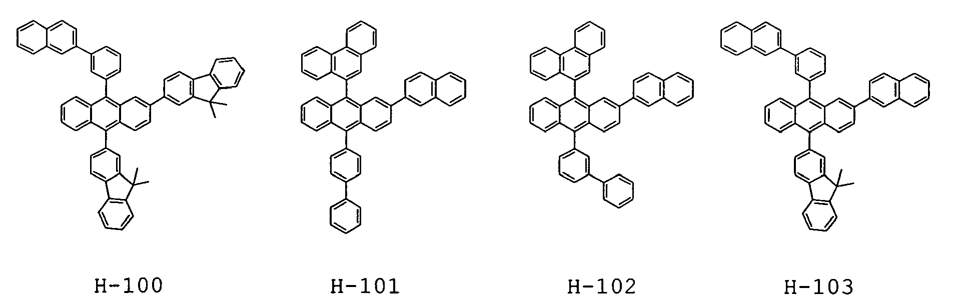

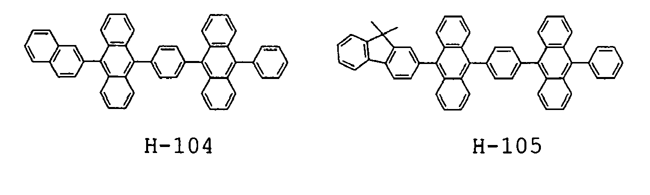

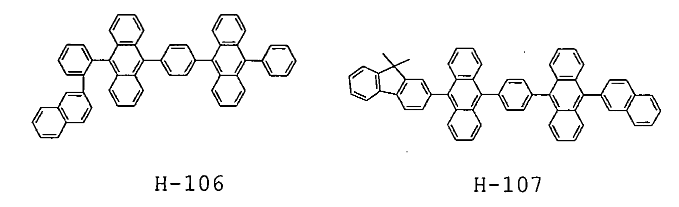

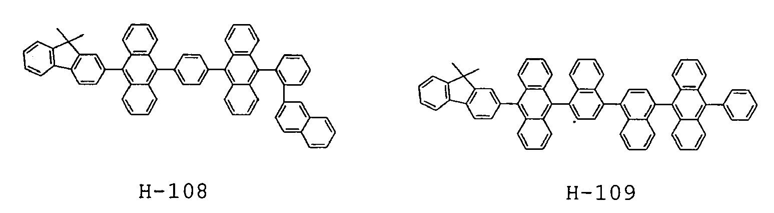

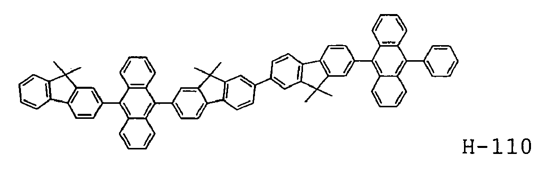

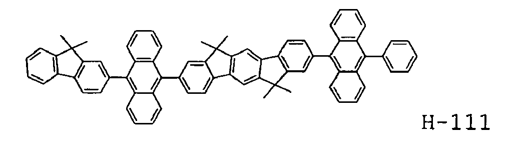

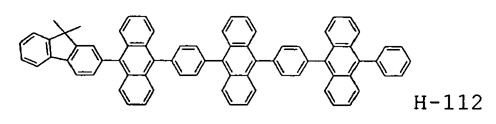

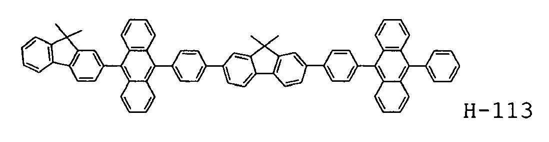

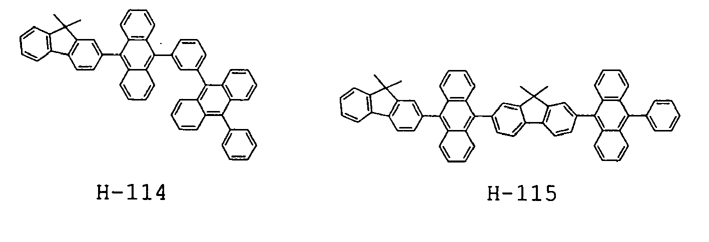

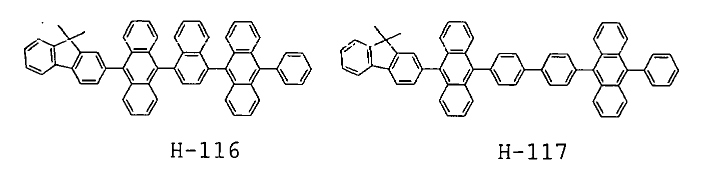

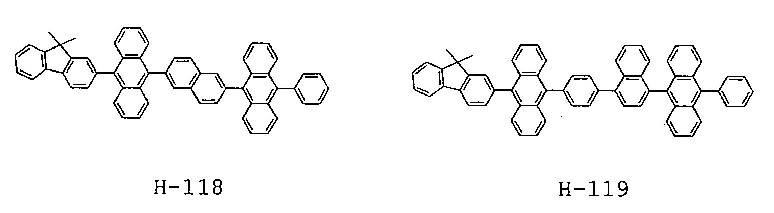

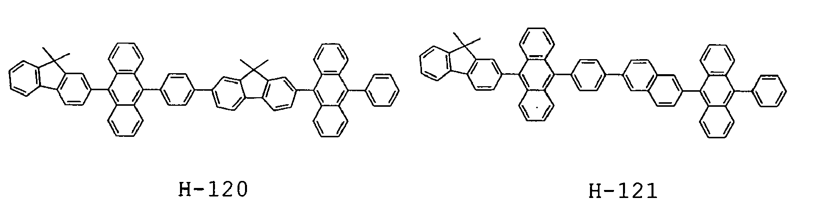

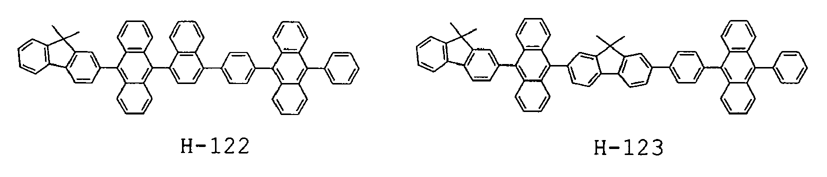

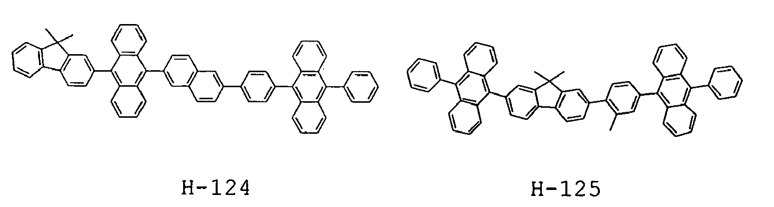

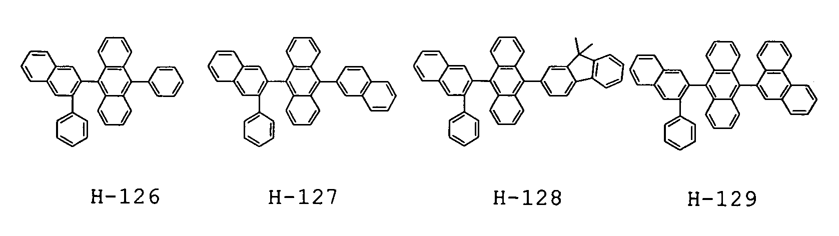

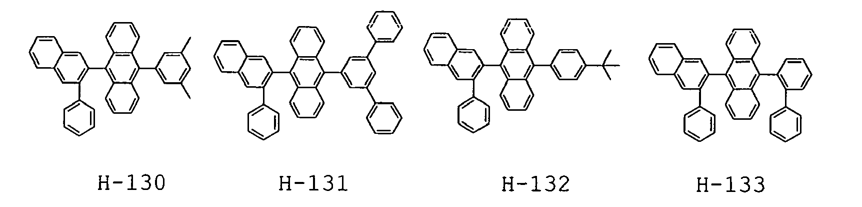

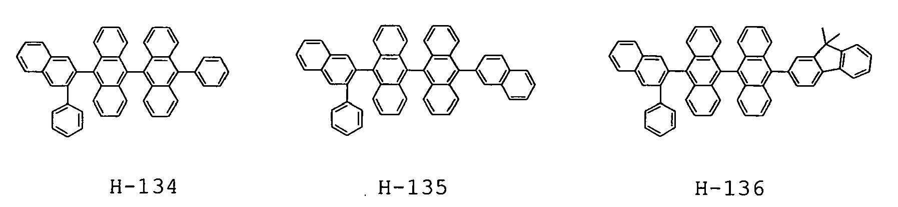

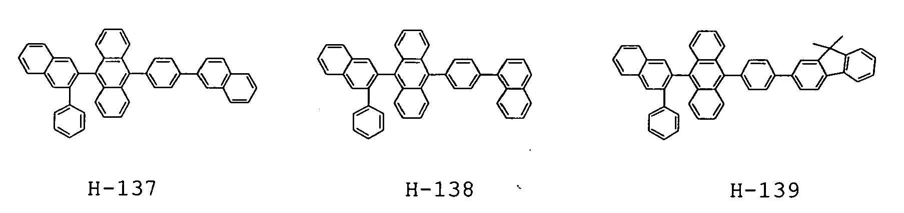

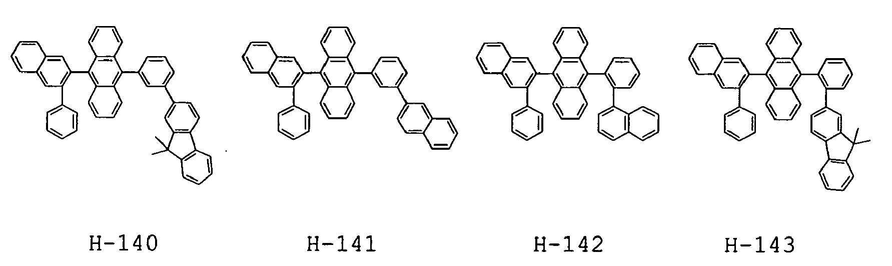

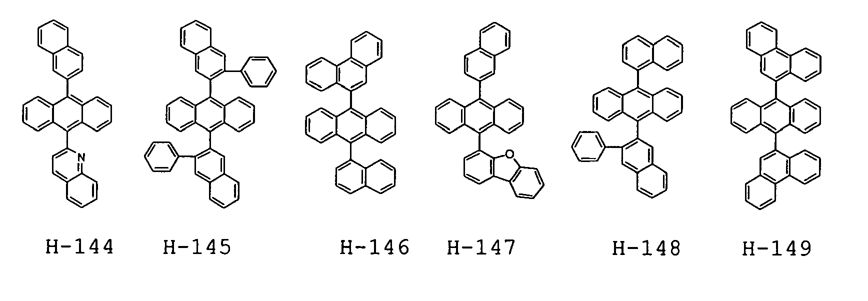

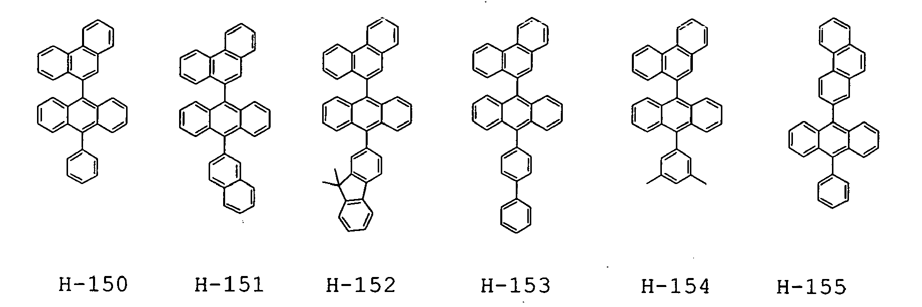

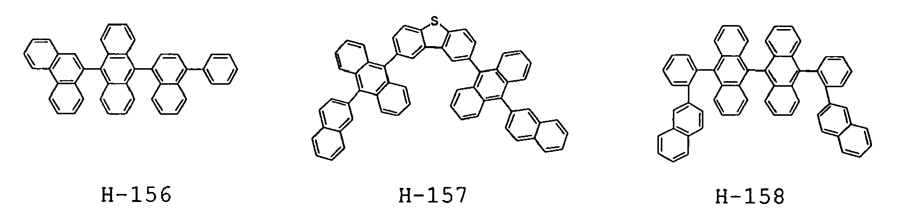

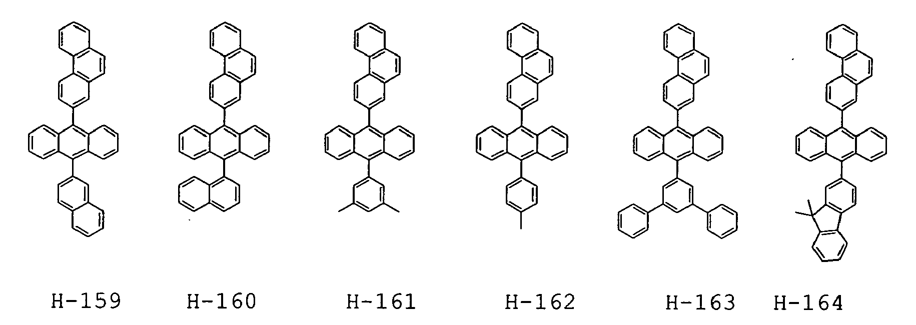

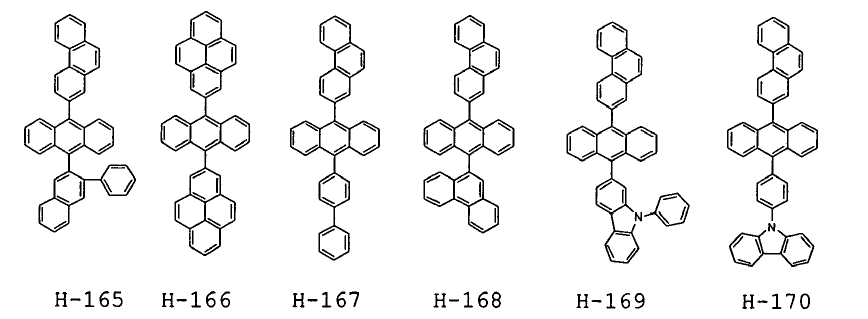

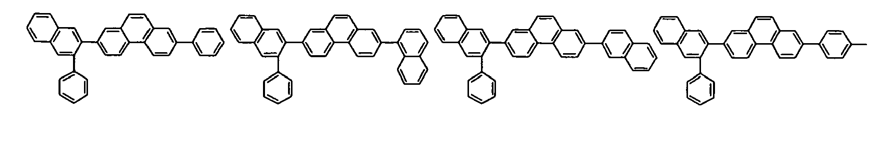

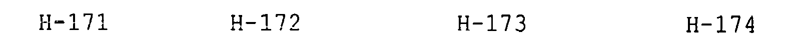

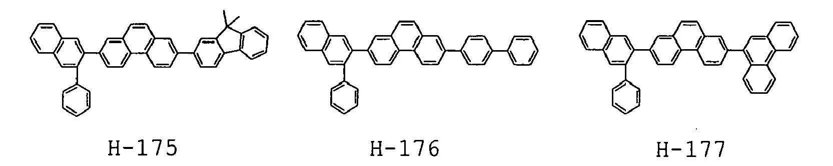

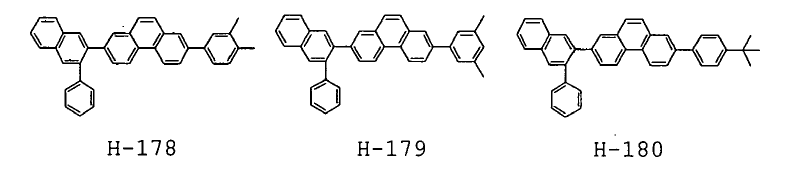

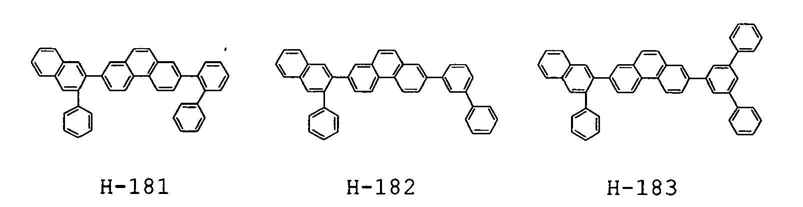

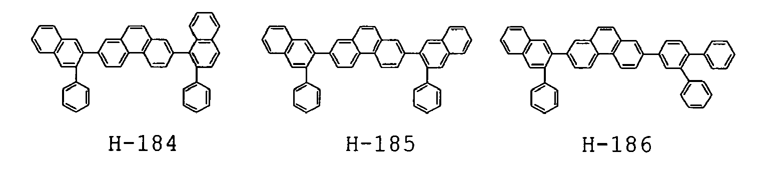

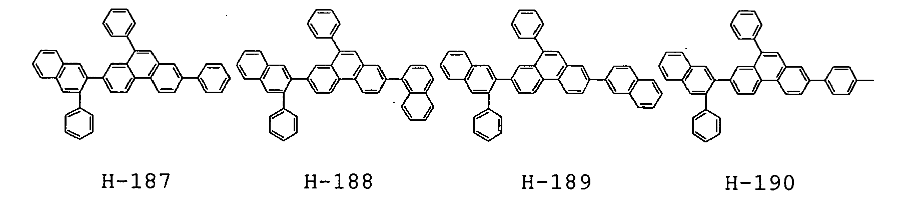

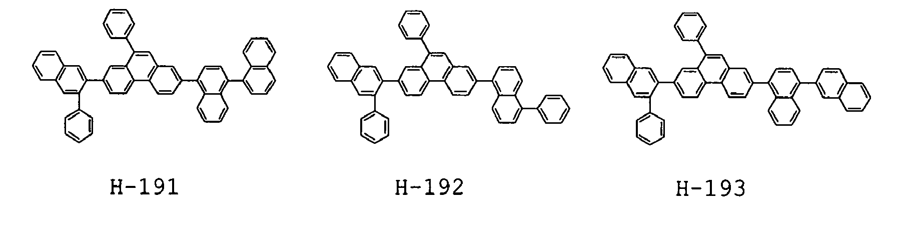

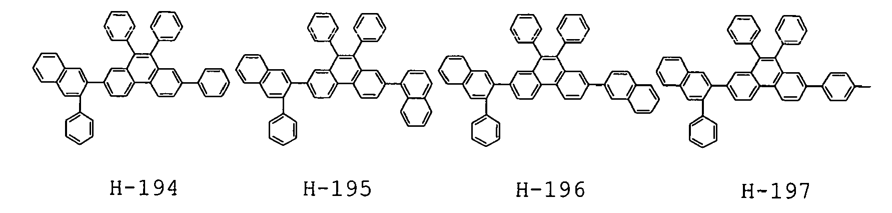

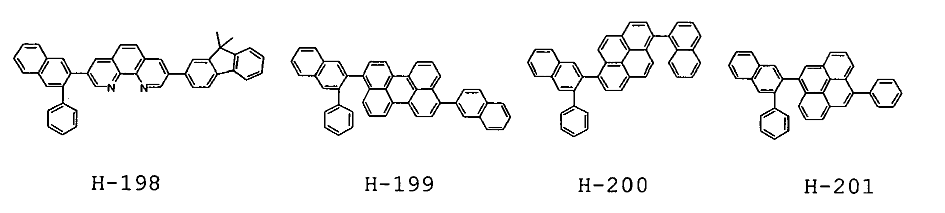

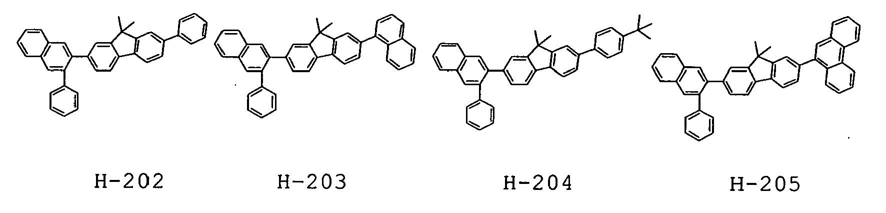

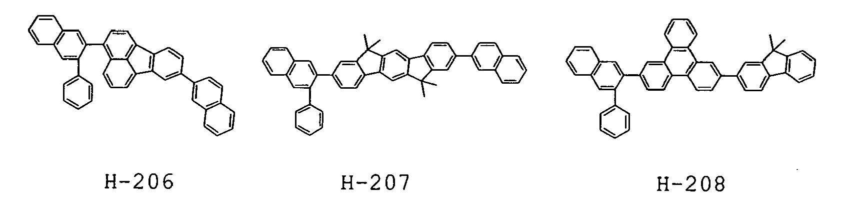

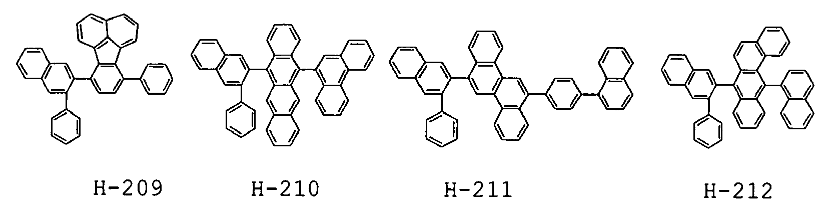

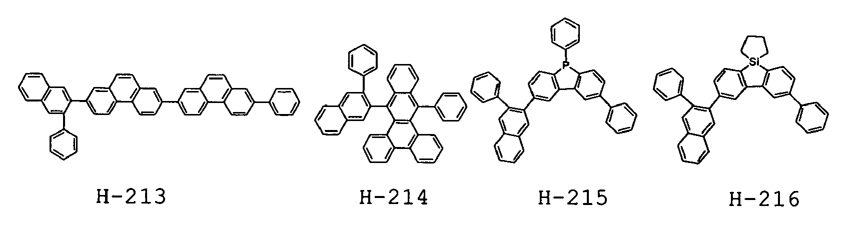

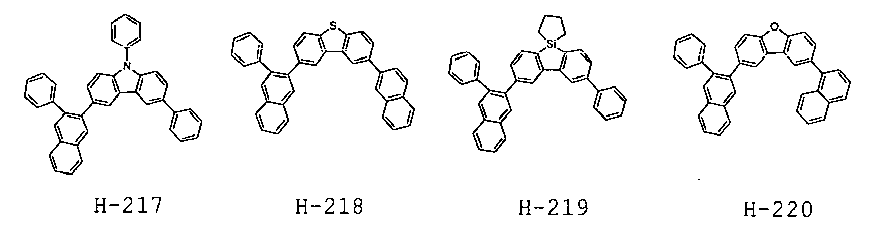

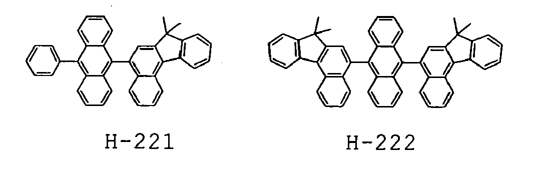

The organic electroluminescent device according to the invention may further comprise one or more compound(s) selected from arylamine compounds and styrylarylamine compounds, as well as the organic electroluminescent compound represented by Chemical Formula (1). Examples of the arylamine or styrylarylamine compounds include the compounds represented by Chemical Formula (8), but they are not restricted thereto: 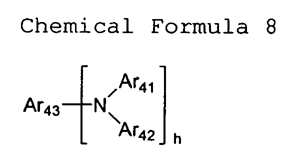 wherein, Ar₄₁ and Ar₄₂ independently represent (C1-C60)alkyl, (C6-C60)aryl, (C4-C60)heteroaryl, (C6-C60)arylamino, (C1-C60)alkylamino, a 5- or 6-membered heterocycloalkyl containing one or more heteroatom(s) selected from N, O and S, or (C3-C60)cycloalkyl, or Ar₄₁ and Ar₄₂ may be linked via (C3-C60)alkylene or (C3-C60)alkenylene with or without a fused ring to form an alicyclic ring, or a monocyclic or polycyclic aromatic ring;
when h is 1, Ar₄₃ represents (C6-C60)aryl, (C4-C60)heteroaryl, or a substituent represented by one of the following structural formulas: 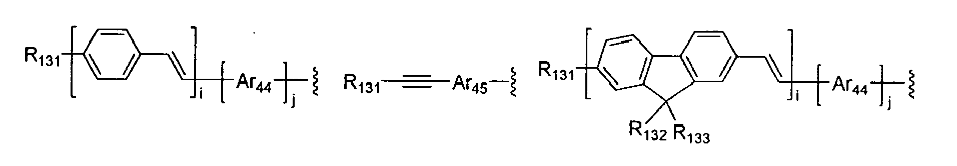 when h is 2, Ar₄₃ represents (C6-C60) arylene, (C4-C60)heteroarylene, or a substituent selected from the following structures: 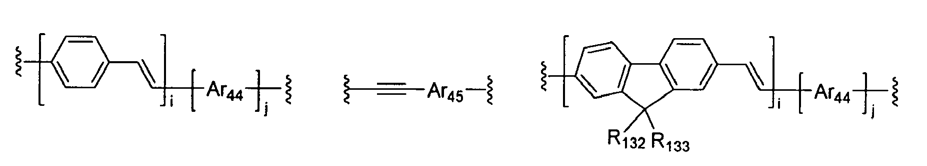 wherein Ar₄₄ and Ar₄₅ independently represent (C6-C60)arylene or (C4-C60)heteroarylene;
R₁₃₁ through R₁₃₃ independently represent hydrogen, deuterium, (C1-C60)alkyl or (C6-C60)aryl;
i is an integer from 1 to 4, j is an integer of 0 or 1; and
the alkyl, aryl, heteroaryl, arylamino, alkylamino, cycloalkyl or heterocycloalkyl of Ar₄₁ and Ar₄₂, or the aryl, heteroaryl, arylene or heteroarylene of Ar₄₃, or the arylene or heteroarylene of Ar₄₄ and Ar₄₅, or the alkyl or aryl of R₁₃₁ through R₁₃₃ may be further substituted by one or more substituent(s) selected from a group consisting of deuterium, halogen, (C1-C60)alkyl, (C6-C60)aryl, (C4-C60)heteroaryl, a 5-or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, cyano, (C1-C60)alkylamino, (C6-C60)arylamino, (C6-C60)ar(C1-C60)alkyl, (C6-C60)aryloxy, (C1-C60)alkyloxy, (C6-C60)arylthio, (C1-C60)alkylthio, (C1-C60)alkoxycarbonyl, (C1-C60)alkylcarbonyl, (C6-C60)arylcarbonyl, carboxyl, nitro and hydroxyl.

The arylamine compounds and styrylarylamine compounds may be more specifically exemplified by the following compounds, but are not restricted thereto.

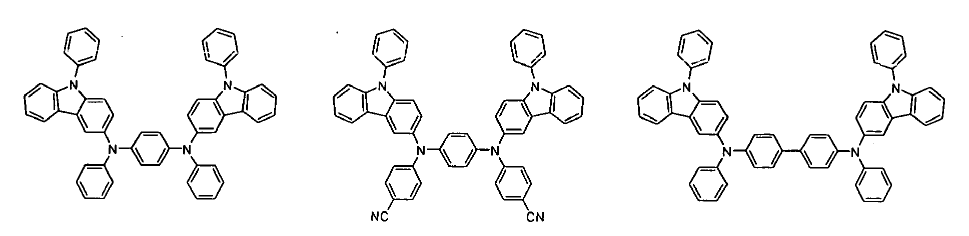

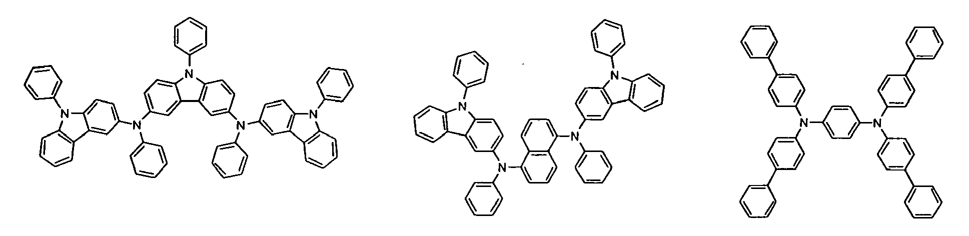

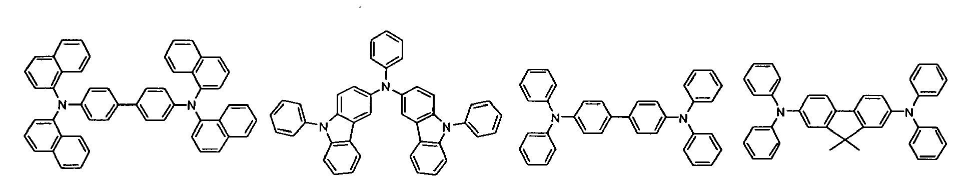

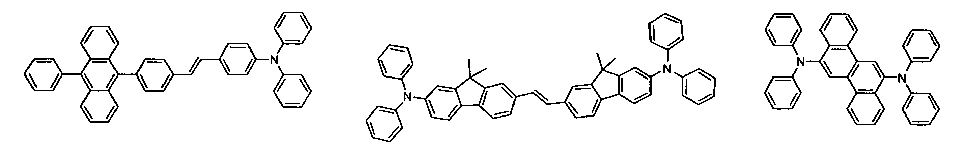

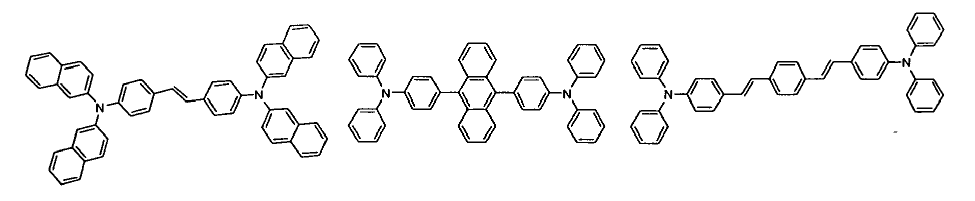

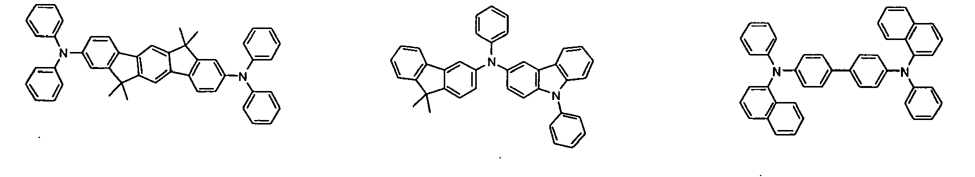

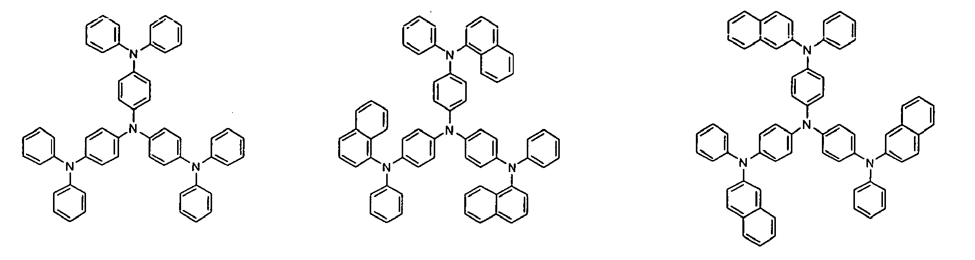

In an organic electroluminescent device according to the present invention, the organic layer may further comprise one or more metal(s) selected from a group consisting of organic metals of Group 1, Group 2, 4^{th} period and 5^{th} period transition metals, lanthanide metals and d-transition elements from the Periodic Table of Elements, as well as the organic electroluminescent compound represented by Chemical Formula (1). The organic layer may comprise an electroluminescent layer and a charge generating layer at the same time.

The present invention can realize an electroluminescent device having a pixel structure of independent light-emitting mode, which comprises an organic electroluminescent device containing the compound of Chemical Formula (1) as a sub-pixel, and one or more sub-pixel(s) comprising one or more metal compounds selected from a group consisting of Ir, Pt, Pd, Rh, Re, Os, Tl, Pb, Bi, In, Sn, Sb, Te, Au and Ag, patterned in parallel at the same time.

Further, the organic electroluminescent device is an organic display wherein the organic layer comprises, in addition to the organic electroluminescent compound represented by Chemical Formula (1), one or more compound(s) selected from compounds having the electroluminescent peak of wavelength of 500 to 560 nm, or those having the electroluminescent peak of wavelength of not less than 560 nm, at the same time. The compounds having electroluminescent peak of wavelength of 500 to 560 nm, or those having the electroluminescent peak of wavelength of not less than 560 nm may be exemplified by the compounds represented by one of Chemical Formulas (9) to (15), but they are not restricted thereto.

Chemical Formula 9 M¹L¹⁰¹L¹⁰²L¹⁰³

In Chemical Formula (9), M¹ is selected from Group 7, 8, 9, 10, 11, 13, 14, 15 and 16 metals in the Periodic Table of Elements, and ligands L¹⁰¹, L¹⁰² and L¹⁰³ are independently selected from the following structures: 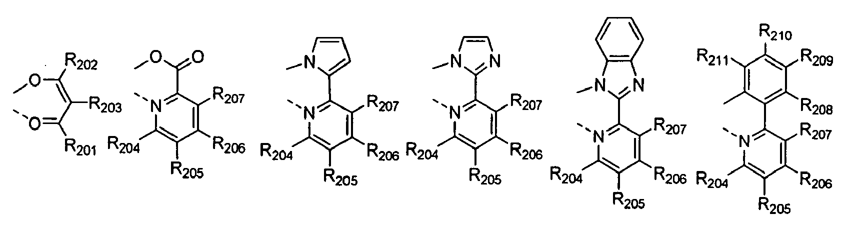 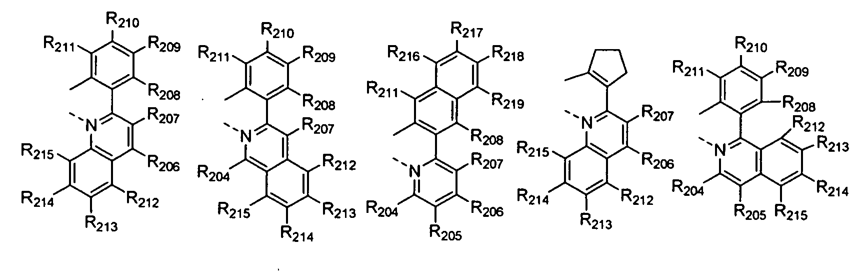 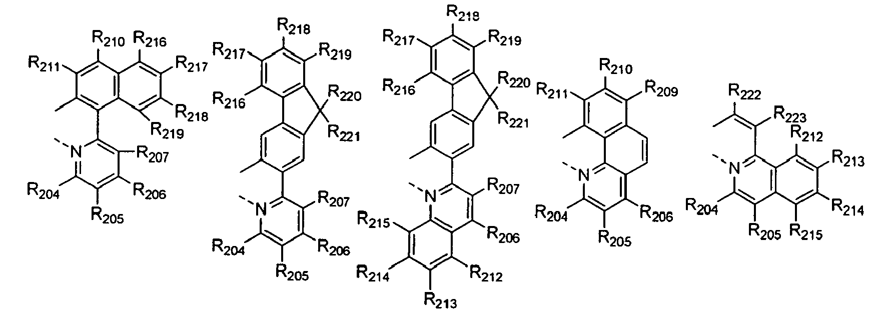 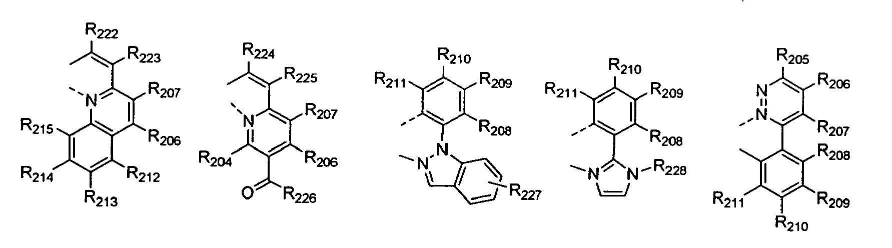 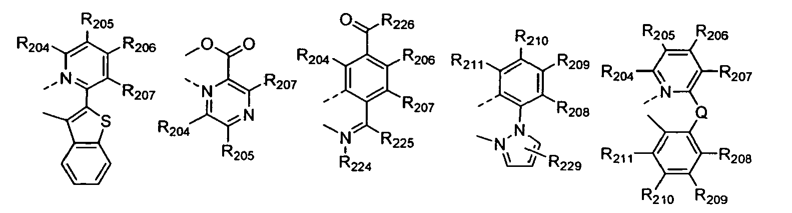 wherein, R₂₀₁ through R₂₀₃ independently represent hydrogen, deuterium, (C1-C60)alkyl with or without halogen substituent(s), (C6-C60)aryl with or without (C1-C60)alkyl substituent(s), or halogen;
R₂₀₄ through R₂₁₉ independently represent hydrogen, deuterium, (C1-C60)alkyl, (C1-C30)alkoxy, (C3-C60)cycloalkyl, (C2-C30)alkenyl, (C6-C60)aryl, mono or di(C1-C30)alkylamino, mono or di(C6-30)arylamino, SF₅, tri(C1-C30)alkylsilyl, di(C1-C30)alkyl(C6-C30)arylsilyl, tri(C6-C30)arylsilyl, cyano or halogen, and the alkyl, cycloalkyl, alkenyl or aryl of R₂₀₄ through R₂₁₉ may be further substituted by one or more substituent(s) selected from deuterium, (C1-C60)alkyl, (C6-C60)aryl and halogen;
R₂₂₀ through R₂₂₃ independently represent hydrogen, deuterium, (C1-C60)alkyl with or without halogen substituent(s), (C6-C60)aryl with or without (C1-C60)alkyl substituent(s);
R₂₂₄ and R₂₂₅ independently represent hydrogen, deuterium, (C1-C60)alkyl, (C6-C60)aryl or halogen, or R₂₂₄ and R₂₂₅ may be linked via (C3-C12)alkylene or (C3-C12)alkenylene with or without a fused ring to form an alicyclic ring, or a monocyclic or polycyclic aromatic ring; and the alkyl or aryl of R₂₂₄ and R₂₂₅, or the alicyclic ring, or the monocyclic or polycyclic aromatic ring formed therefrom via (C3-C12)alkylene or (C3-C12)alkenylene with or without a fused ring may be further substituted by one or more substituent(s) selected from deuterium, (C1-C60)alkyl with or without halogen substituent(s), (C1-C30)alkoxy, halogen, tri(C1-C30)alkylsilyl, tri(C6-C30)arylsilyl and (C6-C60)aryl;
R₂₂₆ represents (C1-C60)alkyl, (C6-C60) aryl, or (C5-C60)heteroaryl containing one or more heteroatom(s) selected from N, O and S, or halogen;
R₂₂₇ through R₂₂₉ independently represent hydrogen, deuterium, (C1-C60)alkyl, (C6-C60)aryl or halogen, and the alkyl or aryl of R₂₂₆ through R₂₂₉ may be further substituted by halogen or (C1-C60)alkyl;
Q represents 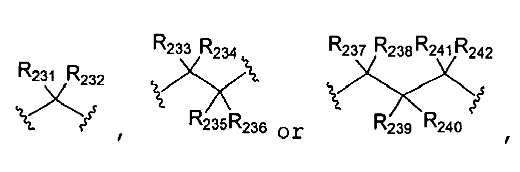 and R₂₃₁ through R₂₄₂ independently represent hydrogen, deuterium, (C1-C60)alkyl with or without halogen substituent(s), (C1-C30)alkoxy, halogen, (C6-C60)aryl, cyano or (C5-C60)cycloalkyl, or each of R₂₃₁ through R₂₄₂ may be linked to an adjacent substituent via alkylene or alkenylene to form a (C5-C7) spiro-ring or (C5-C9) fused ring, or each of them may be linked to R₂₀₇ or R₂₀₈ via alkylene or alkenylene to form a (C5-C7) fused ring.

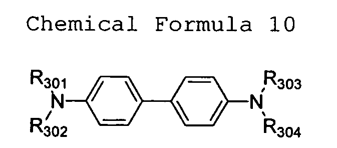

In Chemical Formula (10), R₃₀₁ through R₃₀₄ independently represent (C1-C60)alkyl or (C6-C60)aryl, or each of them may be linked to an adjacent substituent via (C3-C60)alkylene or (C3-C60)alkenylene with or without a fused ring to form an alicyclic ring, or a monocyclic or polycyclic aromatic ring; and the alkyl or aryl of R₃₀₁ through R₃₀₄, or the alicyclic ring, or the monocyclic or polycyclic aromatic ring formed therefrom by linkage via (C3-C60) alkylene or (C3-C60)alkenylene with or without a fused ring may be further substituted by one or more substituent(s) selected from (C1-C60)alkyl with or without halogen substituent(s), (C1-C60)alkoxy, halogen, tri(C1-C60)alkylsilyl, tri(C6-C60)arylsilyl and (C6-C60)aryl.

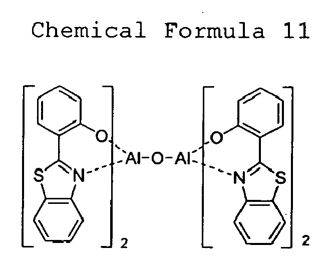

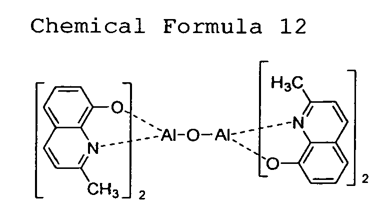

Chemical Formula 13 L²¹⁰L²⁰²M²(T)ₖ

In Chemical Formula (13), the ligands, L²⁰¹ and L²⁰² are independently selected from the following structures: 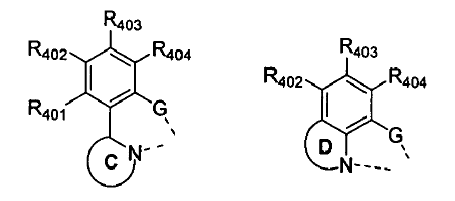 M² is a bivalent or trivalent metal;
k is 0 when M² is a bivalent metal, while k is 1 when M² is a trivalent metal;
T represents (C6-C60)aryloxy or tri(C6-C60)arylsilyl, and the aryloxy and triarylsilyl of T may be further substituted by (C1-C60)alkyl or (C6-C60)aryl;
G represents O, S or Se;
ring C represents oxazole, thiazole, imidazole, oxadiazole, thiadiazole, benzoxazole, benzothiazole, benzimidazole, pyridine or quinoline;
ring D represents pyridine or quinoline, and ring D may be further substituted by (C1-C60)alkyl, or phenyl or naphthyl with or without (C1-C60)alkyl substituent(s);
R₄₀₁ through R₄₀₄ independently represent hydrogen, deuterium, (C1-C60)alkyl, halogen, tri(C1-C60)alkylsilyl, tri(C6-C60)arylsilyl or (C6-C60)aryl, or each of them may be linked to an adjacent substituent via (C3-C60)alkylene or (C3-C60)alkenylene to form a fused ring, and the pyridine or quinoline may form a chemical bond with R₄₀₁ to form a fused ring;
ring C or the aryl group of R₄₀₁ through R₄₀₄ may be further substituted by (C1-C60)alkyl, halogen, (C1-C60)alkyl with halogen substituent(s), phenyl, naphthyl, tri(C1-C60)alkylsilyl, tri(C6-C60)arylsilyl or amino group.

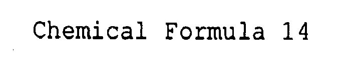

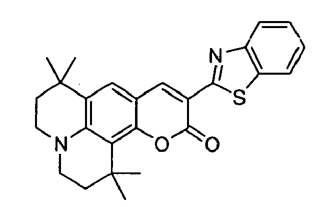

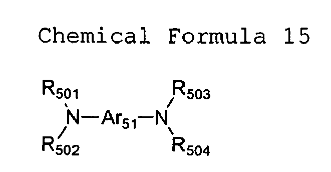

In Chemical Formula (15), Ar₅₁ represents (C6-C60)arylene with or without one or more substituent(s) selected from a group consisting of halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl; and the alkyl, cycloalkyl, heterocycloalkyl, aryl, heteroaryl, arylsilyl, alkylsilyl, alkylamino and arylamino substituent on the arylene may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl;
R₅₀, through R₅₀₄ independently represent (C1-C60)alkyl, (C6-C60)aryl, (C4-C60)heteroaryl, (C6-C60)arylamino, (C1-C60)alkylamino, 5- or 6-membered heterocycloalkyl containing one or more heteroatom(s) selected from N, O and S, or (C3-C60)cycloalkyl, or R₅₀₁ through R₅₀₄ may be linked to an adjacent substituent via (C3-C60)alkylene or (C3-C60)alkenylene with or without a fused ring to form an alicyclic ring or a monocyclic or polycyclic ring; and
the alkyl, aryl, heteroaryl, arylamino, cycloalkyl and heterocycloalkyl of R₅₀₁ through R₅₀₄ may be further substituted by one or more substituent(s) selected from halogen, (C1-C60)alkyl, (C6-C60)aryl, (C4-C60)heteroaryl, 5- or 6-membered heterocycloalkyl containing one or more heteroatom(s) selected from N, O and S, (C3-C60)cycloalkyl, tri(C1-C60)alkylsilyl, di(C1-C60)alkyl(C6-C60)arylsilyl, tri(C6-C60)arylsilyl, adamantyl, (C7-C60)bicycloalkyl, (C2-C60)alkenyl, (C2-C60)alkynyl, (C1-C60)alkoxy, cyano, (C1-C60)alkylamino, (C6-C60)arylamino, (C6-C60)ar(C1-C60)alkyl, (C6-C60)aryloxy, (C6-C60)arylthio, (C1-C60)alkoxycarbonyl, carboxyl, nitro and hydroxyl.

The compounds having electroluminescent peak of wavelength of 500 to 560 nm, or those having electroluminescent peak of wavelength of not less than 560 nm, may be exemplified by the following compounds, but they are not restricted thereto.

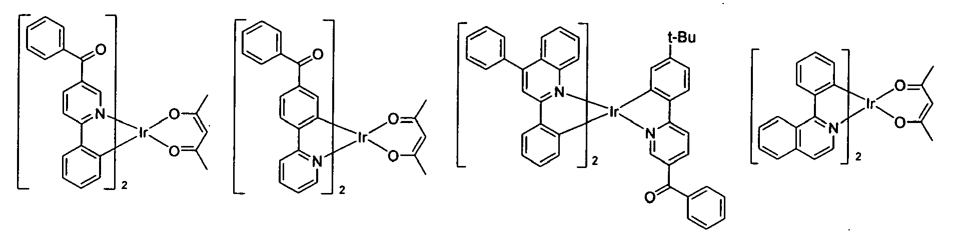

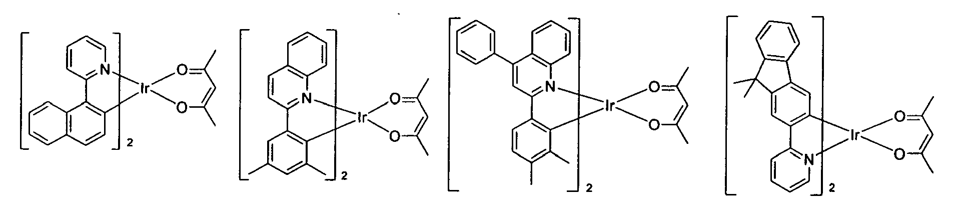

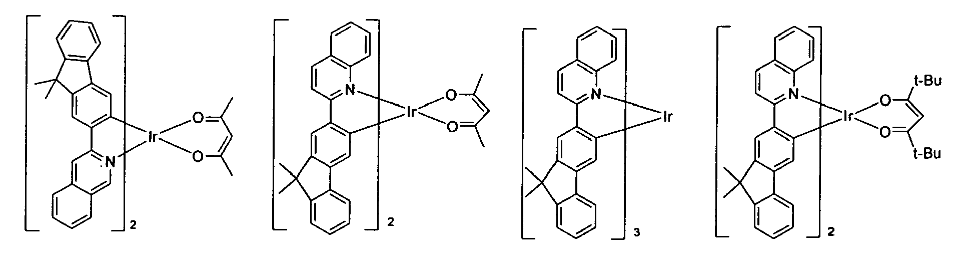

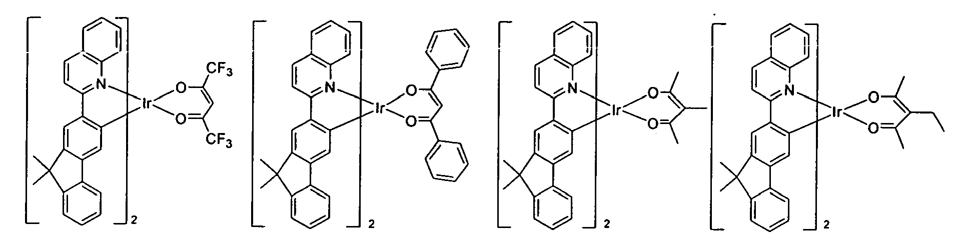

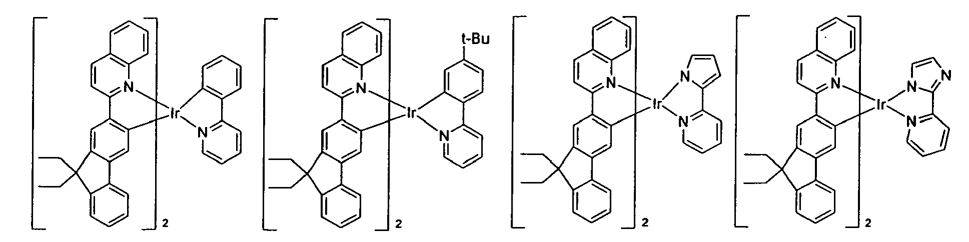

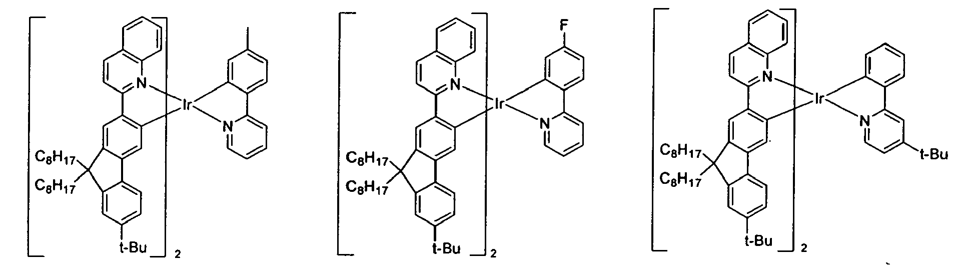

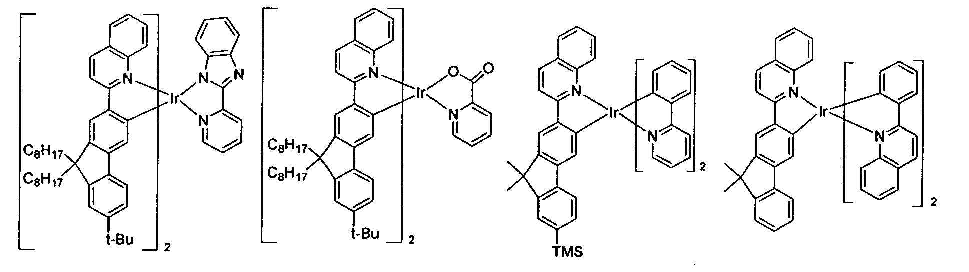

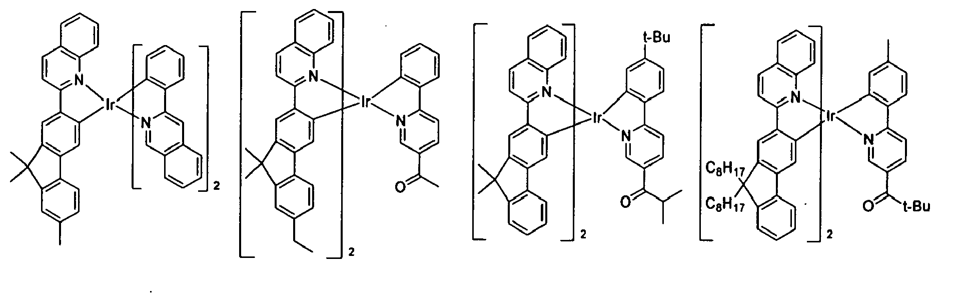

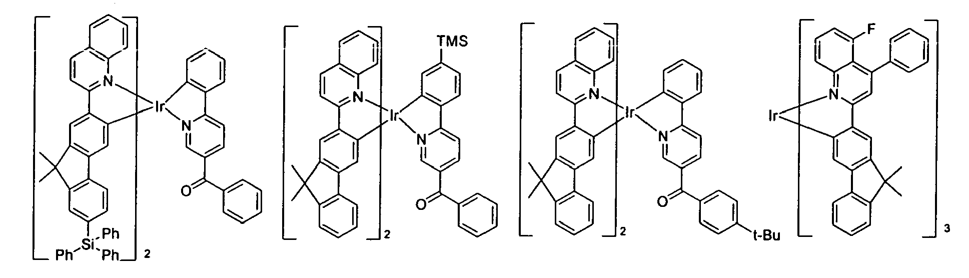

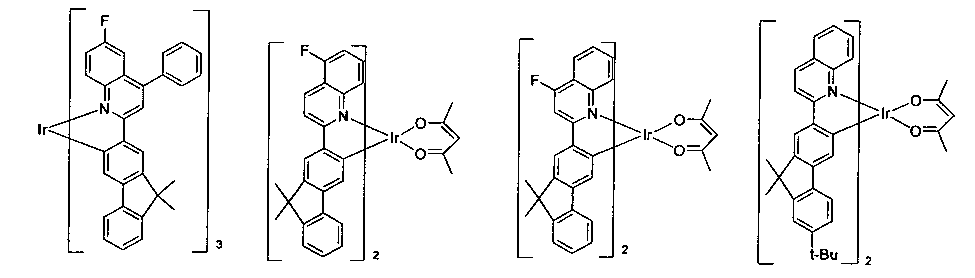

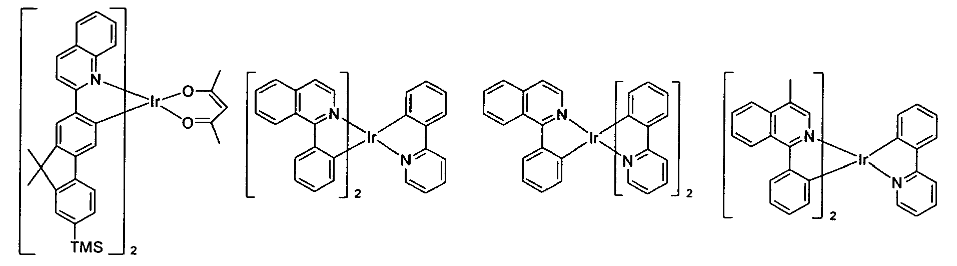

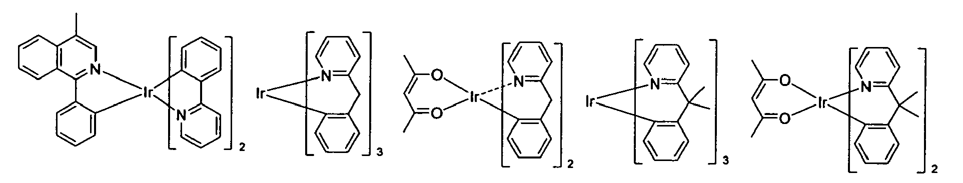

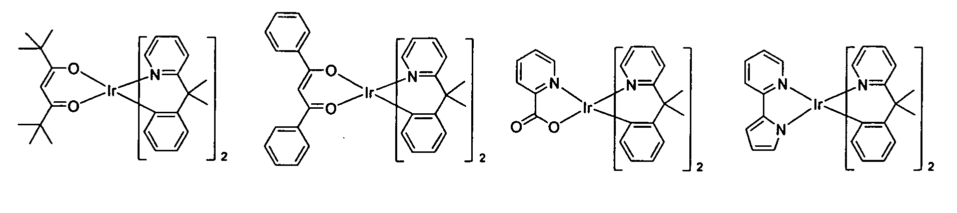

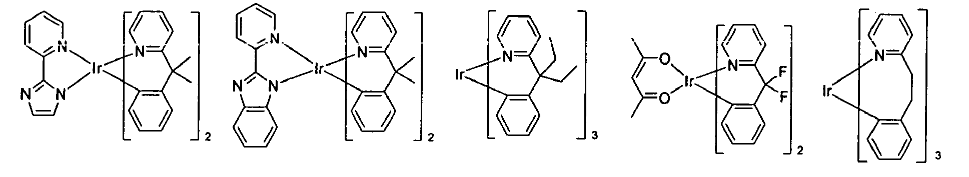

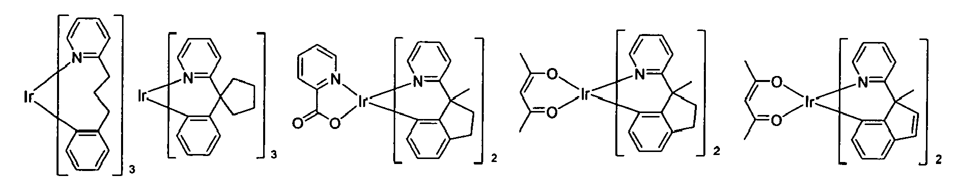

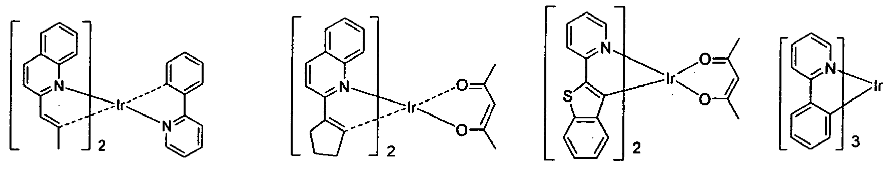

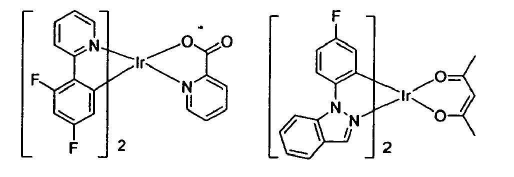

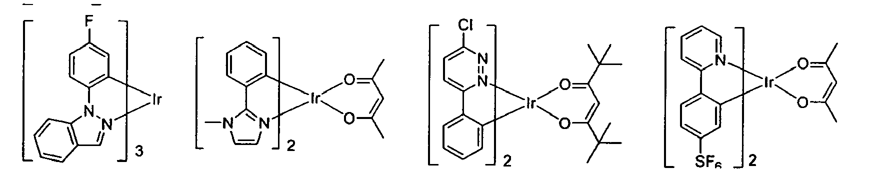

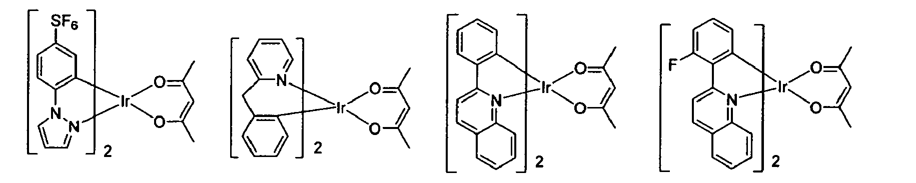

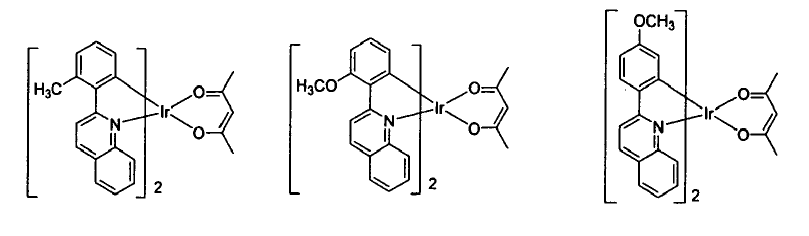

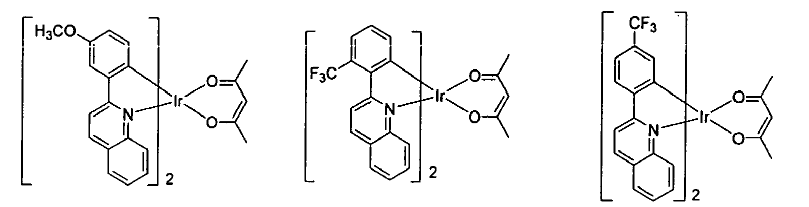

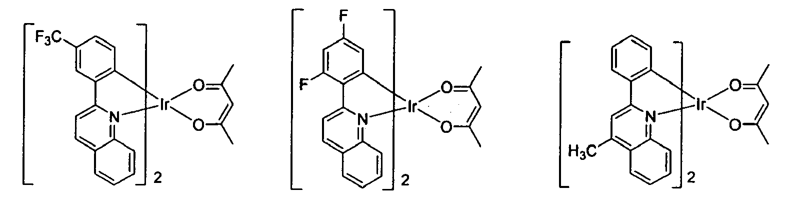

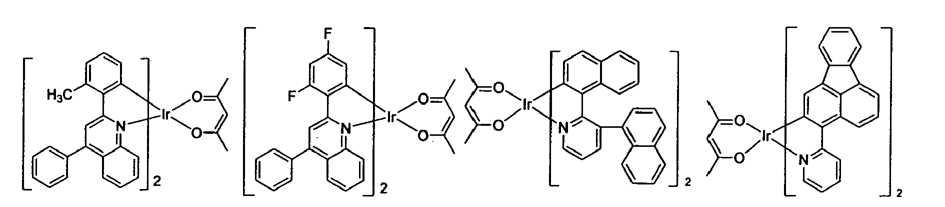

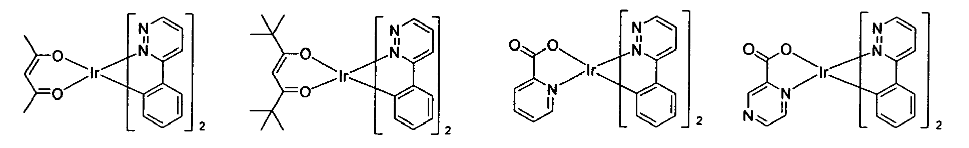

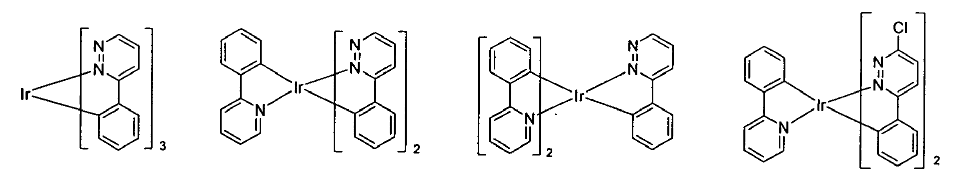

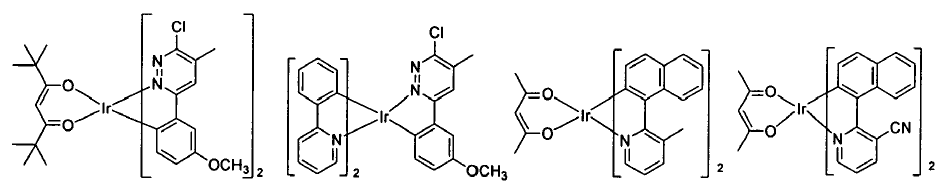

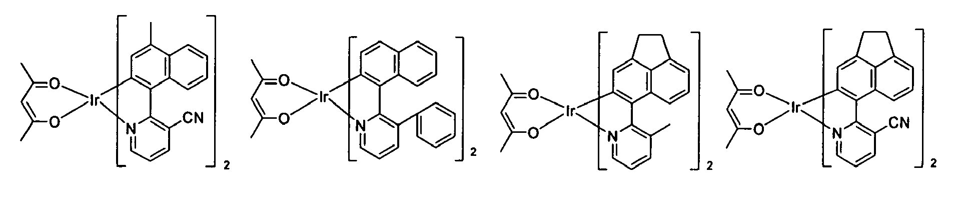

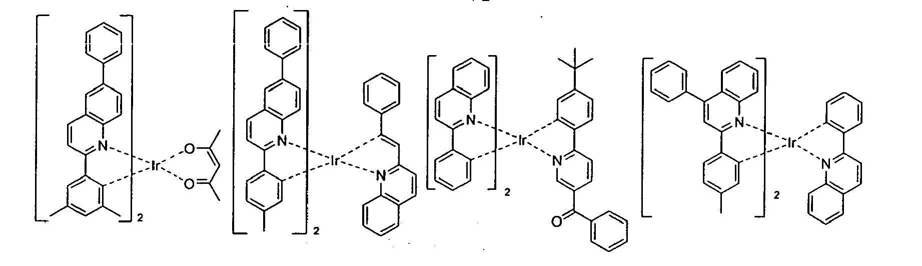

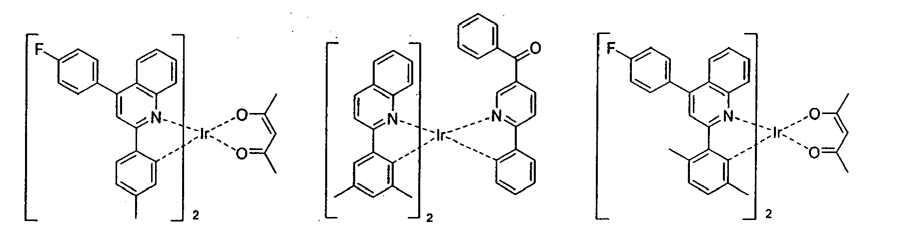

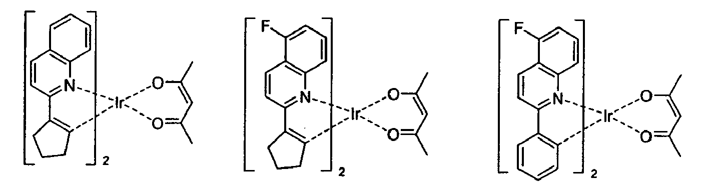

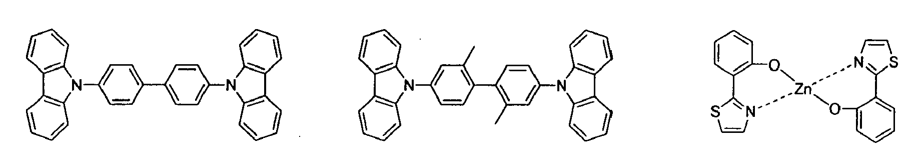

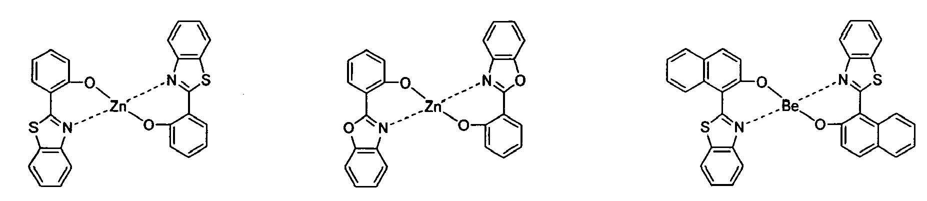

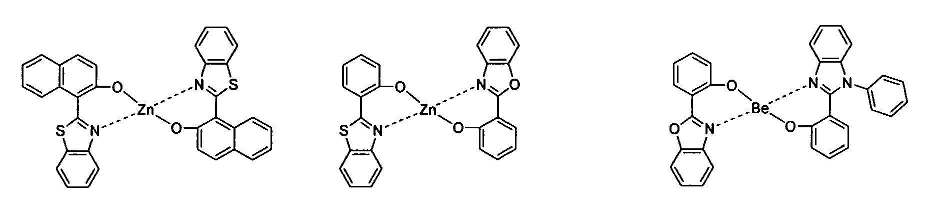

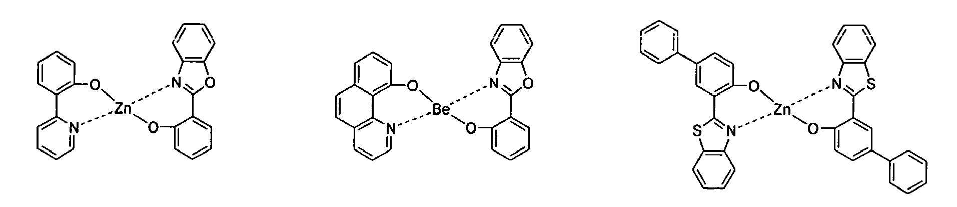

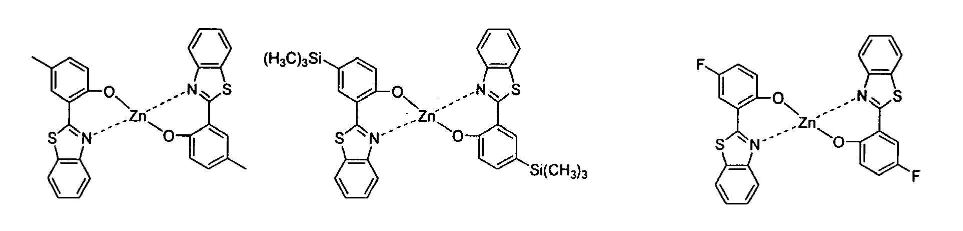

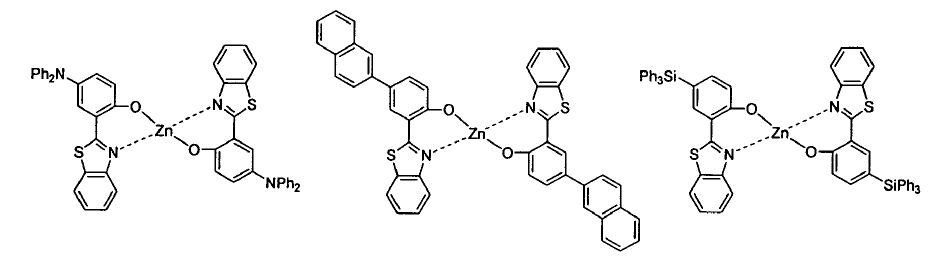

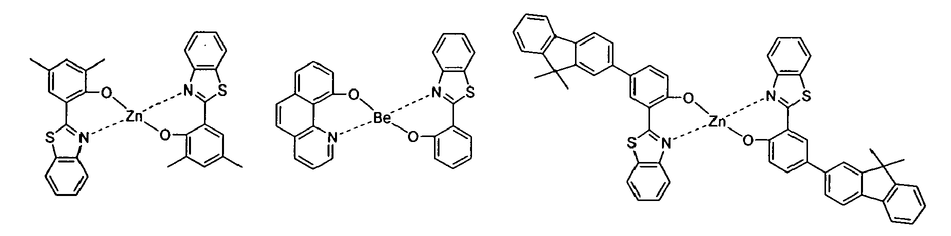

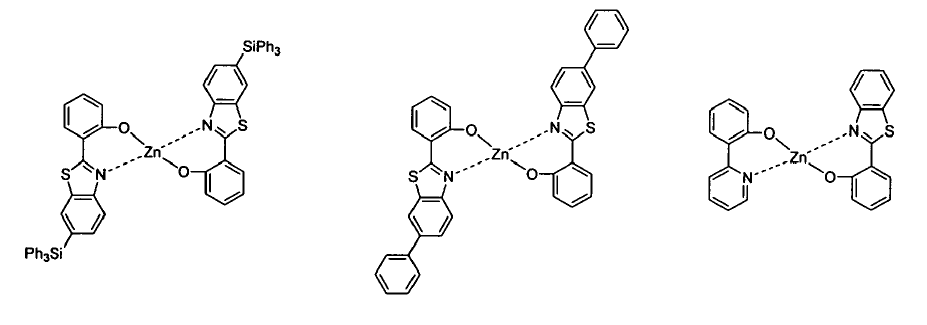

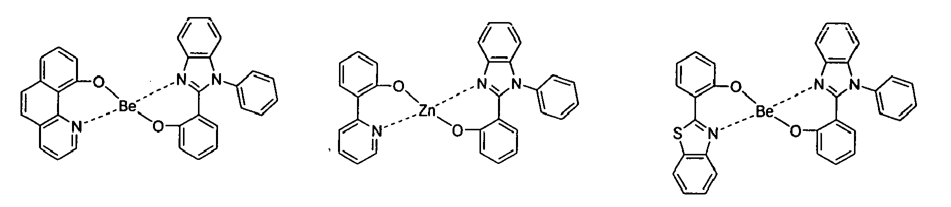

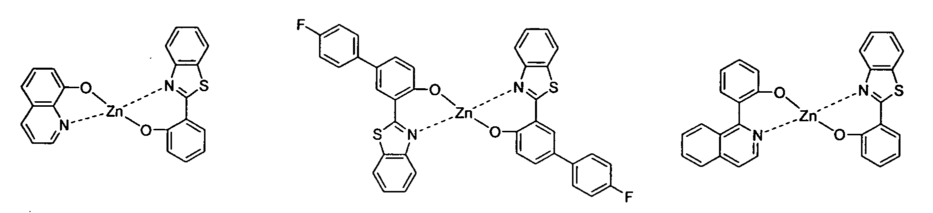

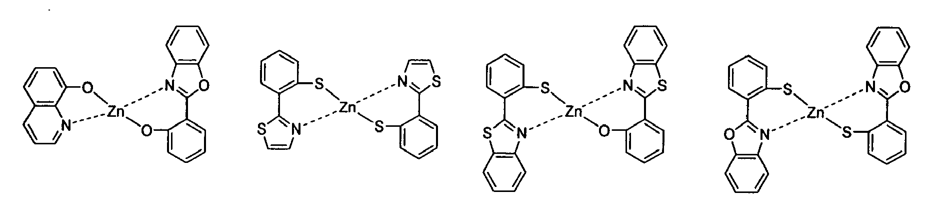

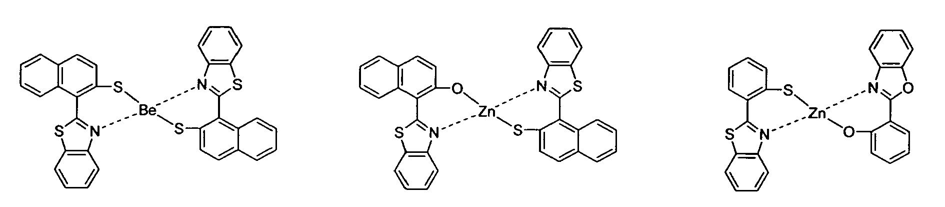

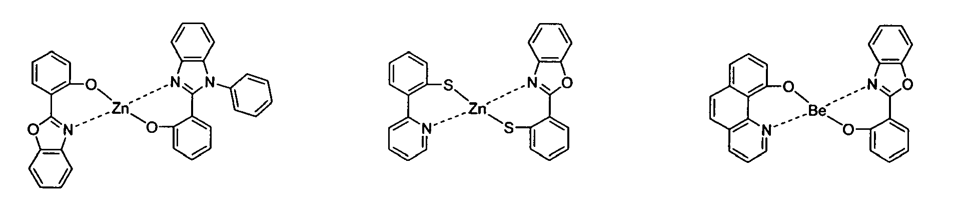

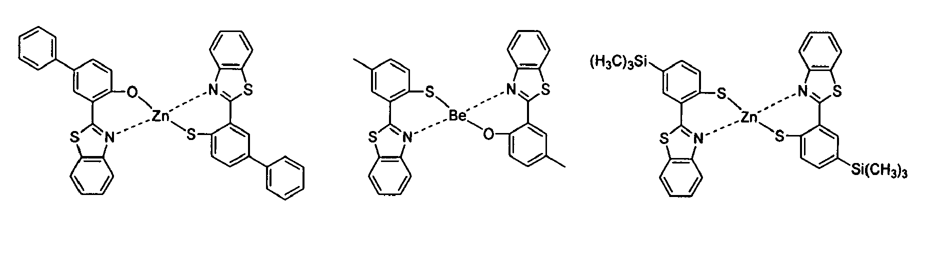

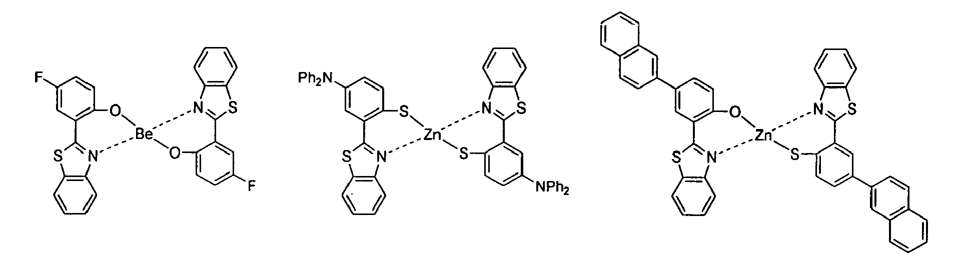

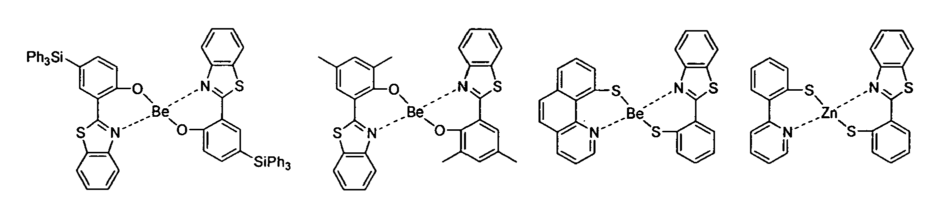

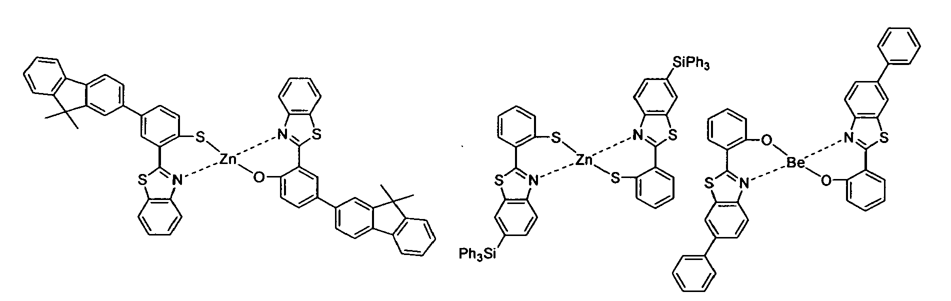

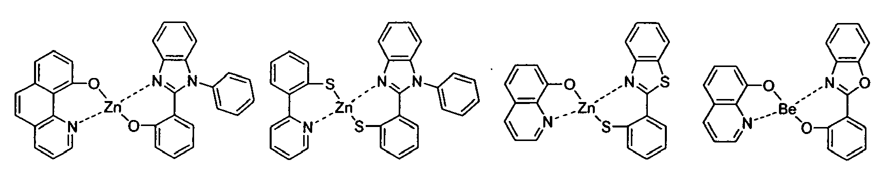

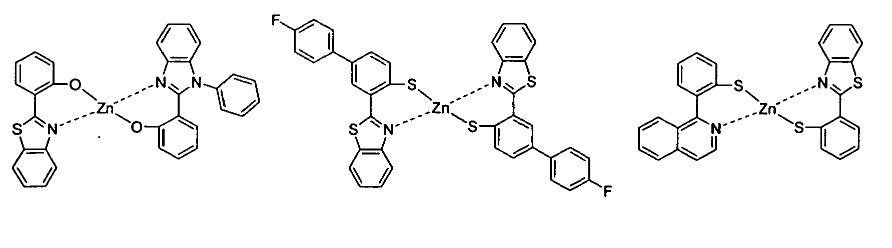

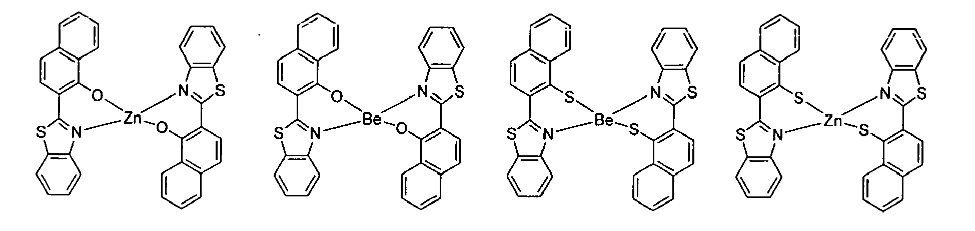

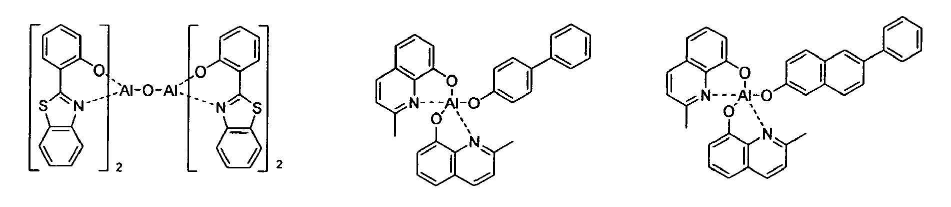

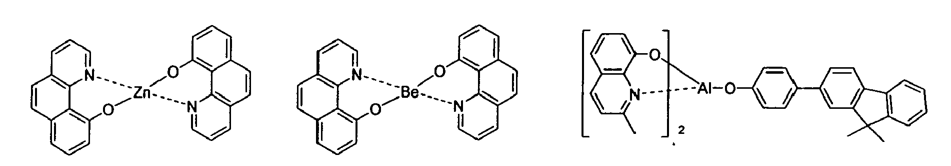

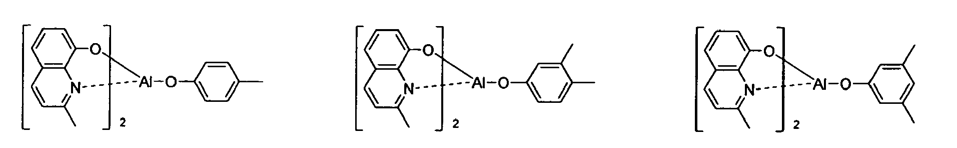

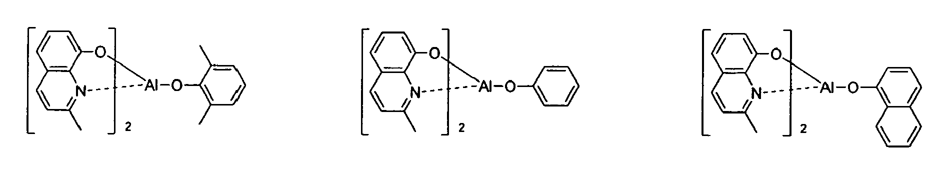

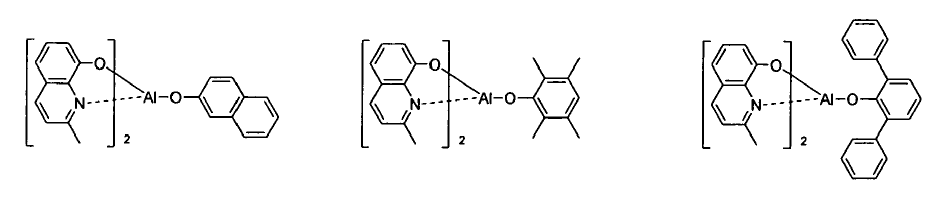

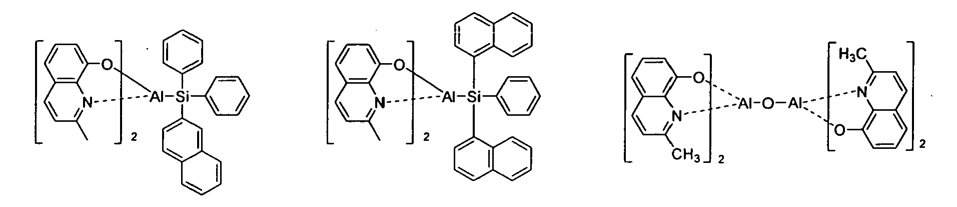

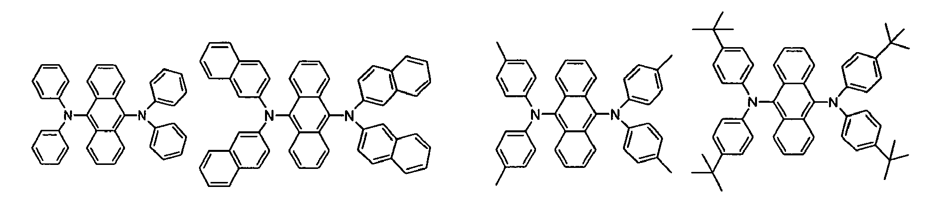

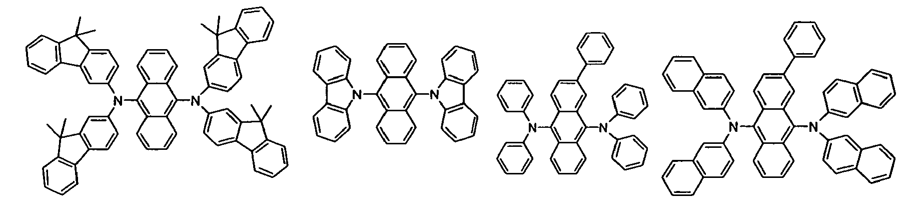

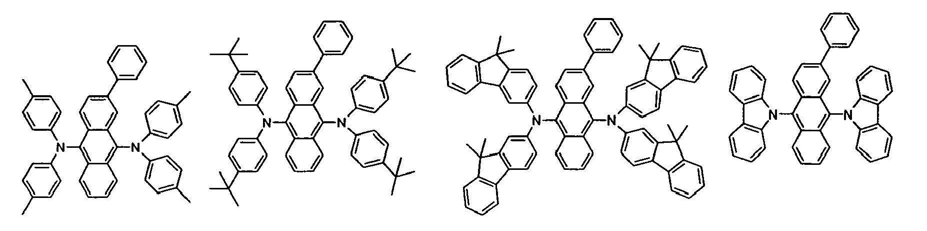

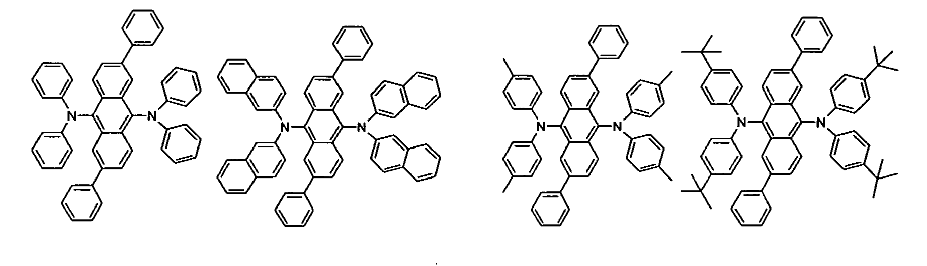

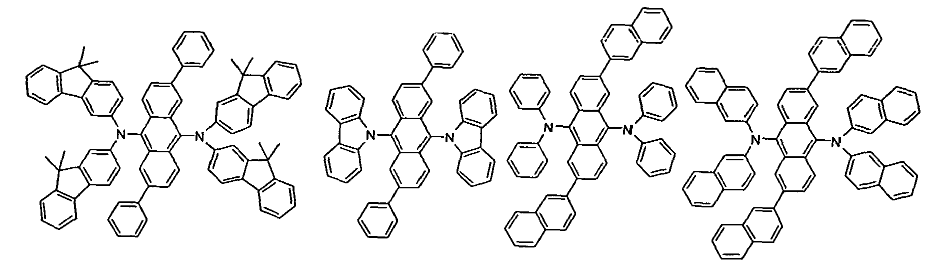

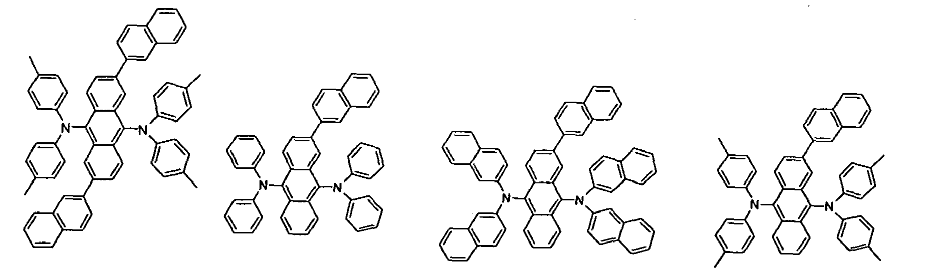

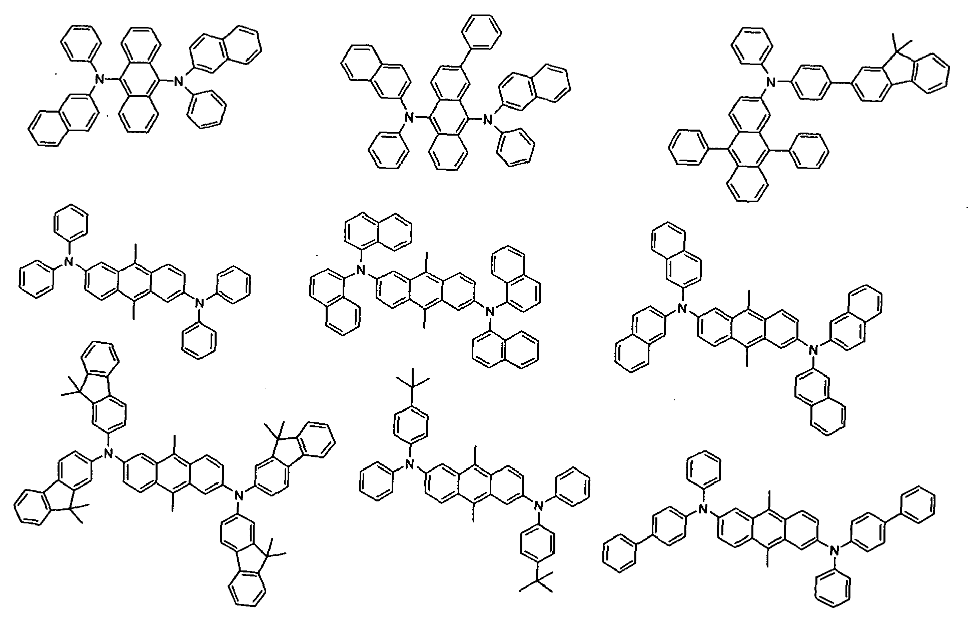

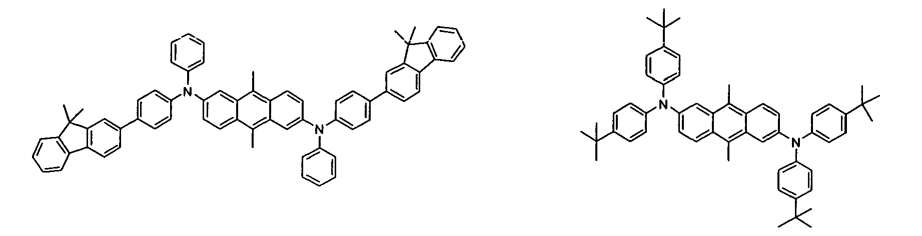

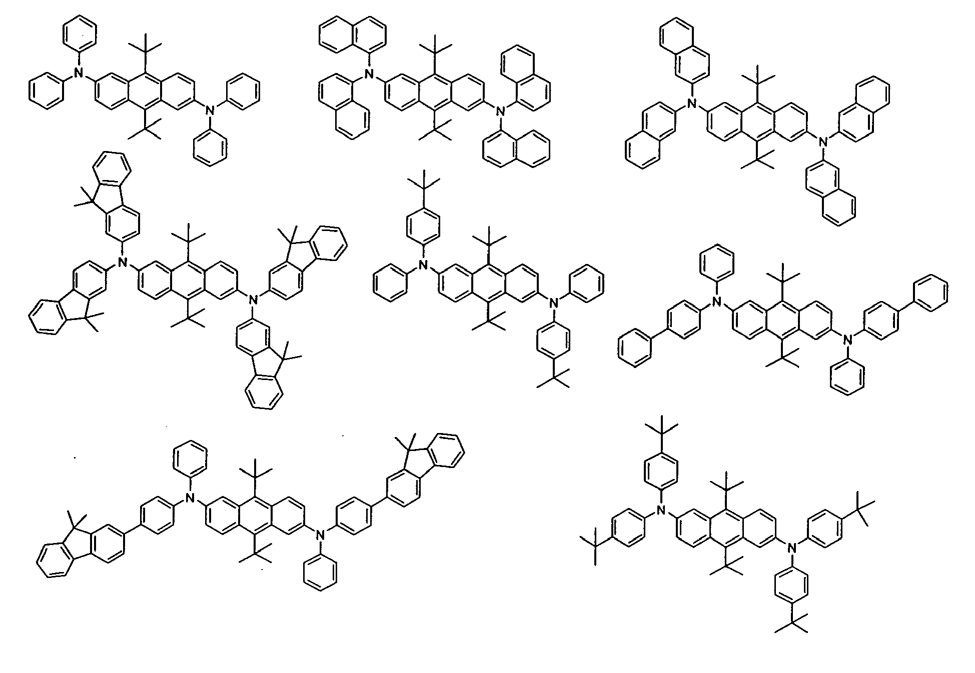

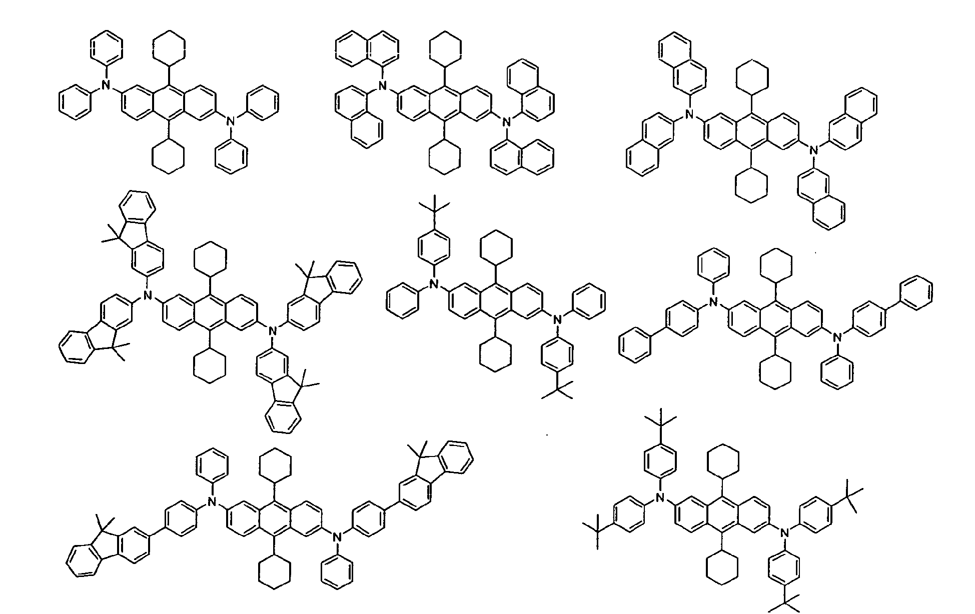

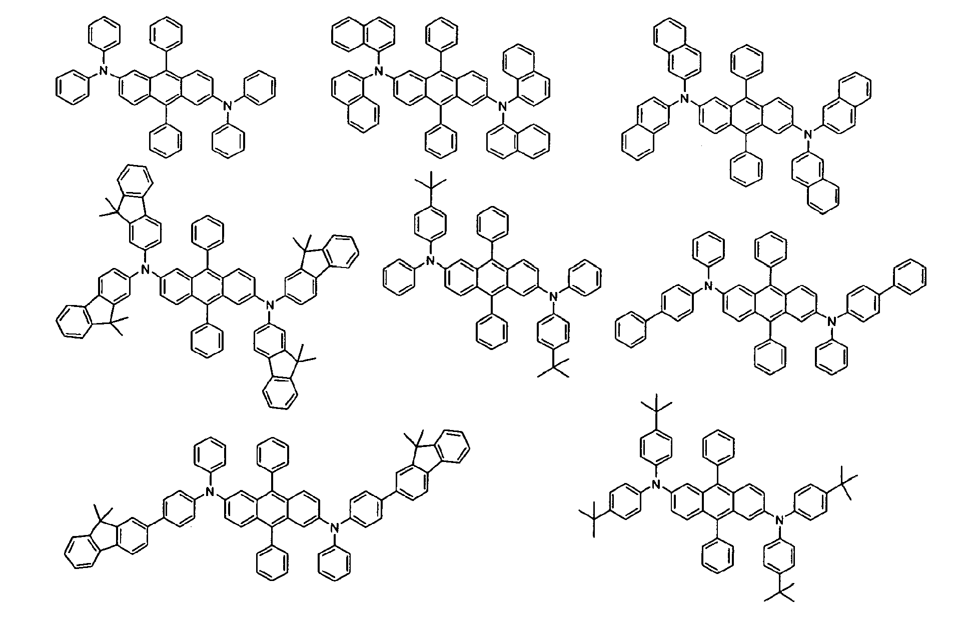

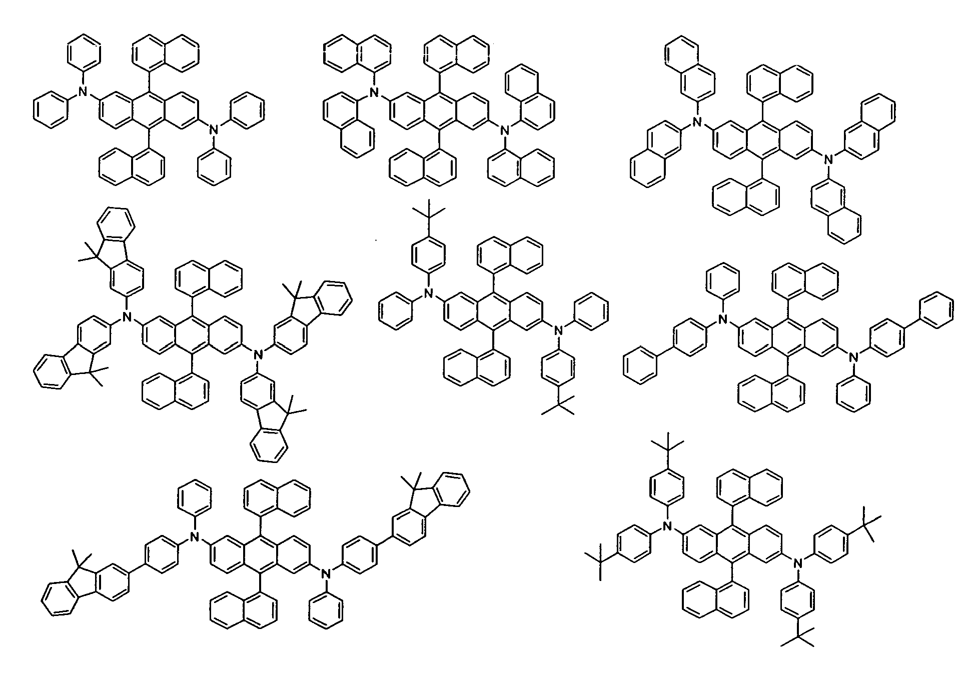

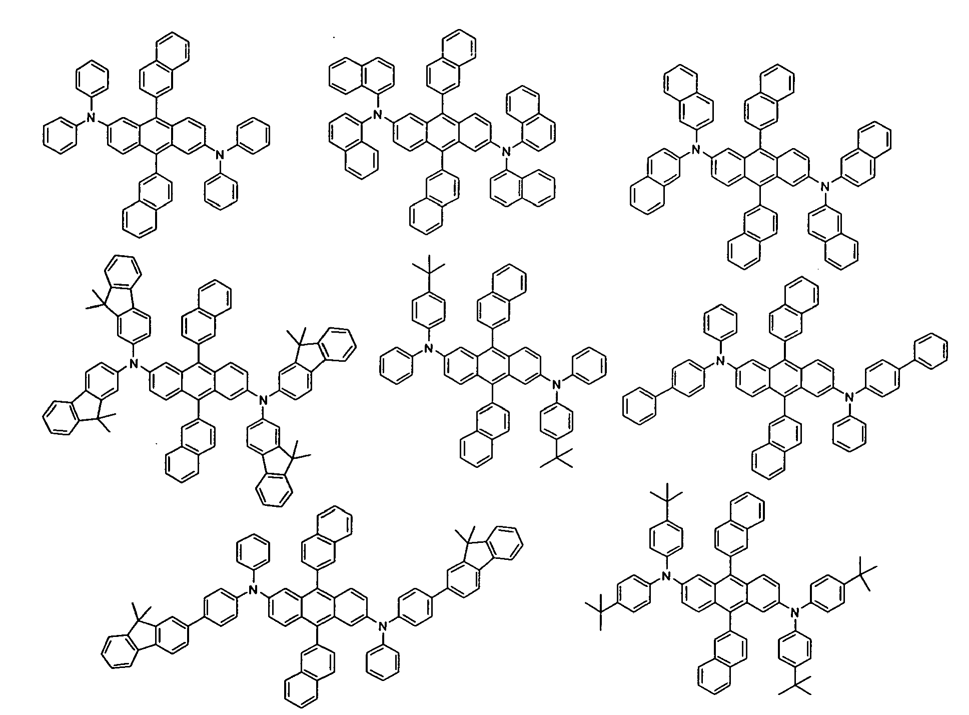

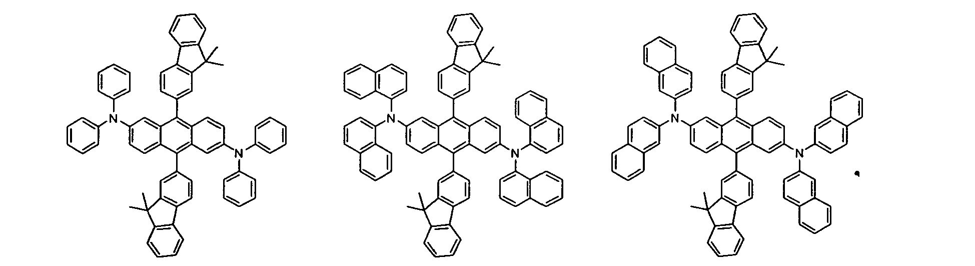

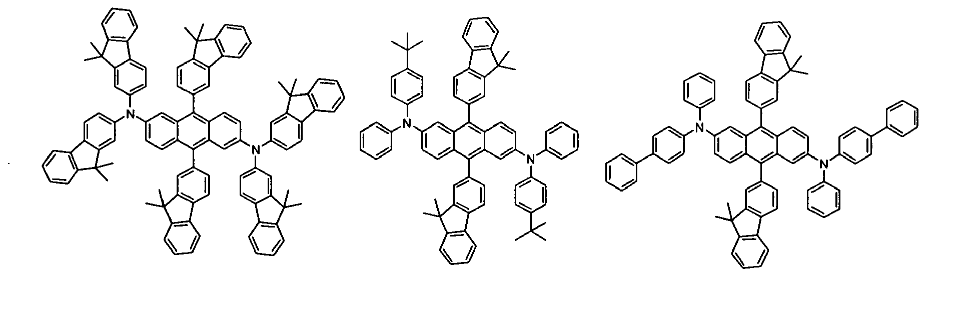

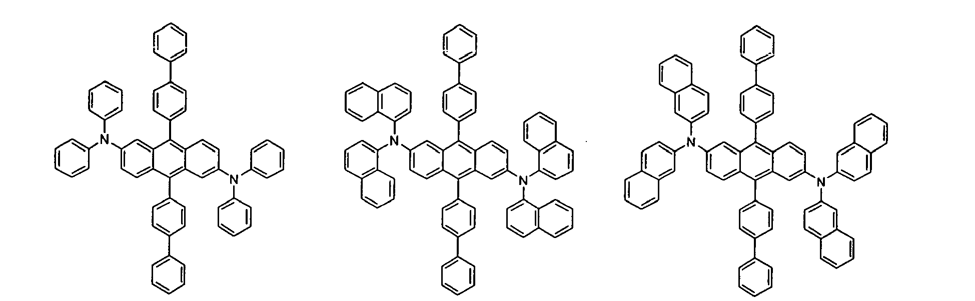

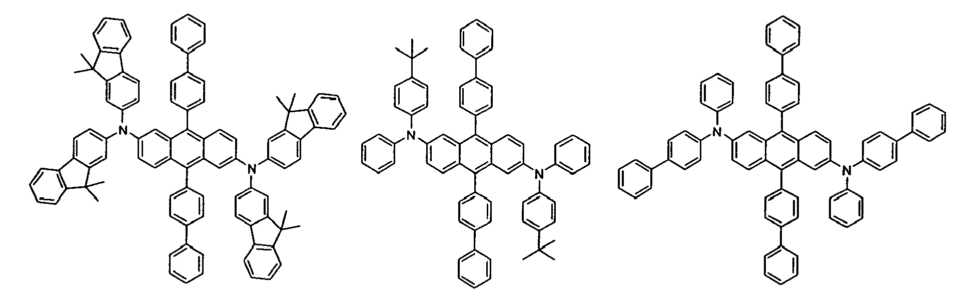

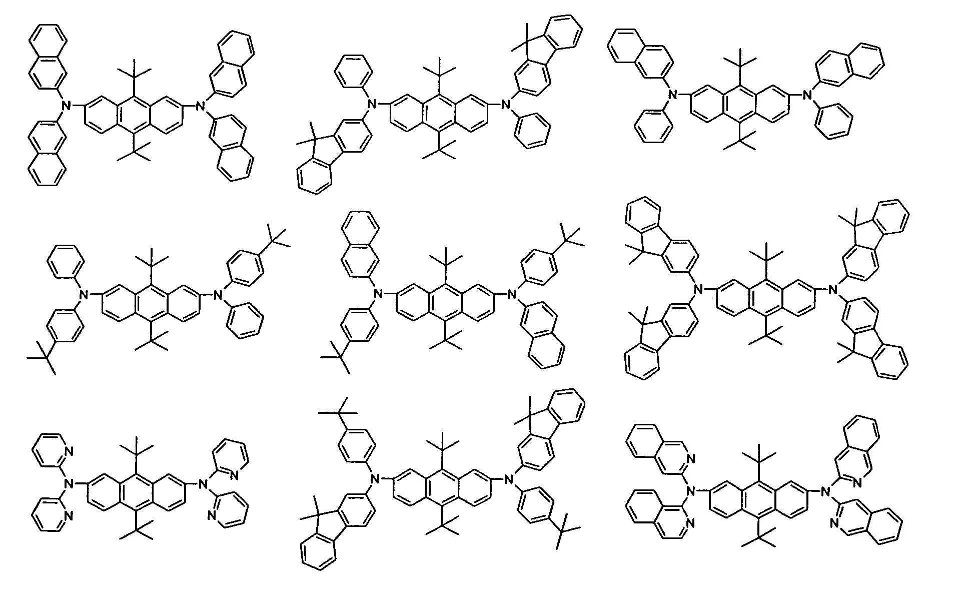

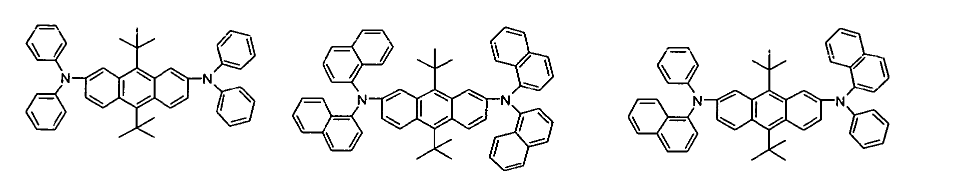

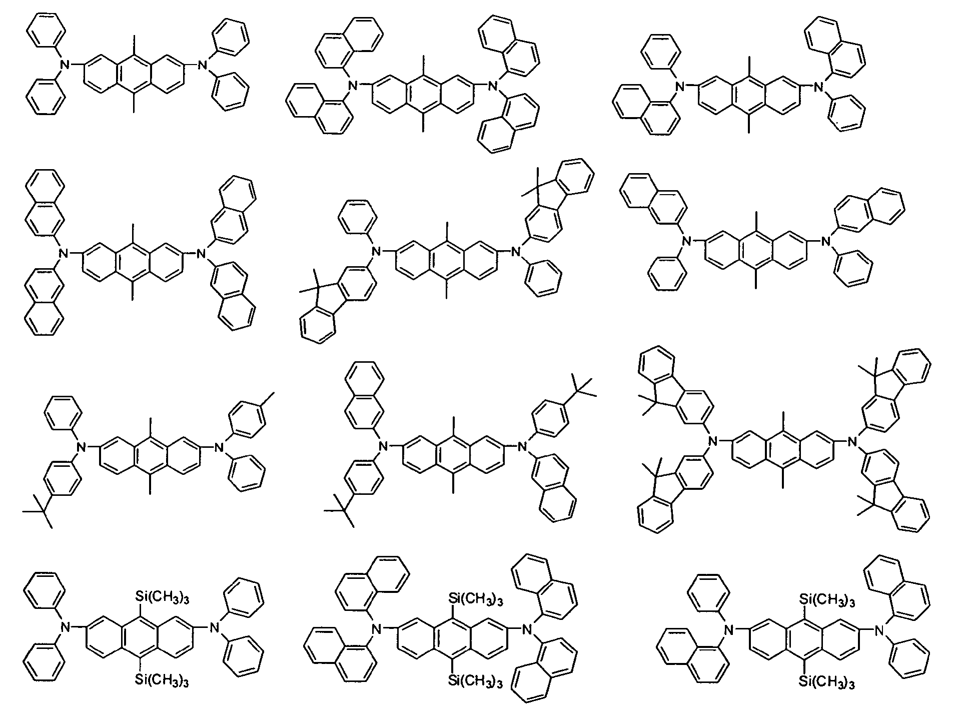

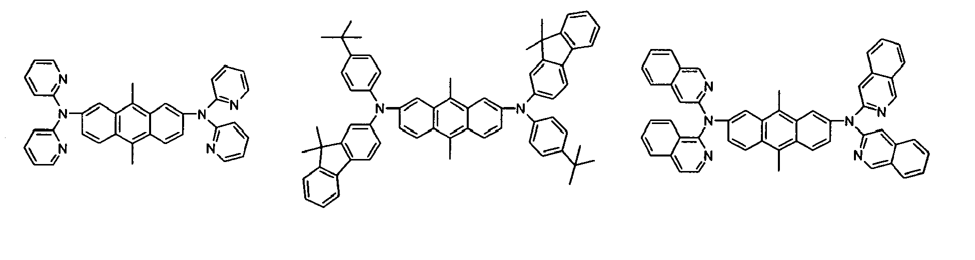

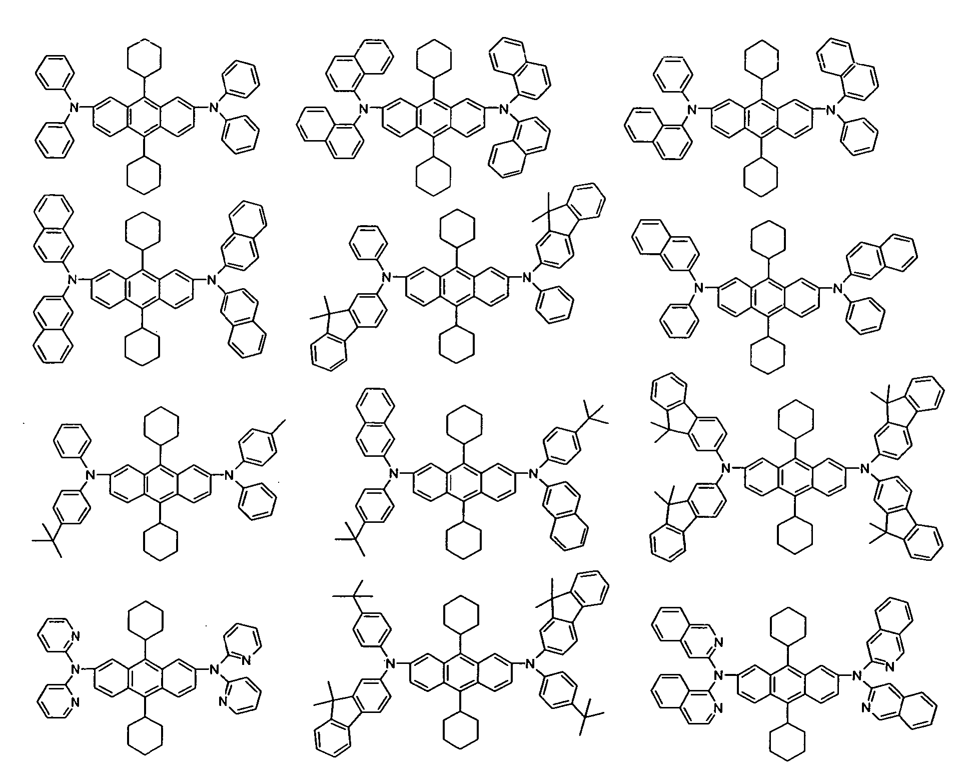

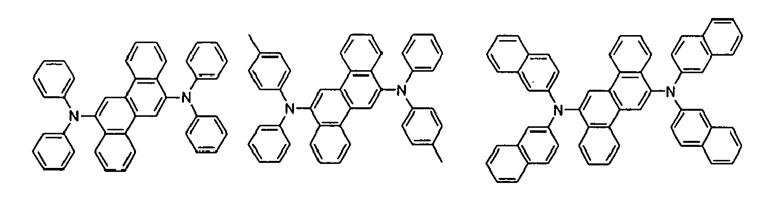

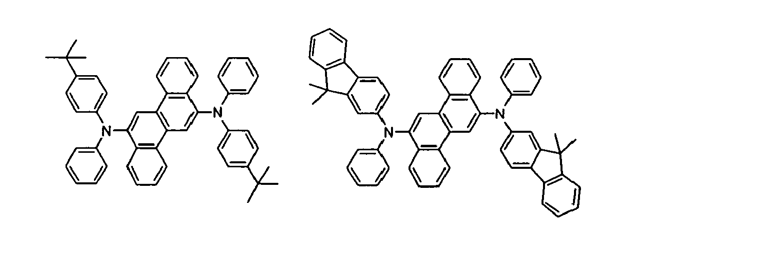

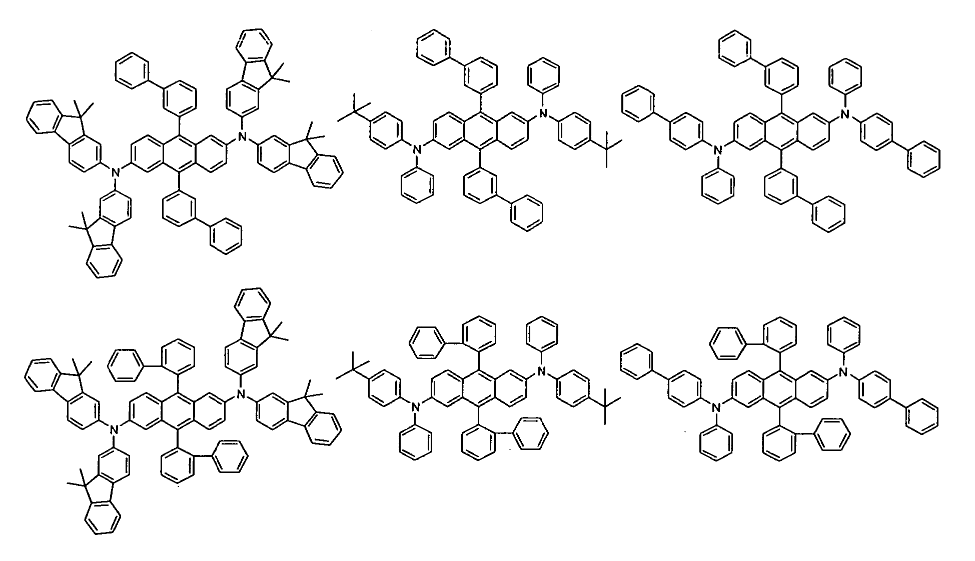

In an electroluminescent device according to the present invention, it is preferable to displace one or more layer(s) (here-in-below, referred to as the "surface layer") selected from chalcogenide layers, metal halide layers and metal oxide layers, on the inner surface of at least one side of the pair of electrodes. Specifically, it is preferable to arrange a chalcogenide layer of silicon and aluminum metal (including oxides) on the anode surface of the EL medium layer, and a metal halide layer or a metal oxide layer on the cathode surface of the EL medium layer. As the result, stability in operation can be obtained.

Examples of chalcogenides preferably include SiOₓ (1≤X≤2), AlOₓ (1≤X≤1.5), SiON, SiAlON, or the like. Examples of metal halides preferably include LiF, MgF₂, CaF₂, fluorides of rare earth metal or the like. Examples of metal oxides preferably include Cs₂O, Li₂O, MgO, SrO, BaO, CaO, or the like.

In an electroluminescent device according to the present invention, it is also preferable to arrange, on at least one surface of the pair of electrodes thus manufactured, a mixed region of electron transport compound and a reductive dopant, or a mixed region of a hole transport compound with an oxidative dopant. Accordingly, the electron transport compound is reduced to an anion, so that injection and transportation of electrons from the mixed region to an EL medium are facilitated. In addition, since the hole transport compound is oxidized to form a cation, injection and transportation of holes from the mixed region to an EL medium are facilitated. Preferable oxidative dopants include various Lewis acids and acceptor compounds. Preferable reductive dopants include alkali metals, alkali metal compounds, alkaline earth metals, rare-earth metals, and mixtures thereof.

The organic electroluminescent compounds according to the present invention, having high blue luminous efficiency and excellent life property of material, is advantageous in that they can be employed to manufacture organic light emitting diodes (OLED's) having excellent operation life.

### Best Mode

The present invention is further described by referring to representative compounds with regard to the organic electroluminescent compounds according to the invention, preparation thereof and luminous properties of the devices manufactured therefrom, but those examples are provided for better understanding of the invention and illustration of the embodiments only, not being intended to limit the scope of the invention by any means.

### Preparation Examples

### [Preparation Example 1] Synthesis of Compound (1)

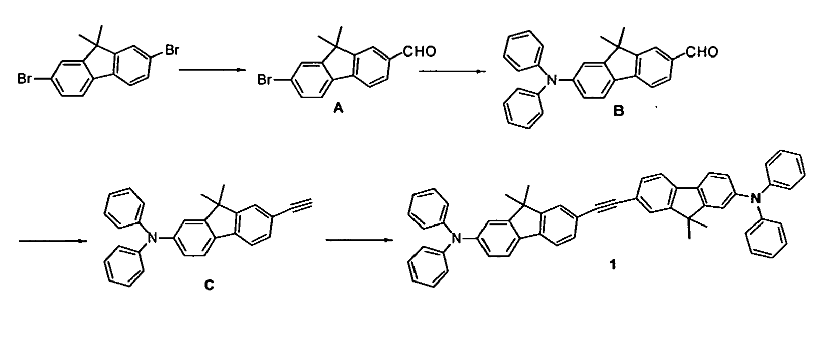

### Preparation of Compound (A)

In tetrahydrofuran (350 mL), dissolved was 2,6-dibromofluorene (53.0 g, 0.15 mol), and n-BuLi (1.6 M in n-hexane) (63.2 mL, 158 mmol) was slowly added dropwise at -78°C thereto. After stirring for 30 minutes, N,N-dimethylformamide (16.3 mL, 211 mmol) was added to the mixture. The temperature was slowly raised, and the reaction mixture was stirred for 2 hours. After adding aqueous NH₄Cl solution (20 mL) and distilled water (20 mL) to quench the reaction, the organic layer was isolated and evaporated under reduced pressure. The residue was recrystallized from methanol: n-hexane (1/1, v/v) (100 mL) to obtain Compound (A) (20.9 g, 69.4 mmol).

### Preparation of Compound (B)

The aldehyde compound (A) thus obtained (20.9 g, 69.4 mmol), diphenylamine (12.5 g, 104.1 mmol), cesium carbonate (24.1 g, 104.1 mmol) and palladium acetate (Pd(OAc)₂) (332 mg, 2.1 mmol) were suspended in toluene (800 mL). After adding tri-t-butyl phosphine (P(t-Bu)₃) (0.60 g, 4.2 mmol) thereto, the resultant mixture was stirred at 120°C for 4 hours. Aqueous saturated ammonium chloride solution (30 mL) was added, and the mixture was extracted with ethyl acetate (50 mL) and filtered. Recrystallization from methanol: n-hexane (1/1, v/v) (50 mL) gave Compound (B) (15.2 g, 39.0 mmol).

### Preparation of Compound (C)

Triphenyl phosphine (50 g, 190.6 mmol) was dissolved in dichloromethane (260 mL), and tetrabromomethane (CBr₄) (31.6 g, 95.3 mmol) solution was slowly added thereto over 10 minutes. The mixture was stirred at room temperature until the solution became dark brown, and water (40 mL) was slowly added thereto to quench the reaction. The mixture was extracted, and the extract was dried under reduced pressure to obtain solid. The solid was added to methanol, and stirred under reflux. The insoluble solid was filtered off, and the filtrate was evaporated under reduced pressure. Recrystallization from ethyl acetate/ methanol gave phosphine complex (45 g, 75%).

The phosphine complex thus obtained (19.8 g, 38.5 mmol) and potassium t-butoxide (KOC(CH₃)₃) (4.3 g, 38.5 mmol) were dissolved in tetrahydrofuran (250 mL), and Compound (B) (5 g, 12.8 mmol) was added thereto. After stirring at room temperature for 10 minutes, potassium t-butoxide (KOC(CH₃)₃) (11.5 g, 102.7 mmol) was added thereto, and the mixture was stirred at room temperature for 2 hours. When the reaction was completed, the reaction mixture was extracted by using water and ether, and dried under reduced pressure. Purification via column chromatography gave Compound (C) (1.9 g, 38%).

### Preparation of Compound (1)

Compound (C) (10 g, 25.9 mmol), 7-bromo-9,9-dimethyl-N,N-diphenyl-9H-fluoren-2-amine (12.7 g, 28.8 mmol), Pd(dba)₂ (0.2 g, 0.4 mmol), triphenylphosphine (0.8 g, 2.9 mmol) and copper (I) iodide (CuI) (0.5 g, 2.6 mmol) were dissolved in triethylamine (260 mL), and the solution was stirred under reflux for 24 hours. When the reaction was completed, the reaction mixture was cooled to room temperature, and extracted by using dichloromethane and water, and the extract was dried under reduced pressure. Purification via column chromatography gave Compound (1) (16 g, 72%).

### [Preparation Example 2] Preparation of Compound (1081)

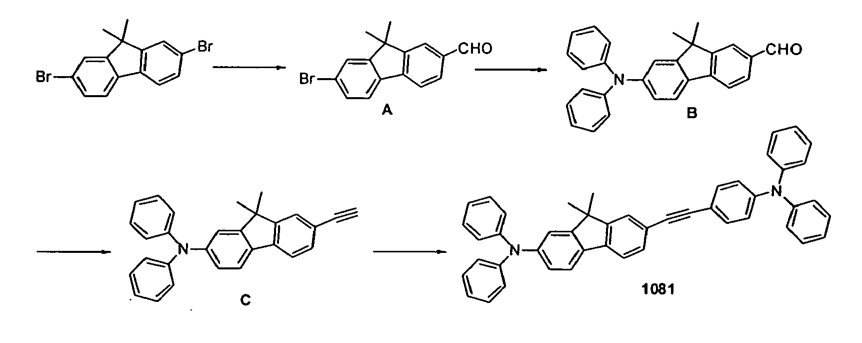

### Preparation of Compound (A)

In tetrahydrofuran (350 mL), dissolved was 2,6-dibromofluorene (53.0 g, 0.15 mmol), and n-BuLi (1.6 M in n-hexane) (63.2 mL, 158 mmol) was slowly added dropwise thereto at -78°C. After stirring for 30 minutes, N,N-dimethylformamide (16.3 mL, 211 mmol) was added thereto. The temperature was slowly raised, and stirring continued for 2 hours. Then, aqueous NH₄Cl solution (20 mL) and distilled water (20 mL) were added thereto to quench the reaction. The organic layer isolated was evaporated under reduced pressure. Recrystallization from methanol: n-hexane (1/1, v/v) (100 mL) gave Compound (A) (20.9 g, 69.4 mmol).

### Preparation of Compound (B)

The aldehyde compound (A) thus obtained (20.9 9 g, 69.4 mmol), diphenylamine (12.5 g, 104.1 mmol), cesium carbonate (24.1 g, 104.1 mmol) and palladium acetate (Pd(OAc)₂) (332 mg, 2.1 mmol) were suspended in toluene (800 mL). Tri-t-butyl phosphine (P(t-Bu)₃) (0.60 g, 4.2 mmol) was added thereto, and the resultant mixture was stirred at 120°C for 4 hours. Aqueous saturated ammonium chloride solution (30 mL) was added thereto, and the mixture was extracted with ethyl acetate (50 mL), and filtered. Recrystallization from methanol: n-hexane (1/1, v/v) (50 mL) gave Compound (B) (15.2 g, 39.0 mmol).

### Preparation of Compound (C)

Triphenyl phosphine (50 g, 190.6 mmol) was dissolved in dichloromethane (260 mL), and tetrabromomethane (CBr₄) (31.6 g, 95.3 mmol) solution was slowly added thereto over 10 minutes. The mixture was stirred at room temperature until the solution became dark brown, and water (40 mL) was slowly added thereto to quench the reaction. The mixture was extracted, and the extract was dried under reduced pressure to obtain solid. The solid was added to methanol, and stirred under reflux. The insoluble solid was filtered off, and the filtrate was evaporated under reduced pressure. Recrystallization from ethyl acetate/ methanol gave phosphine complex (45 g, 75%).

The phosphine complex thus obtained (19.8 g, 38.5 mmol) and potassium t-butoxide (KOC(CH₃)₃) (4.3 g, 38.5 mmol) were dissolved in tetrahydrofuran (250 mL), and Compound (B) (5 g, 12.8 mmol) was added thereto. After stirring at room temperature for 10 minutes, potassium t-butoxide (KOC(CH₃)₃) (11.5 g, 102.7 mmol) was added thereto, and the mixture was stirred at room temperature for 2 hours. When the reaction was completed, the reaction mixture was extracted by using water and ether, and dried under reduced pressure. Purification via column chromatography gave Compound (C) (1.9 g, 38%).

### Preparation of Compound (1081)

In tetrahydrofuran (30 mL), dissolved were N-(4-bromophenyl)-N-phenylbenzenamine (0.9 g, 2.9 mmol), Pd₂dba₃ (12 mg, 0.013 mmol), tri-t-butyl phosphine (6.4 µL) (0.013 mmol, 50 wt% in toluene) and triethylamine (0.5 mL, 3.9 mmol). After stirring the solution for 5 minutes, Compound (C) (1 g, 2.6 mmol) was added thereto, and the resultant mixture was stirred under reflux for 12 hours. When the reaction was completed, the reaction mixture was cooled to room temperature, and extracted by using dichloromethane and water. The extract was dried under reduced pressure and purified by column chromatography to obtain Compound (1081) (0.96 g, 59%).

According to the same procedure as Preparation Examples 1 and 2, the organic electroluminescent compounds (Compounds 1 to 2771) listed in Table 1 were prepared, of which the ¹H NMR and MS/FAB data are listed in Table 2.

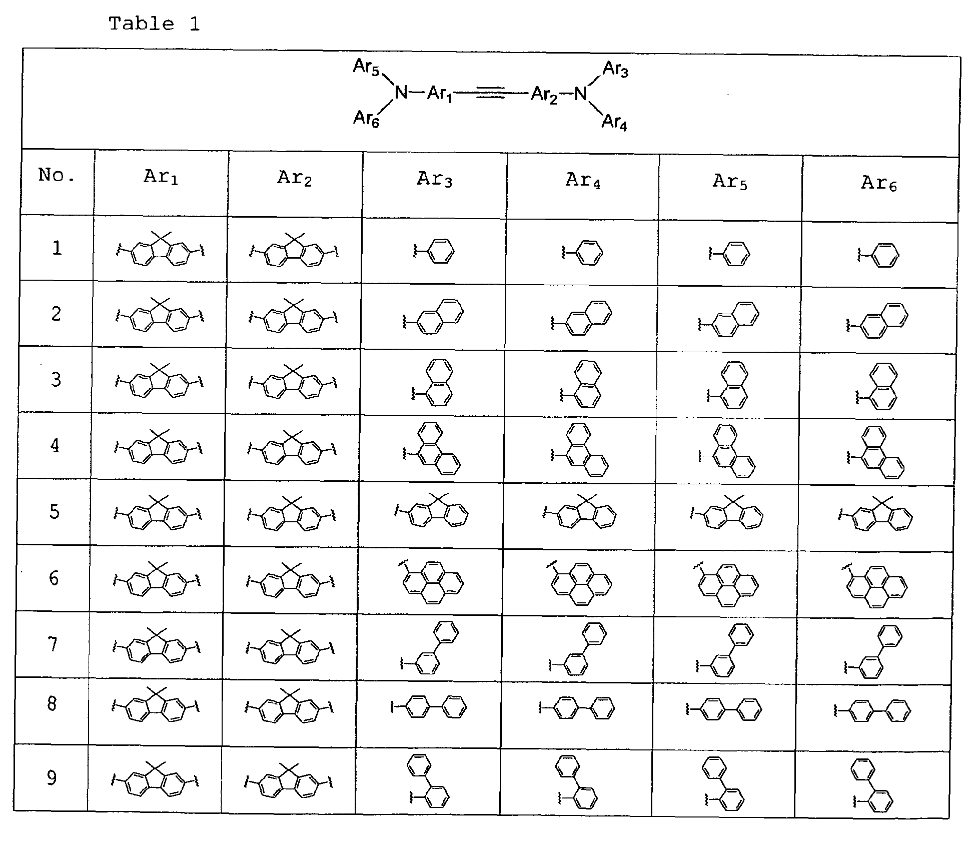

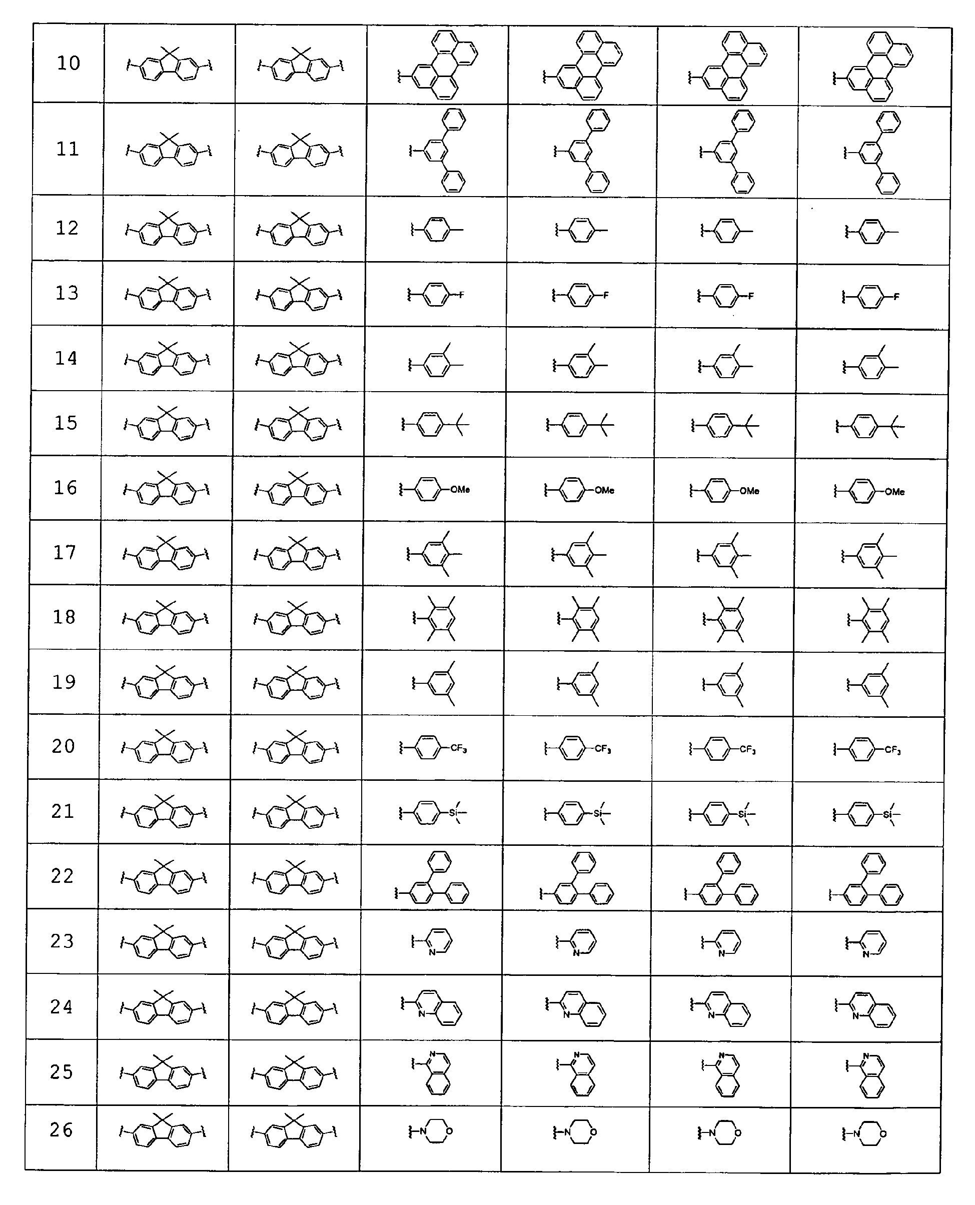

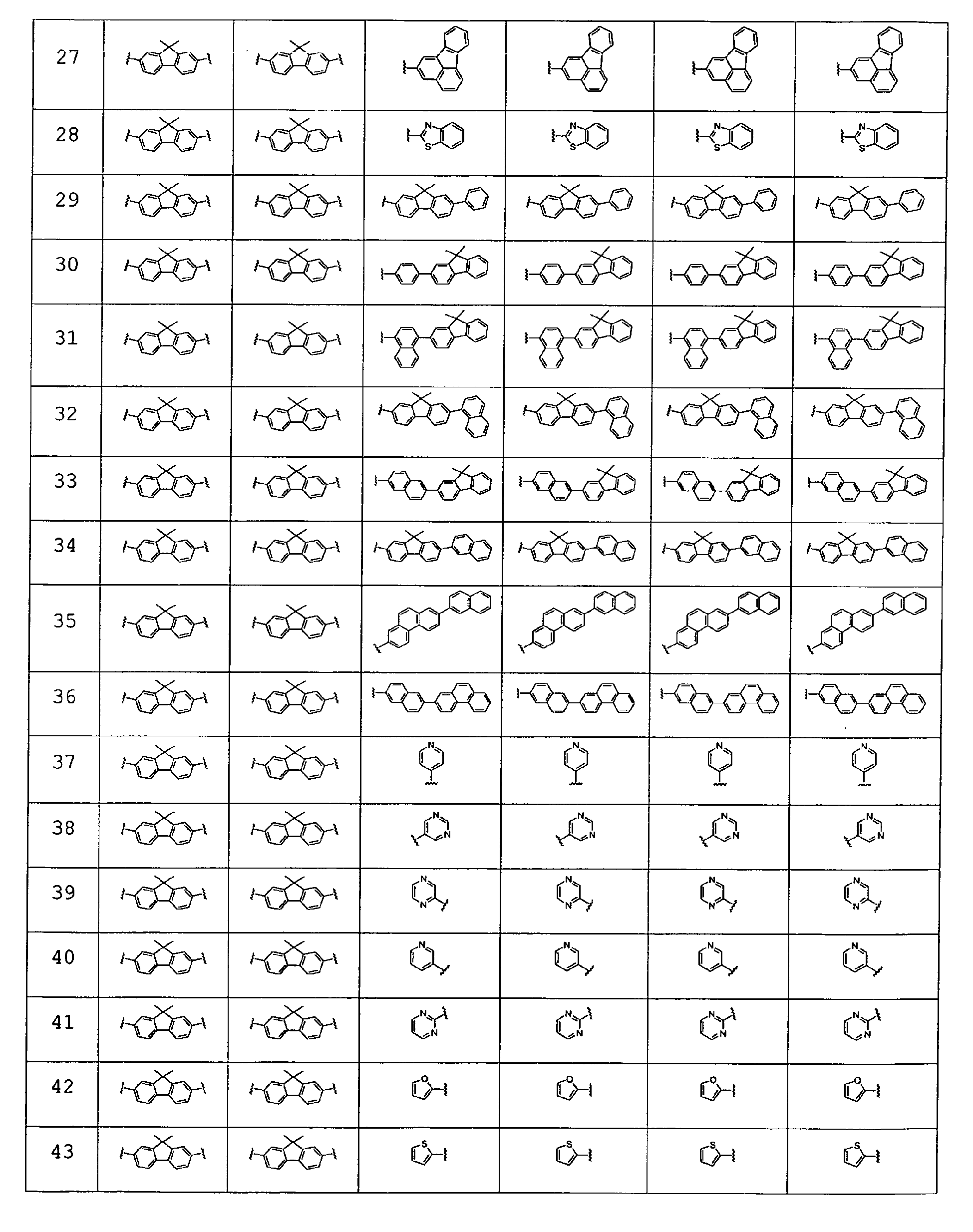

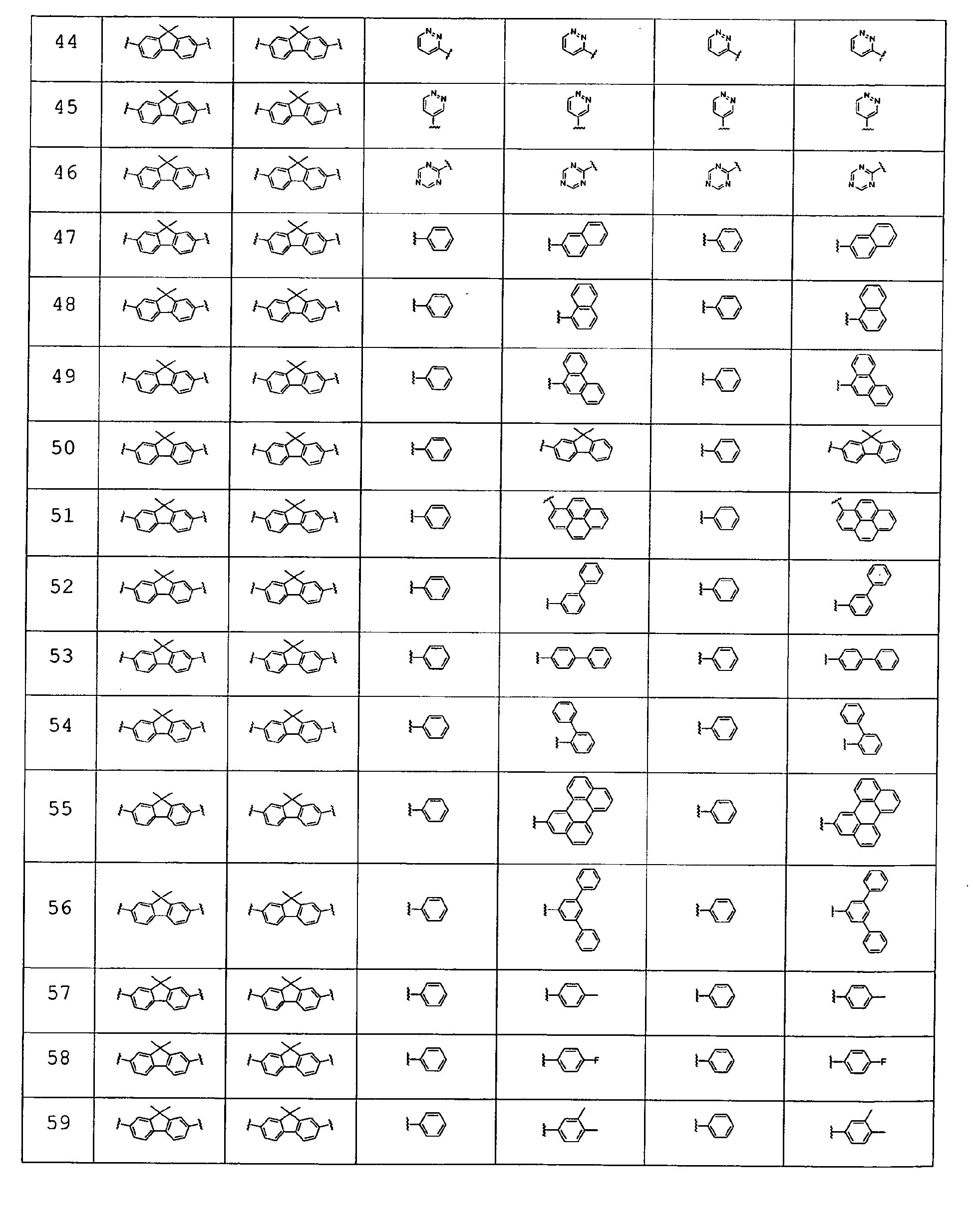

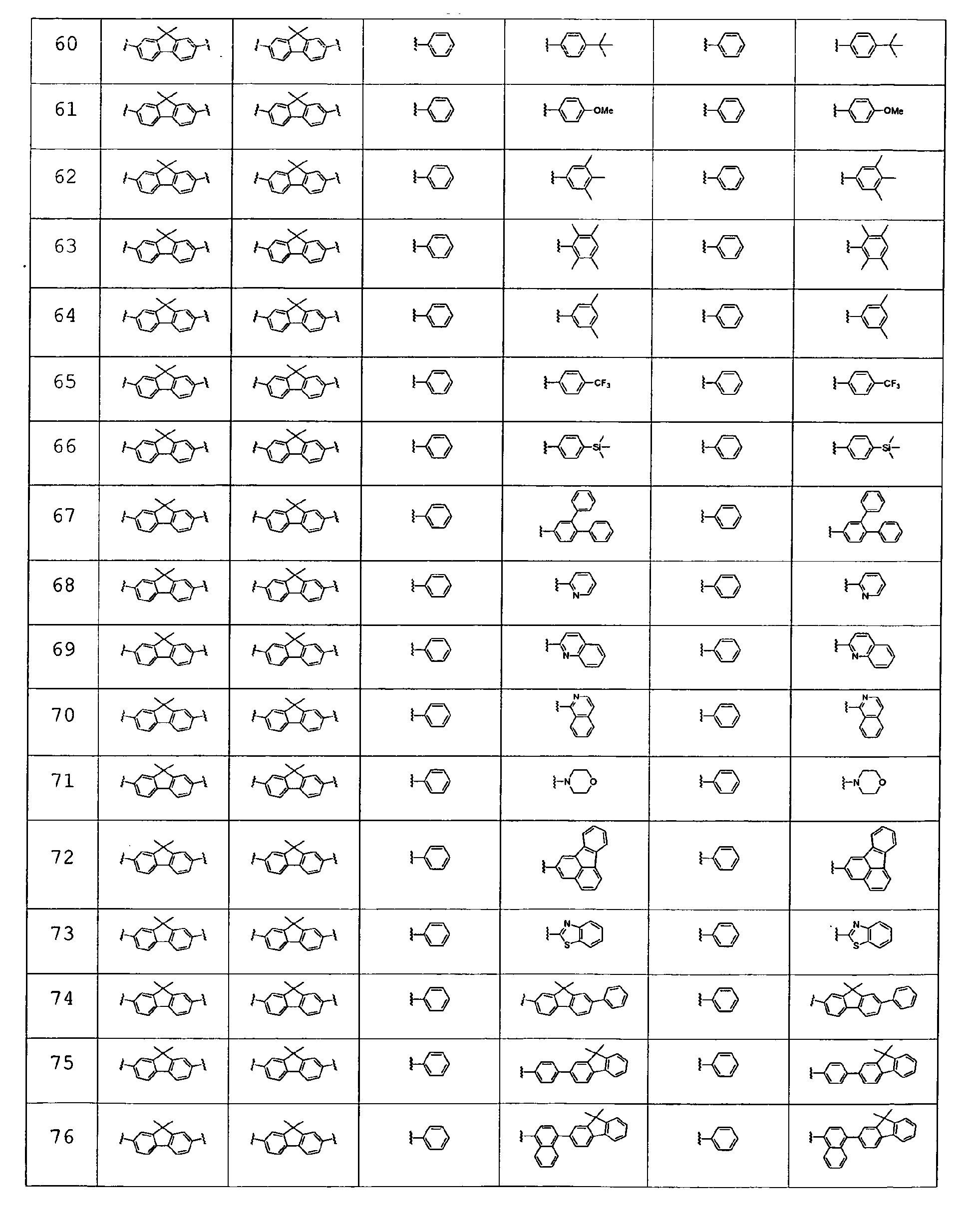

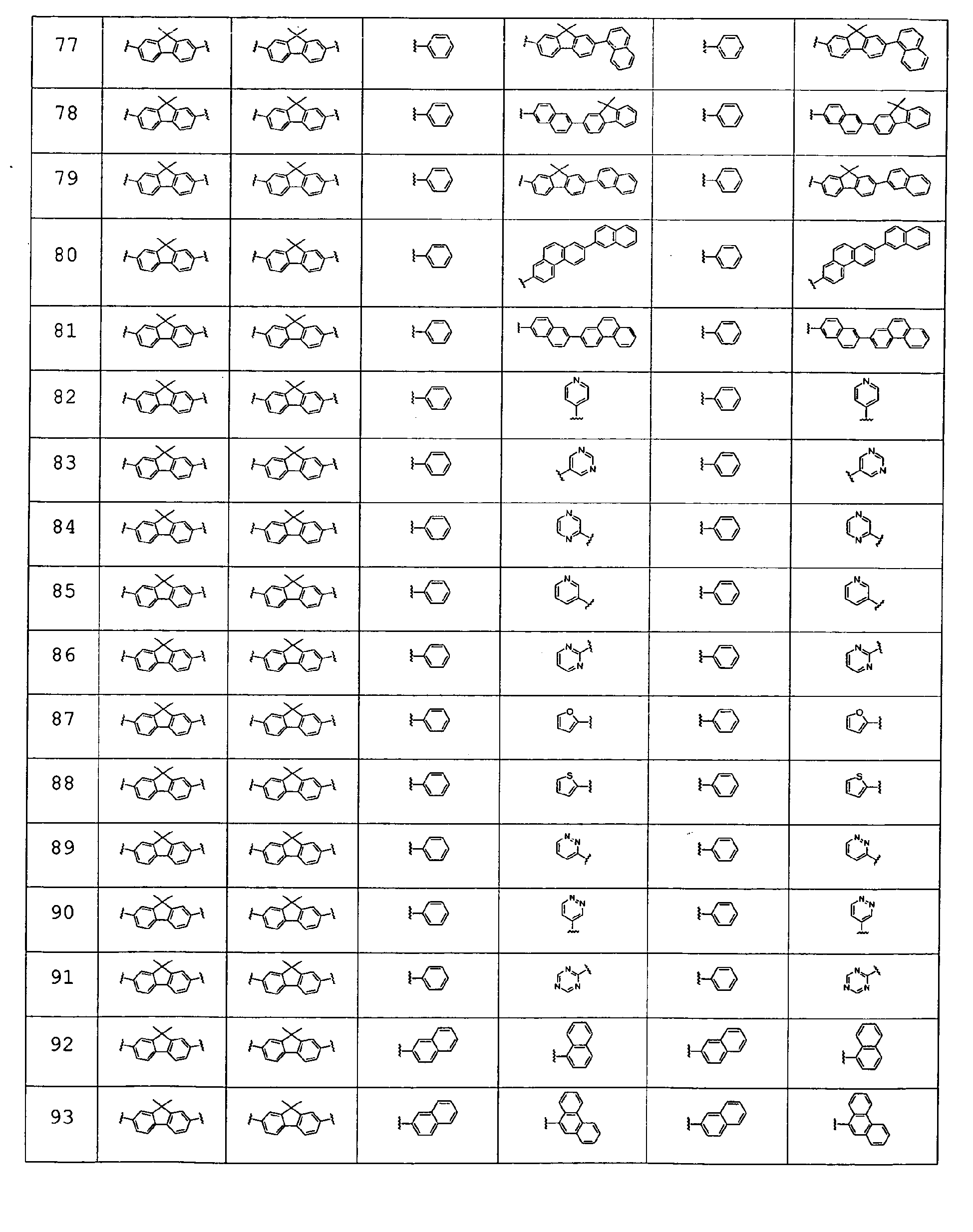

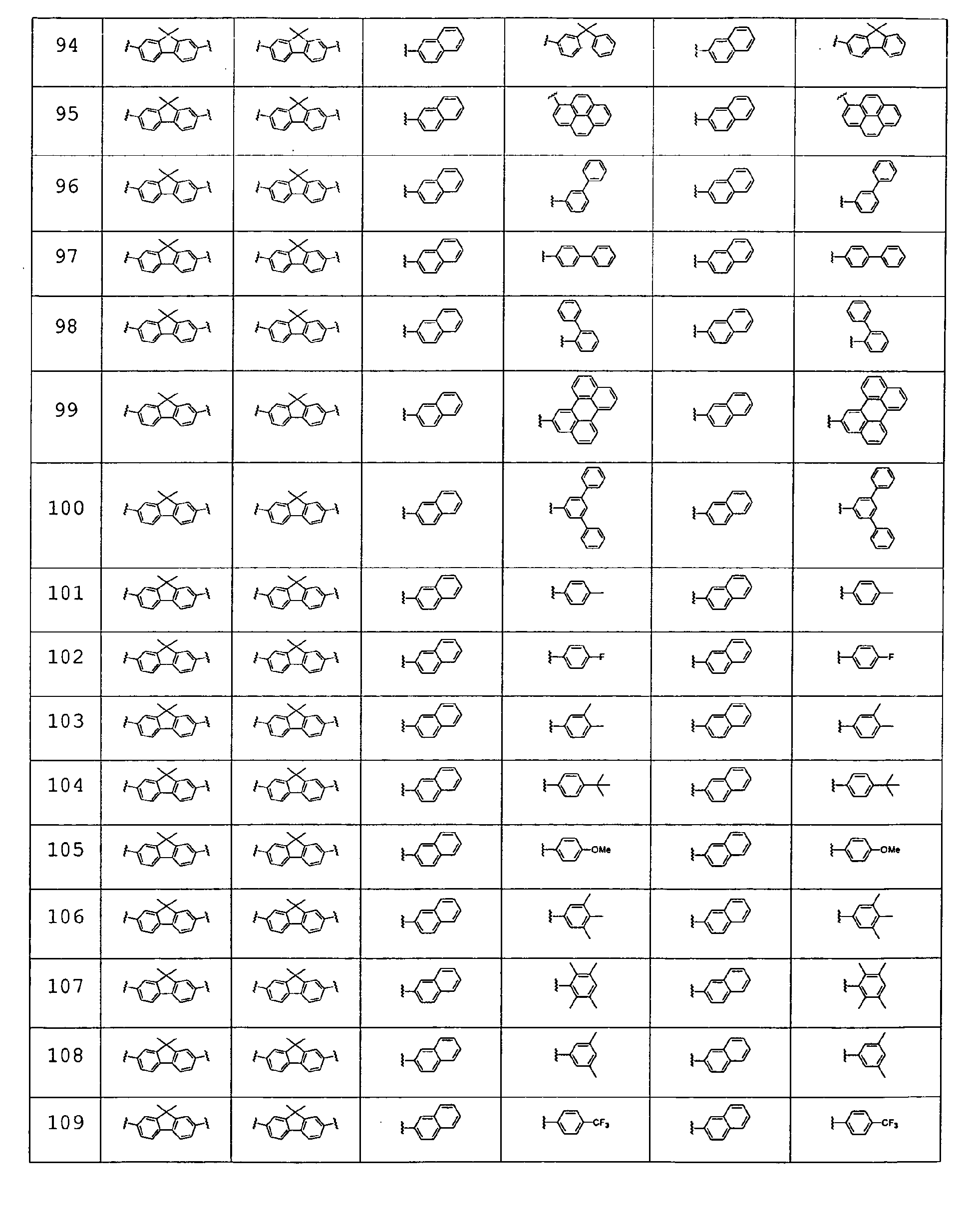

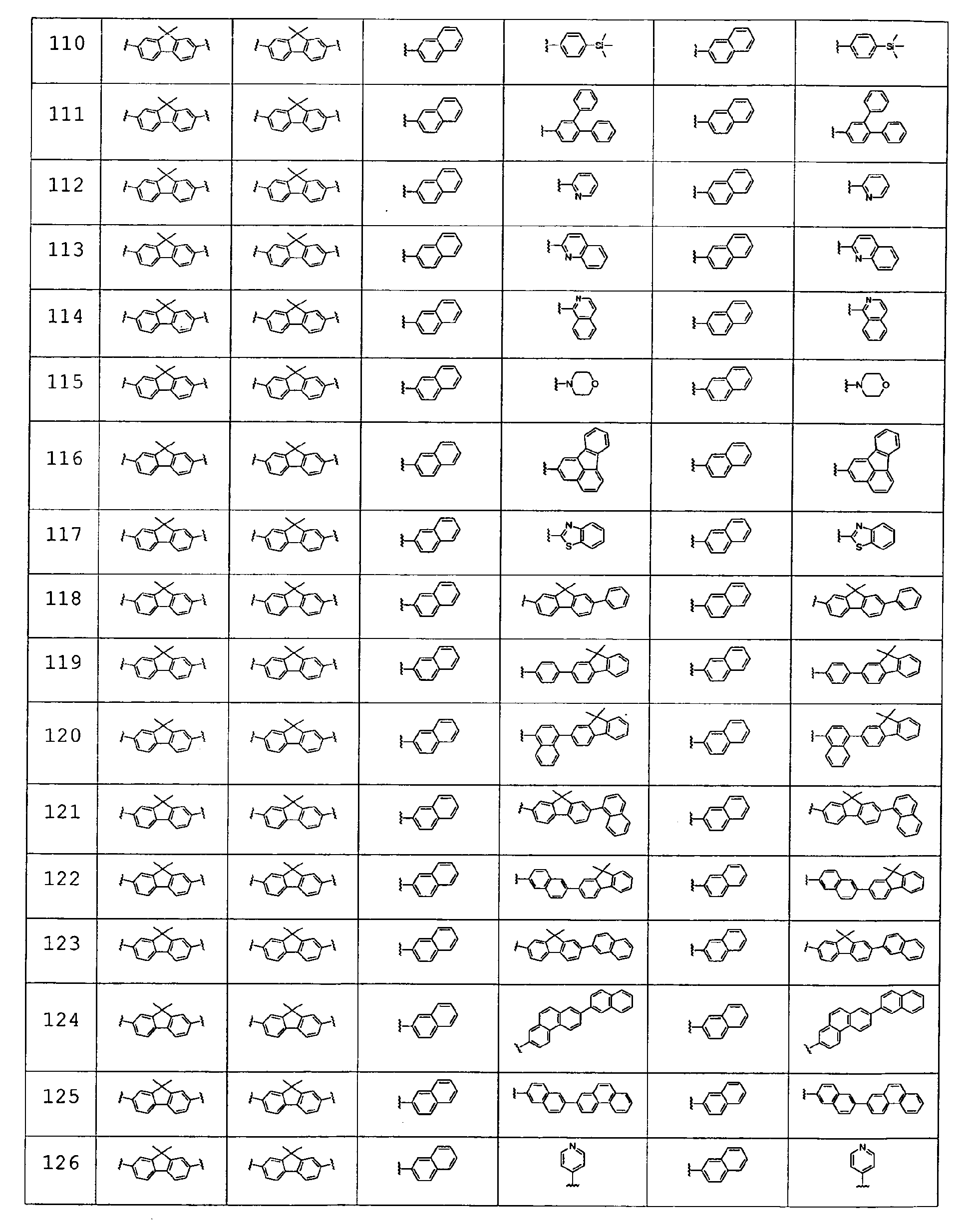

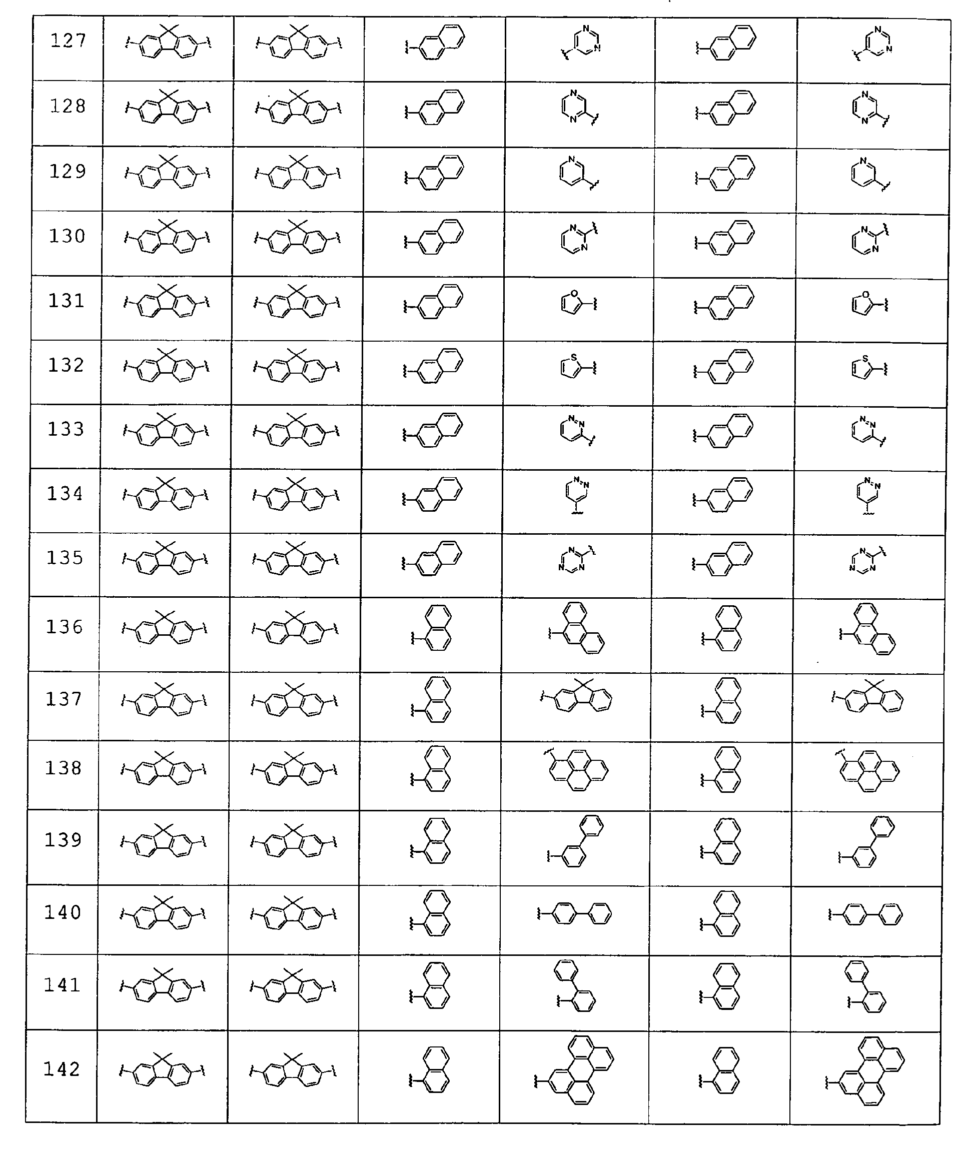

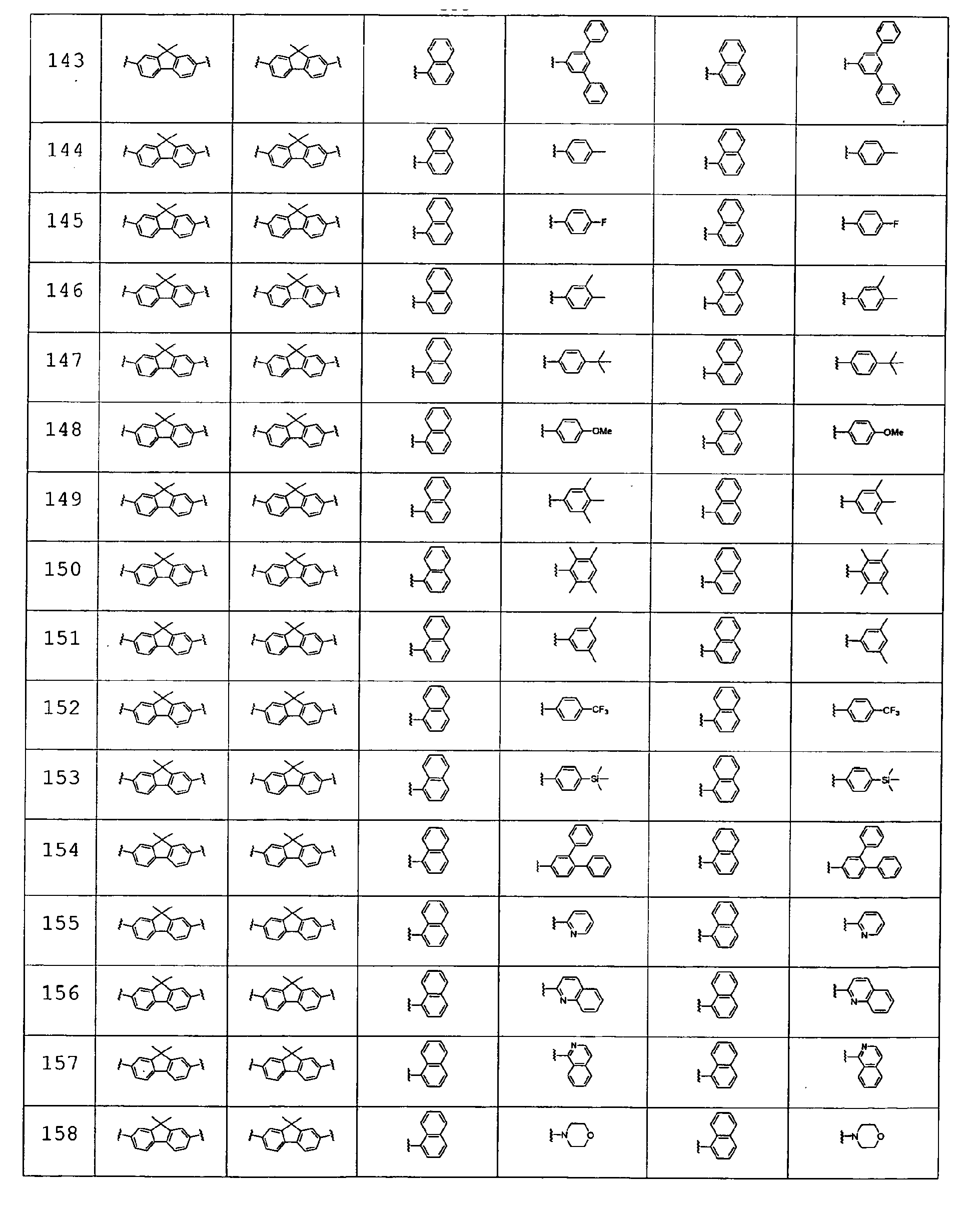

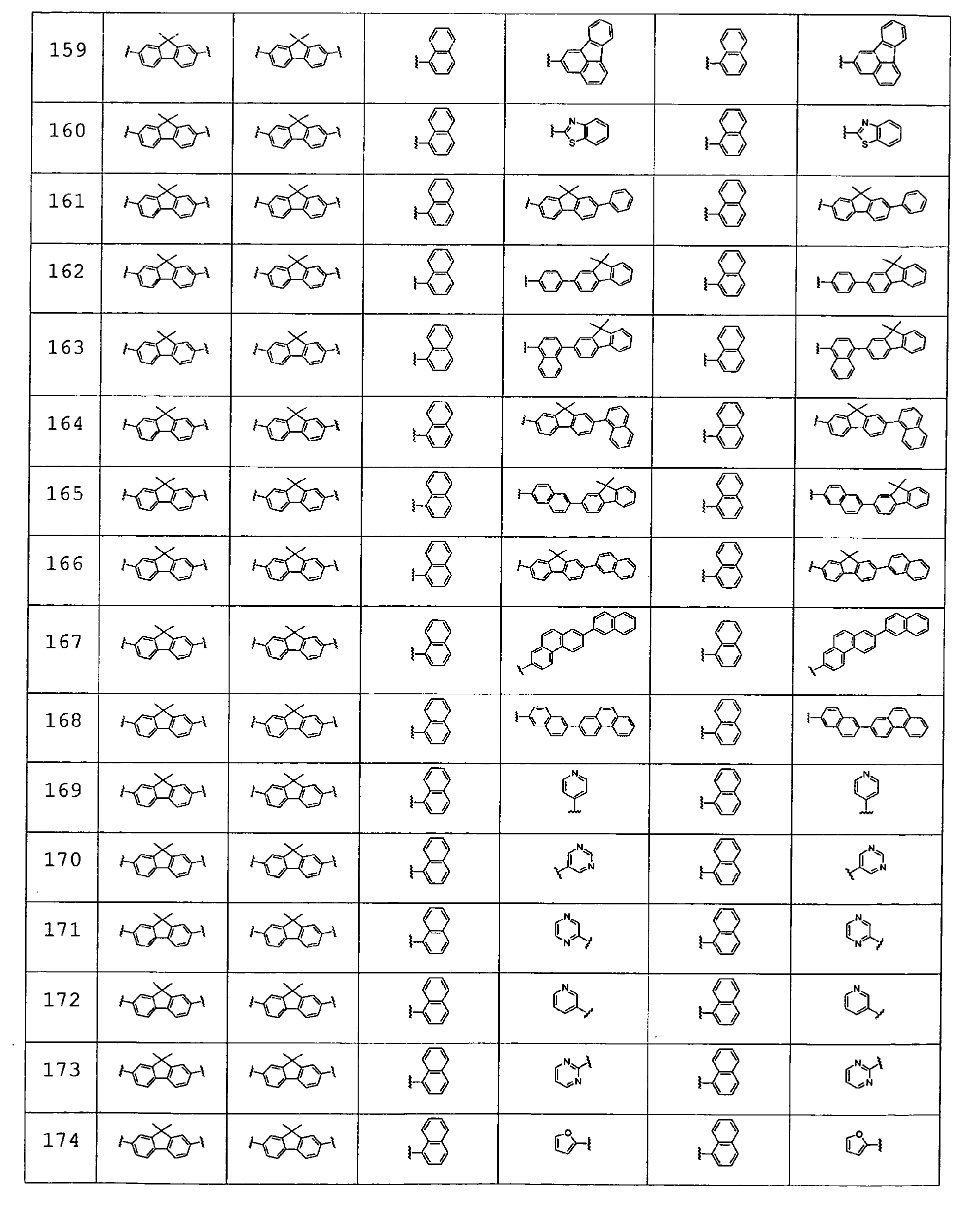

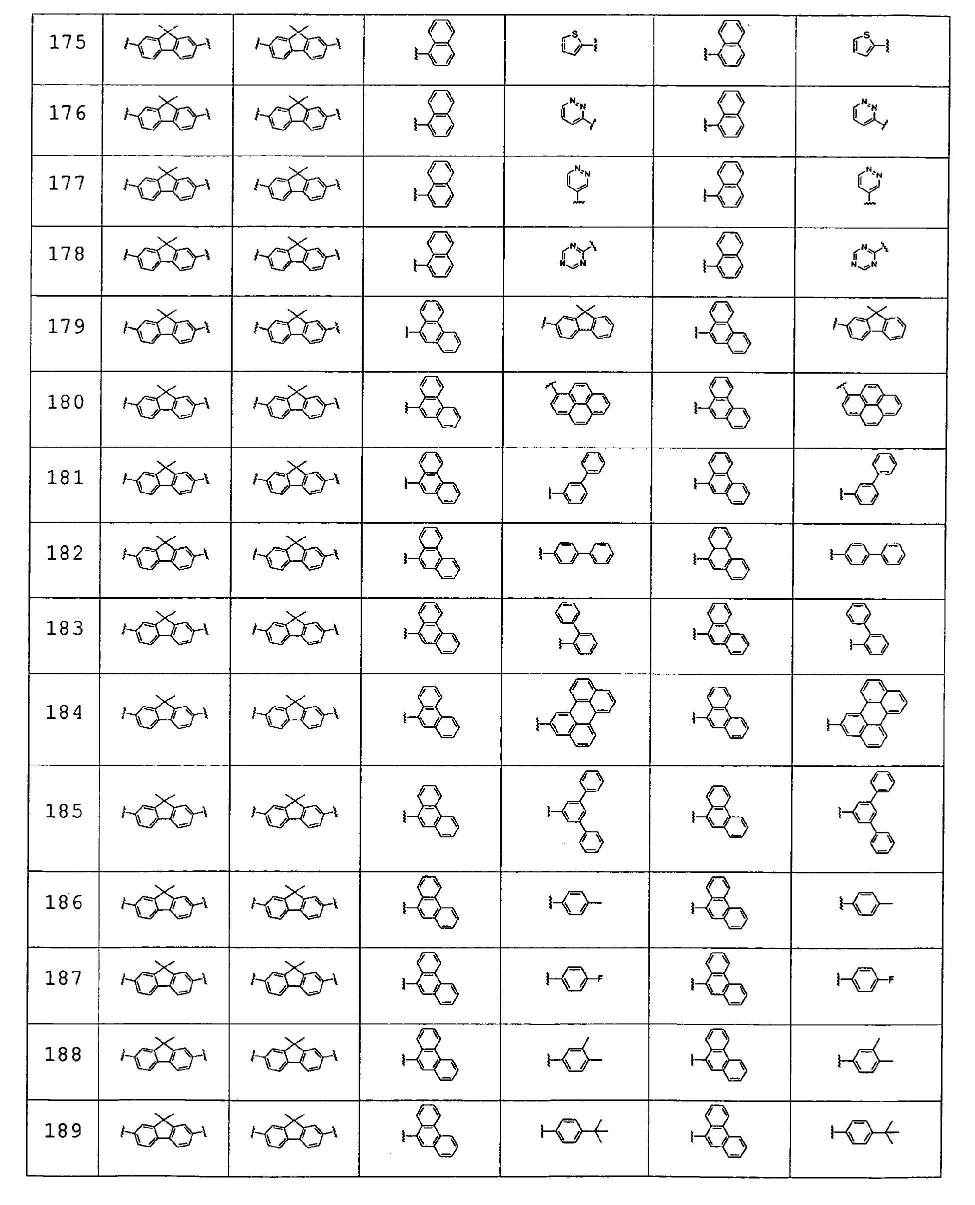

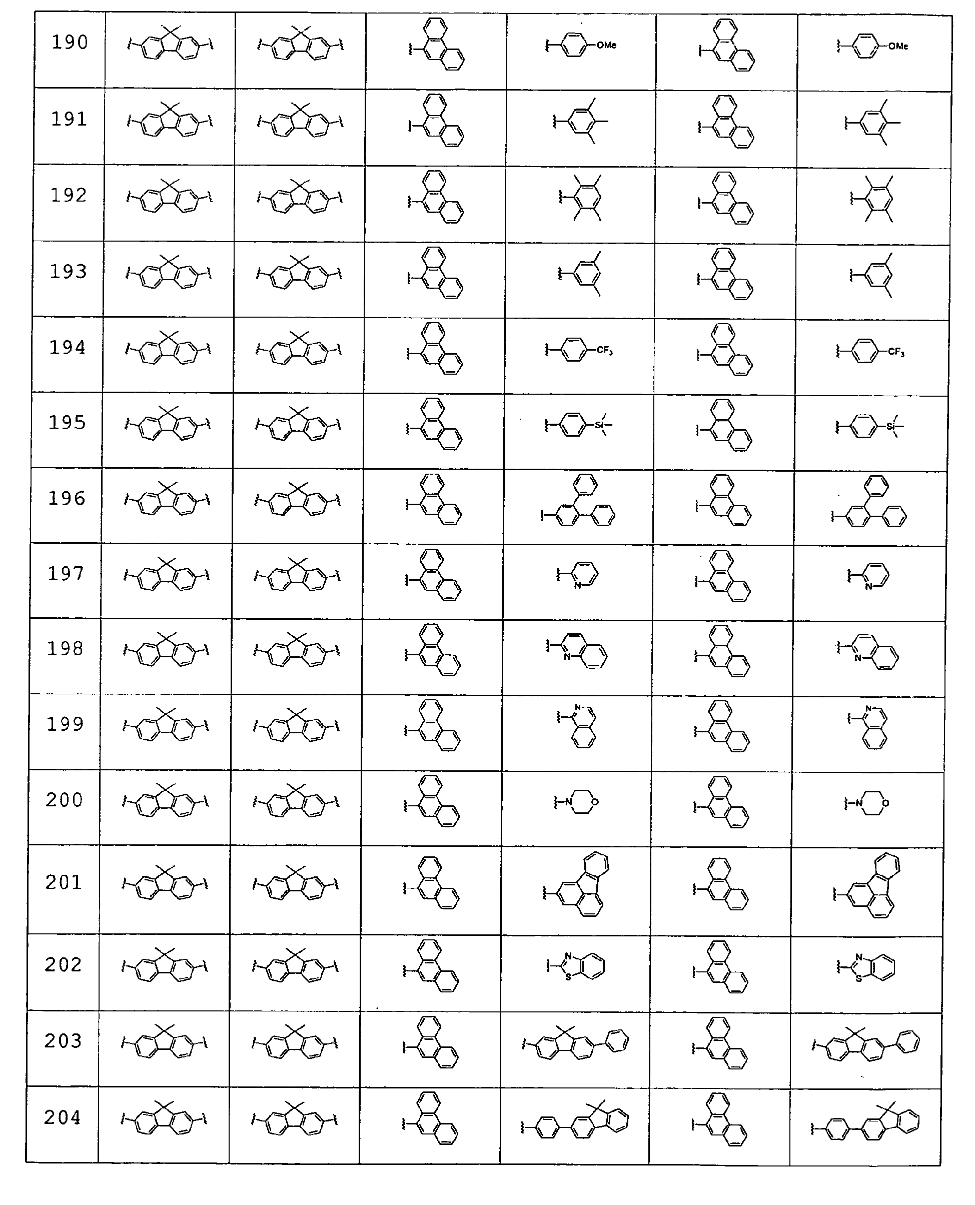

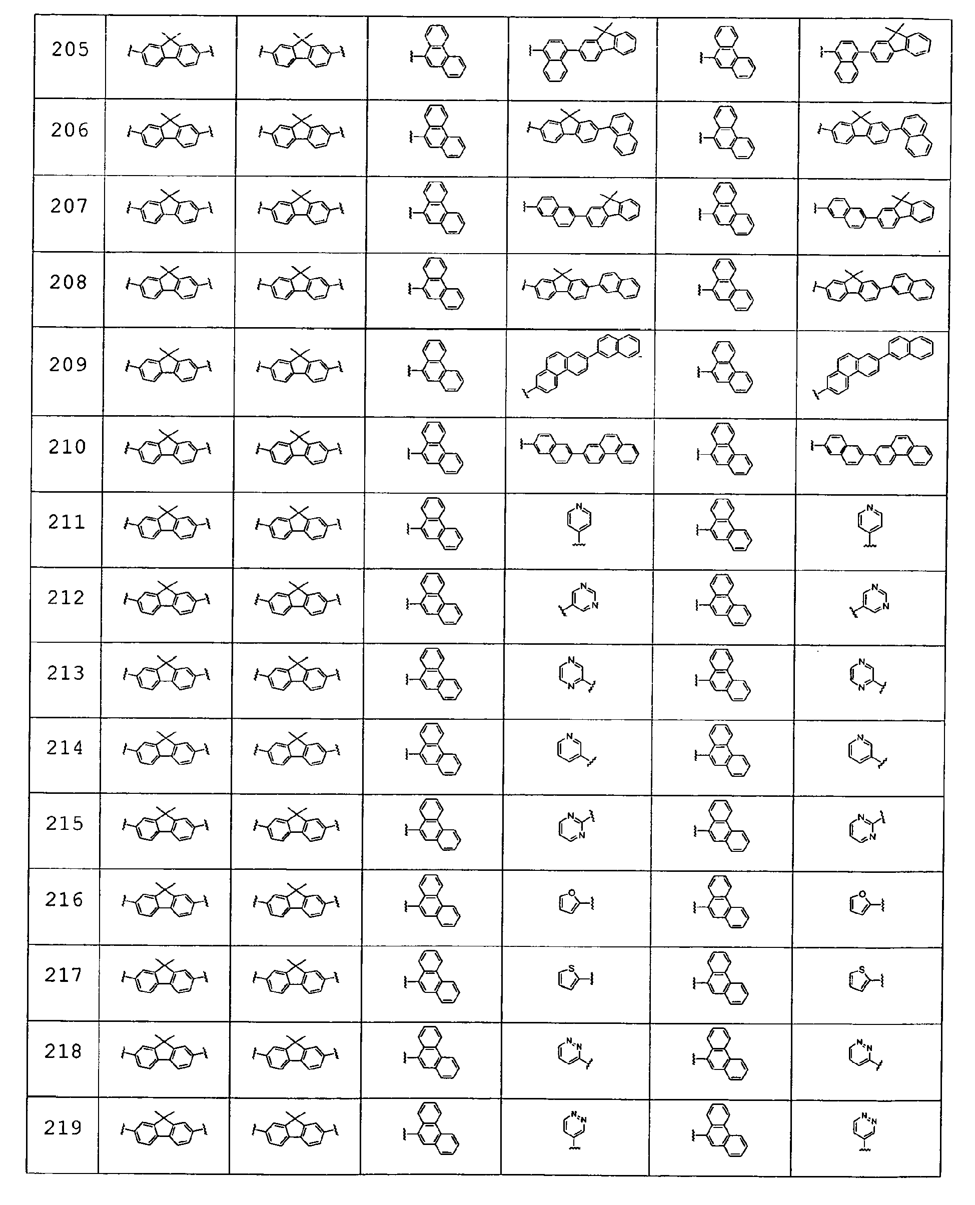

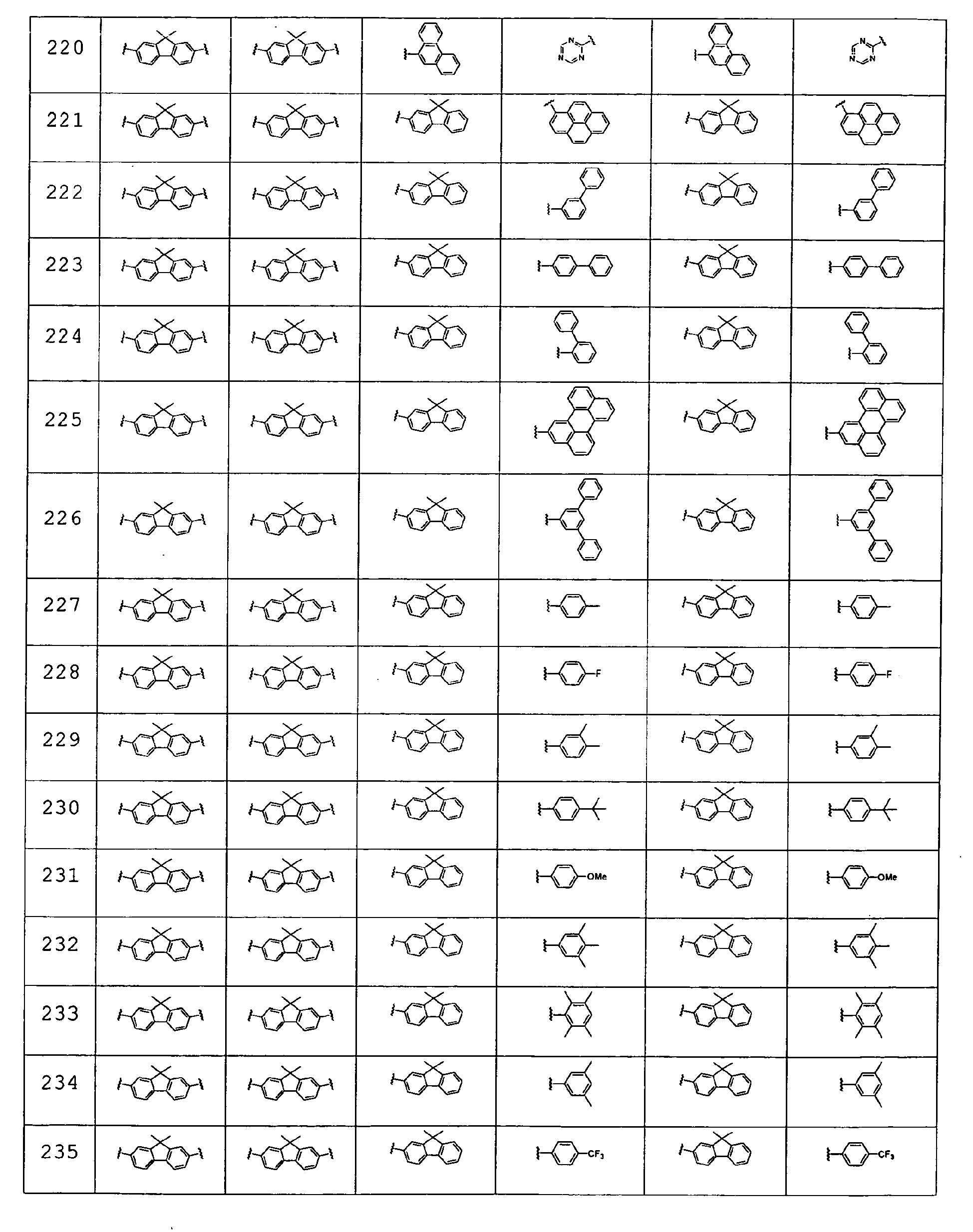

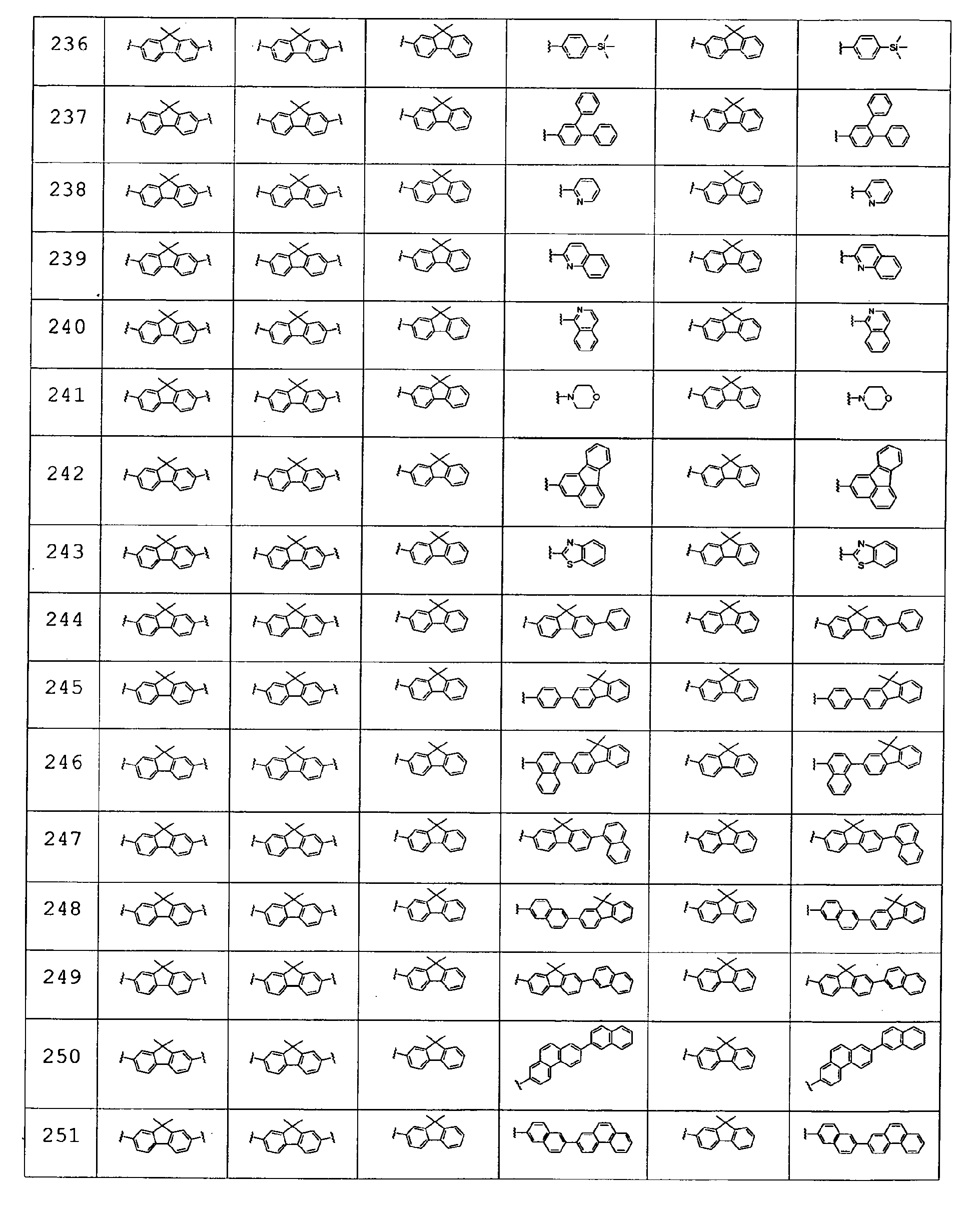

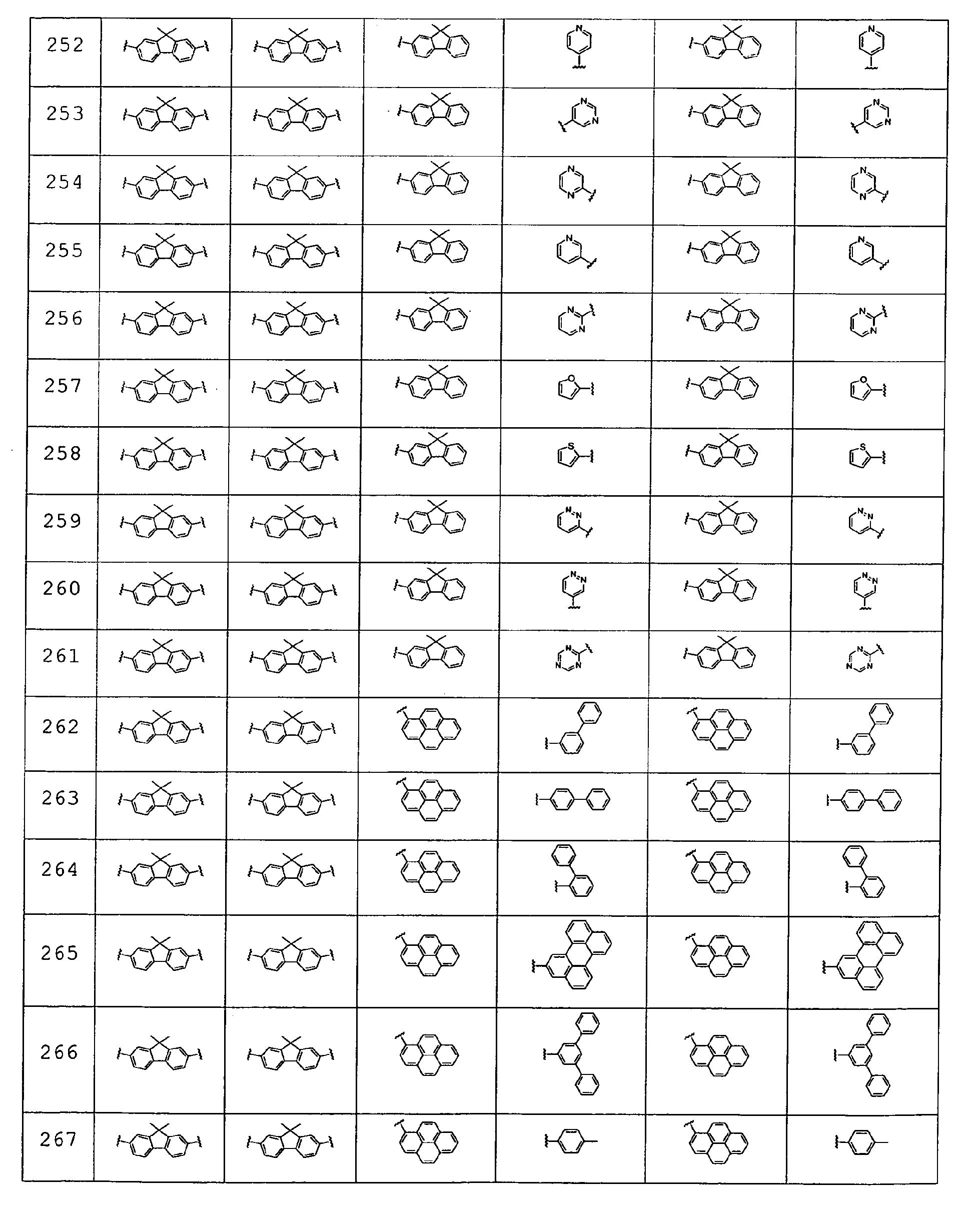

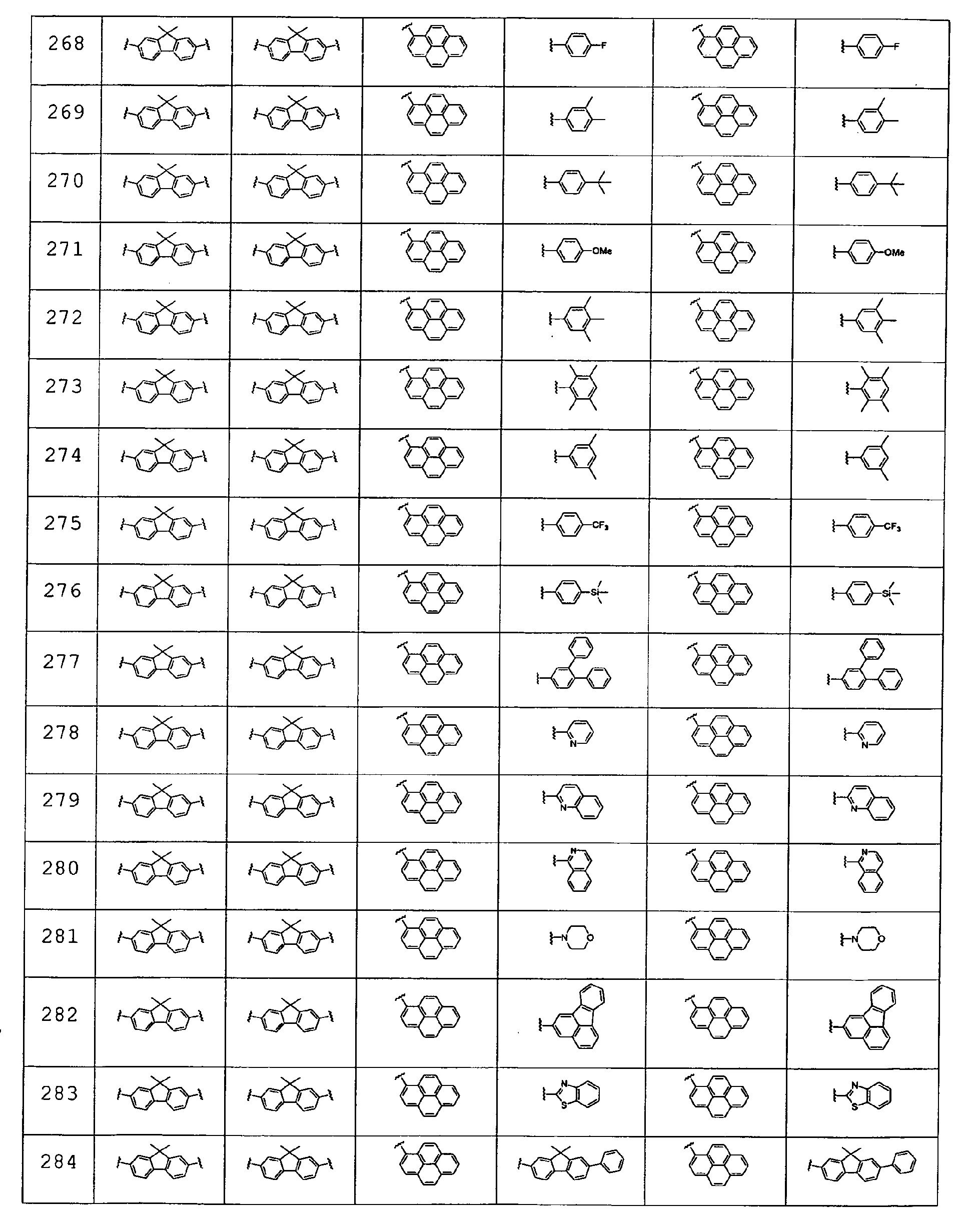

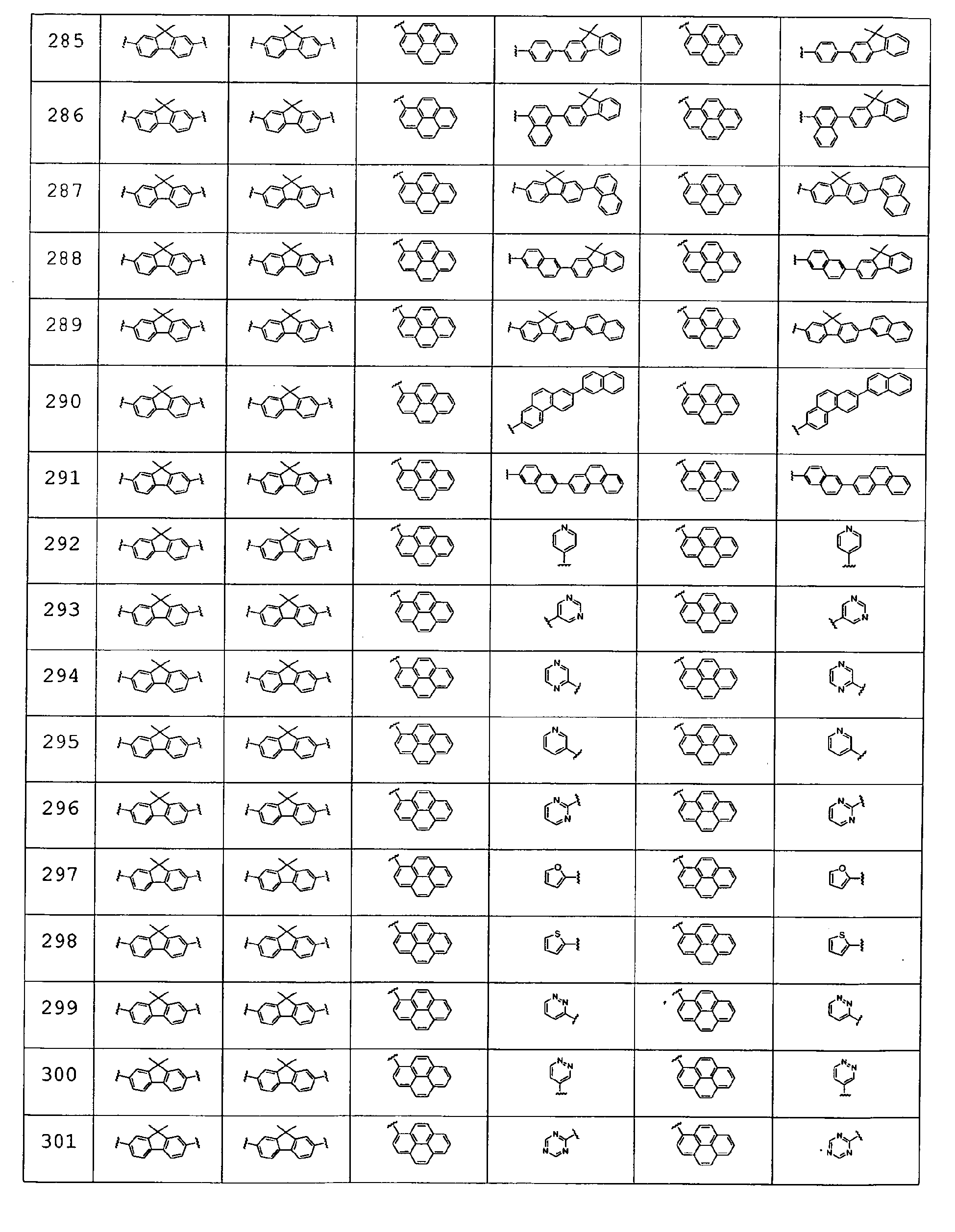

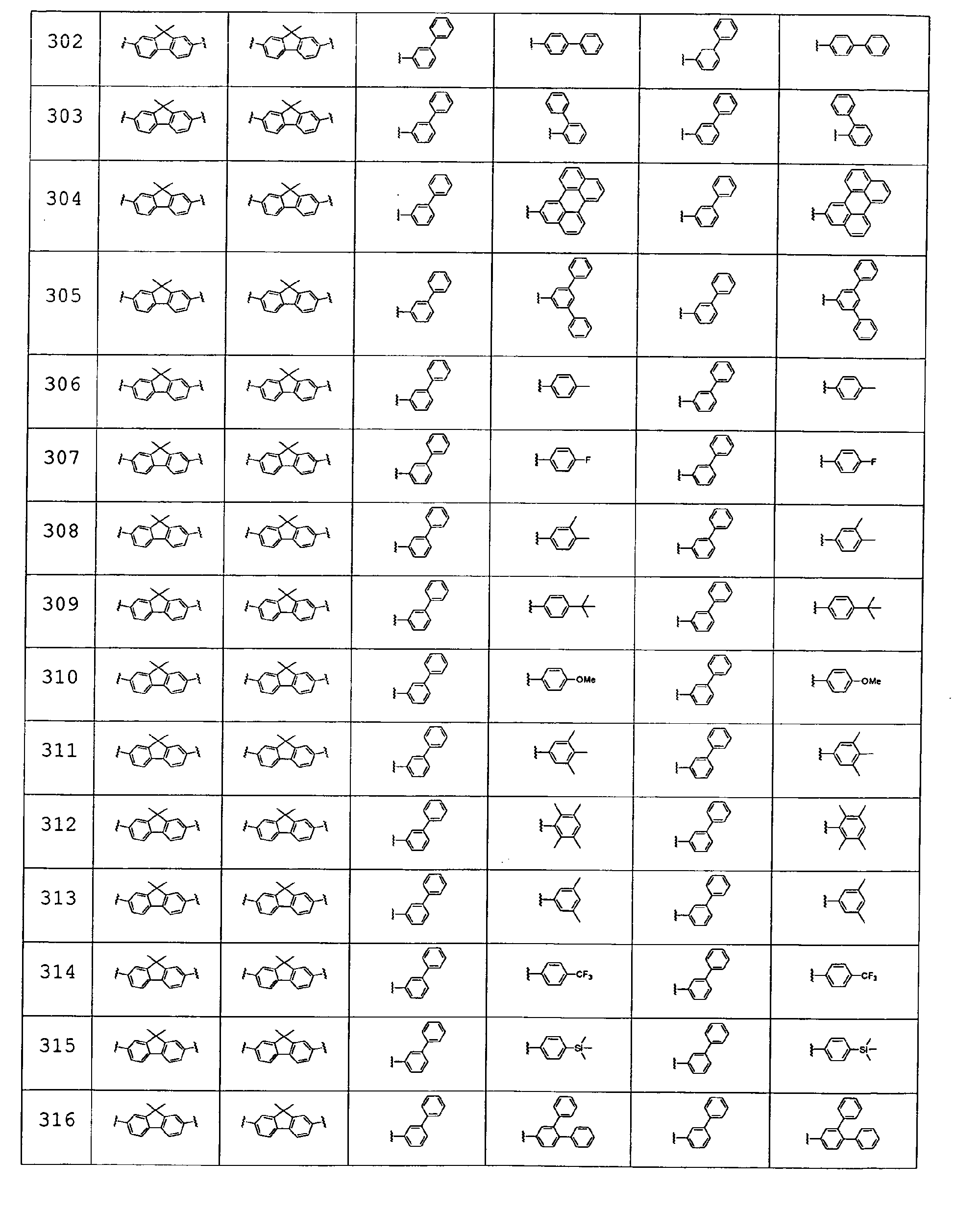

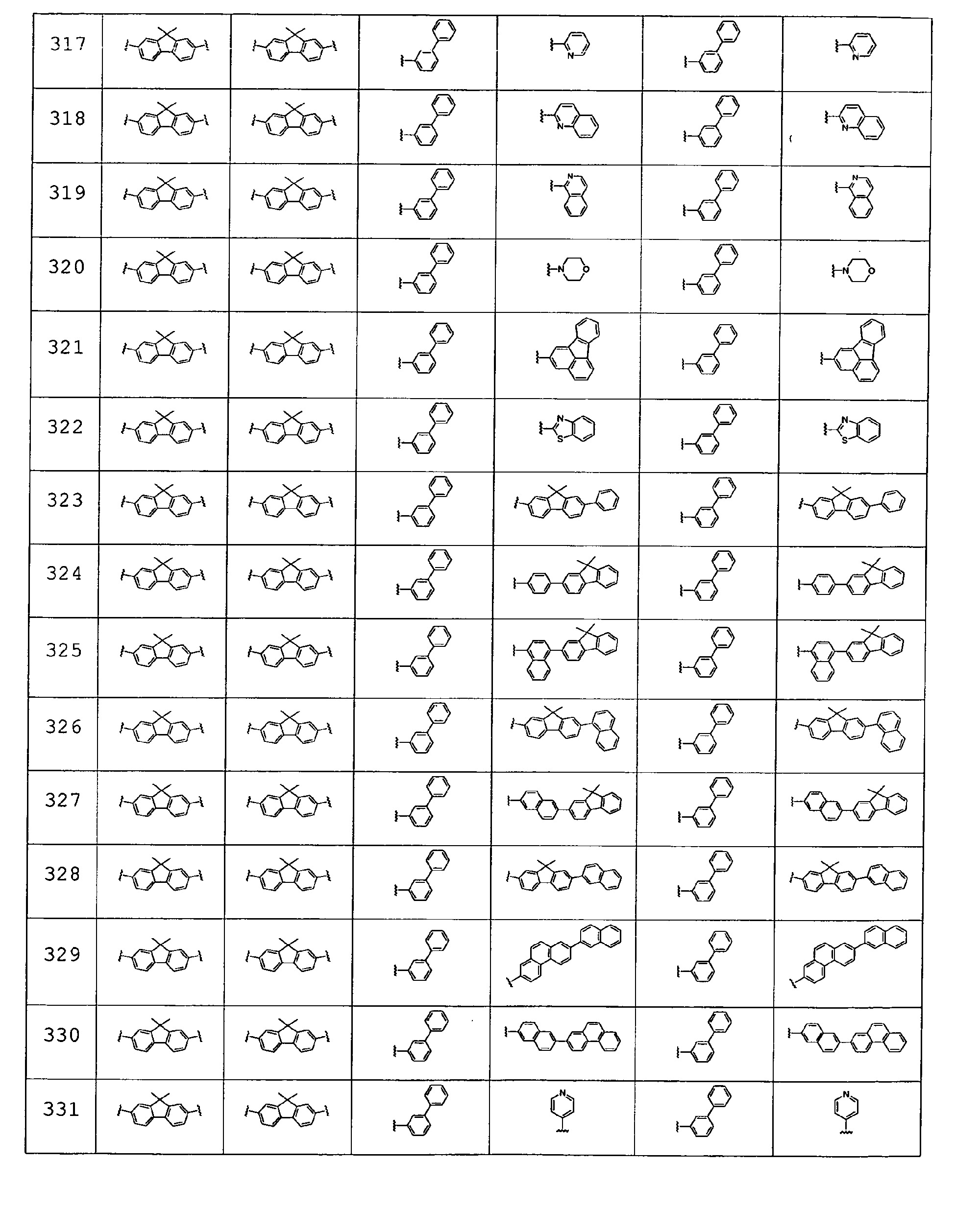

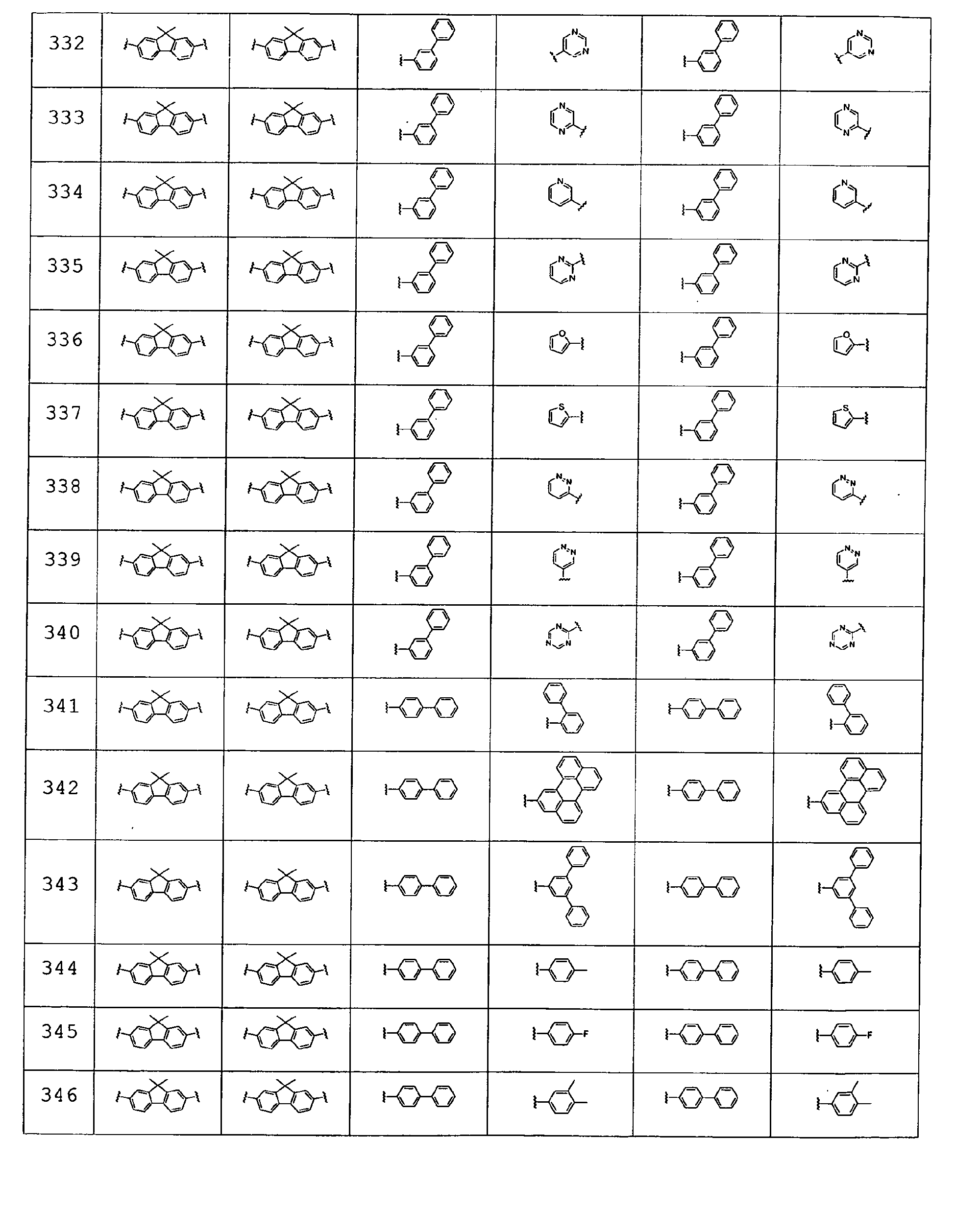

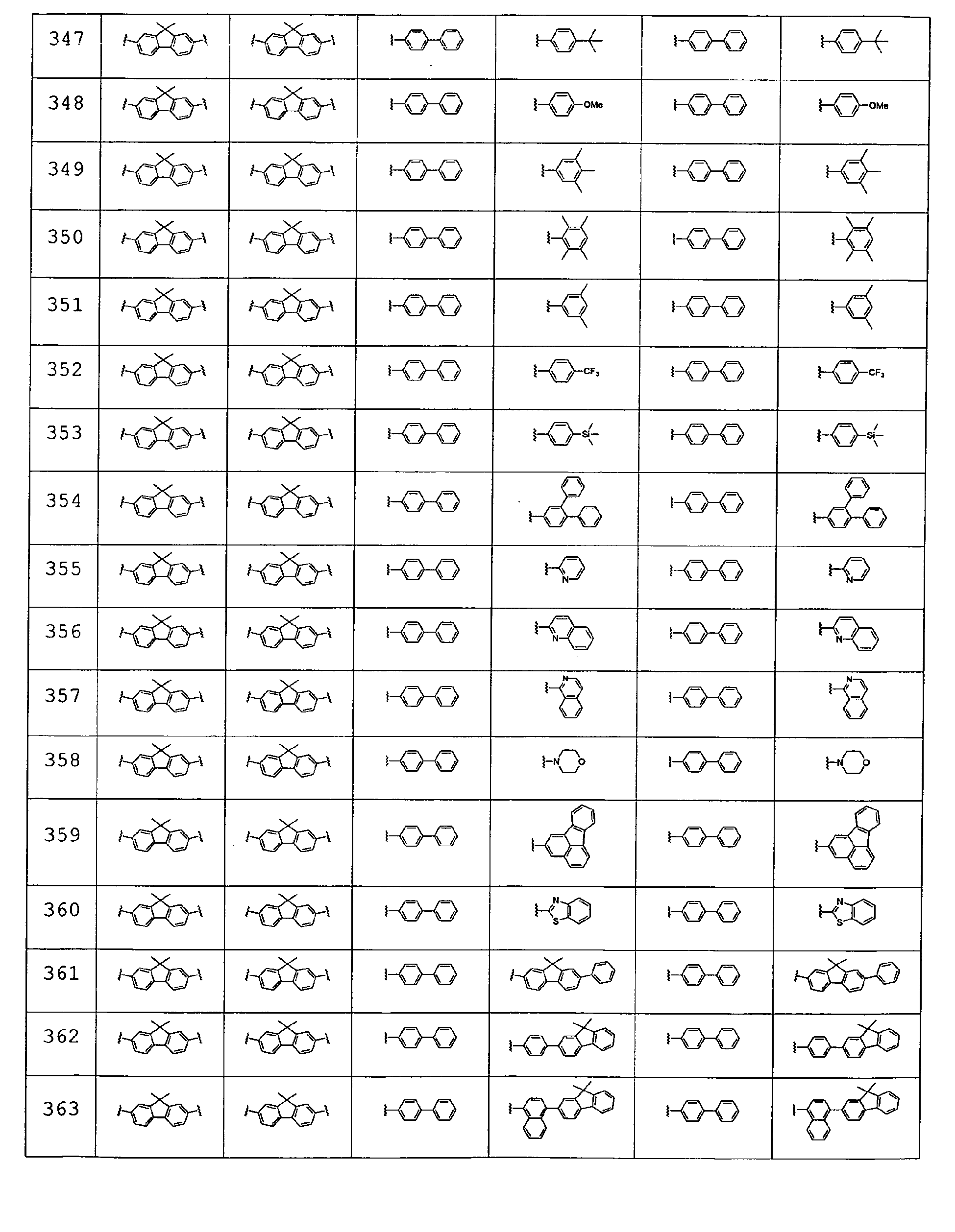

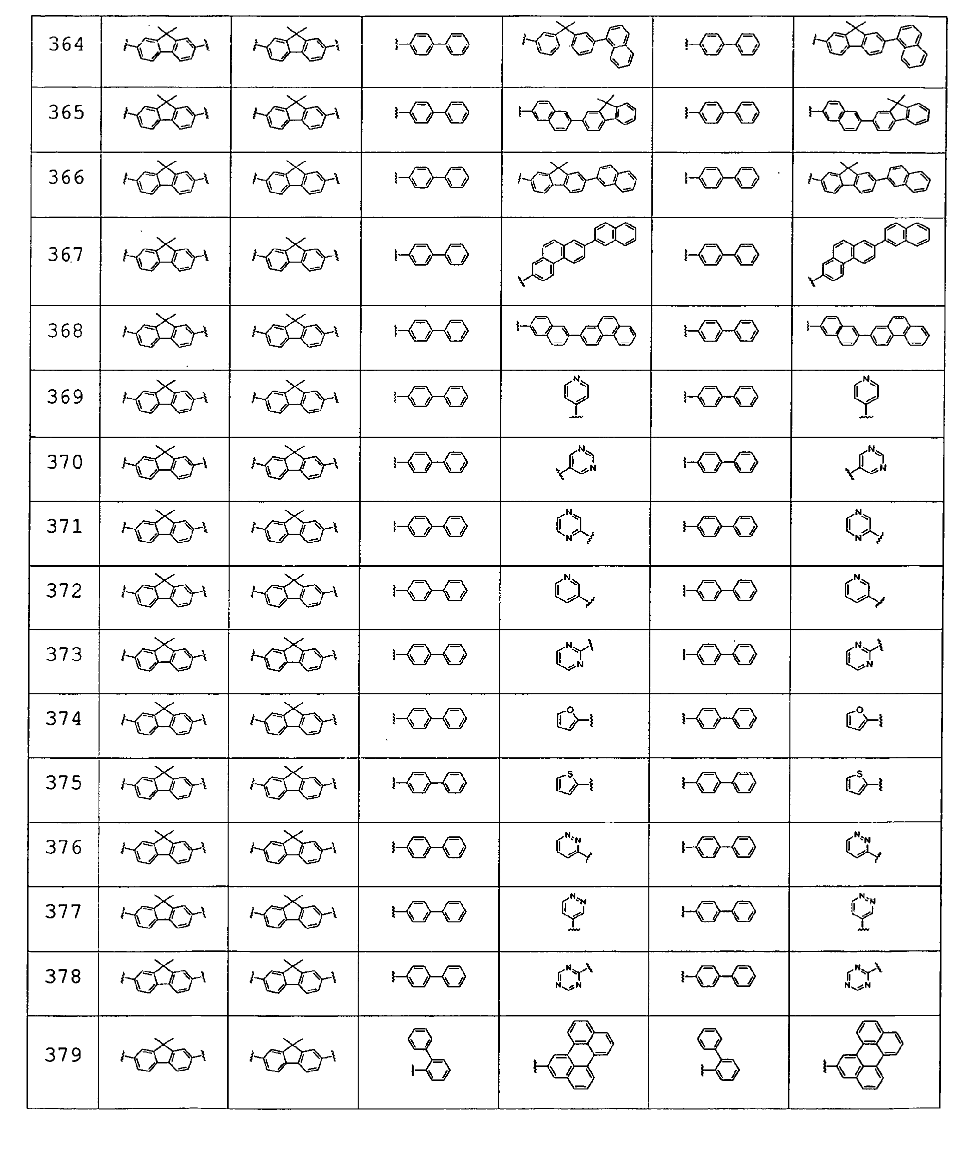

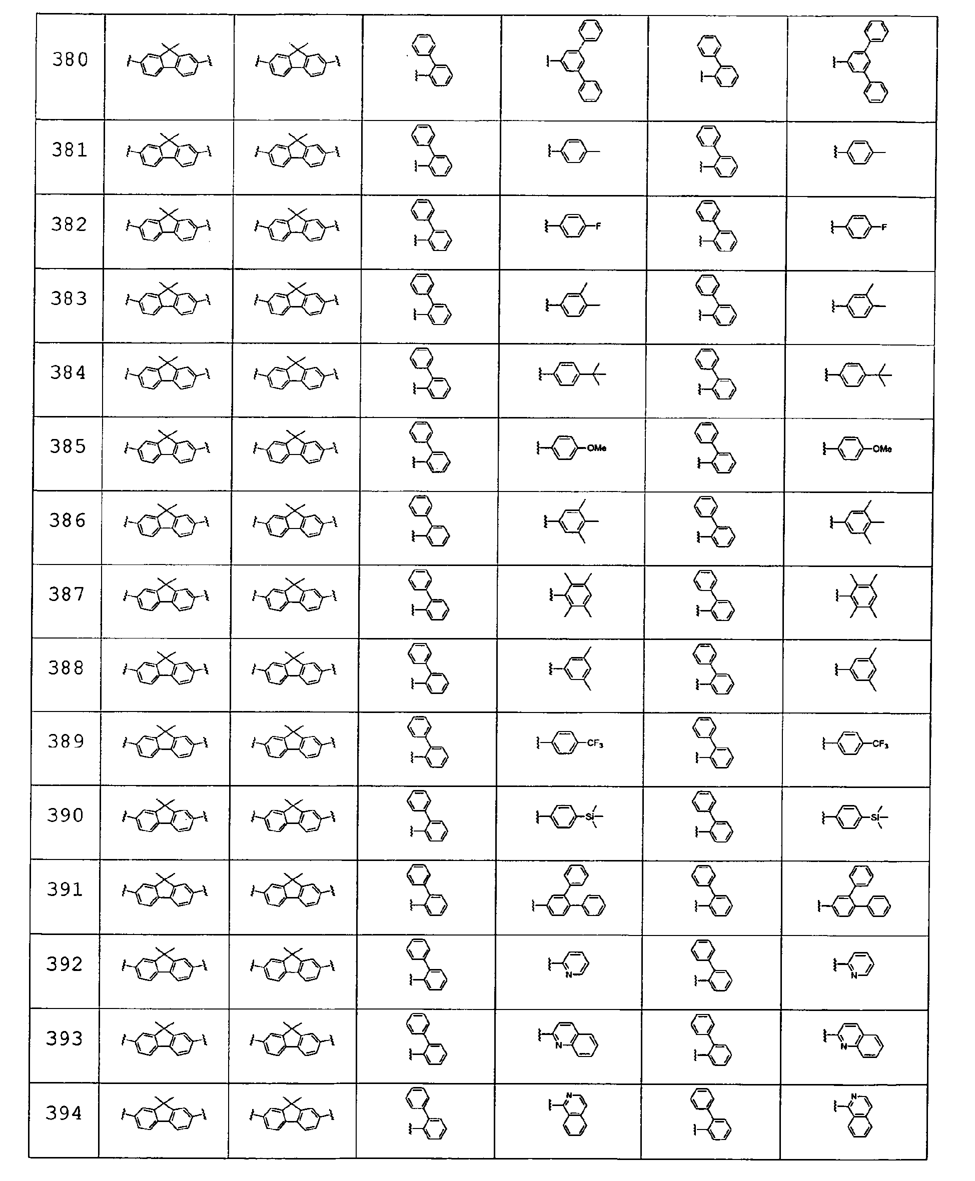

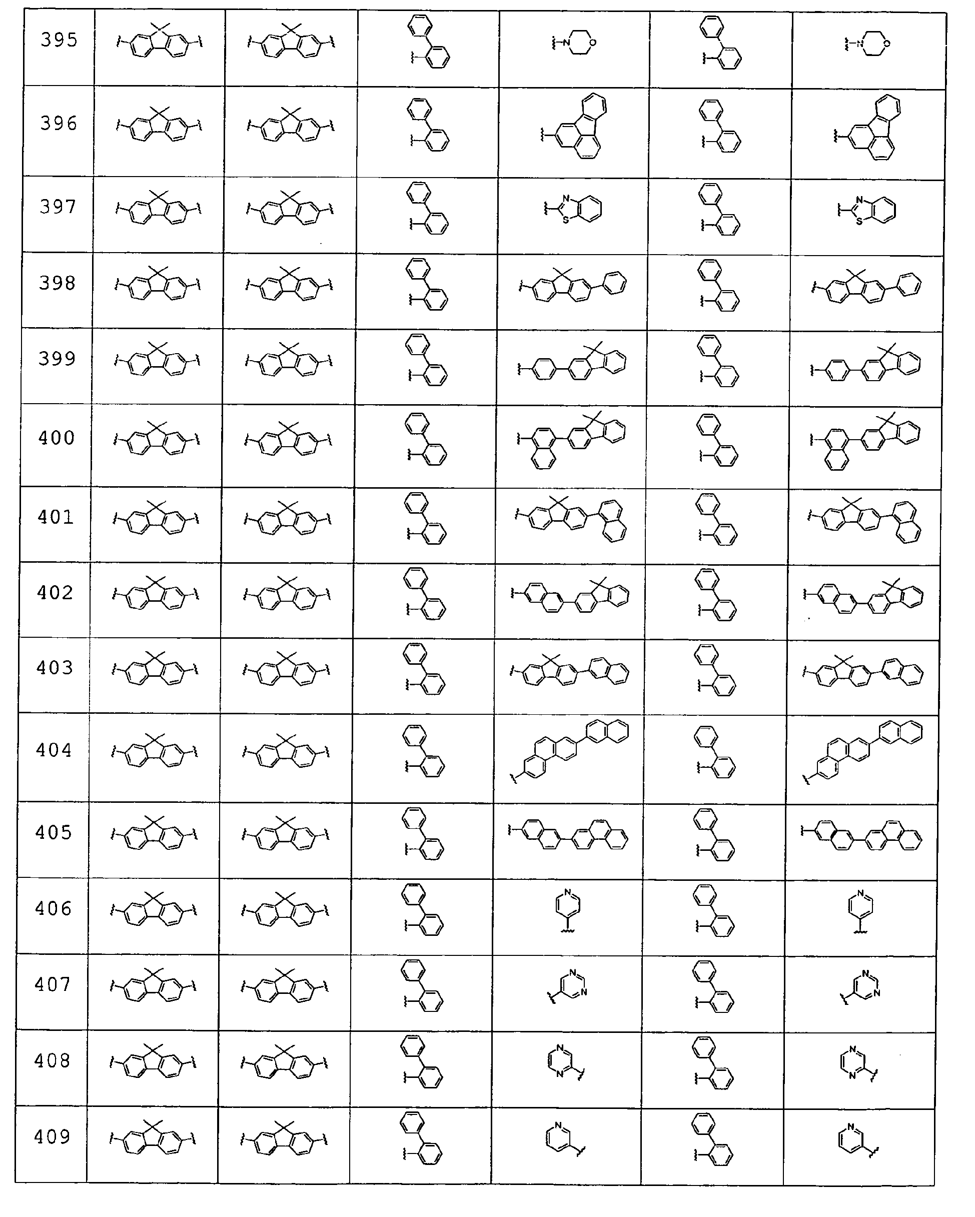

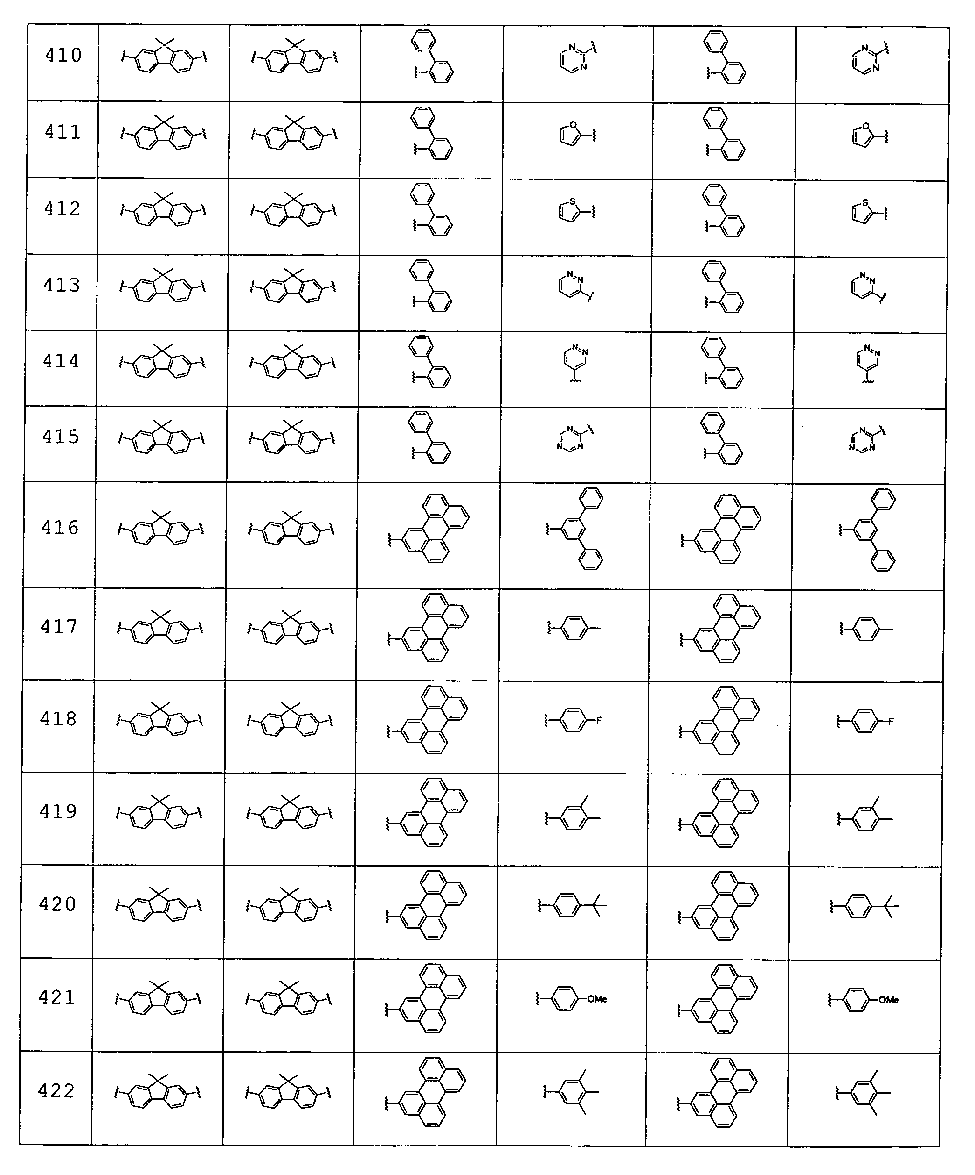

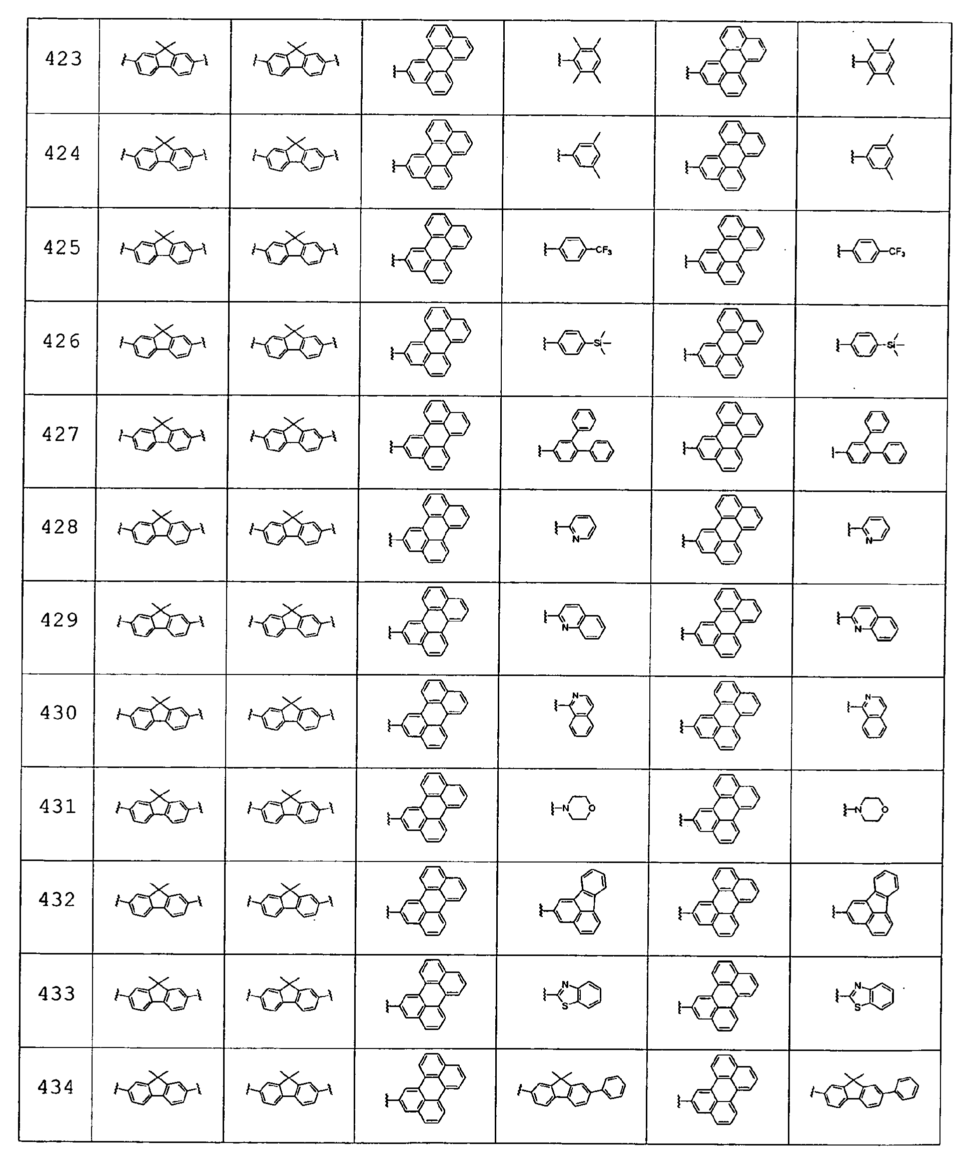

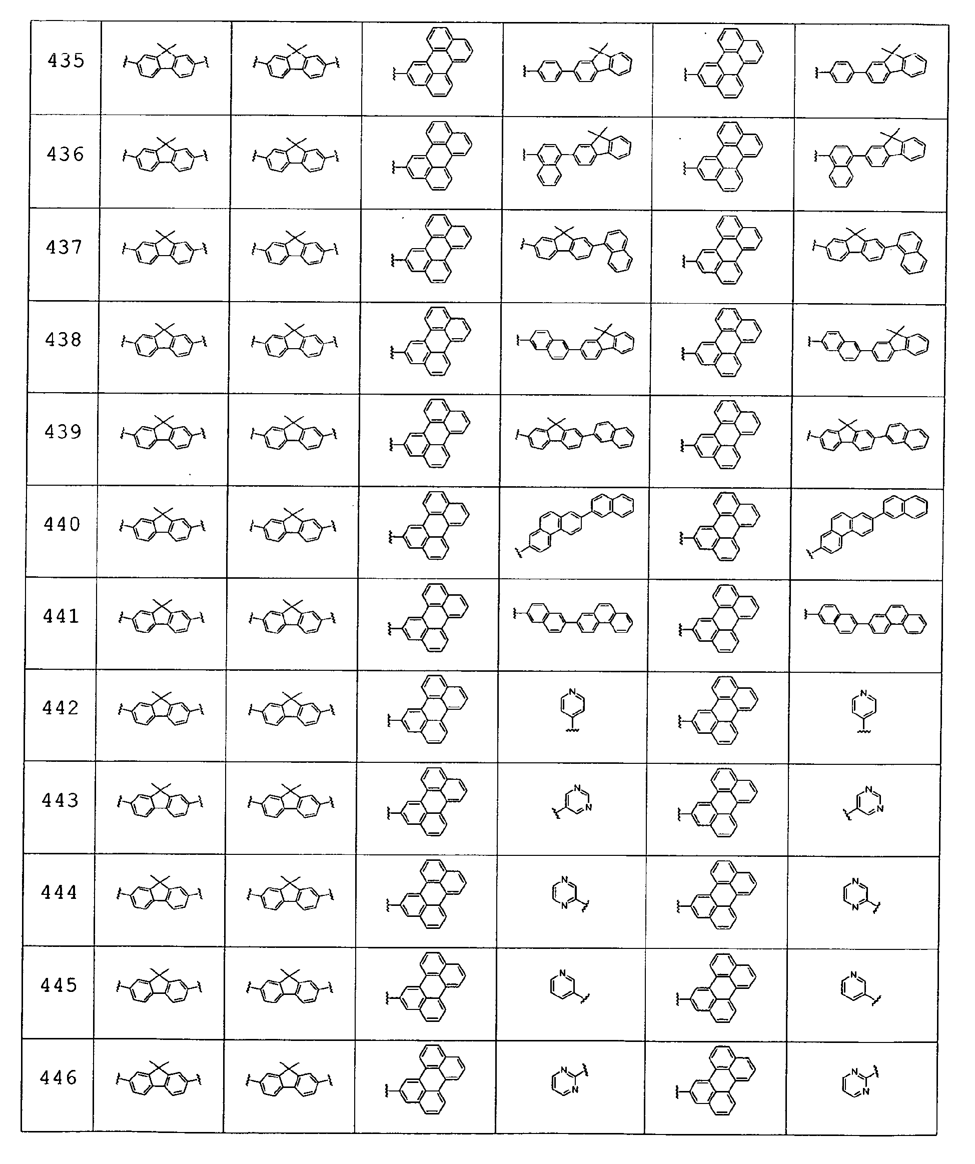

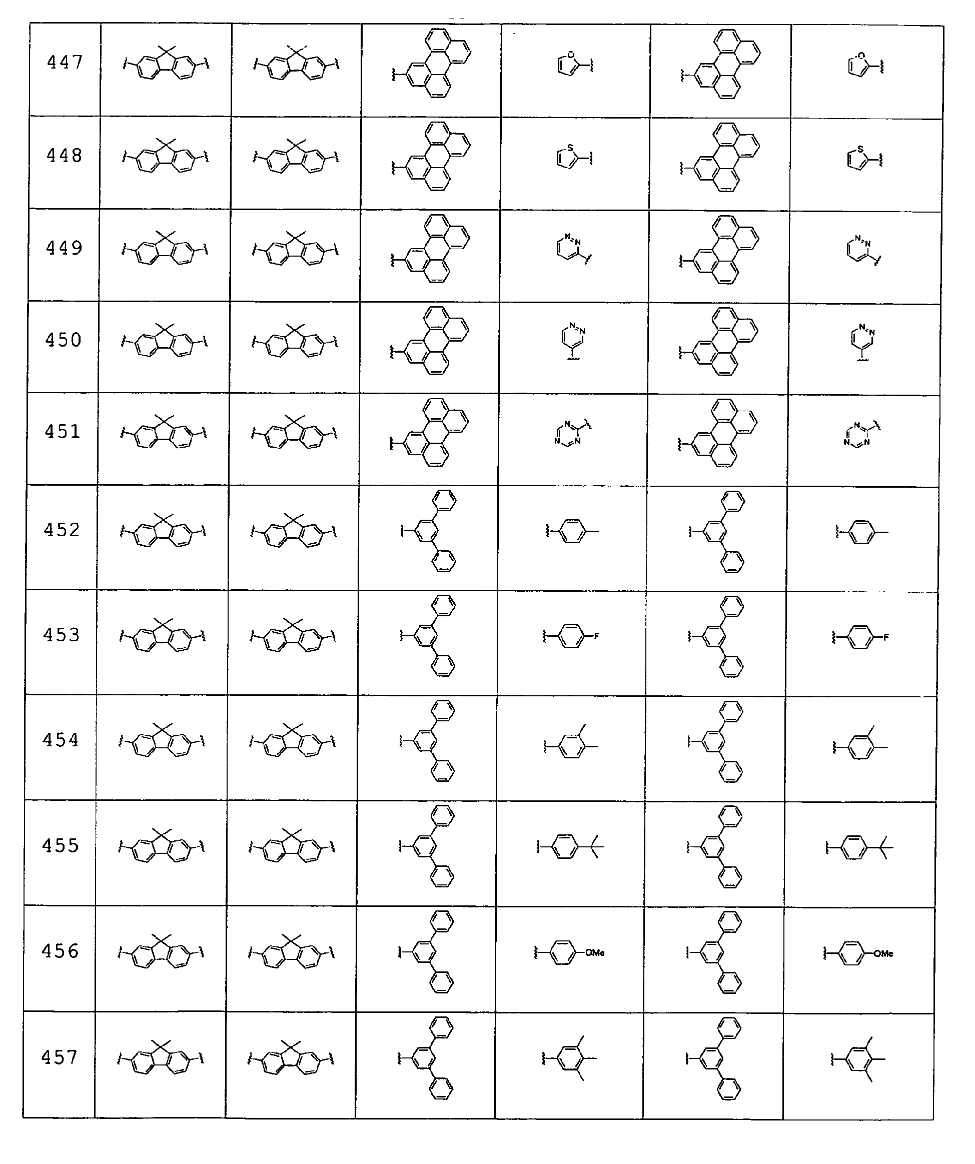

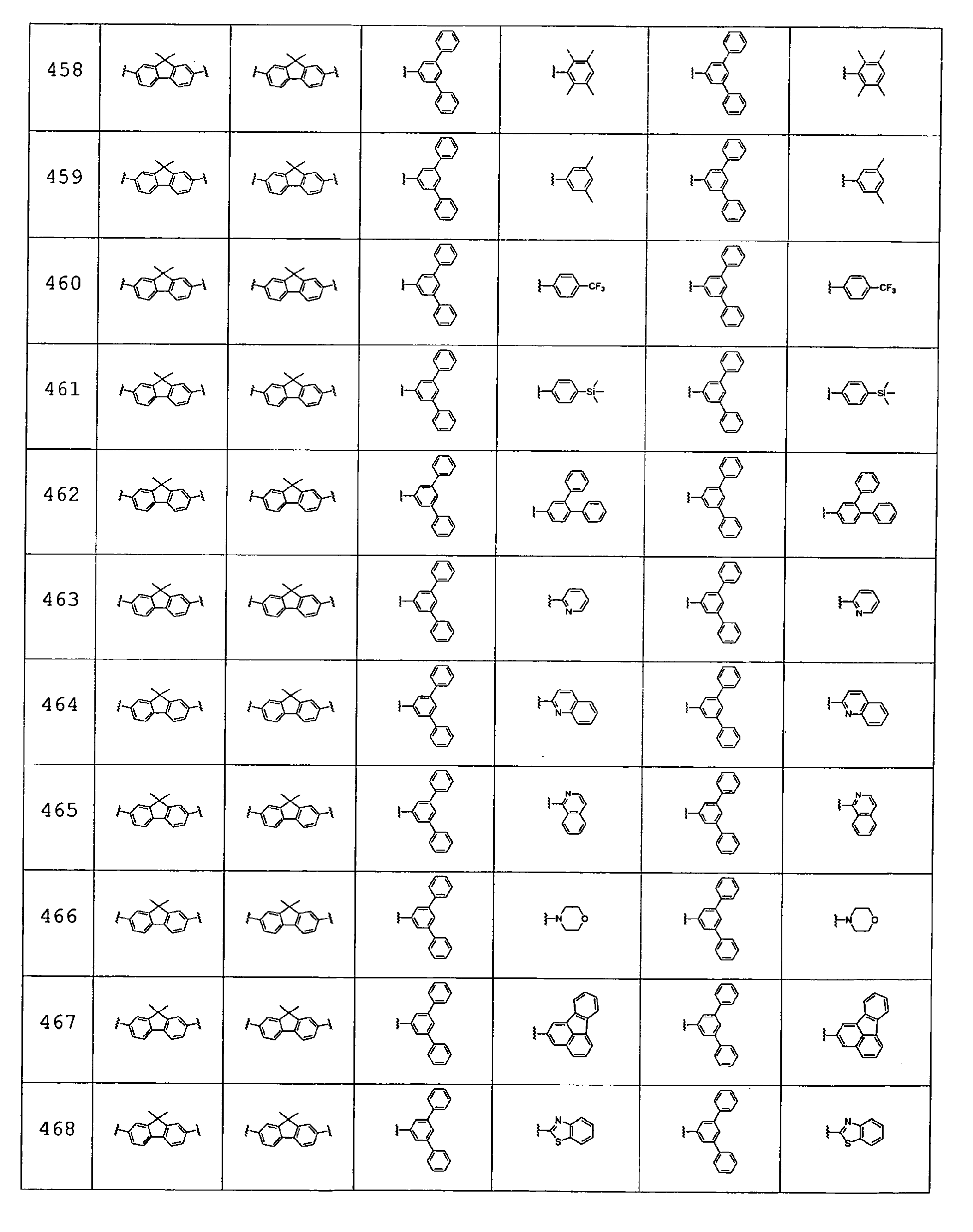

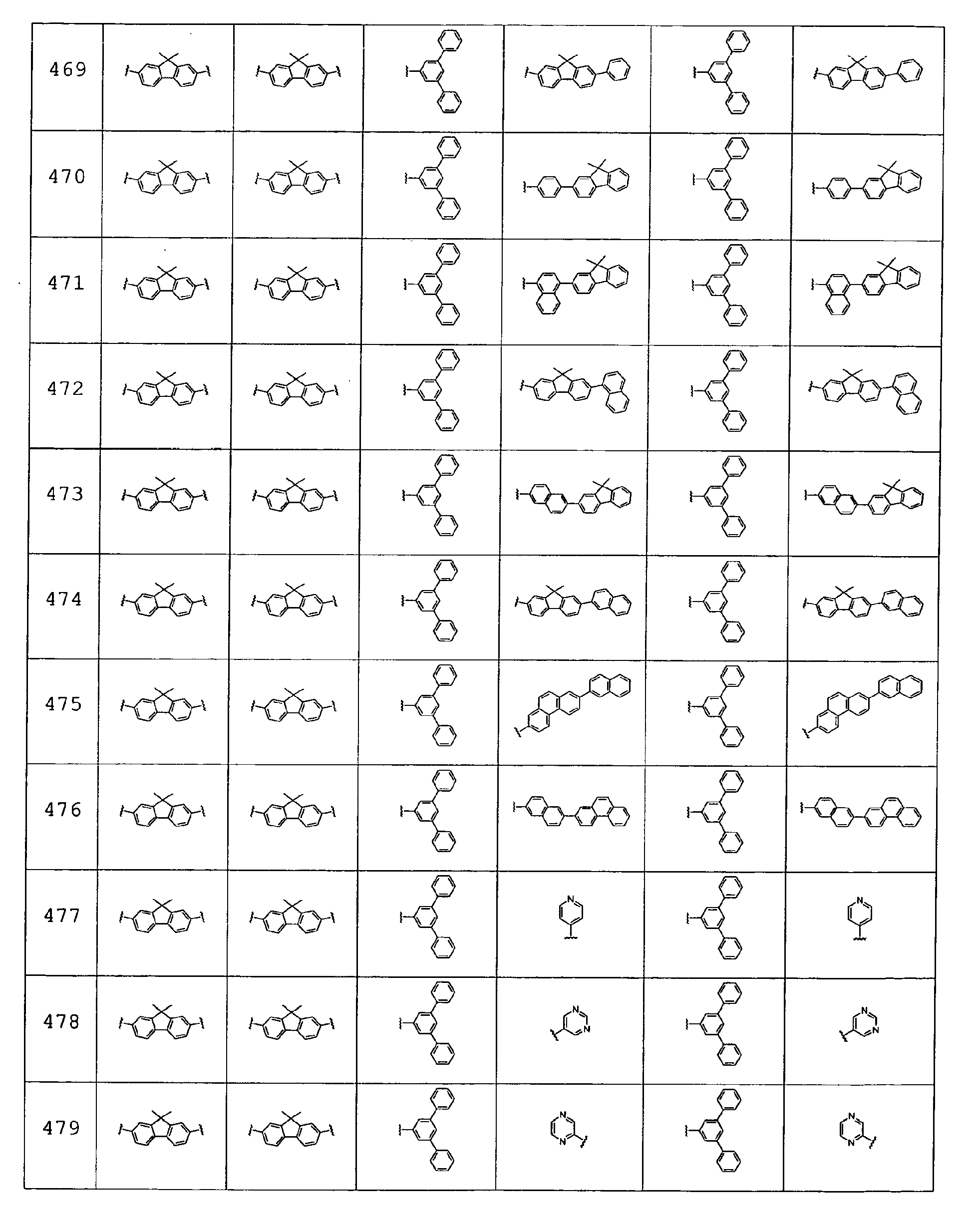

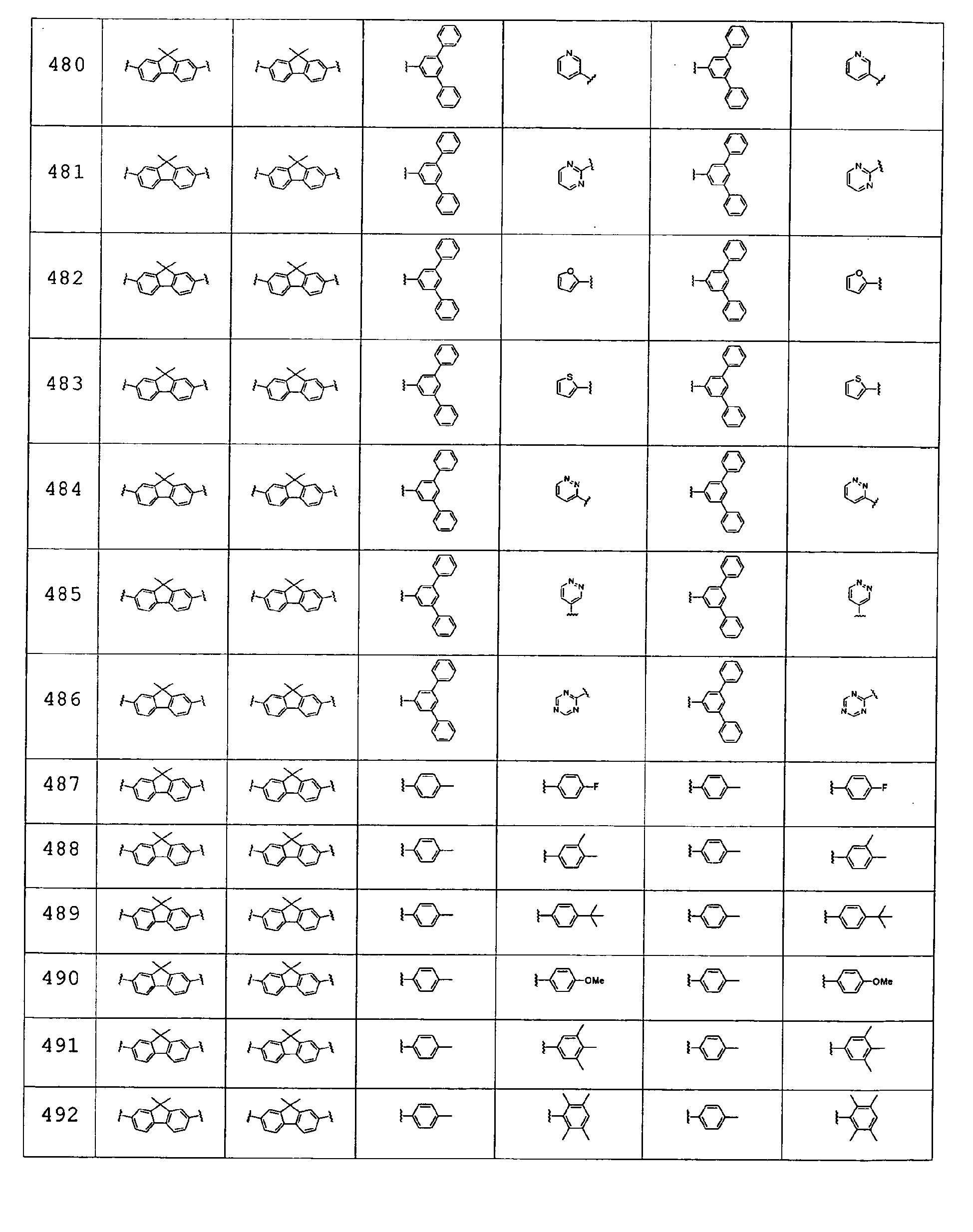

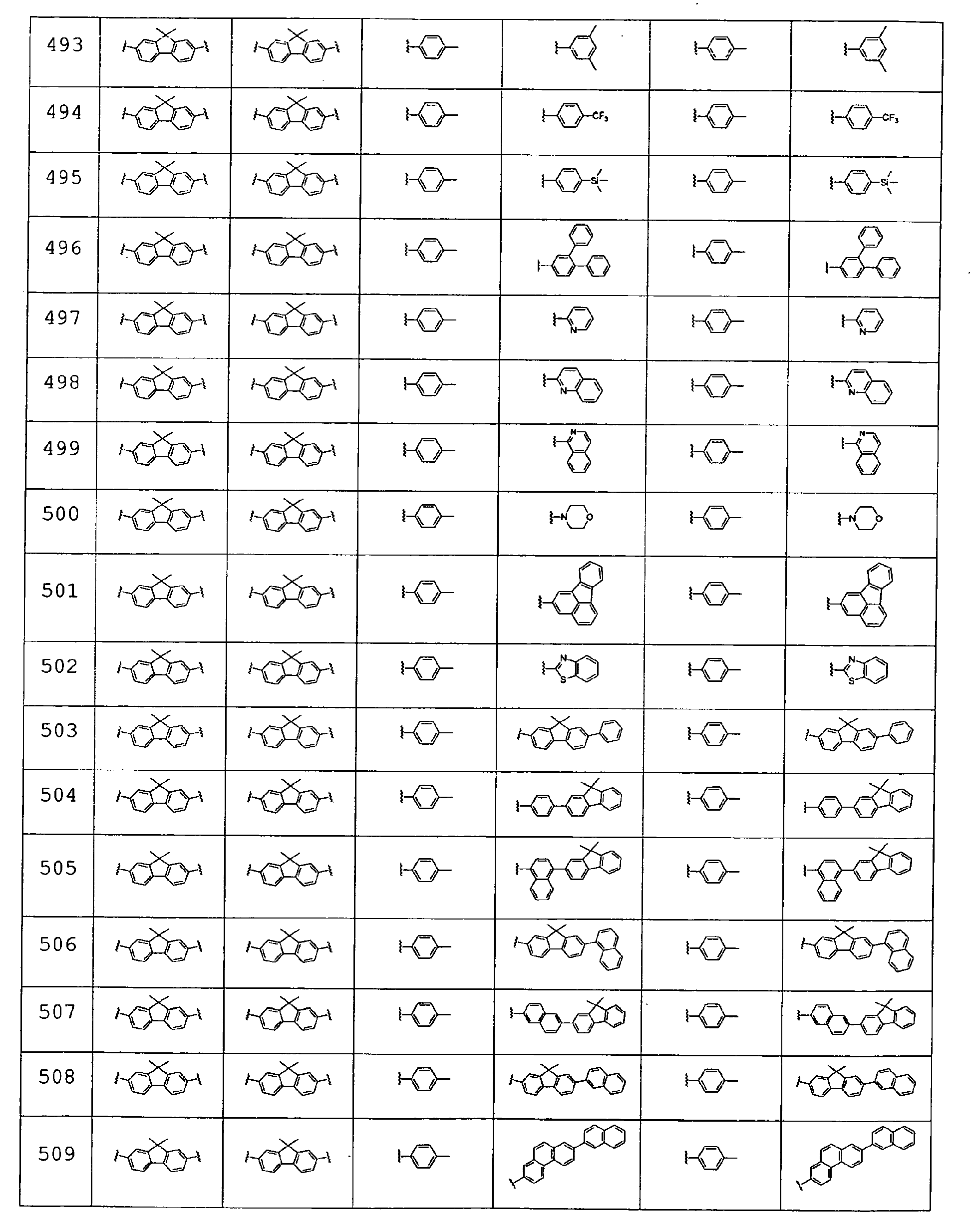

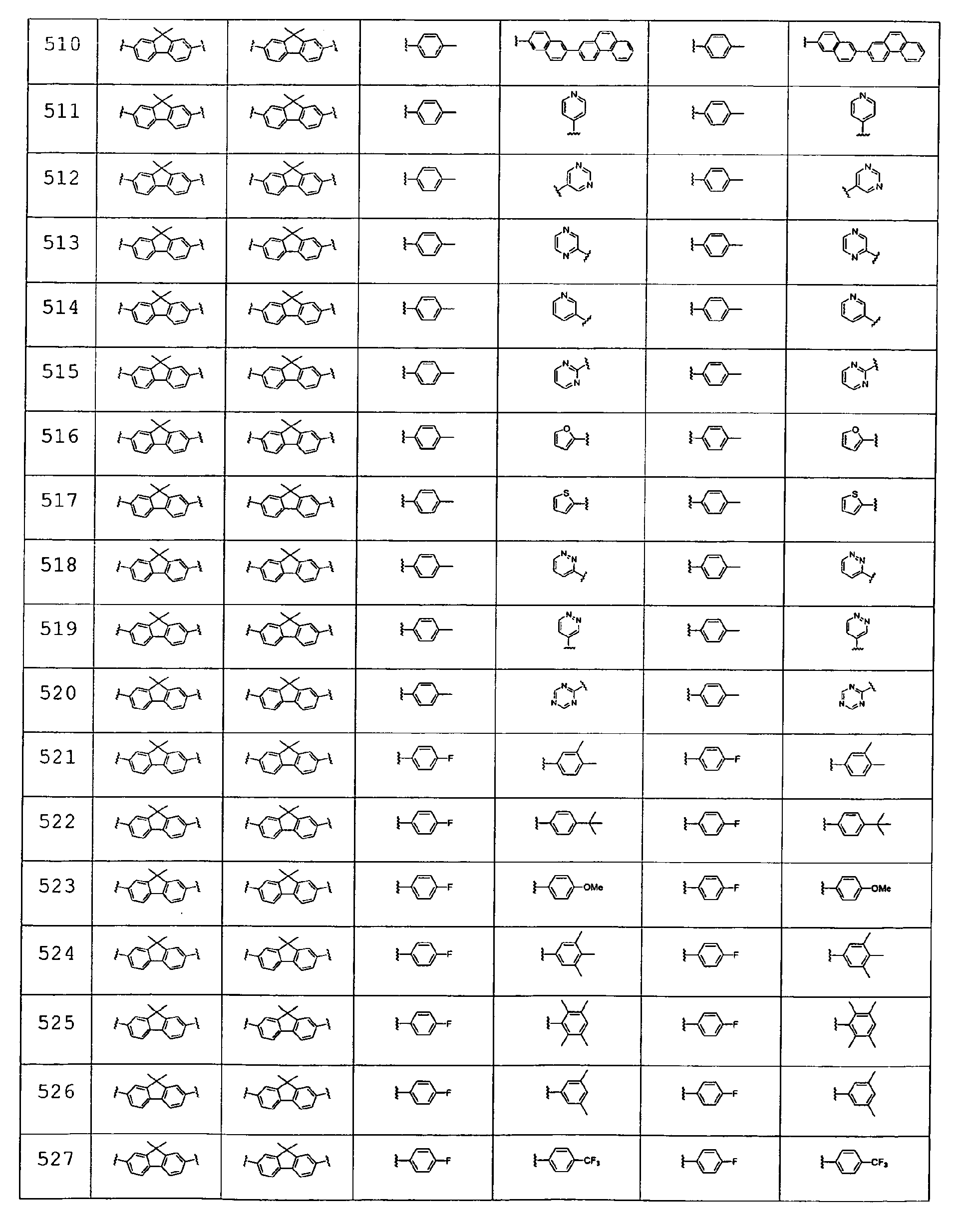

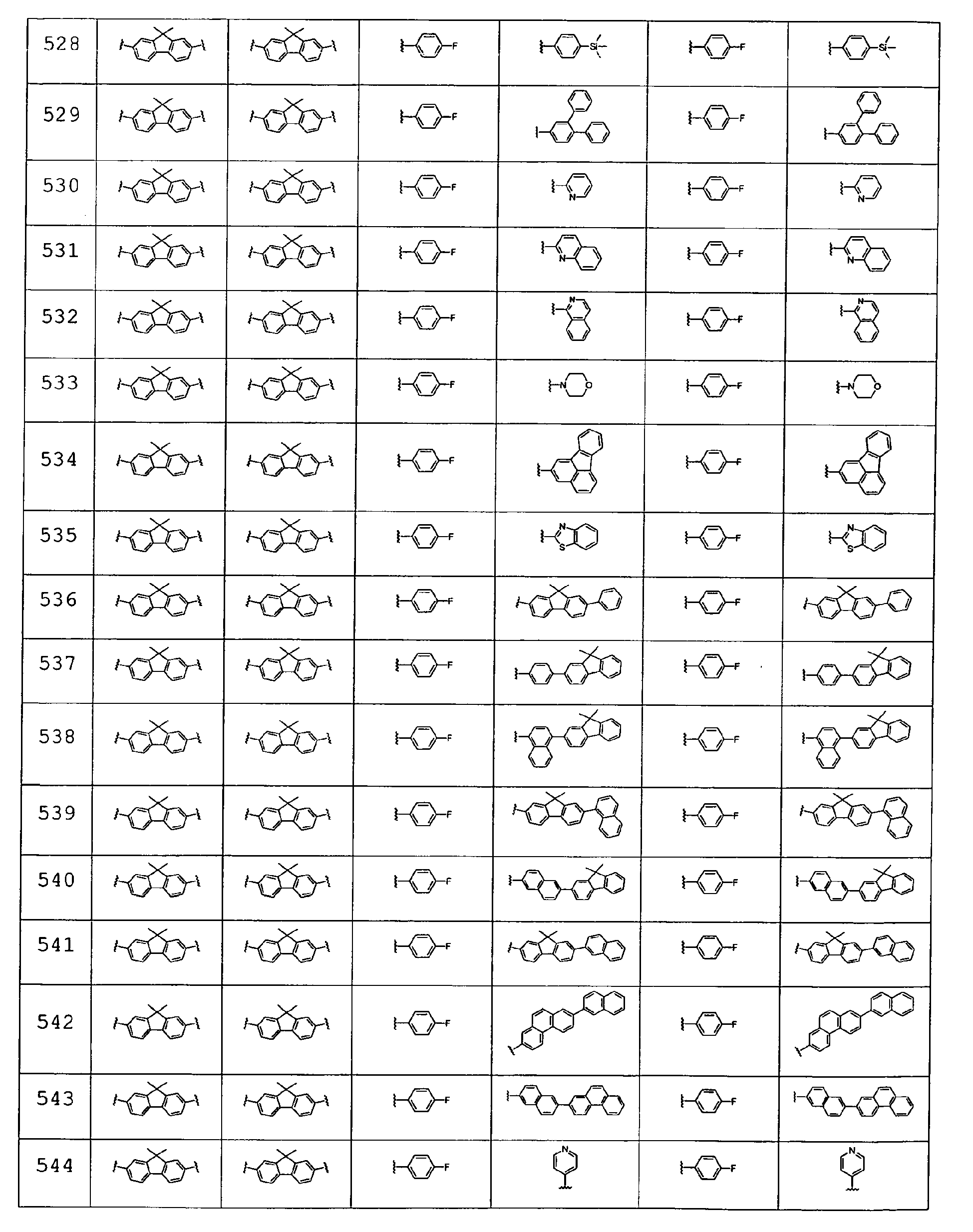

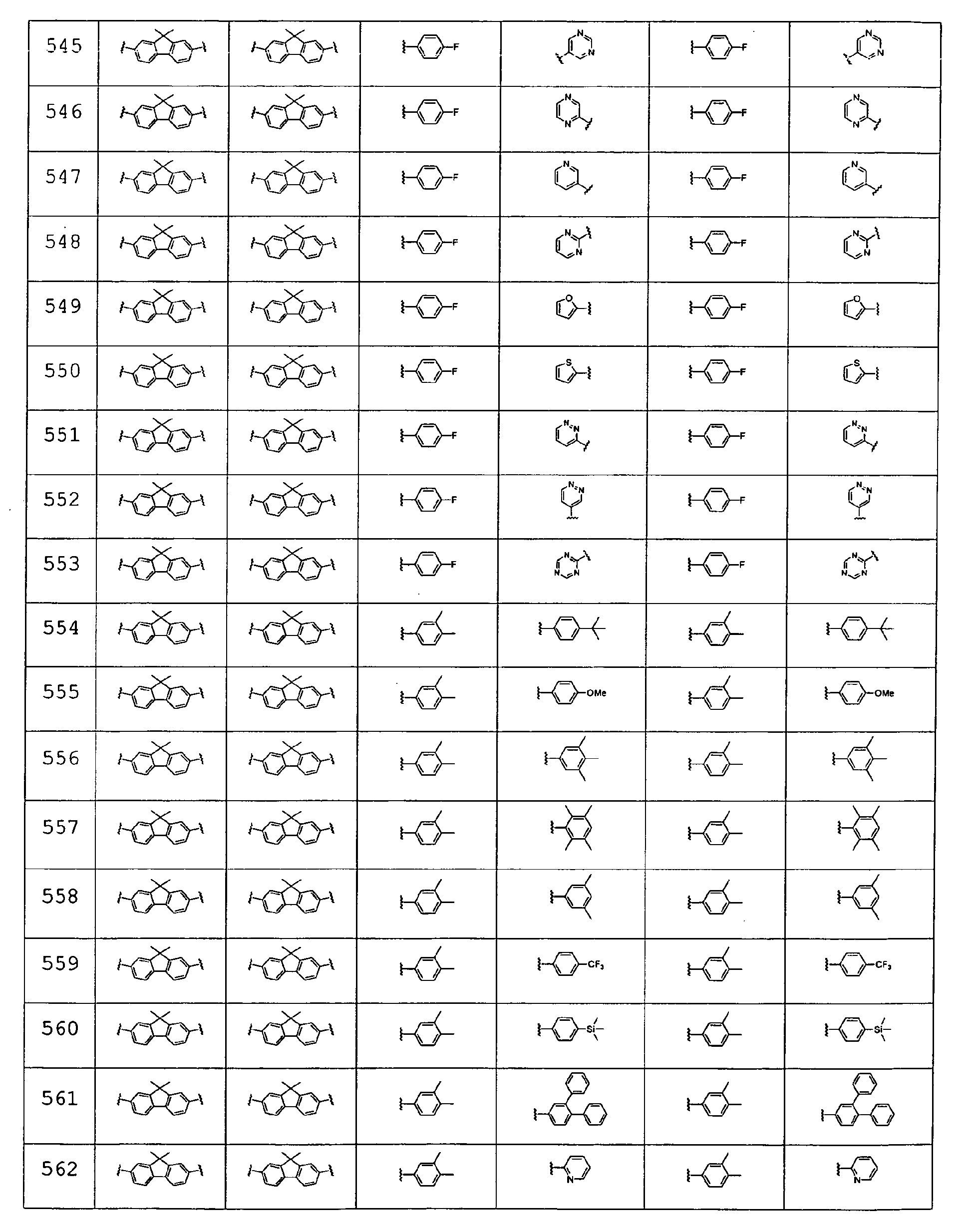

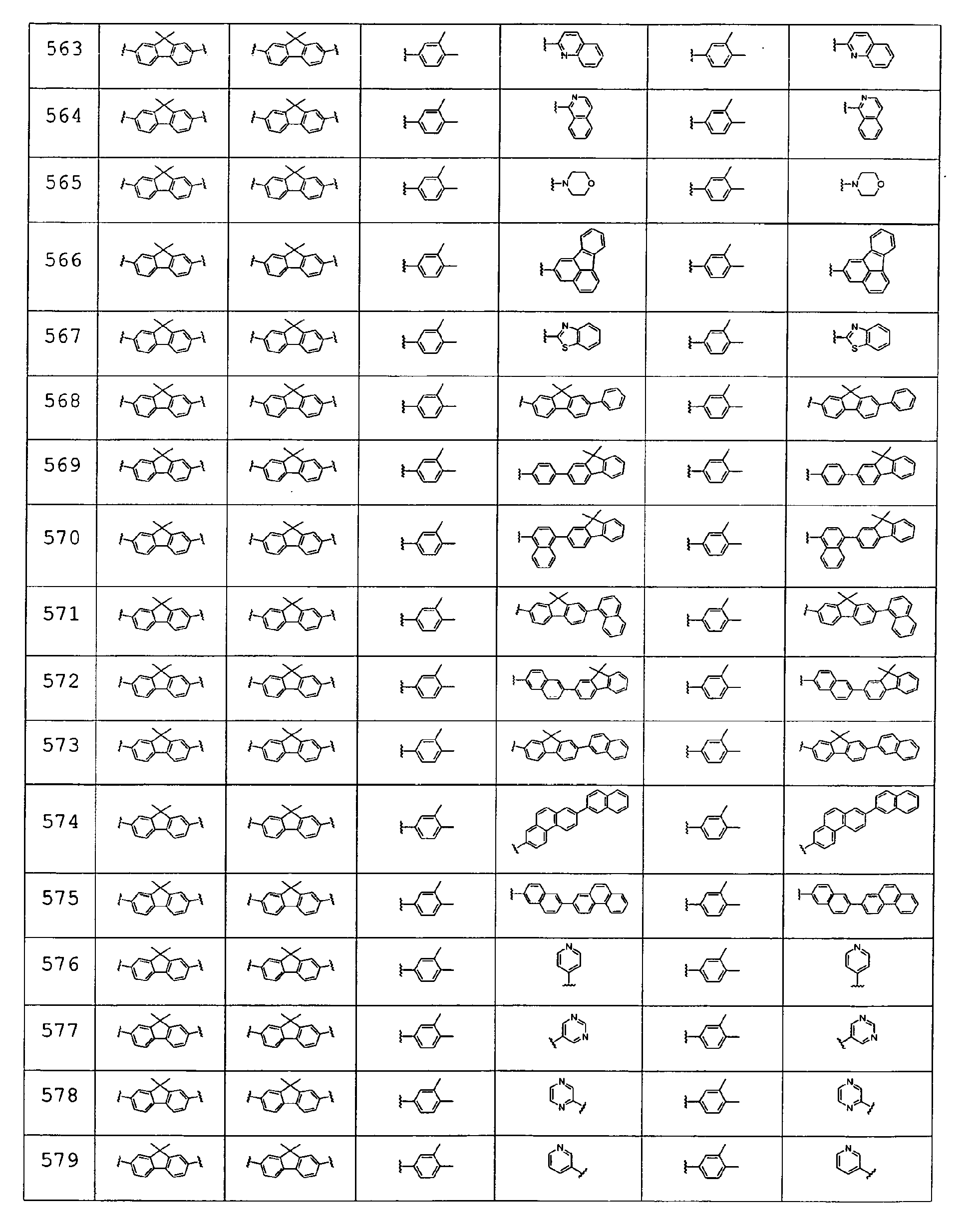

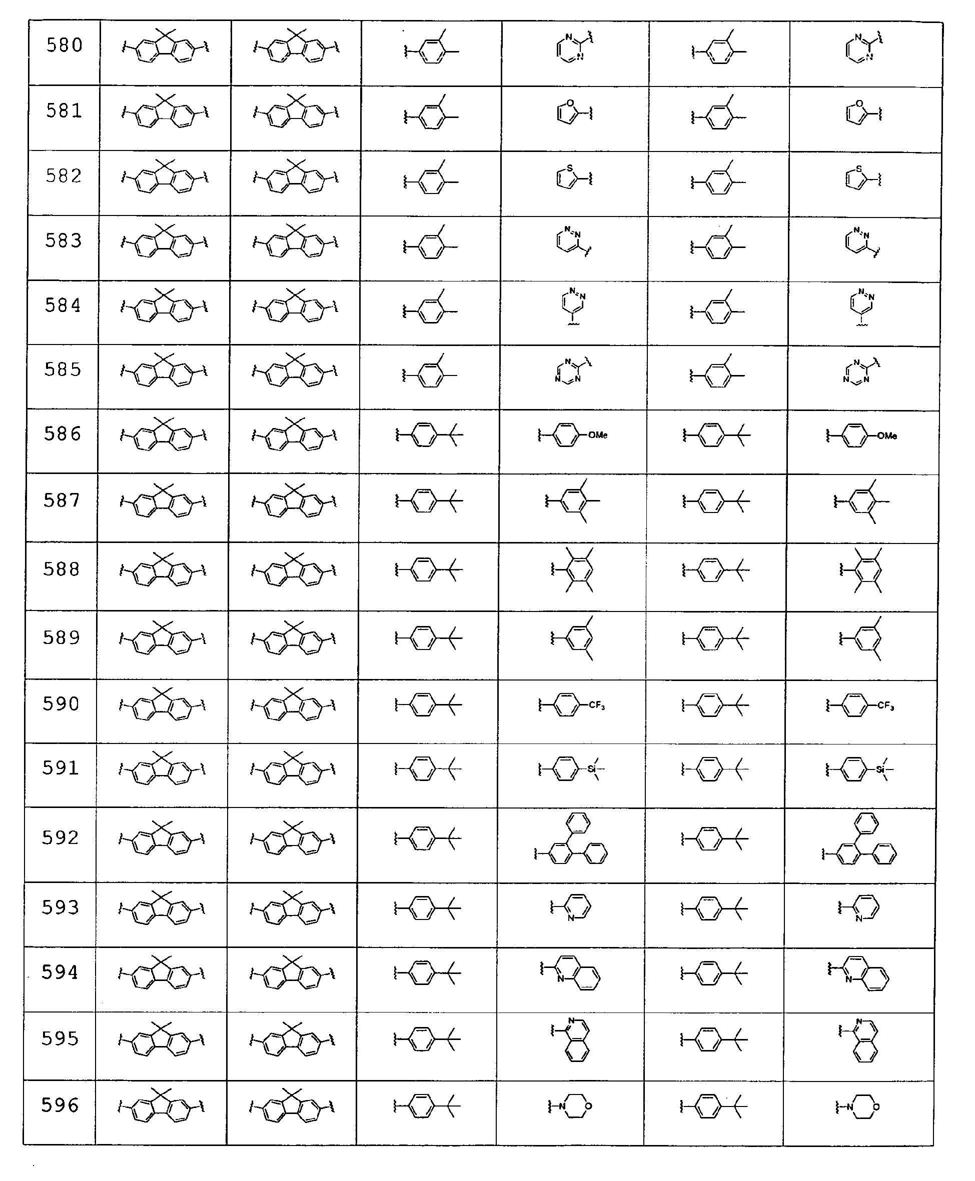

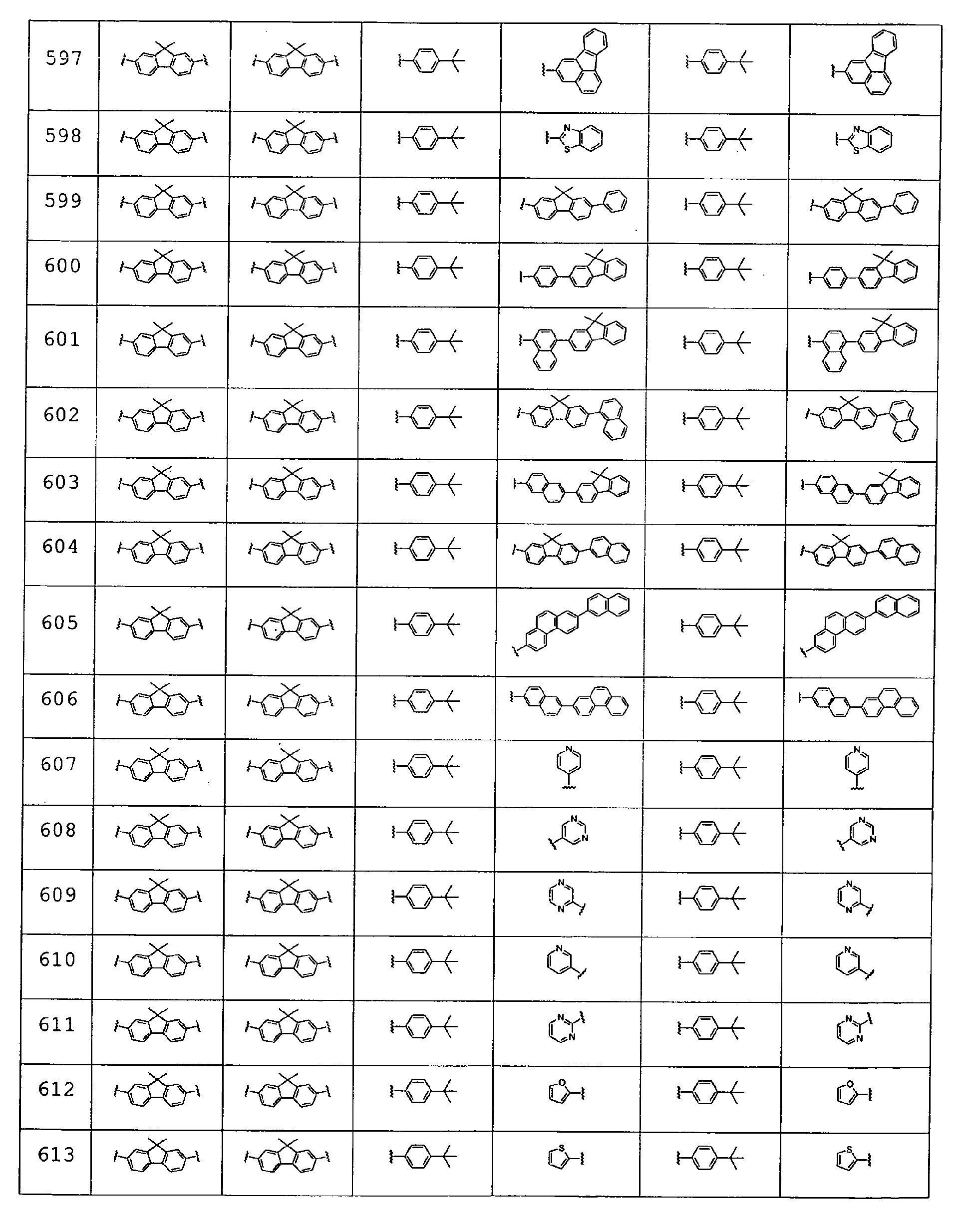

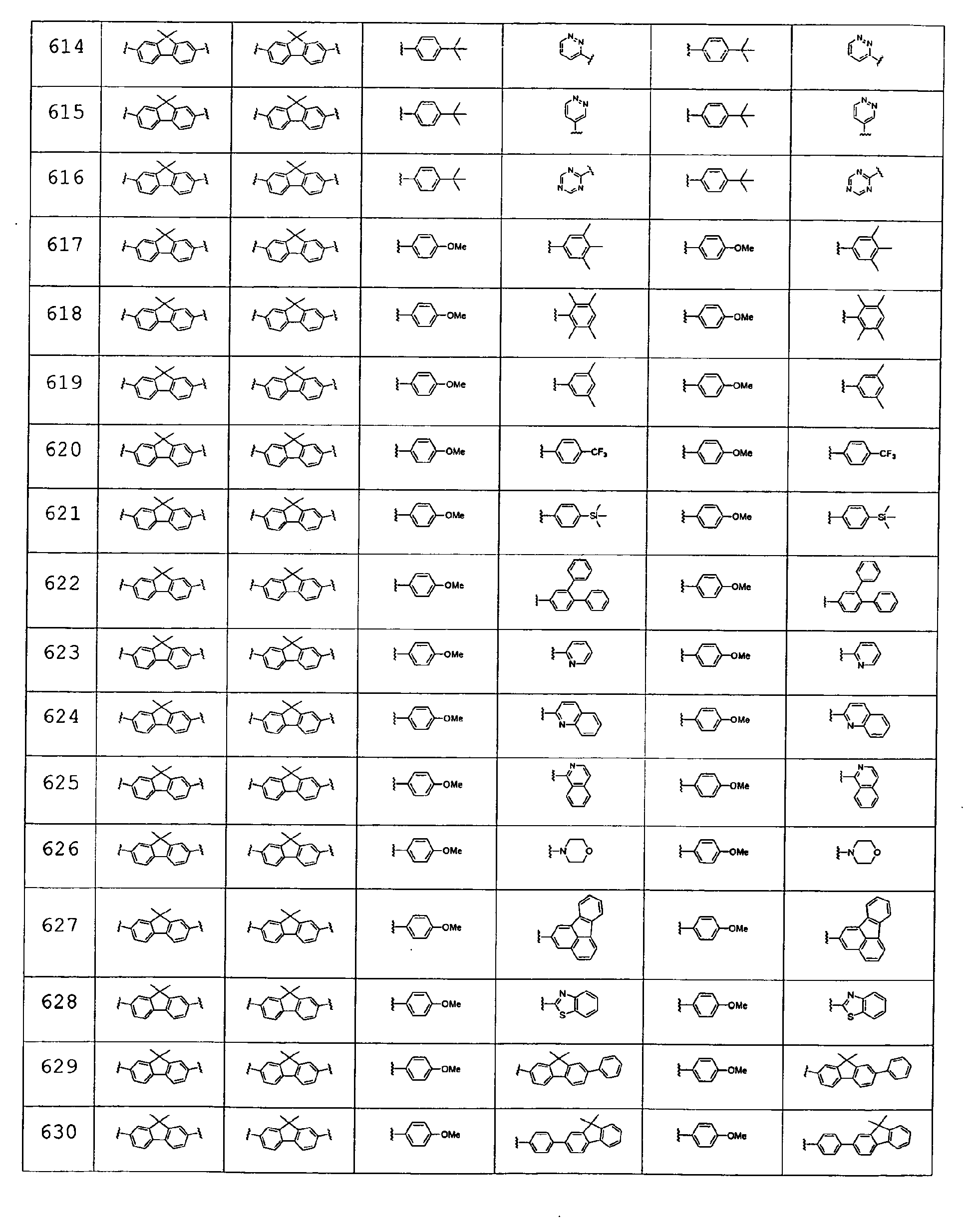

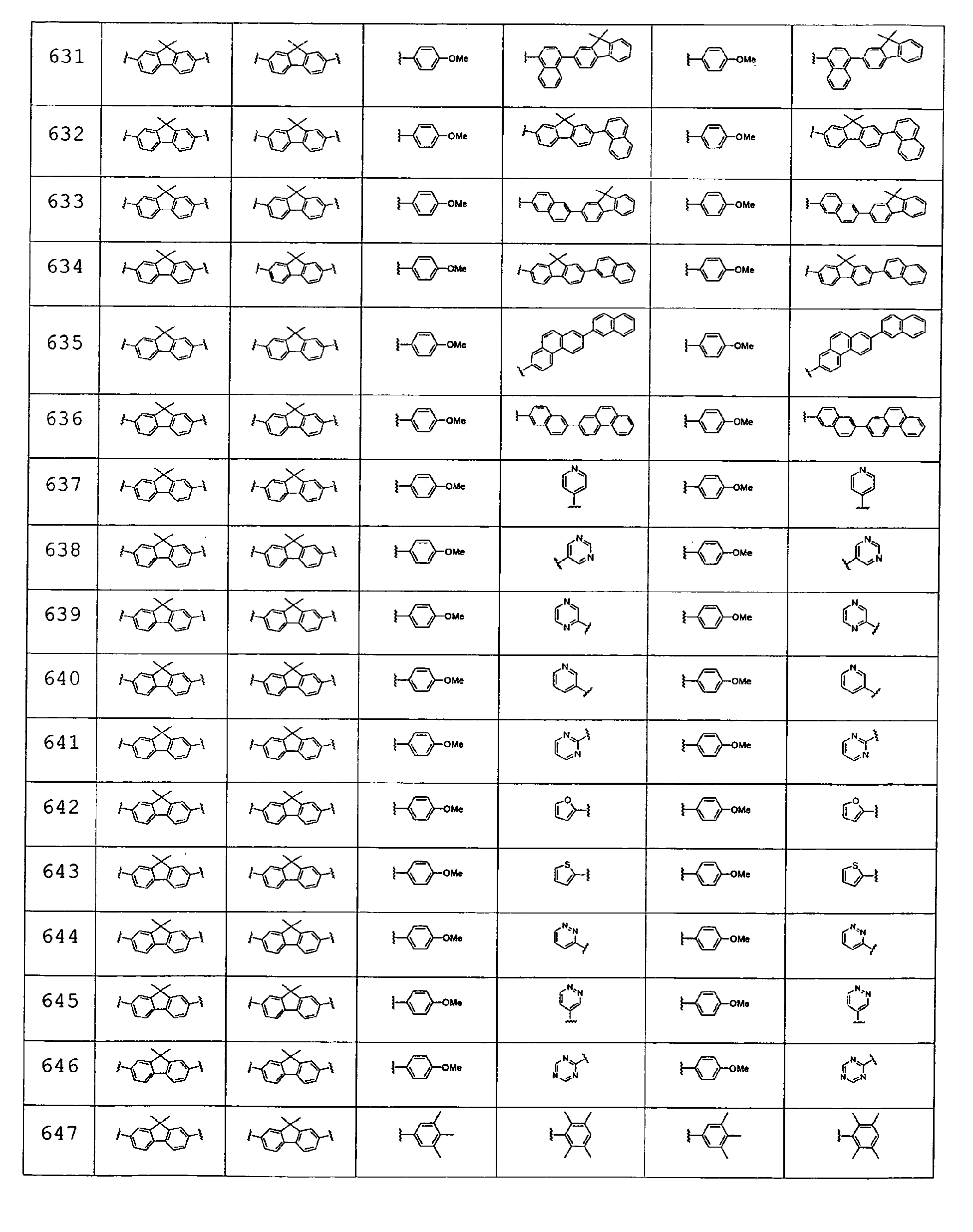

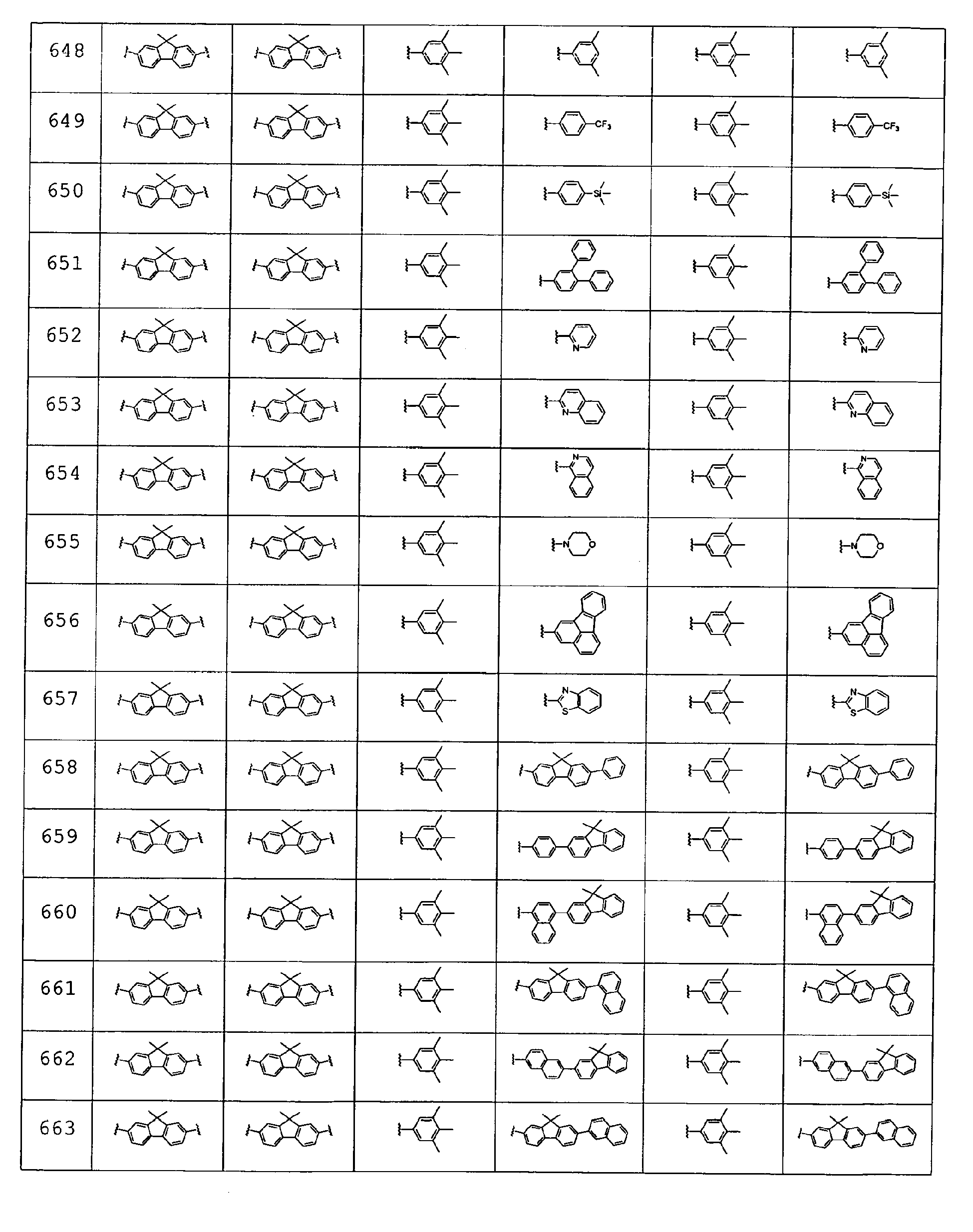

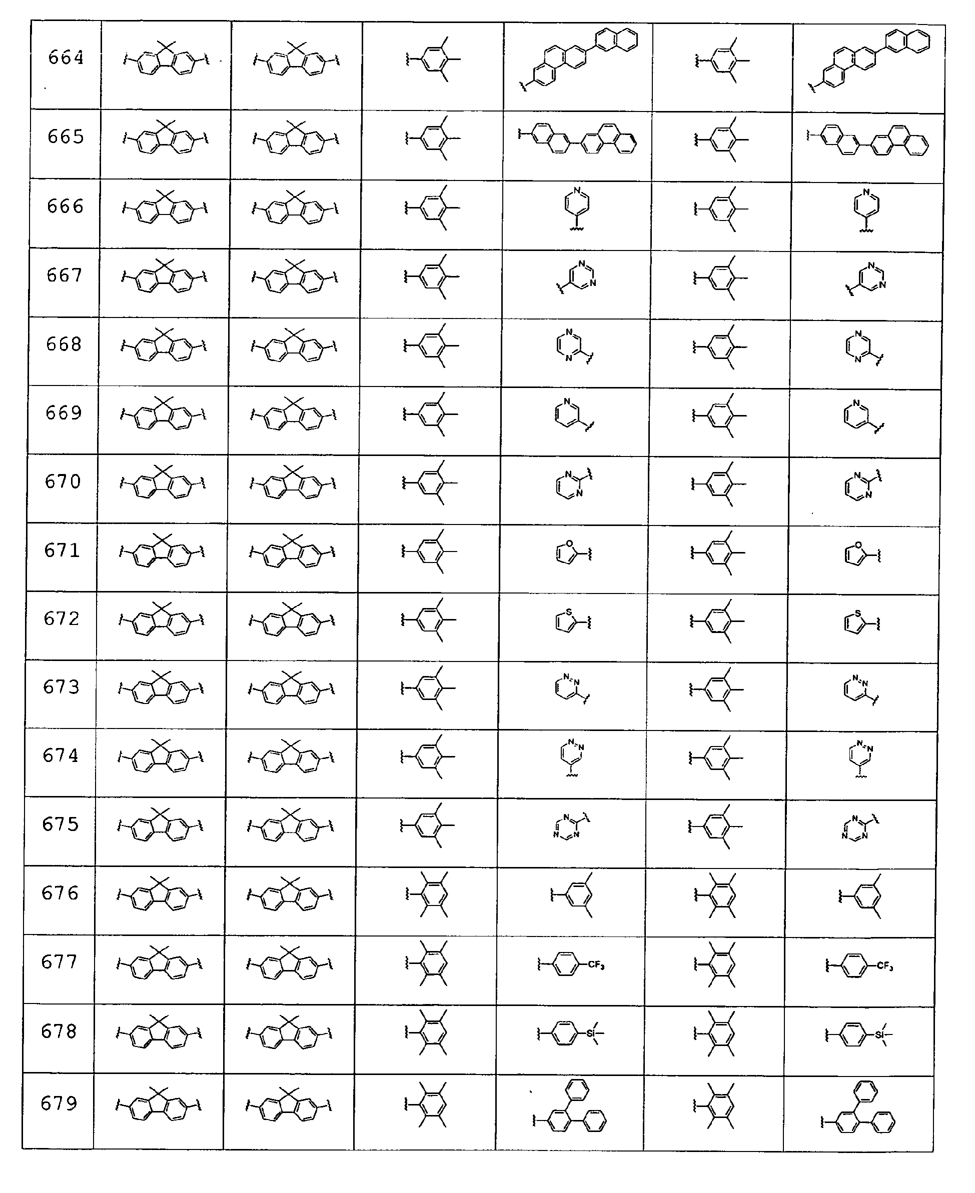

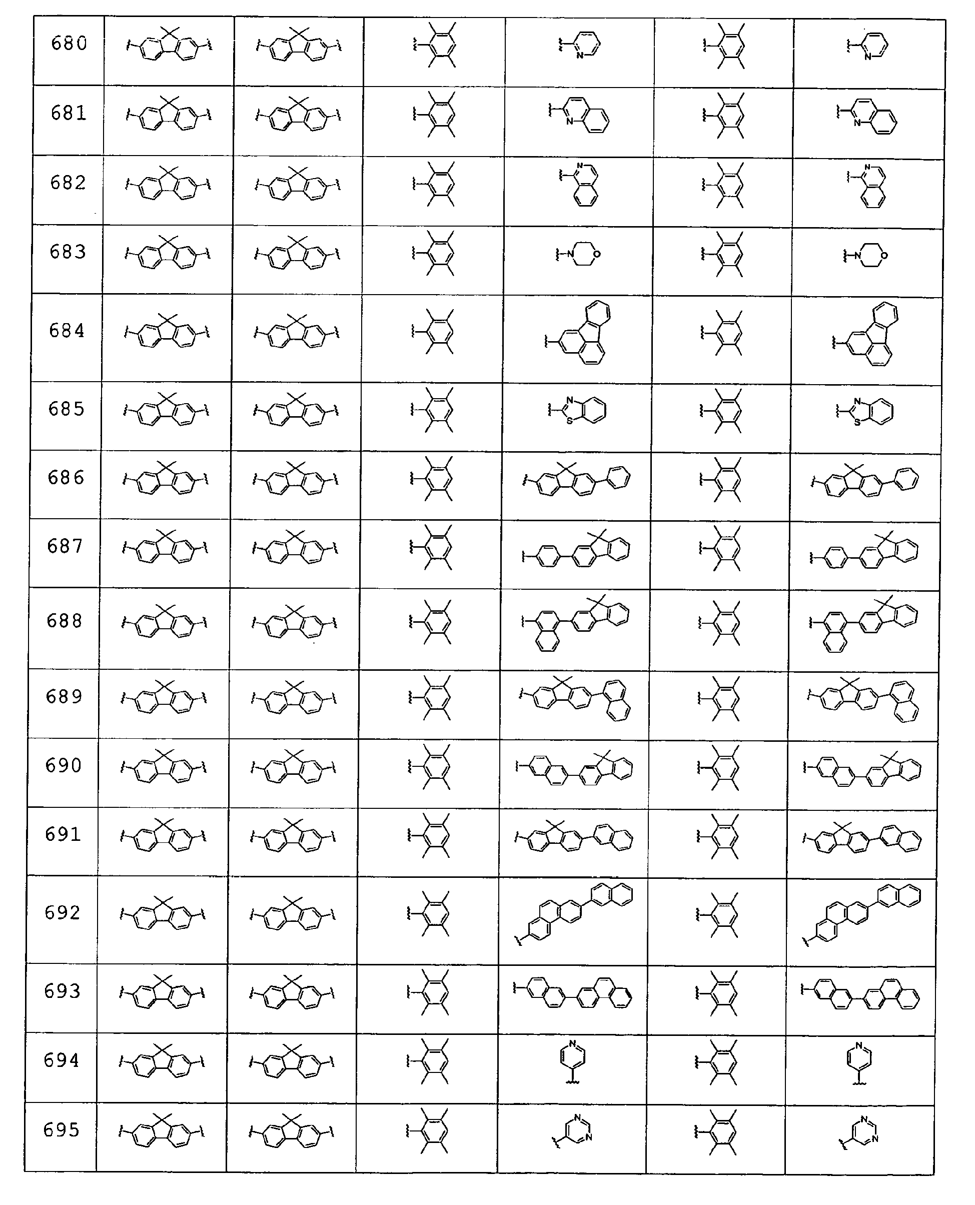

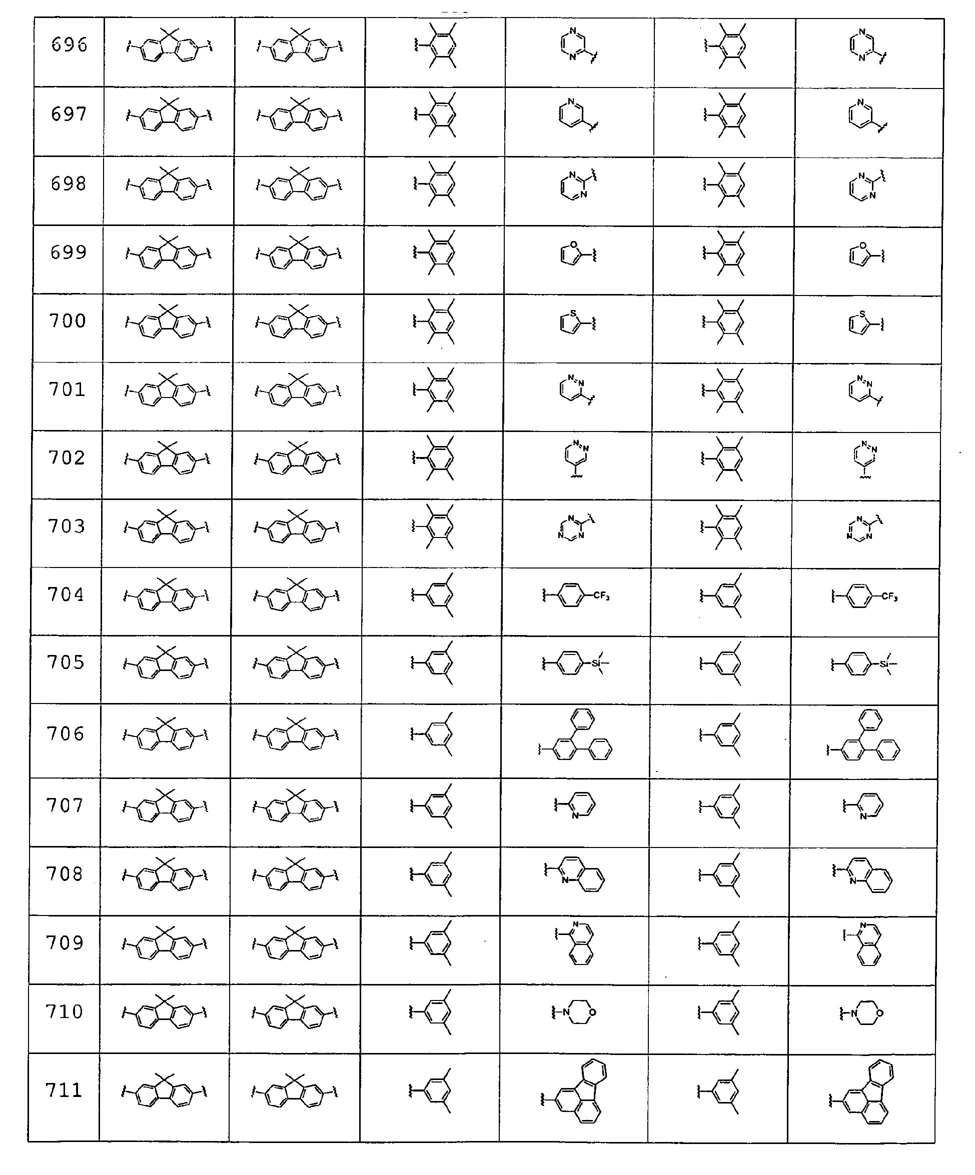

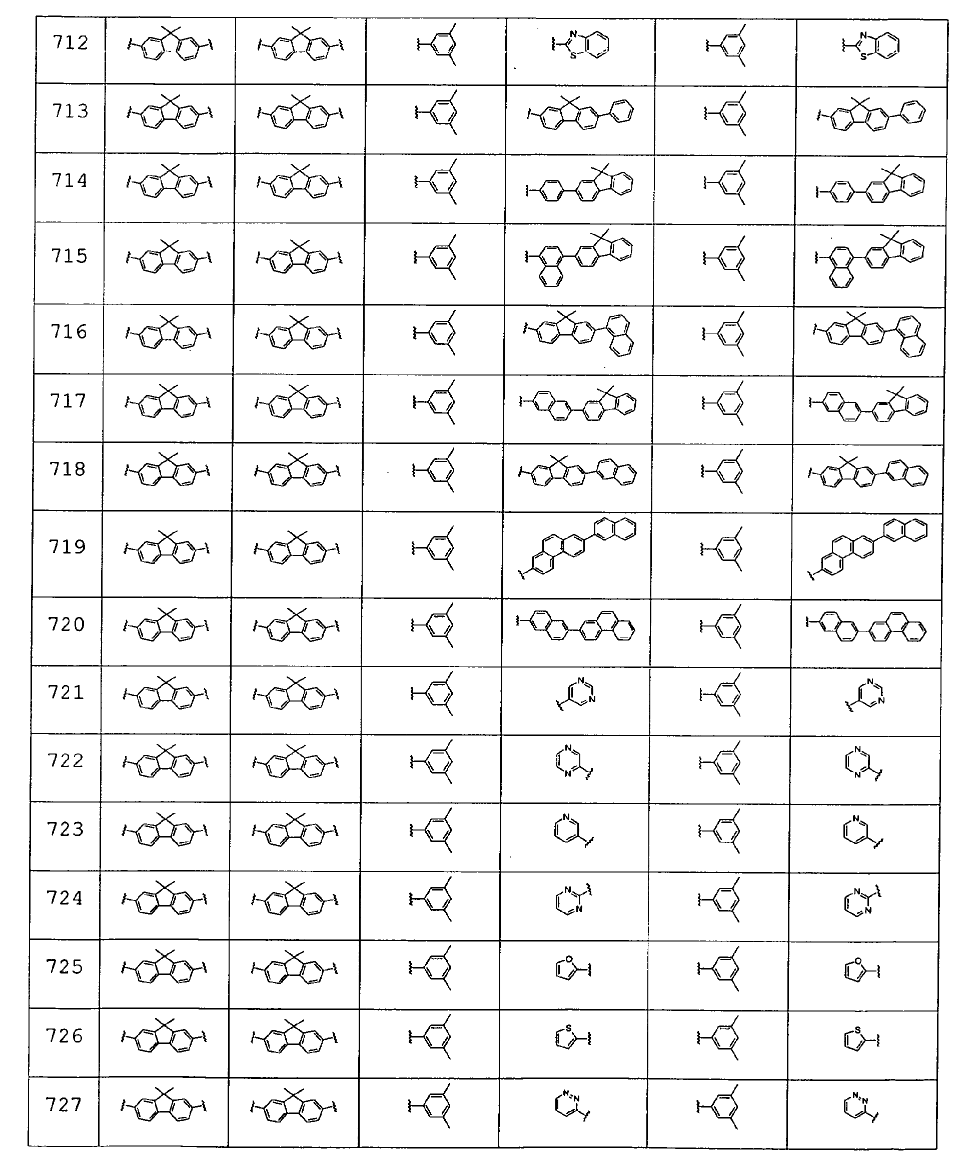

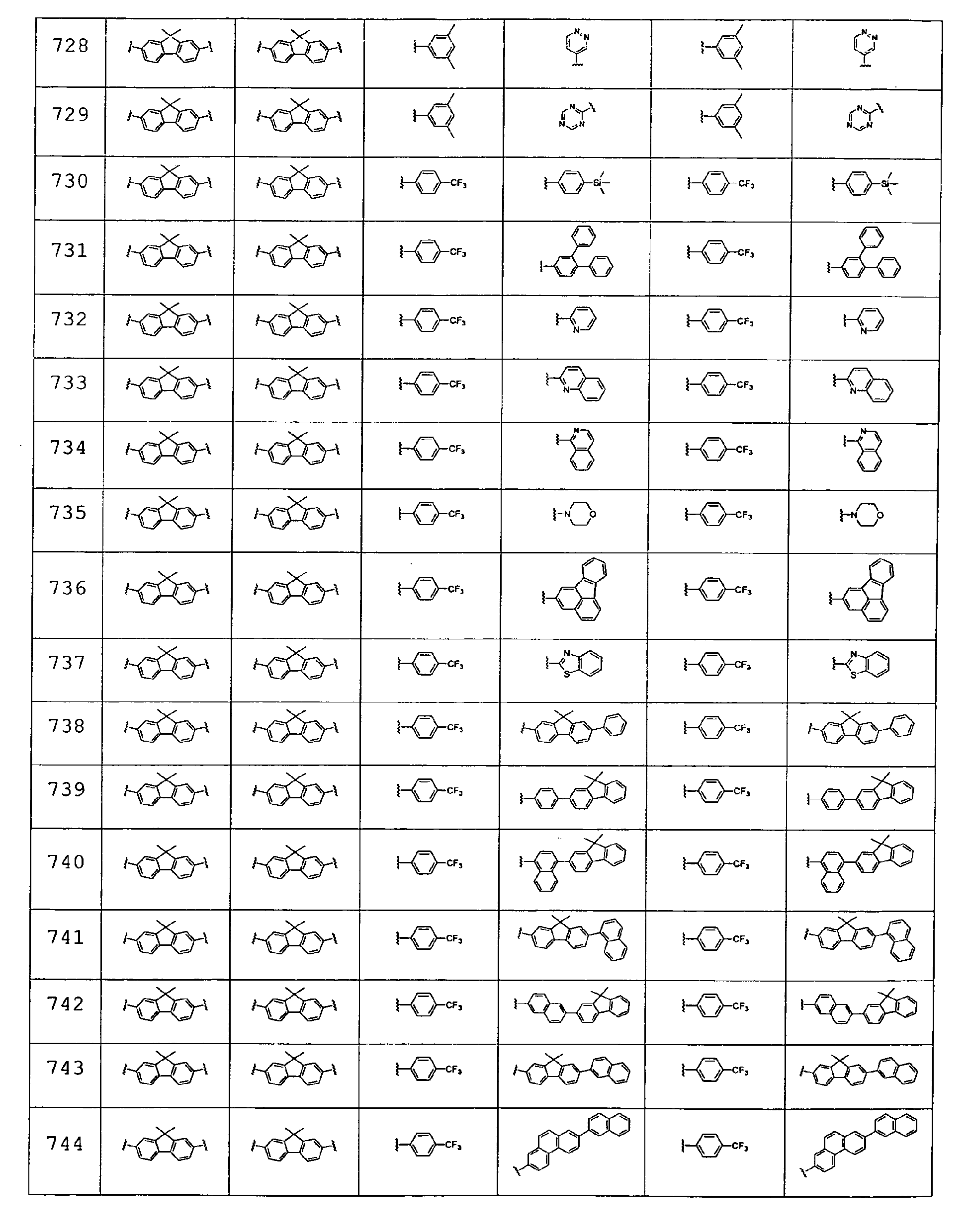

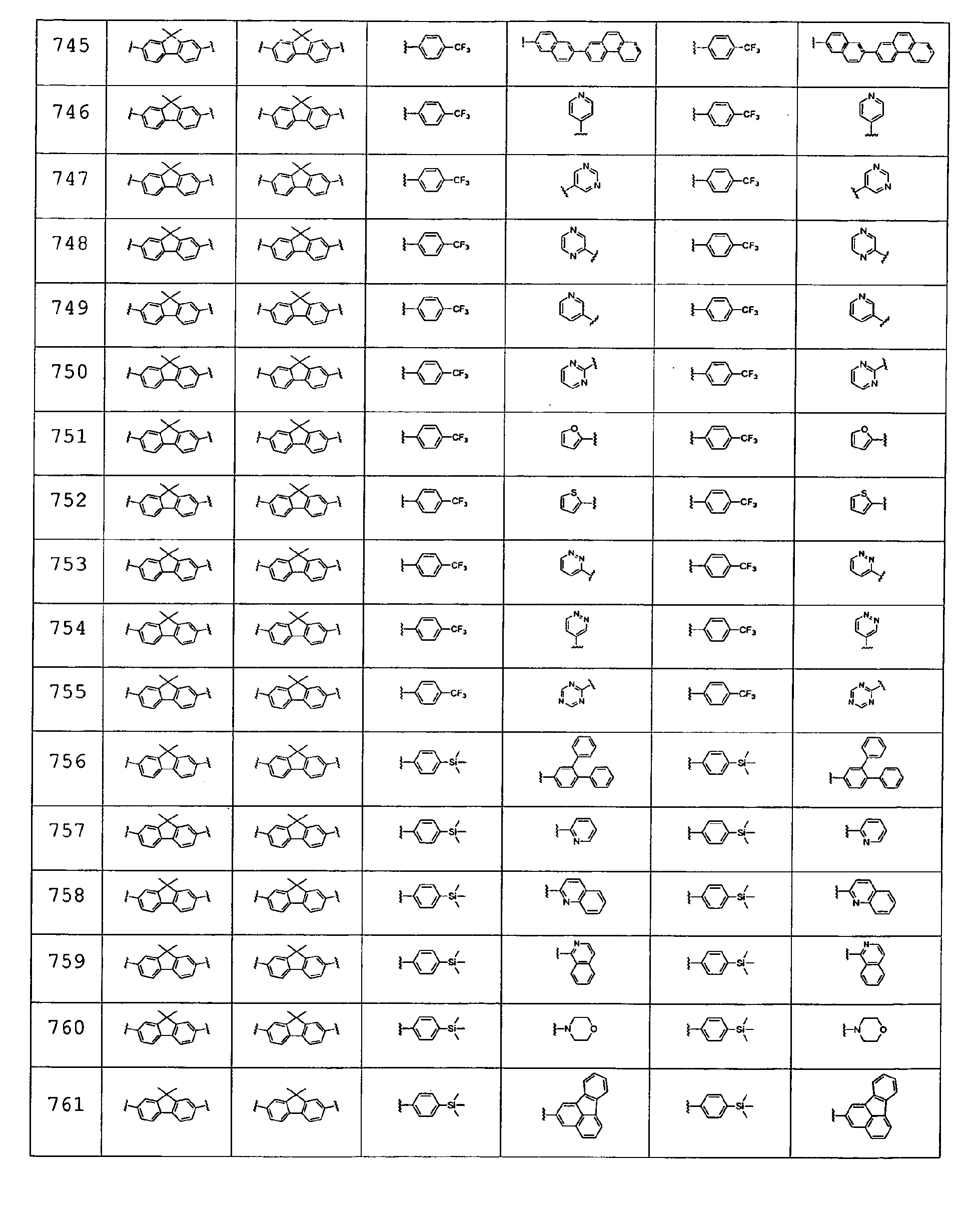

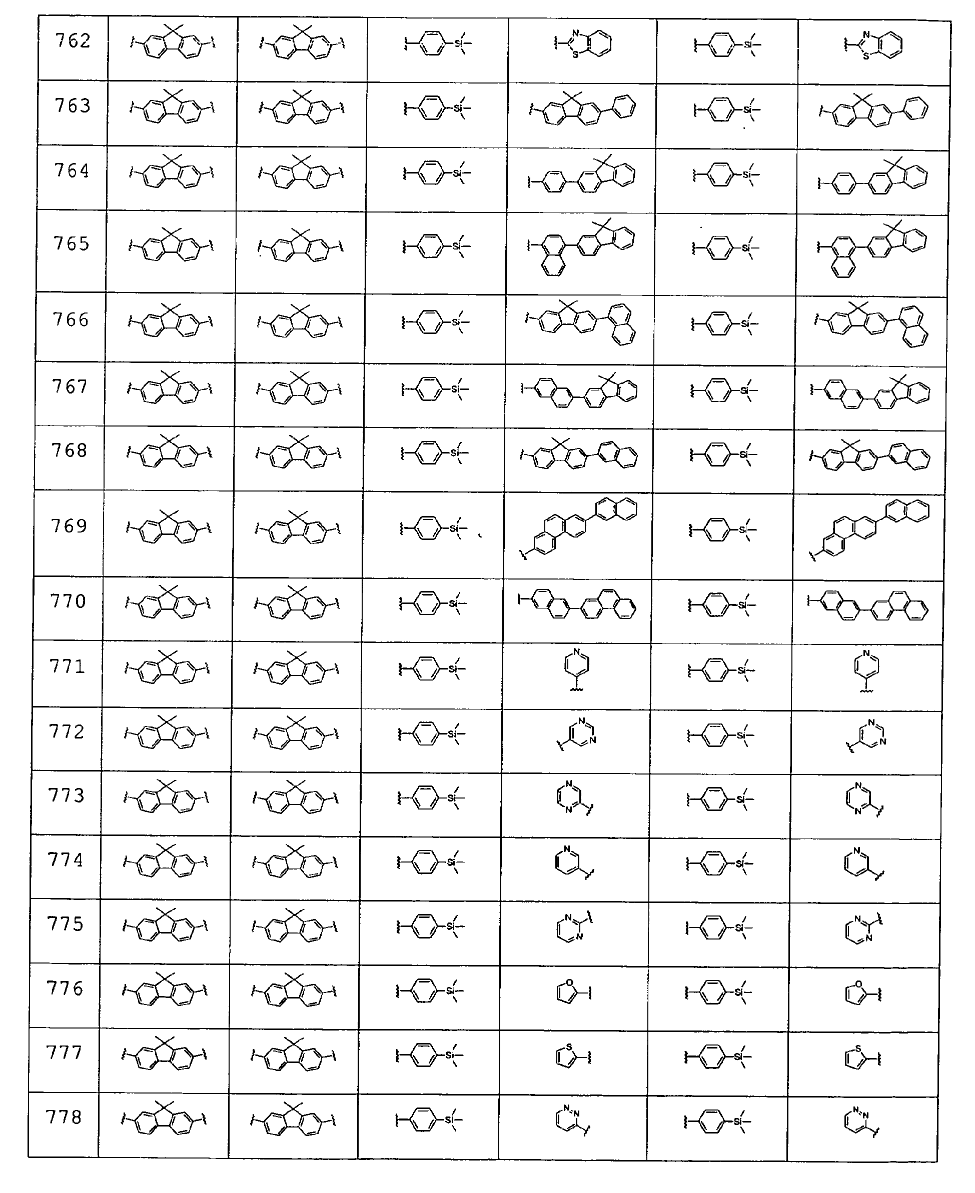

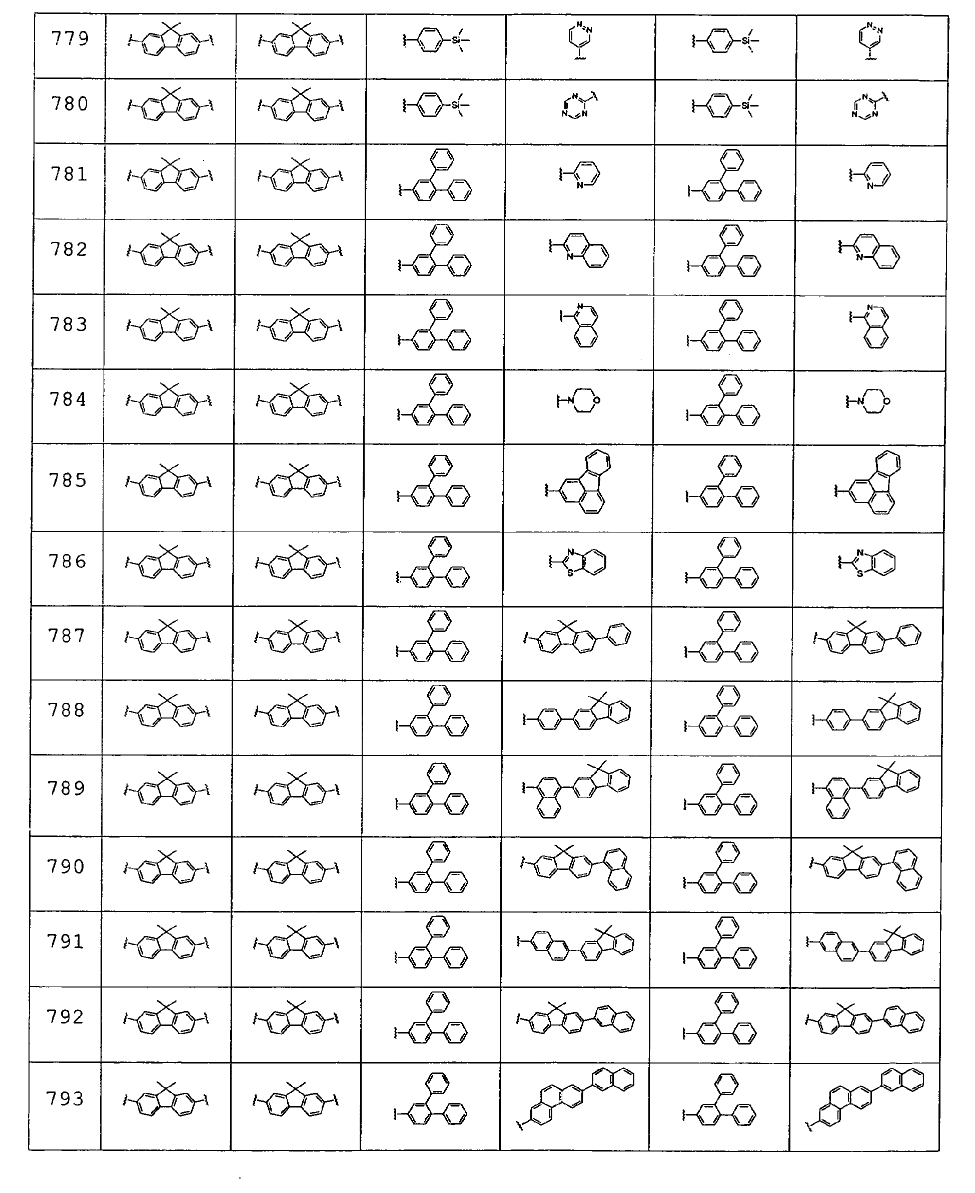

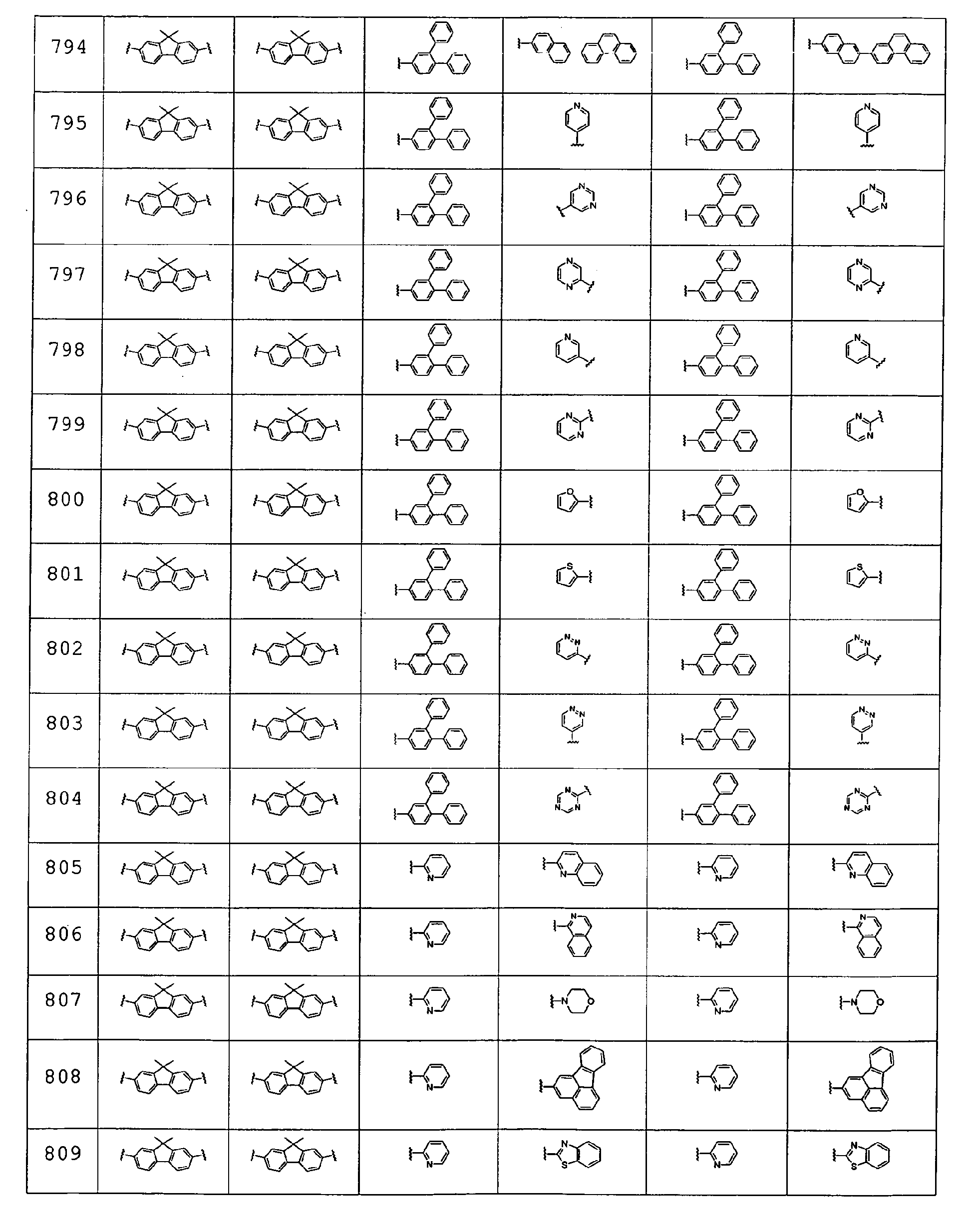

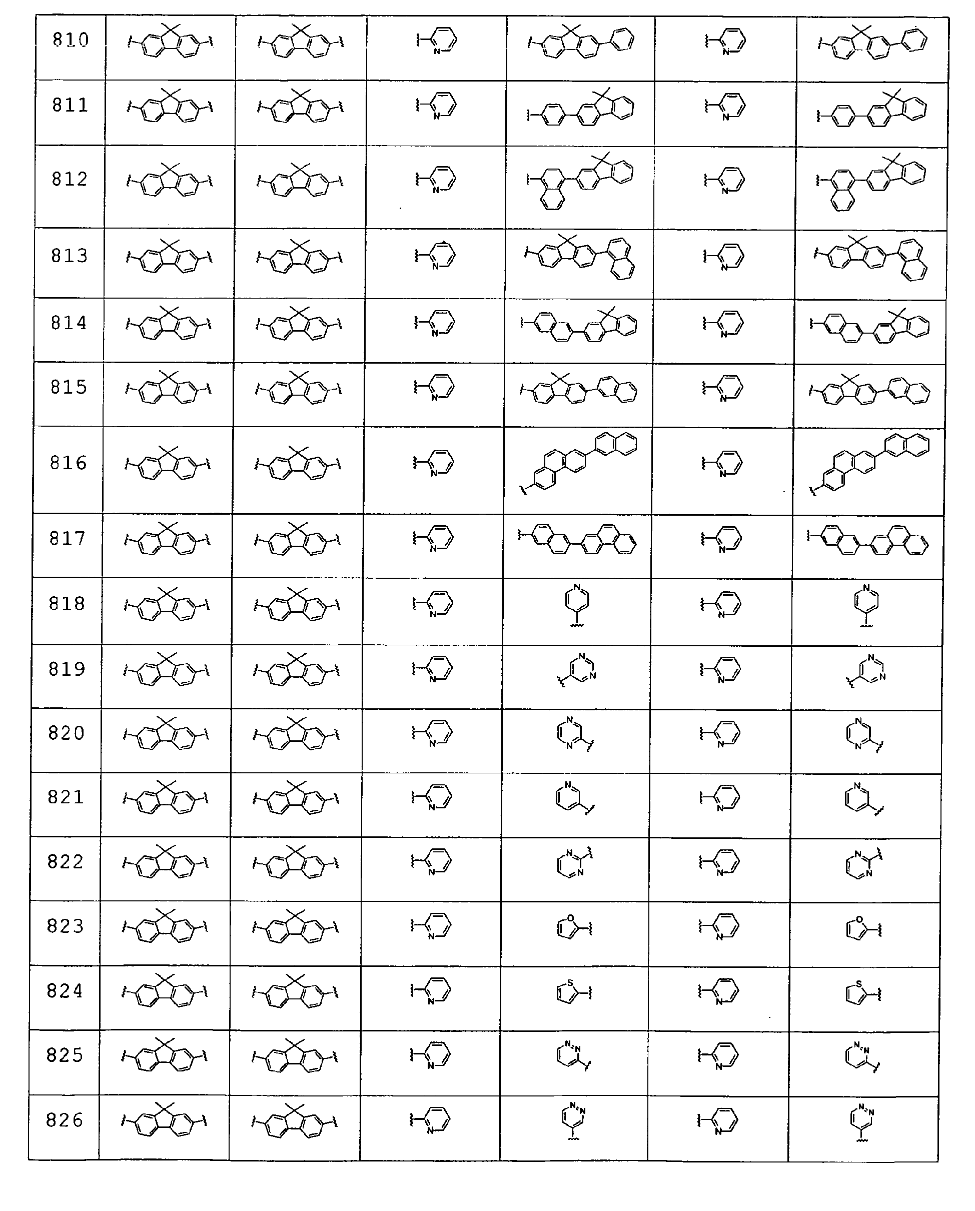

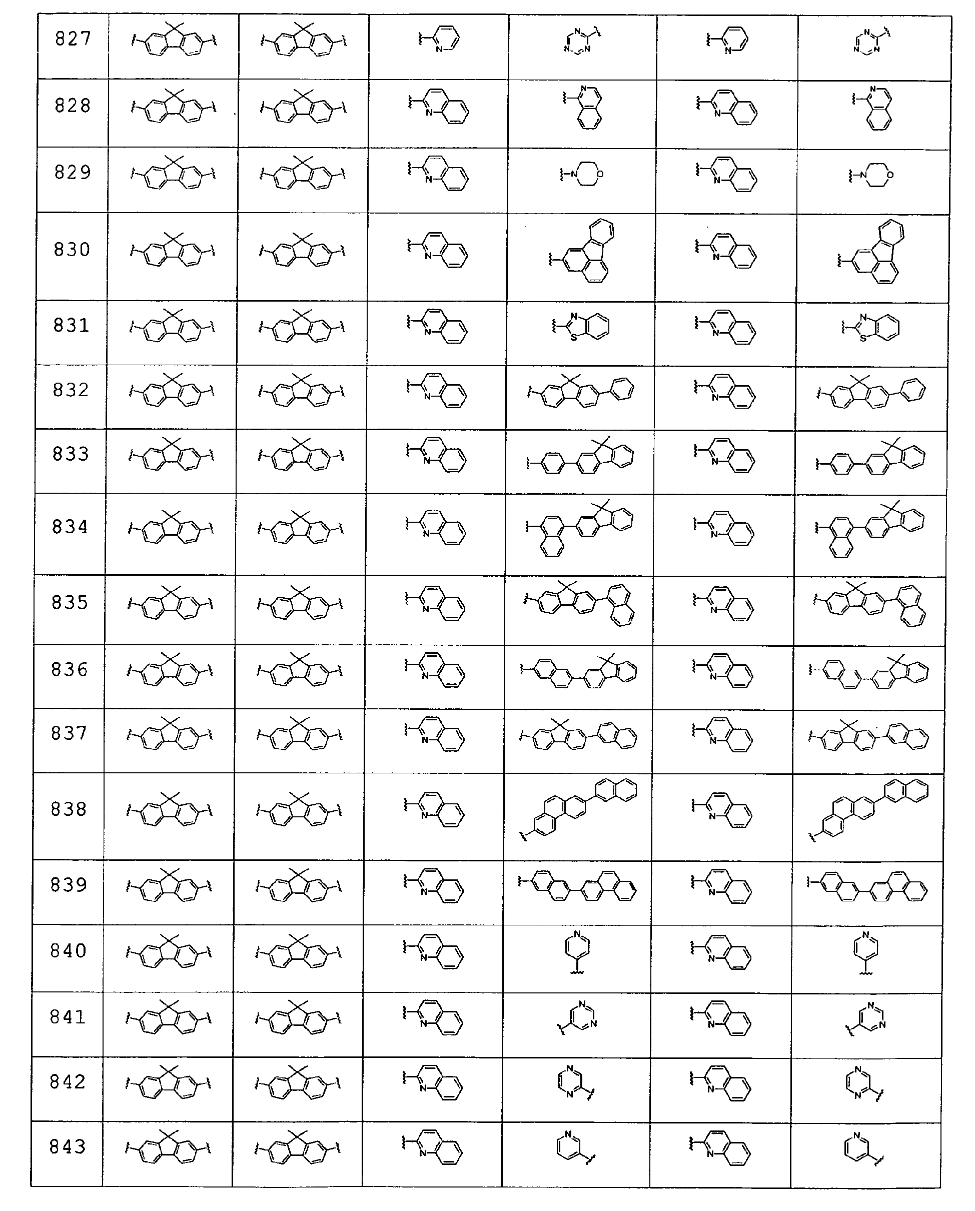

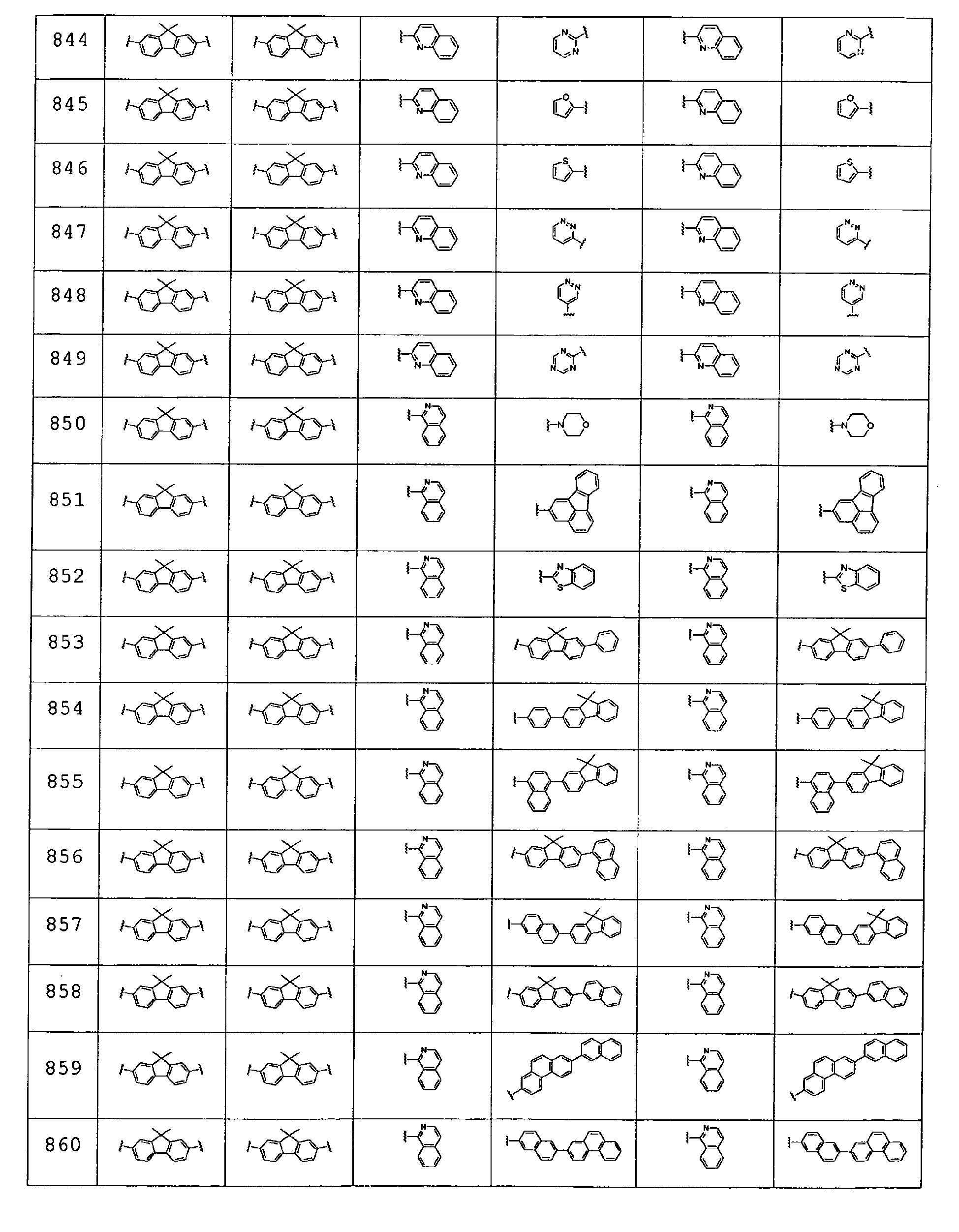

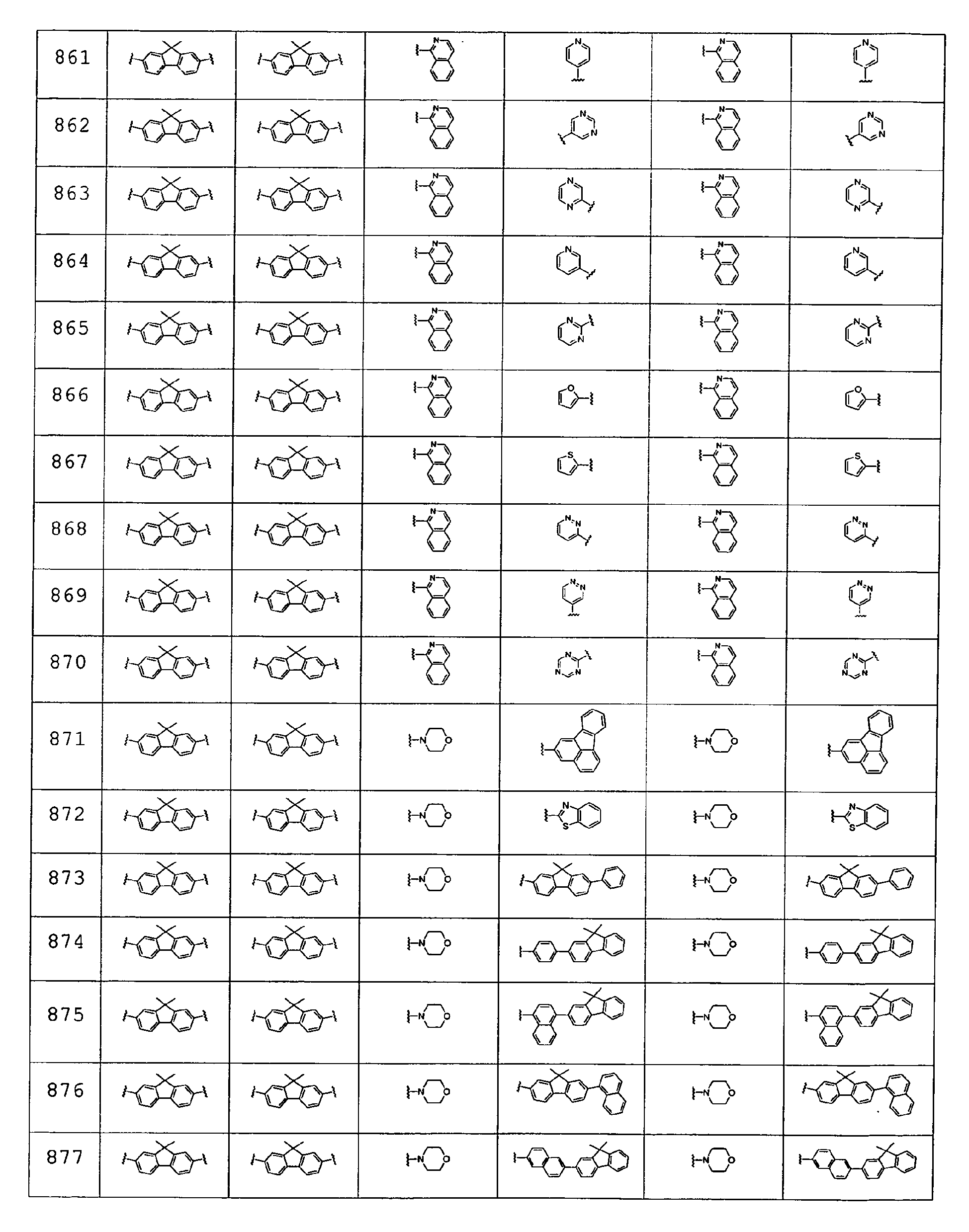

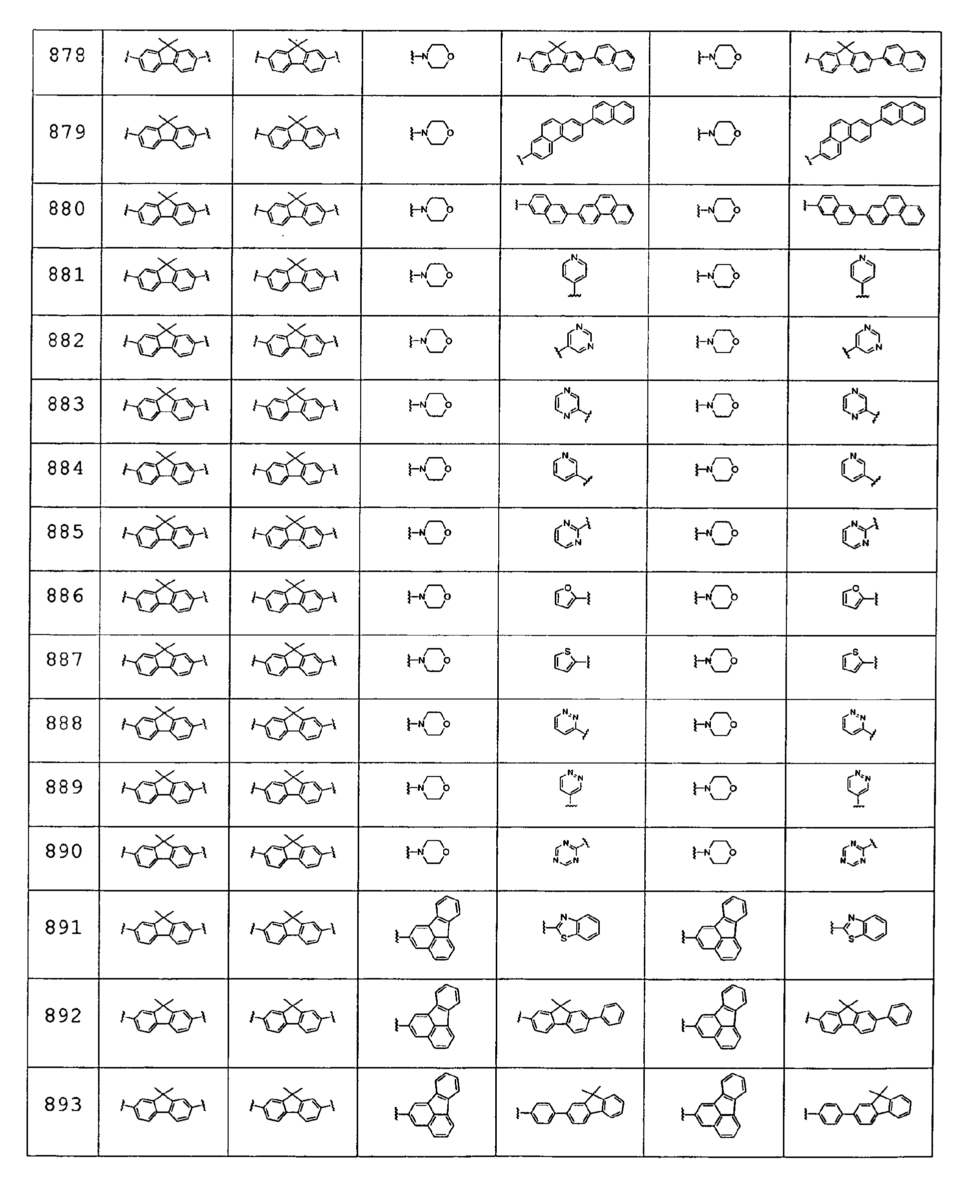

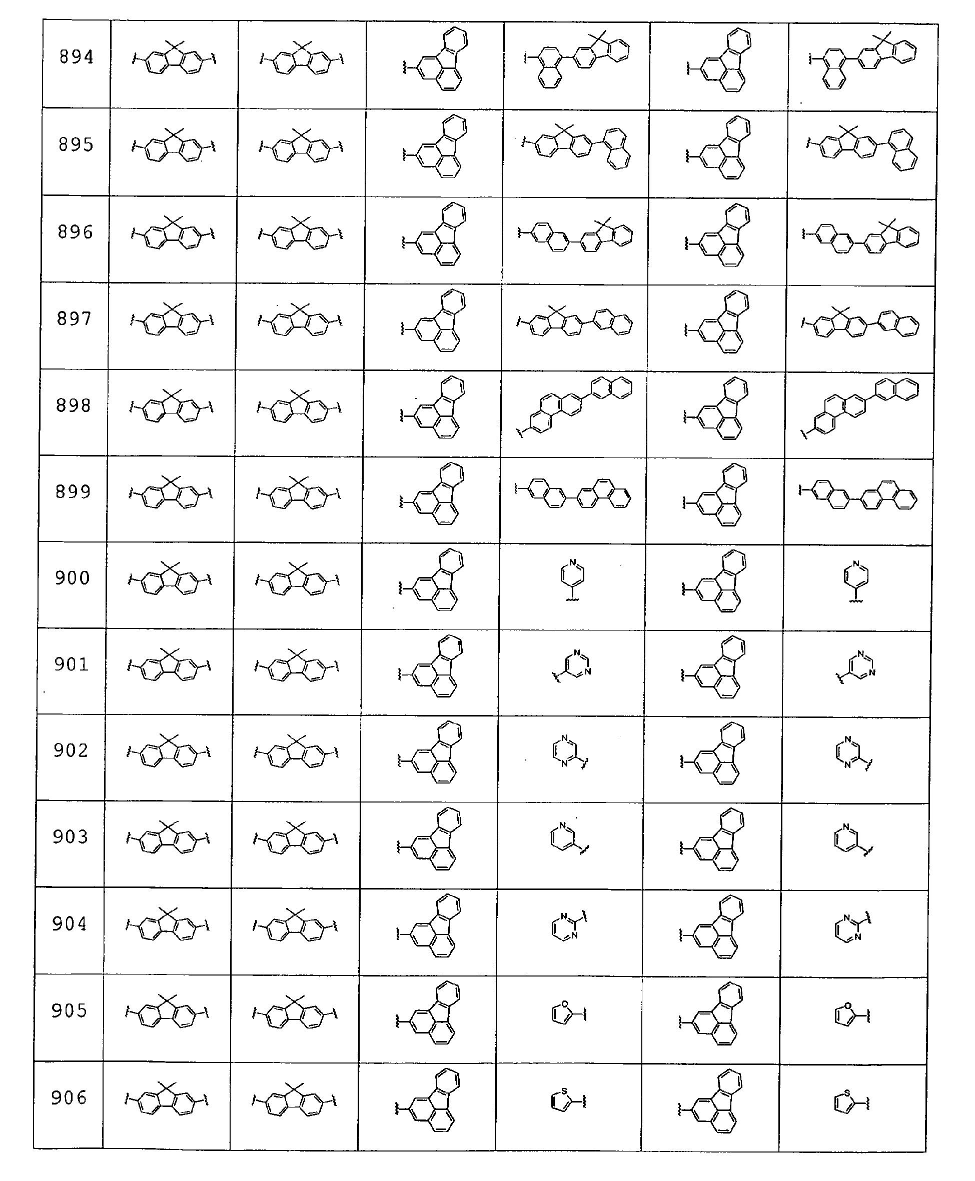

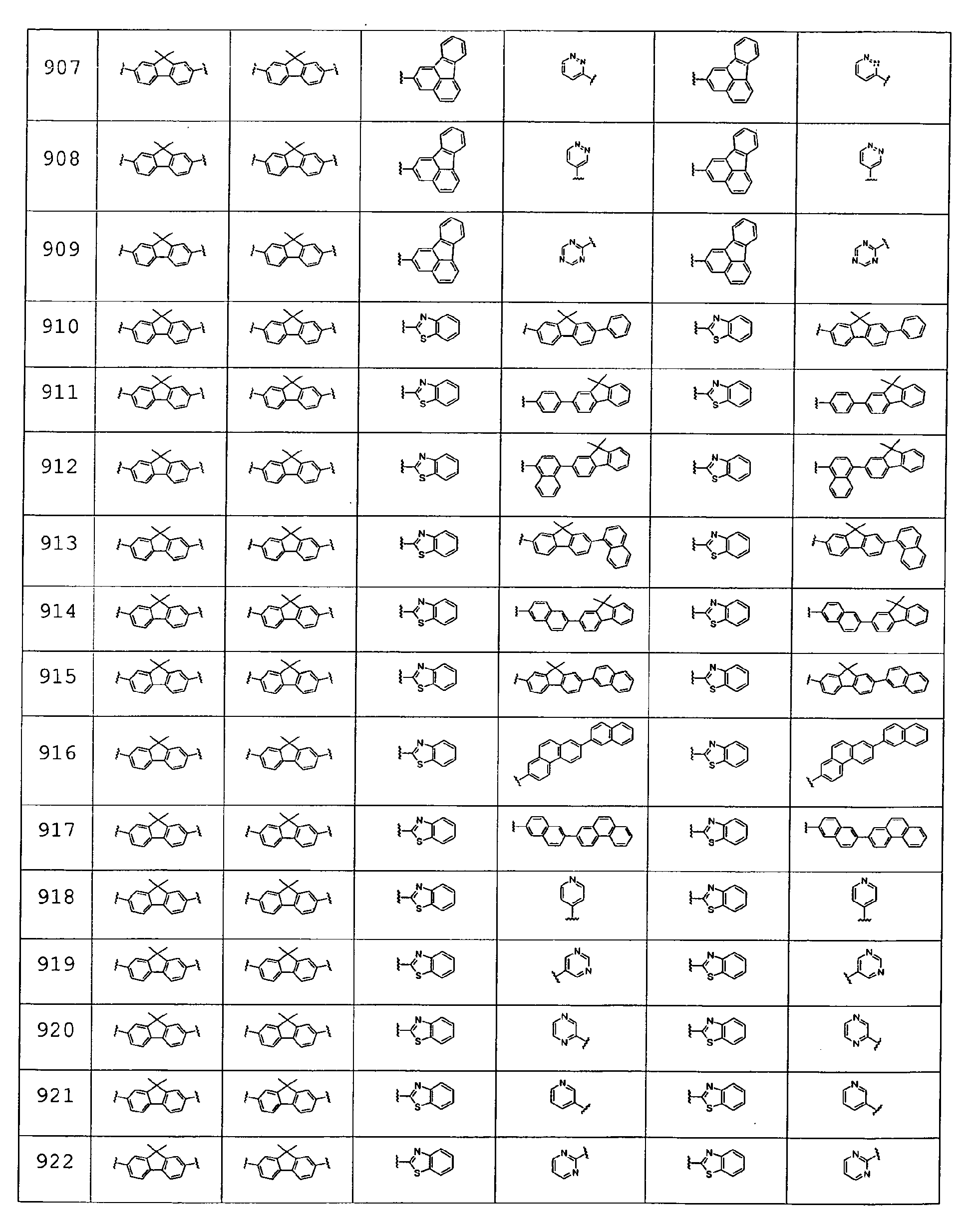

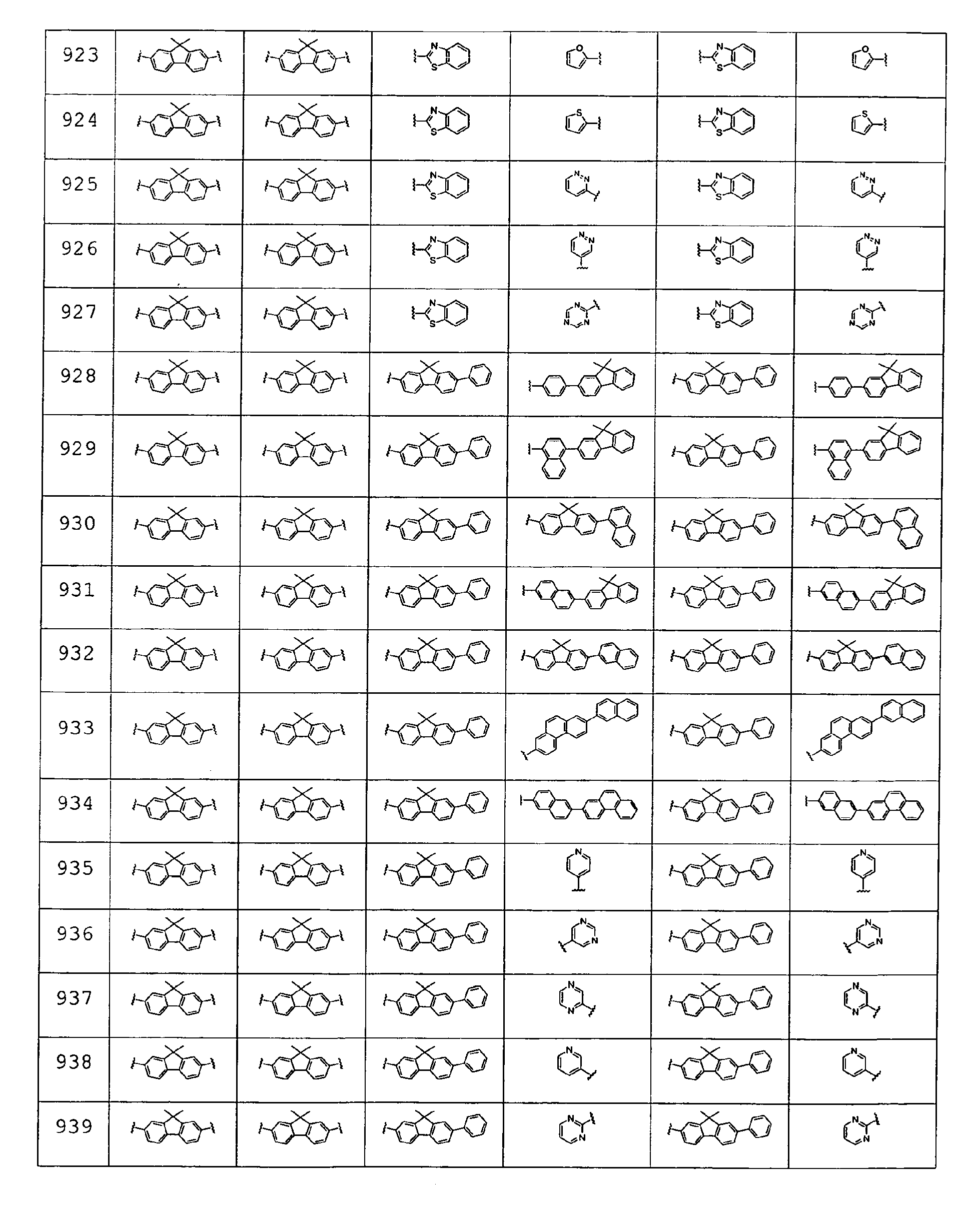

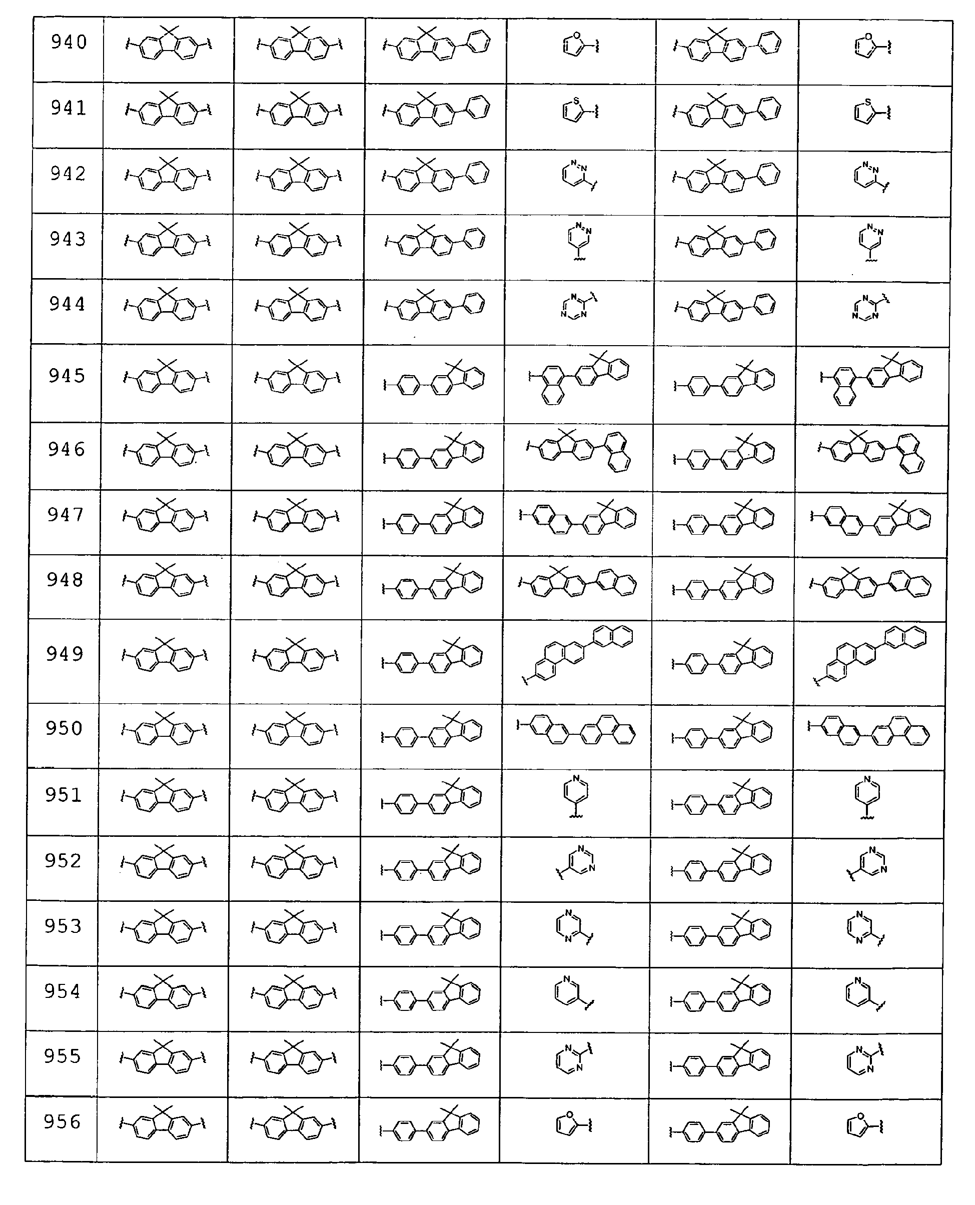

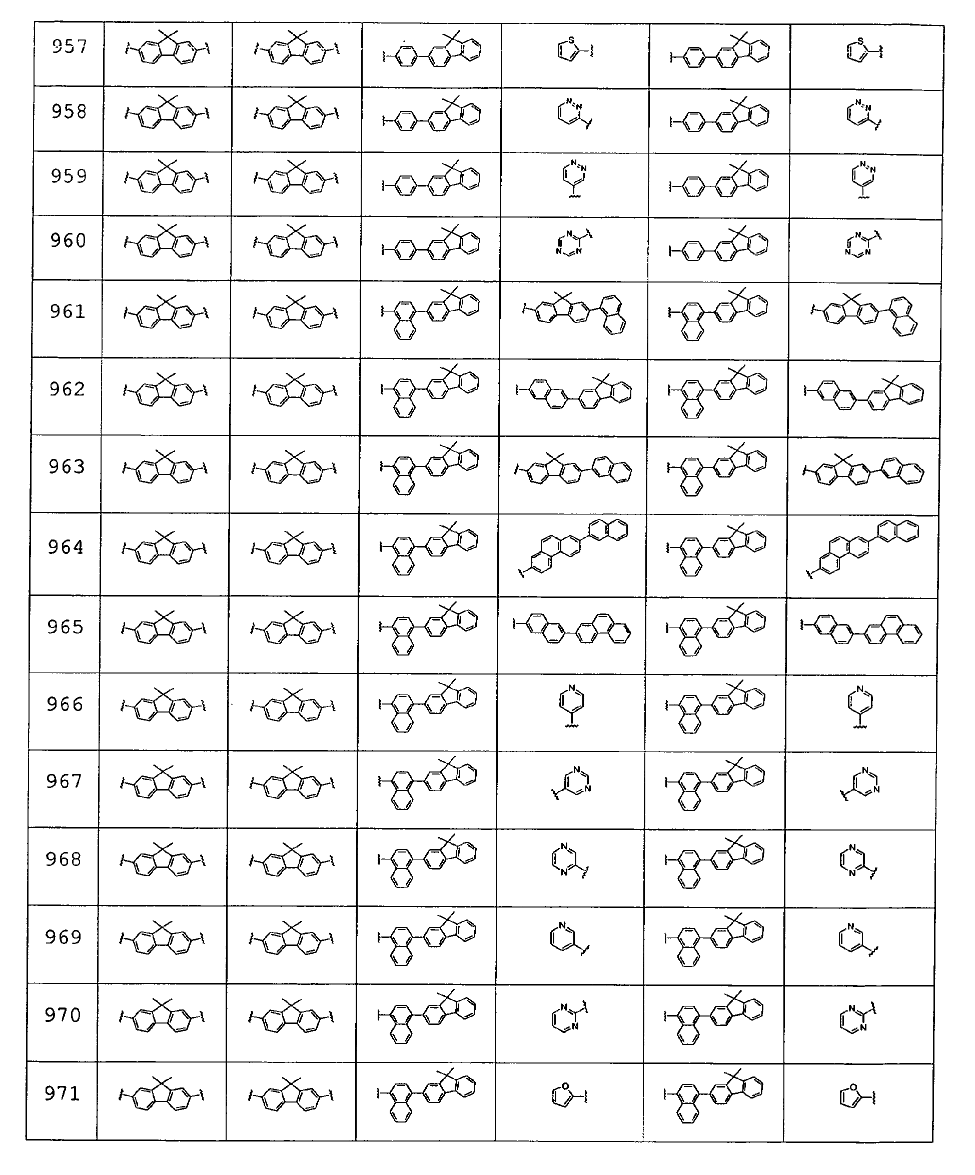

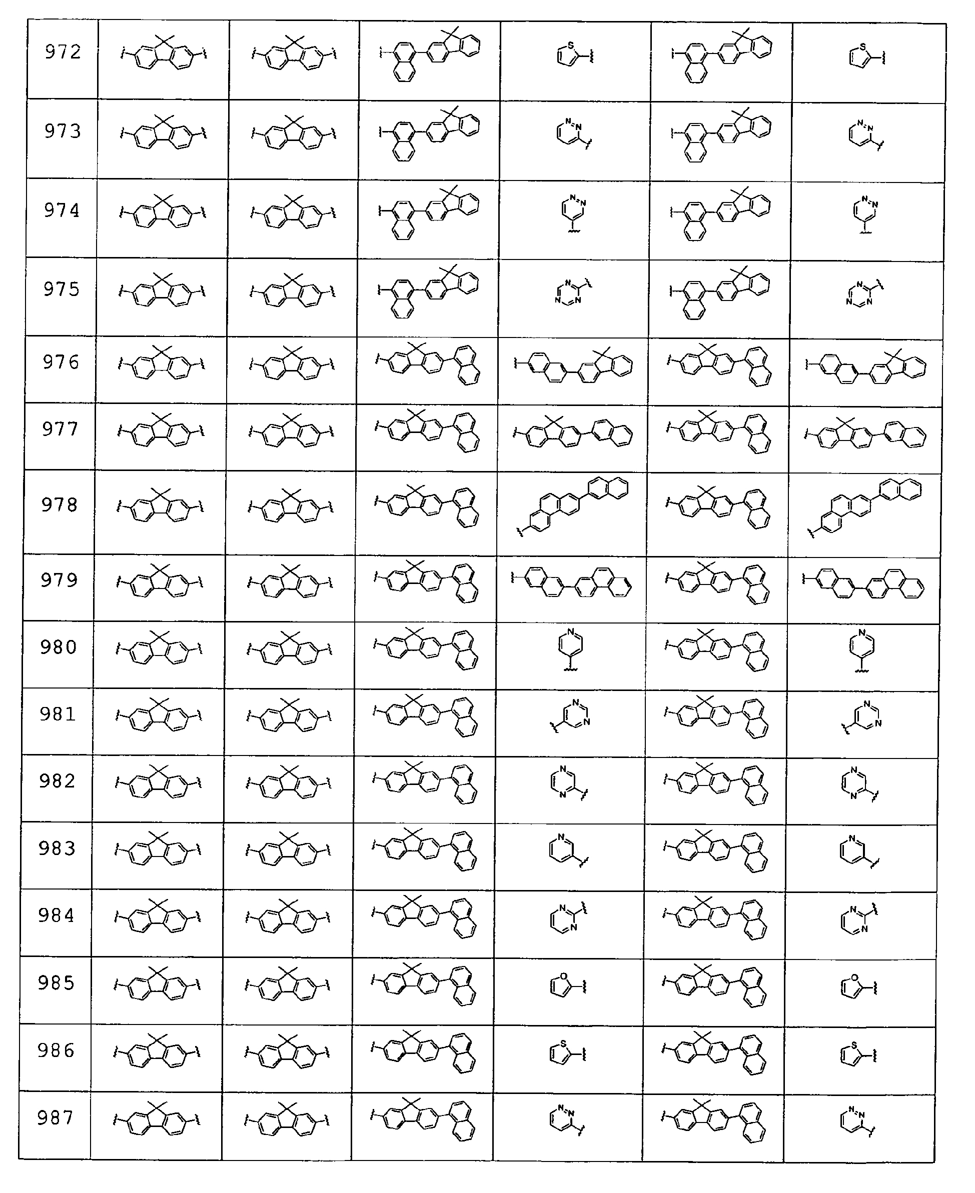

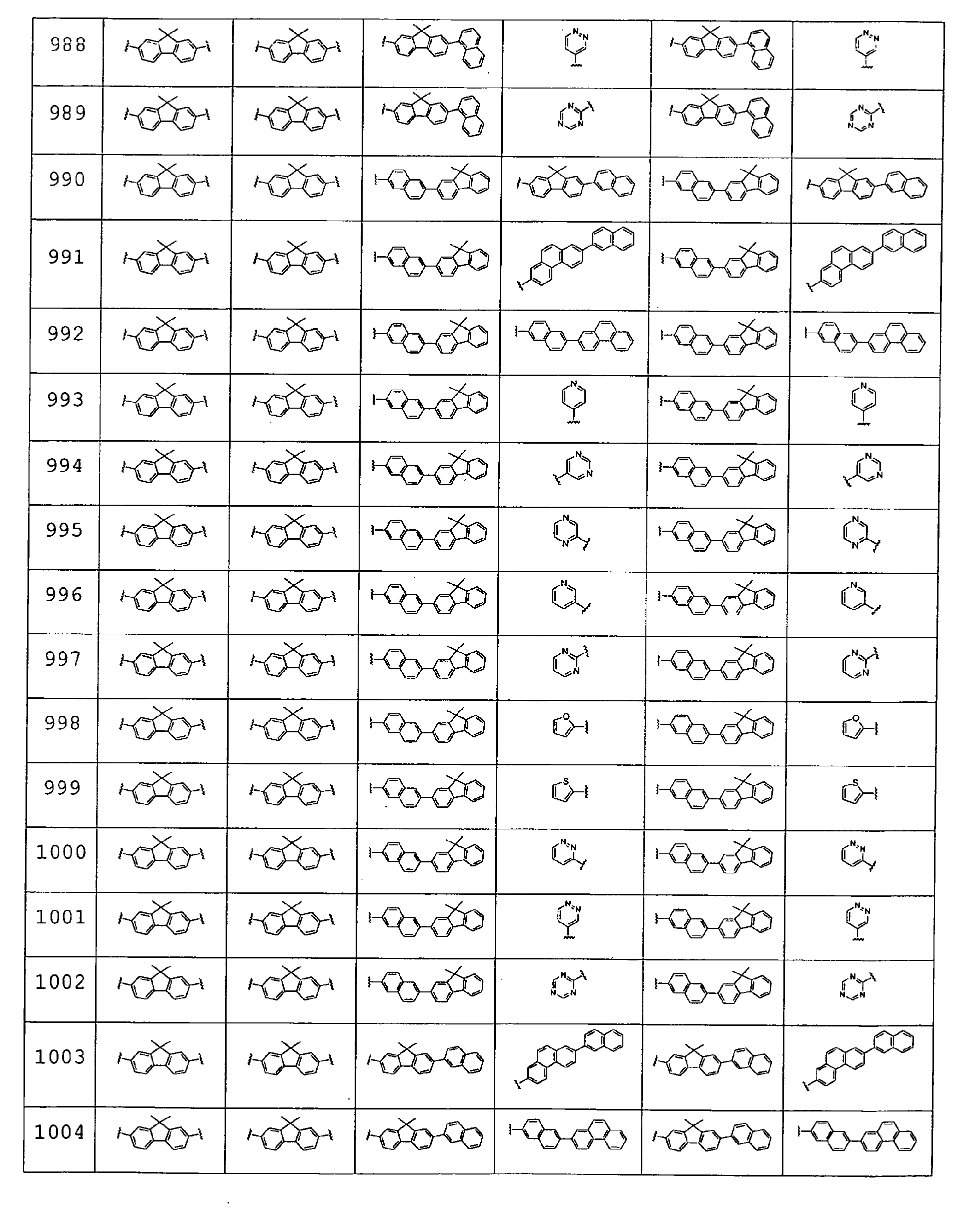

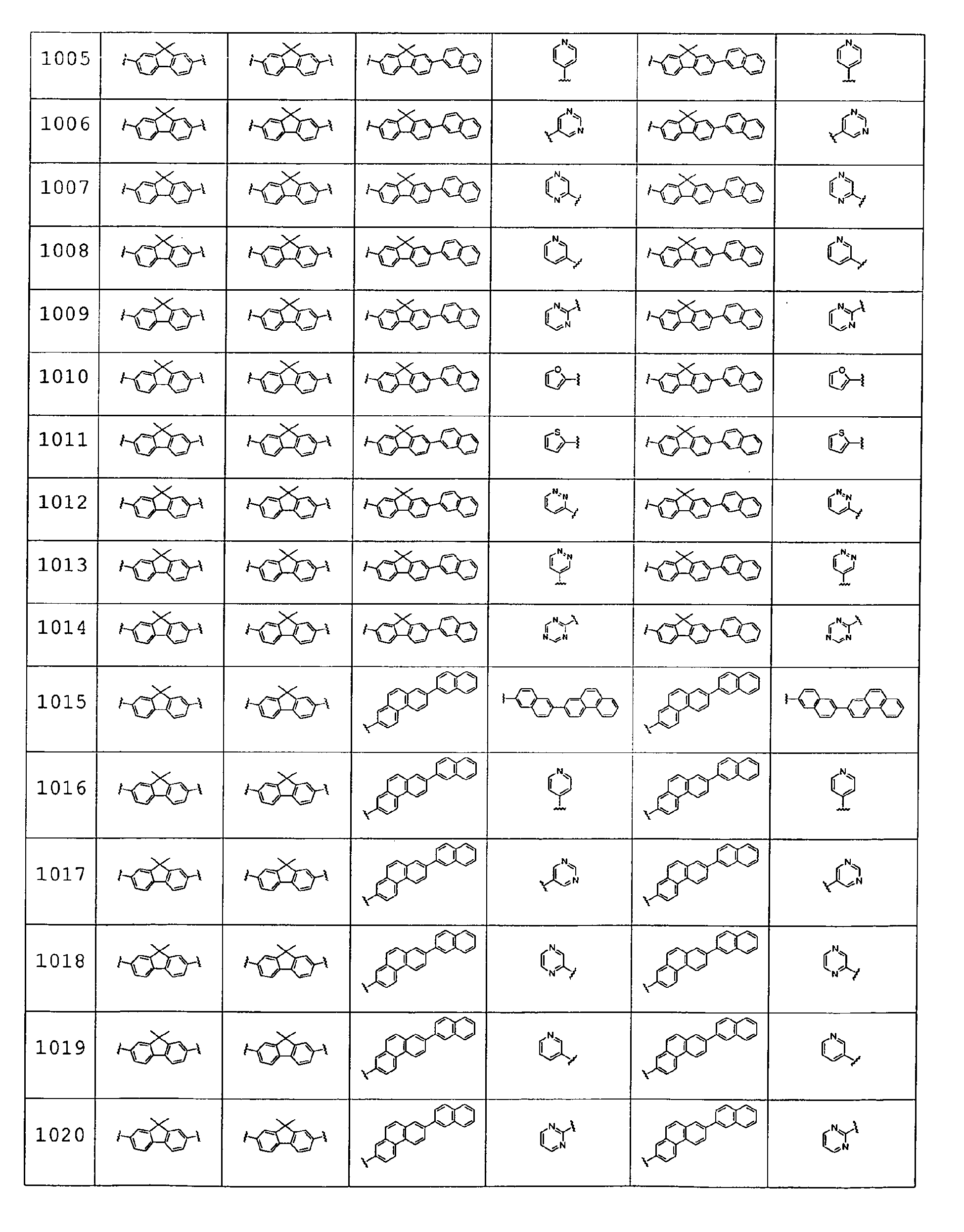

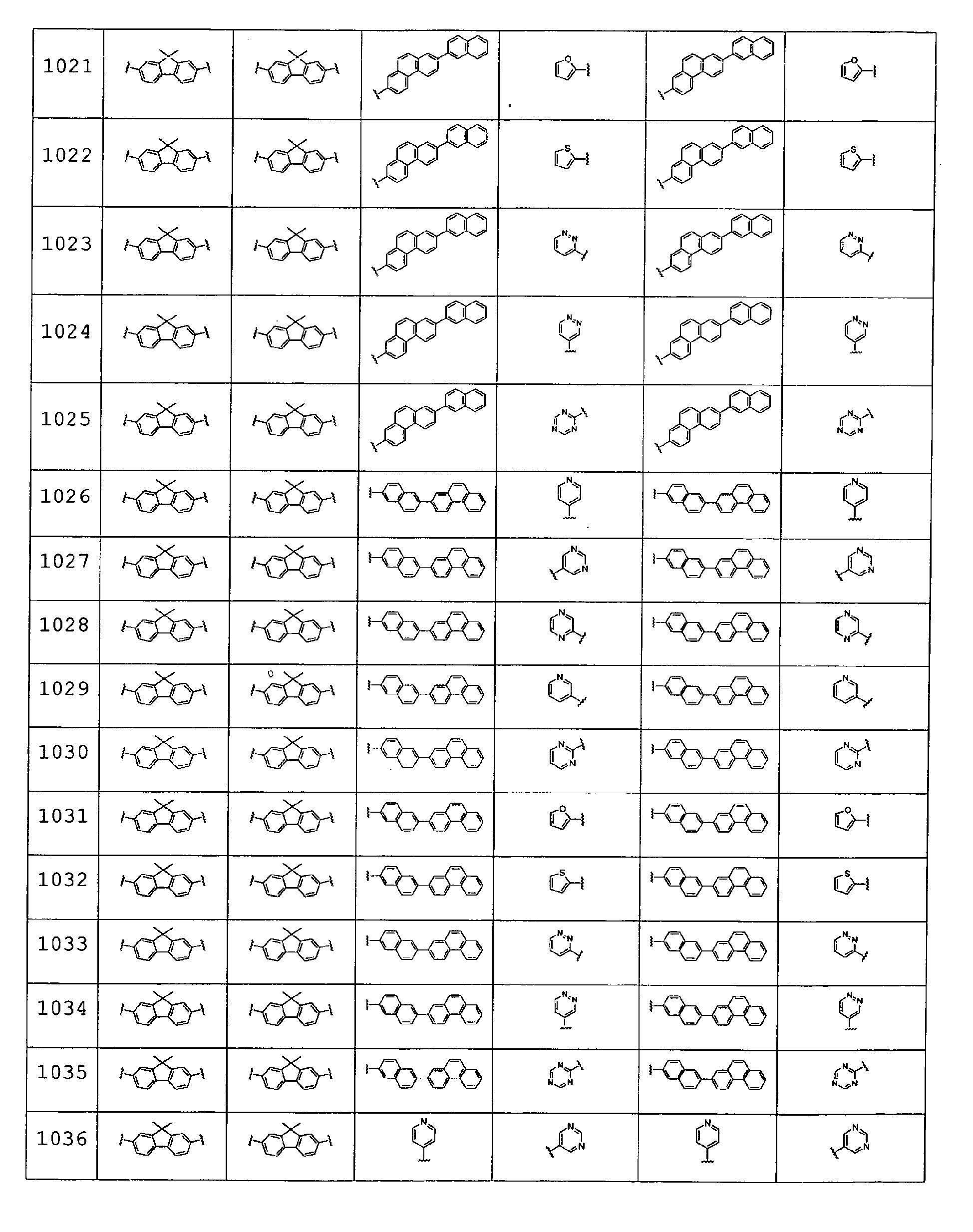

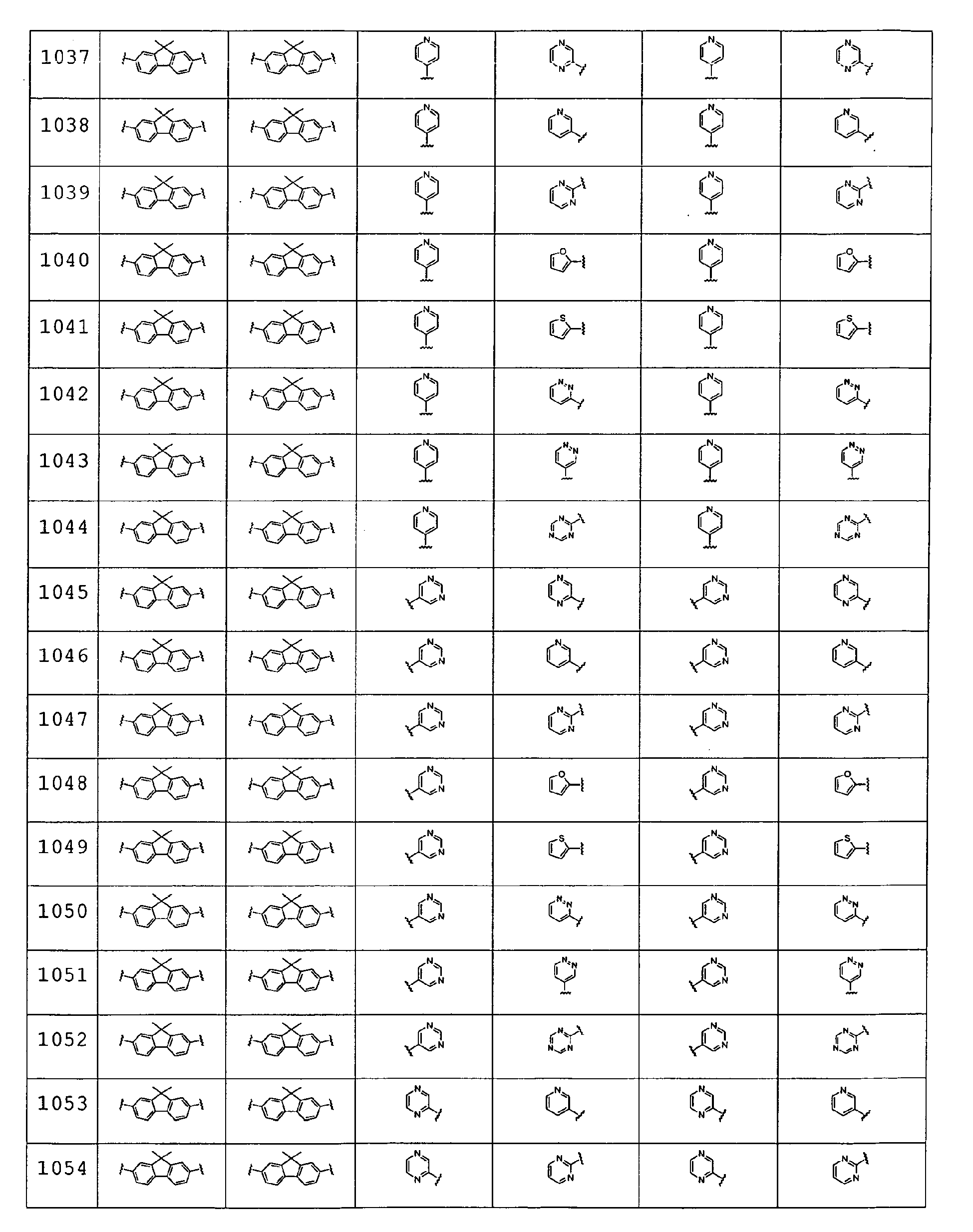

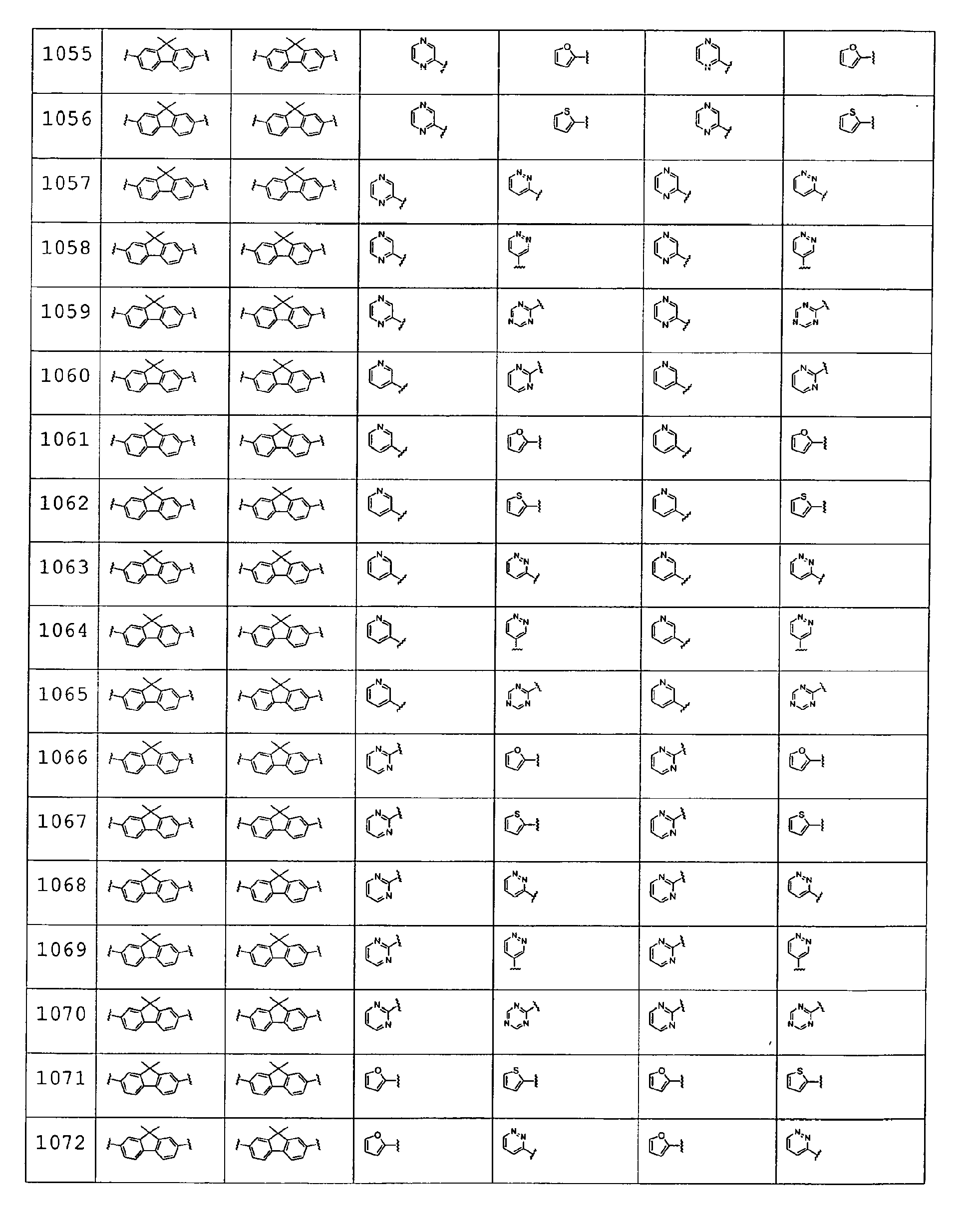

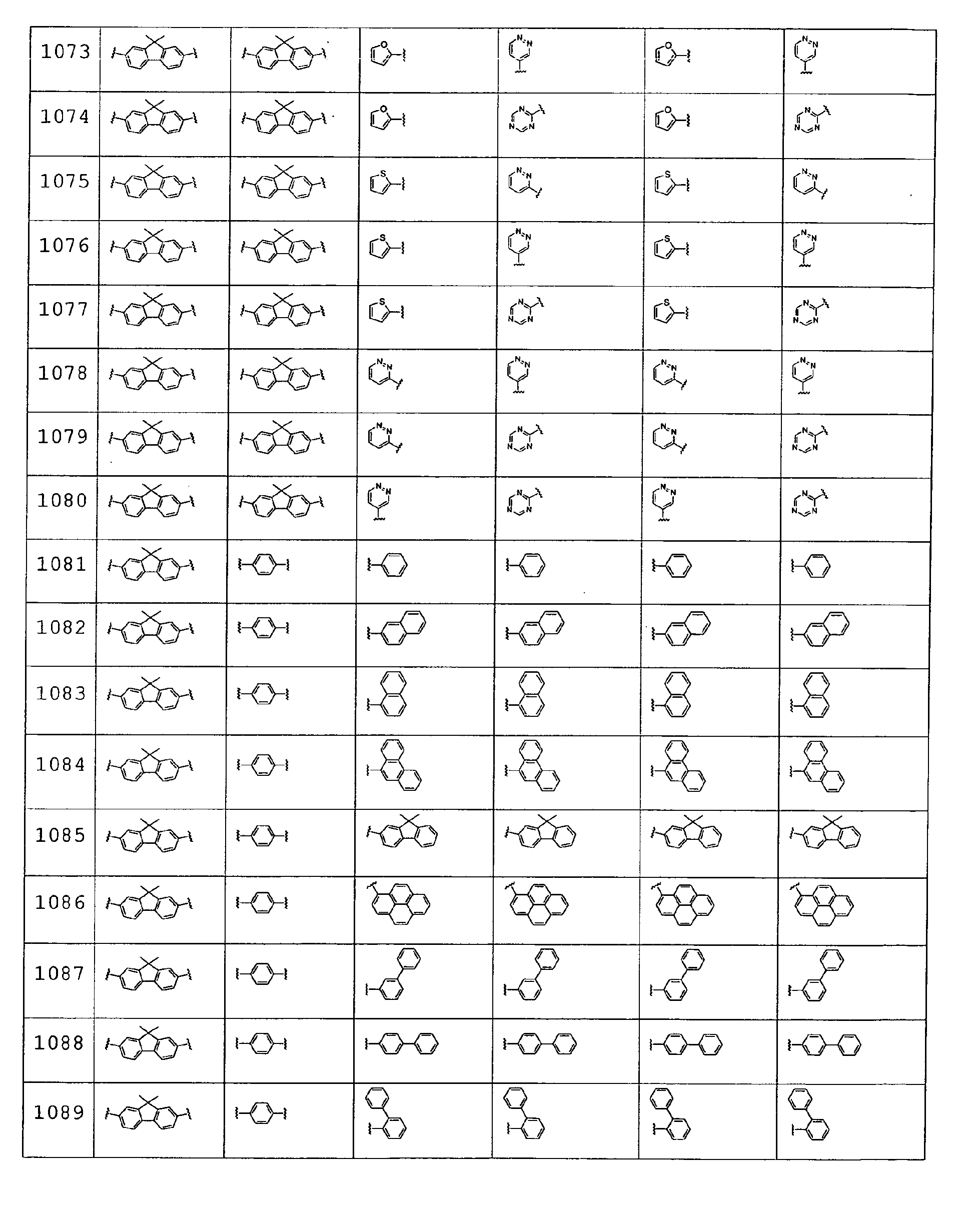

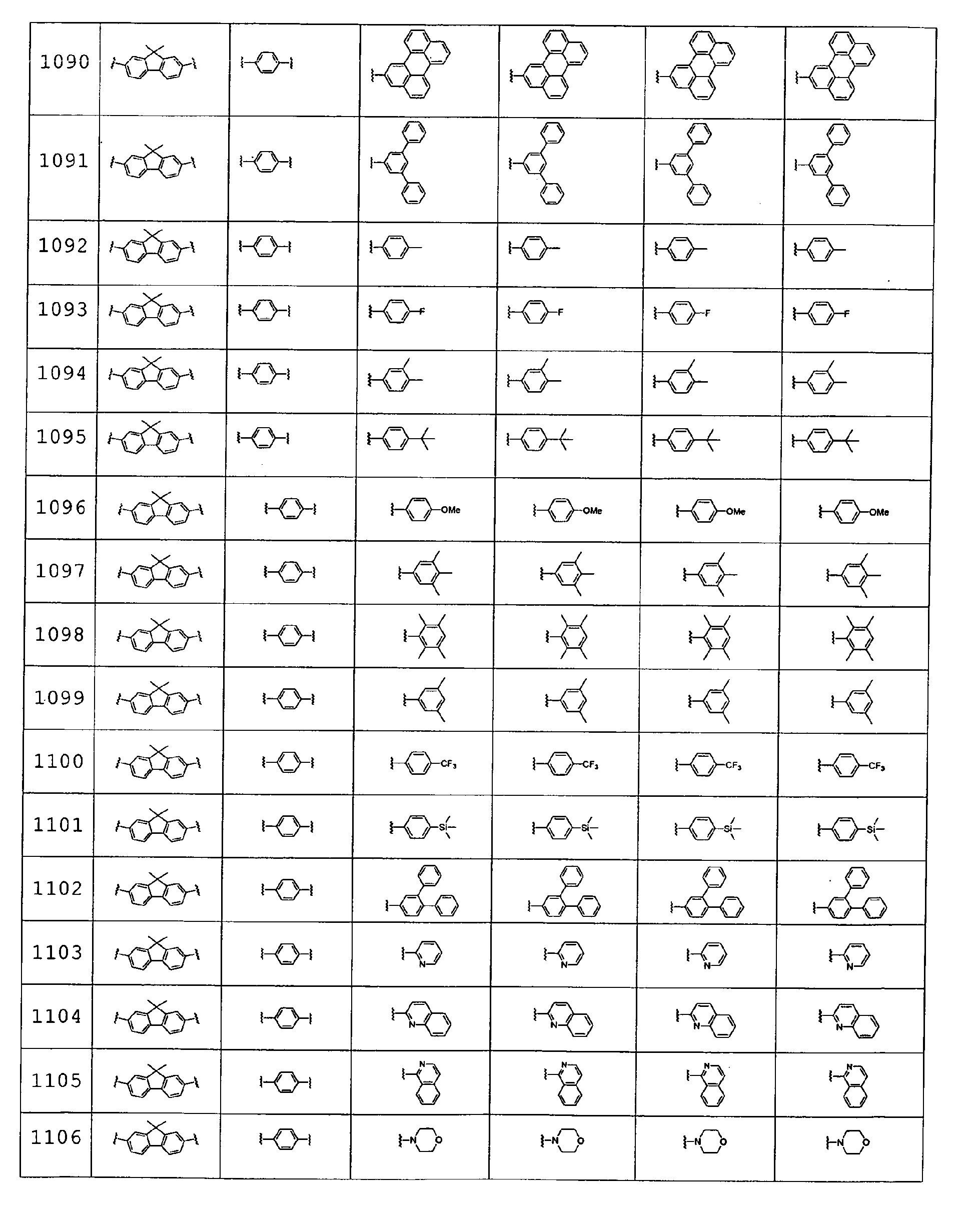

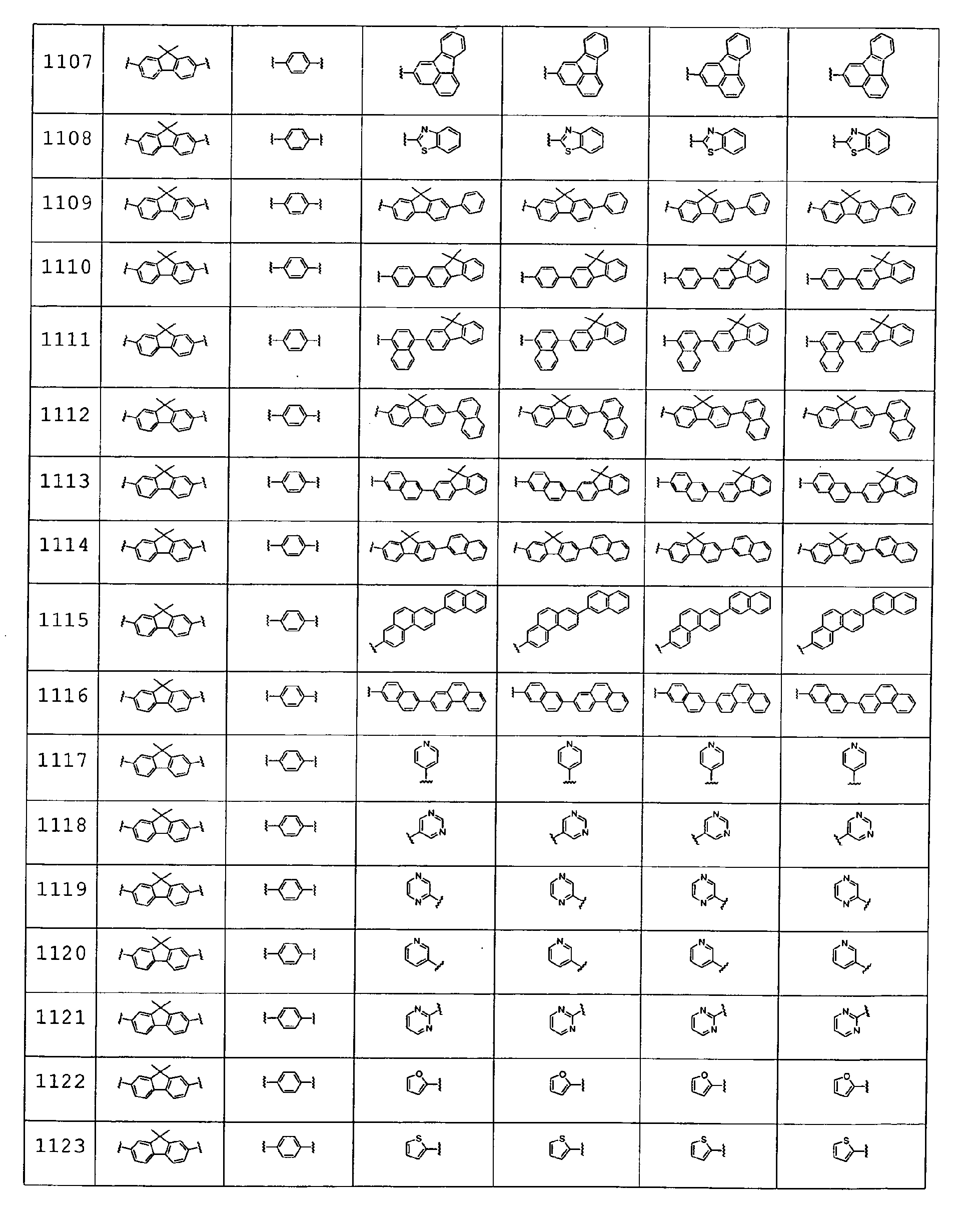

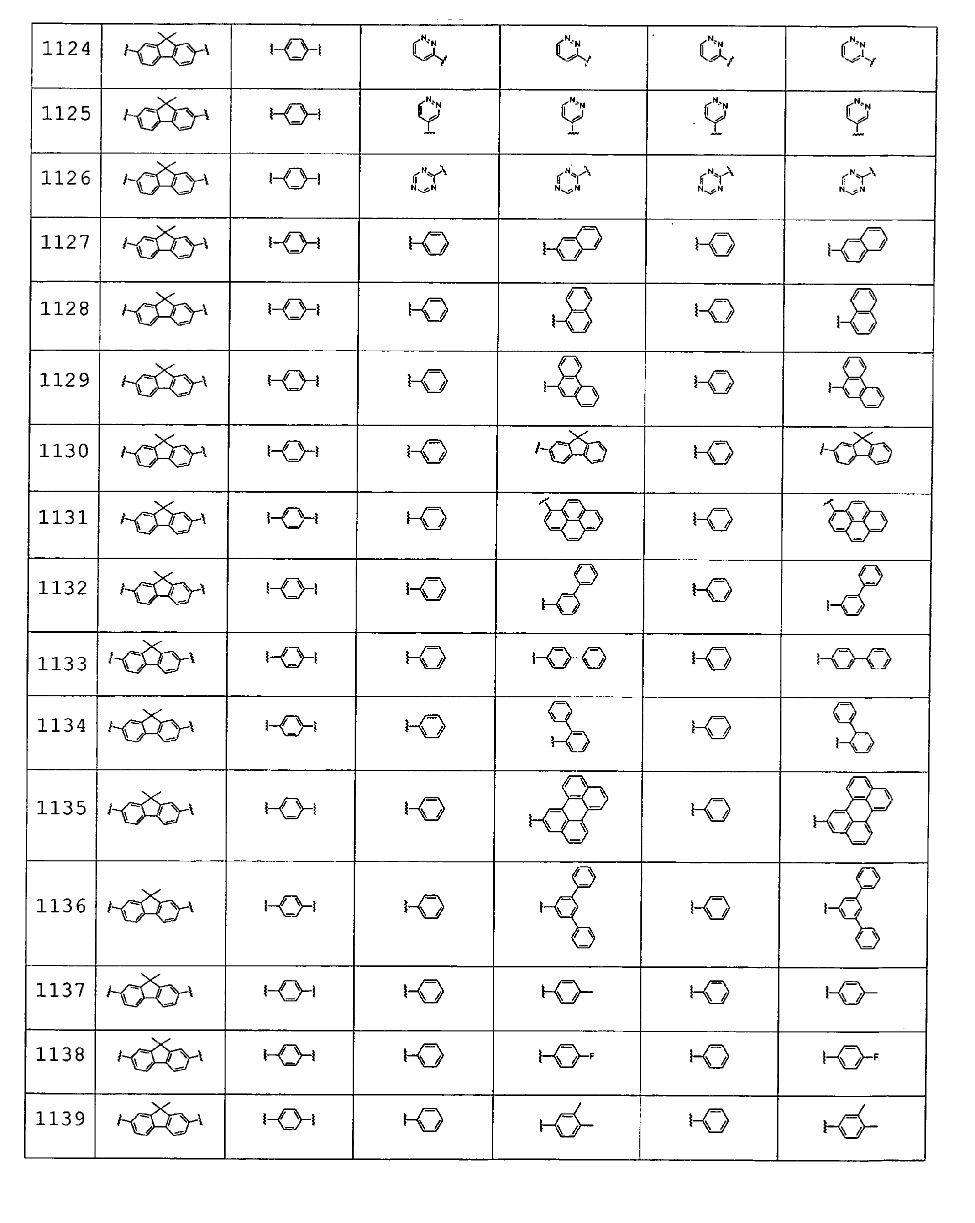

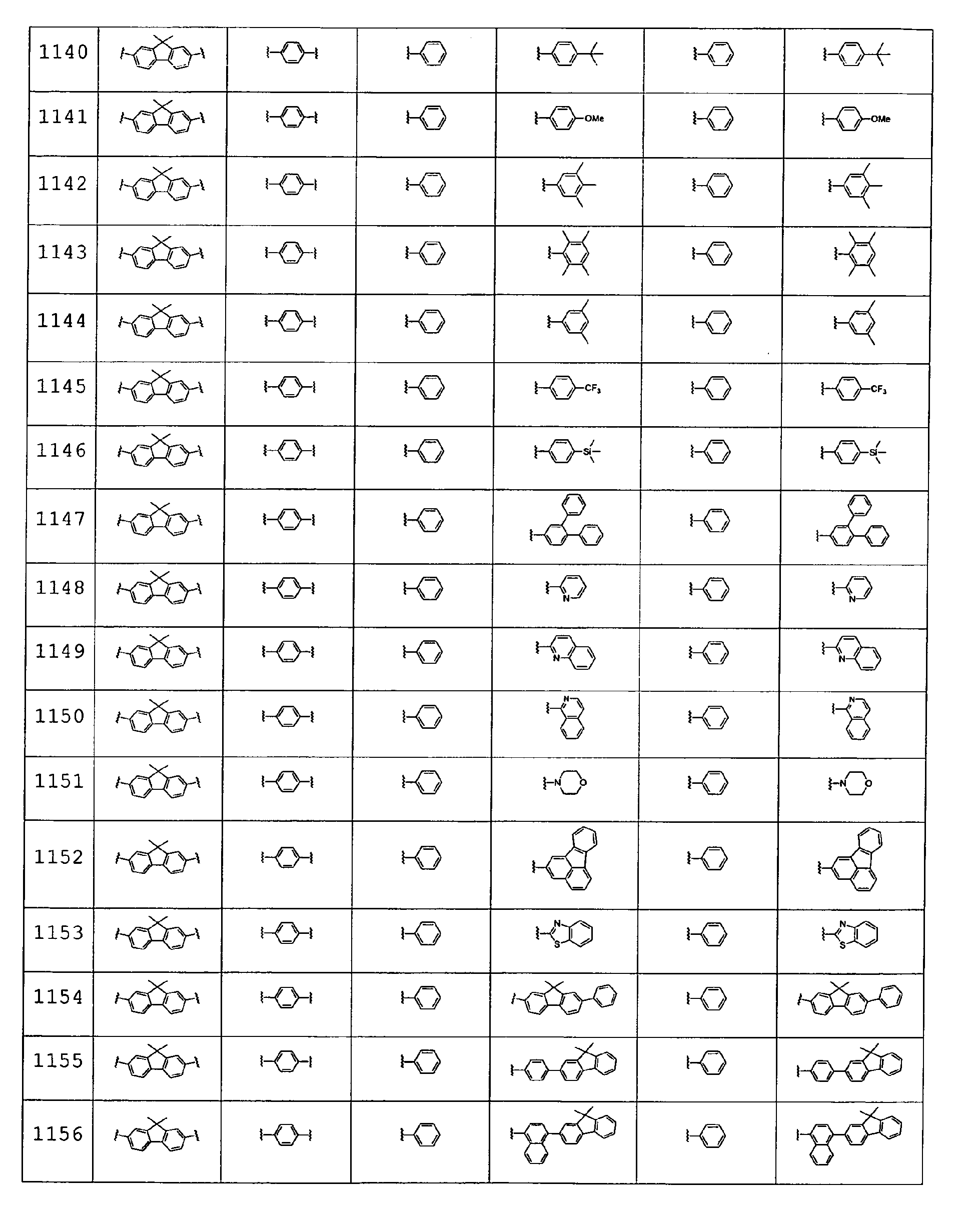

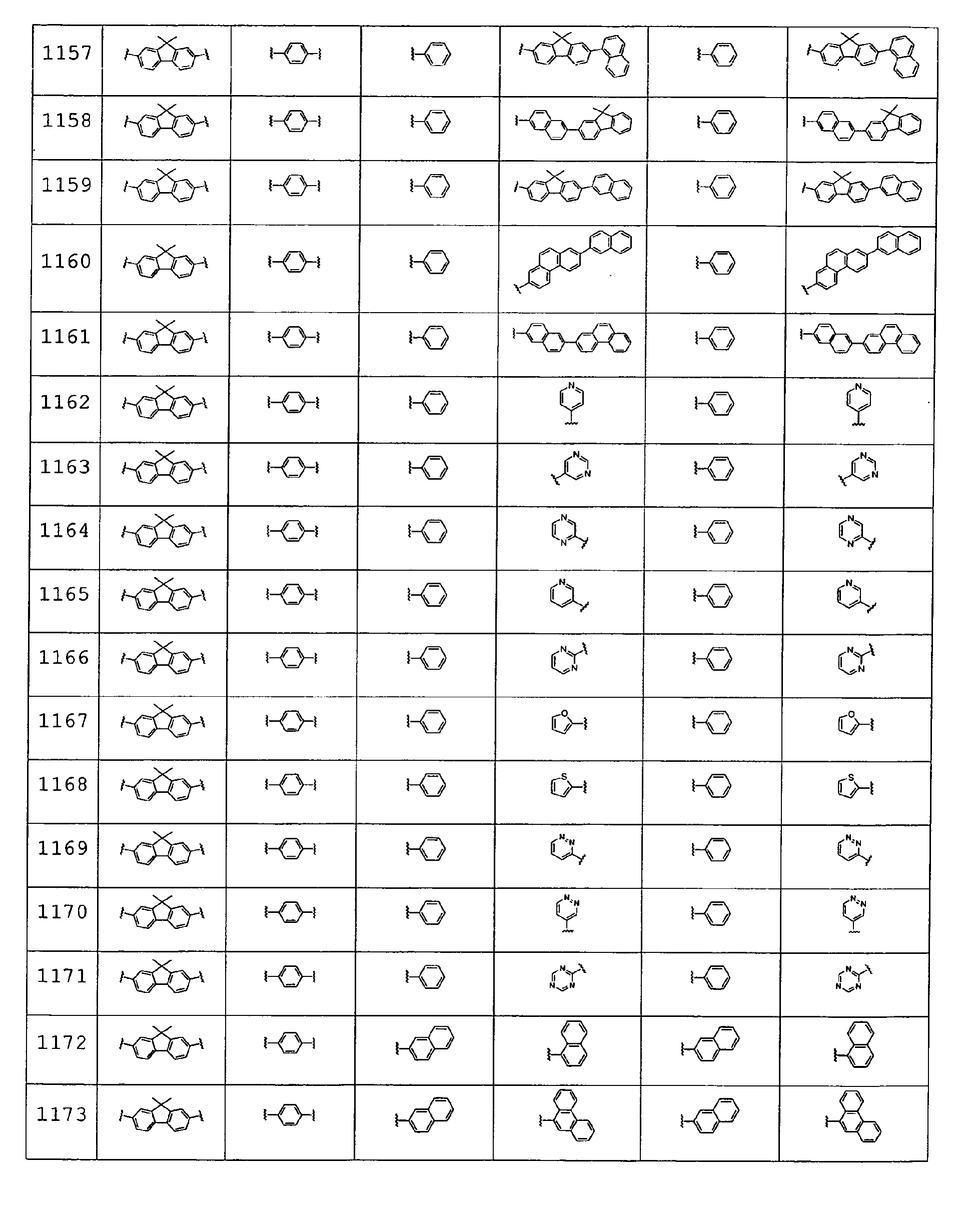

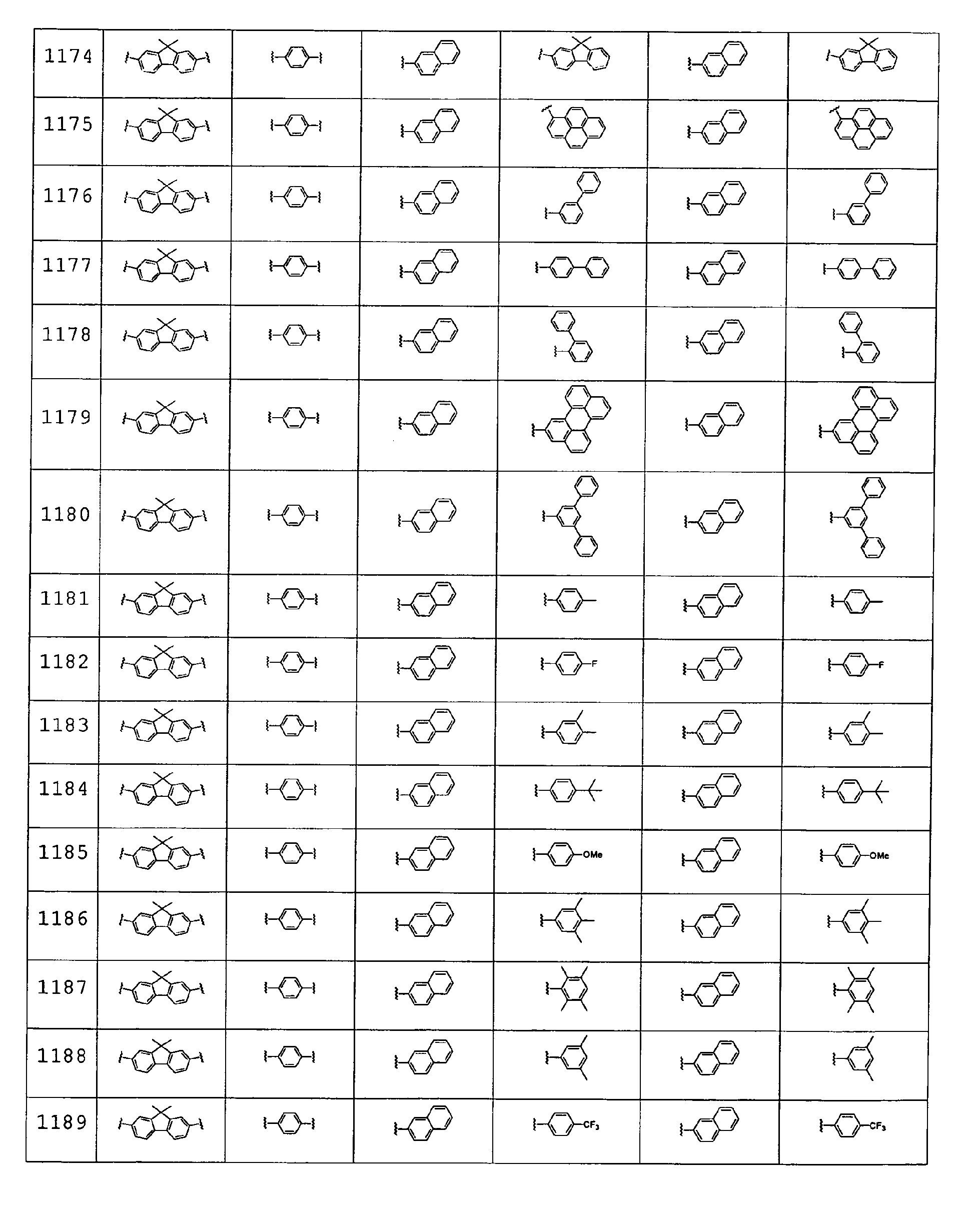

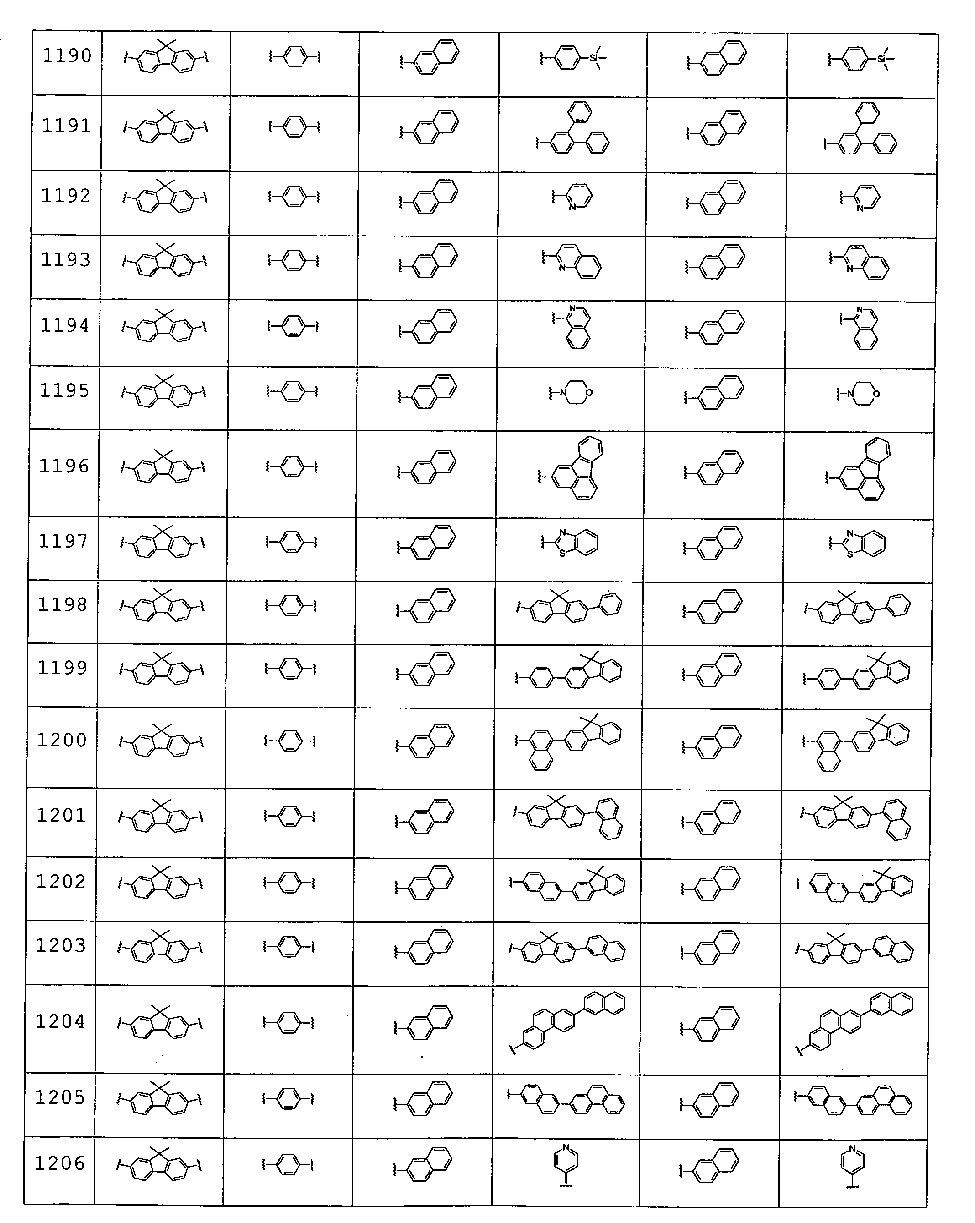

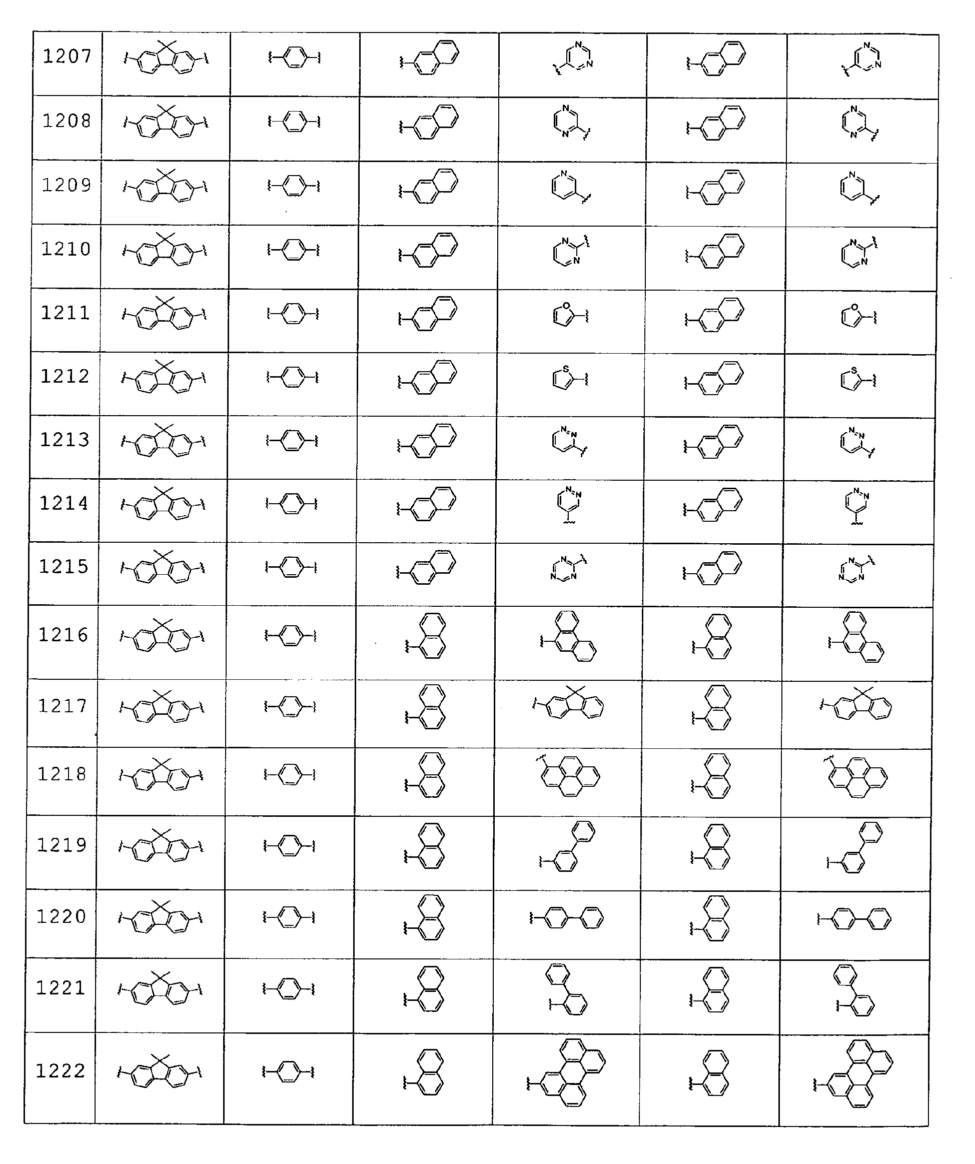

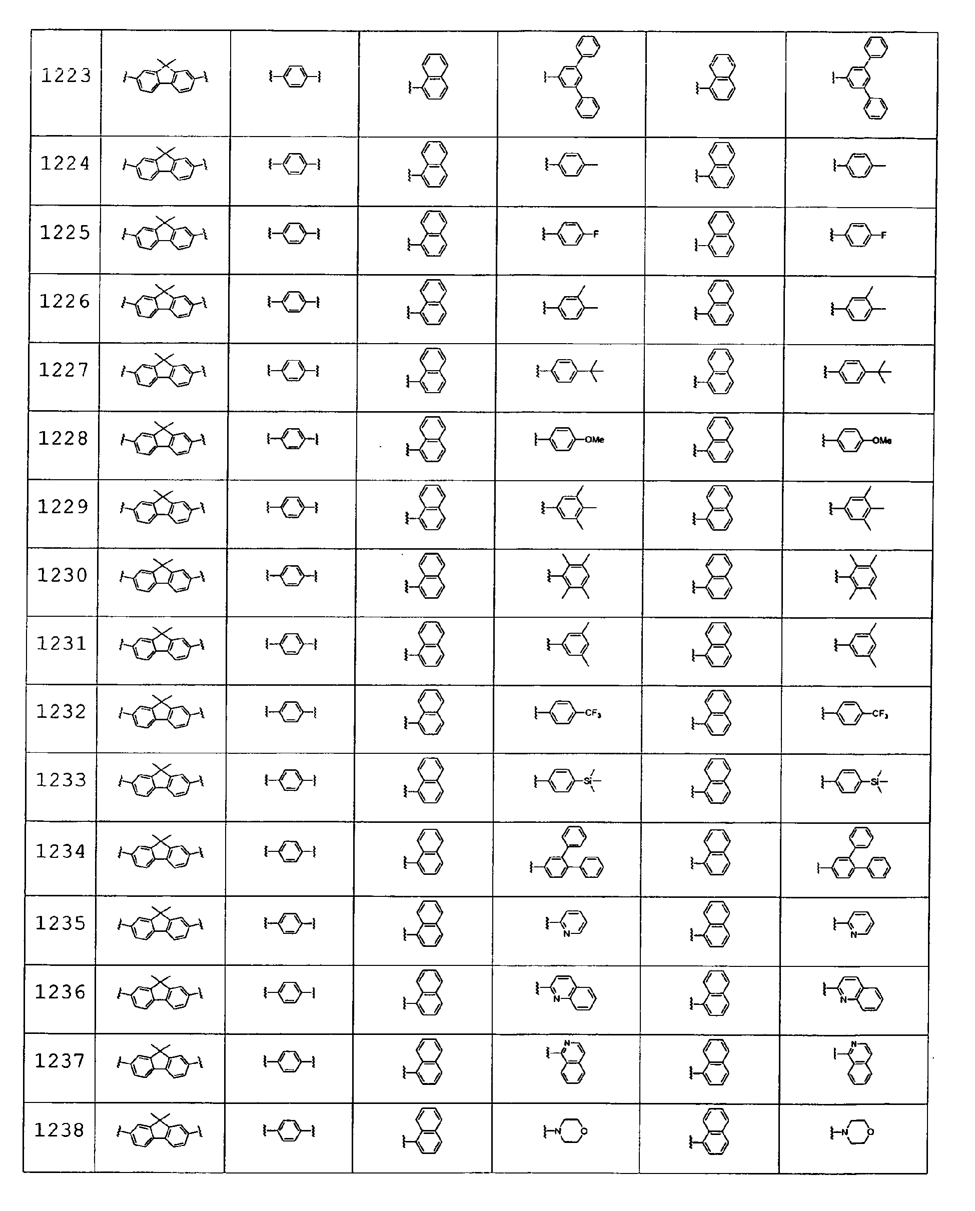

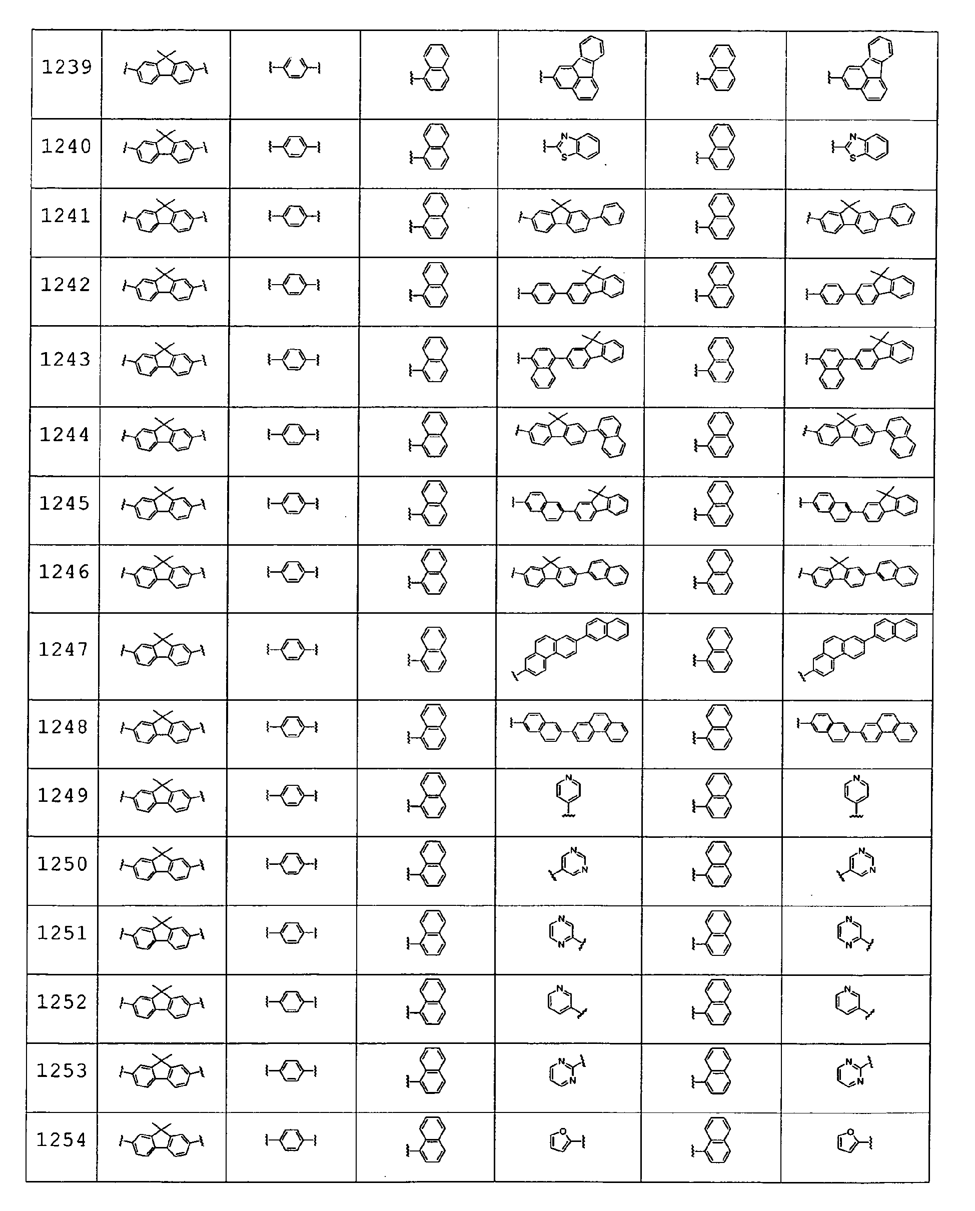

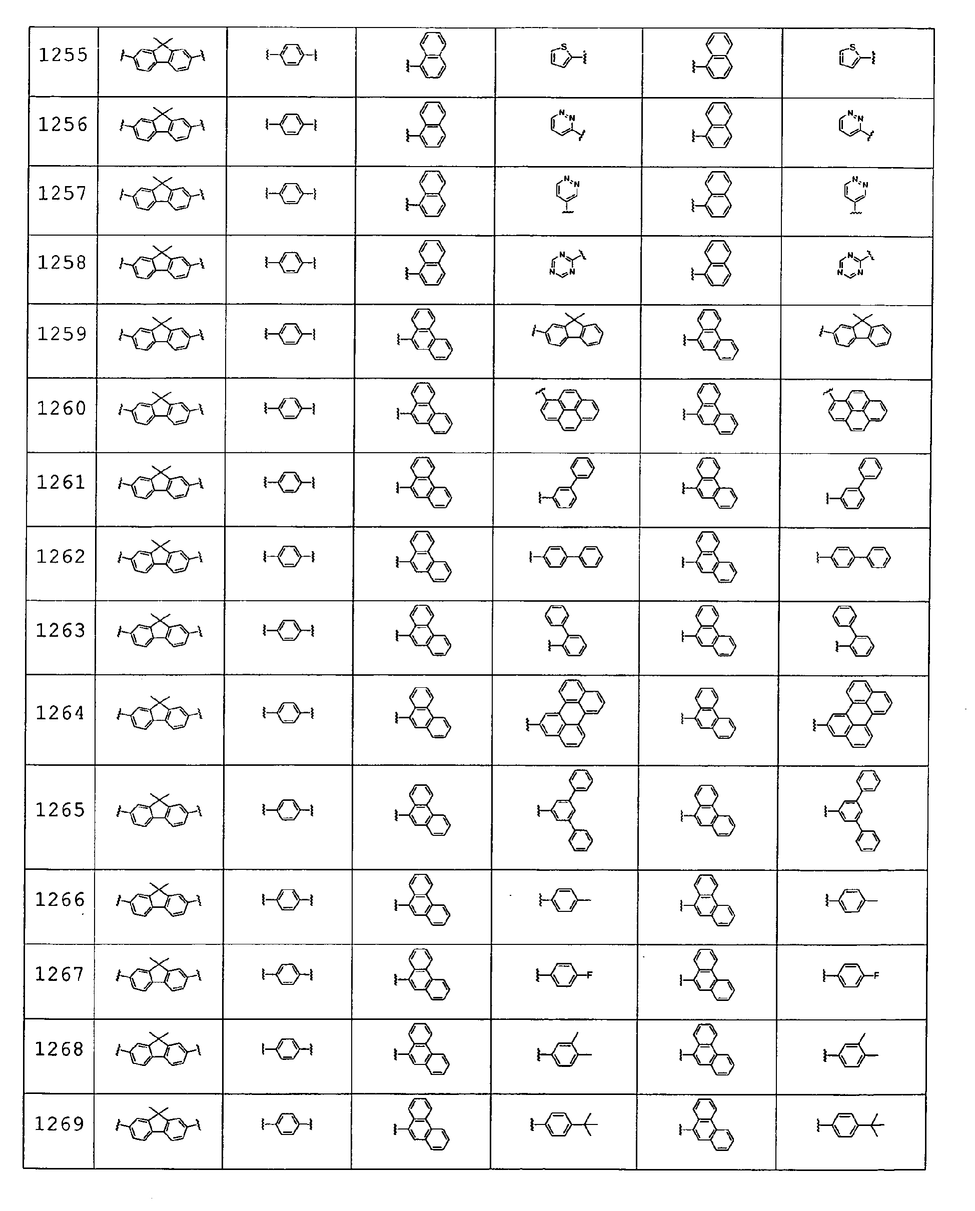

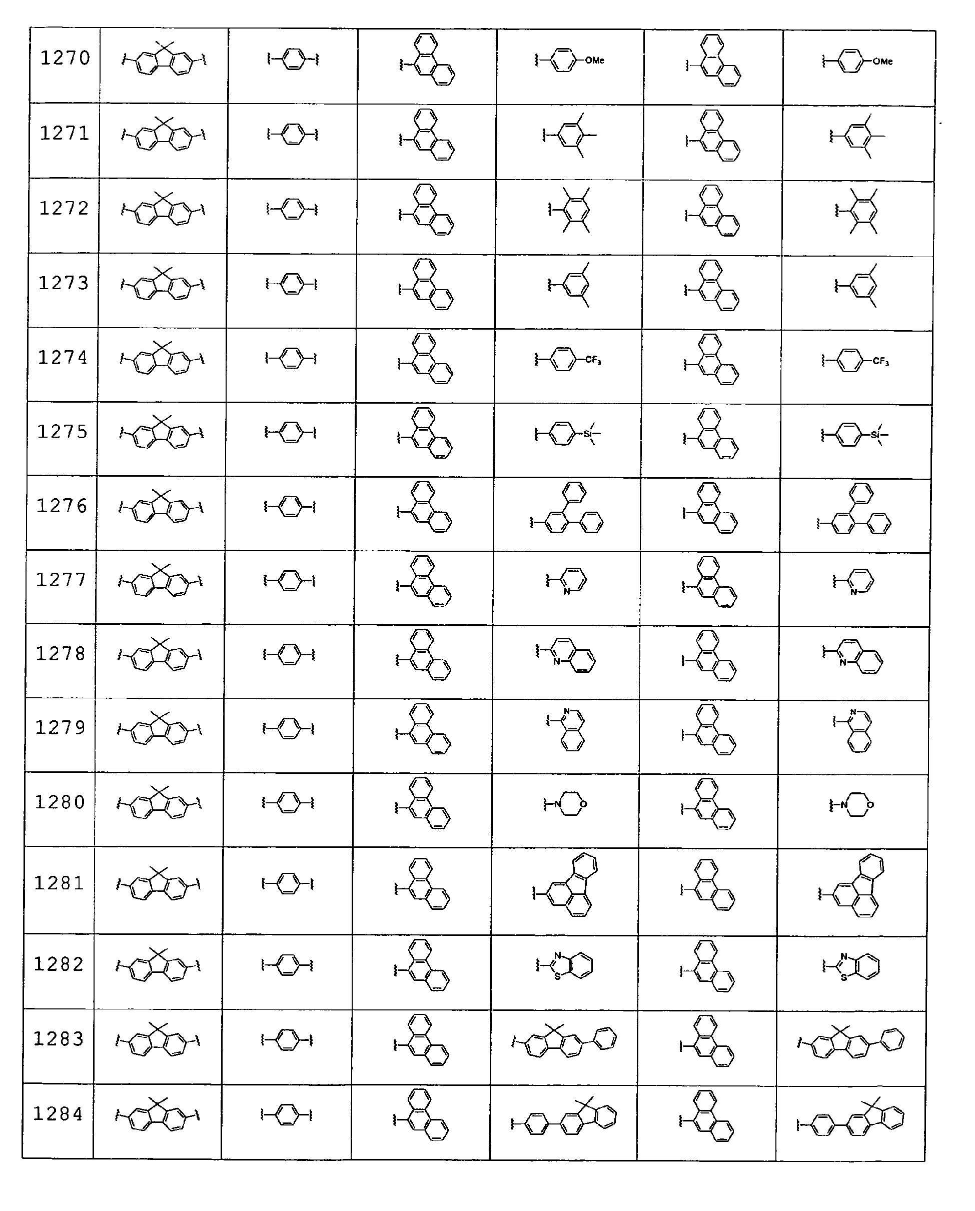

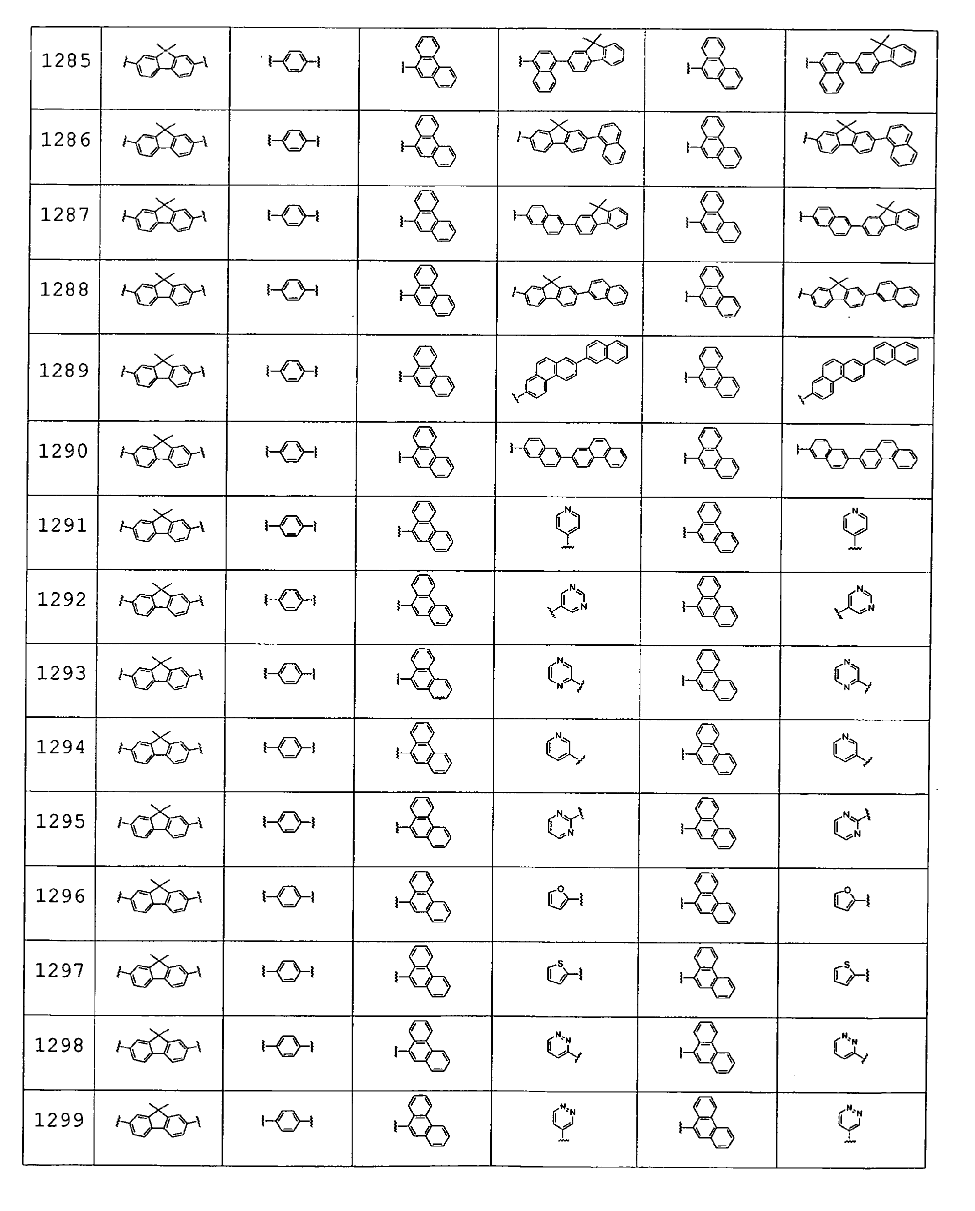

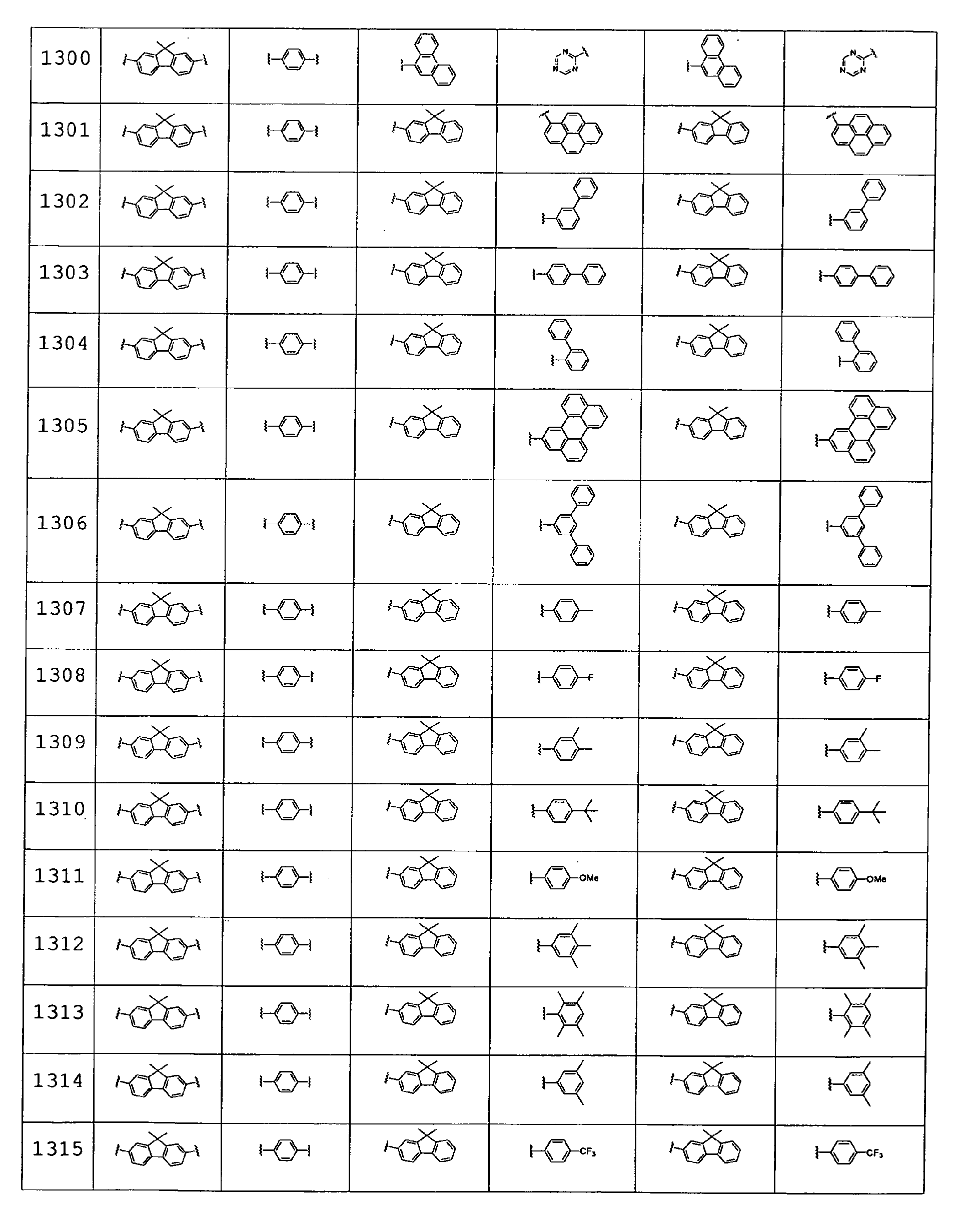

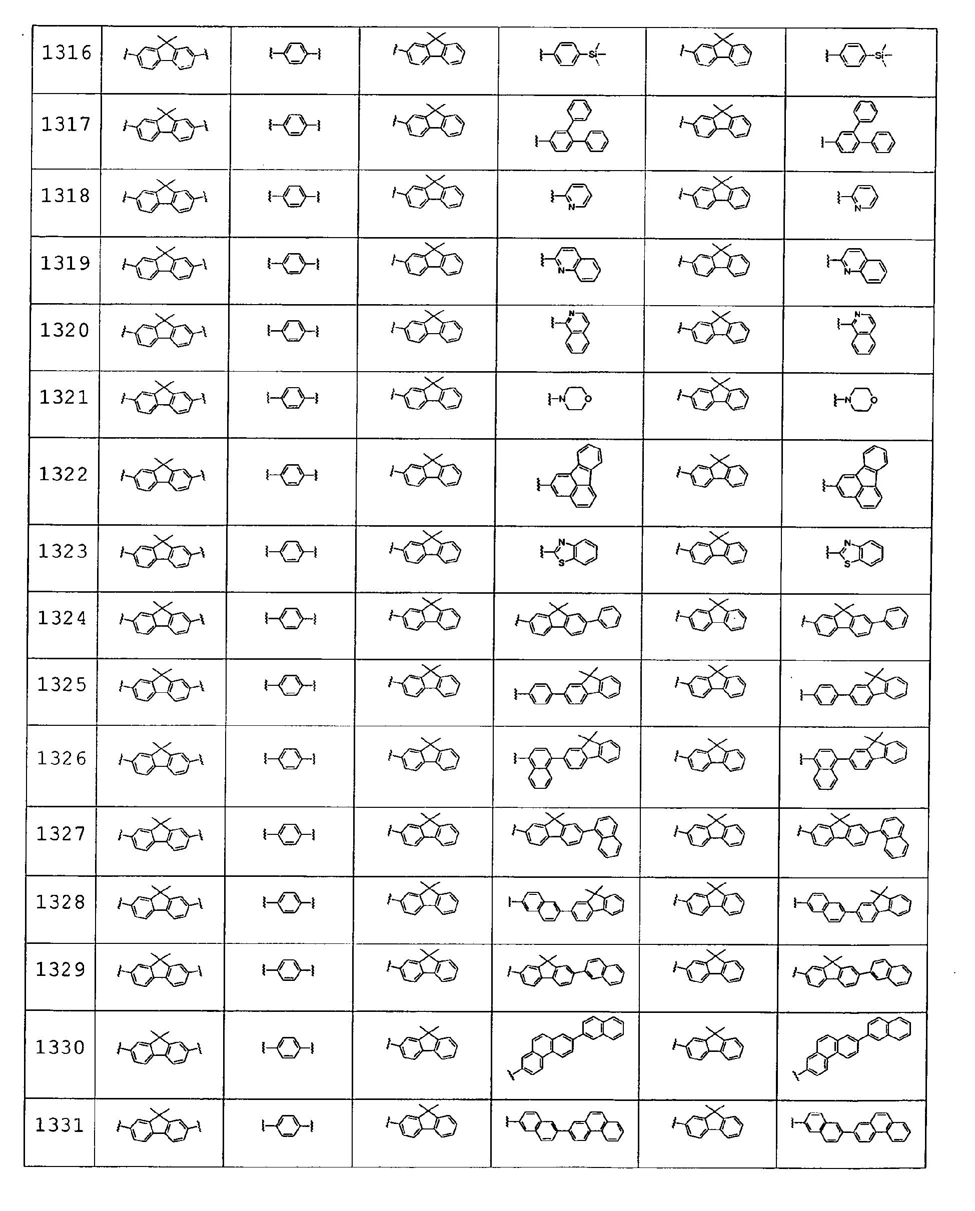

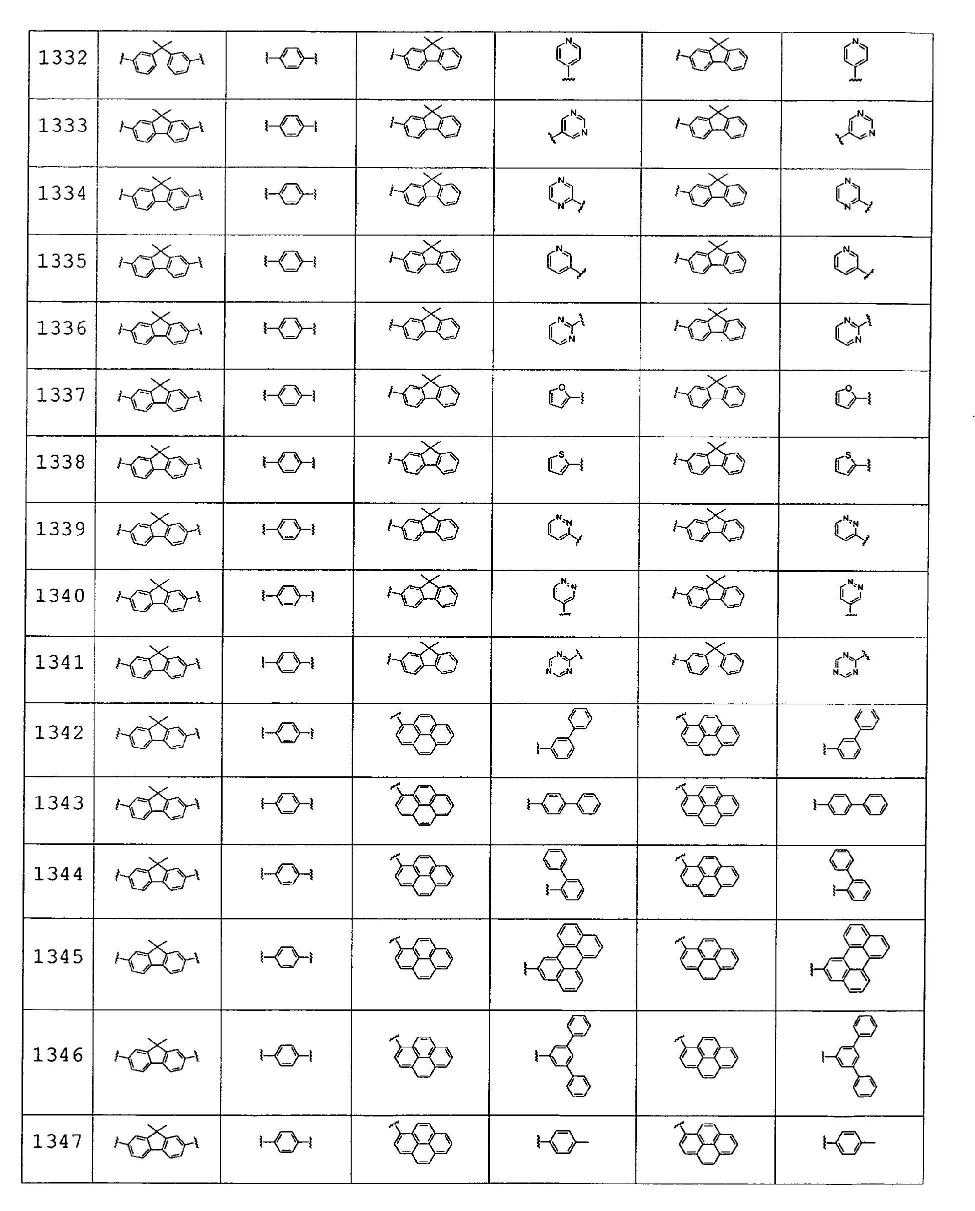

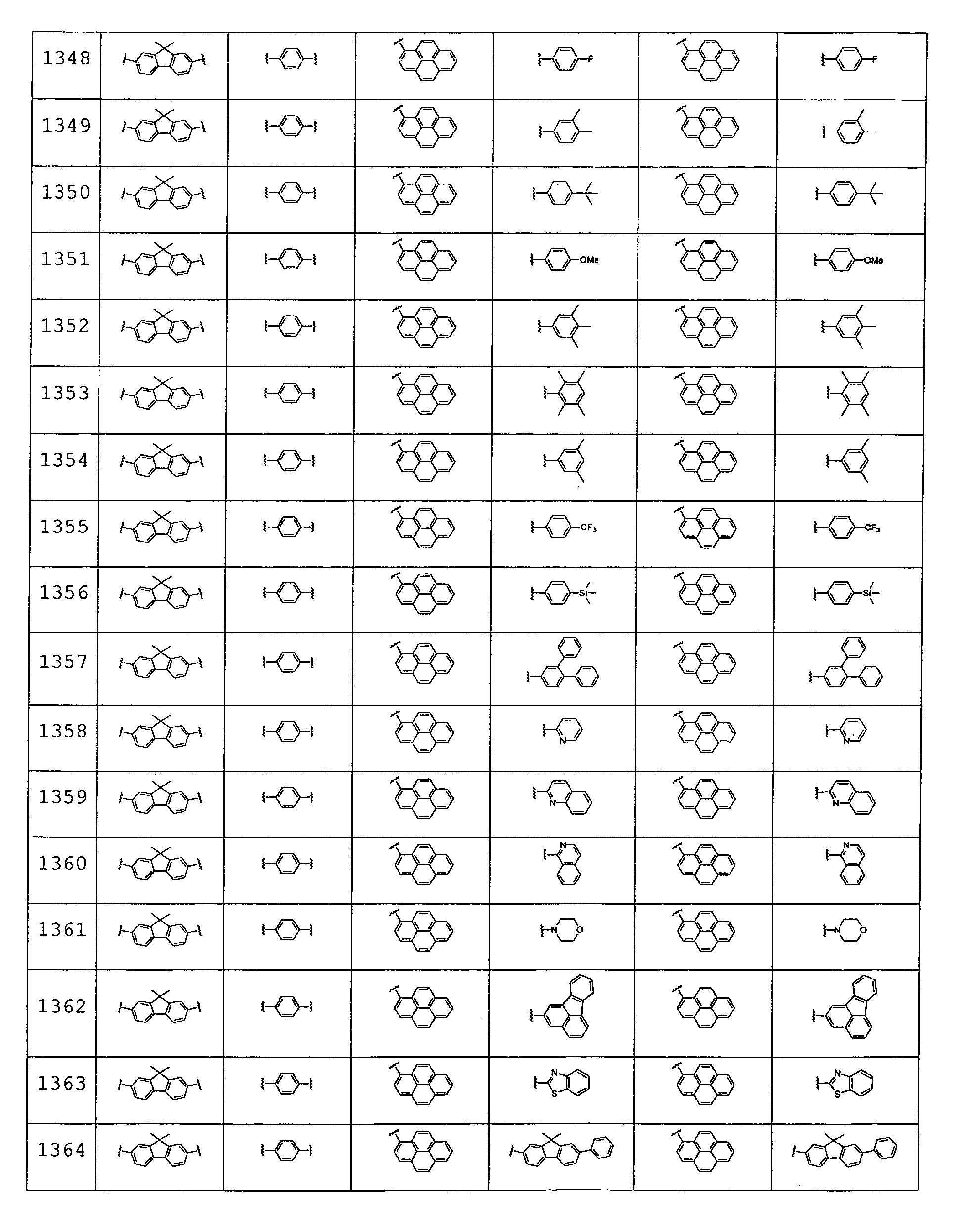

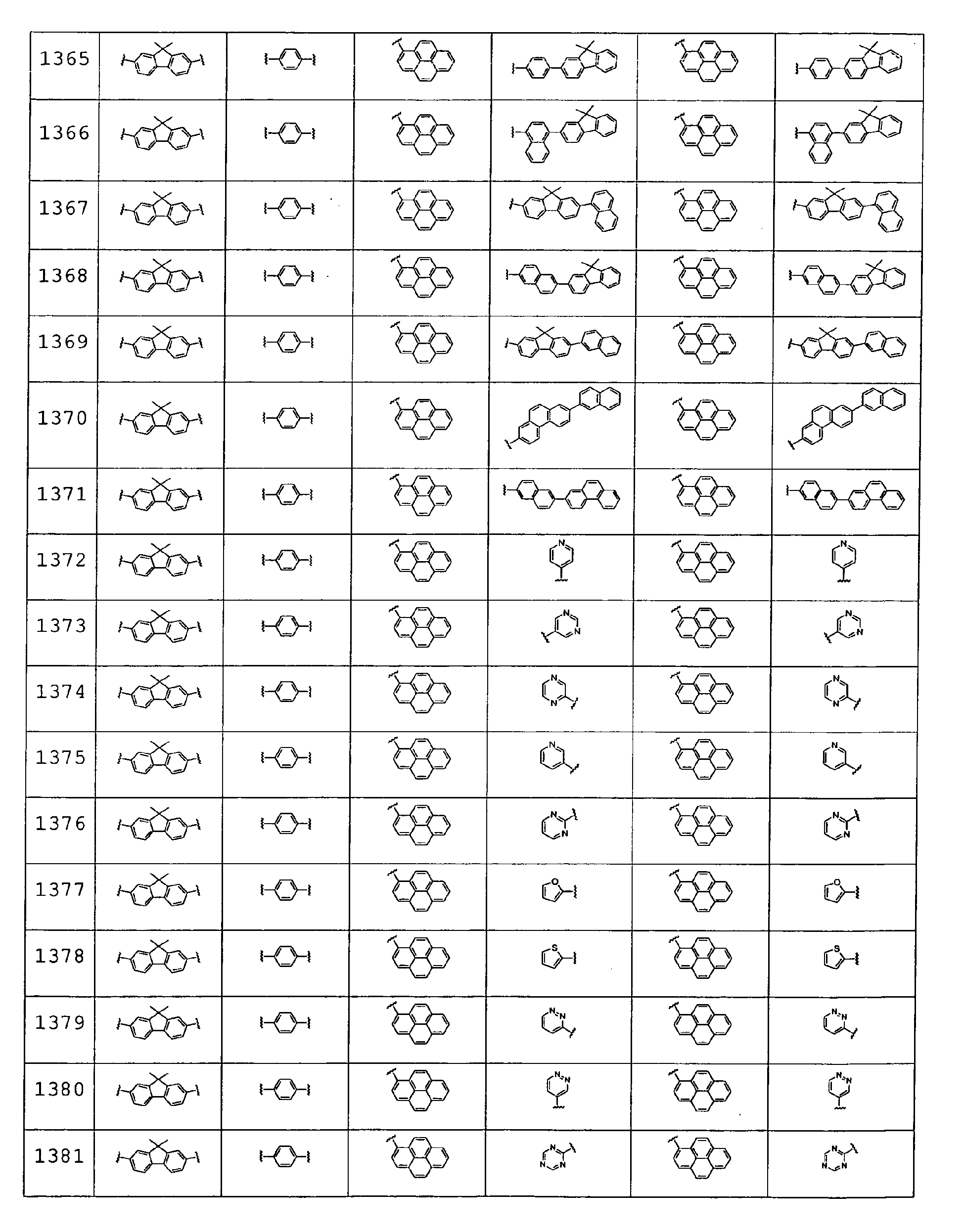

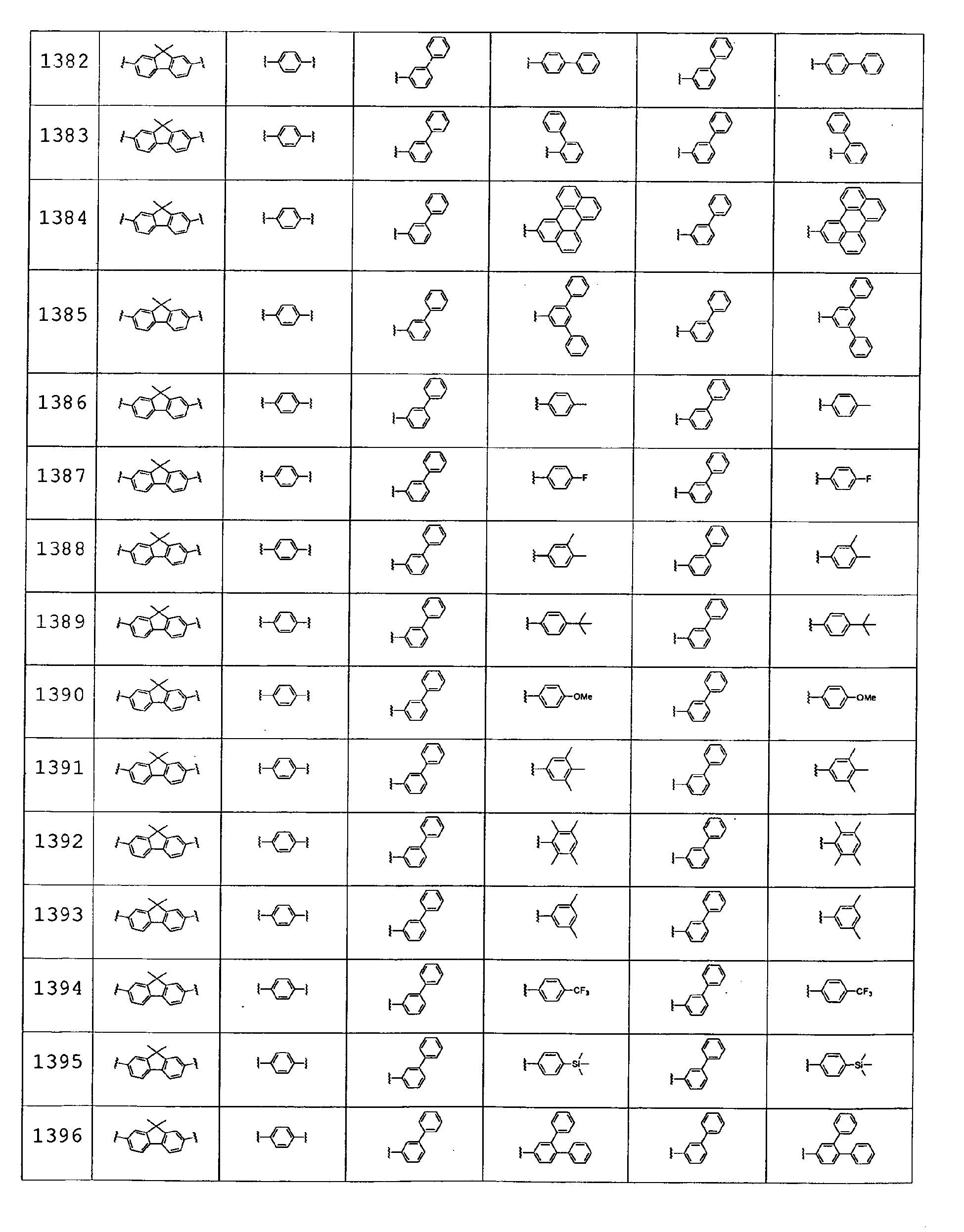

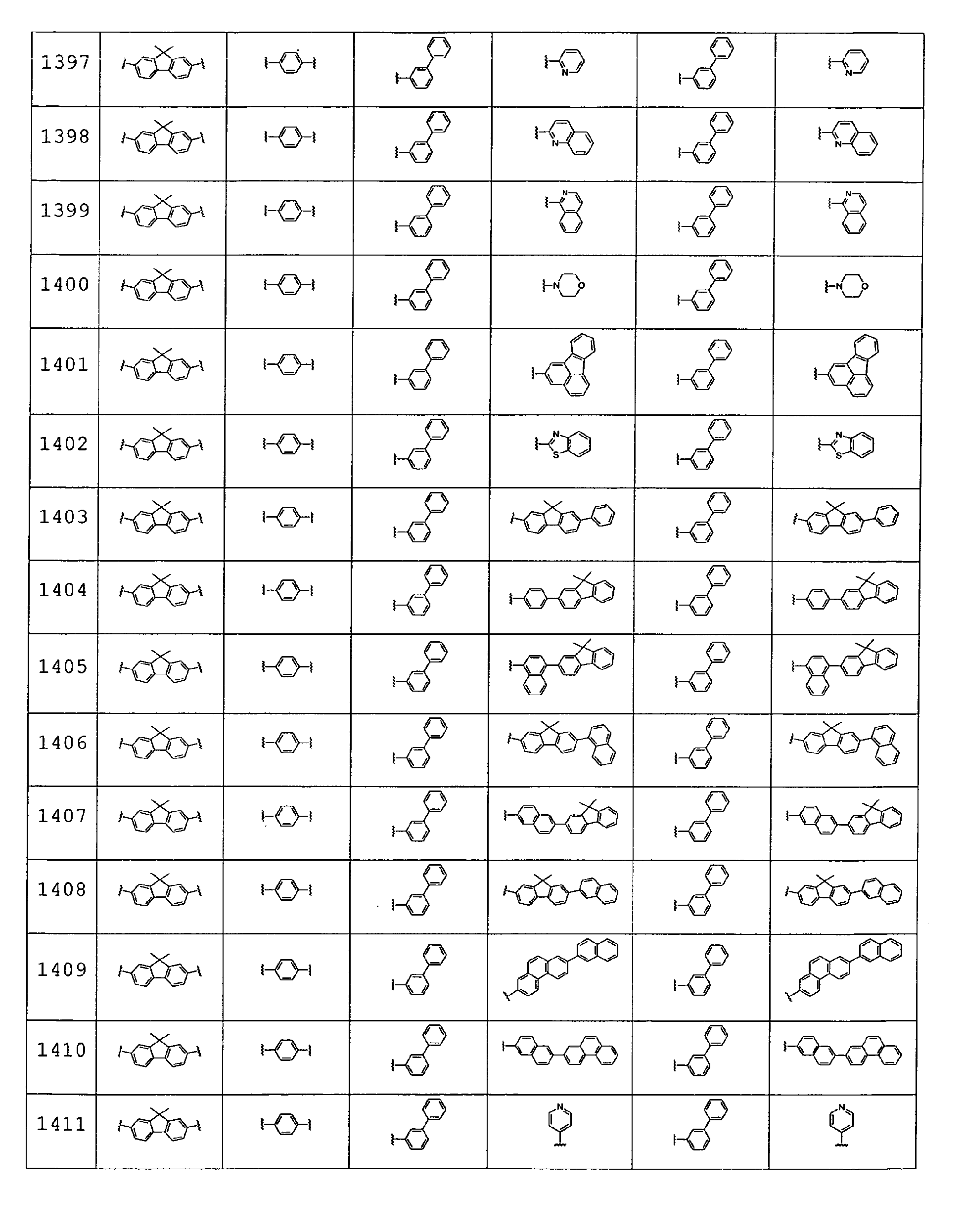

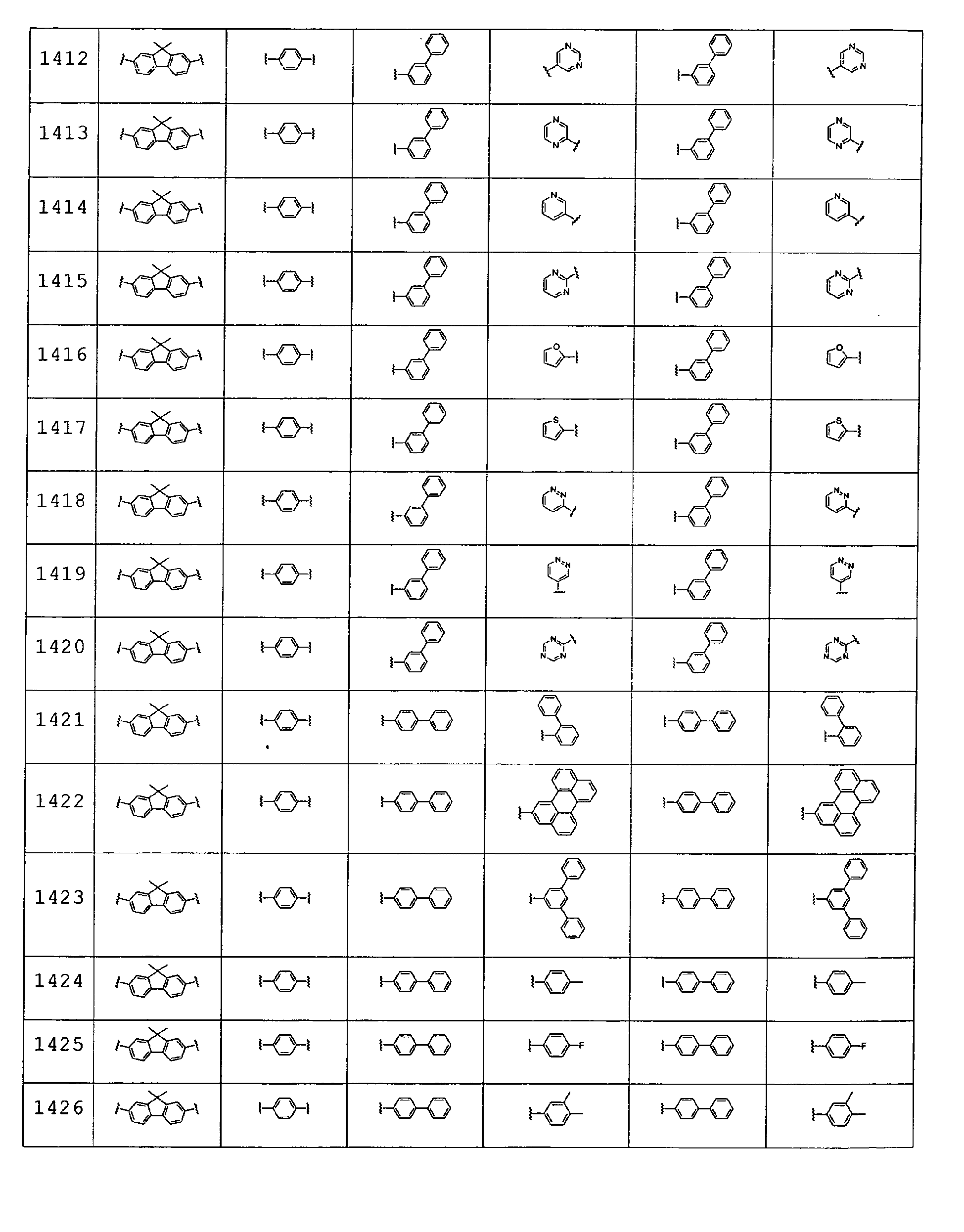

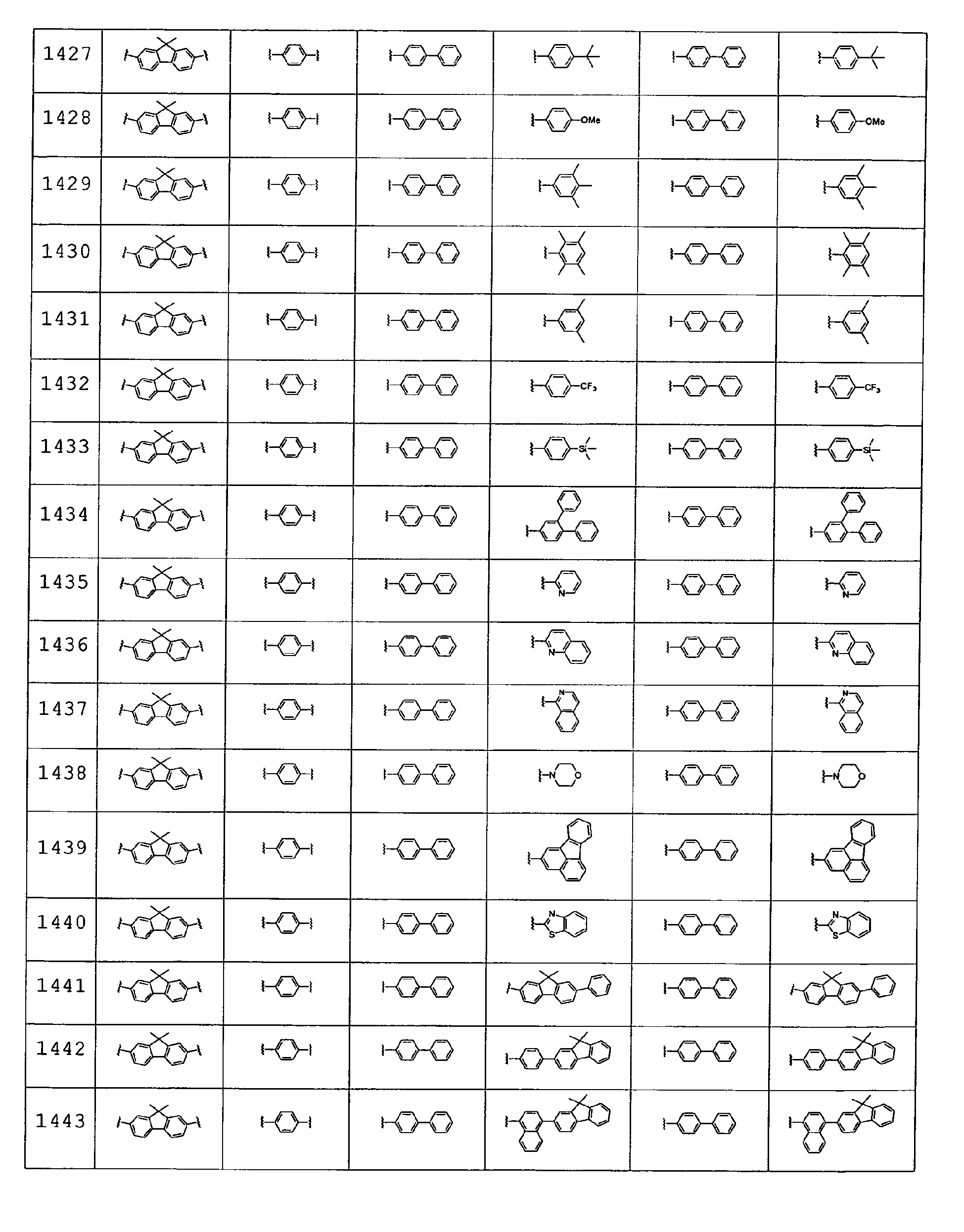

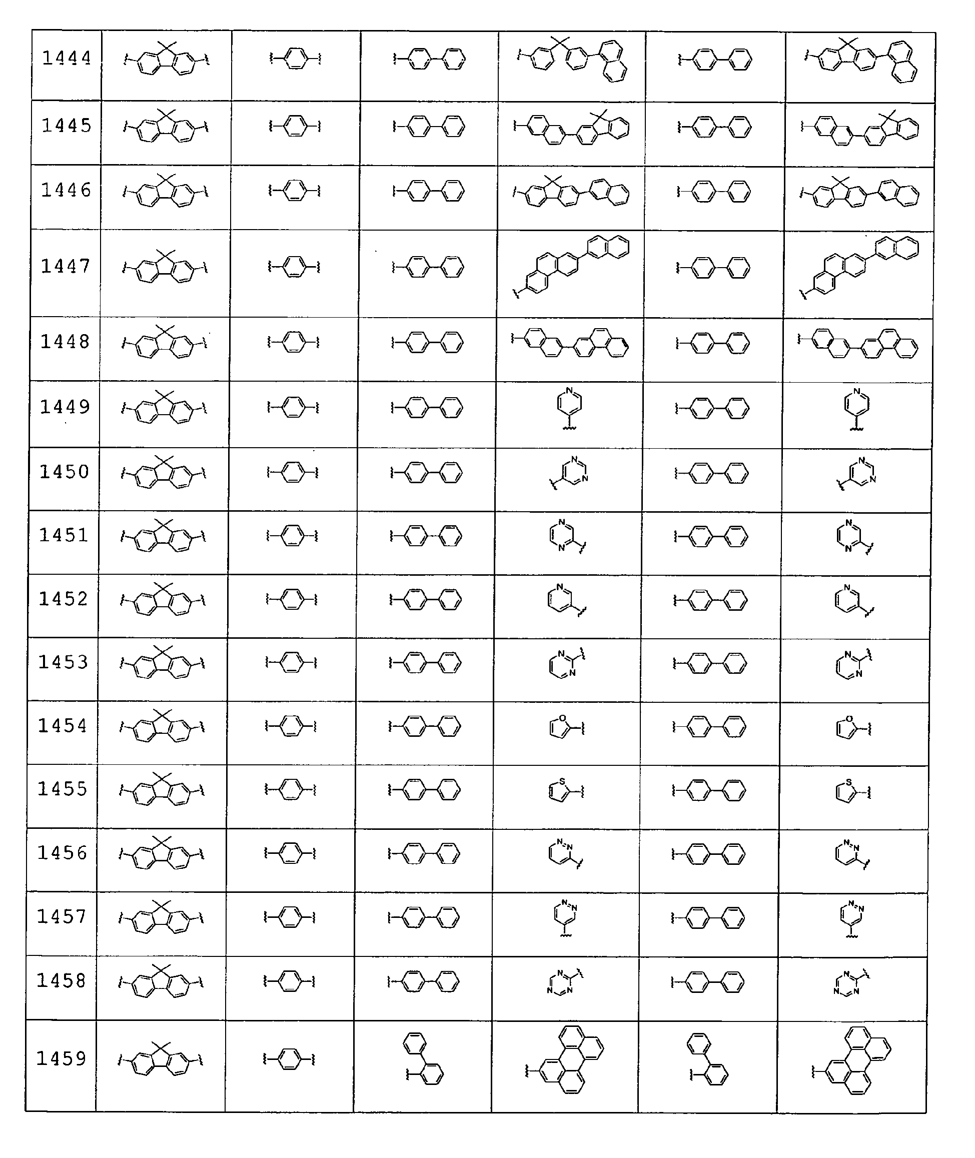

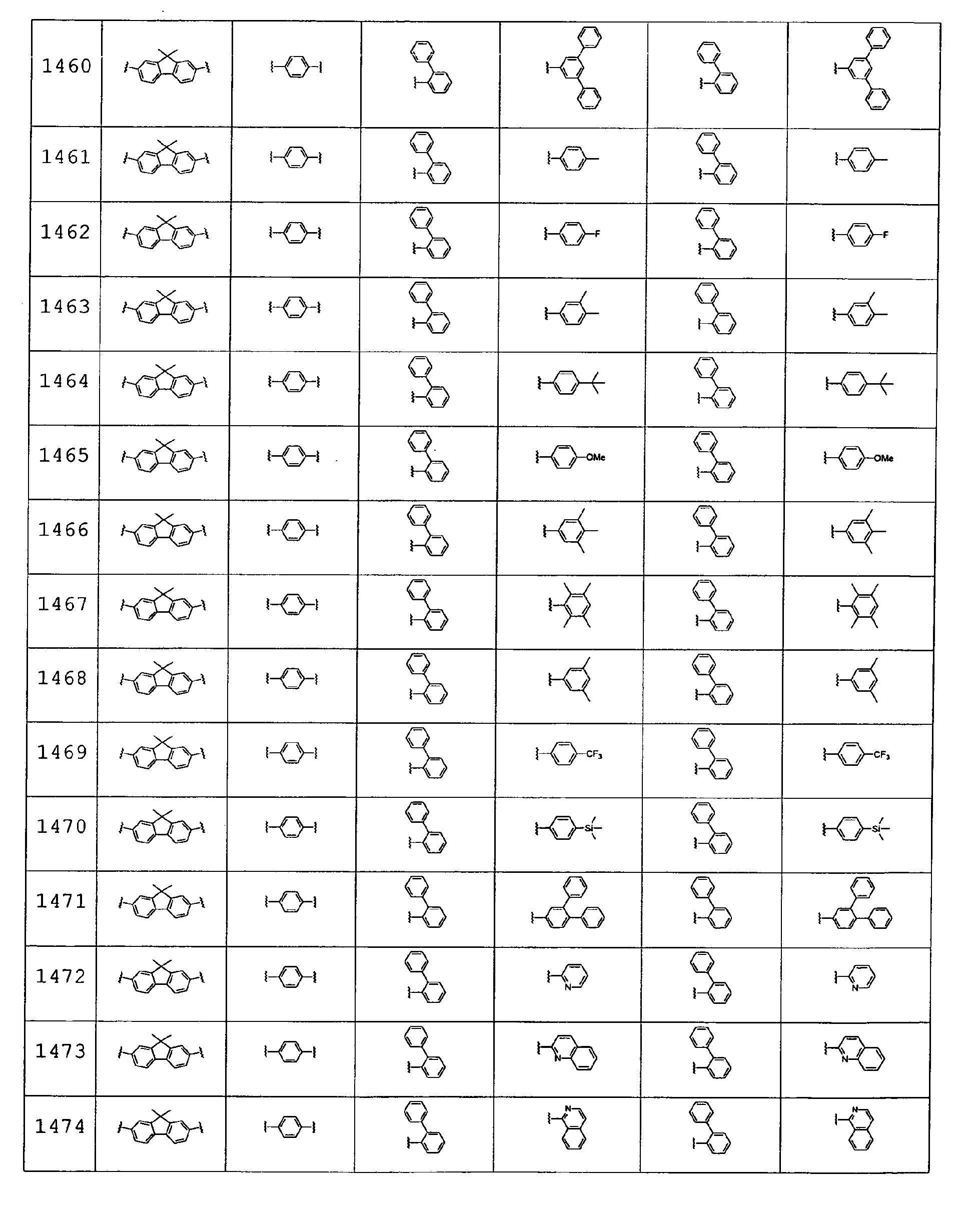

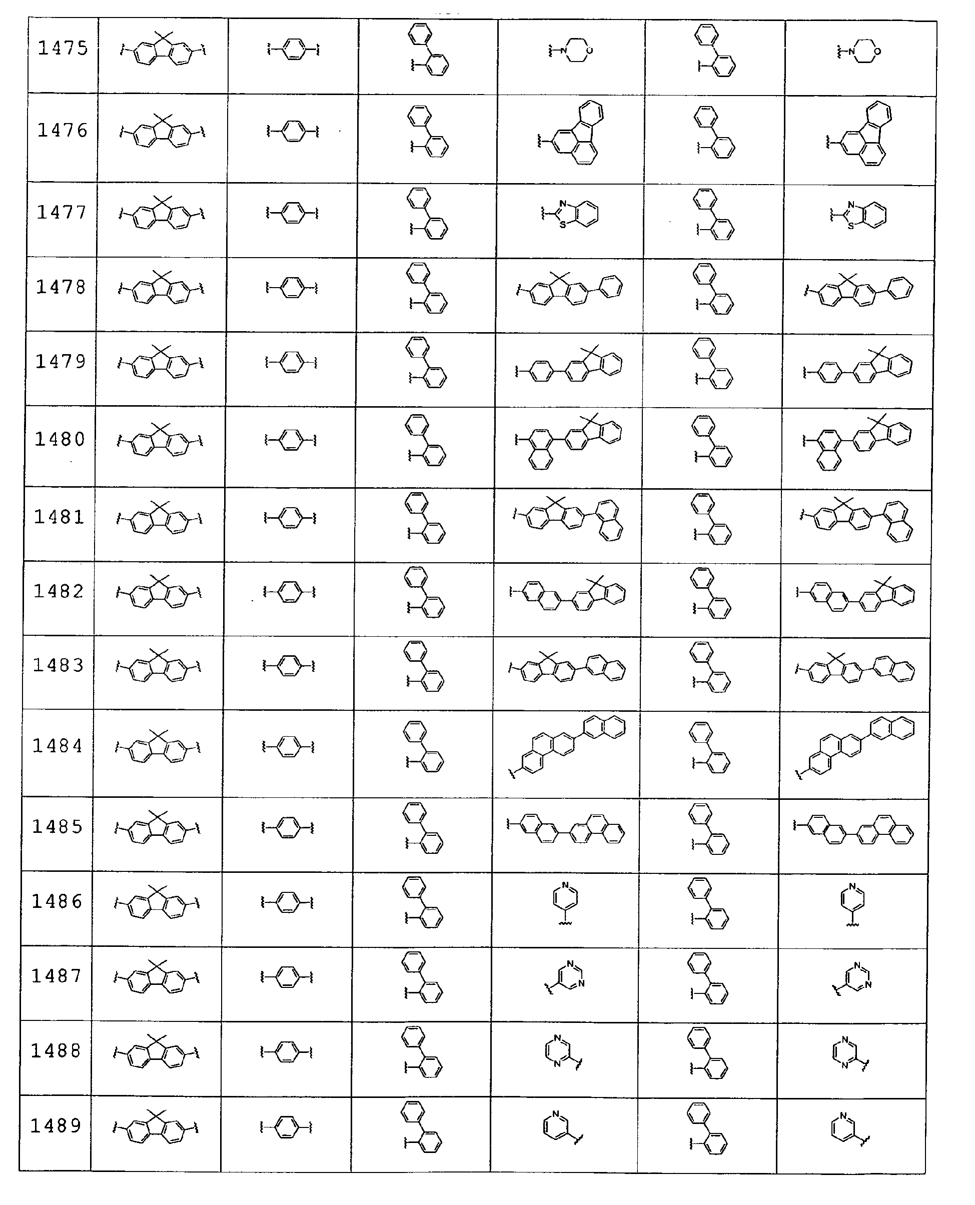

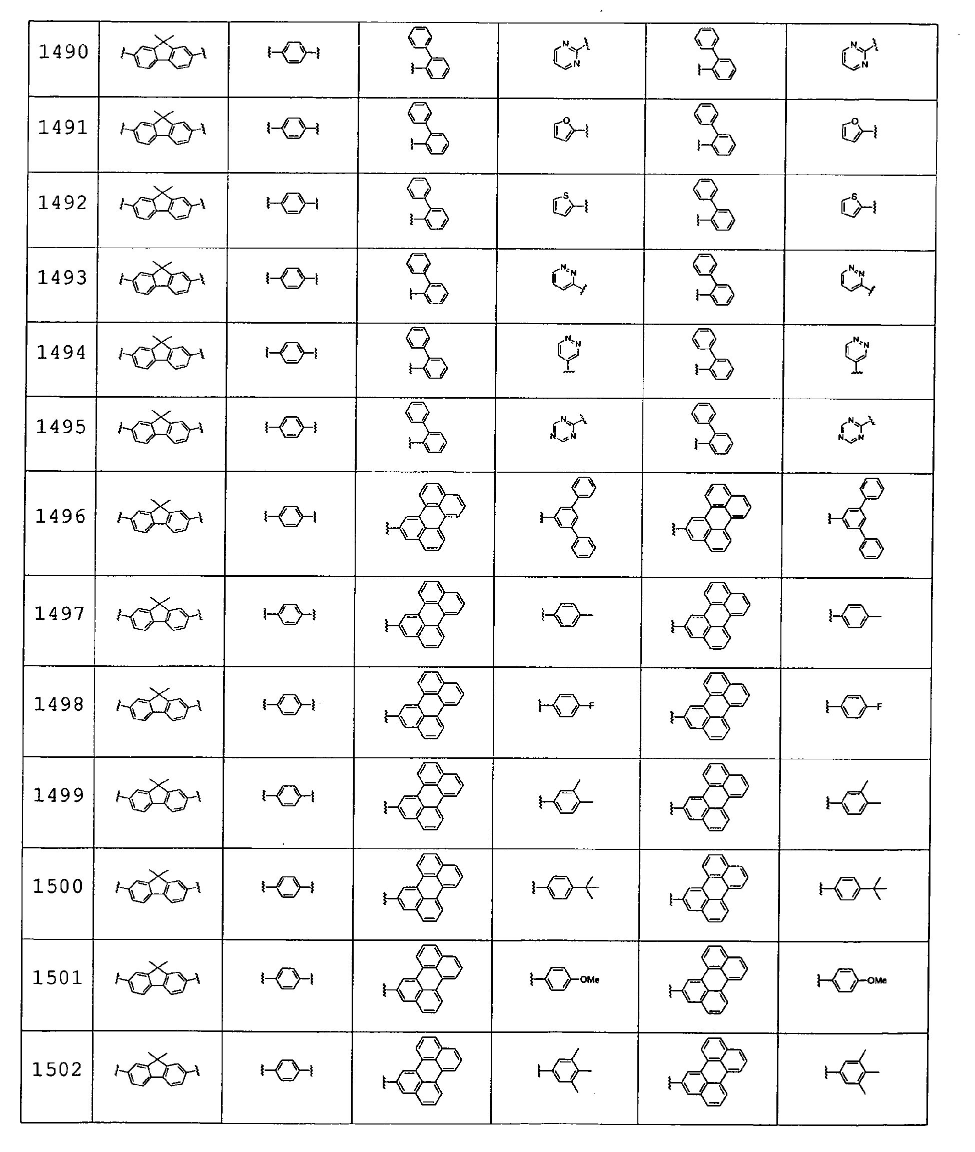

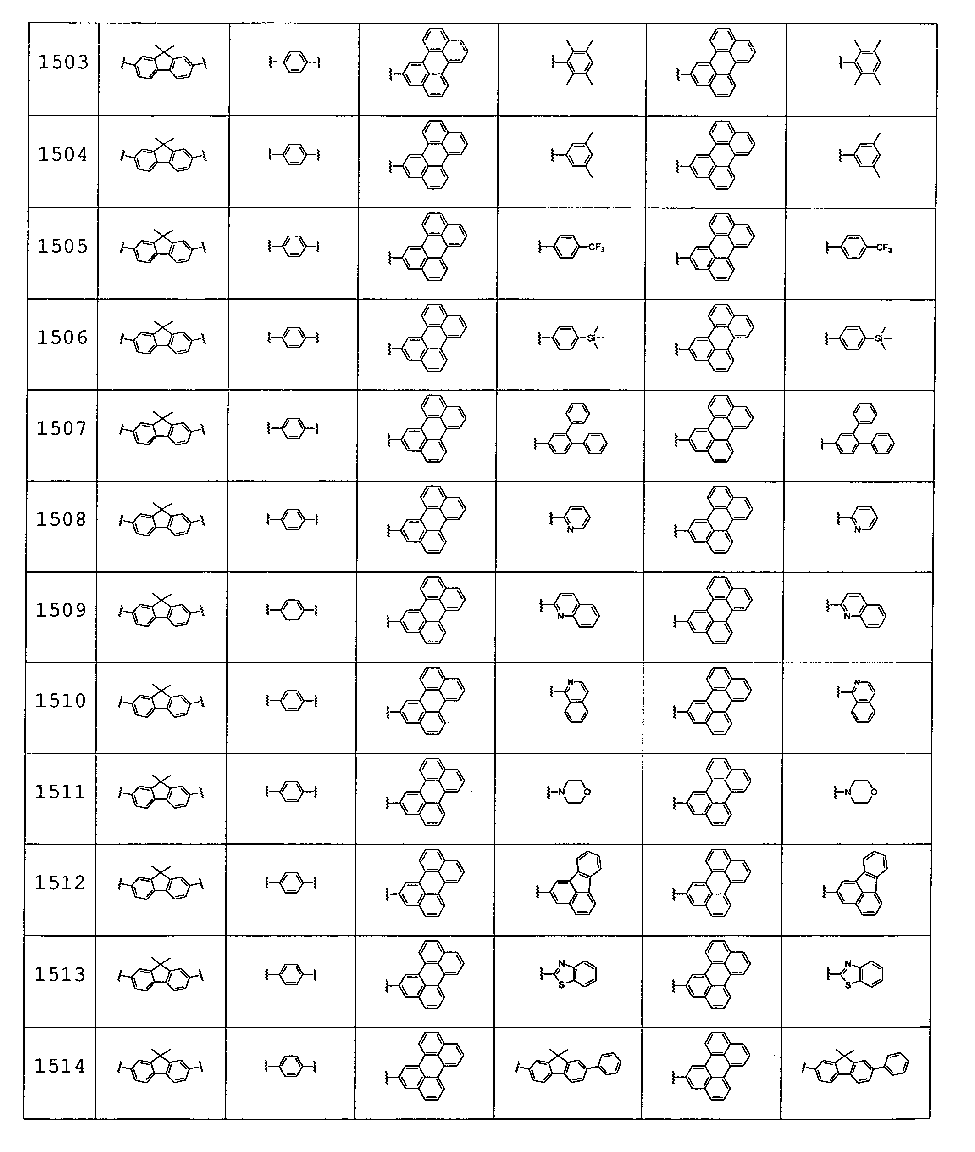

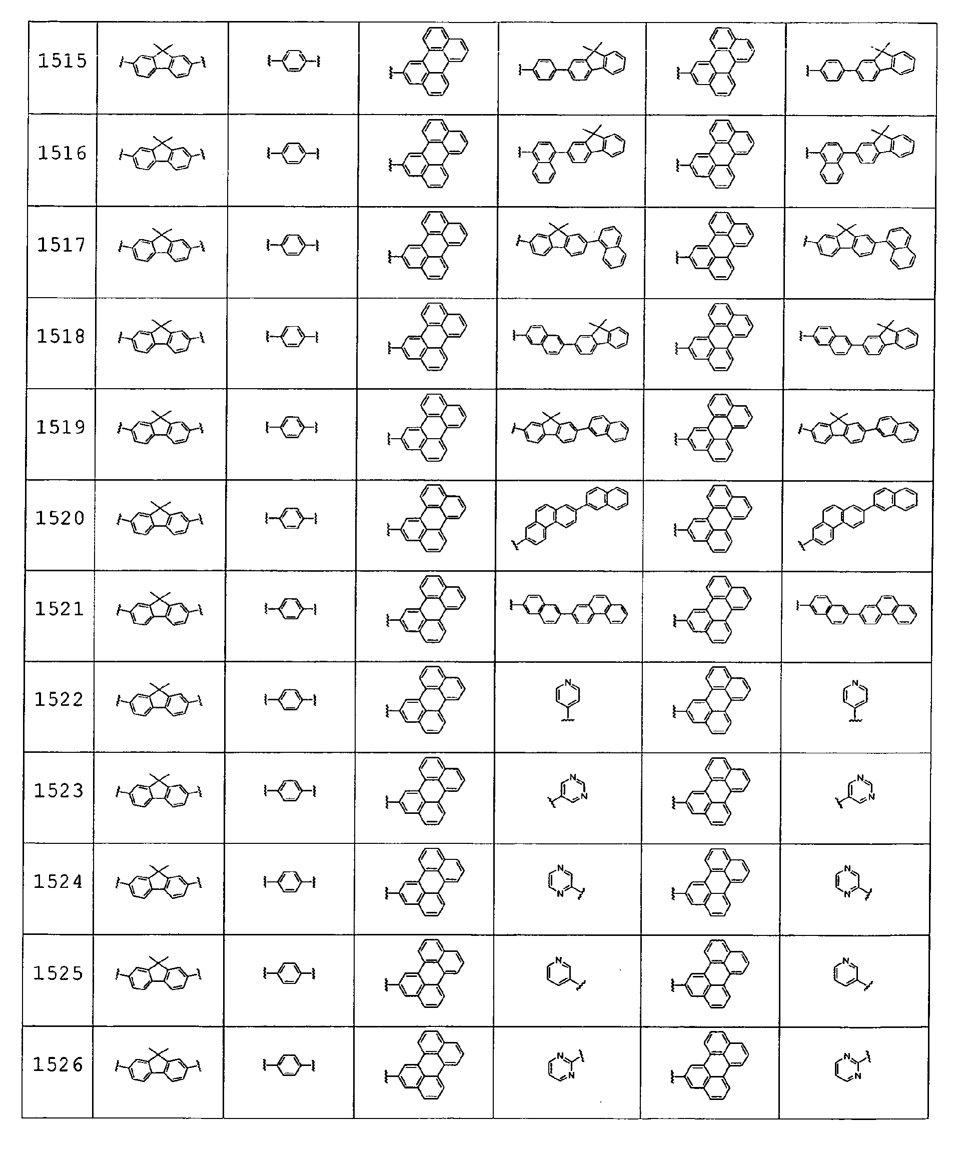

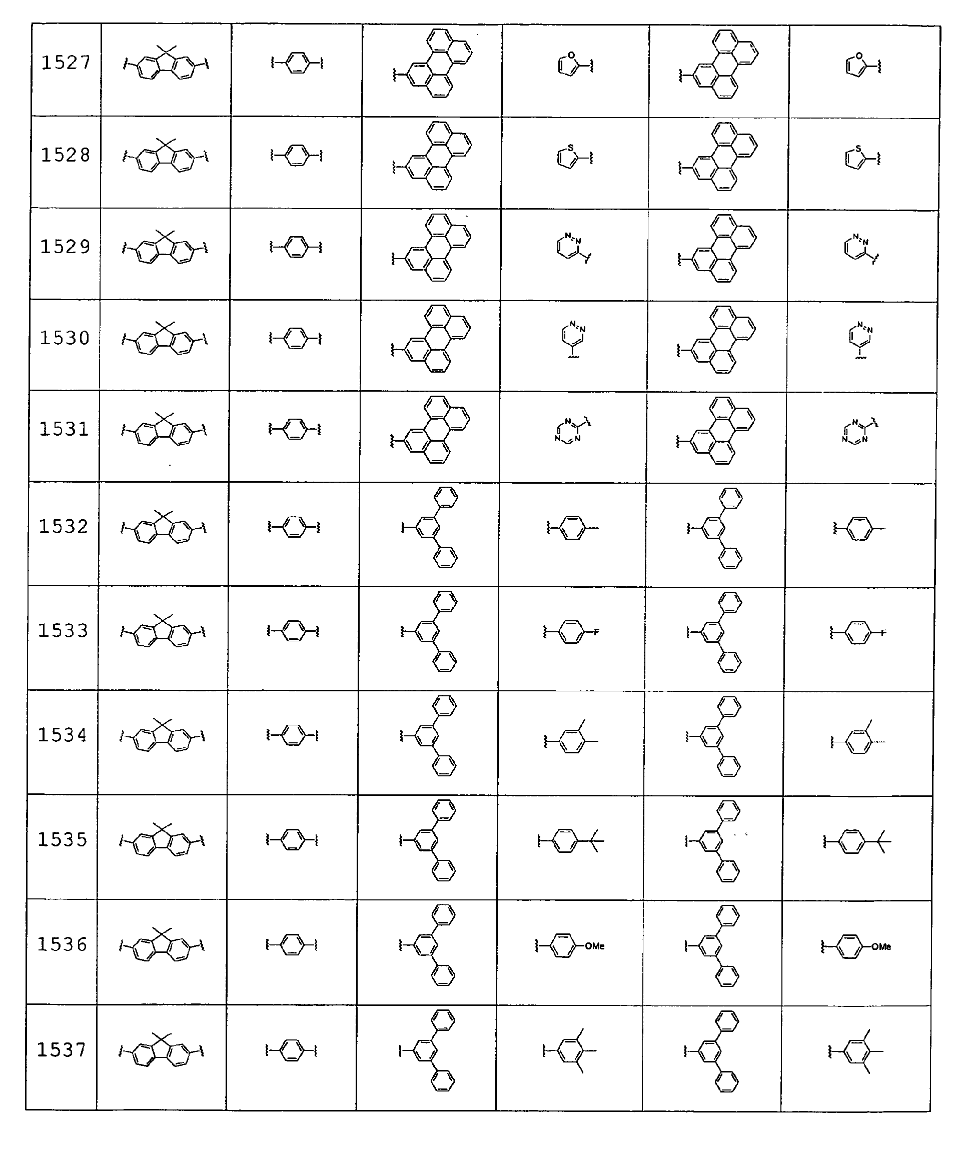

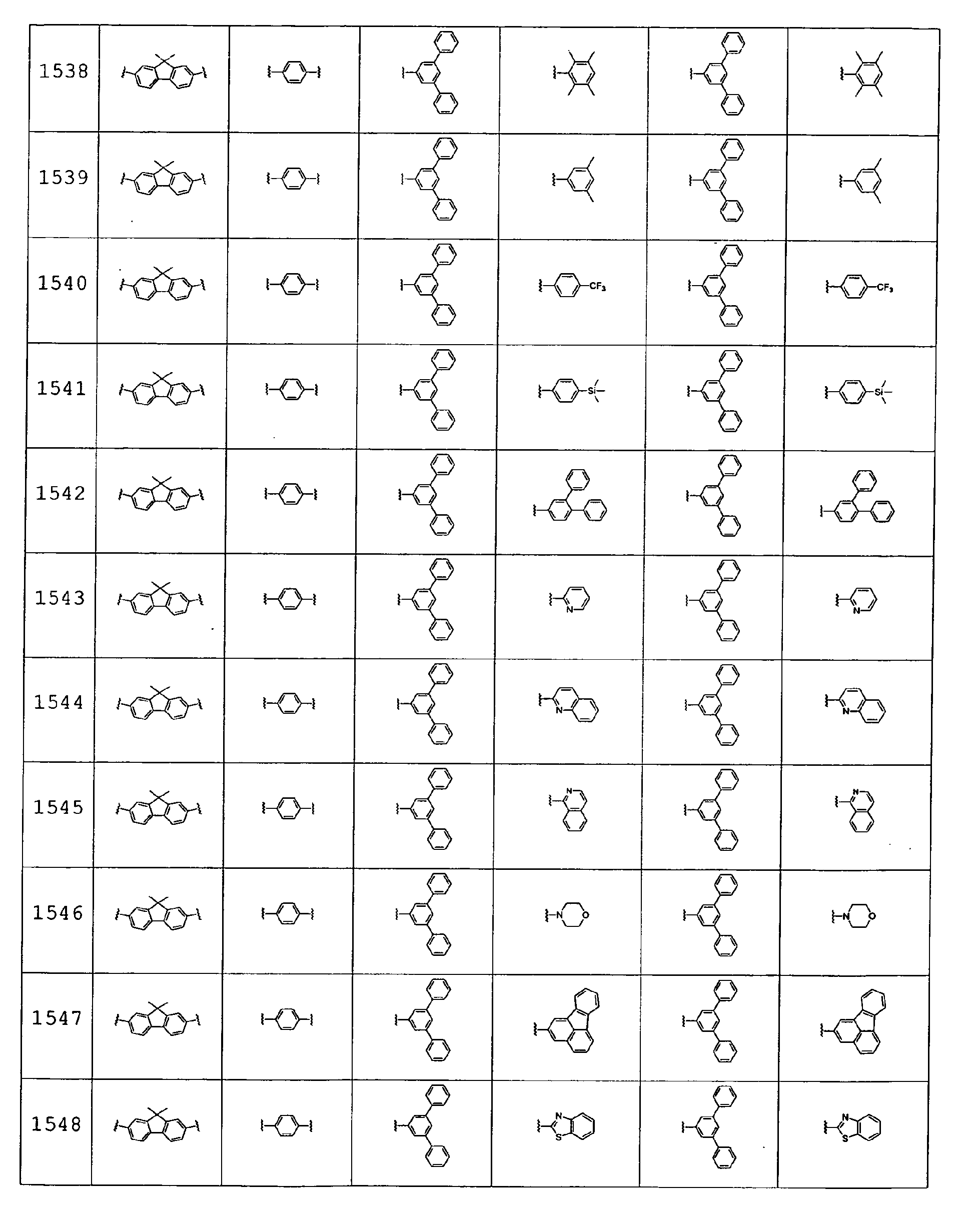

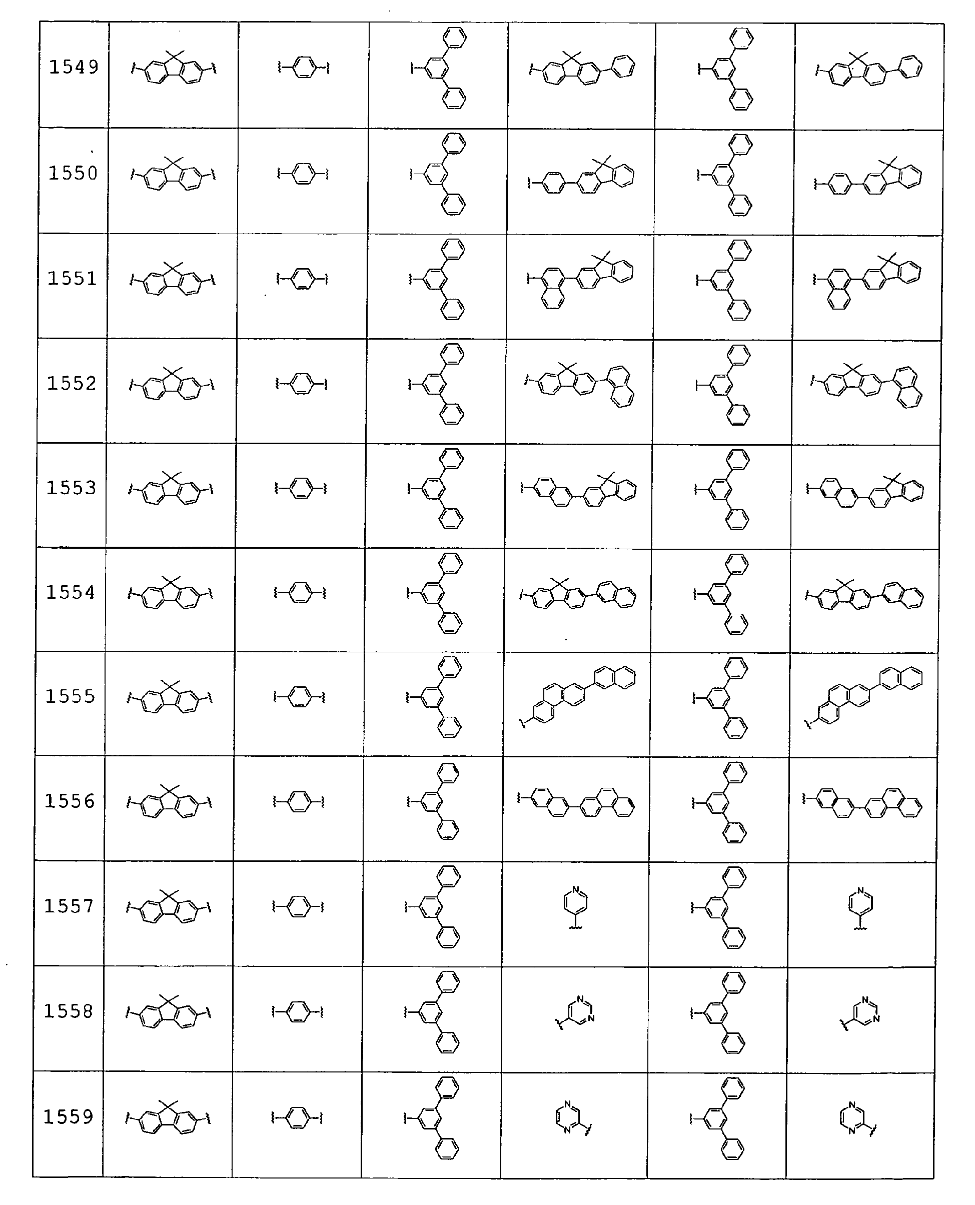

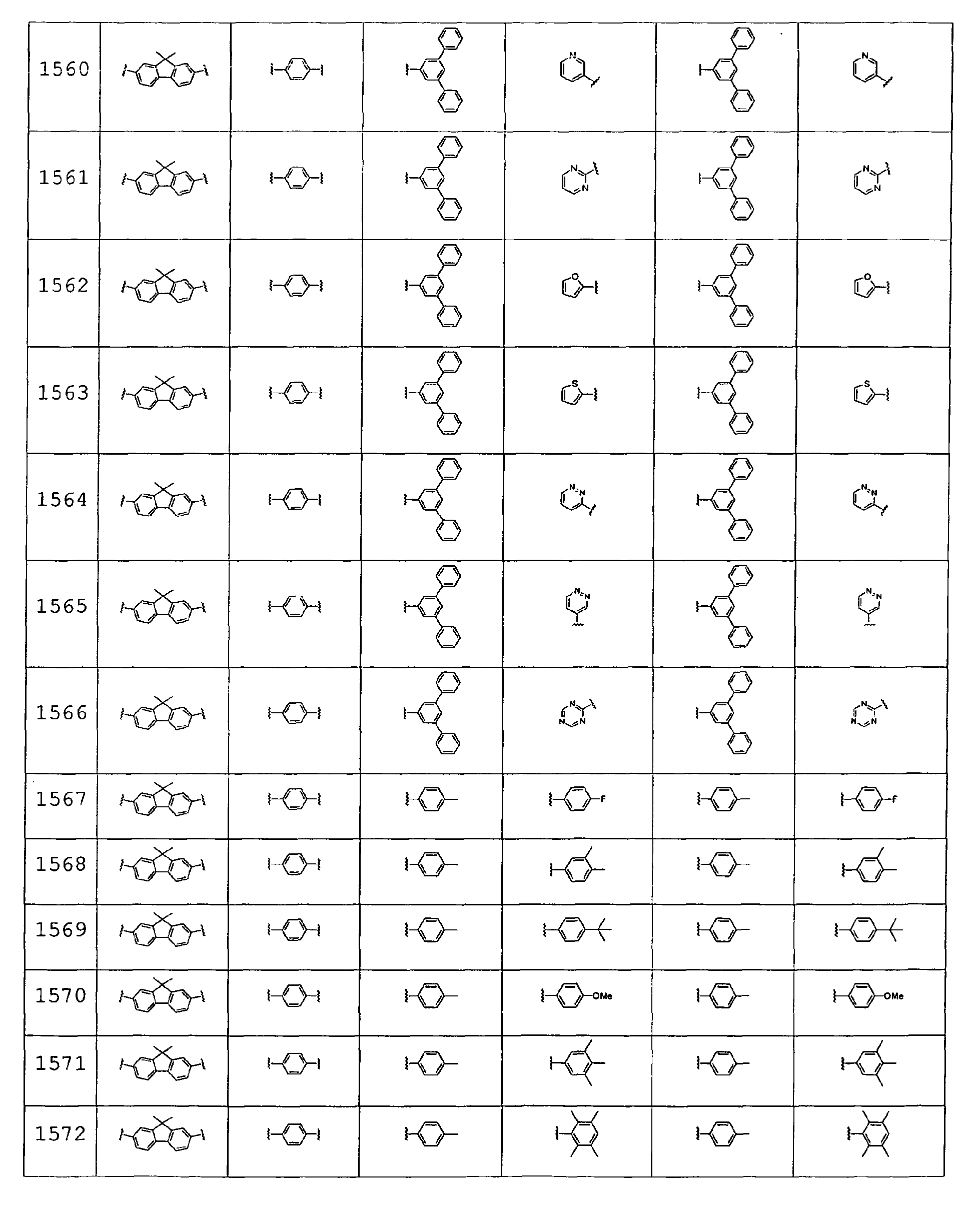

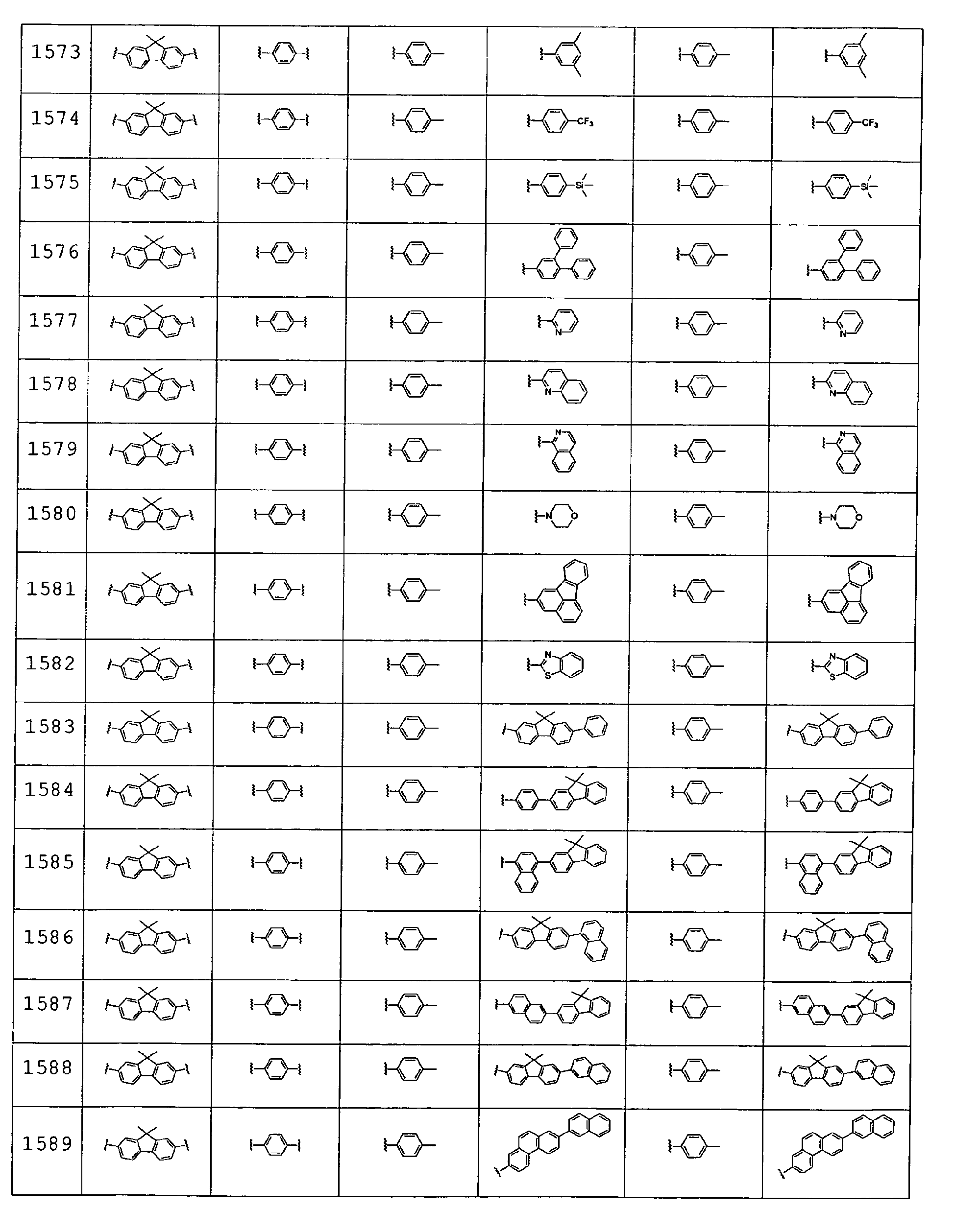

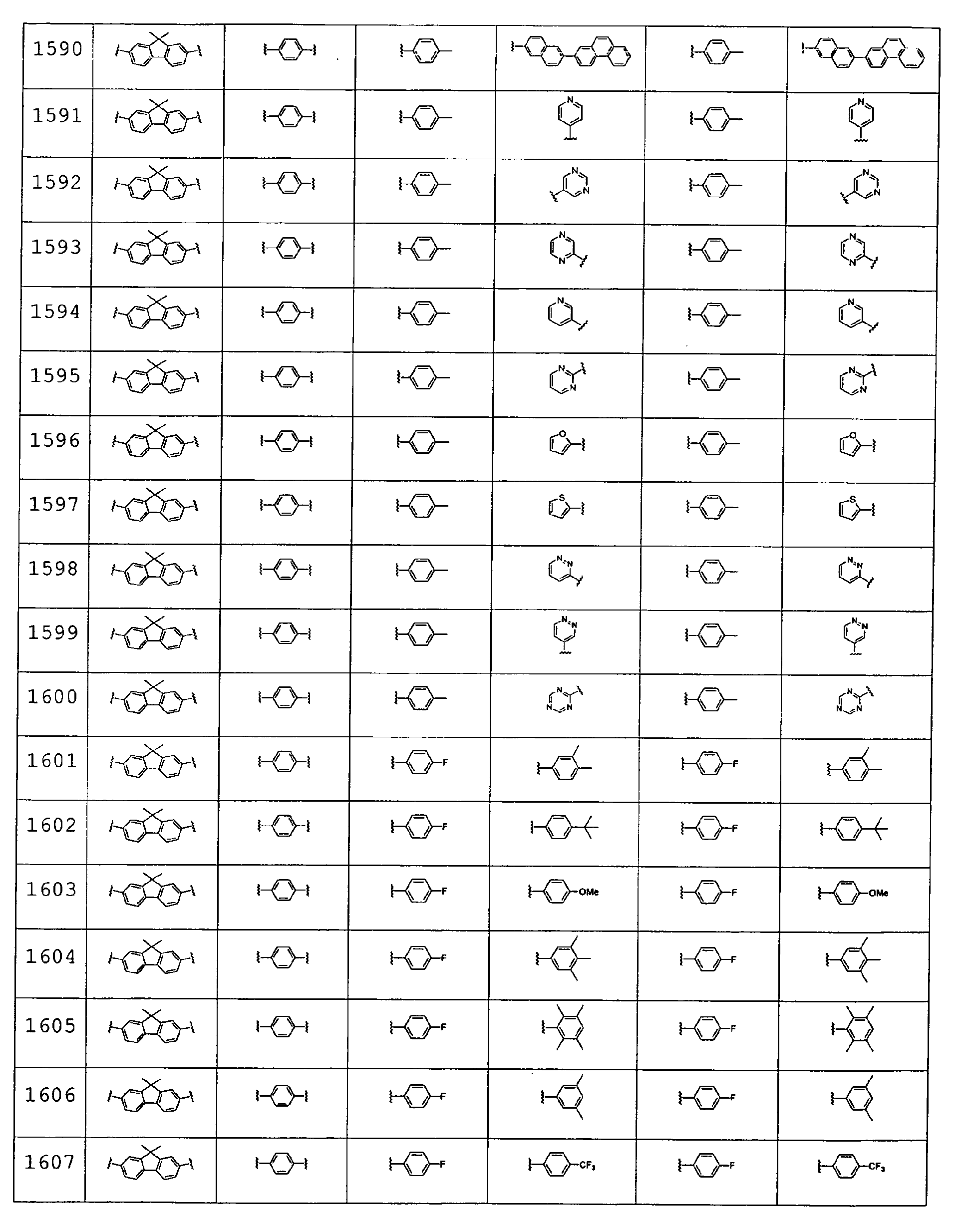

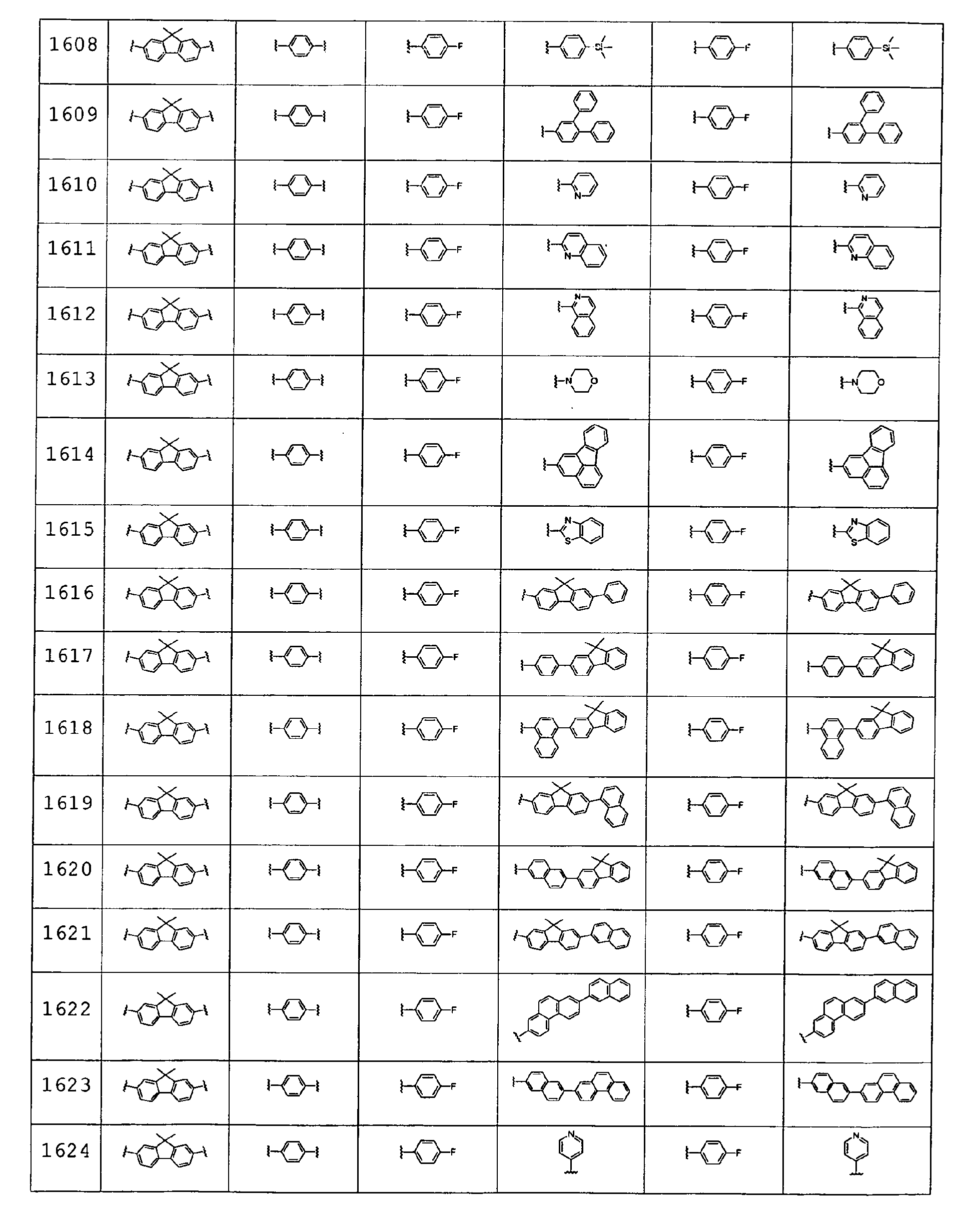

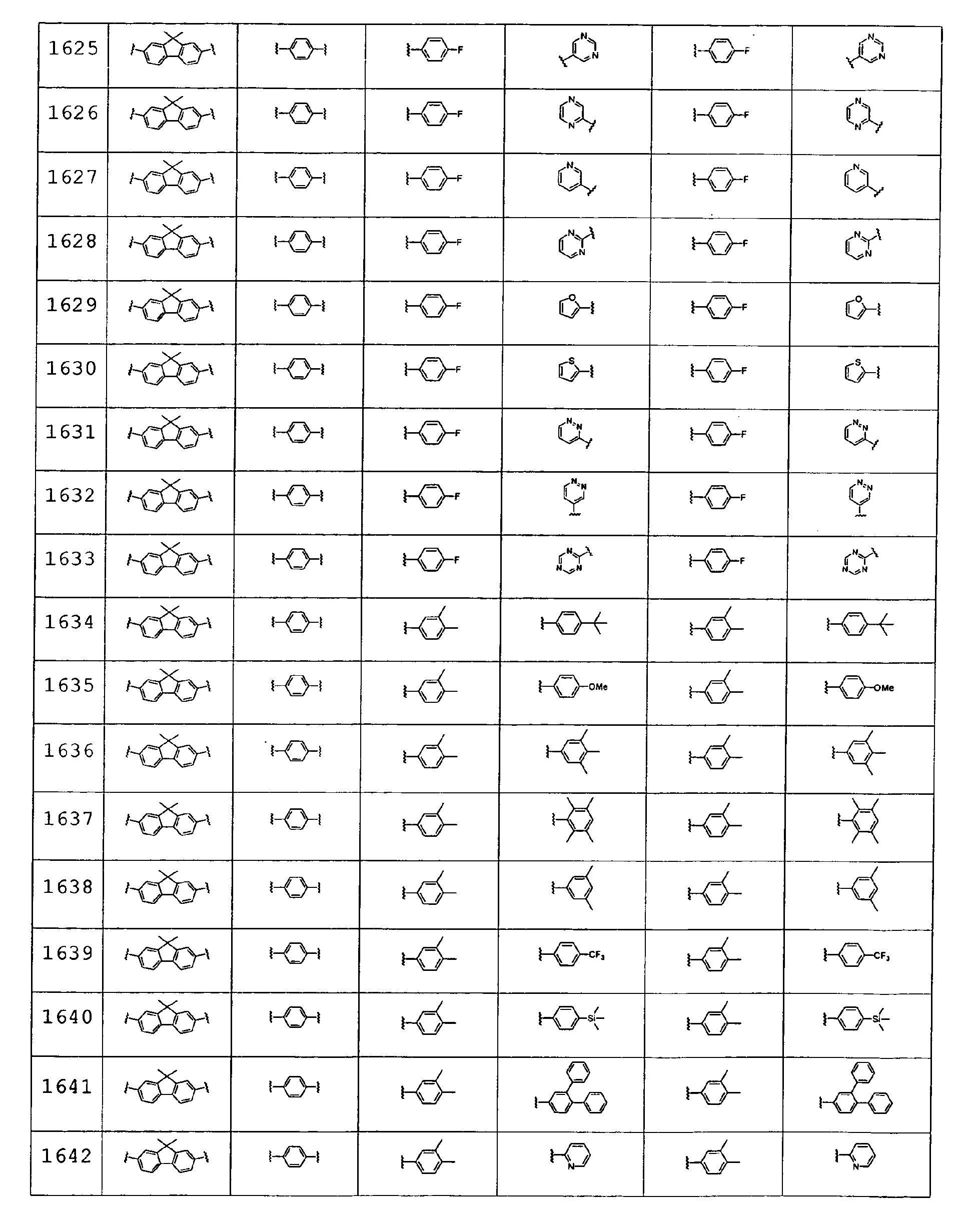

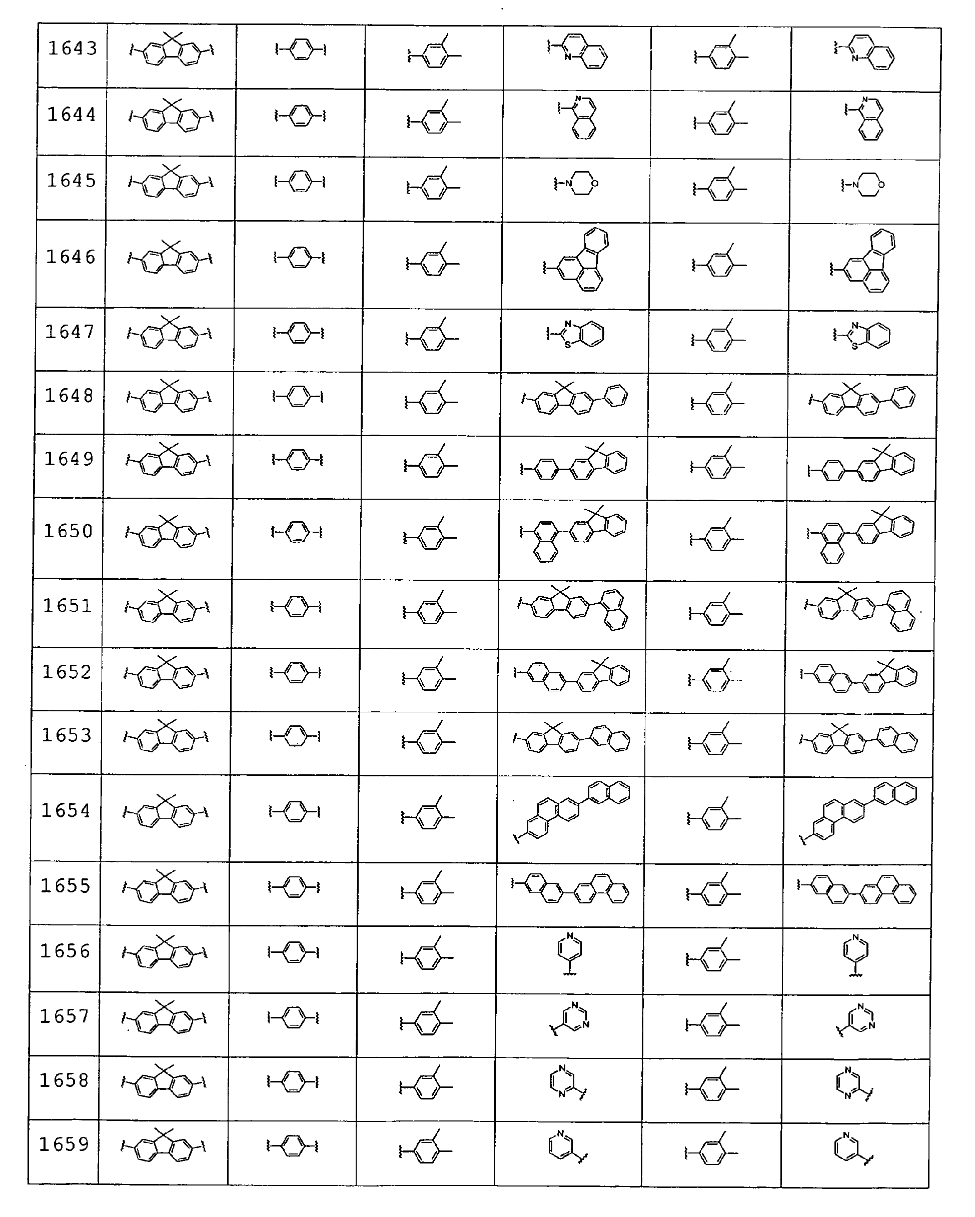

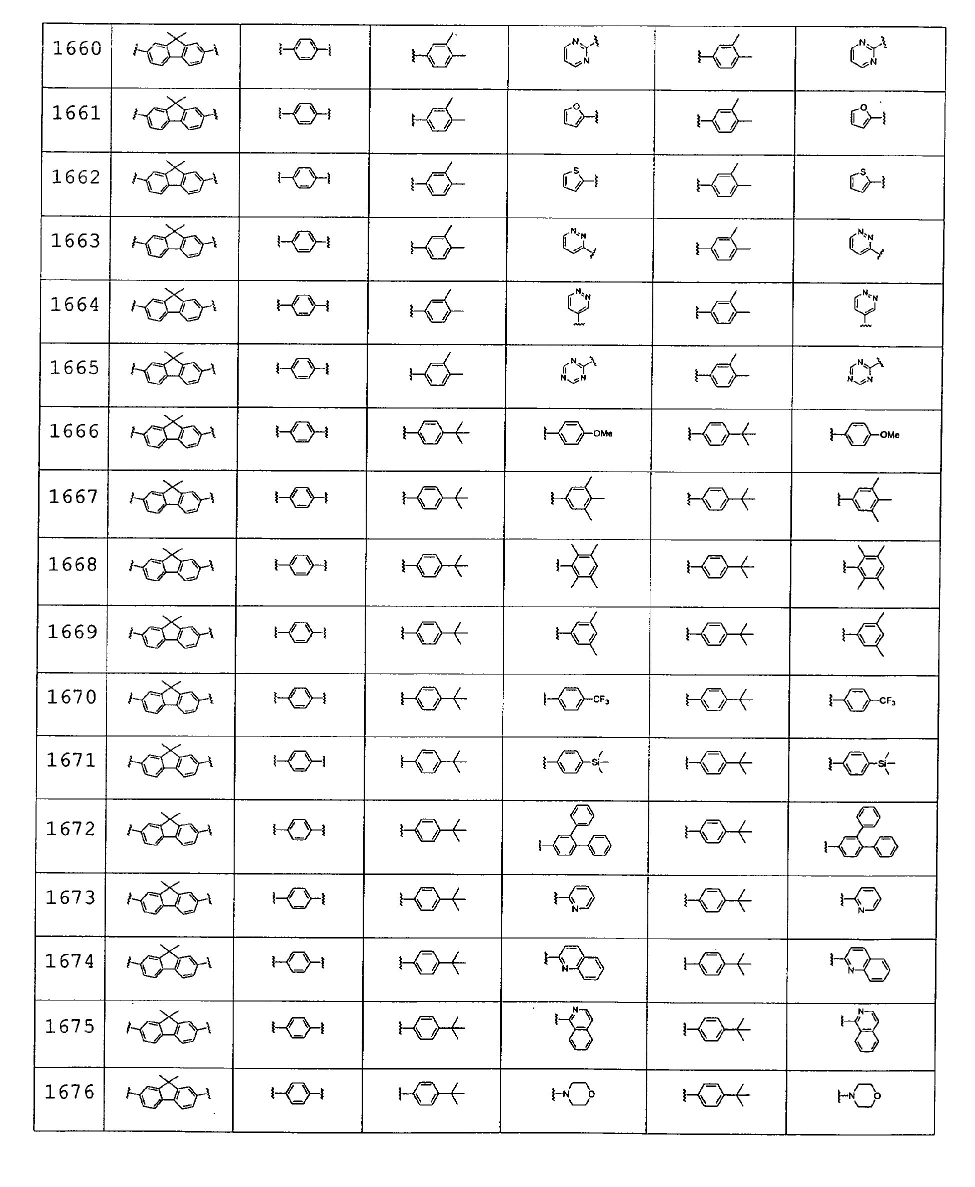

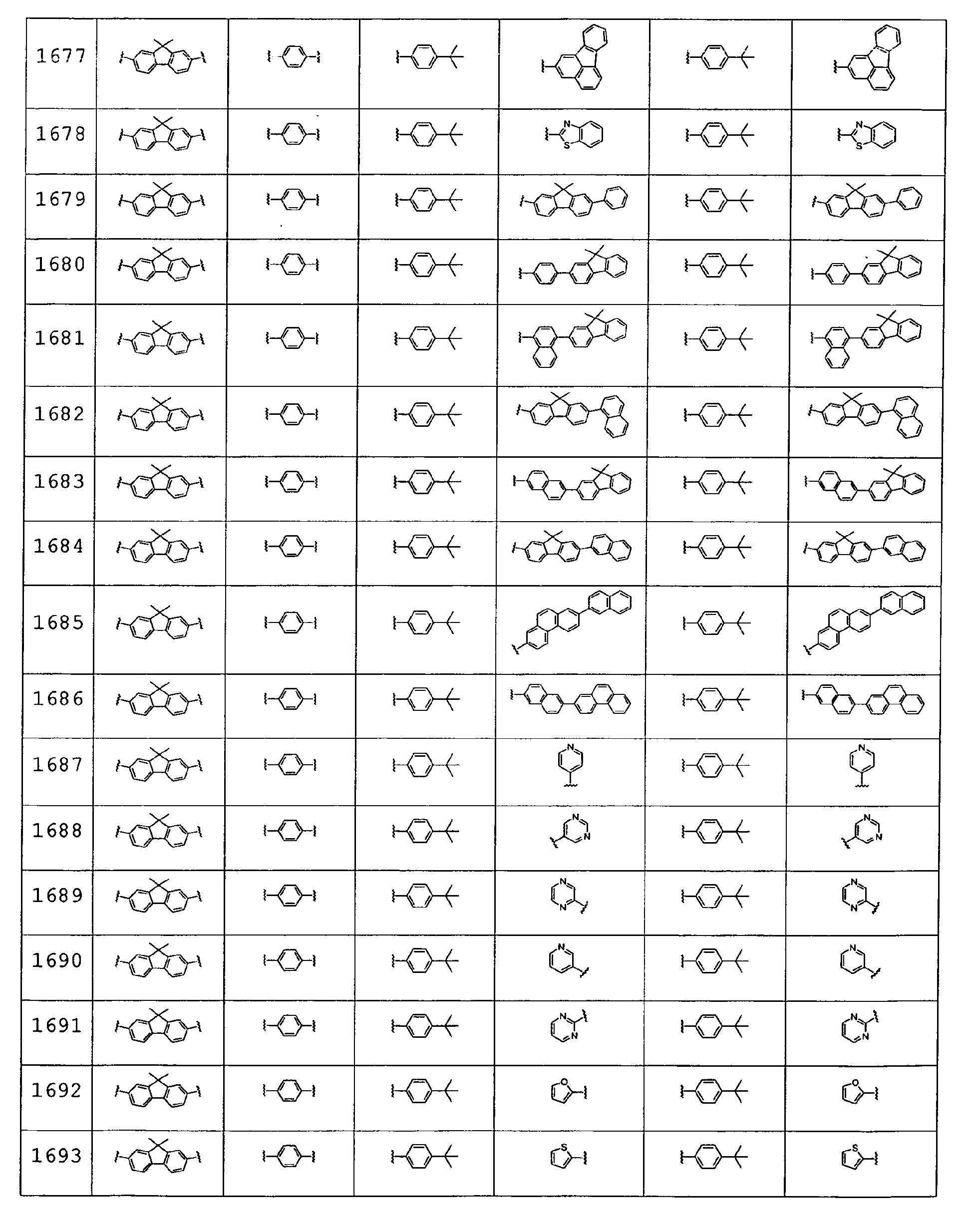

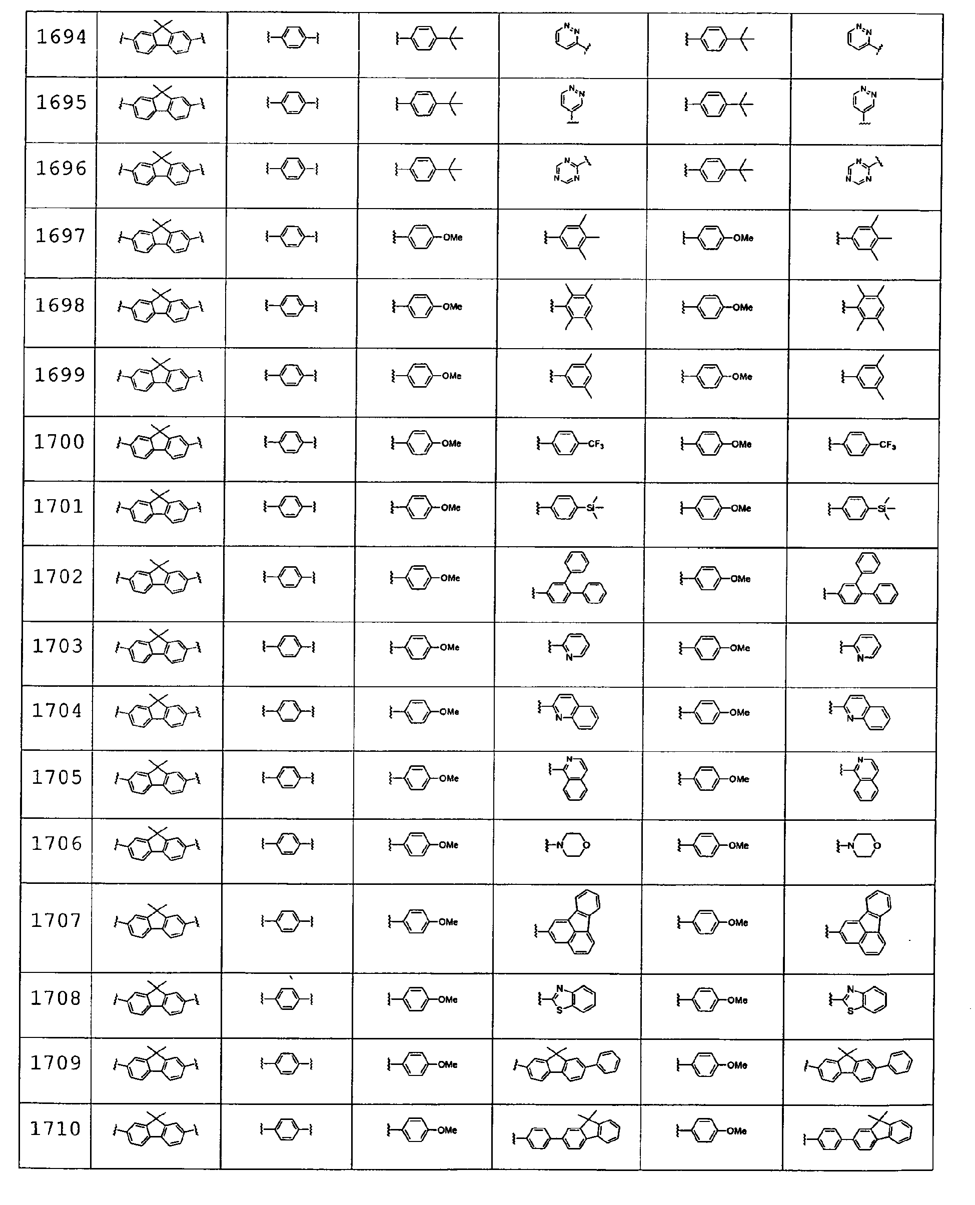

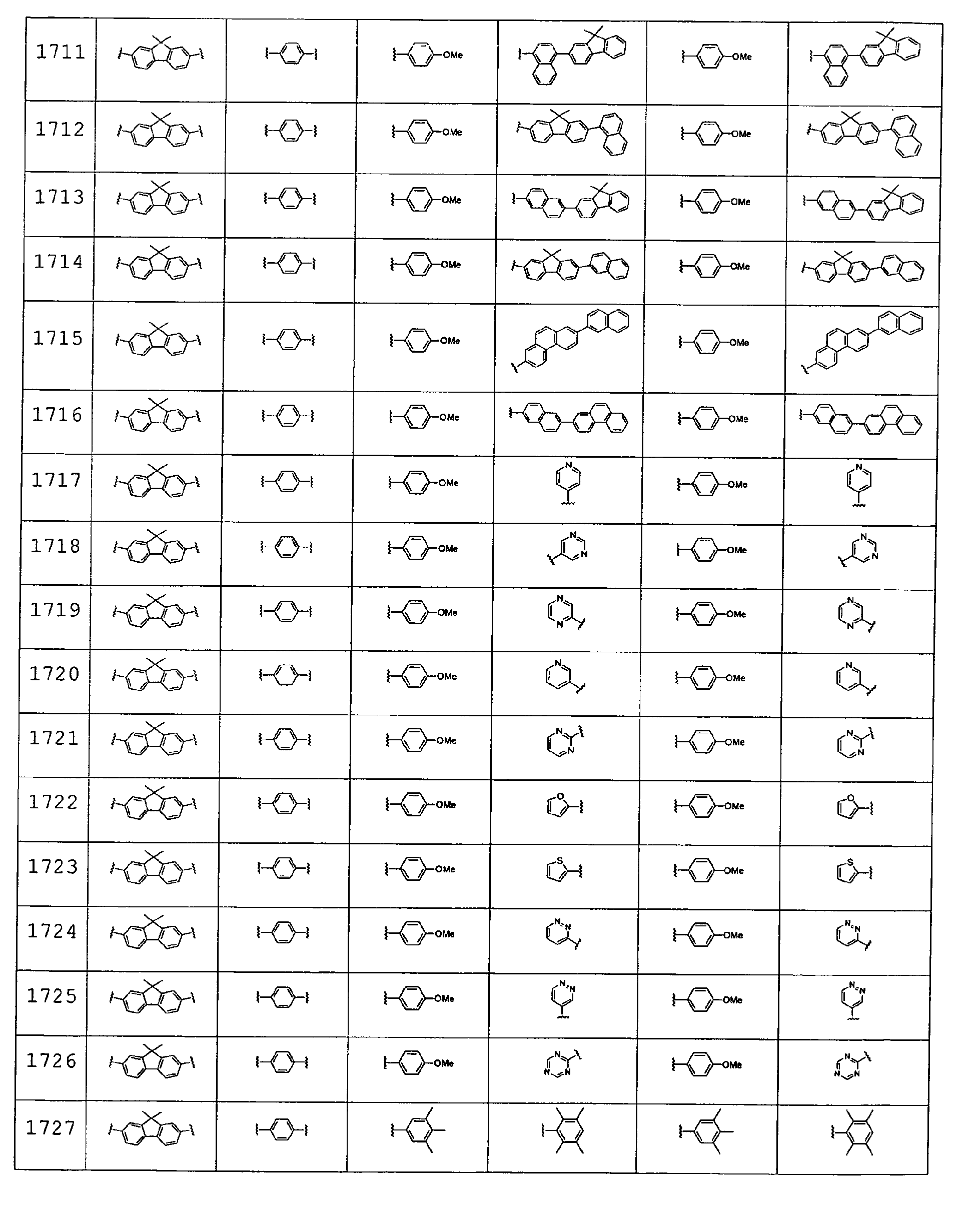

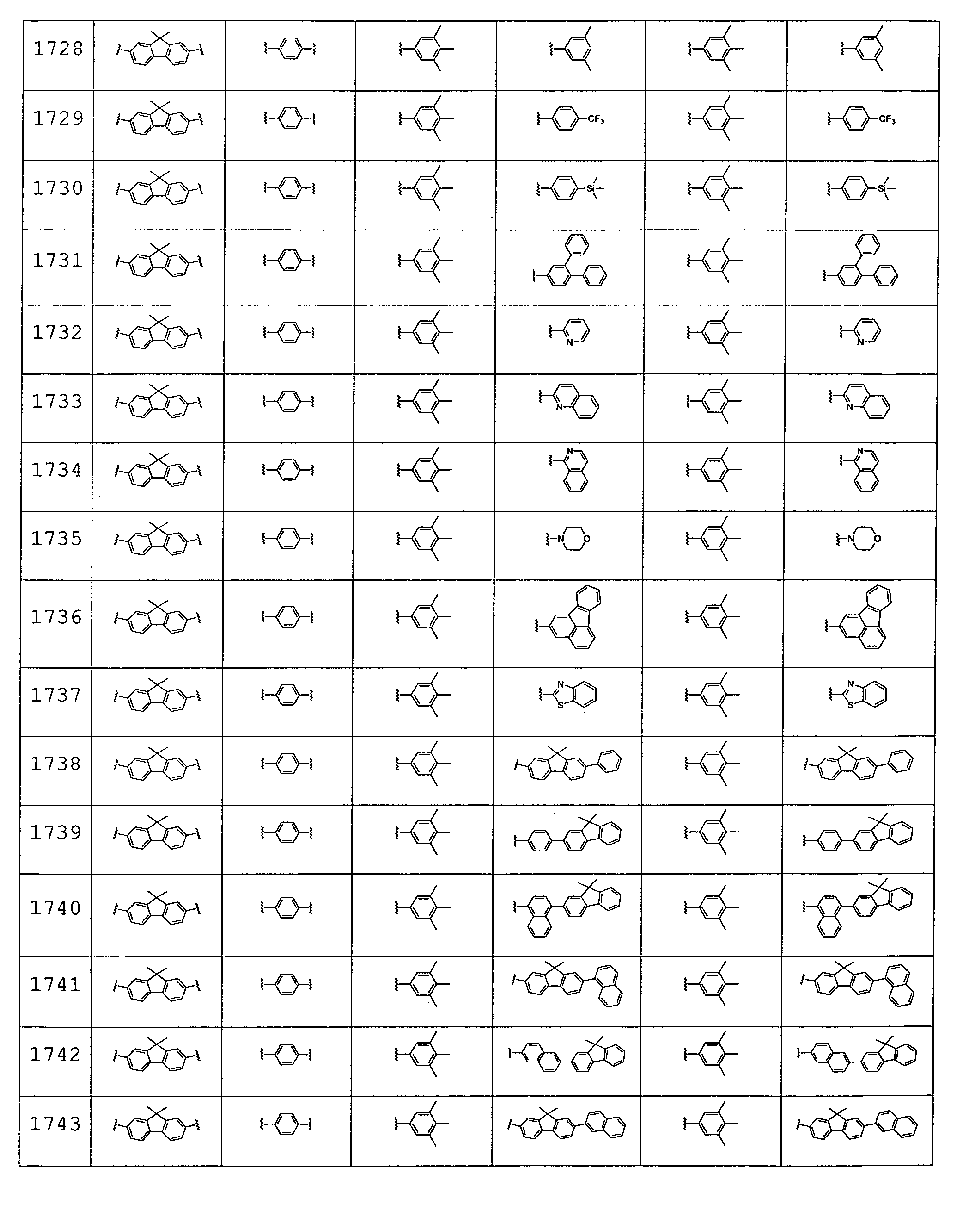

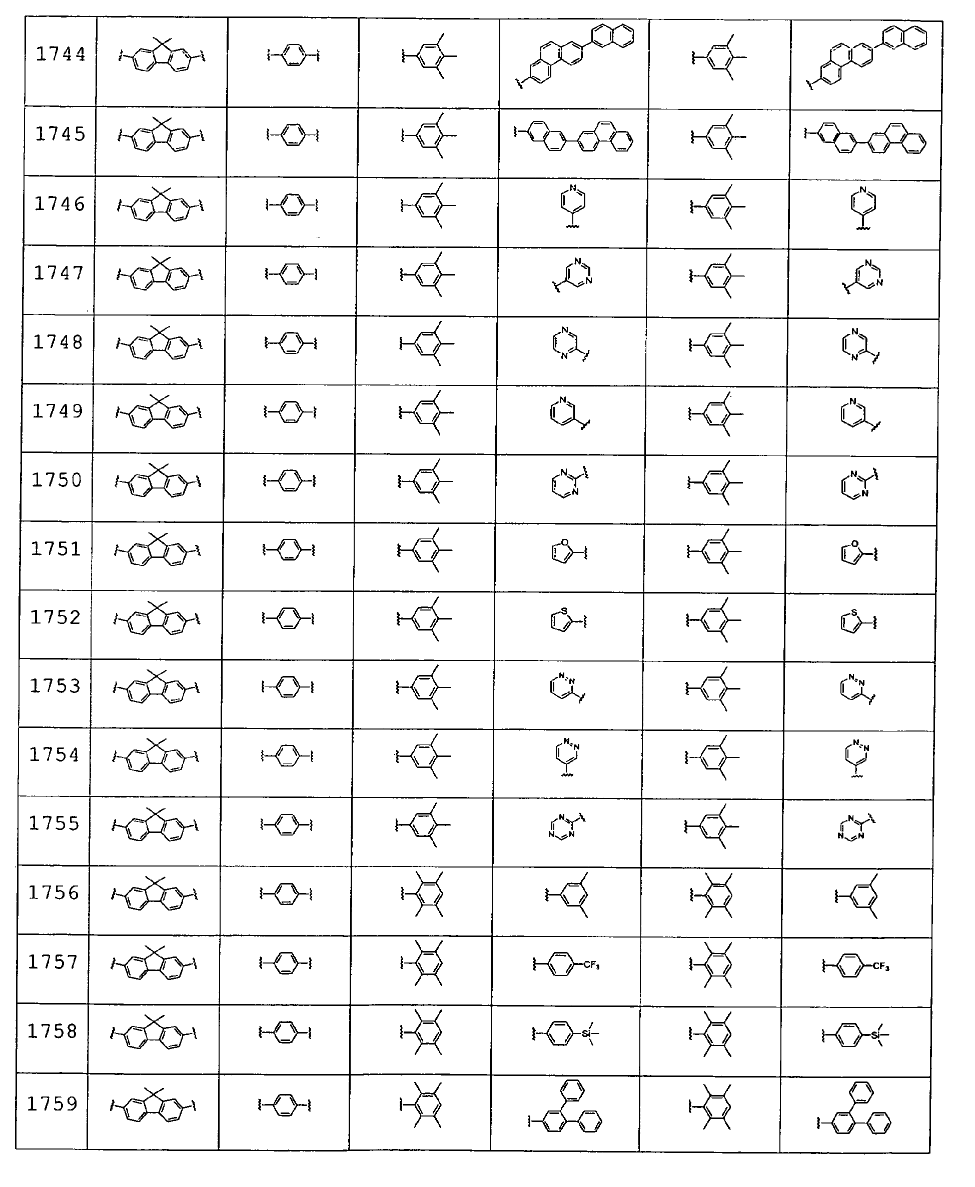

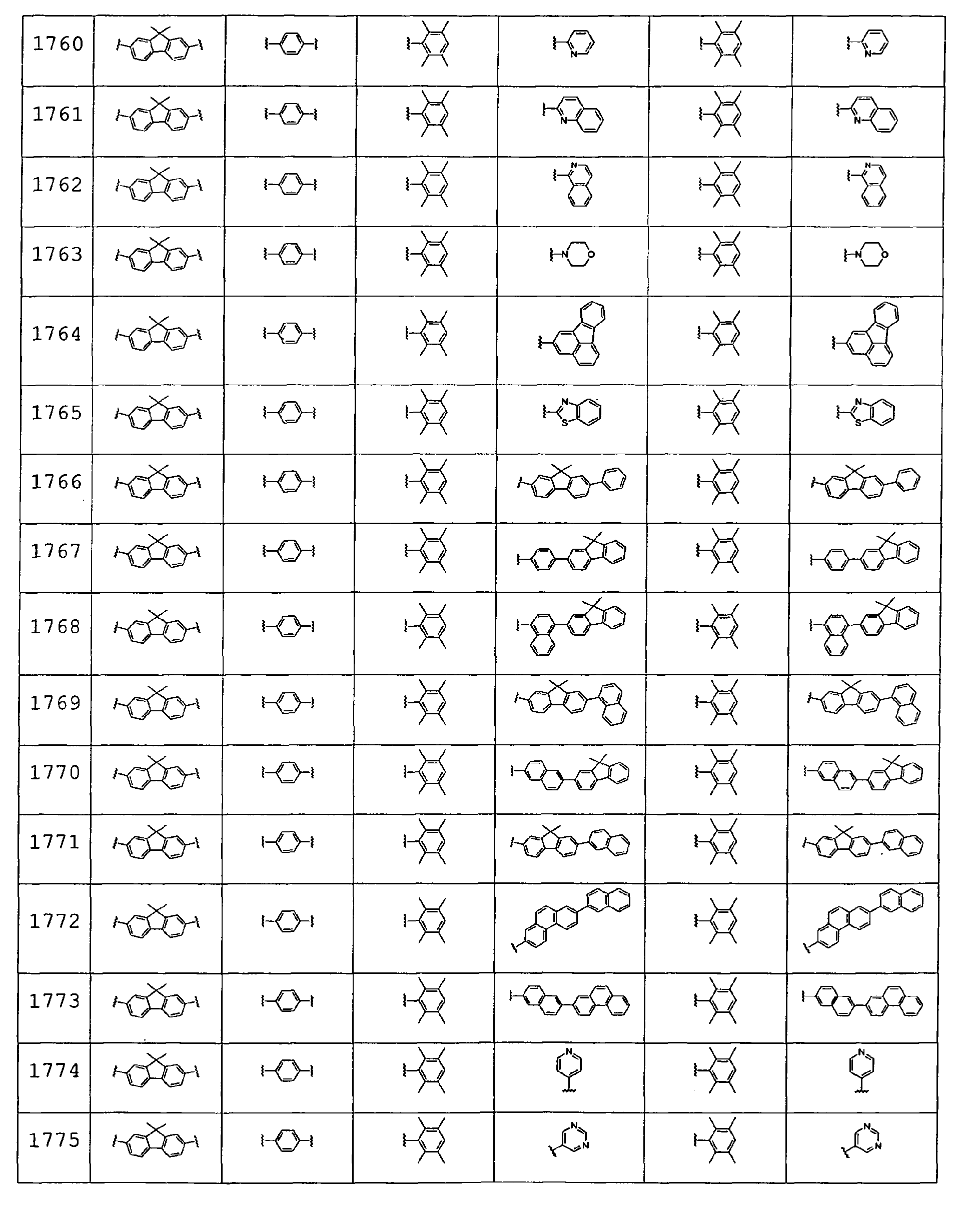

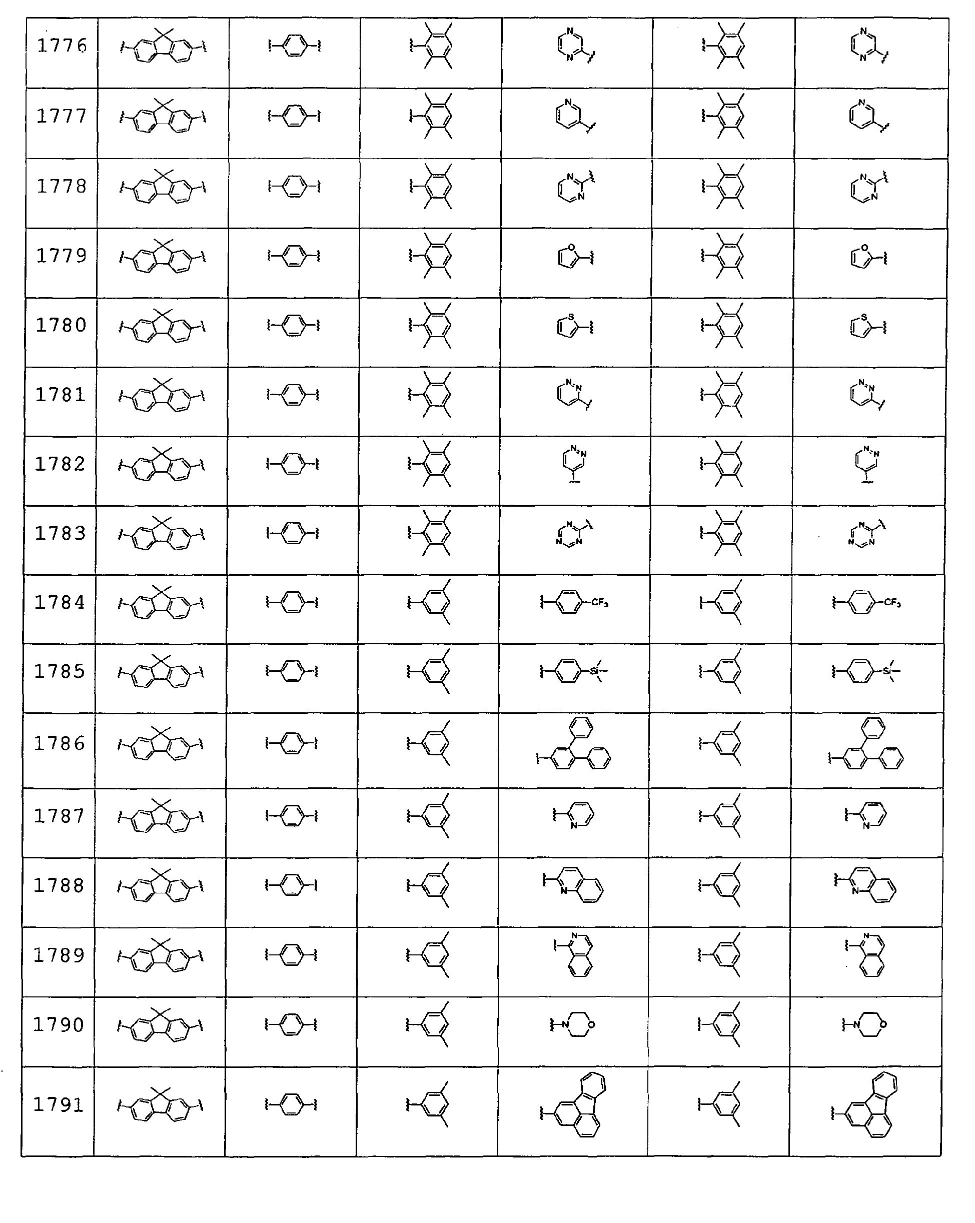

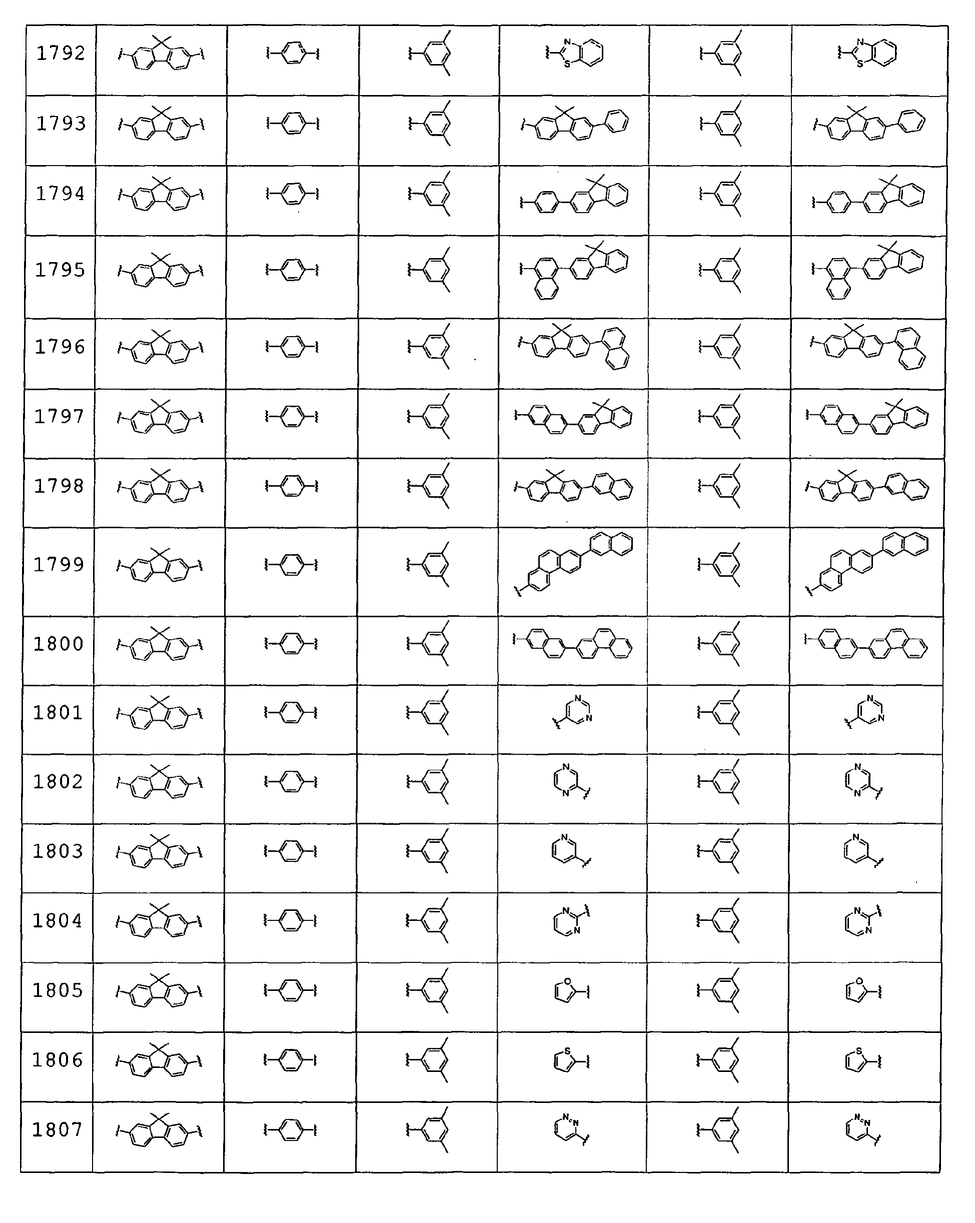

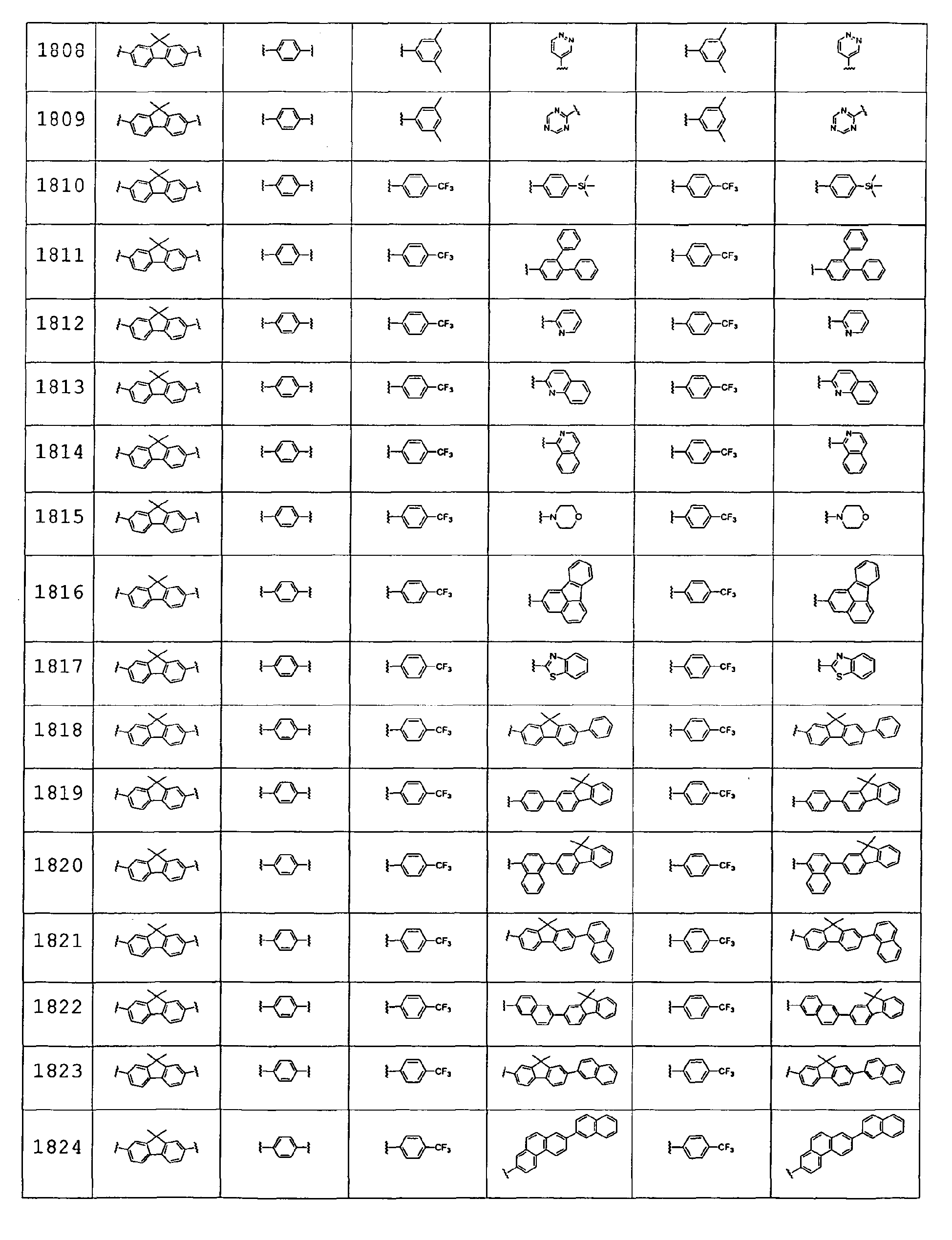

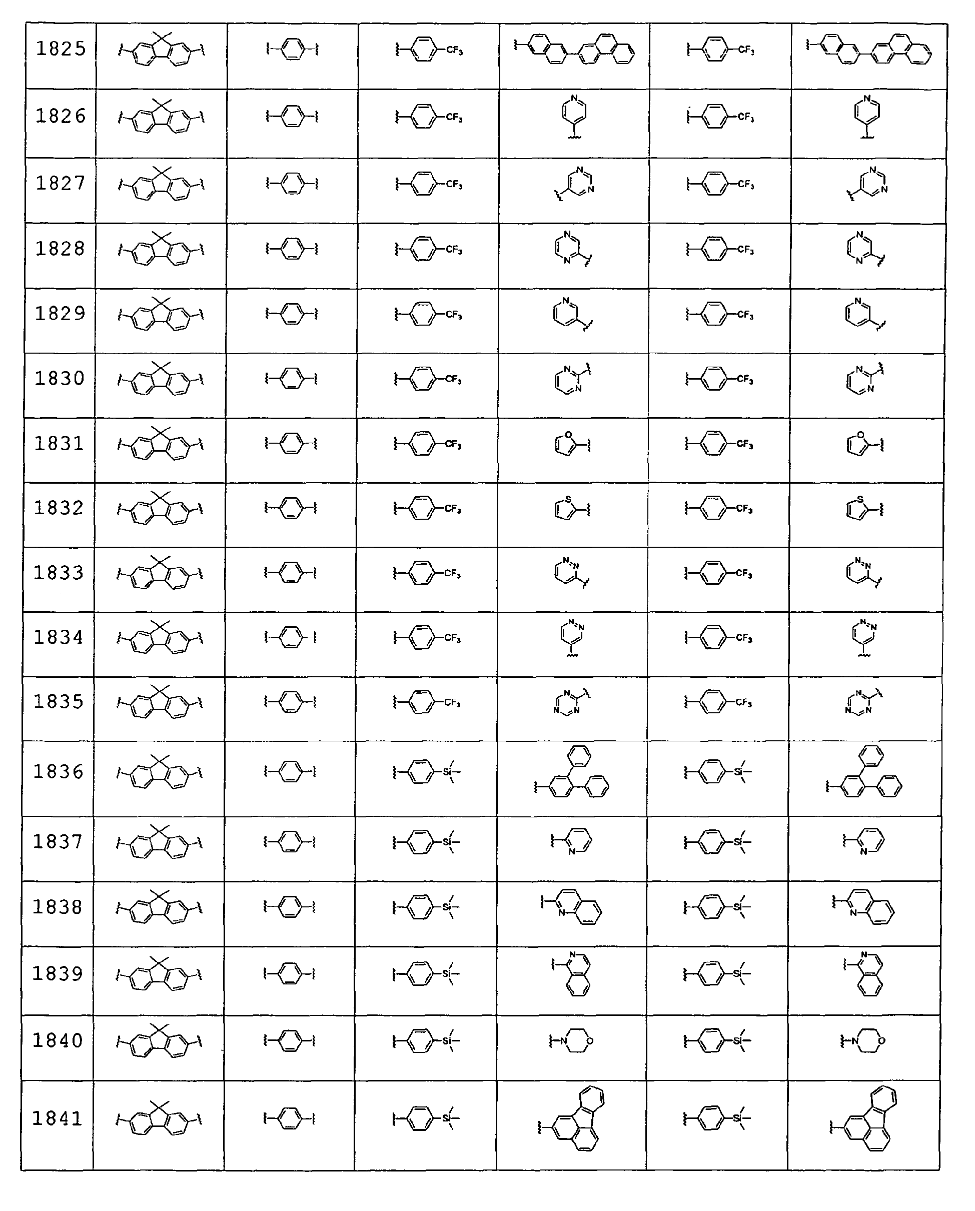

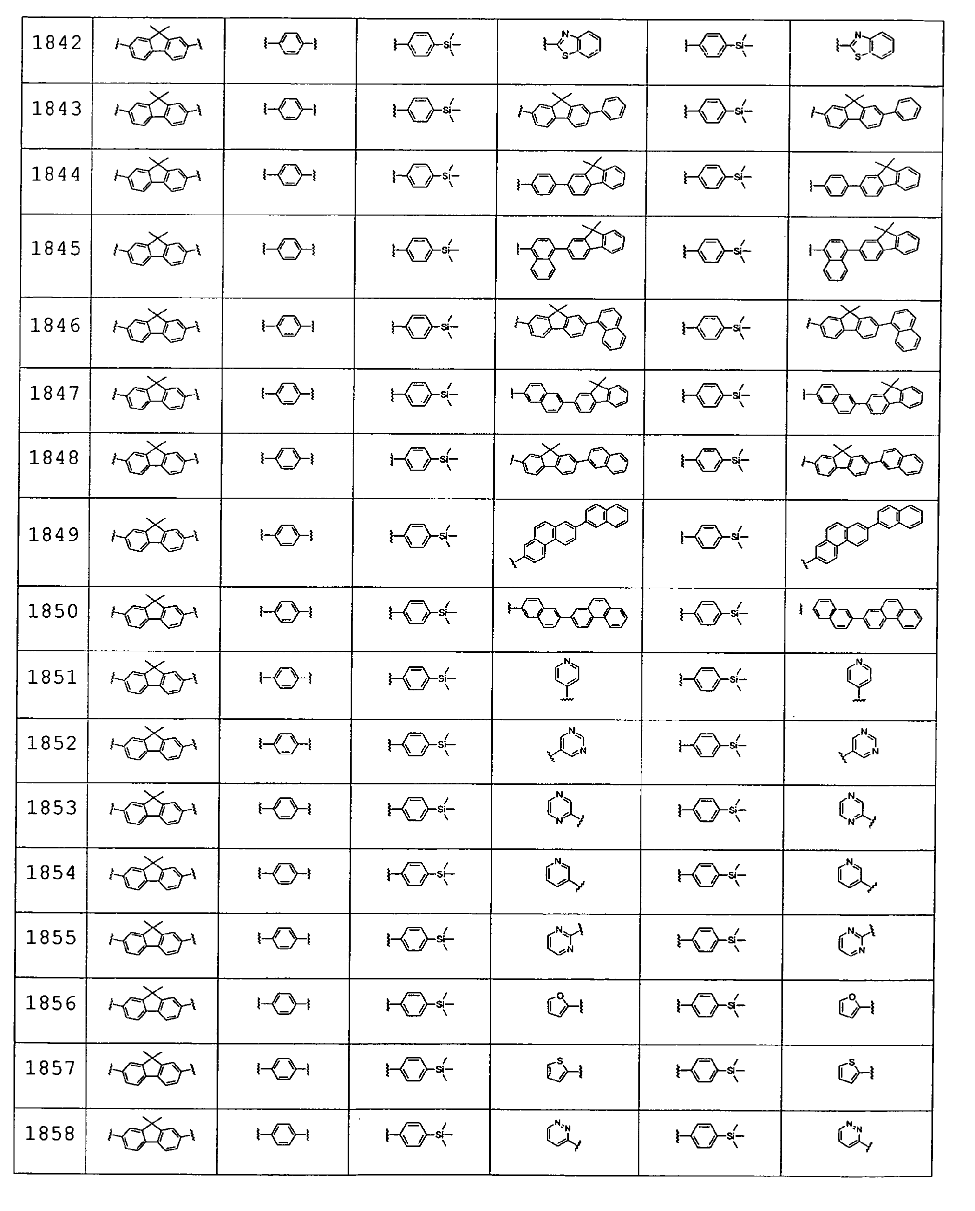

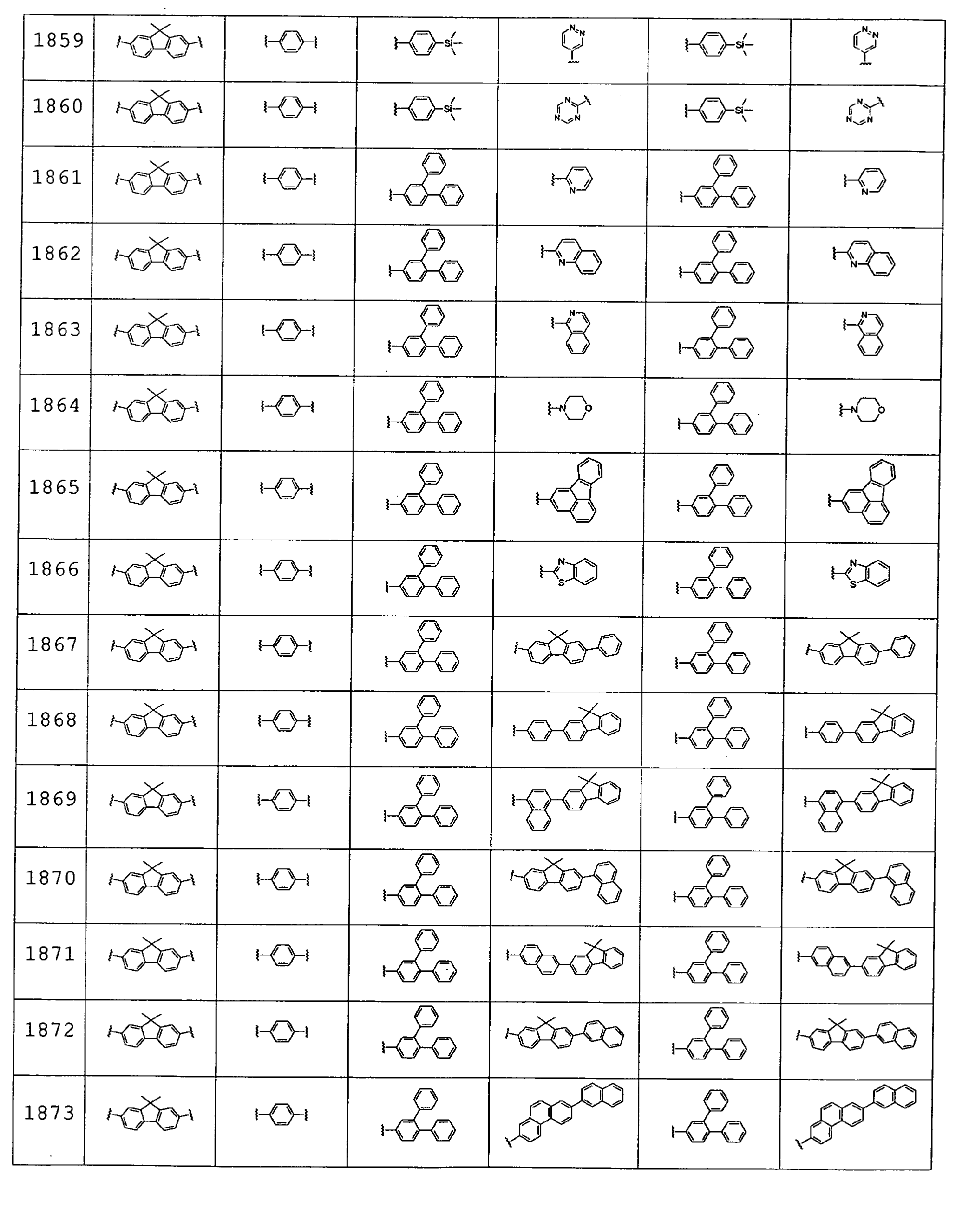

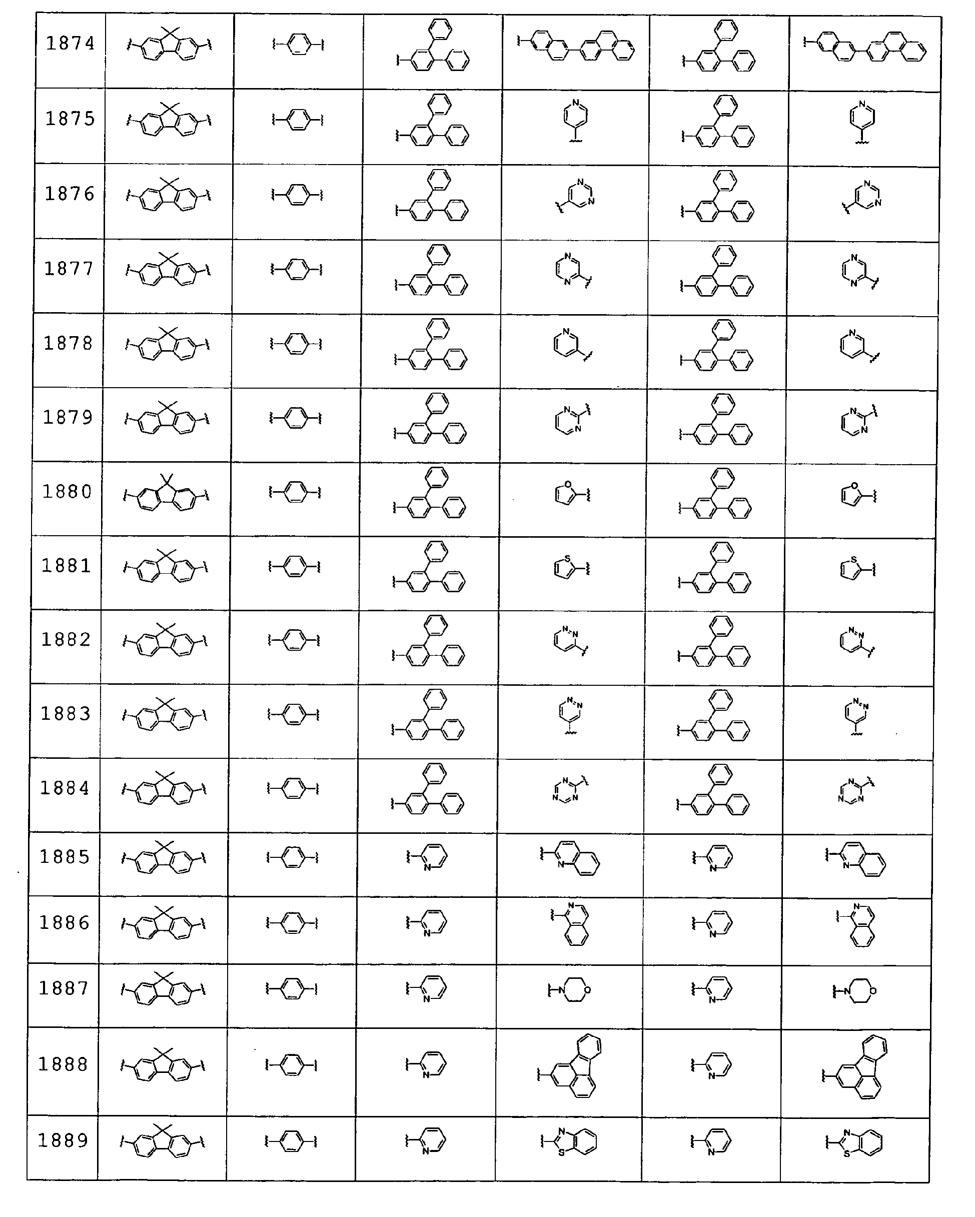

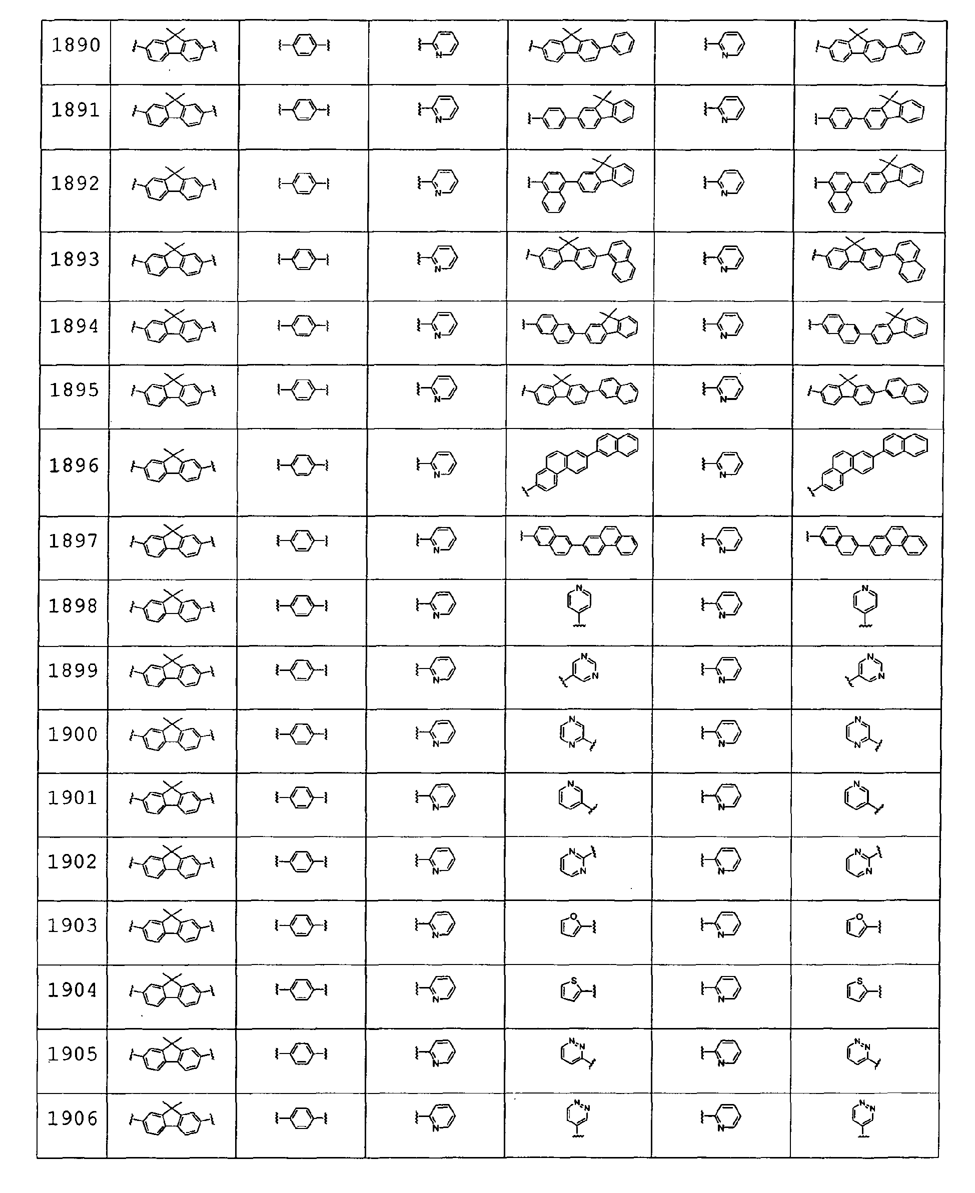

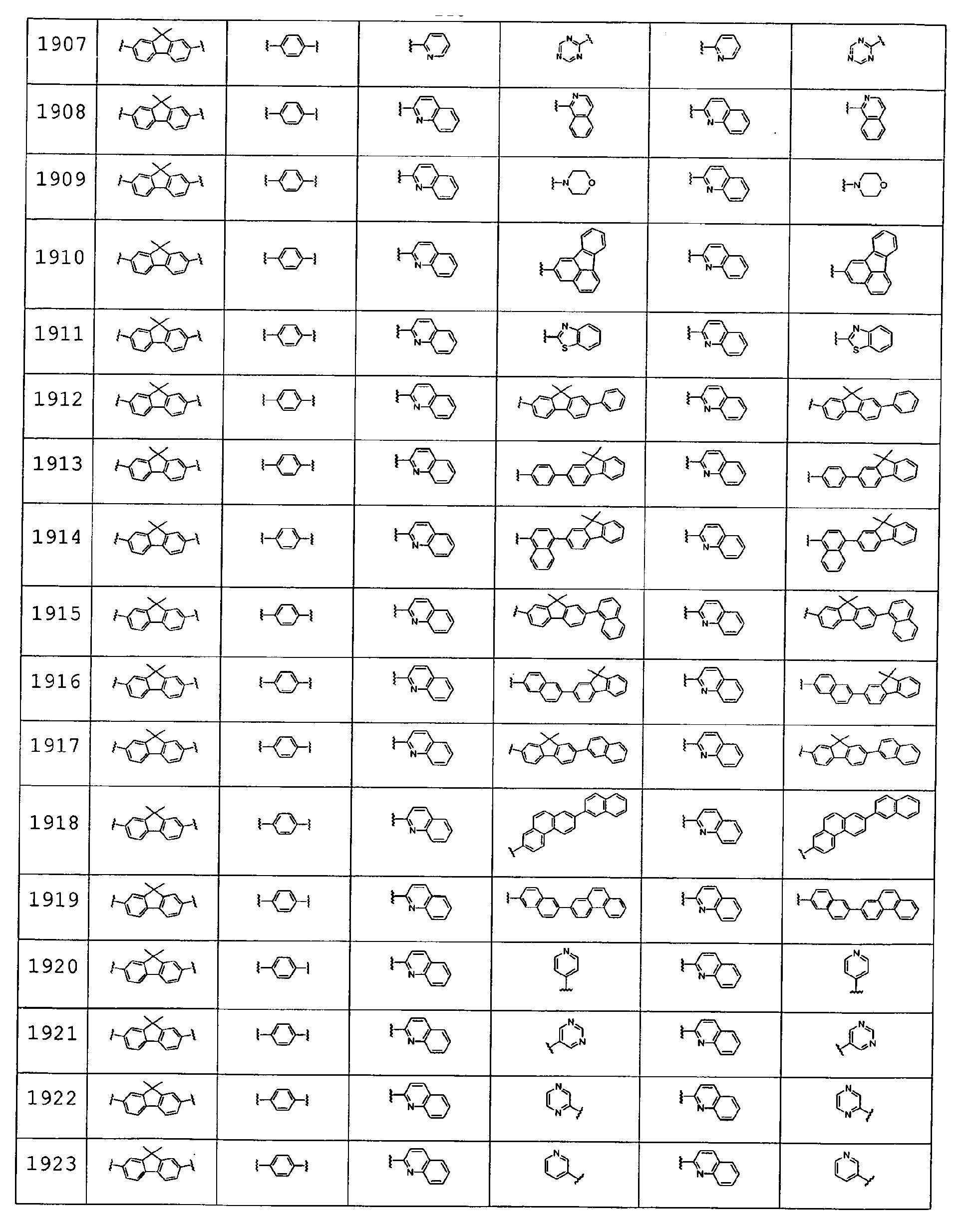

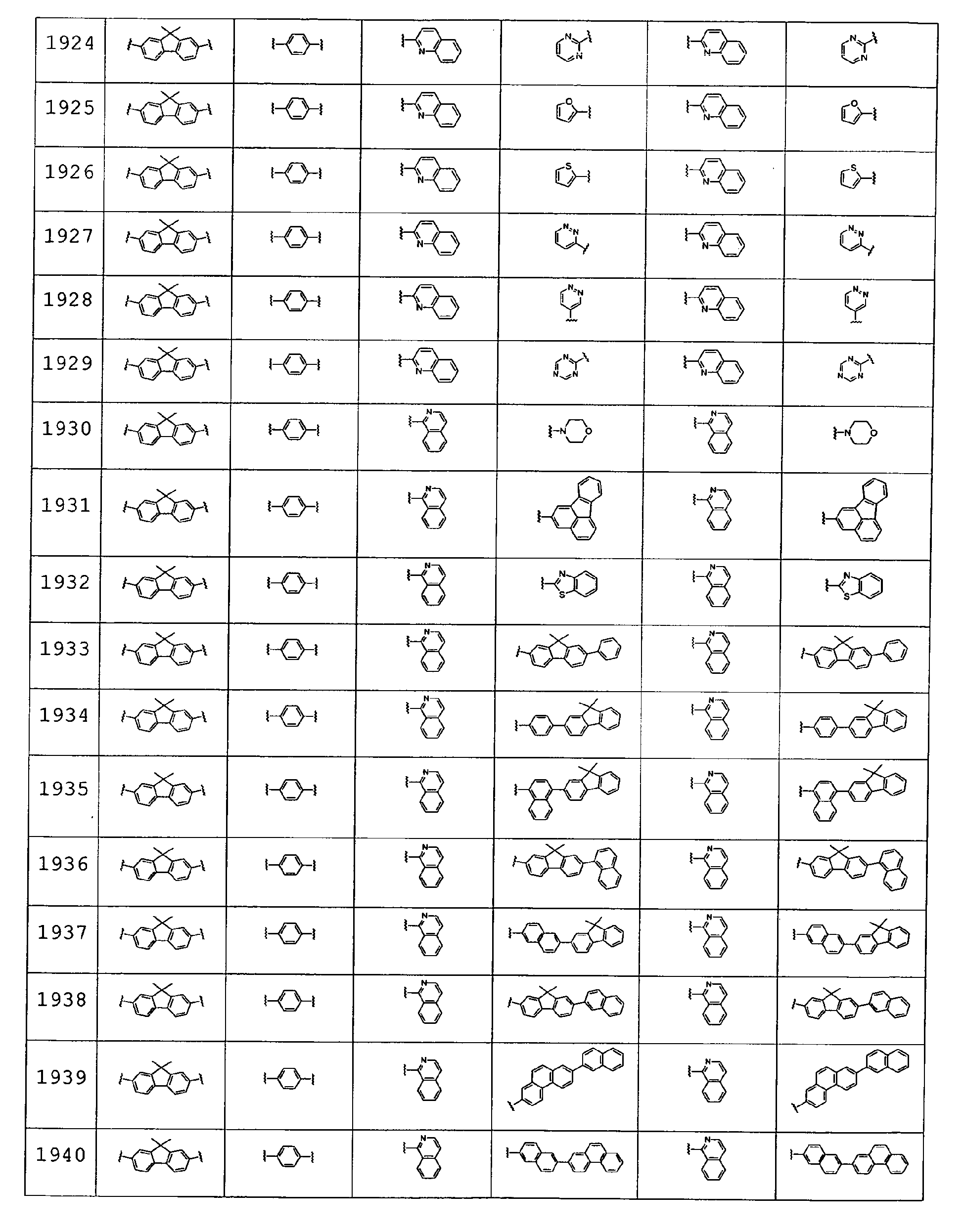

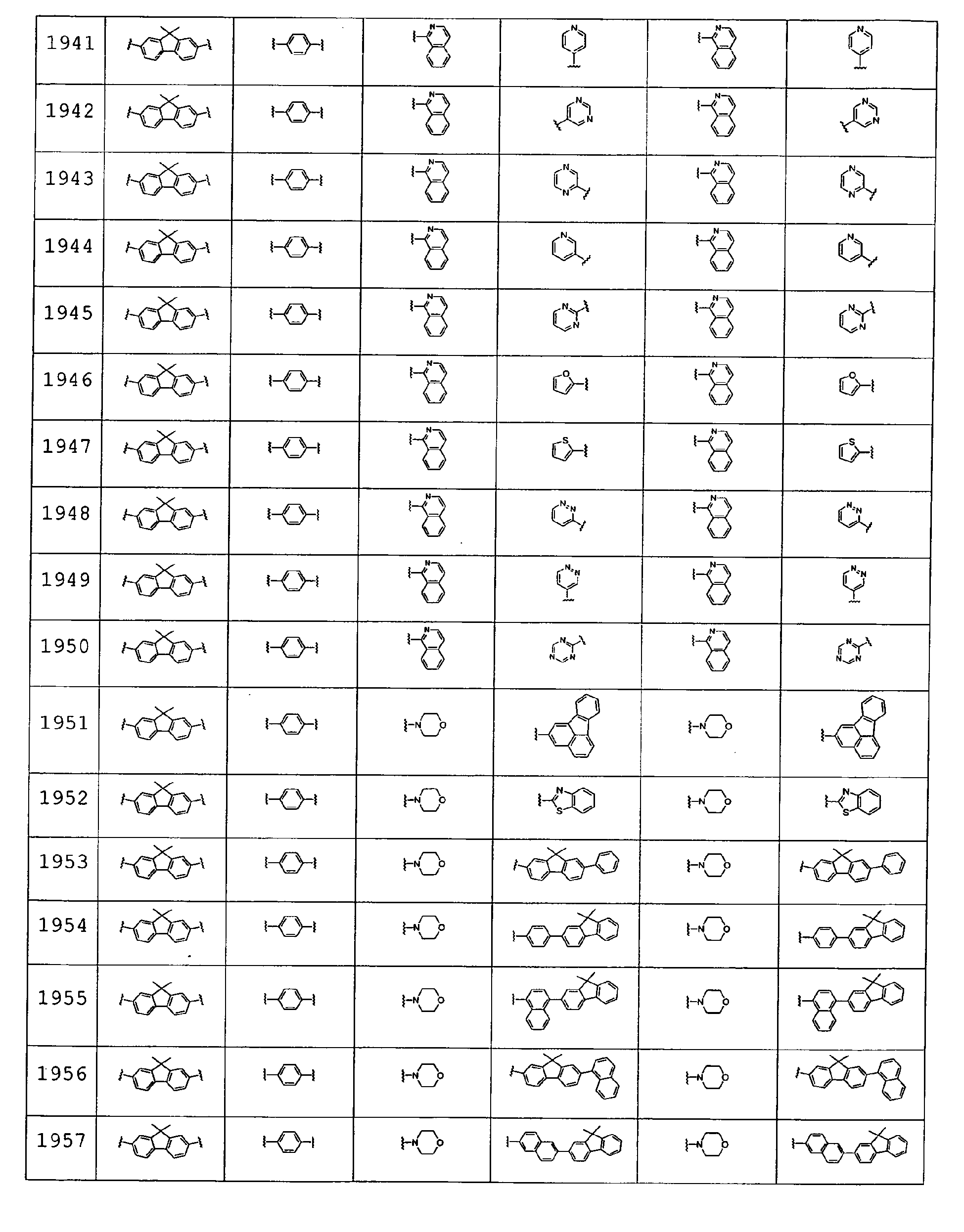

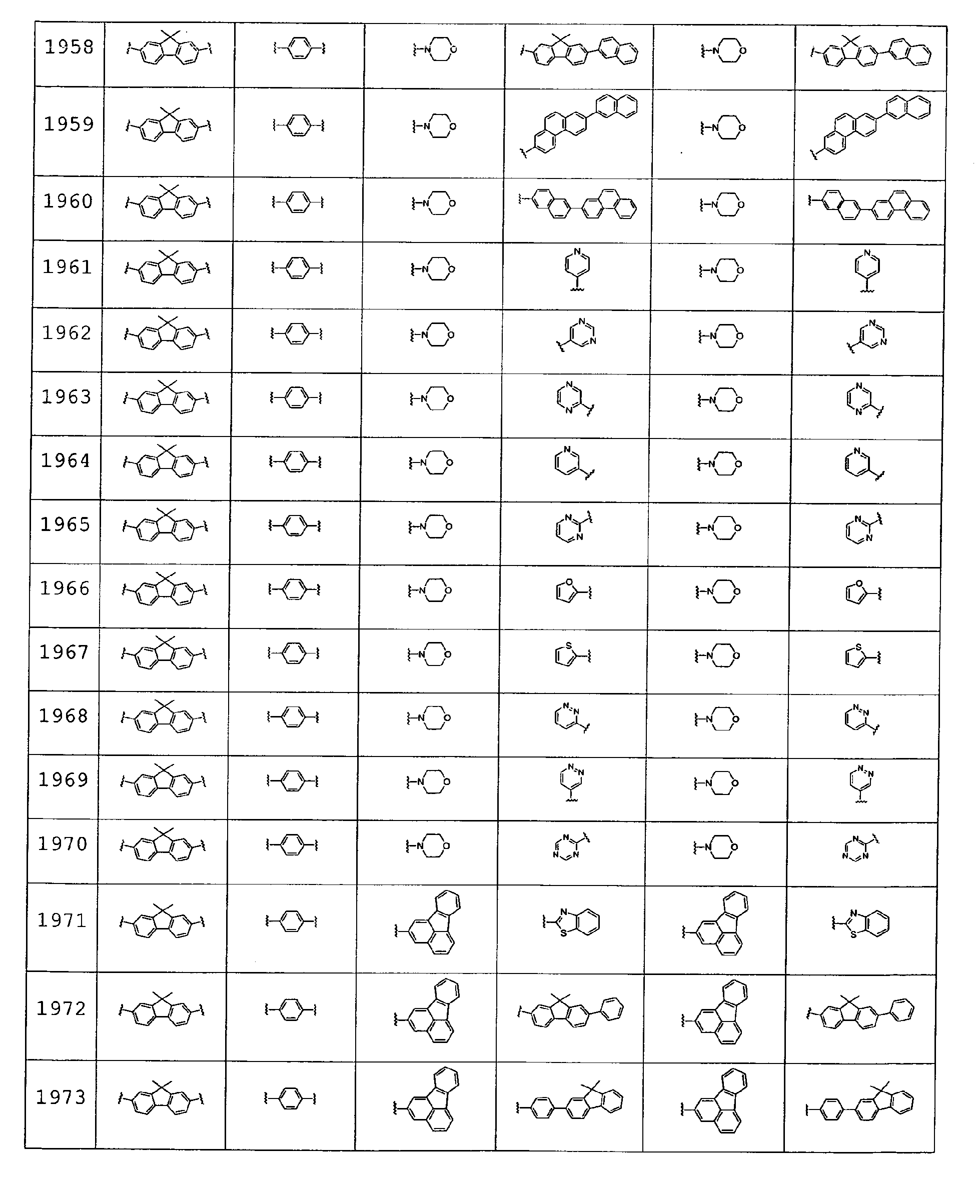

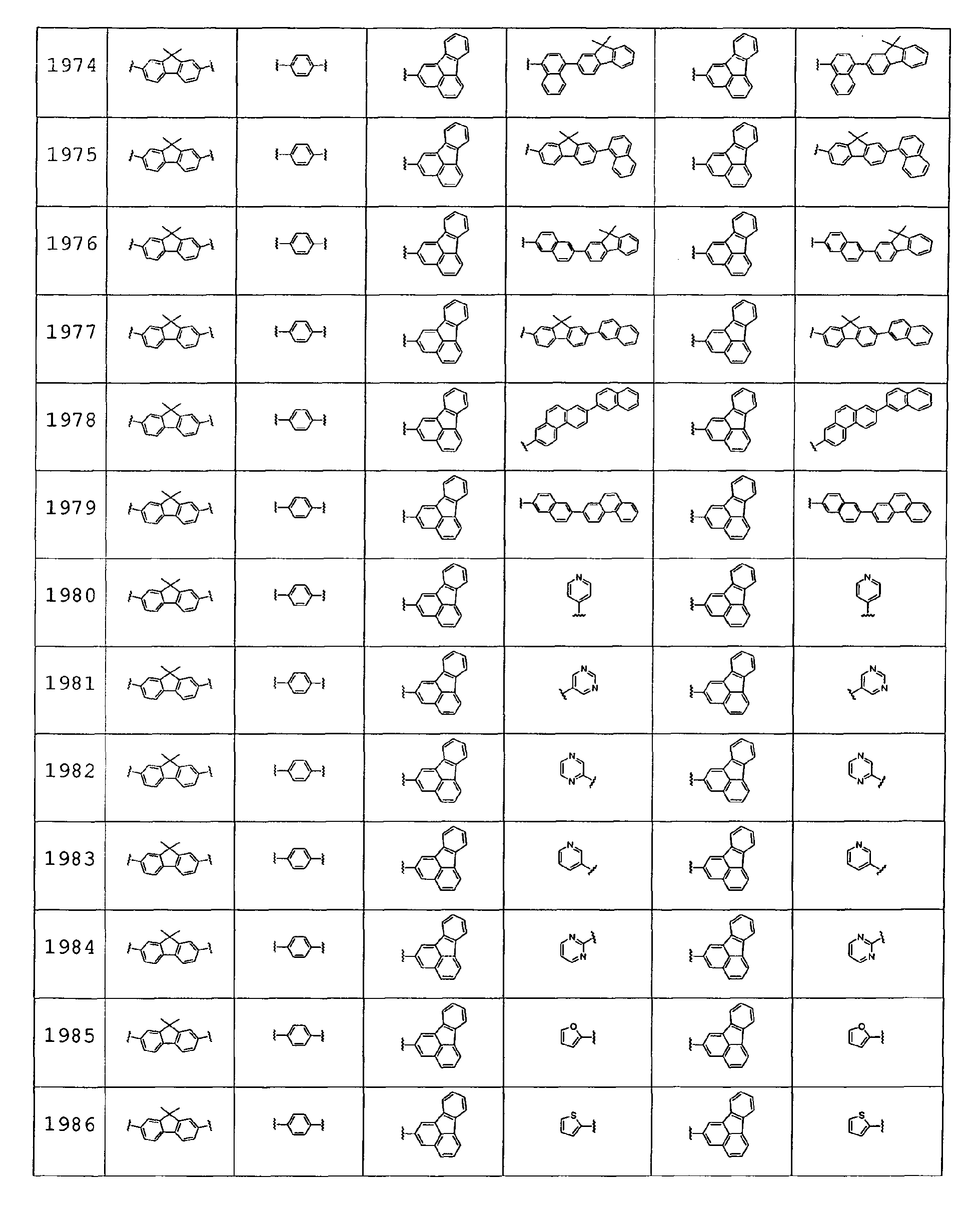

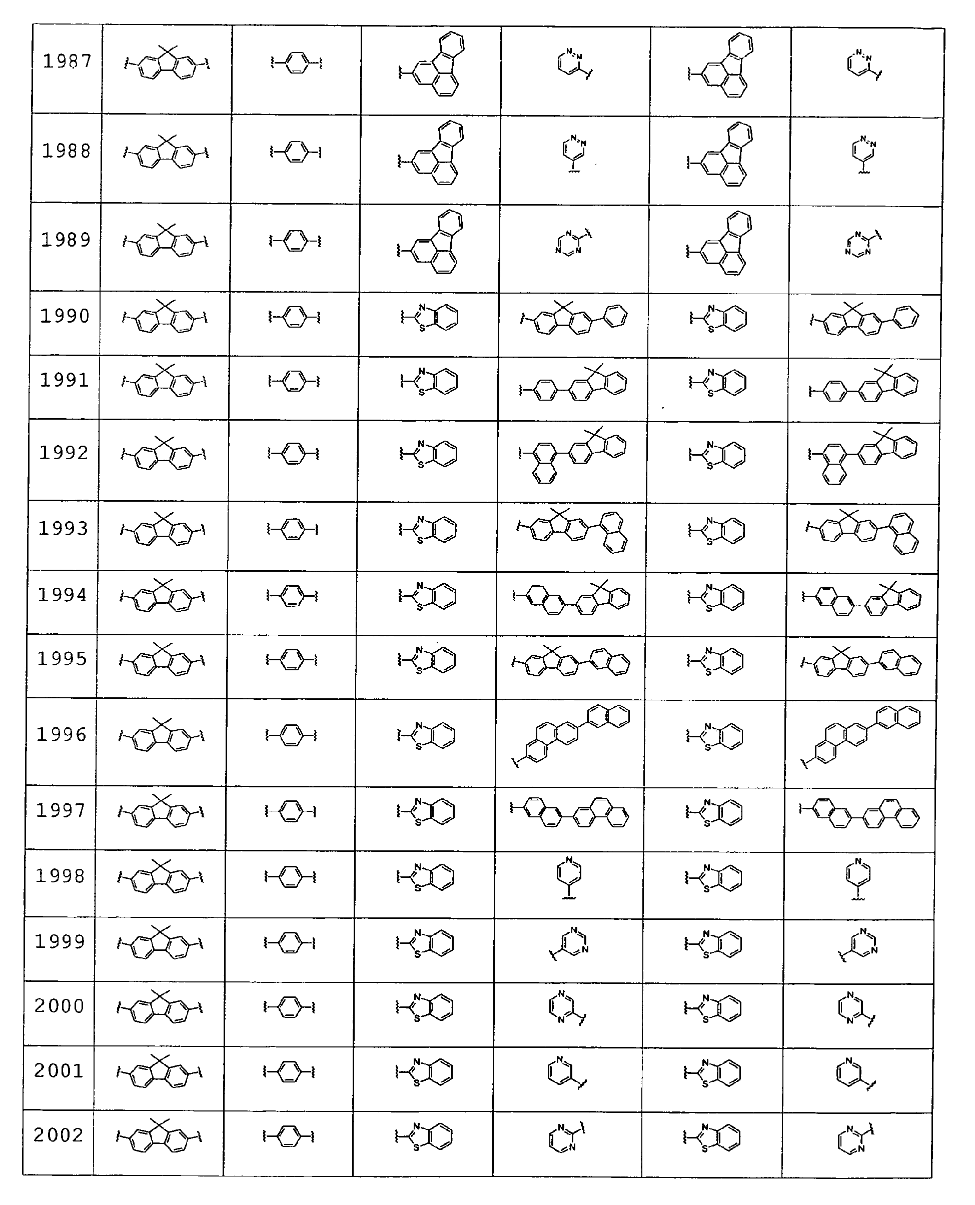

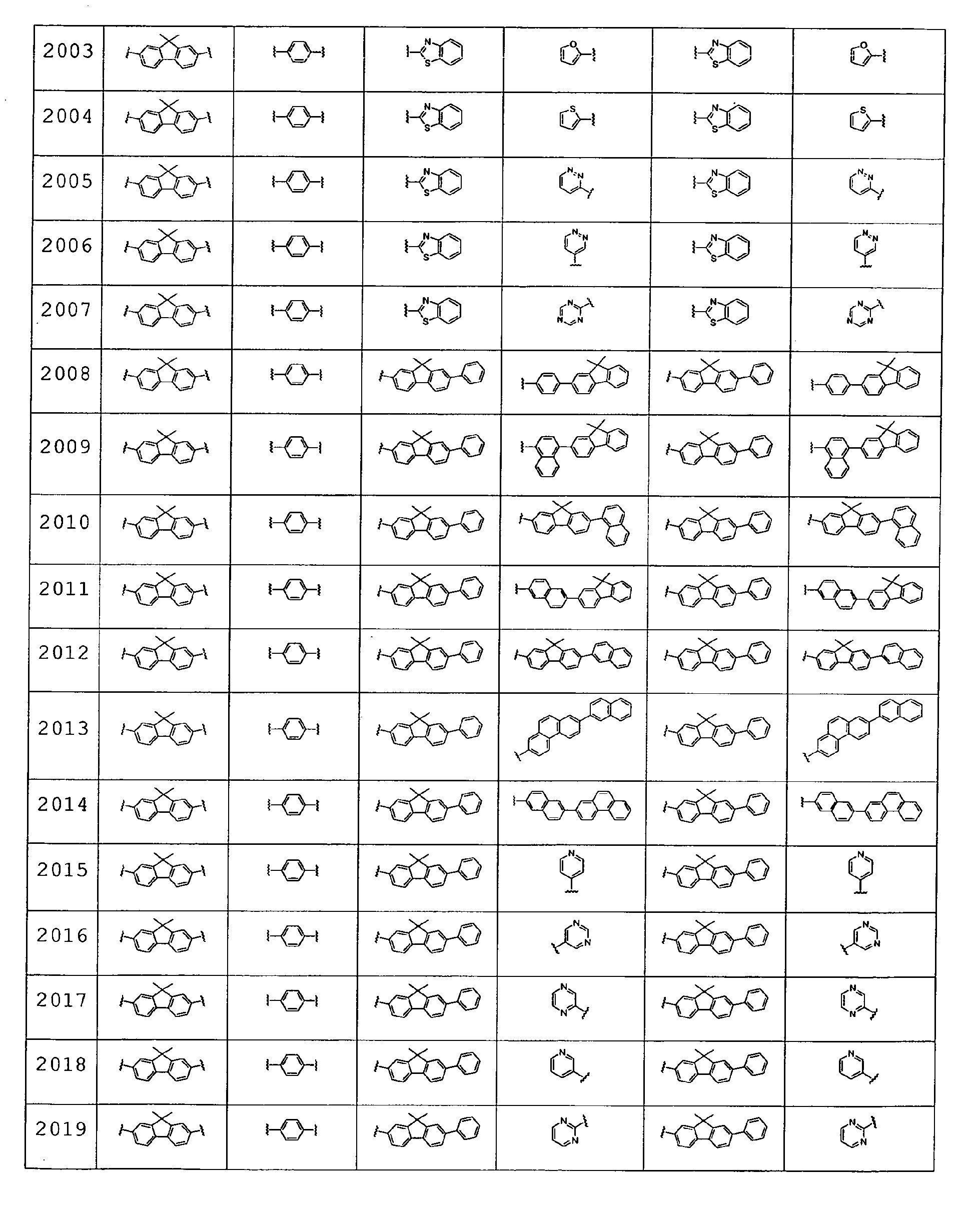

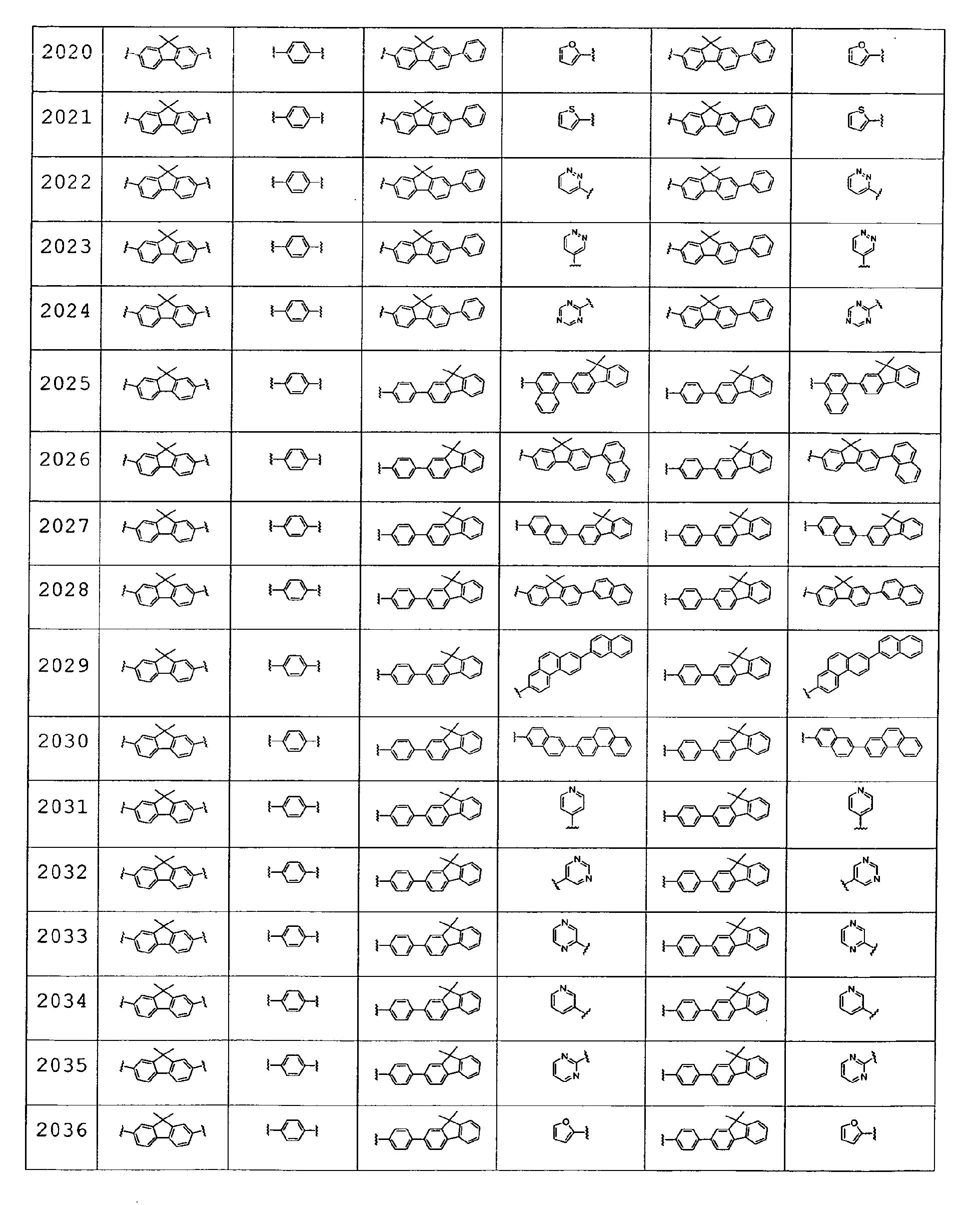

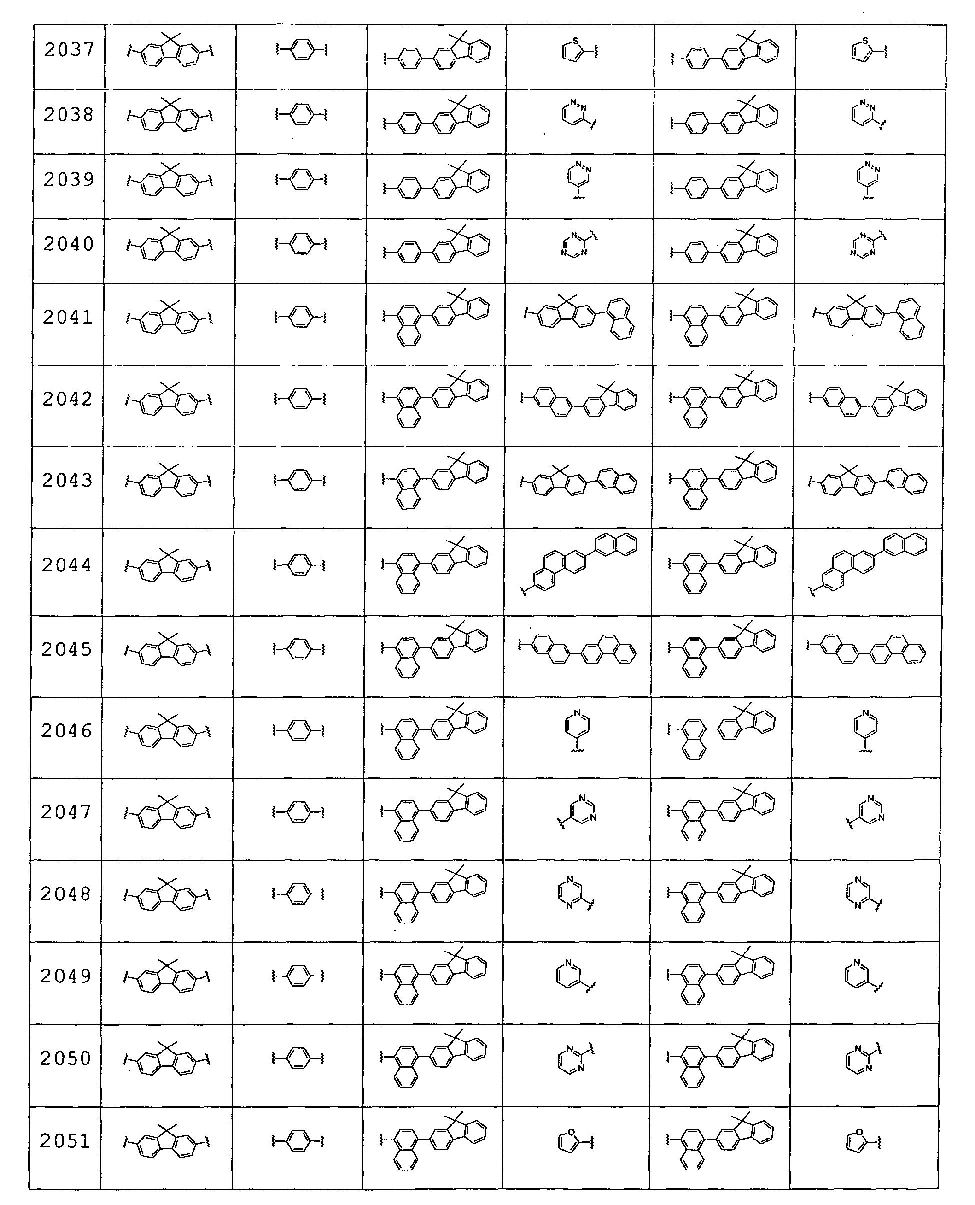

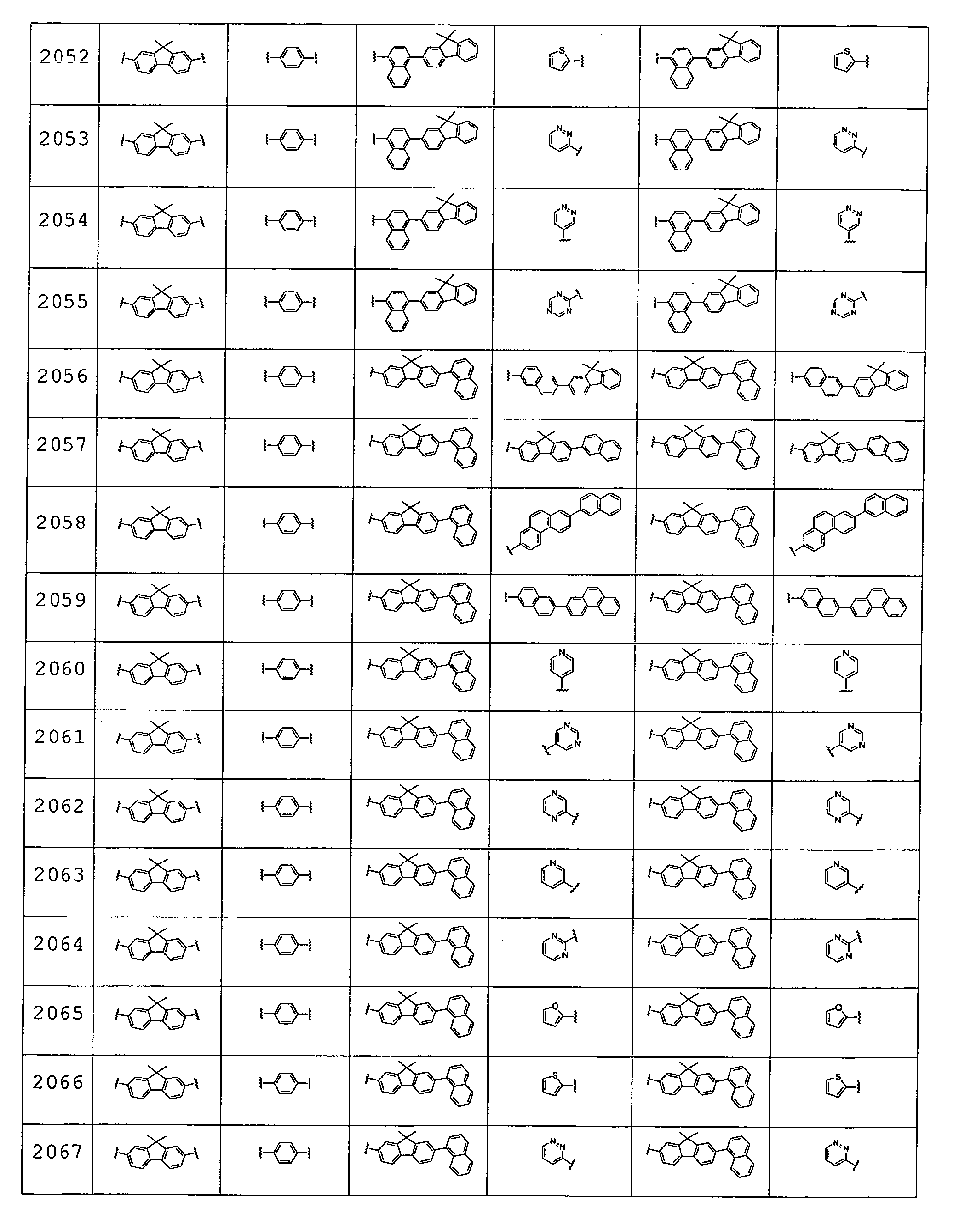

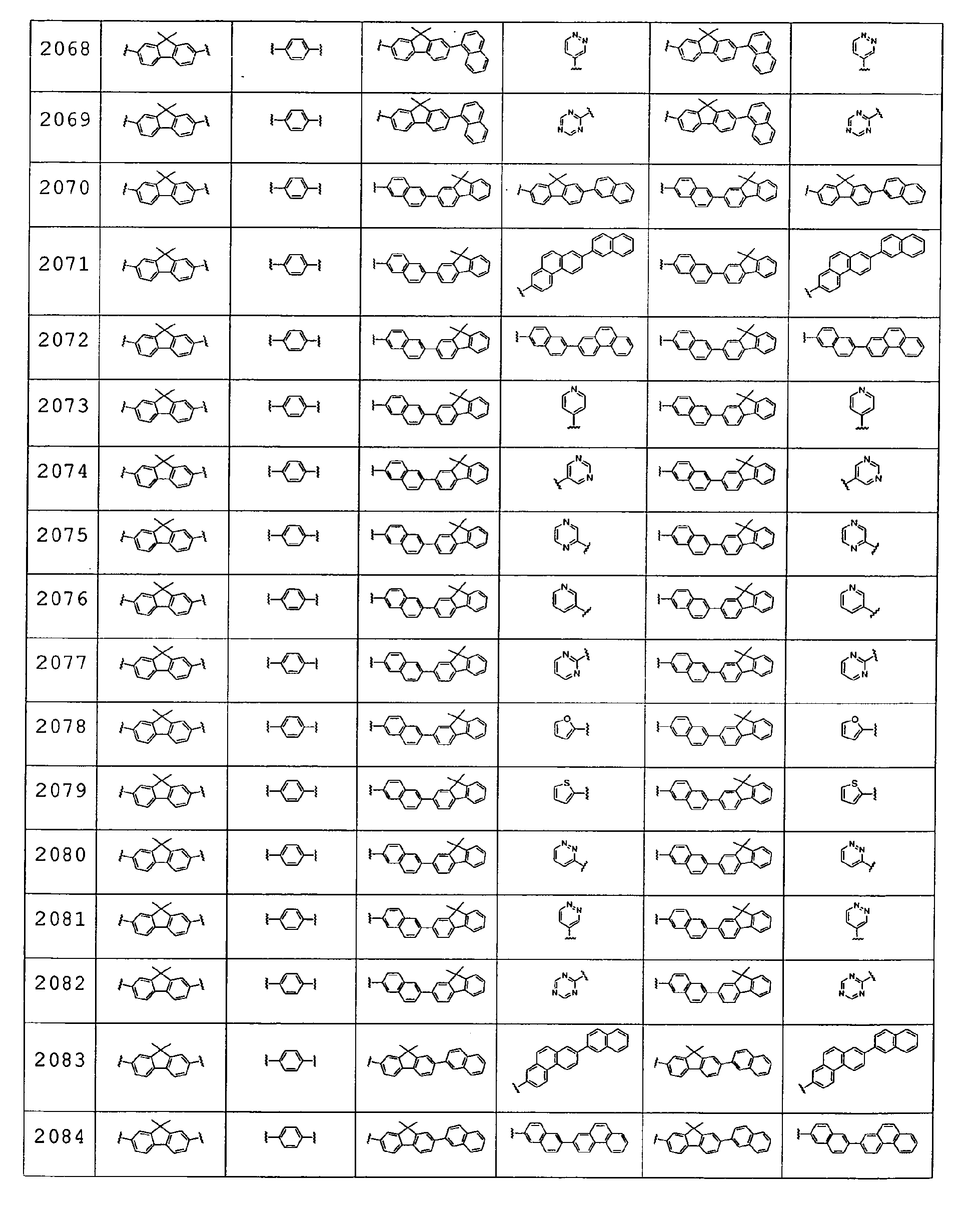

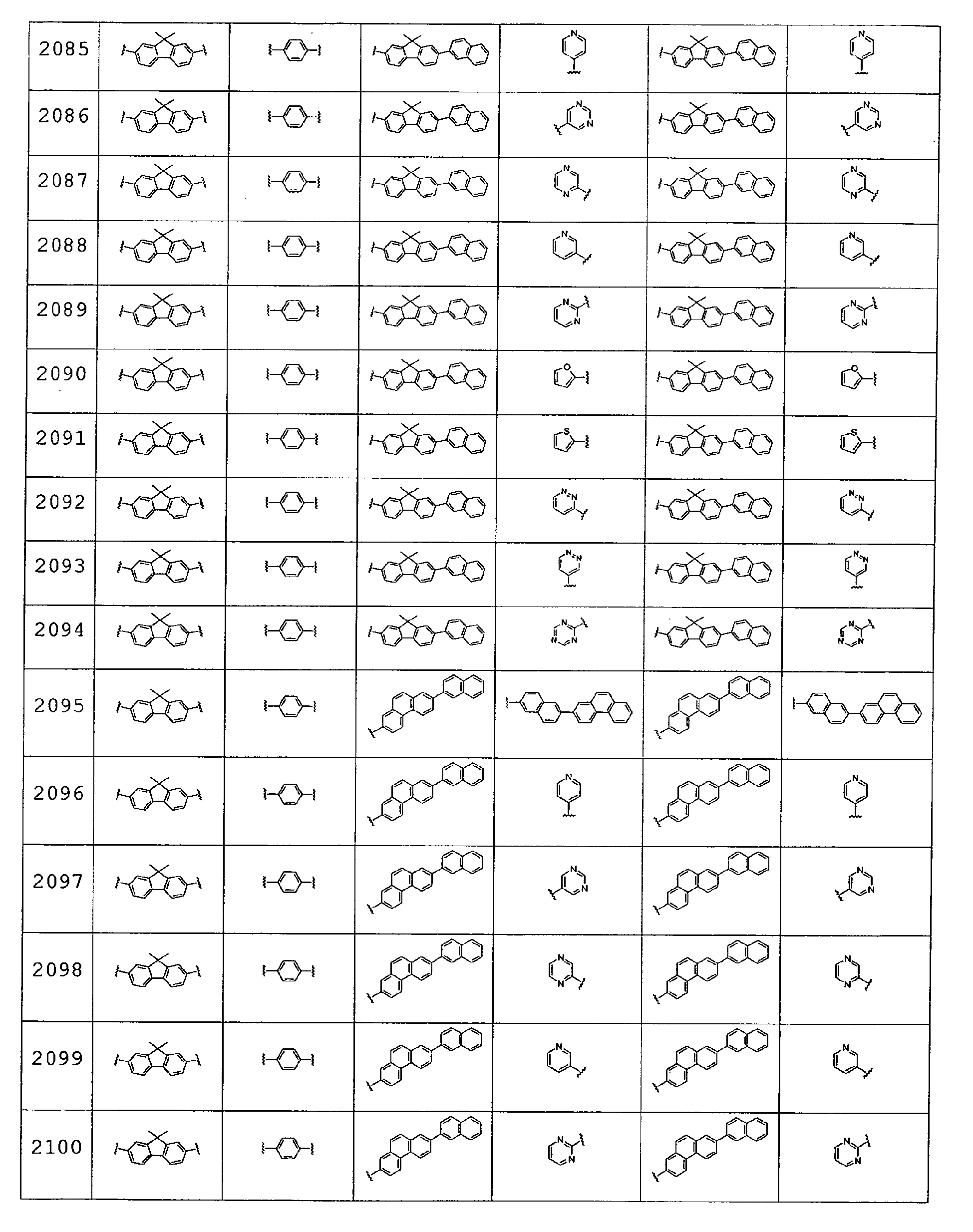

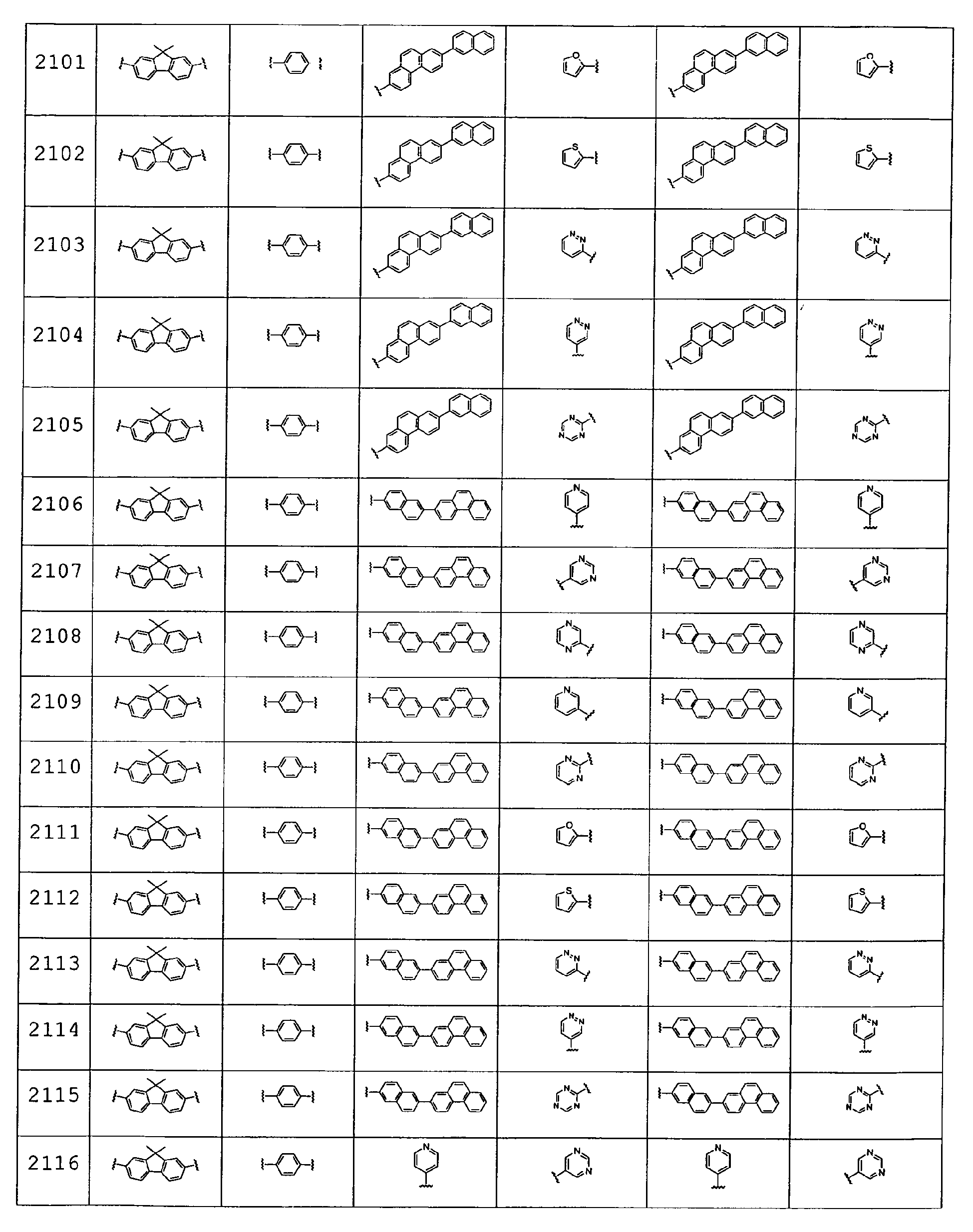

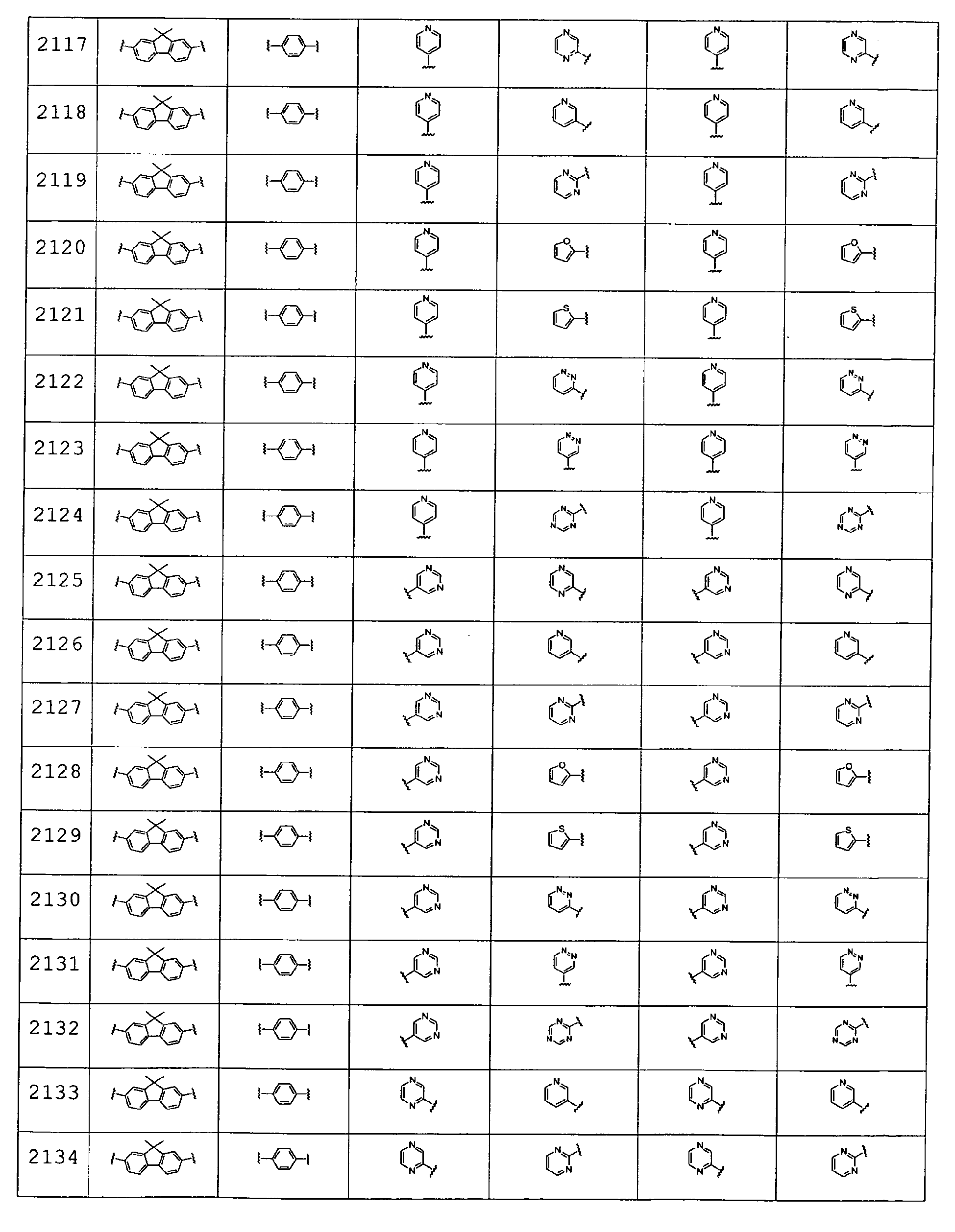

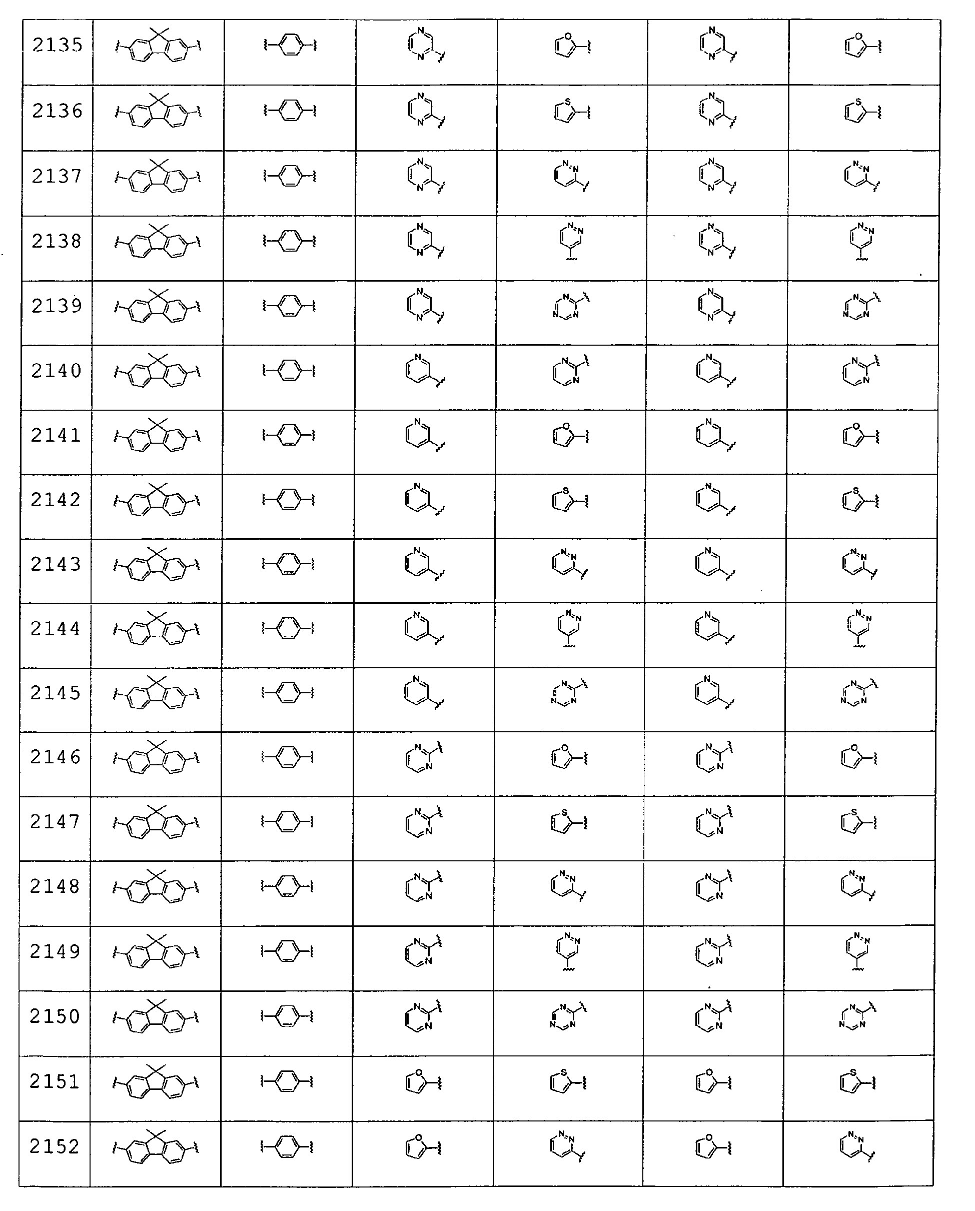

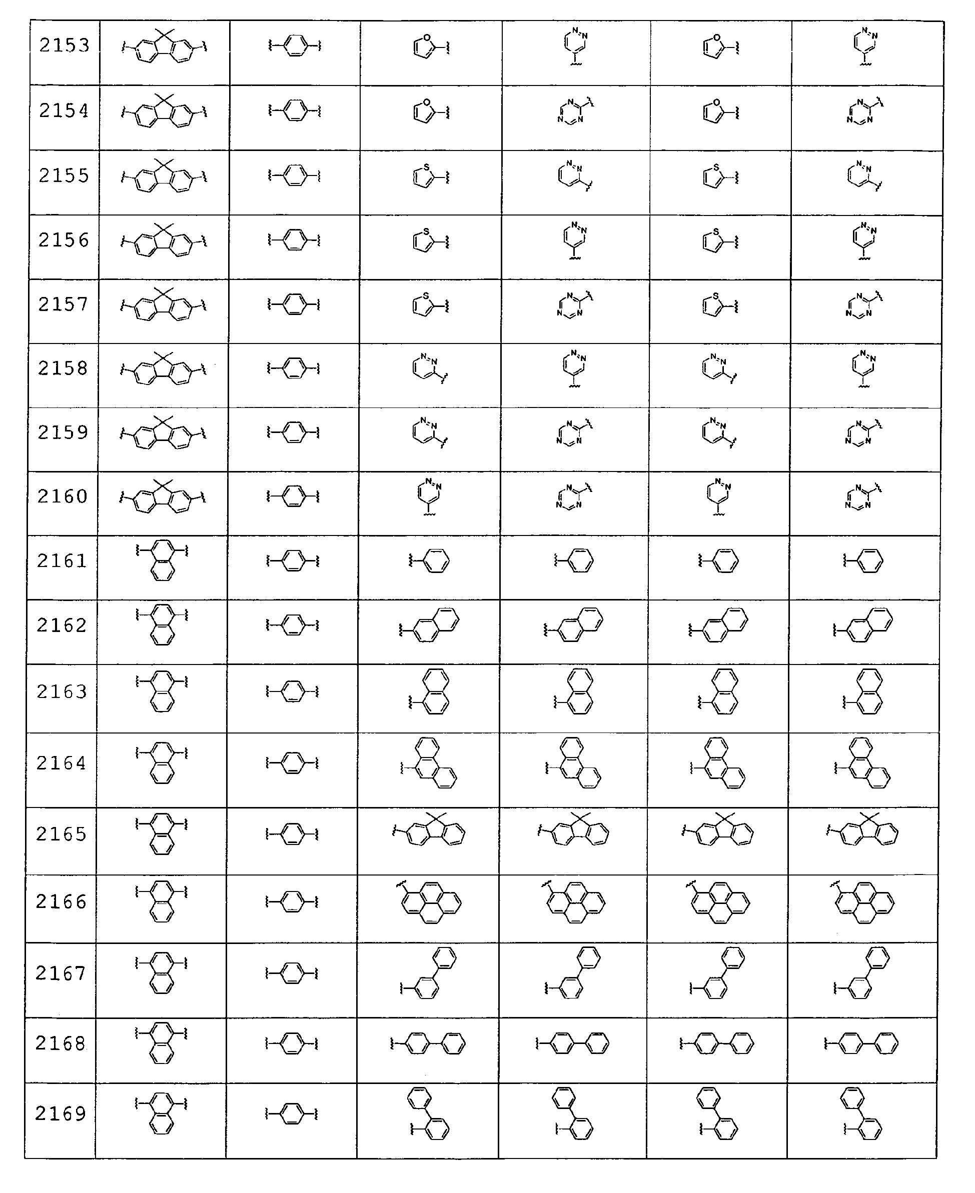

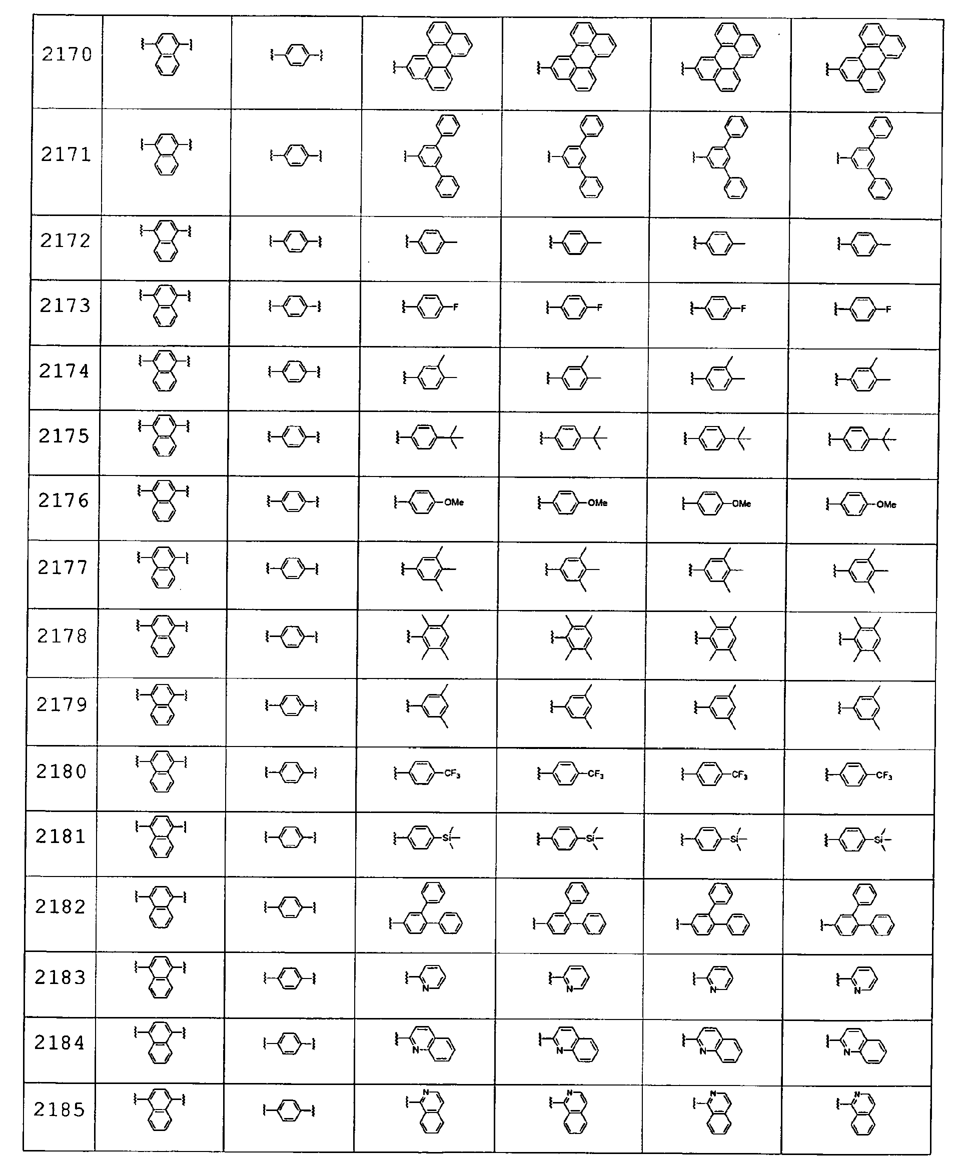

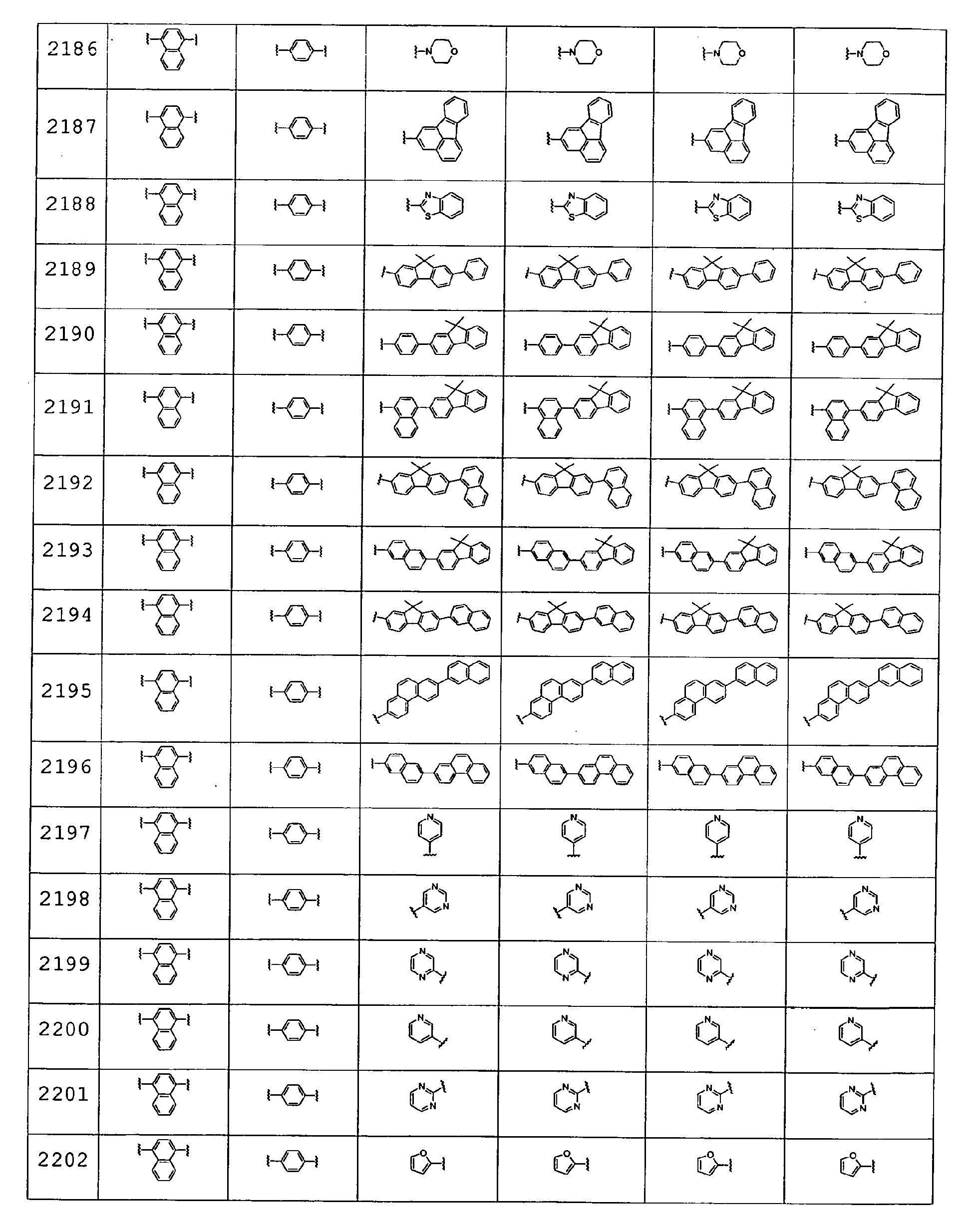

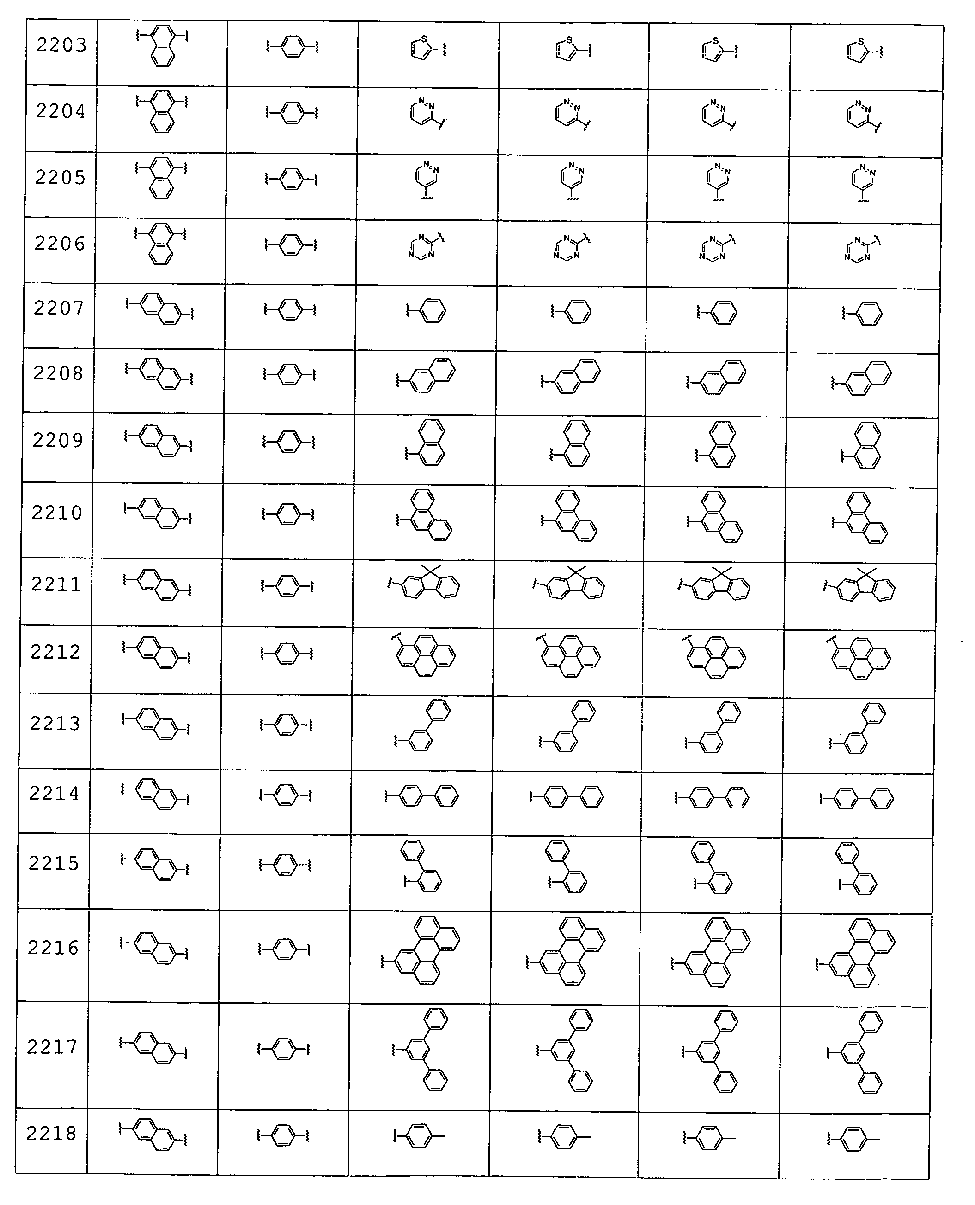

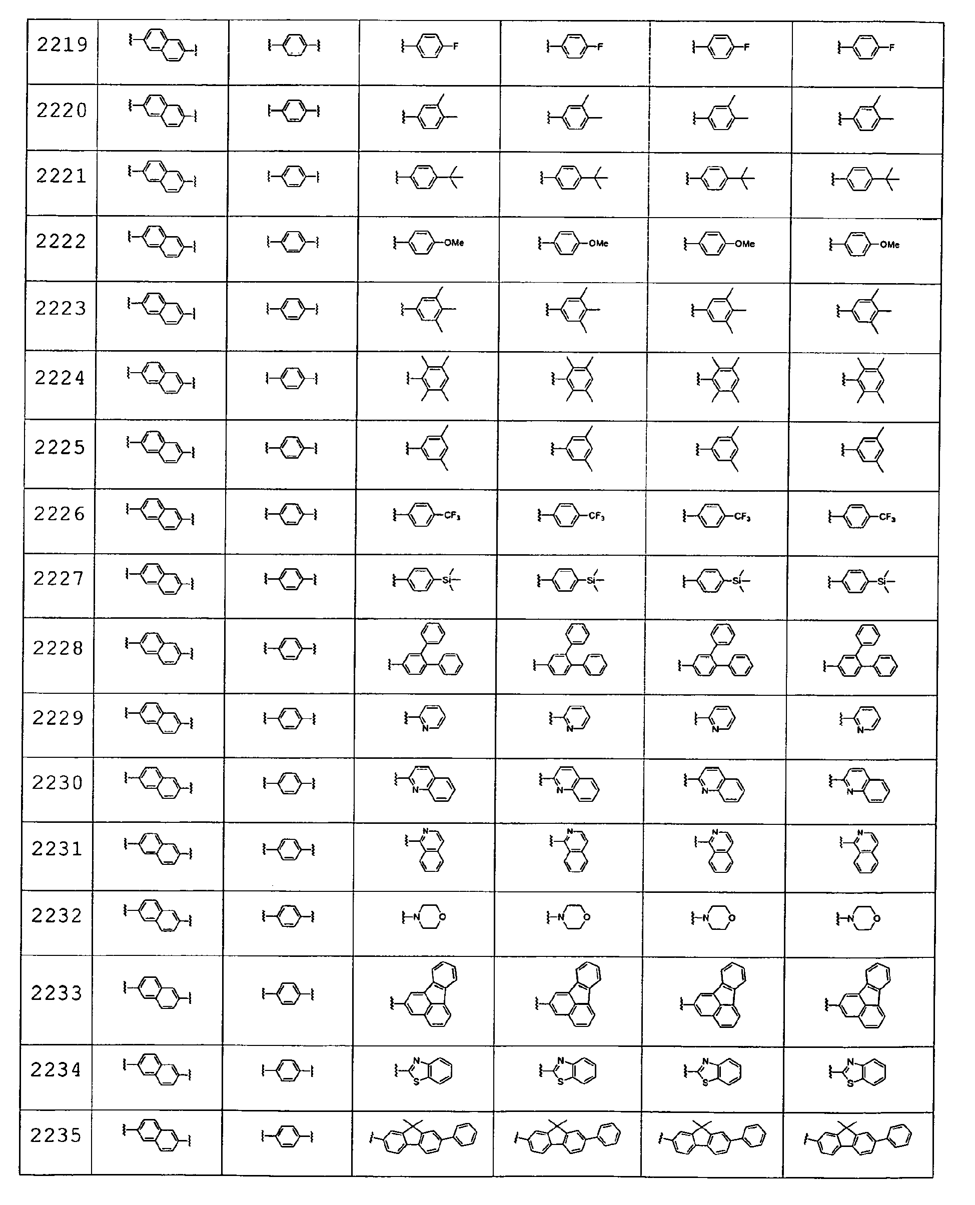

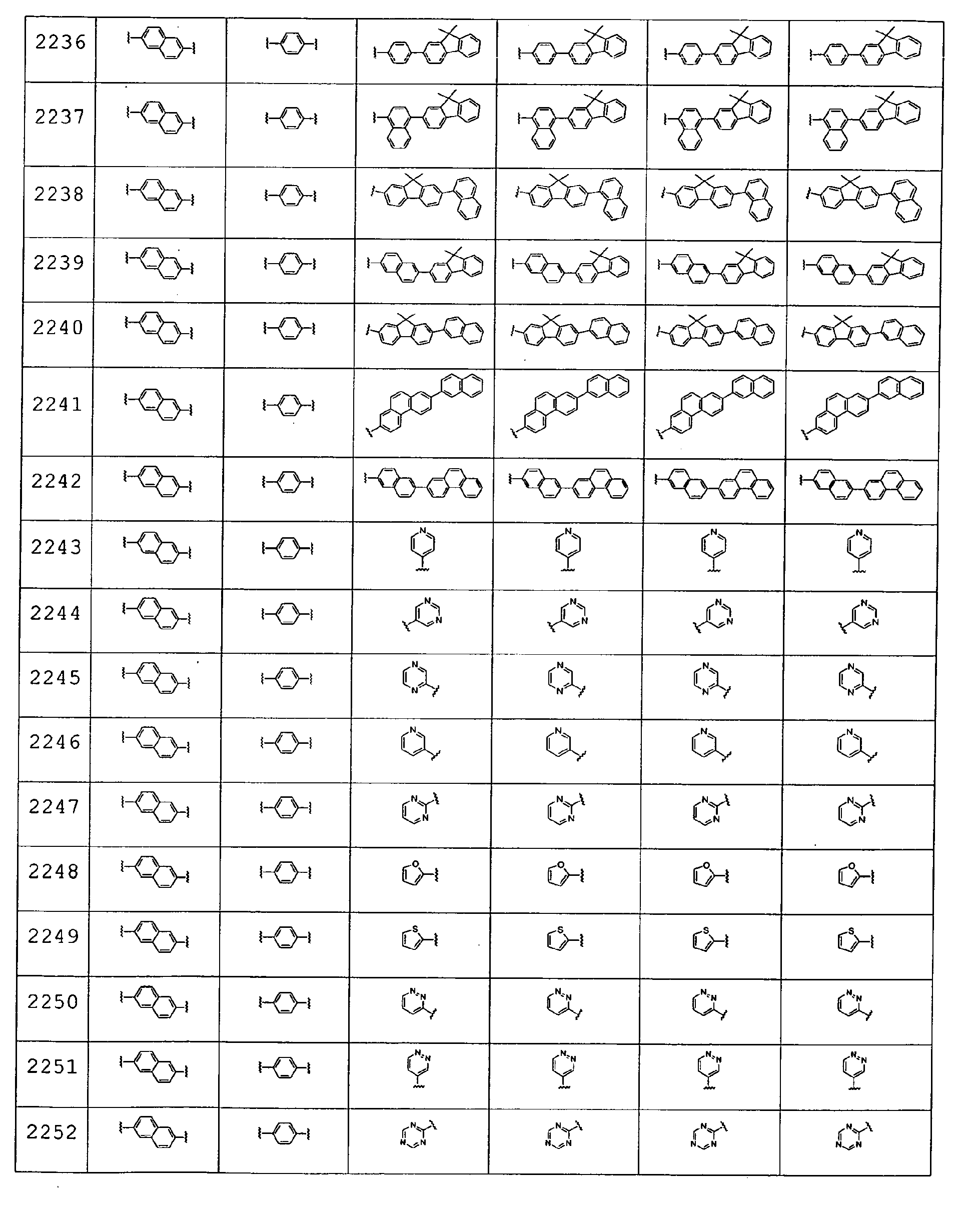

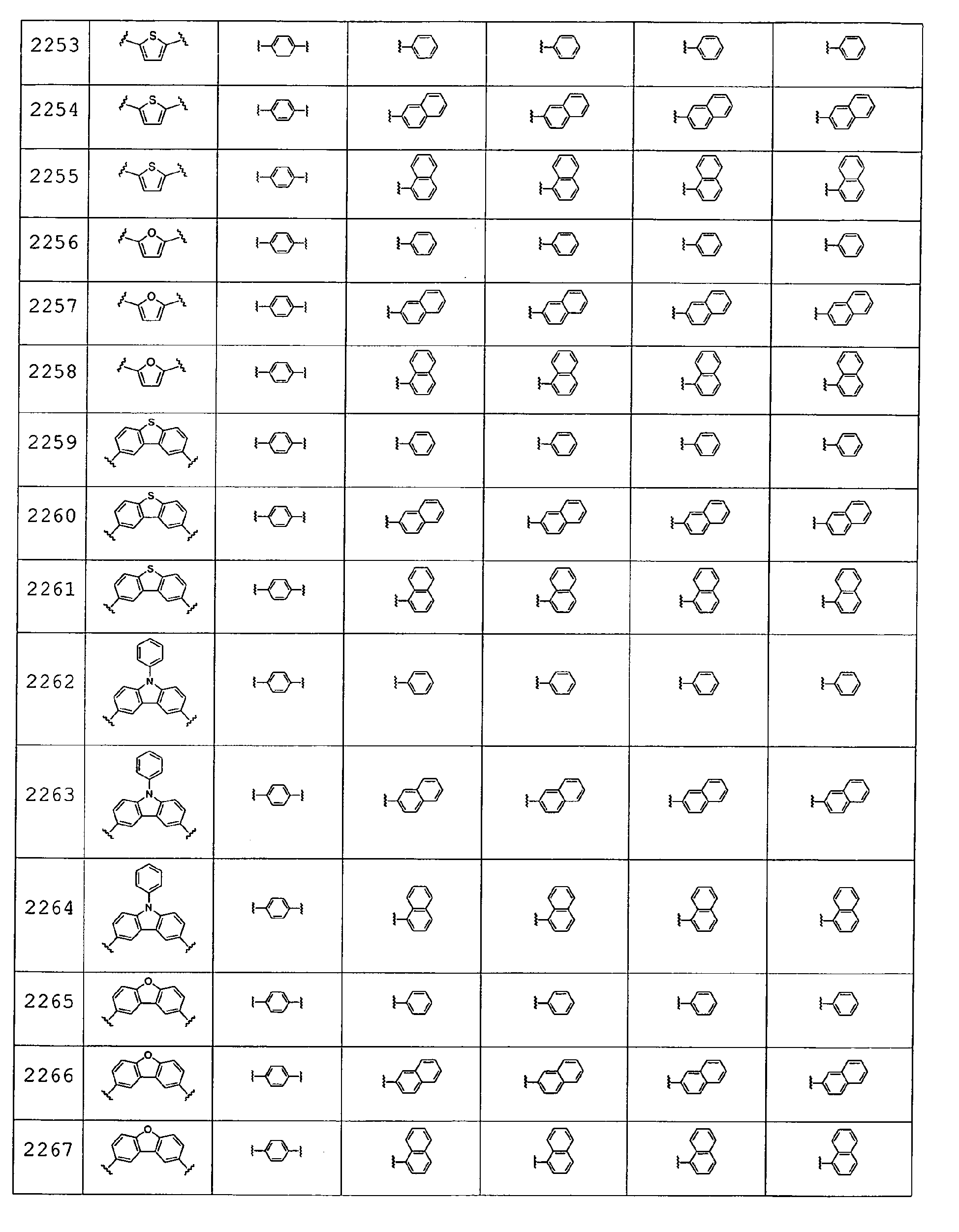

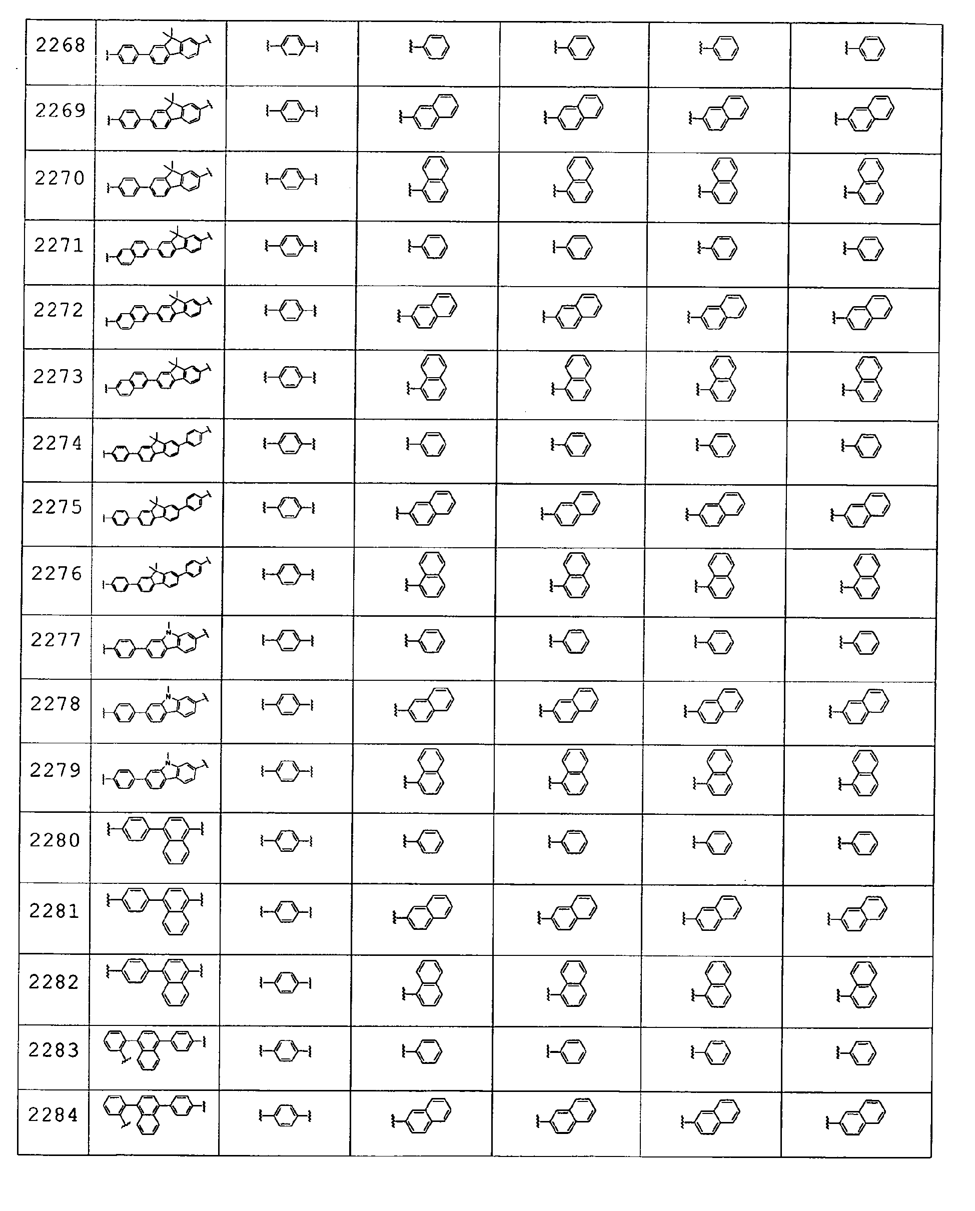

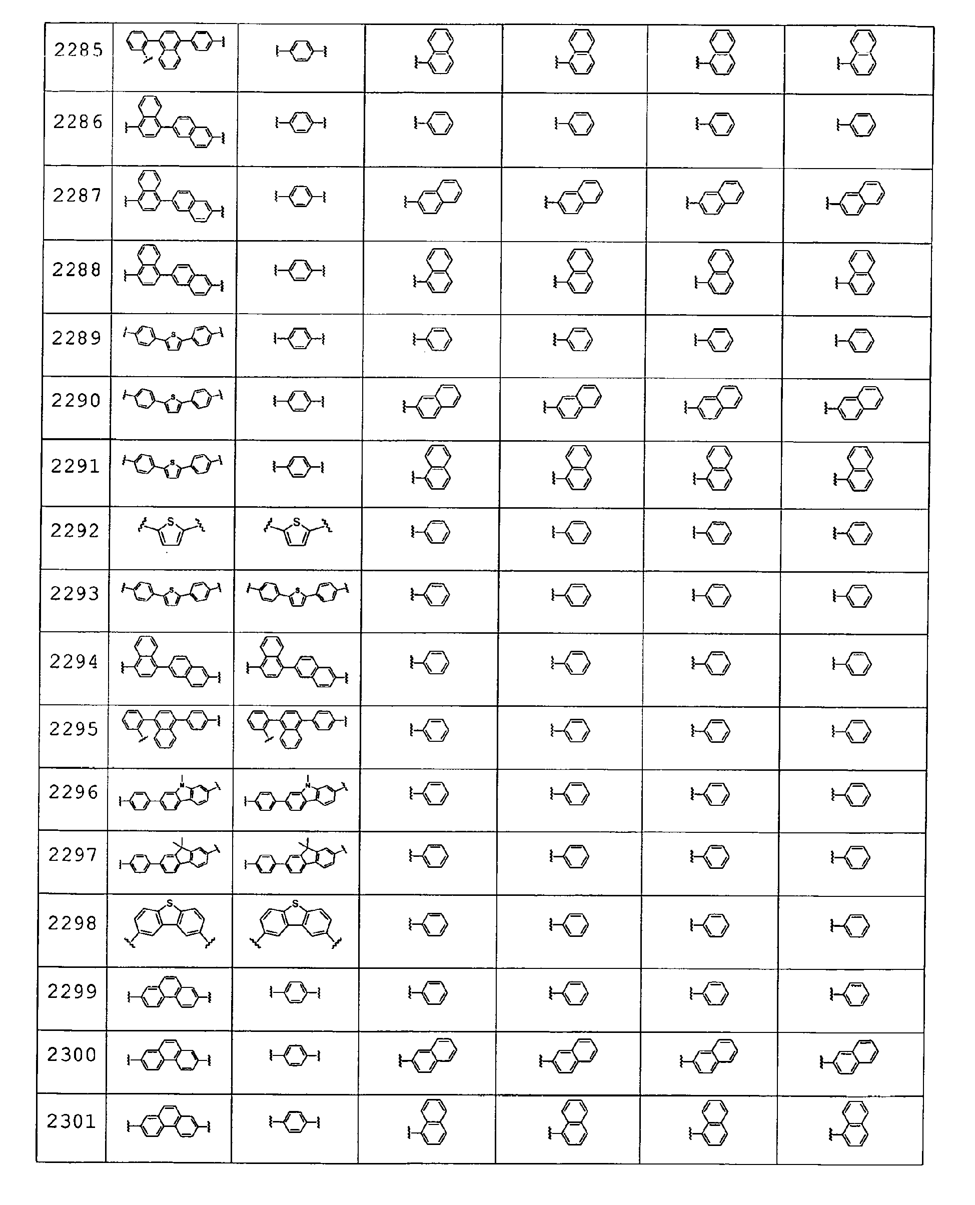

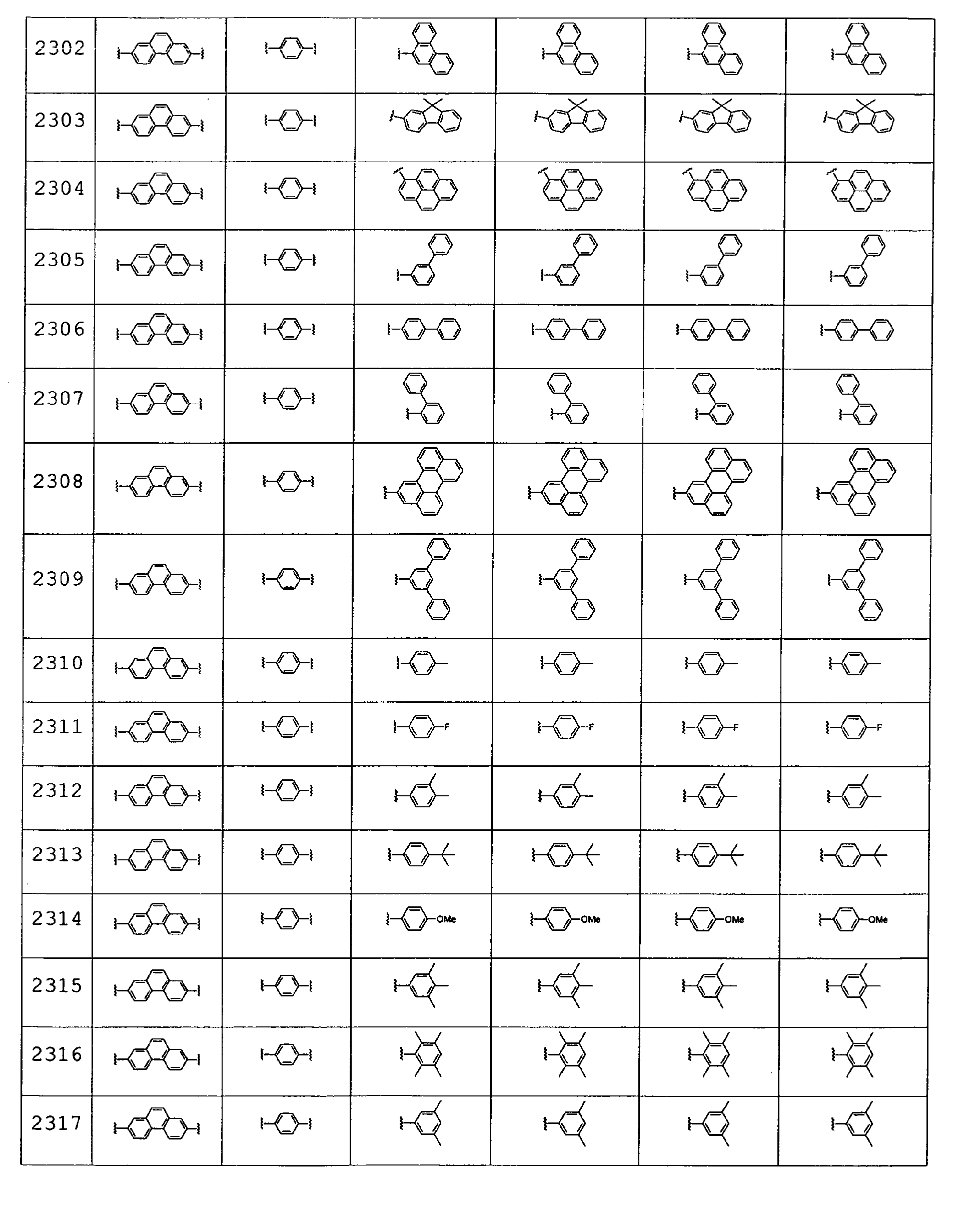

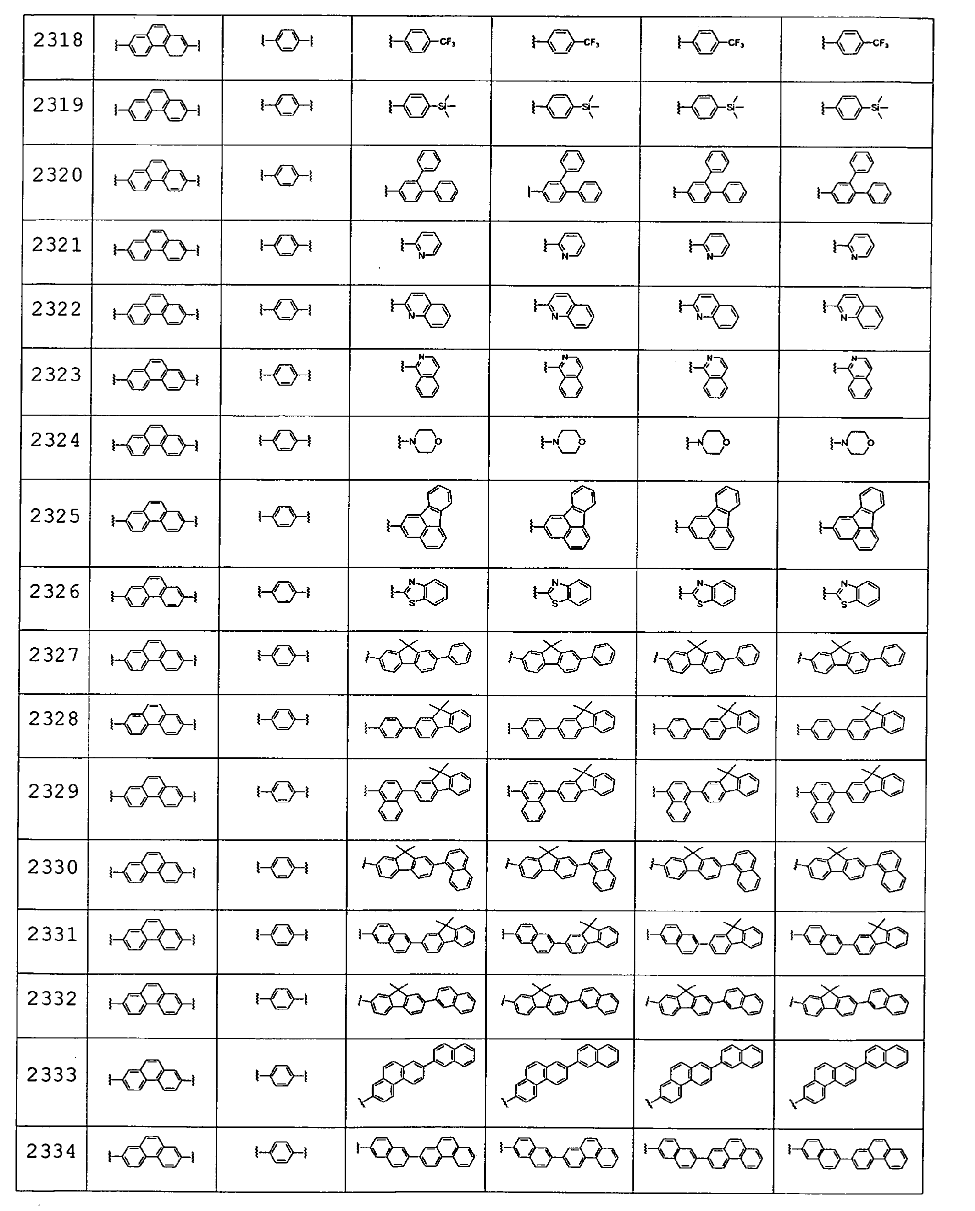

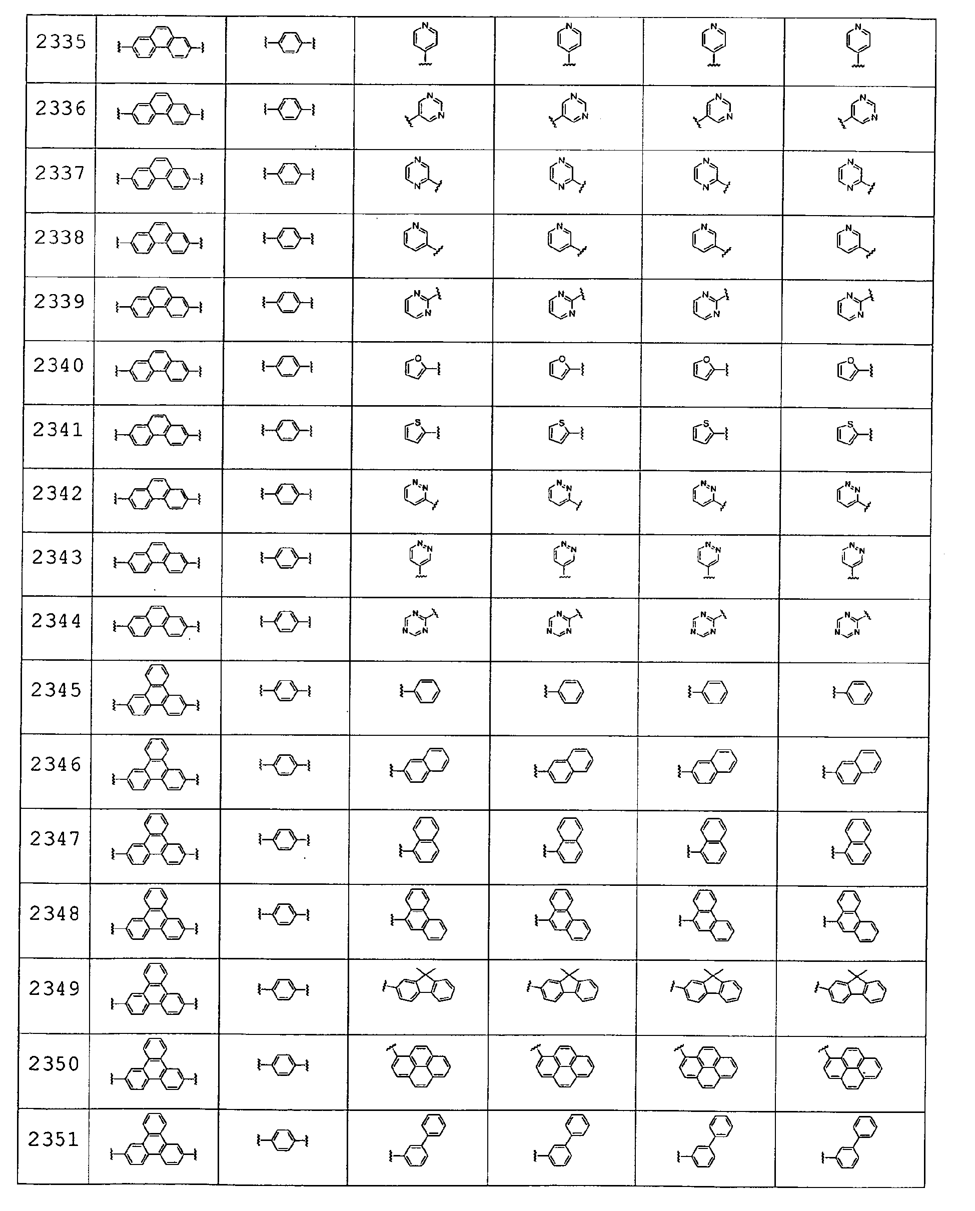

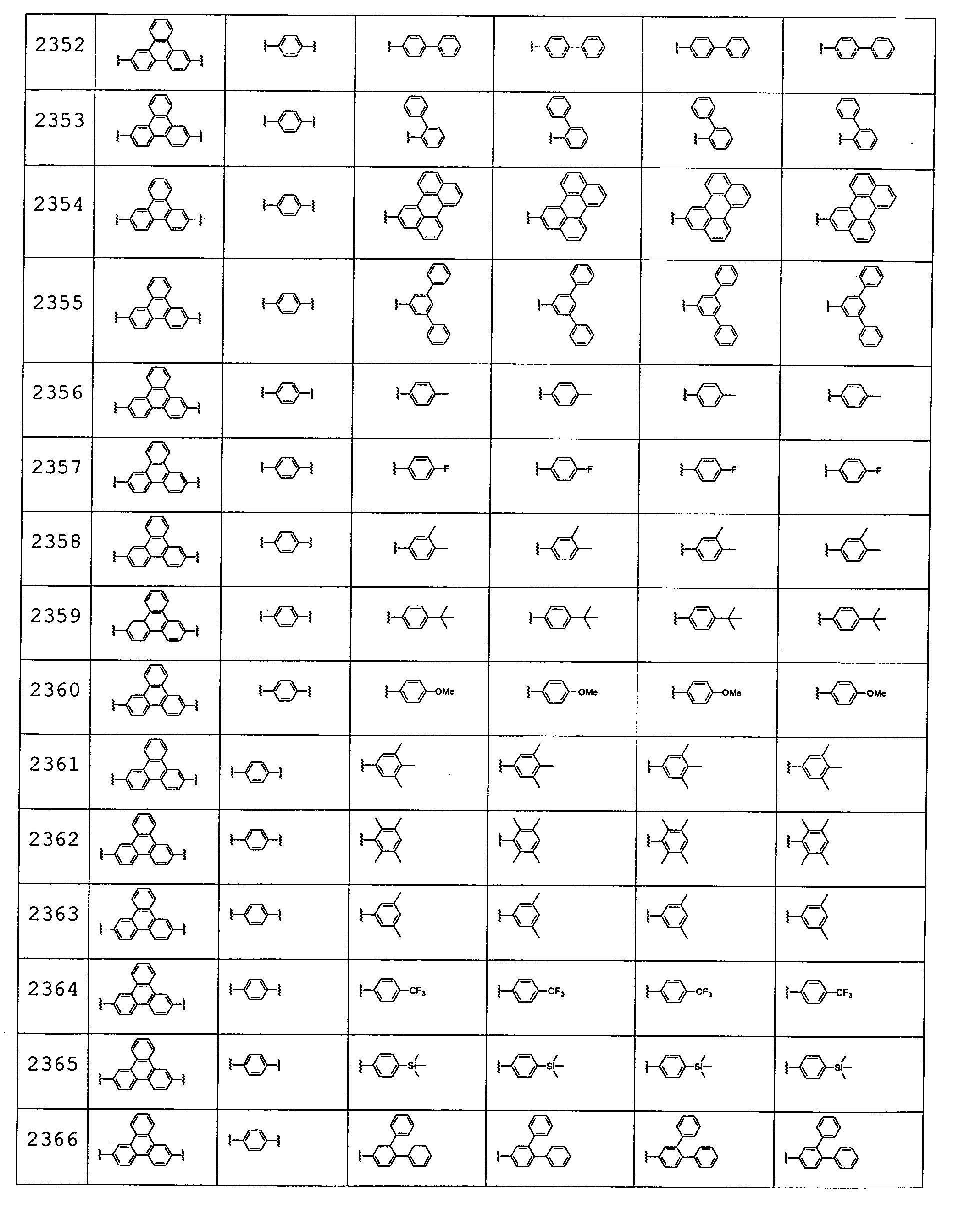

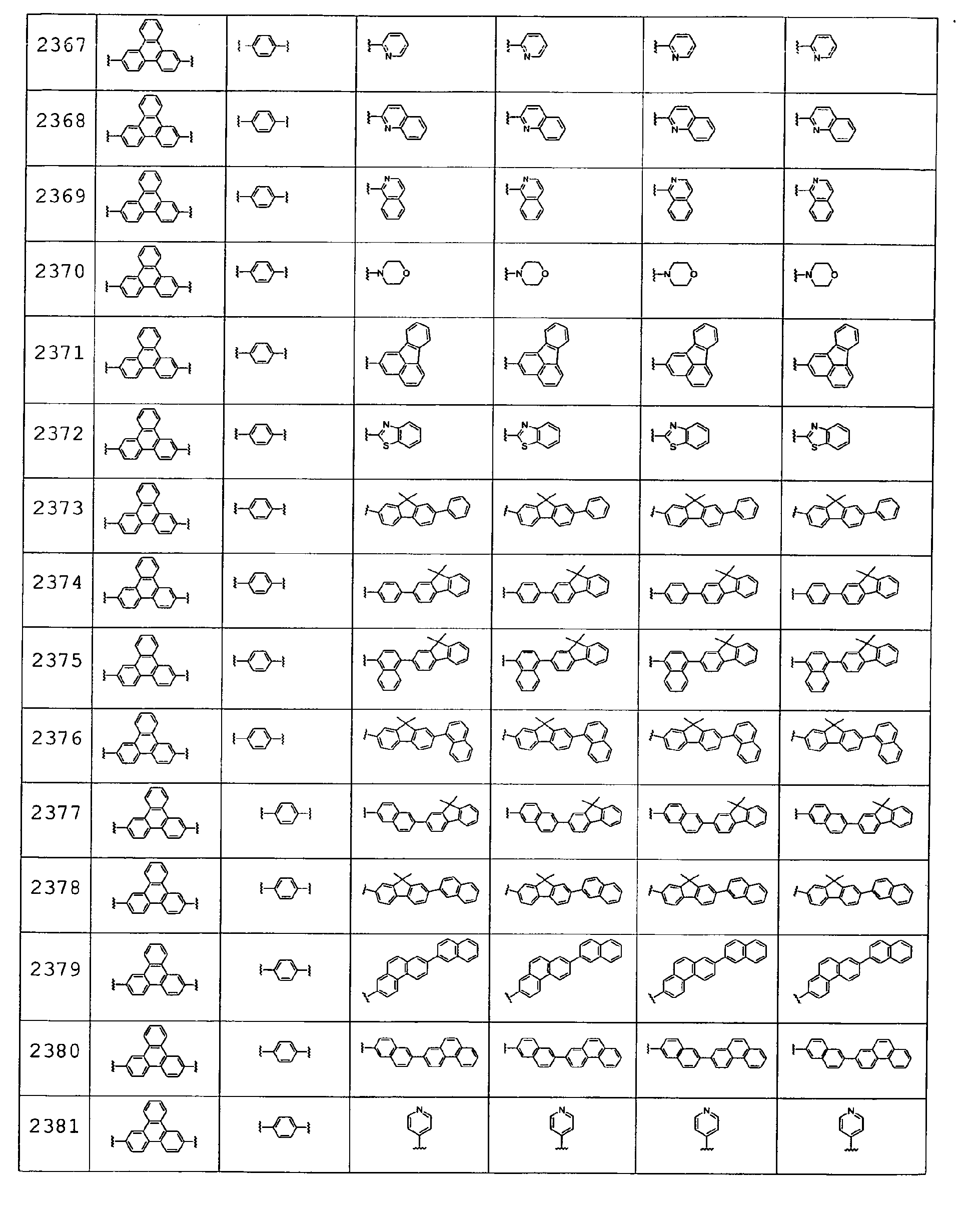

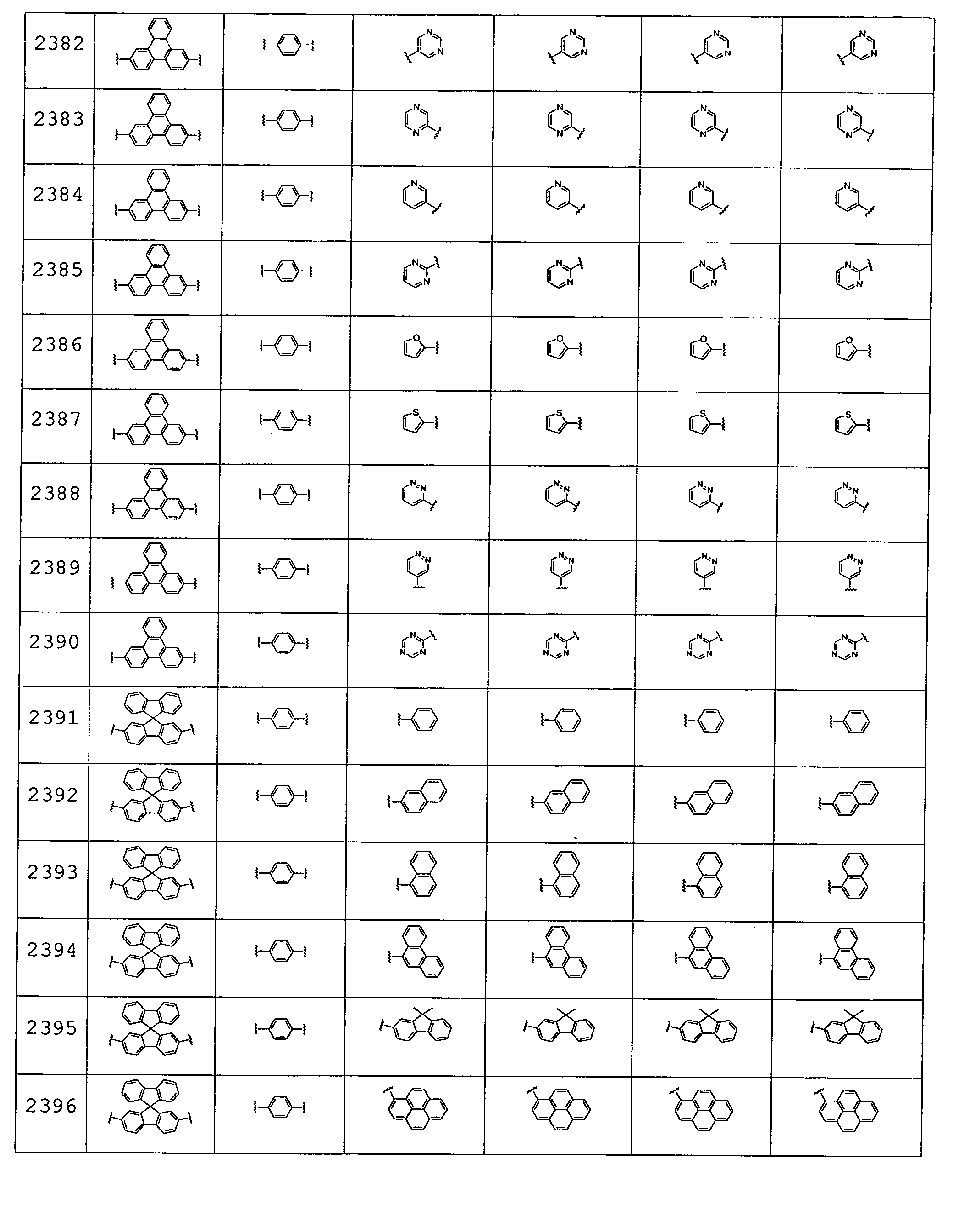

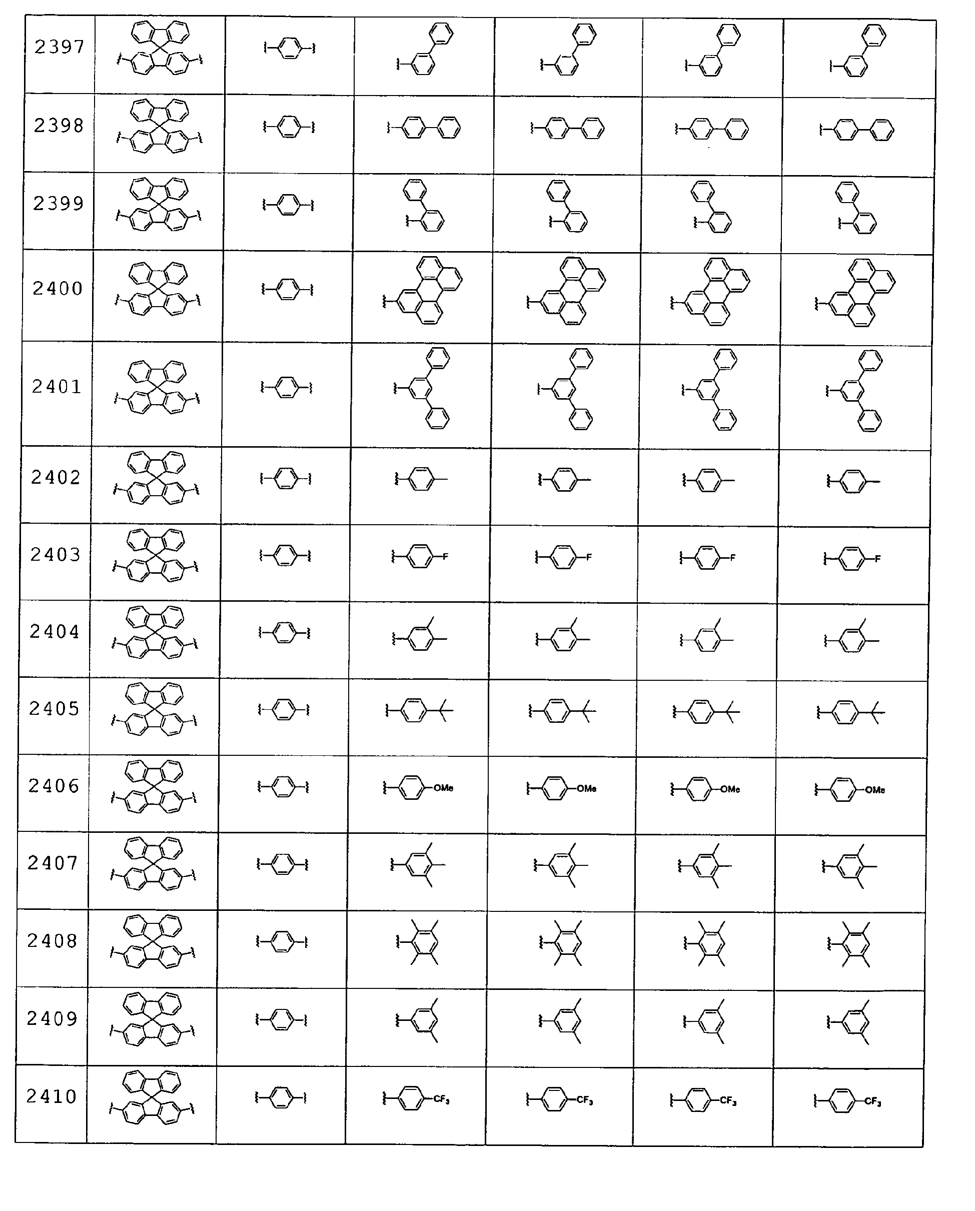

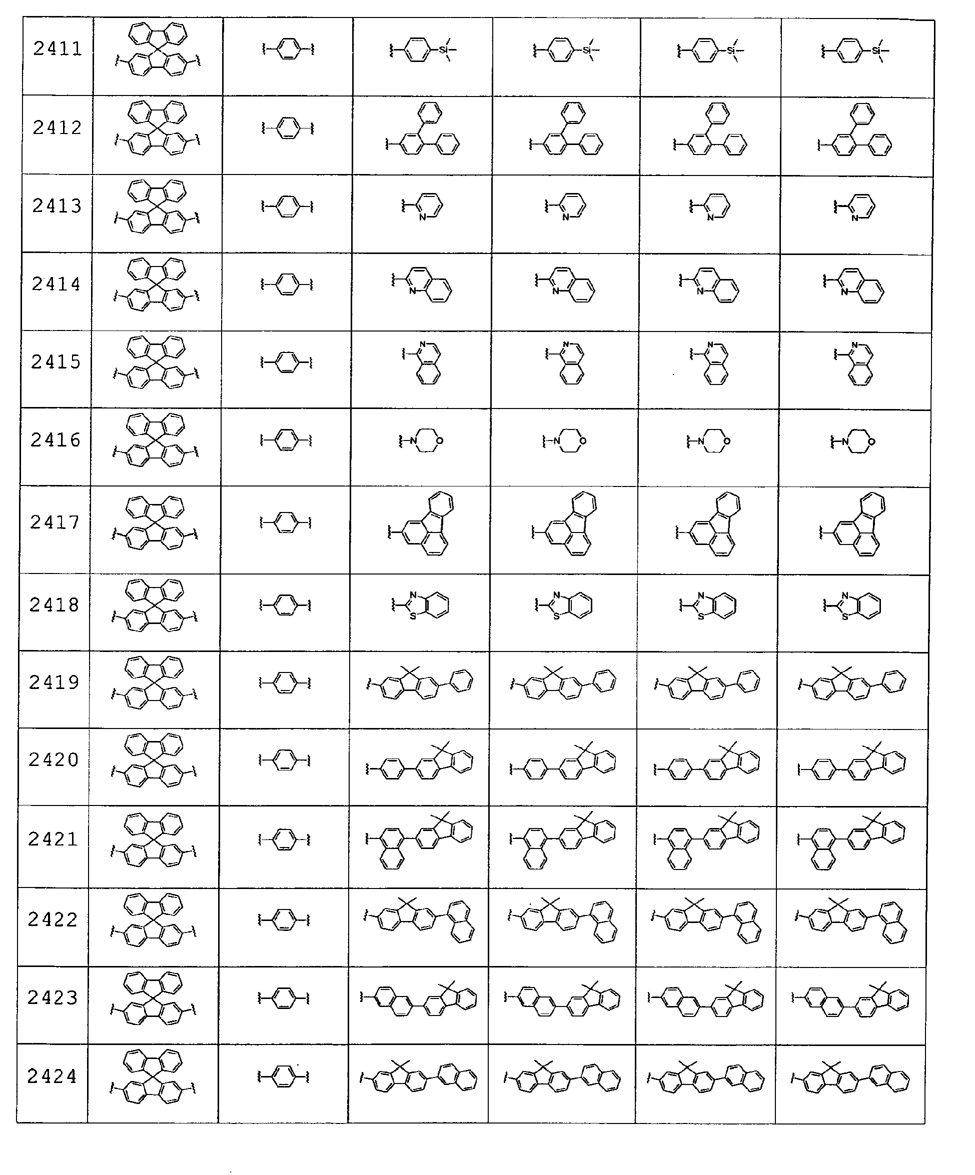

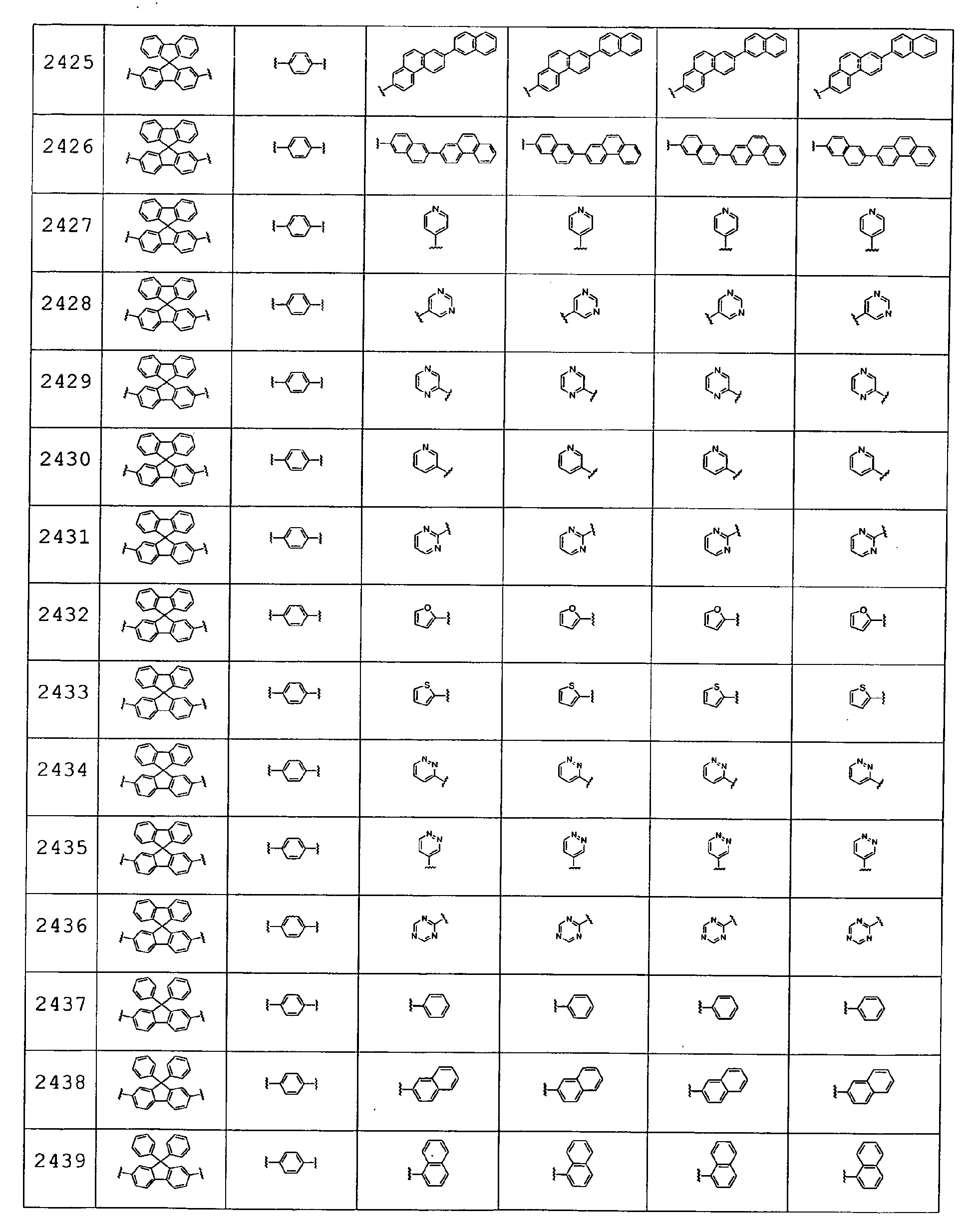

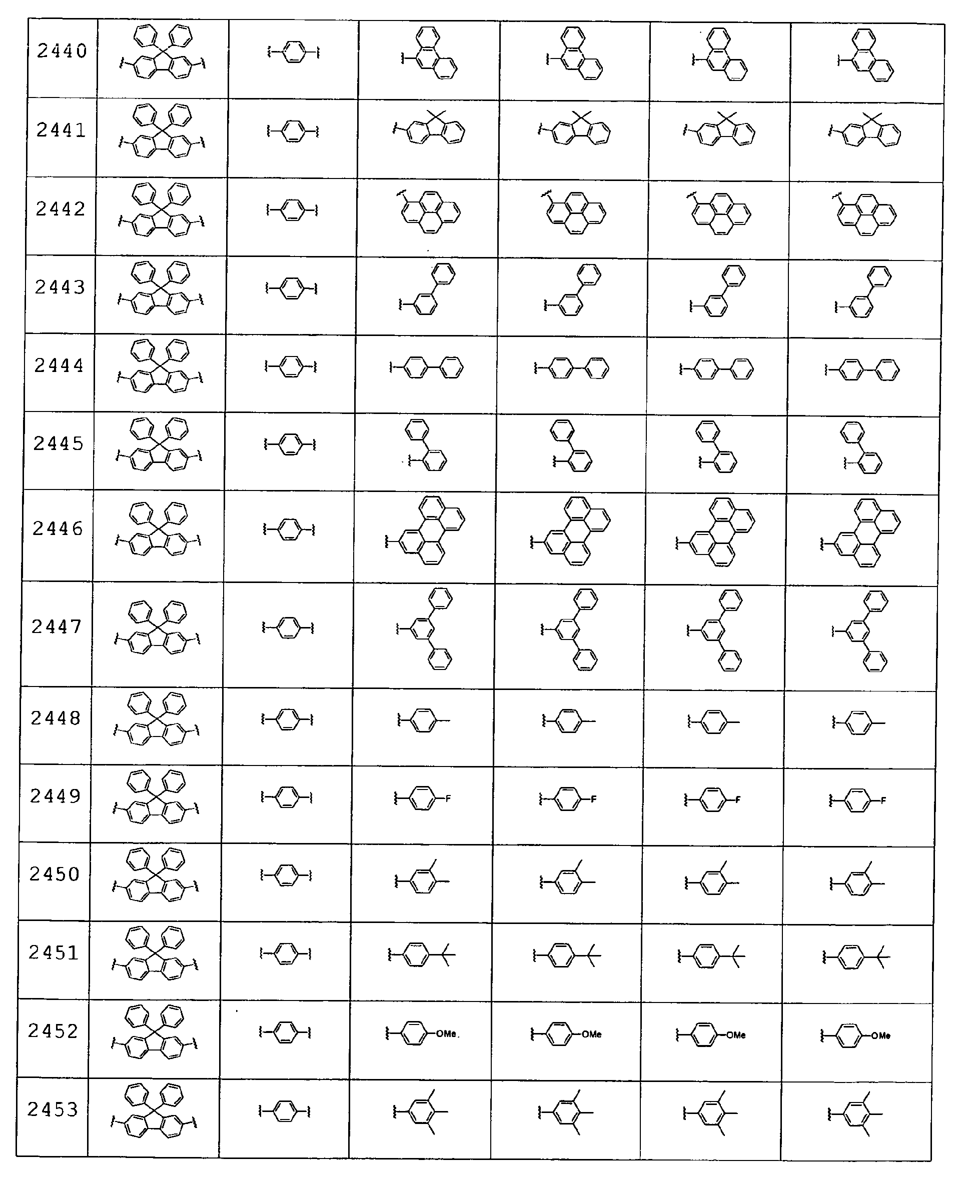

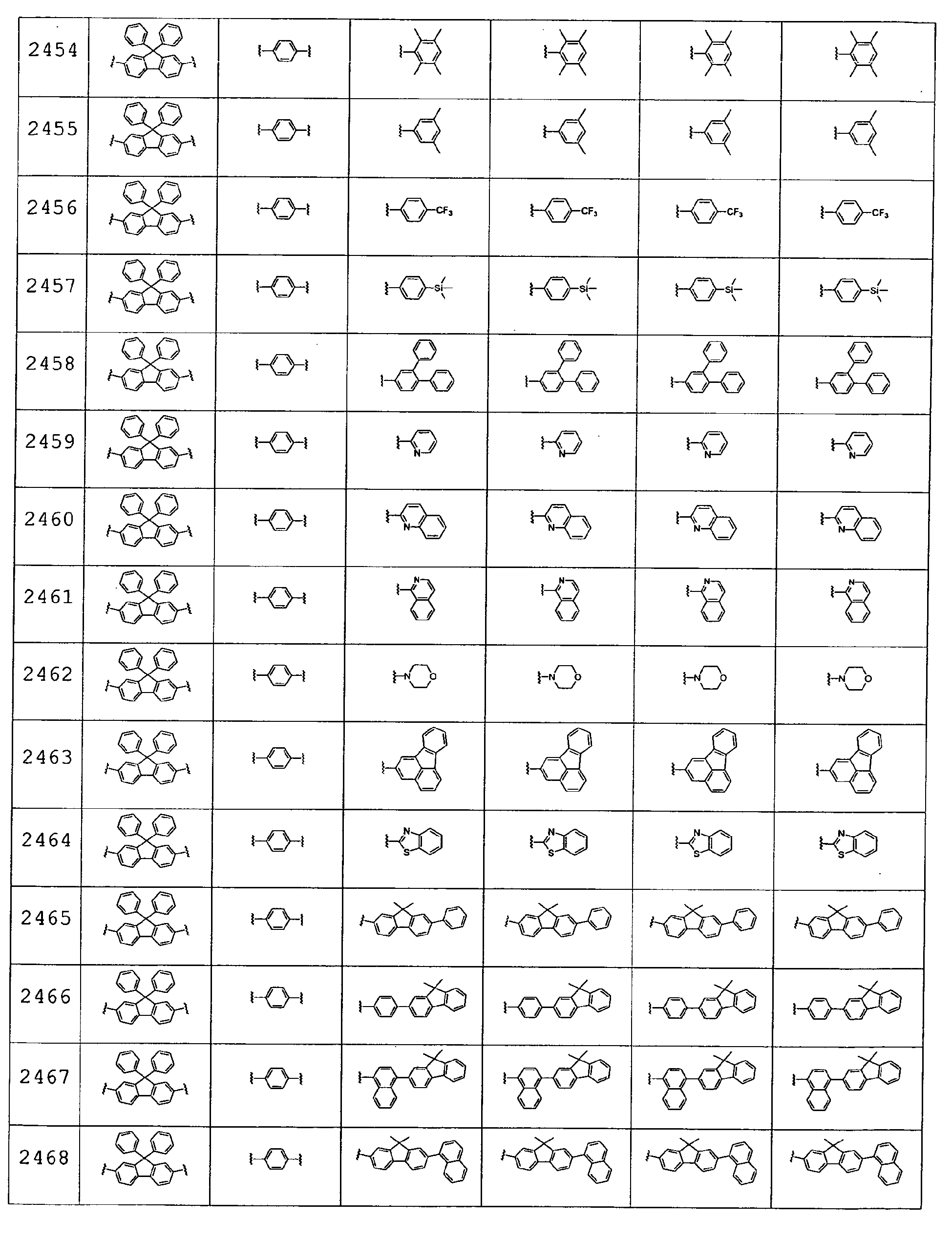

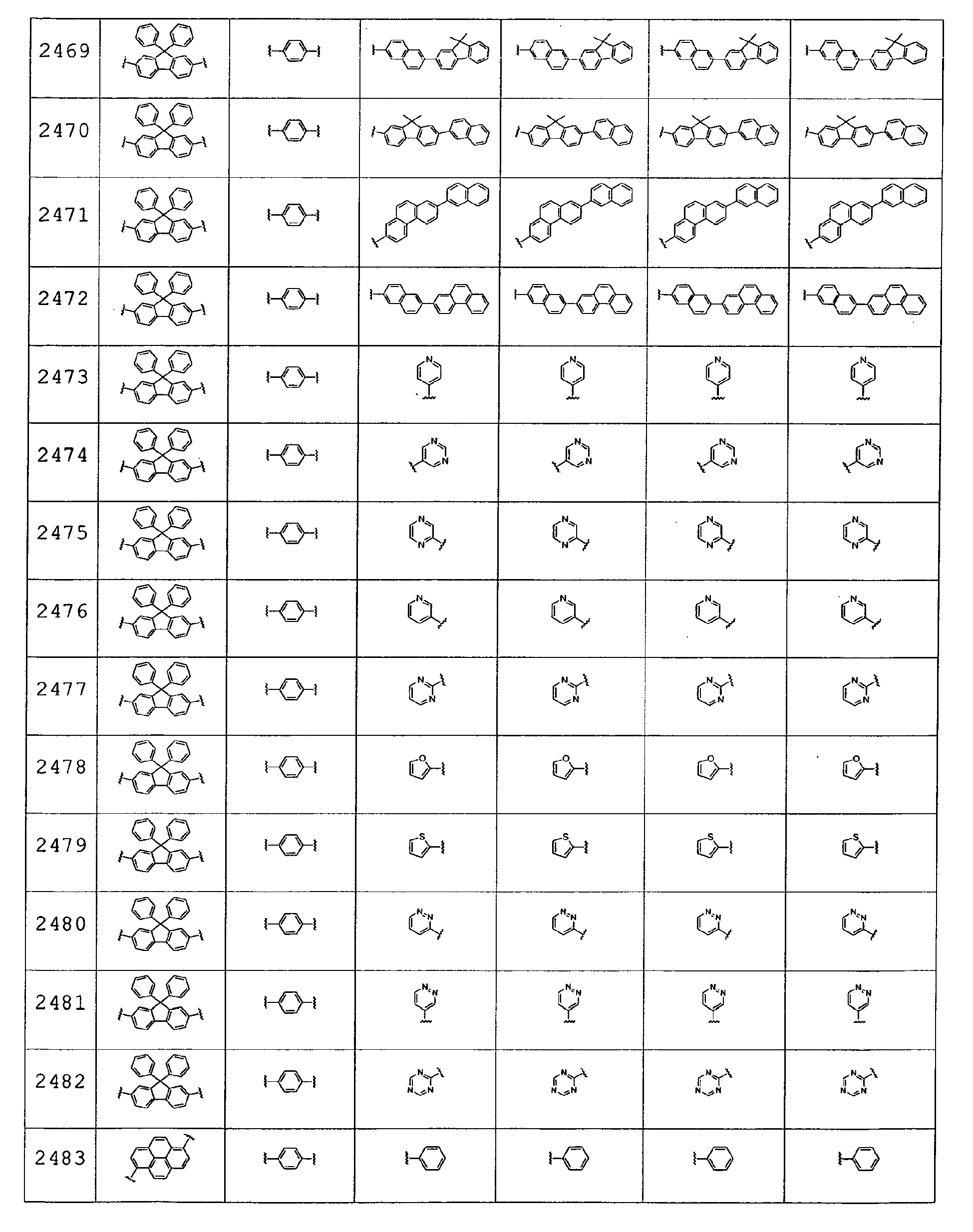

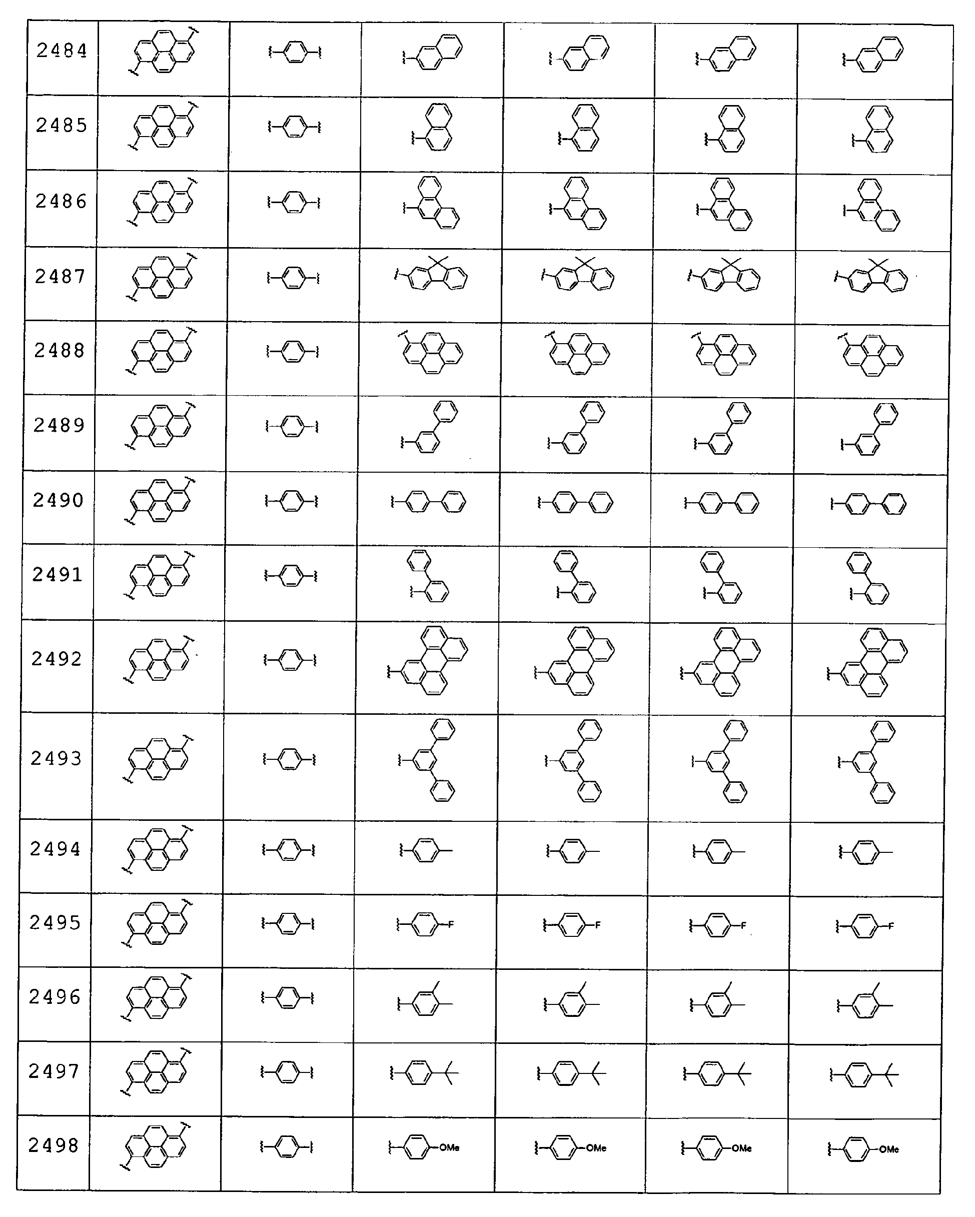

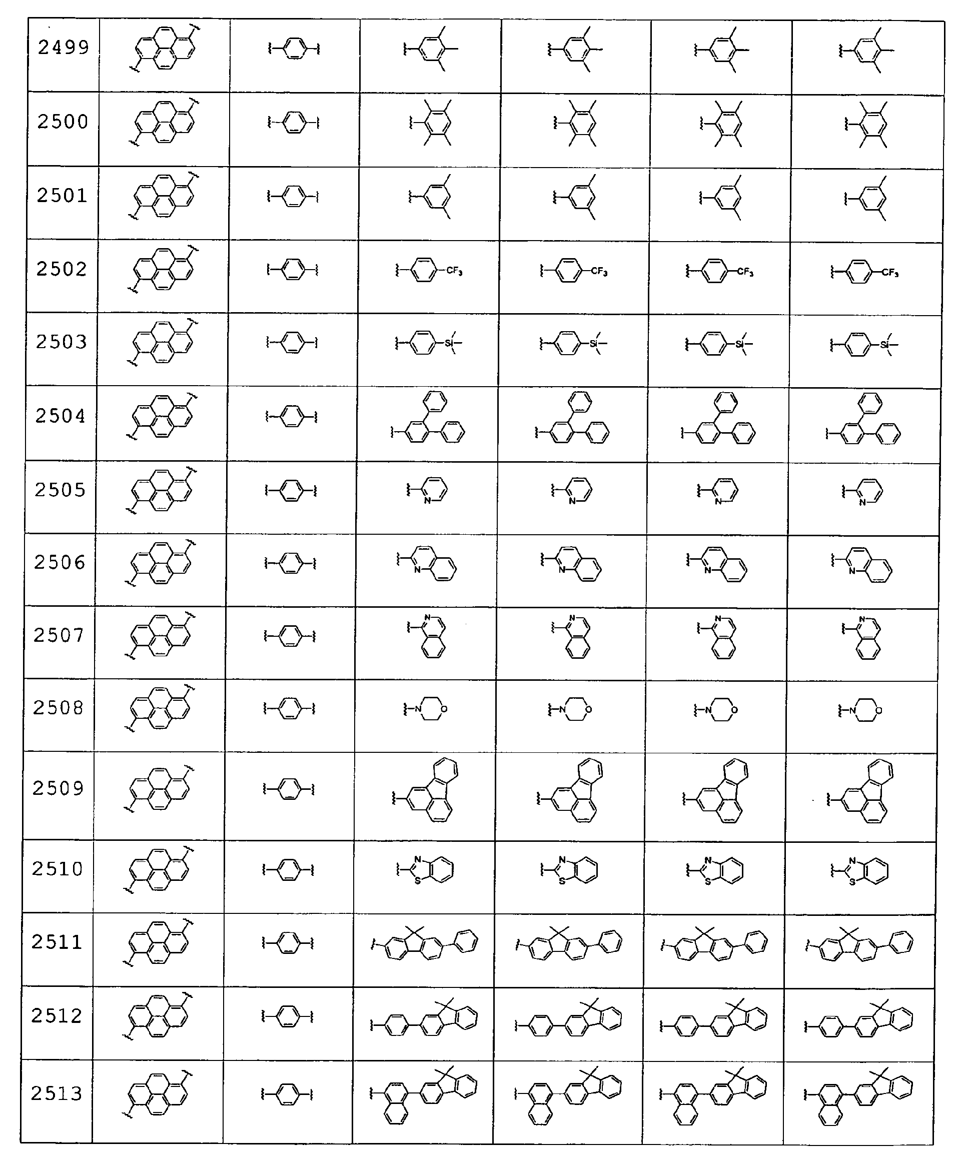

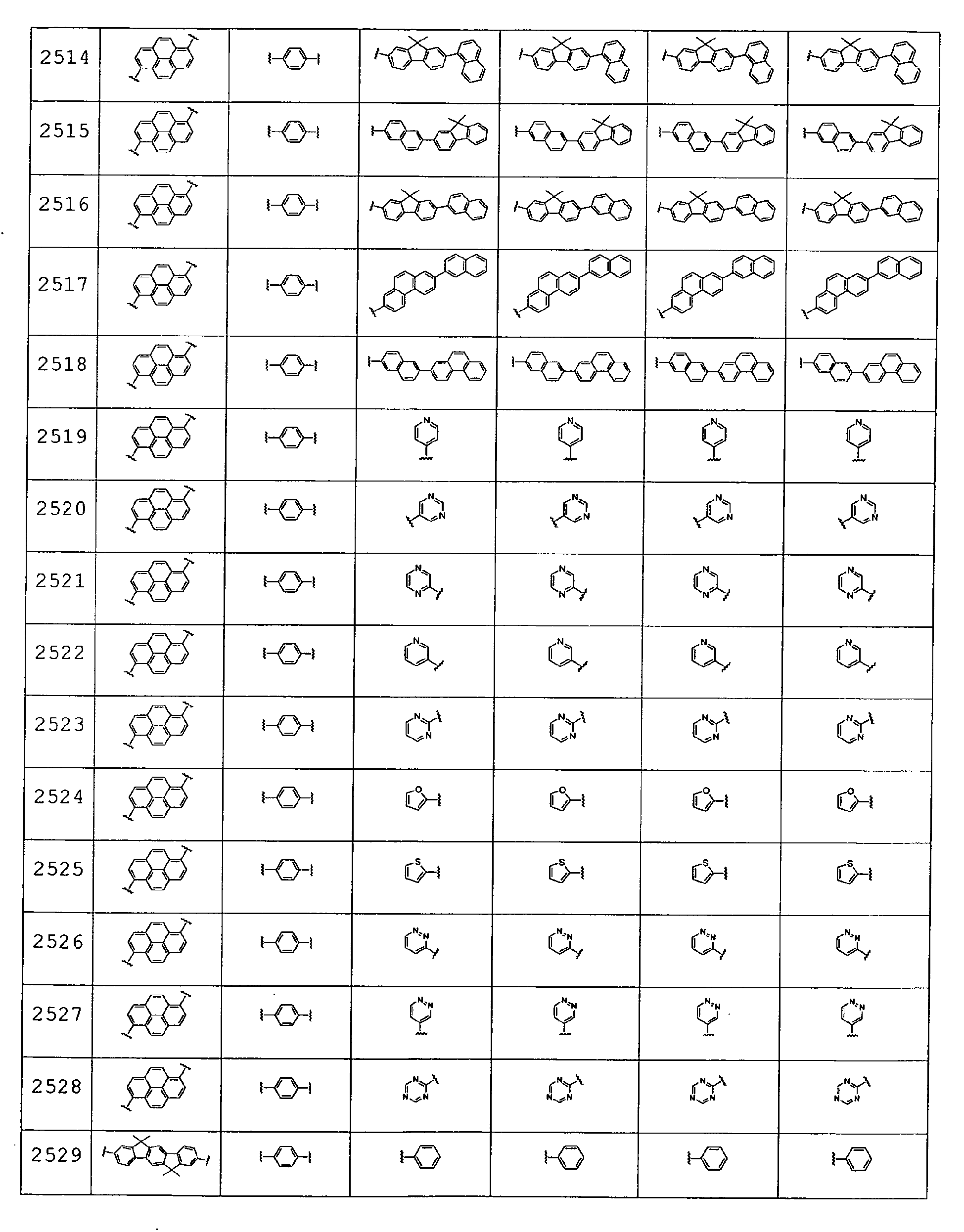

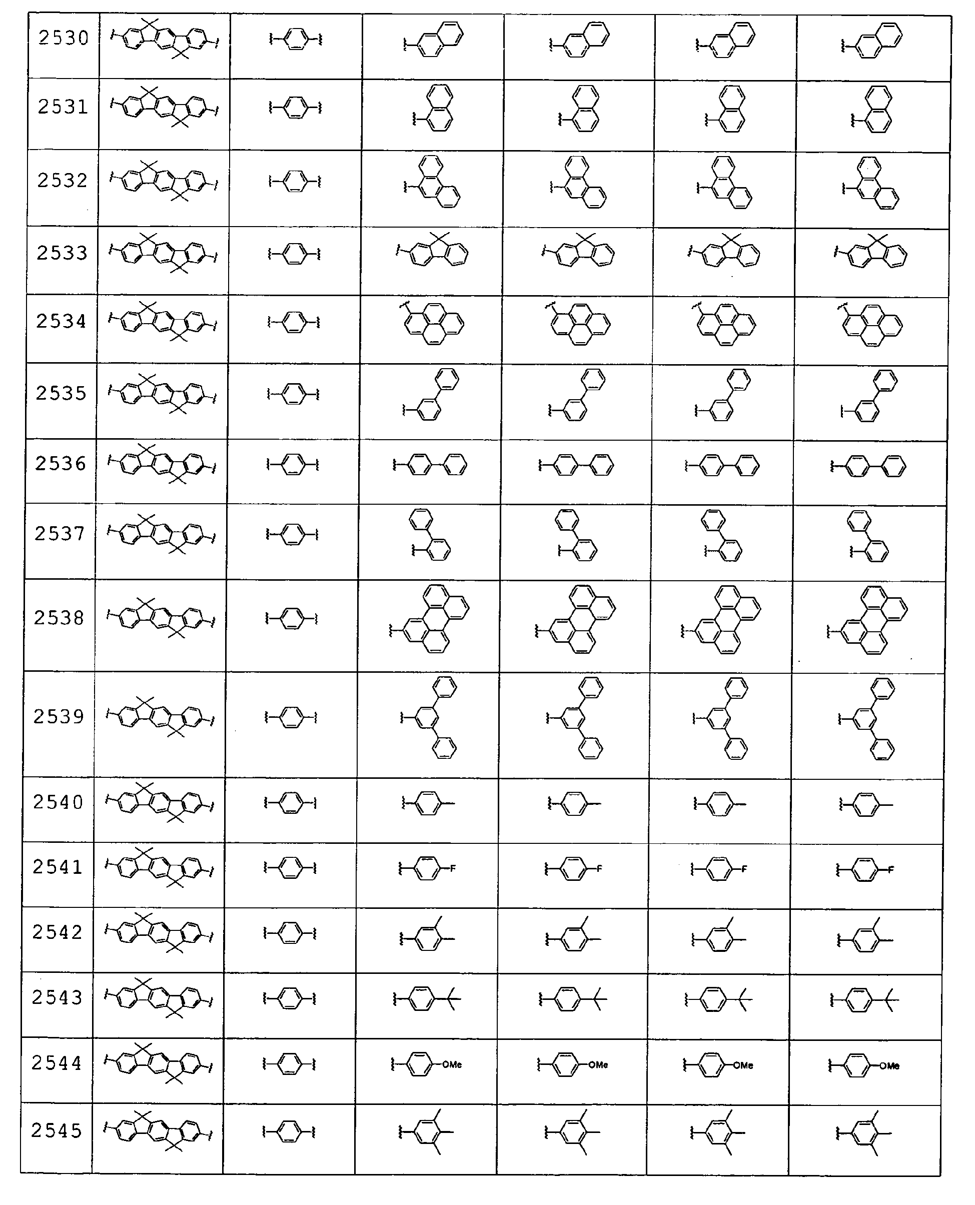

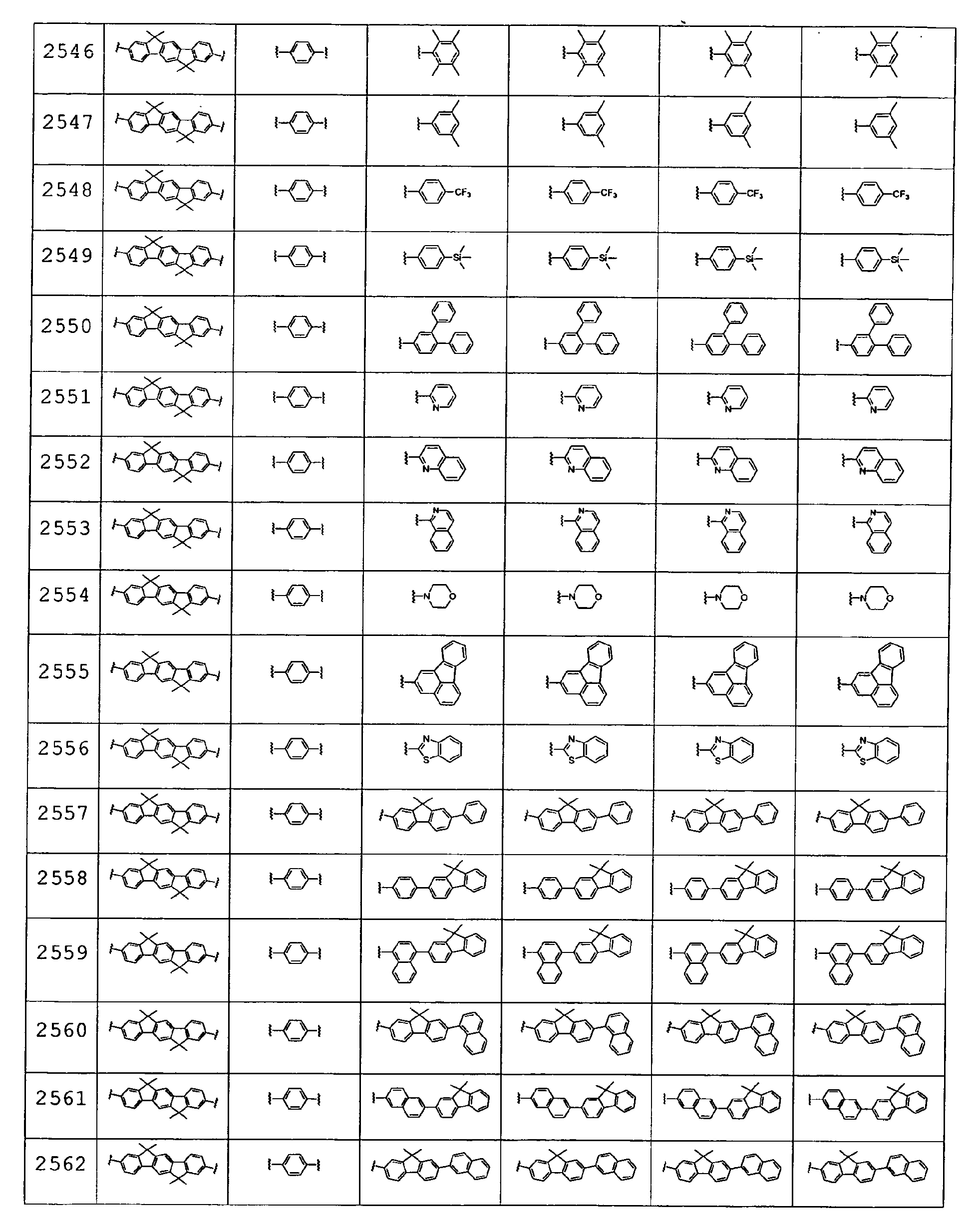

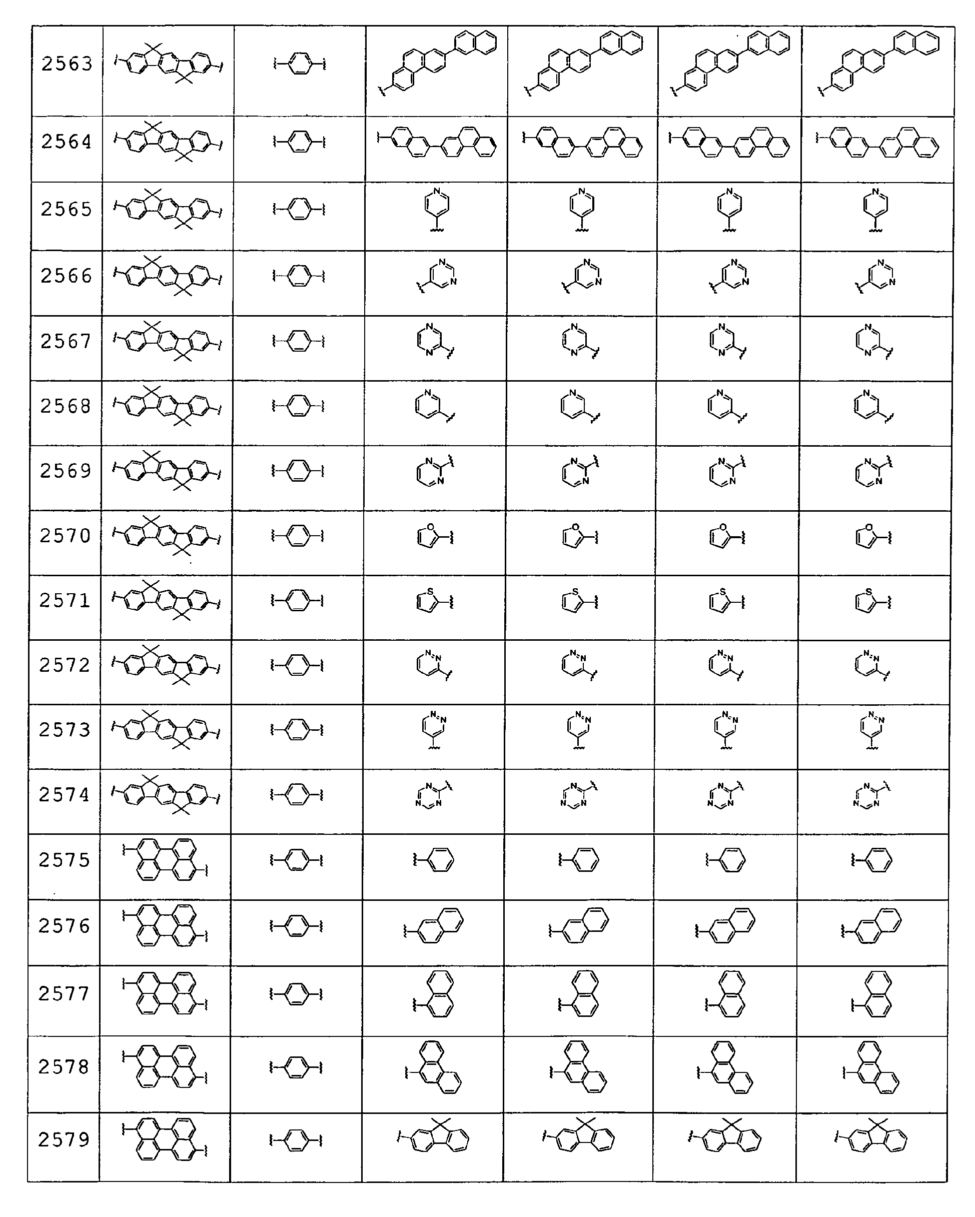

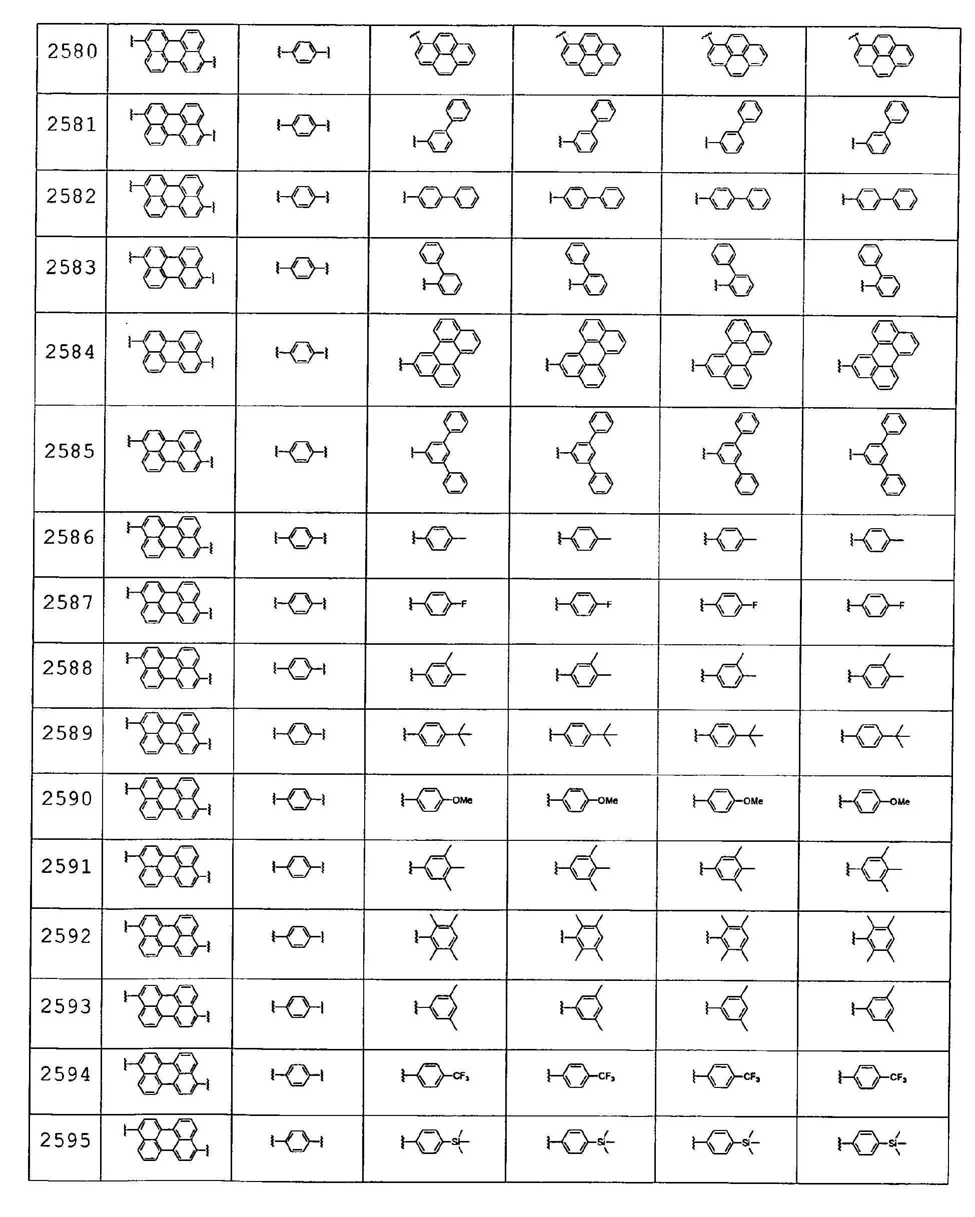

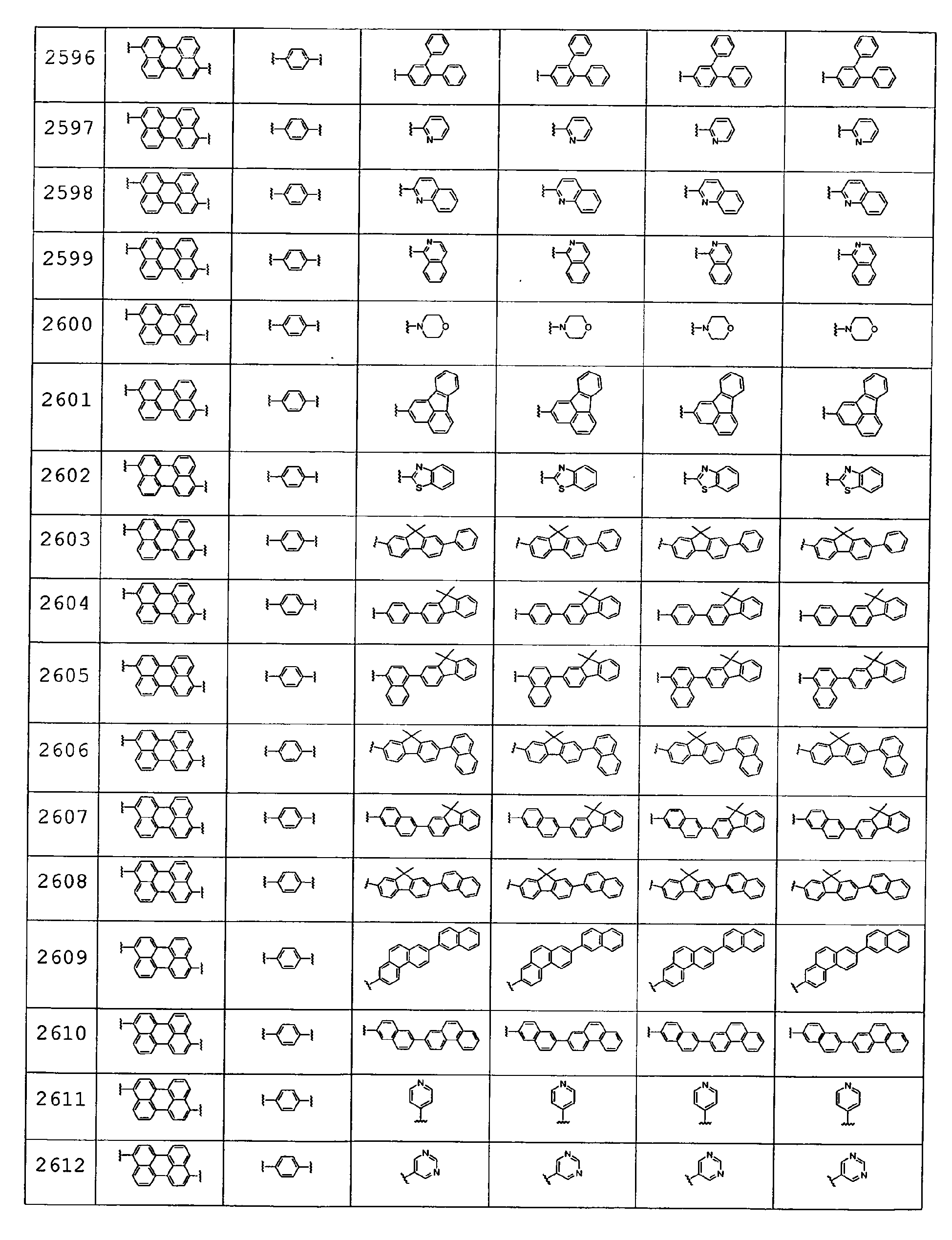

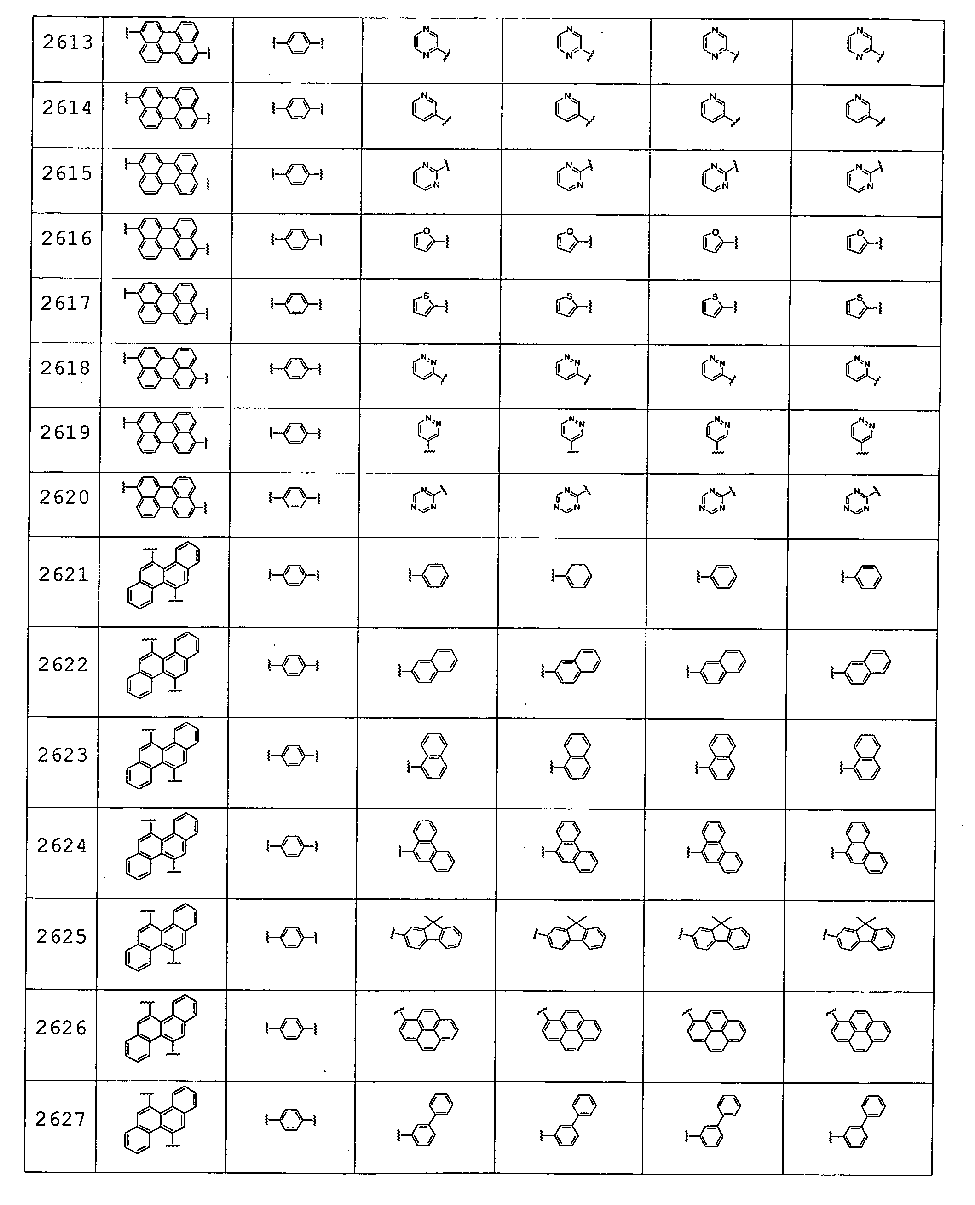

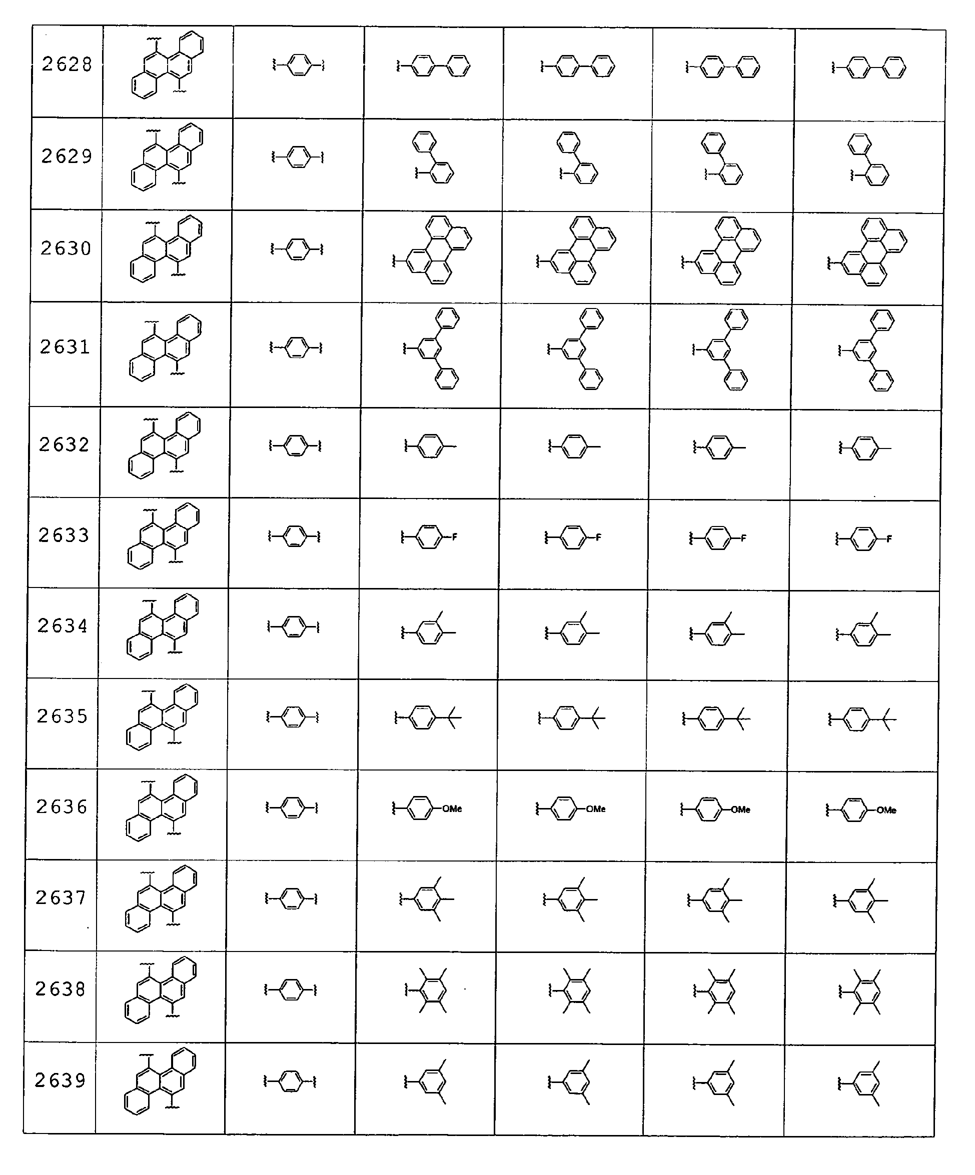

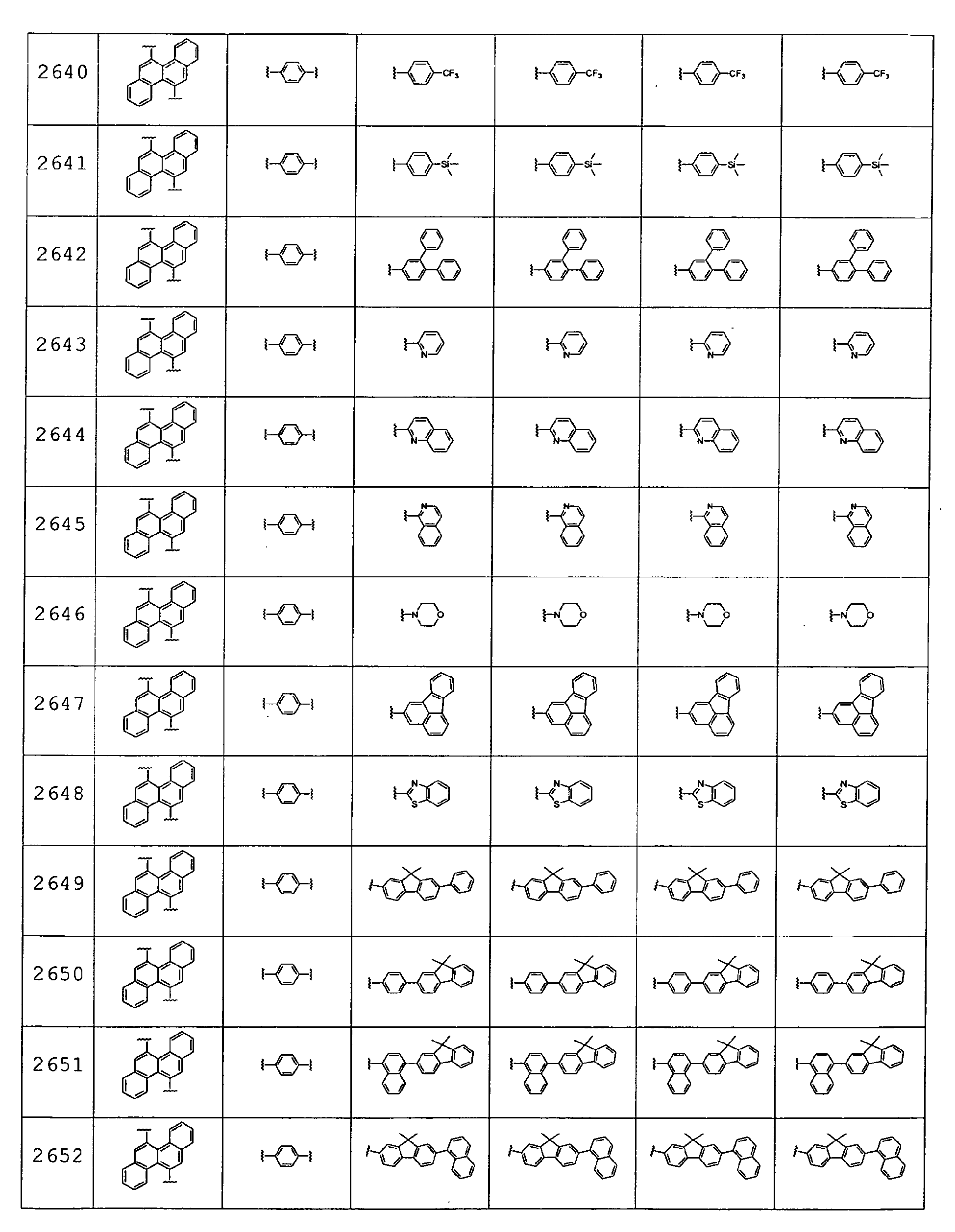

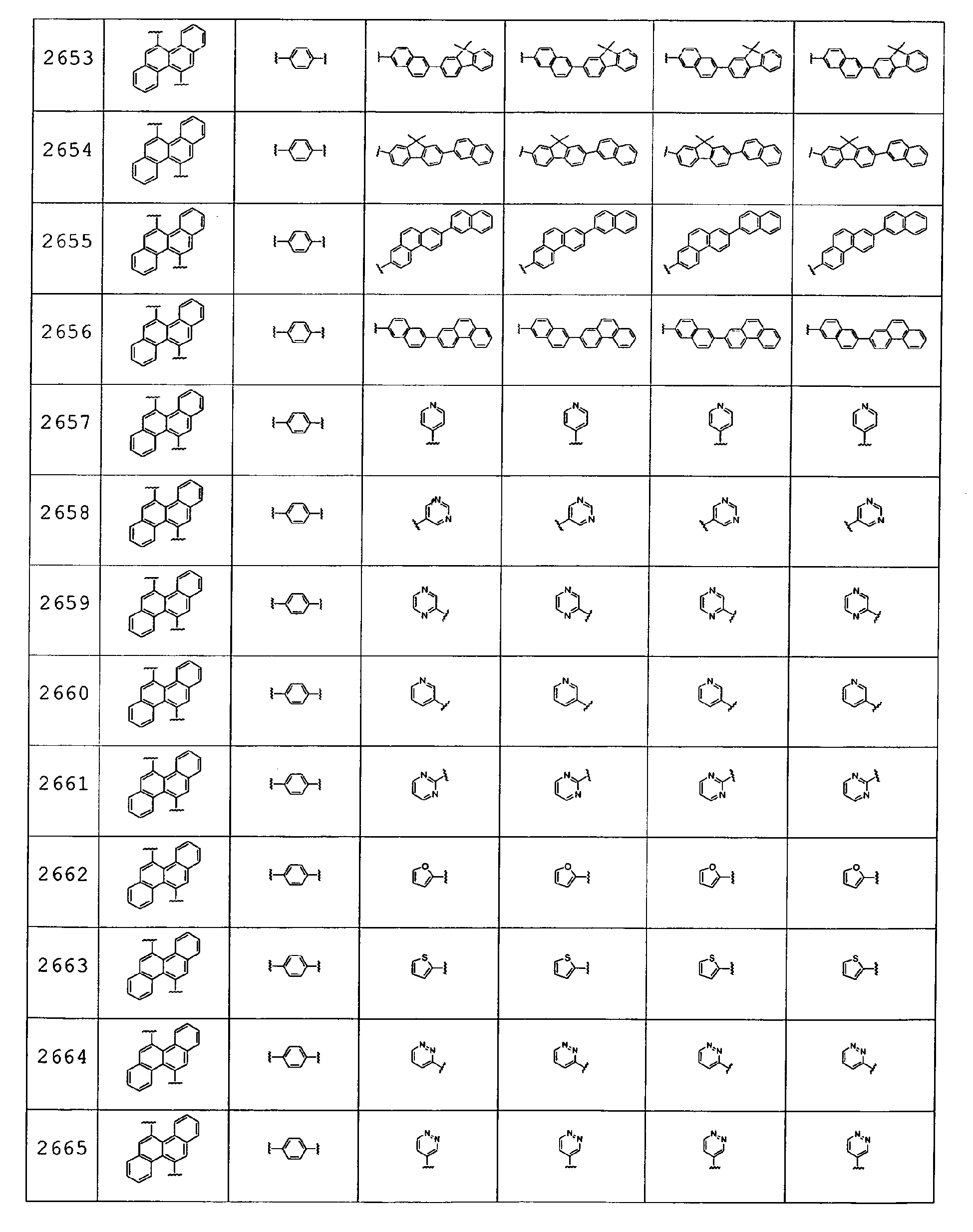

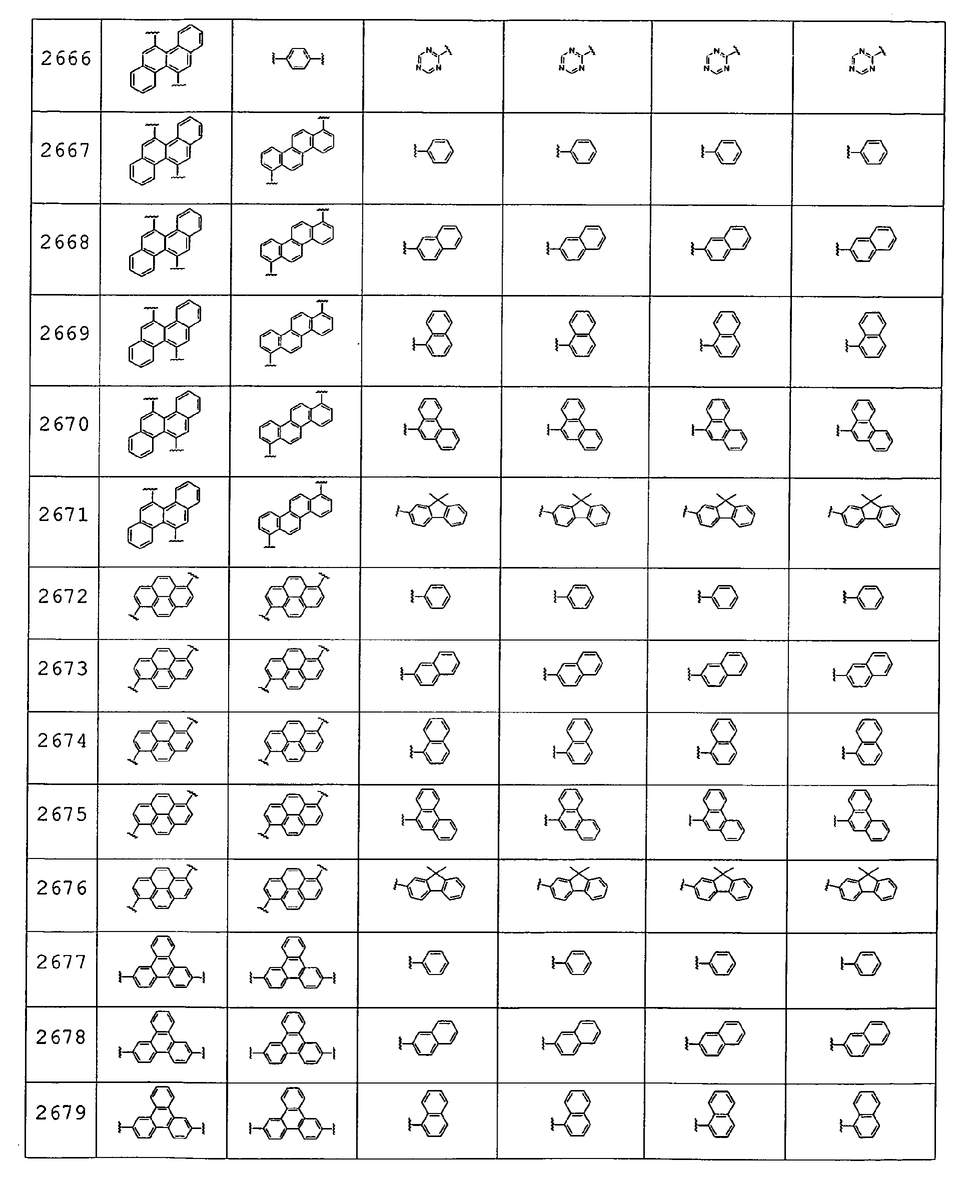

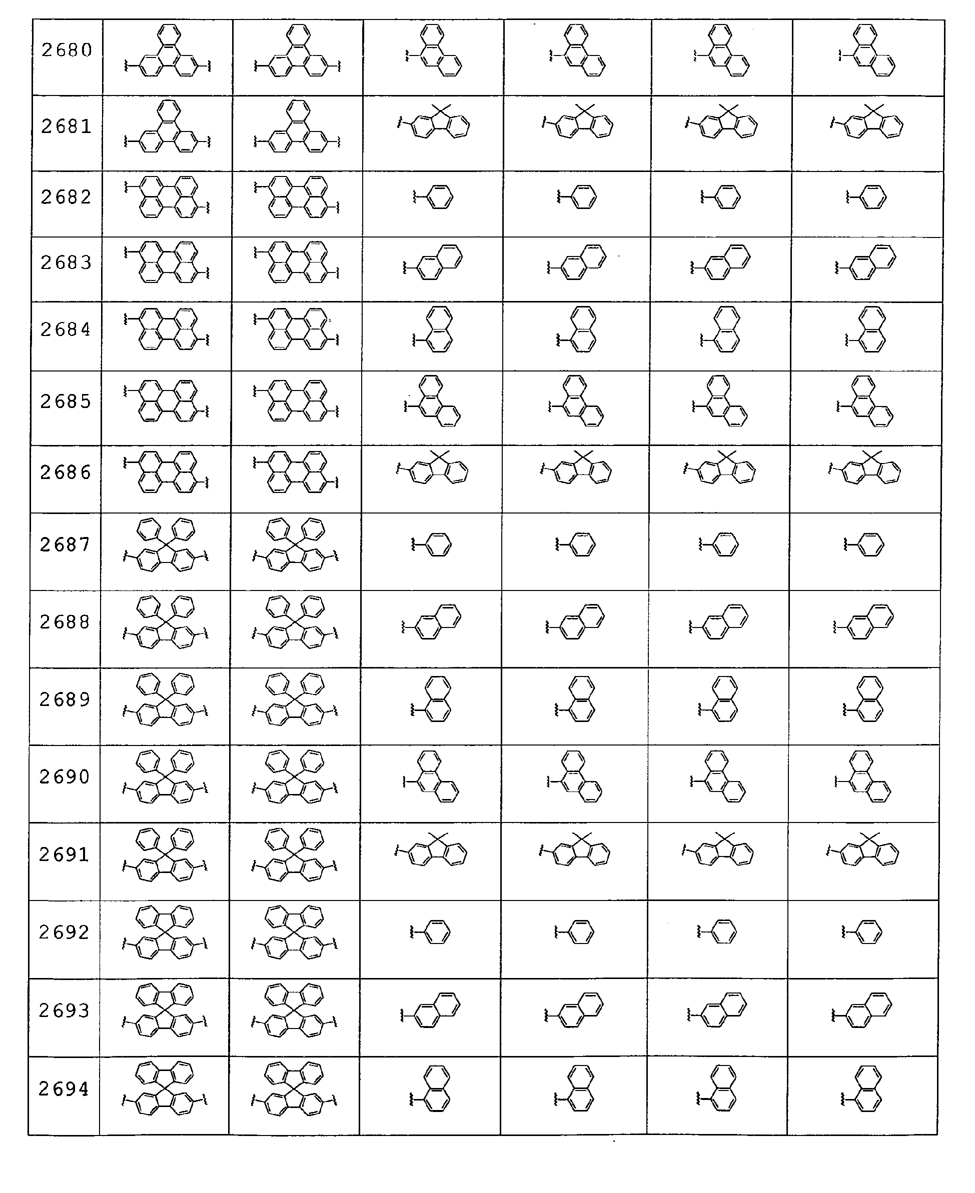

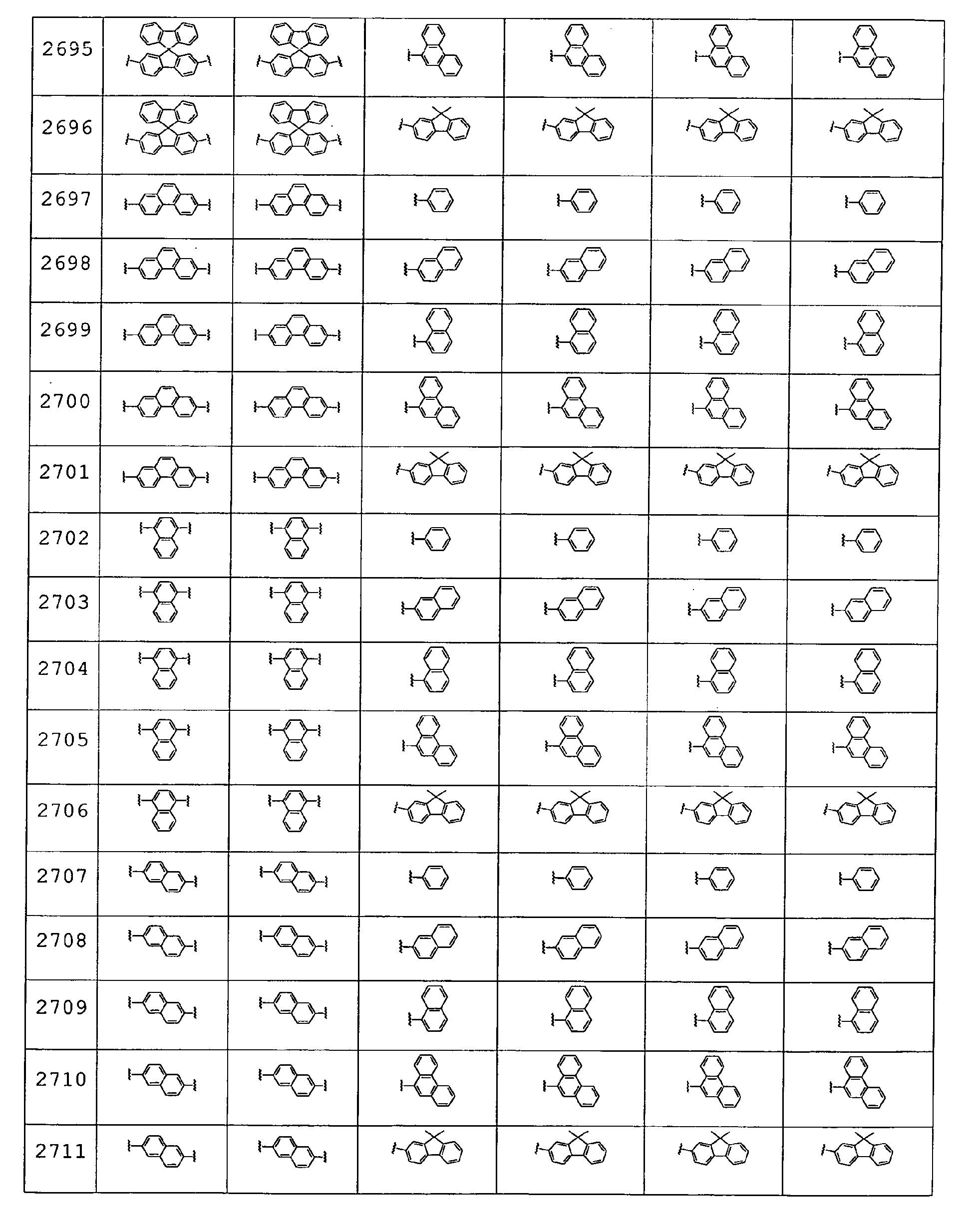

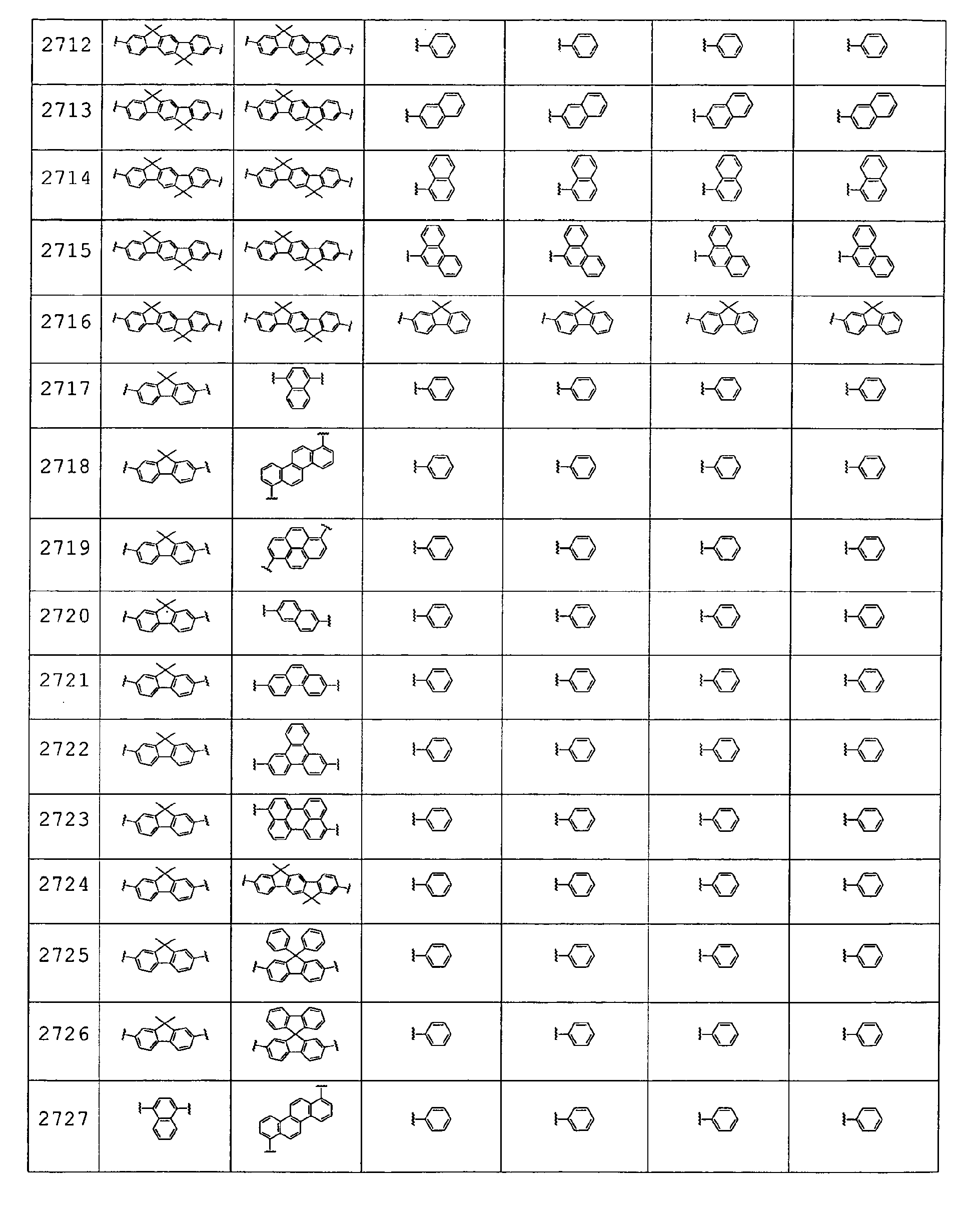

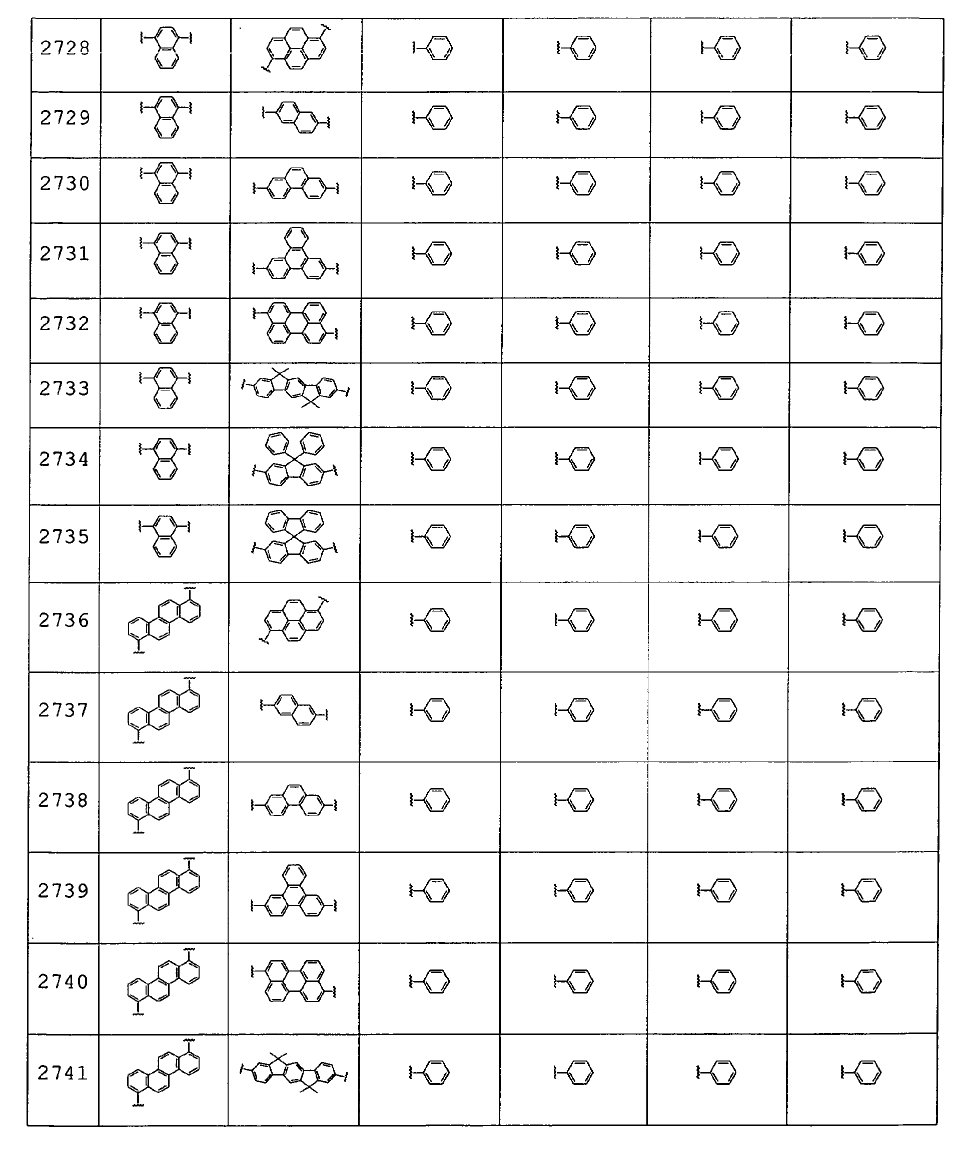

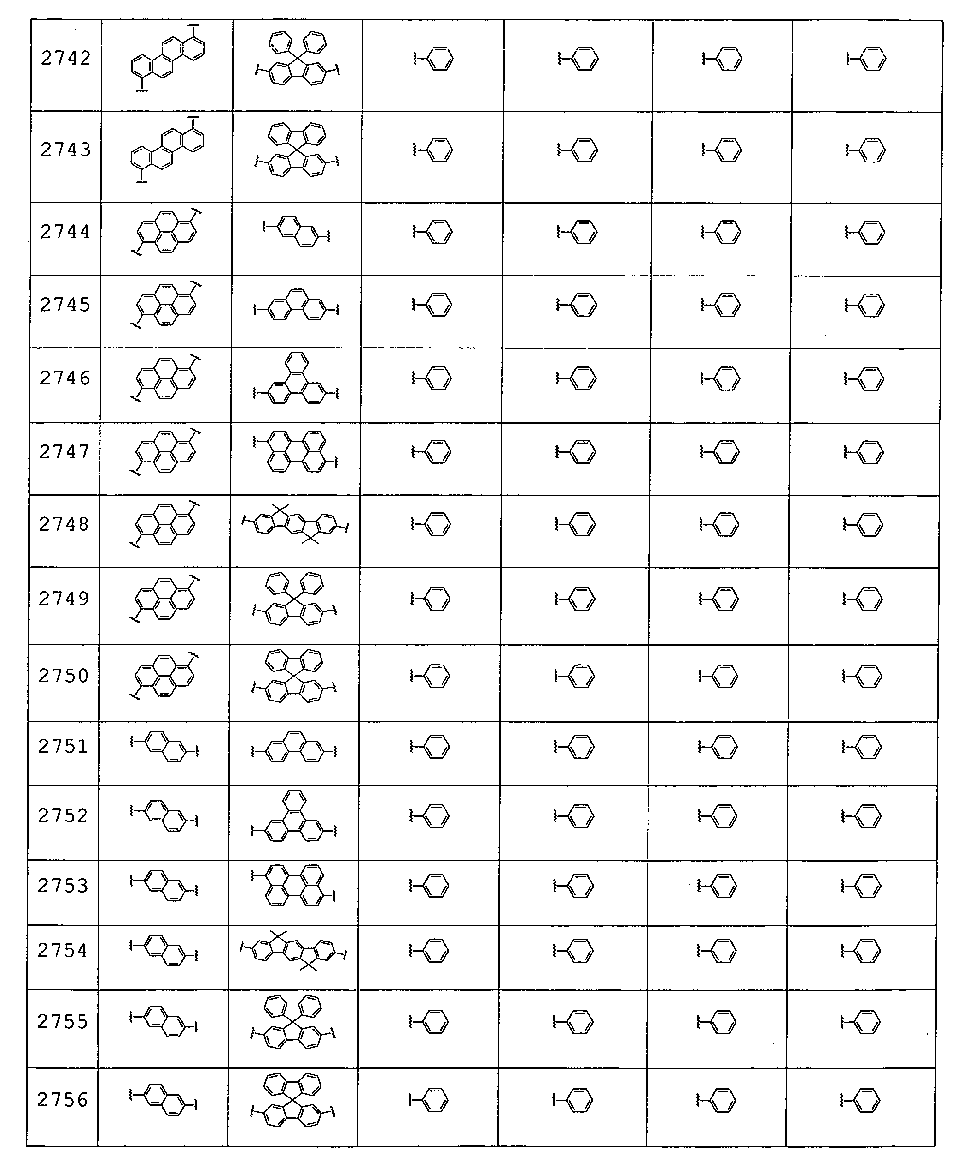

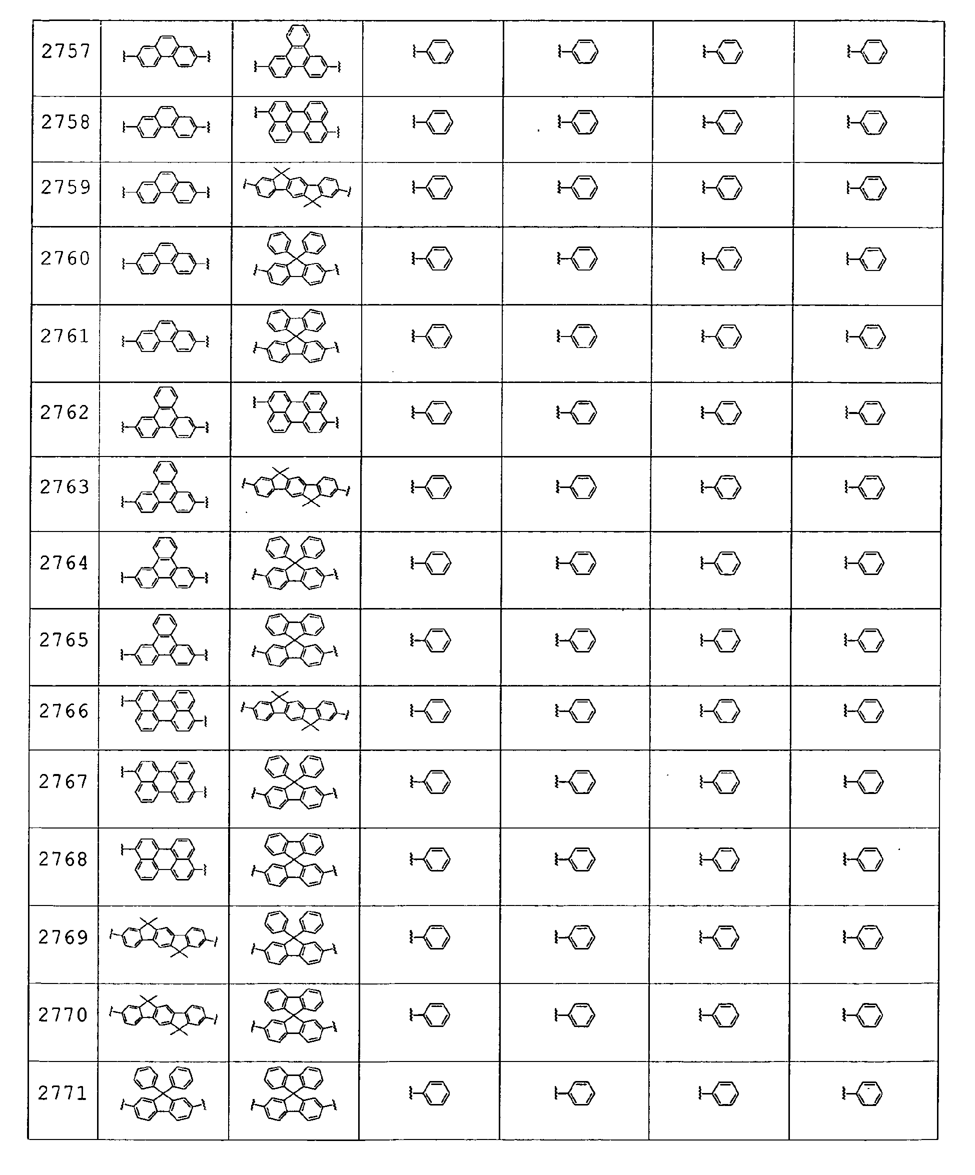

**Table 2**

| Comp. | ¹H NMR(CDCl₃, 200 MHz) | MS/FAB | |
|---|---|---|---|
| | | found | calculated |
| 1 | δ = 7.83(d, 2H), 7.75(d, 2H), 7.62-7.58(m, 4H), 7.20(m, 8H), 6.81-6.75(m, 6H), 6.63-6.58(m, 10H), 1.72(s, 12H) | 744.96 | 744.35 |
| 2 | δ = 7.88-7.74(m, 20H), 7.62-7.49(m, 12H), 7.36(m, 4H), 6.75(s, 2H), 6.58(m, 2H), 1.72(s, 12H) | 945.20 | 944.41 |
| 3 | δ = 8.07-8.02(m, 8H), 7.83(m, 2H), 7.75(s, 2H), 7.62-7.53(m, 16H), 7.38(m, 4H), 6.98(m, 4H), 6.75(s, 2H), 6.58(m, 2H), 1.72(s, 12H) | 945.20 | 944.41 |
| 5 | δ = 7.87-7.83(m, 6H), 7.75(s, 2H), 7.62-7.55(m, 12H), 7.38(m, 4H), 7.28(m, 4H), 6.75(s, 6H), 6.58(m, 6H), 1.72(s, 36H) | , 1209.60 | 1208.60 |
| 8 | δ = 7.83(d, 2H), 7.75(d, 2H), 7.58-7.41(m, 32H), 6.75(s, 2H), 6.69(m, 8H), 6.58(m, 2H), 1.72(s, 12H) | 1049.35 | 1048.48 |
| 12 | δ = 7.83(d, 2H), 7.75(d, 2H), 7.62-7.58(m, 4H), 6.98(m, 8H), 6.75(s, 2H), 6.58-6.51(m, 10H), 2.34(s, 12H), 1.72(s, 12H) | 801.07 | 800.41 |
| 15 | δ = 7.83(d, 2H), 7.75(d, 2H), 7.62-7.58(m, 4H), 7.01(m, 8H), 6.75(m, 2H), 6.58-6.55(m, 10H), 1.72(s, 12H), 1.35(s, 36H) | 969.60 | 968.60 |
| 19 | δ = 7.83(d, 2H), 7.75(d, 2H), 7.62-7.58(m, 4H), 6.75-6.71(m, 6H), 6.58(m, 2H), 6.36(m, 8H), 2.34(s, 24H), 1.72 (s, 12H) | 857.17 | 856.48 |
| 30 | δ = 7.93-7.83(m, 10H), 7.77-7.75(m, 6H), 7.63-7.54(m, 20H),7.38(m, 4H), 7.28(m, 4H), 6.75(s, 2H), 6.69(m, 8H0, 6.58(m, 2H), 1.72(s, 36H) | 1513.99 | 1512.73 |
| 47 | δ =7.88-7.74(m, 12H), 7.62-7.49(m, 8H), 7.36(m, 2H), 7.20(m, 4H), 6.81-6.75(m, 4H), 6.63-6.58(m, 6H), 1.72(s, 12H) | 845.08 | 844.38 |
| 49 | δ =8.93(m, 4H), 8.12(m, 4h), 7.88-7.75(m, 12H), 7.62-7.58(m, 4H), 7.20(m, 4H), 6.91(s, 2H), 6.81-6.75(m, 4H), 6.63-6.58(m, 6H), 1.72(s, 12H) | 945.20 | 944.41 |
| 52 | δ = 7.83(d, 2H), 7.75(d, 2H),k 7.62-7.41(m, 16H), 7.20(m, 4H), 6.89-6.75(m, 8H), 6.63-6.58(m, 8H), 1.72(s, 12H) | 897.15 | 896.41 |
| 54 | δ = 7.83(d, 2H), 7.75(d, 2H), 7.62-7.51(m, 10H), 7.41(m, 2h), 7.20-7.16(m, 6H), 7.08(m, 4H), 6.87-6.75(m, 6H), 6.69-6.58(m, 8H), 1.72(s, 12H) | 897.15 | 896.41 |
| 57 | δ = 7.83(d, 2H), 7.75(d, 2H), 7.62-7.58(m, 4H), 7.20(m, 4H), 6.98(m, 4H), 6.81(m, 4H), 6.75(s, 2H), 6.63-6.51(m, 10H), 2.34(s, 6H), 1.72(s, 12H) | 773.02 | 772.38 |
| 60 | δ = 7.83(d, 2H), 7.75(d, 2H), 7.62-7.58(m, 4H), 7.20(m, 4H), 7.01(m, 4H), 6.81-6.75(m, 4H), 6.63-6.55(m, 10H), 1.72(s, 12H), 1.35(s, 18H) | 857.17 | 856.48 |
| 68 | δ = 8.07(m, 2H), 7.83(d, 2H), 7.75(d, 2H), 7.62-7.55(m, 6H), 7.20(m, 4H), 6.81-6.58(m, 14H), 1.72(s, 12H) | 746.94 | 746.34 |
| 75 | δ = 7.93-7.83(m, 6H), 7.77-7.75(m, 4H), 7.62-7.54(m, 12H), 7.38(m, 2H), 7.28(m ,2H), 7.20(m, 4H), 6.81-6.75(m, 7.20(m, 4H), 6.81-6.75(m, 4H), 6.69-6.63(m, 10H), 1.72(s, 24H) | 1129.47 | 1128.54 |
| 94 | δ = 7.88-7.74(m, 14H), 7.62-7.49(m, 12H), 7.38-7.36(m, 4H), 7.28(m, 2H), 6.75(m, 4H), 56.58(m, 4H), 1.72(s, 24H) | 1077.40 | 1076.51 |
| 98 | δ = 7.88-7.74(m, 12H), 7.62-7.36(m, 18H), 7.16-7.08(m, 6H), 6.87(m, 2H), 6.75(m, 2H), 6.69(m, 2H), 6.58(m, 2H), 1.72(s, 12H) | 997.27 | 996.44 |
| 110 | δ = 7.88-7.74(m, 12H), 7.62-7.58(m, 4H), 7.50-7.49(m, 4H), 7.36(m, 2H), 7.15(m, 4H), 6.75(m, 2H), 6.61-6.58(m, 6H), 1.72(s, 12H), 0.25 (s, 18H) | 989.44 | 988.46 |
| 117 | δ = 8.18(m, 2H), 8.01(m, 2H), 7.88-7.74(m, 12H), 7.62-7.49(m, 12H), 6.75(m, 2H), 6.58(m, 2H), 1.72(s, 12H) | 959.23 | 958.32 |
| 137 | δ = 8.07-8.02(m, 4H), 7.87-7.83(m, 4H), 7.75(m, 2H), 7.62-7.53(m, 14H), 7.38(m, 4H), 7.28(m, 2H), 6.98(m, 2H), 6.75(m, 4H), 6.58(m, 4H), 1.72(s, 24H) | 1077.40 | 1076.51 |
| 161 | δ = 8.07-8.02(m, 4H), 7.93(m, 2H), 7.83(m ,2H), 7.77-7.75(m, 4H), 7.63-7.51(m, 22H), 7.41-7.38(m, 4H), 6.98(m, 2H), 6.75(m, 4H), 6.58(m, 4H), 1.72(s, 24H) | 1229.59 | 1228.57 |
| 182 | δ = 8.93(m, 4H), 8.12(m, 4H), 7.88-7.75(m, 12H), 7.62-7.41(m, 18H), 6.91(m, 2H), 6.75-6.69(m, 6H), 6.58(m, 2H), 1.72(s, 12H) | 1097.39 | 1096.48 |
| 193 | δ = 8.93(m, 4H), 8.12(m, 4H), 7.88-7.75(m ,12H), 7.62-7.58(m, 4H), 6.91(m, 2H), 6.75-6.71(m, 4H), 6.58(m, 2H), 6.36(m, 4H), 2.34(s, 12H), 1.72(s, 12H) | 1001.30 | 1000.48 |
| 198 | δ = 8.93(m, 4H), 8.22(m, 2H), 8.12(m, 4H), 7.94-7.57(m, 24H), 7.37(m ,2H), 6.91(m ,2H), 6.75(m, 2H), 6.58(m, 2H), 1.72(s, 12H) | 1047.29 | 1046.43 |
| 220 | δ = 8.93(m, 4H), 8.47(m, 4H), 8.12(m, 4H), 7.88-7.75(m, 12H), 7.62-7.58(m, 4H), 6.91(m, 2H), 6.75(m, 2H), 6.58(m, 2H), 1.72(s, 12H) | 951.13 | 950.38 |
| 226 | δ =7.87-7.83(m, 4H), 7.75(m, 2H), 7.62-7.51(m, 24H), 7.41-7.38(m, 6H), 7.28(m, 2H), 7.06(m, 2H), 6.85(m, 4h), 6.75(m, 4h), 6.58(m, 4H), 1.72(s, 24H) | 1281.67 | 1280.60 |
| 230 | δ = 7.87-7.83(m, 4H), 7.75(m, 2H), 7.62-7.55(m, 8H), 7.38(m, 2H), 7.28(m, 2H), 7.01(m, 4H), 6.75(m, 4H), 6.58-6.55(m, 8H), 1.72(s, 24H), 1.35(s, 18H) | , 1089.49 | 1088.60 |
| 240 | δ =8.28(m, 2H), 8.17(m, 2H), 7.87-7.83(m, 4H), 7.75(m, 2h), 7.62-7.55(m, 10H), 7.47-7.38(m, 6H), 7.28(m, 2H), 6.99(m, 2H), 6.75(m, 4H), 6.58(m, 4H), 1.72(s, 24H) | 1079.38 | 1078.50 |
| 243 | δ = 8.18(m, 2H), 8.01(m, 2H), 7.87-7.83(m, 4H), 7.75(m, 2H), 7.62-7.53(m, 12H), 7.38(m, 2H), 7.28(m, 2H), 6.75(m, 4H), 6.58(m, 4H), 1.72(s, 24H) | 1091.43 | 1090.41 |
| 263 | δ = 8.12(m, 4H), 7.88-7.82(m, 10H), 7.75-7.71(m, 6H), 7.62-7.51(m, 16H), 7.41(m, 2H), 6.91(m, 2H), 6.75-6.69(m, 6H), 6.58(m, 2H), 1.72(s, 12H) | 1145.43 | 1144.48 |
| 278 | δ = 8.12-8.07(m, 6H), 7.88-7.82(m, 10H), 7.75-7.71(m, 6H), 7.62-7.55(m, 6H), 6.91(m, 2h), 6.75-6.58(m, 8H), 1.72(s, 12H) | 995.22 | 994.40 |
| 302 | δ = 7.83(m, 2H), 7.75(m, 2H), 7.62-7.41(m, 30H), 6.89-6.88(m, 4H), 6.75(m,2H), 6.69(m, 4H), 6.59-6.58(m, 4H), 1.72(s, 12H) | 1049.35 | 1048.48 |
| 306 | δ = 7.83(m, 2H), 7.75(m, 2H), 7.62-7.41(m, 16H), 6.98(m, 4H), 6.88(m, 4H), 6.75(m, 2H), 6.59-6.51(m, 8H), 2.34(s, 6H), 1.72(s, 12H) | 925.21 | 924.44 |
| 313 | δ = 7.83(m, 2H), 7.75(m, 2H), 7.62-7.41(m, 16H), 6.89-6.88(m, 4H), 6.75-6.71(m, 4H), 6.59-6.58(m, 4H), 6.36(m, 4H), 2.34(s, 12H), 1.72(s, 12H) | 953.26 | 952.48 |
| 344 | δ = 7.83(m, 2H), 7.75(m, 2H), 7.62-7.51(m, 16H), 7.41(m, 2H), 6.98(m, 4H), 6.75-6.69(m, 6H), 6.58-6.51(m, 6H), 2.34(s, 6H), 1.72(s, 12H) | 925.21 | 924.44 |
| 347 | δ = 7.83(m, 2H), 7.75(m ,2H), 7.62-7.51(m, 16H), 7.41(m, 2H), 7.01(m, 4H), 6.75(m, 2H), 6.69(m, 4H), 6.58-6.55(m, 6H), 1.72(s, 12H), 1.35(s, 18H) | 1009.37 | 1008.54 |
| 351 | δ = 7.83(m, 2h), 7.75(m, 2H), 7.62-7.51(m, 16H), 7.41(m, 2H), 6.75-6.69(m, 8H), 6.58(m, 2H), 6.36(m, 4H), 2.34(s, 12H), 1.72(s, 12H) | 953.26 | 952.48 |
| 354 | δ = 7.83-7.75(m, 12H), 7.62-7.41(m, 32H), 6.95(m, 2H), 6.75-6.65(m, 8H), 6.58(m ,2H), 1.72(s, 12H) | 1201.54 | 1200.54 |
| 389 | δ = 7.83(m ,2H), 7.75(m, 2H), 7.62-7.37(m, 20H), 6.88(m, 4H), 6.75(m, 2h), 6.59-6.56(m, 8H), 1.72(s, 12H) | 1033.15 | 1032.39 |
| 398 | δ = 7.93(m ,2H), 7.83(m, 2H), 7.77-7.75(m, 4h), 7.63-7.41(m, 30H), 6.88(m, 4H), 6.75(m, 4H), 6.59-6.58(m, 6H), 1.72(s, 24H) | 1281.67 | 1280.60 |
| 420 | δ = 7.91(m, 6H), 7.83(m, 2H), 7.75(m, 2H), 7.62-7.58(m, 4H), 7.39-7.35(m, 14H), 7.01(m, 4H), 6.75(m, 2H), 6.58-6.51(m, 8H), 1.72(s, 12H), 1.35(s, 18H) | 1205.57 | 1204.57 |
| 421 | δ = 7.91(m, 6H), 7.83(m, 2H), 7.75(m, 2H), 7.62-7.58(m, 4H), 7.39-7.35(m, 14H), 6.75-6.74(m, 6H), 6.58-6.51(m, 8H), 3.85(s, 6H), 1.72(s, 12H) | 153.41 | 1152.47 |
| 457 | δ = 7.83(m, 2H), 7.75(m, 2H), 7.62- 7.41(m, 24H), 7.06(m, 2H), 6.85(m, 4H), 6.75(m, 2H), 6.58(m, 2H), 6.24(m, 4H), 2.34(s, 12H), 2.18(s, 6H), 1.72(s, 12H) | 1133.51 | 1132.57 |
| 466 | δ = 7.83-7.75(m, 6H), 7.58-7.41(m, 22H), 7.15(m, 2H), 7.05(m, 4H), 6.95(m, 2H), 6.78(m, 2H), 3.65(m, 8H), 6.11(m, 8H), 1.72(s, 12H) | 1067.36 | 1066.52 |
| 489 | δ = 7.83(m, 2H), 7.75(m, 2H), 7.62-7.50(m, 4H), 7.01-6.98 (m, 8H), 6.75(m, 2H), 6.58-6.51(m, 10H), 2.34(s, 6H), 1.72(s, 12H), 1.35(s, 18H) | 885.23 | 884.51 |
| 493 | δ = 7.83(m, 2H), 7.75(m, 2H), 7.2-7.58(m, 4H), 6.98(m, 4H), 6.75-6.71(m, 4H), 6.58(m, 2H), 6.51(m, 4H), 6.36(m, 4H), 2.34(s, 18H), 1.72(s, 12H) | 829.12 | 828.44 |
| 511 | δ = 8.46(m, 4H), 7.83(m, 2H), 7.75(m, 2H), 7.62-7.58(m, 4H), 6.98(m, 8H), 6.75(m, 2H), 6.71-6.51(m, 6H), 2.34(s, 6H), 1.72(s, 12H) | 774.99 | 774.37 |
| 528 | δ = 7.83(m ,2H), 7.75(m, 2H), 7.62-7.58(m, 4H), 7.15(m, 4H), 6.99(m, 4h), 6.75(m, 2H), 6.61(m, 10H), 1.72(s, 12H), 0.25(s, 18H) | 925.31 | 924.41 |
| 537 | δ = 7.93-7.83(m, 6H), 7.77-7.75(m, 4H), 7.63-7.55(m, 12H), 7.38(m, 2H), 7.28(m, 2h), 6.99(m, 4H), 6.75(m, 2H), 6.69(m, 4H), 6.61-6.58(m, 6H), 1.72(s, 24H) | 1165.45 | 1164.52 |
| 554 | δ = 7.83(m, 2H), 7.75(m, 2H), 7.62-7.58(m, 4H), 7.01(m, 4H), 6.86(m ,2H), 6.75(m, 2H), 6.58-6.55(m, 6H), 6.43(m, 2H), 6.32(m, 2H), 2.34(s, 12H), 1.72(s, 12), 1.35(s, 18H) | 913.28 | 912.54 |
| 558 | δ = 7.83(m, 2H), 7.759m, 2H), 7.62-7.58(m, 4H), 6.86(m ,2H), 6.75-6.71(m, 4H), 6.58(m, 2H), 6.43(m ,2H), 6.36-6.32(m, 6H), 2.34(s, 24H), 1.72(s, 12H) | 857.17 | 856.48 |
| 568 | δ = 7.93(m, 2H), 7.83(m, 2H), 7.77-7.75(m, 4H), 7.63-7.51(m, 16H), 7.41(m ,2H), 6.86(m ,2H), 6.75(m, 4H), 6.58(m, 4H), 6.43(m, 2H), 6.32(m ,2H), 2.34(s, 12H), 1.72(s, 24H) | 1185.58 | 1184.60 |
| 571 | δ = 8.55(m, 2H), 8.42(m, 2H), 8.08-8.04(m, 4H), 7.93(m, 2H), 7.83(m, 2H), 7.77-7.75(m, 4H), 7.63-7.55(m, 14H), 6.86(m, 2H), 6.75(m, 4H), 6.58(m, 4H), 6.43(m, 2H), 6.32(m ,2H), 2.34(s, 12H), 1.72(s, 24H) | 1285.70 | 1284.63 |
| 617 | δ = 7.83(m, 2H), 7.75(m, 2H), 7.62- 7.58(m, 4H), 6.75-6.74(m, 6H), 6.52(m ,6H), 6.24(s, 4H), 3.83(s, 6H), 2.34(s, 12H), 2.18(s, 6H), 1.72(s, 12H) | 889.17 | 888.47 |
| 681 | δ = 8.22(m, 2H), 7.94(m, 2H), 7.83(m ,2H), 7.75-7.57(m ,12H), 7.37(m ,2H), 6.75(m ,2H), 6.58(m ,2H), 6.22(s, 2H), 2.34(s, 12H), 2.12(s, 12H), 1.72(s, 12H) | 959.27 | 958.50 |
| 711 | δ = 8.42(m, 4H), 8.10(m, 4H), 7.83(m ,2H), 7.75(m, 2H), 7.67-7.49(m, 10H), 7.34(m ,2H), 6.91(m, 2H), 6.75- 6.71(m, 4H), 6.58(m ,2H), 6.36(m ,4H), 2.34(s, 12H), 1.72(s, 12H) | 1049.35 | 1048.35 |
| 741 | δ = 8.55(m, 2H), 8.42(m, 2H), 8.08-8.04(m, 4H), 7.93(m ,2H), 7.77-7.75(m ,4H), 7.63-7.55(m, 14H), 7.37(m, 4H), 6.75(m ,4H), 6.58-6.56(m, 8H), 1.72(s, 24H) | 1365.59 | 1364.54 |
| 757 | δ = 8.07(m, 2H), 7.83(m, 2H), 7.75(m ,2H), 7.62-7.55(m ,6H), 7.15(m ,4H), 6.75-6.70(m ,4H), 6.62-6.58(m, 8H), 1.72(s, 12H), 0.25(s, 18H) | 891.30 | 890.42 |
| 764 | δ = 7.93-7.83(m, 6H), 7.77-7.75(m, 4H), 7.63-7.54(m, 12H), 7.38(m, 2H), 7.28(m, 2H), 7.15(m, 4H), 6.75(m ,2H), 6.69(m, 4H), 6.61-6.58(m, 6H), 1.72(s, 24H), 0.25(s, 18H) | 1273.84 | 1272.62 |
| 805 | δ = 8.22(m, 2H), 8.07(m, 2H), 7.94(m, 2H), 7.83(m, 2H), 7.75-7.55(m, 14H), 7.37(m, 2H), 6.70-6.58(m, 8H), 1.72(s, 12H) | 849.03 | 849.36 |
| 832 | δ = 8.22(m, 2H), 7.94-7.93(m, 4H), 7.83(m, 2H), 7.75-7.51(m, 26H), 7.41-7.37(m, 4H), 6.75(m, 4H), 6.58(m, 4H), 1.72(s, 24H) | 1231.57 | 1230.56 |
| 881 | δ = 8.46(m, 4H), 7.83-7.75(m, 6H), 7.58(m, 2H), 6.99-6.93(m, 6H), 6.78(m ,2H), 3.65(m, 8H), 3.11(m, 8H), 1.72 (s, 12H) | 764.96 | 764.38 |
| 910 | δ = 8.18(m, 2H), 8.01(m, 2H), 7.93-7.83(m, 6H), 7.77-7.75(m, 4H), 7.63-7.53(m, 16H), 7.38(m, 2H), 7.28(m, 2H), 6.75(m ,2h), 6.69(m, 4H), 6.58(m ,2H), 1.72(s, 24H) | 1243.62 | 1242.47 |
| 928 | δ = 7.93-7.83(m, 8H), 7.77-7.75(m, 6H), 7.63-7.51(m, 24H), 7.41-7.38(m, 4H), 7.28(m, 2H), 6.75(m, 4H), 6.69(m, 4h), 6.58(m, 4H), 1.72(s, 36H) | 1513.99 | 1512.73 |
| 1004 | δ = 8.99-8.93(m, 4H), 8.34(m ,2H), 8.12-8.10(m, 4H), 8.00-7.71(m, 24H), 7.63-7.54(m, 18H), 6.75-6.69(m, 8H), 6.58(m 4H), 1.72(s, 24H) | 1582.02 | 1580.69 |
| 1047 | δ = 8.90(m, 2H), 8.50-8.45(m, 8H), 7.83(m, 2H), 7.75(m, 2H), 7.62-7.58(m, 4H), 6.93(m ,2H), 6.58(m ,2H), 1.72(s, 12H) | 752.87 | 752.31 |
| 1081 | δ = 7.83(m ,1H), 7.75(m, 1H), 7.62-7.58(m, 2H), 7.34(m ,2H), 7.20(m, 8H), 6.81-6.75(m, 6H), 6.63-6.59(m, 10H), 1.72(s, 6H) | 628.80 | 628.29 |
| 1082 | δ = 7.88-7.74(m, 18H), 7.62-7.49(m, 10H), 7.36-7.34(m, 6H), 6.75(m, 1h), 6.58(m, 3H), 1.72(s, 6H) | 829.04 | 828.35 |
| 1083 | δ = 8.07-8.02(m, 8H), 7.83(m ,1H), 7.75(m ,1H), 7.62-7.53(m, 14H), 7.38-7.34(m, 6H), 6.98(m, 4H), 6.75(m, 1H), 6.59(m, 3H), 1.72(s, 6H) | 829.04 | 828.35 |
| 1085 | δ = 7.87-7.83(m, 5H), 7.75(m, 1H), 7.62-7.55(m, 10H), 7.38-7.28(m, 10H), 6.75(m, 5H), 6.58(m, 7H), 1.72(s, 30H) | 1093.44 | 1092.54 |
| 1088 | δ = 7.83(m, 1H), 7.75(m, 1H), 7.62-7.41(m, 32H), 6.75(m, 1H), 6.59(m, 8H), 6.58 (m, 3H), 1.72 (s, 6H) | 933.19 | 932.41 |
| 1092 | δ = 7.83(m, 1H), 7.75(m, 1H), 7.62-7.58(m, 2H), 7.34(m, 2H), 6.98(m, 8H), 6.75(m, 1H), 6.58-6.51(m, 11H), 2.34(s, 12H), 1.72(s, 6H) | 684.91 | 684.35 |
| 1093 | δ = 7.83(m, 1H), 7.75(m, 1H), 7.62-7.58(m, 2H), 7.34(m, 2H), 6.99(m, 8H), 6.75(m, 1H), 6.61-6.58(m ,11H), 1.72(s, 6H) | 700.76 | 700.25 |
| 1095 | δ = 7.83(m, 1H), 7.75(m, 1H), 7.62-7.58(m, 2H), 7.34(m ,2H), 7.01(m, 8H), 6.75(m, 1H), 6.58-6.55(m, 11H), 1.72(s, 6H), 1.35(s, 36H) | 853.23 | 852.54 |
| 1104 | δ = 8.22(m, 4H), 7.94(m, 4H), 7.83(m, 1H), 7.75-7.57(m, 15H), 7.37-7.34(m, 6H), 6.75(m, 1H), 6.58(m, 3H), 1.72(s, 6H) | 832.99 | 832.33 |
| 1127 | δ =7.88-7.74(m, 10H), 7.62-7.49(m, 6H), 7.36-7.34(m, 4H), 7.20(m, 4H), 6.81-6.75(m, 3H), 6.63-6.58(m, 7H), 1.72(s, 6H) | 728.92 | 728.32 |
| 1128 | δ = 8.07-8.02(m, 4H), 7.83(m, 1H), 7.75(m, 1H), 7.62-7.53(m, 8H), 7.38-7.34(m, 4H), 7.20(m, 4H), 6.98(m ,2H), 6.81-6.75(m, 3H), 6.63-6.58(m, 7H), 1.72(s, 6H) | 728.92 | 728.32 |
| 1136 | δ = 7.83(m, 1H), 7.75(m, 1H), 7.62-7.34(m, 24H), 7.20 (m, 4H), 7.06(m, 2H), 6.85-6.81(m, 7H), 6.63-6.58(m, 7H), 1.72(s, 6H) | 933.19 | 932.41 |
| 1140 | δ = 7.83(m, 1H), 7.75(m ,1H), 7.62-7.58(m, 2H), 7.34(m, 2H), 7.20(m, 4H), 7.01(m, 4H), 6.81-6.75(m, 3H), 6.63-6.55(m, 11H), 1.72 (s, 6H), 1.35(s, 18H) | 741.01 | 740.41 |
| 1142 | δ = 7.83(m, 1H), 7.75(m, 1H), 7.62-7.58(m, 2H), 7.34(m, 2H), 7.20(m, 4H), 6.81-6.75(m, 3H), 6.63-6.58(m, 7H), 6.24(m ,4H), 2.34(s, 12H), 2.18(s, 6H), 1.72 (s, 6H) | 712.96 | 712.38 |
| 1154 | δ = 7.93(m, 2H), 7.83(m, 1H), 7.77-7.75(m, 3H), 7.63-7.51(m, 14H), 7.41-7.34(m ,4H), 7.20(m, 4H), 6.81-6.75(m, 5H), 1.72 (s, 18H) | 1013.31 | 1012.48 |
| 1159 | δ = 8.00-7.92(m, 8H), 7.83(m, 1H), 7.77-7.73(m, 5H), 7.63-7.58(m, 12H), 7.34(m ,2H), 7.20(m ,4H), 6.81-6.75(m, 5H), 6.63-6.58(m, 9H), 1.72(s, 18H) | 1113.43 | 1112.51 |
| 1174 | δ = 7.88-7.759m, 12H), 7.62-7.49(m ,10H), 7.38-7.28(m, 8H0, 6.75(m, 3H), 6.58(m, 5H), 1.72(s, 18H) | 961.24 | 960.44 |
| 1177 | δ = 7.88-7.74(m, 10H), 7.62-7.36(m, 24H), 6.75(m, 1H), 6.69(m, 4H), 6.59-6.58(m, 3H), 1.72(s, 6H) | 880.11 | 880.38 |
| 1180 | δ = 7.88-7.74(m, 10H), 7.62-7.34(m, 30H), 7.06(m, 2H), 6.85(m, 4H), 6.75(m ,1H), 6.59-6.58(m, 3H), 1.72(s, 6H) | 1033.30 | 1032.44 |
| 1181 | δ = 7.88-7.74(m, 10H), 7.62-7.49(m, 6H), 7.36-7.34(m, 4H), 6.99(m, 4h), 6.75(m, 1h), 6.61-6.58(m, 7H), 1.72(s, 6H) | 756.97 | 756.35 |
| 1182 | δ =7.88-7.74(m, 10H), 7.62-7.49(m, 6H), 7.36-7.34(m, 4H), 6.99(m, 4H), 6.75(m ,1H), 6.61-6.58(m, 7H), 1.72(s, 6H) | 764.90 | 764.30 |
| 1184 | δ = 7.88-7.74(m, 10H), 7.62 7.58(m ,2H), 7.50-7.49(m, 4H), 7.36- 7.34(m, 4H), 7.01(m, 4H), 6.75(m, 1h), 6.58-6.55(m, 7H), 1.72(s, 6H), 1.35(s, 18H) | 841.13 | 840.44 |
| 1185 | δ = 7.88-7.74(m, 10H), 7.62-7.49(m, 6H), 7.36-7.34(m, 4H), 6.75-6.74(m, 5H), 6.59-6.52(m, 7H), 3.83(s, 6H), 1.72(s, 6H) | 788.97 | 788.34 |
| 1189 | δ = 7.88-7.74(m, 10H), 7.62-7.49(m, 6H), 7.37-7.34(m, 8H), 6.75(m, 1H), 6.59-6.56(m, 7H), 1.72(s, 6H) | 864.92 | 864.29 |
| 1190 | δ = 7.88-7.74(m, 10H), 7.62-7.58(m, 2H), 7.50-7.49(m, 4H), 7.36-7.34(m, 4H), 7.15(m, 4H), 6.75(m, 1H), 6.61-6.59(m, 7H), 1.72(s, 6H), 0.25(s, 18H) | 873.28 | 872.40 |
| 1192 | δ = 8.07(m, 2H), 7.88-7.74(m, 10H), 7.62-7.49(m, 8H), 7.36-7.34(m, 4H), 6.75-6.58(m, 8H), 1.72(s, 6H) | 730.90 | 730.31 |
| 1197 | δ = 8.18(m, 2H), 8.01(m, 2H), 7.88-7.74(m, 10H), 7.58-7.49(m, 10H), 7.36-7.34(m, 4H), 6.75(m, 1H), 6.59-6.58(m, 3H), 1.72(s, 6H) | 843.07 | 842.25 |
| 1221 | δ = 8.07-8.02(m, 4H), 7.83(m, 1H), 7.75(m ,1H), 7.62-7.38(m, 20H), 7.16-7.08(m, 6H), 6.98(m, 2H), 6.87(m ,2H), 6.75-6.69(m, 3H), 6.58(m, 3H), 1.72(s, 6H) | 881.11 | 880.38 |
| 1227 | δ = 8.07-7.02(m, 4H), 7.83(m, 1H), 7.75(m, 1H), 7.62-7.53(m, 8H), 7.38-7.34(m, 4H), 7.01-6.98(m, 6H), 6.75(m, 1H), 6.59-6.55(m, 7H), 1.72(s, 6H), 1.35(s, 18H) | 841.13 | 840.44 |
| 1248 | δ = 8.99-8.93(m, 4H), 8.34(m, 2H), 8.12-8.02(m, 8H), 7.88-7.38(m, 34H), 6.98(m, 2H), 6.75(m, 1H), 6.59-6.58(m, 3H), 1.72(s, 6H) | 1181.46 | 1180.48 |
| 1259 | δ = 8.93(m, 4H), 8.12(m, 4H), 7.88-7.75(m, 12H), 7.62-7.55(m, 6H), 7.38-7.28(m, 6H), 6.91(m ,2H), 6.75(m, 3H), 6.58(m, 5H), 1.72(s, 18H) | 1061.36 | 1060.48 |
| 1266 | δ = 8.93(m, 4H), 8.12(m, 4H), 7.88-7.75(m, 10H), 7.62-7.58(m, 2H), 7.34(m, 2H), 6.98-6.91(m ,6H), 6.75(m, 1H), 6.59-6.51(m, 7H), 2.34(s, 6H), 1.72(s, 6H) | 857.09 | 856.38 |
| 1272 | δ = 8.93(m, 4H), 8.12(m, 4H), 7.88-7.75(m, 10H), 7.62-7.58(m, 2H), 7.34(m, 2H), 6.91(m, 2H), 6.75(m, 1H), 6.59(m, 3H), 6.22(m, 2H), 2.34(s, 12H), 2.12(s, 12H), 1.72(s, 6H) | 941.25 | 940.48 |
| 1297 | δ = 8.93(m, 4H), 8.12(m, 4H), 7.88-7.75(m, 10H), 7.62-7.58(m, 2H), 7.34(m, 2H), 6.91-6.84(m, 4H), 6.75-6.72(m, 3H), 6.59-6.58(m, 3H), 6.01(m, 2H), 1.72(s, 6H) | 841.09 | 840.26 |
| 1314 | δ = 7.87-7.83(m, 3H), 7.75(m, 1H), 7.62-7.55(m, 6H), 7.38-7.28(m, 6H), 6.75-6.71(m, 5H), 6.58(m, 5H), 6.36(m, 4H), 2.34(s, 12H), 1.72(s, 18H) | 917.23 | 916.48 |
| 1348 | δ = 7.83(m, 1H), 7.75(m, 1H), 7.62-7.58(m, 2H), 7.34(m, 2H), 7.20(m, 4H), 6.81-6.71(m, 9H), 6.64-6.58(m, 13H), 6.39-6.23(m, 6H), 3.66(m, 4H), 1.72(s, 6H) | 881.11 | 880.38 |
| 1382 | δ = 7.83(m, 1H), 7.75(m, 1H), 7.62 7.41(m, 30H), 6.89-6.88(m, 4H), 6.75(m, 1H), 6.69(m, 4H), 6.59-6.58(m, 5H), 1.72(s, 6H) | 933.19 | 932.41 |
| 1386 | δ = 7.83(m, 1H), 7.75(m, 1H), 7.62-7.41(m, 16H), 6.98(m, 4H), 6.89-6.88(m, 4H), 6.75(m, 1H), 6.59-6.51(m,9H), 2.34(s, 6H), 1.72(s, 6H) | 809.05 | 808.38 |
| 1390 | δ = 7.83(m, 1H), 7.75(m, 1H), 7.62 7.41(m, 16H), 6.88(m, 4H), 6.75(m, 5H), 6.59-6.52(m, 9H), 3.83(s, 6H), 1.72(s, 6H) | 841.05 | 840.37 |
| 1400 | δ = 7.83-7.75(m, 3H), 7.62-7.41(m, 15H), 7.09(m, 2H), 6.97-6.95(m, 3H), 6.79(m, 5H), 3.65(m, 8H), 3.11(m, 8H), 1.72(s, 6H) | 799.01 | 798.39 |
| 1405 | δ = 8.49(m ,2H), 8.07(m, 2H), 7.93-7.75(m, 10H), 7.62-7.41(m, 28H), 7.04(m ,2H), 6.88(m ,4H), 6.75(m, 1H), 6.59(m, 5H), 1.72(s, 18H) | 1265.62 | 1264.57 |
| 1429 | δ = 7.83(m, 1H), 7.62-7.51(m, 14H), 7.41-7.34(m, 4H), 6.75(m ,1H), 6.69(m ,4H), 6.58(m, 3H), 6.24(m ,4H), 2.34(s, 12H), 2.18(s, 6H), 1.72(s, 6H) | 865.15 | 864.44 |
| 1462 | δ = 7.83(m ,1H), 7.75(m ,1H), 7.62-7.51(m, 8H), 7.41-7.34(m, 4H), 7.16-6.99(m, 10H), 6.87(m ,2H), 6.69-6.58(m, 10H), 1.72(s, 6H) | 816.97 | 816.33 |
| 1479 | δ = 7.93-7.83(m, 5H), 7.77-7.75(m ,3H), 7.63-7.51(m, 16H), 7.41-7.28(m, 8H), 7.16(m, 2H), 7.08(m, 4H), 6.87(m ,2H), 6.75(m ,1H), 6.69(m, 6H), 6.58(m, 3H), 1.72 (s, 18H) | 1165.51 | 1164.54 |
| 1500 | δ = 8.42(m ,4H), 8.10(m ,4H), 7.83(m ,1H), 7.75(m, 1H), 7.62-7.49(m, 8H), 7.34(m ,4H), 7.01(m ,4H), 6.91(m ,2H), 6.75(m ,1H), 6.59-6.55(m, 7H), 1.72(s, 6H), 1.35(s, 18H) | 989.29 | 988.48 |
| 1533 | δ = 7.83(m, 1H), 7.75(m, 1H), 7.62-7.41(m ,24H), 7.06-6.99(m, 6H), 6.85(m ,4H), 6.75(m ,1H), 6.61-6.58(m, 7H), 1.72(s, 6H) | 969.17 | 968.39 |
| 1569 | δ = 7.83(m ,1h), 7.75(m ,1H), 7.62-7.58(m ,2H), 7.34(m ,2H), 7.01-6.98(m, 8H), 6.75(m ,1H), 6.58-6.51(m, 11H), 2.34(s, 6H), 1.72(s, 6H), 1.35(s, 18H) | 769.07 | 768.44 |
| 1606 | δ = 7.83(m ,1h), 7.75(m ,1h), 7.62-7.58(m ,2H), 7.34(m ,2H), 6.99(m ,4H), 6.75-6.71(m ,3H), 6.61-6.58(m, 7H), 6.36(m ,4H), 2.34(s, 12H), 1.72(s, 6H) | 720.89 | 720.33 |
| 1739 | δ = 7.93-7.83(m ,5H), 7.77-7.75(m, 3H), 7.63-7.54(m ,10H), 7.38-7.28(m, 6H), 6.75-6.69(m ,5H), 6.58(m ,3h), 6.24(s, 4H), 2.34(s, 12H), 2.18(s, 6H), 1.72(s, 18H) | 1097.47 | 1096.57 |
| 1757 | δ = 7.83(m, 1H), 7.74(m, 1H), 7.62-7.58(m ,2H), 7.37-7.34(m, 6H), 6.75(m ,1H), 6.58-6.56(m ,7H), 6.22(s, 2H), 2.34(s, 12H), 2.12(s, 12H), 1.72(s, 6H) | 877.01 | 876.39 |
| 1793 | δ = 7.93(m, 2H), 7.83(m ,1H), 7.77-7.75(m ,3H), 7.62-7.51(m, 14H), 7.41-7.34(m ,4H)m 6.75-6.71(m, 5H), 6.58(m, 5H), 6.36(m, 4H), 2.34(s, 12H), 1.72(s, 18H) | 1069.42 | 1068.54 |
| 1844 | δ = 7.93-7.83(m, 5H), 7.77-7.75(m, 3H), 7.62-7.54(m, 10H), 7.38-7.28(m, 6H), 7.38-7.28(m, 6H), 7.15(m, 4H), 6.75-6.69(m, 5H), 6.61-6.58(m, 7H), 1.72(s, 18H), 0.25(s, 18H) | 1157.68 | 1156.55 |
| 1933 | δ = 8.28(m ,2H), 8.17(m ,2H), 7.93(m ,2H), 7.83(m, 1H), 7.77-7.75(m, 3H), 7.62-7.41(m, 24H), 6.99(m, 2H), 6.75(m, 3H), 6.59-6.58(m, 5H), 1.72(s, 18H) | 1115.41 | 1114.50 |
| 2008 | δ = 7.93-7.83(m, 7H), 7.77-7.75(m, 5H), 7.63-7.51(m, 22H), 7.41-7.28(m, 8H), 6.75(m, 3H), 6.69(m, 4H), 6.58(m, 5H), 14.72(s, 30H) | 1397.83 | 1396.66 |
| 2193 | δ = 8.47-8.35(m, 10H), 7.83(m, 1H), 7.75(m, 1H), 7.62-7.58(m ,2H), 7.34(m ,2H), 6.75(m ,1H), 6.59- 6.58(m ,2H), 1.72(s, 6H) | 638.68 | 638.24 |
| 2161 | δ = 8.02(m, 2H), 7.54-7.53(m ,2H), 7.35-7.34(m ,3H), 7.20(m, 8H), 6.94(m ,1H), 6.81(m ,4H), 6.63(m, 10H) | 562.70 | 562.24 |
| 2207 | δ = 8.21(m, 1H), 7.84(m, 1H), 7.74(m, 1H), 7.54-7.48(m, 3H), 7.34(m, 2H), 7.20(m, 8H), 6.81(m, 4H), 6.63(m, 10H) | 562.70 | 562.24 |
| 2300 | δ = 8.89(m, 1H), 8.68(m, 1H), 8.32(m ,1H), 8.08(m ,1H), 7.71(m ,2H), 7.34-7.32(m, 3H), 7.20(m, 8H), 7.08(m, 1H), 6.81(m, 4H), 6.63(m, 10H) | 612.76 | 612.26 |
| 2347 | δ = 8.93-8.89(m, 2H), 8.32(m, 1H), 8.13-8.08(m ,3H), 7.88-7.82(m ,3H), 7.34(m ,2H), 7.20(m, 8H), 7.02(m, 1H), 6.81(m, 4H), 6.63(m, 10H) | 662.82 | 662.27 |
| 2391 | δ = 7.83(m, 1H), 7.75(m, 3H), 7.62-7.58(m, 2H), 7.35-7.34(m, 4H), 7.20-7.16(m, 12H), 6.81-6.75(m, 5H), 6.63-6.58(m, 11H) | 750.93 | 750.30 |
| 2437 | δ = 7.83(m, 1H), 7.75(m ,1H), 7.62-7.58(m ,2H), 7.34-7.20(m ,20H), 6.81-6.75(m, 5H), 6.63-6.58(m, 11H) | 752.94 | 752.32 |
| 2483 | δ = 7.69(m, 2H), 7.33-7.20(m, 14H), 6.87-6.77(m, 6H), 6.63-6.61(m, 7H), 6.31-6.23(m, 3H), 4.59(m ,1H), 3.66(m 1H) | 638.80 | 628.27 |
| 2529 | δ = 8.05(m ,1H), 7.81-7.77(m ,2H), 7.69-7.62(m ,3H), 7.34(m, 2H), 7.20(m, 8H), 6.81-6.75(m, 5H), 6.63-6.58(m, 11H), 1.72(s, 12H) | 744.96 | 744.35 |
| 2575 | δ = 7.91-7.87(m, 3H), 7.74(m ,1H), 7.59(m ,1H), 7.39-7.34(m ,6H), 7.20(m, 8H), 6.81(m ,4H), 6.63(m, 11H) | 686.84 | 686.27 |
| 2621 | δ = 9.13(m, 1H), 8.93(m ,2H), 8.12(m 2H), 7.88-7.82(m, 4H), 7.34(m 2H), 7.20(m, 8H), 6.91(m 1H), 6.81(m, 4H), 6.63(m, 10H) | 662.82 | 662.27 |

### [Example 1] Manufacture of OLED's by using the compounds of the invention

An OLED device was manufactured by using the electroluminescent compound according to the invention.

First, a transparent electrode ITO thin film (15 Ω/□) prepared from glass for OLED was subjected to ultrasonic washing with trichloroethylene, acetone, ethanol and distilled water, sequentially, and stored in isopropanol before use.

Then, an ITO substrate was equipped in a substrate folder of a vacuum vapor-deposit device, and 4,4',4"-tris(N,N-(2-naphthyl)-phenylamino)triphenylamine (2-TNATA) (of which the structure is shown below) was placed in a cell of the vacuum vapor-deposit device, which was then ventilated up to 10⁻⁶ torr of vacuum in the chamber. Electric current was applied to the cell to evaporate 2-TNATA, thereby providing vapor-deposit of a hole injection layer having 60 nm of thickness on the ITO substrate.

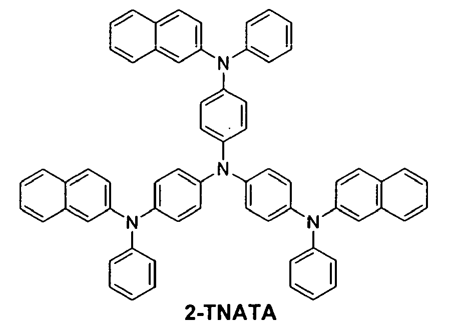

Then, to another cell of the vacuum vapor-deposit device, charged was N,N'-bis(α-naphthyl)-N,N'-diphenyl-4,4'-diamine (NPB) (of which the structure is shown below), and electric current was applied to the cell to evaporate NPB, thereby providing vapor-deposit of a hole transport layer of 20 nm of thickness on the hole injection layer.

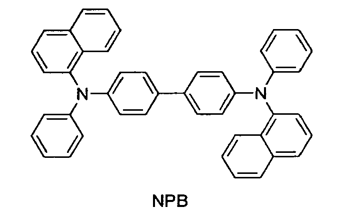

After forming the hole injection layer and hole transport layer, an electroluminescent layer was vapor-deposited as follows. To one cell of a vacuum vapor-deposit device, charged was dinaphthylanthracene (DNA) (of which the structure is shown below) as electroluminescent material, and a compound according to the invention (e.g. Compound (2545)) was charged to another cell. An electroluminescent layer was vapor-deposited with a thickness of 30 nm on the hole transport layer at a vapor-deposition rate of 100:1.

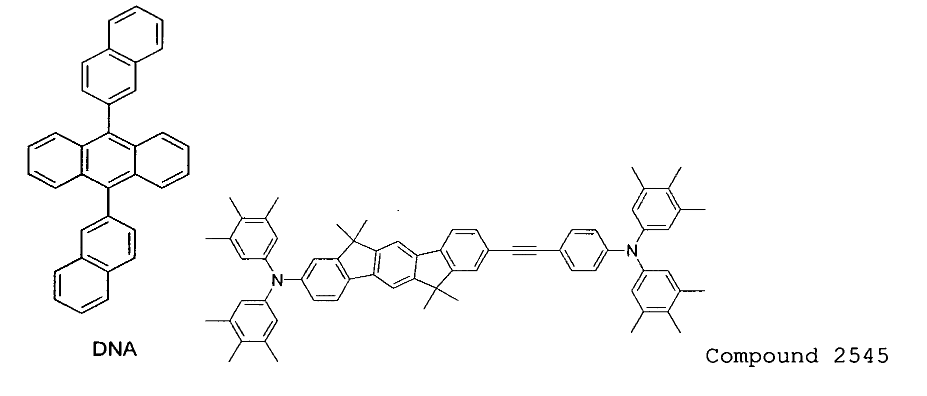

Then, tris(8-hydroxyquinoline)aluminum (III) (Alq) (of which the structure is shown below) was vapor-deposited as an electron transport layer with a thickness of 20 nm, and lithium quinolate (Liq) (of which the structure shown below) was vapor-deposited as an electron injection layer with a thickness of 1 to 2 nm. Thereafter, an Al cathode was vapor-deposited with a thickness of 150 nm by using another vacuum vapor-deposit device to manufacture an OLED.

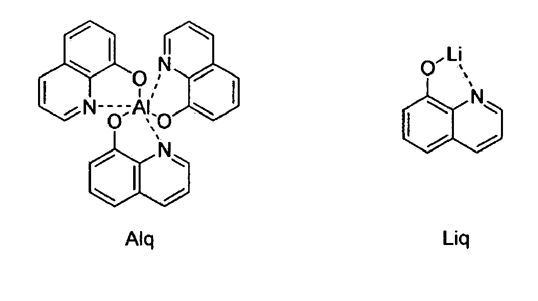

Each material employed for manufacturing an OLED was used as the electroluminescent material after purifying via vacuum sublimation under 10⁻⁶ torr.

### [Comparative Example 1] Manufacture of an OLED by using conventional electroluminescent material

After forming a hole injection layer and hole transport layer according to the same procedure described in Example 1, dinaphthylanthracene (DNA) was charged to one cell of said vacuum vapor-deposit device as a blue electroluminescent material, and Compound (A) (of which the structure is shown below) was charged to another cell as another blue electroluminescent material. Then an electroluminescent layer having 30 nm of thickness was vapor-deposited on the hole transport layer at the vapor-deposition rate of 100:1.

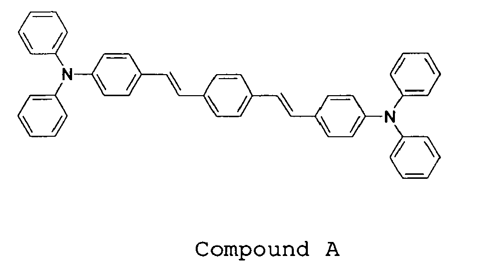

Then, an electron transport layer and electron injection layer were vapor-deposited according to the same procedure of Example 1, and an Al cathode was vapor-deposited thereon with a thickness of 150 nm by using another vacuum vapor-deposit device to manufacture an OLED.

### [Example 2] Electroluminescent properties of OLED's manufactured

The luminous efficiencies of the OLED's comprising the organic EL compound according to the present invention (Examples 1) or conventional EL compound (Comparative Example 1) were measured at 1,000 cd/m², and the results are shown in Table 3.

**Table 3**

| NO. | EL material 1 | EL material 2 | Luminous efficiency (cd/A) | Color | Luminous efficiency/Y |
|---|---|---|---|---|---|
| 1 | DNA | Compound 2 | 6.2 | Blue | 47.7 |
| 2 | DNA | Compound 12 | 5.9 | Blue | 45.4 |
| 3 | DNA | Compound 34 | 5.1 | Blue | 42.5 |
| 4 | DNA | Compound 347 | 6.2 | Blue | 47.7 |
| 5 | DNA | Compound 713 | 6.7 | Blue | 51.5 |
| 6 | DNA | Compound 817 | 7.2 | Blue | 51.4 |
| 7 | DNA | Compound 1084 | 5.7 | Blue | 43.8 |
| 8 | DNA | Compound 1115 | 6.5 | Blue | 50.0 |
| 9 | DNA | Compound 1186 | 6.6 | Blue | 50.7 |
| 10 | DNA | Compound 1427 | 5.9 | Blue | 49.2 |
| 11 | DNA | Compound 1684 | 7.1 | Blue | 50.7 |
| 12 | DNA | Compound 2189 | 7.0 | Blue | 53.8 |
| 13 | DNA | Compound 2227 | 6.8 | Blue | 52.3 |
| 14 | DNA | Compound 2301 | 7.0 | Blue | 53.8 |
| 15 | DNA | Compound 2395 | 5.8 | Blue | 44.6 |
| 16 | DNA | Compound 2450 | 6.2 | Blue | 47.7 |
| 17 | DNA | Compound 2545 | 7.7 | Blue | 54.2 |
| 18 | DNA | Compound 2585 | 7.1 | Blue | 53.6 |
| 19 | DNA | Compound 2628 | 6.7 | Blue | 47.8 |
| 20 | DNA | Compound 2677 | 6.3 | Blue | 51.0 |
| 21 | DNA | Compound 2694 | 6.2 | Blue | 47.3 |
| 22 | DNA | Compound 2713 | 7.2 | Blue | 49.2 |
| Comp. Ex.1 | DNA | Compound A | 7.3 | Jade green | 35.8 |

As can be seen from Table 3, it is found that the OLED's employing the organic EL compounds according to the present invention exhibited higher "luminous efficiency/Y" value (which shows similar tendency to quantum efficiency) as compared to an OLED comprising DNA: Compound (A) as conventional EL material (Comparative Example 1).

Accordingly, it is found that acetylene as backbone or the organic electroluminescent compounds according to the invention contributes to a material of high quantum efficiency, and the compounds enable realization higher efficiency and better color purity than conventional EL compounds. Particularly, Compound (2545) exhibited enhanced "luminous efficieny/Y" value by at least 60% as compared to the conventional EL material.

It is anticipated that the molecular structure comprising a triple bond, rather than the structure comprised of simple aromatic conjugation, provides the effect of enhancing overlap between orbitals of individual aromatic rings in the molecular structure to result in improved performance.

As shown above, the organic electroluminescent compounds according to the present invention can be employed as blue electroluminescent material of high efficiency, and provide advantages in terms of luminance, power consumption and device life, as being employed in OLED's, as compared to conventional full-colored OLED's.

## Claims

1. An organic electroluminescent compound represented by Chemical Formula (1): 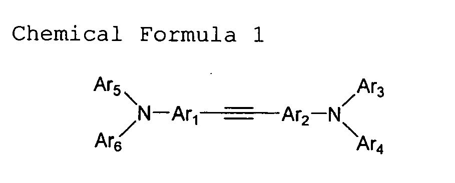 wherein,
Ar₁ and Ar₂ independently represent (C6-C60)arylene or (C5-C60)heteroarylene, and the arylene or heteroarylene of Ar₁ and Ar₂ may be further substituted by one or more substituent(s) selected from deuterium, linear or branched (C1-C60)alkyl and (C6-C60)aryl;
Ar₃ through Ar₆ independently represent linear or branched (C1-C60)alkyl, (C3-C60)cycloalkyl, 5- or 6-membered heterocycloalkyl containing one or more heteroatom(s) selected from N, O and S, (C6-C60)aryl or (C3-C60)heteroaryl, or Ar₃ and Ar₅, or Ar₆ and Ar₇ may be linked via (C3-C60)alkylene or (C3-C60)alkenylene with or without a fused ring to form an alicyclic ring, or a monocyclic or polycyclic aromatic ring; and
the aryl or heteroaryl of Ar₃ through Ar₆ may be further substituted by one or more substituent(s) selected from a group consisting of deuterium, (C6-C60)aryl with or without linear or branched (C1-C60)alkyl or (C6-C60)aryl substituent, linear or branched (C1-C60)alkyl with or without halogen substituent(s), (C1-C30)alkoxy, (C3-C60)cycloalkyl, halogen, cyano, tri(C1-C30)alkylsilyl, di(C1-C30)alkyl(C6-C30)arylsilyl and tri(C6-C30)arylsilyl.

2. The organic electroluminescent compound according to claim 1, wherein Ar₁ and Ar₂ are independently selected from from the following structures: 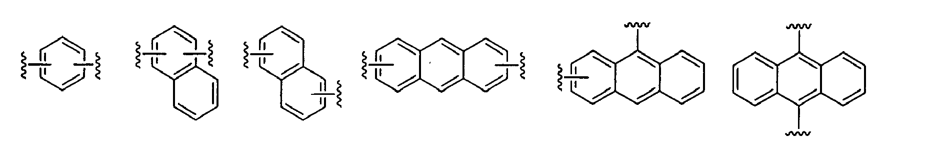 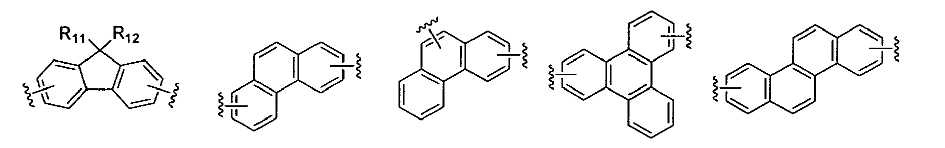 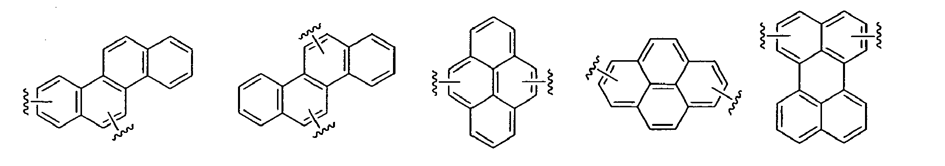 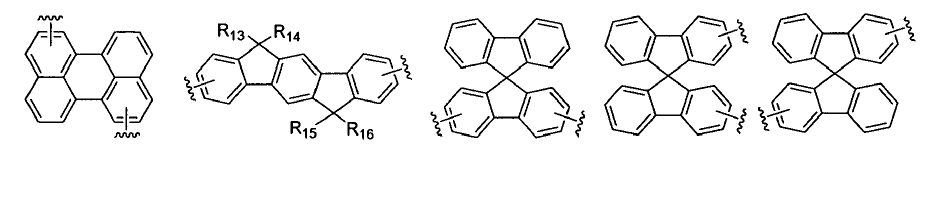 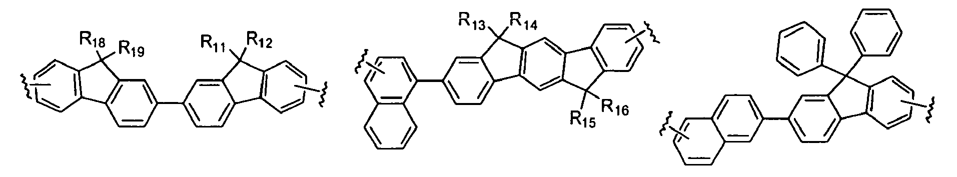 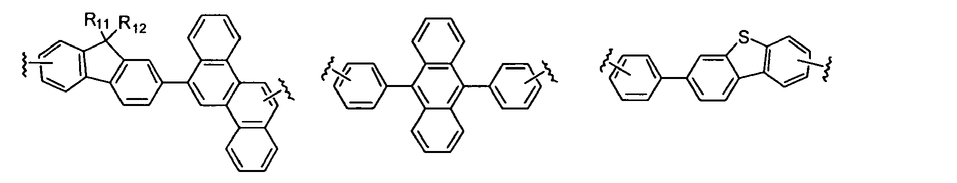 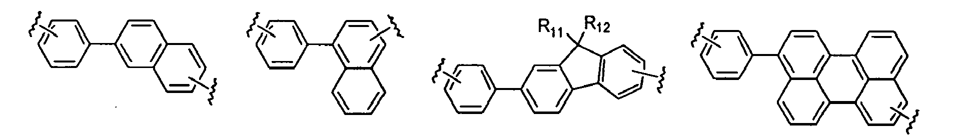 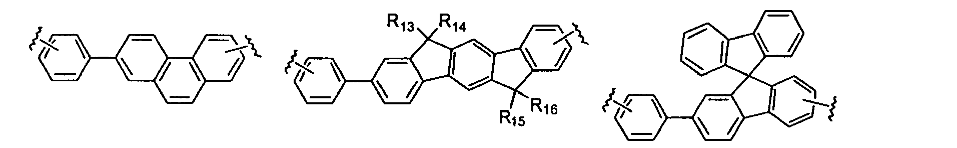 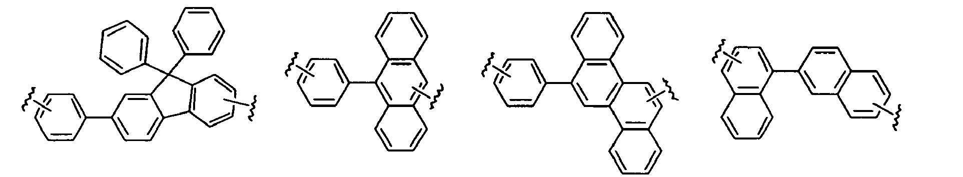 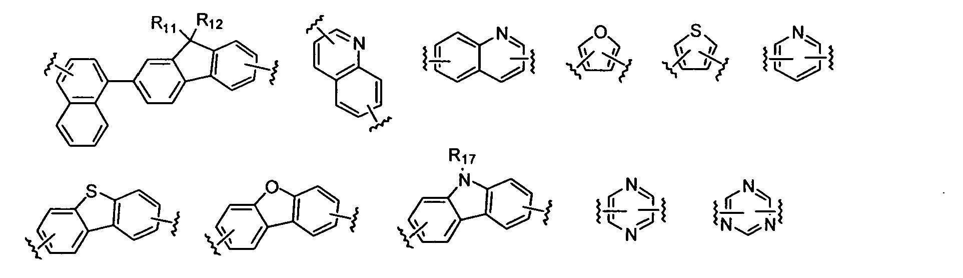 wherein, R₁₁ through R₁₉ independently represent hydrogen, linear or branched (C1-C60)alkyl or (C6-C60)aryl, and the aryl may be further substituted by linear or branched (C1-C60)alkyl.

3. An organic electroluminescent device which is comprised of a first electrode; a second electrode; and at least one organic layer(s) interposed between the first electrode and the second electrode; wherein the organic layer comprises one or more organic electroluminescent compound(s)represented by the Chemical Formula (1): 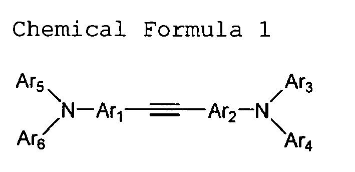 wherein,
Ar₁ and Ar₂ independently represent (C6-C60)arylene or (C5-C60)heteroarylene, and the arylene or heteroarylene of Ar₁ and Ar₂ may be further substituted by one or more substituent(s) selected from deuterium, linear or branched (C1-C60)alkyl and (C6-C60)aryl;
Ar₃ through Ar₆ independently represent linear or branched (C1-C60)alkyl, (C3-C60)cycloalkyl, 5- or 6-membered heterocycloalkyl containing one or more heteroatom(s) selected from N, O and S, (C6-C60)aryl or (C3-C60)heteroaryl, or Ar₃ and Ar₅, or Ar₆ and Ar₇ may be linked via (C3-C60)alkylene or (C3-C60)alkenylene with or without a fused ring to form an alicyclic ring, or a monocyclic or polycyclic aromatic ring; and
the aryl or heteroaryl of Ar₃ through Ar₆ may be further substituted by one or more substituent(s) selected from a group consisting of deuterium, (C6-C60)aryl with or without linear or branched (C1-C60)alkyl or (C6-C60)aryl substituent, linear or branched (C1-C60)alkyl with or without halogen substituent(s), (C1-C30)alkoxy, (C3-C60)cycloalkyl, halogen, cyano, tri(C1-C30)alkylsilyl, di(C1-C30)alkyl(C6-C30)arylsilyl and tri(C6-C30)arylsilyl.
and one or more host(s) selected from the compounds represented by Chemical Formula (2) or (3):
Chemical Formula 2 (Ar₁₁)ₐ-A- (Ar₁₂)_{b}
Chemical Formula 3 (Ar₁₁)ₐ-An-(Ar₁₂)_{b}
wherein,
Ar₁₁ and Ar₁₂ are independently selected from hydrogen, (C1-C60)alkyl, (C1-C60)alkoxy, halogen, (C4-C60)heteroaryl, (C5-C60)cycloalkyl and (C6-C60)aryl; and the cycloalkyl, aryl or heteroaryl of Ar₁₁ and Ar₁₂ may be further substituted by one or more substituent(s) selected from a group consisting of (C6-C60)aryl or (C4-C60)heteroaryl with or without one or more substituent(s) selected from a group consisting of deuterium, (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arvlsilvl; (C1-C60)alkyl with or without halogen substituent(s), (C1-C60)alkoxy, (C3-C60)cycloalkyl, halogen, cyano, tri(C1-C60)alkylsilyl, di(C1-C60)alkyl(C6-C60)arylsilyl and tri(C6-C60)arylsilyl;
A represents (C6-C60)arylene or (C4-C60)heteroarylene;
a and b independently represent an integer from 0 to 4; and
An comprises anthracene backbone with or without a substituent.

4. The organic electroluminescent device according to claim 3, wherein the organic layer comprises one or more compound(s) selected from a group consisting of arylamine compounds and styrylarylamine compounds.

5. The organic electroluminescent device according to claim 3, wherein the organic layer comprises one or more metal(s) selected from a group consisting of organic metals of Group 1, Group 2, 4^{th} period and 5^{th} period transition metals, lanthanide metals and d-transition elements from the Periodic Table of Elements.

6. The organic electroluminescent device according to claim 3, which is an organic display comprising a compound having the electroluminescent peak with wavelength of 500 to 560 nm.

7. The organic electroluminescent device according to claim 3, wherein the organic layer comprises an electroluminescent layer and a charge generating layer.

8. The organic electroluminescent device according to claim 3, wherein a mixed region of reductive dopant and organic substance, or a mixed region of oxidative dopant and organic substance is placed on the inner surface of one or both electrode(s) among the pair of electrodes.

9. An organic solar cell which comprises an organic electroluminescent compound represented by Chemical Formula (1): 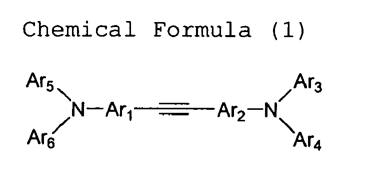 wherein,
Ar₁ and Ar₂ independently represent (C6-C60)arylene or (C5-C60)heteroarylene, and the arylene or heteroarylene of Ar₁ and Ar₂ may be further substituted by one or more substituent(s) selected from deuterium, linear or branched (C1-C60)alkyl and (C6-C60)aryl;
Ar₃ through Ar₆ independently represent linear or branched (C1-C60)alkyl, (C3-C60)cycloalkyl, 5- or 6-membered heterocycloalkyl containing one or more heteroatom(s) selected from N, O and S, (C6-C60)aryl or (C3-C60)heteroaryl, or Ar₃ and Ar₅, or Ar₆ and Ar₇ may be linked via (C3-C60) alkylene or (C3-C60)alkenylene with or without a fused ring to form an alicyclic ring, or a monocyclic or polycyclic aromatic ring; and
the aryl or heteroaryl of Ar₃ through Ar₆ may be further substituted by one or more substituent(s) selected from a group consisting of deuterium, (C6-C60)aryl with or without linear or branched (C1-C60)alkyl or (C6-C60)aryl substituent, linear or branched (C1-C60)alkyl with or without halogen substituent(s), (C1-C30)alkoxy, (C3-C60)cycloalkyl, halogen, cyano, tri(C1-C30)alkylsilyl, di(C1-C30)alkyl(C6-C30)arylsilyl and tri(C6-C30)arylsilyl.
